(19) Europäisches Patentamt
European Patent Office
Office européen des brevets

(11) EP 1 140 895 B1

(12) EUROPEAN PATENT SPECIFICATION

(45) Date of publication and mention of the grant of the patent:
**24.03.2004 Bulletin 2004/13**

(21) Application number: **00900122.3**

(22) Date of filing: **06.01.2000**

(51) Int Cl.[7]: **C07D 335/02**, A61K 31/38, A61K 31/385, A61K 31/44, C07D 339/08, C07D 333/48, C07D 409/04, C07D 409/12, C07D 409/14, A61P 29/00, A61P 31/18

(86) International application number:
**PCT/JP2000/000018**

(87) International publication number:
**WO 2000/040576 (13.07.2000 Gazette 2000/28)**

(54) **THIOPYRAN COMPOUNDS AS INHIBITORS OF MMP**

THIOPYRANDERIVATE AS MMP-INHIBITOREN

DERIVES DE THIOPYRANE COMME INHIBITEURS DE MMP

(84) Designated Contracting States:
**AT BE CH CY DE DK ES FI FR GB GR IE IT LI LU MC NL PT SE**

(30) Priority: **07.01.1999 AU PP806899**
**19.07.1999 AU PQ170299**

(43) Date of publication of application:
**10.10.2001 Bulletin 2001/41**

(73) Proprietor: **FUJISAWA PHARMACEUTICAL CO., LTD.**
**Osaka-shi Osaka 541-8514 (JP)**

(72) Inventors:
- **TANIGUCHI, Kiyoshi**
  **Kobe-shi Hyogo 654-0153 (JP)**
- **NEYA, Masahiro**
  **Tsuchiura-shi Ibaraki 300-0065 (JP)**
- **TERASAWA, Takeshi**
  **Izumi-shi Osaka 594-0041 (JP)**
- **YAMAZAKI, Hitoshi**
  **Tsukuba-shi Ibaraki 305-0035 (JP)**
- **SATO, Kentaro**
  **Tsukuba-shi Ibaraki 305-0035 (JP)**
- **HOSOI, Kumi**
  **Susono-shi Shizuoka, 410-1128 (JP)**
- **TOMISHIMA, Yasuyo**
  **Osaka-shi Osaka 531-0072 (JP)**
- **YOSHIDA, Noriko**
  **Tsukuba-shi Ibaraki 305-0035 (JP)**
- **IMAMURA, Yoshimasa**
  **Tsukuba-shi Ibaraki 305-0035 (JP)**
- **TAKASUGI, Hisashi**
  **Sakai-shi Osaka 591-8031 (JP)**
- **SETOI, Hiroyuki**
  **Tsukuba-shi Ibaraki 305-0044 (JP)**

(74) Representative:
**Gille Hrabal Struck Neidlein Prop Roos**
**Patentanwälte,**
**Brucknerstrasse 20**
**40593 Düsseldorf (DE)**

(56) References cited:
**EP-A- 0 326 297**

- **I. STAHL ET AL.: "2,2-DISUBSTITUIERTE 1,3-DITHIANE" CHEMISCHE BERICHTE., vol. 113, no. 2, 1980, pages 800-5, XP002151298 VERLAG CHEMIE GMBH. WEINHEIM., DE ISSN: 0009-2940**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 140 895 B1

## Description

Field of the Invention

The present invention relates to new compounds and pharmaceutically acceptable salts thereof.

More particularly, it relates to new compounds and pharmaceutically acceptable salts thereof which are useful as inhibitors of matrix metalloproteinases (hereinafter to be referred to as MMP) or the production of tumor necrosis factor α (hereinafter to be referred to as TNF α), to pharmaceutical compositions comprising the same, to use of the same as medicaments, and to methods for using the same for the manufacture of medicaments in the treatment and/or the prevention of MMP- or TNF α-mediated diseases.

Background Art

Some compounds to be useful as metalloproteinase inhibitors, or the like are known (WO 97/20824, etc.).

Disclosure of the Invention

One object of the present invention is to provide new and useful cyclic compounds and pharmaceutically acceptable salts thereof, and to provide a process for preparing said new cyclic compound and salts thereof, which have pharmacological activities such as MMP- or TNF α- inhibitory activity and the like.

Another object of the present invention is to provide a pharmaceutical composition comprising, as an active ingredient, said cyclic compound or a pharmaceutically acceptable salt thereof.

A further object of the present invention is to provide use of said cyclic compounds and pharmaceutically acceptable salts thereof as medicaments for prophylactic and therapeutic treatment of MMP- or TNF α-mediated diseases.

The compounds of the present invention have inhibitory activity on MMP or the production of TNF α, and are useful for the treatment and/or prevention of diseases such as stroke, arthritis, cancer, tissue ulceration, decubitus ulcer, restenosis, periodontal disease, epidermolysis bullosa, scleritis, psoriasis and other diseases characterized by matrix metalloproteinase activity, as well as AIDS, sepsis, septic shock and other diseases caused by the production of TNF α.

There are a number of structurally related metalloproteases which effect the breakdown of structural proteins. Matrix-degrading metalloproteases, such as gelatinase (MMP-2, MMP-9), stromelysin (MMP-3) and collagenase (MMP-1, MMP-8, MMP-13), are involved in tissue matrix degradation and have been implicated in many pathological conditions involving abnormal connective tissue and basement membrane matrix metabolism, such as arthritis (e.g., osteoarthritis and rheumatoid arthritis, etc.), cerebral disease (e.g., stroke, etc.), tissue ulceration (e.g., corneal, epidermal and gastric ulcerations, etc.), abnormal wound healing, periodontal disease, bone disease (e.g., Paget's disease and osteoporosis, etc.), tumor metastasis or invasion and HIV-infection.

A tumor necrosis factor is recognized to be involved in many infections and autoimmune diseases. Furthermore, it has been shown that TNF is the prime mediator of the inflammatory response seen in sepsis and septic shock.

The object compounds of the present invention are novel and can be represented by the following formula (I):

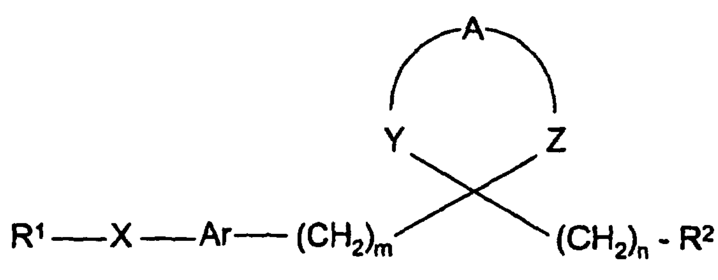

(I)

in which $R^1$ is ($C_1$-$C_6$) alkyl, optionally substituted heterocyclic group or optionally substituted ($C_6$-$C_{10}$)aryl,
$R^2$ is carboxy, protected carboxy, hydroxyaminocarbonyl, tetrahydropyranyloxyaminocarbonyl, or phenyl ($C_1$-$C_6$) alkylaminocarbonyl,
Ar is optionally substituted ($C_6$-$C_{10}$)aryl or optionally substituted heterocyclic group,
A is ($C_1$-$C_6$) alkylene,

X is oxa or a single bond,
Y is thia, sulfinyl or sulfonyl,
Z is methylene,
m and n are each an integer of 0 to 6, and
$1 \leq m+n \leq 6$,
and its salt,
wherein the heterocyclic group of $R^1$ and Ar are selected from the group consisting of the following (1) to (14),

(1) unsaturated 3- to 8-membered, heteromonocyclic group containing 1 to 4 nitrogen atoms,
(2) saturated 3- to 8-membered, heteromonocyclic group containing 1 to 4 nitrogen atoms,
(3) unsaturated 3- to 8-membered, heteromonocyclic group containing 1 or 2 sulfur atoms,
(4) unsaturated condensed 7- to 13-membered, heterocyclic group containing 1 to 5 nitrogen atoms,
(5) unsaturated 3- to 8-membered, heteromonocyclic group containing 1 or 2 oxygen atoms,
(6) saturated 3- to 8-membered, heteromonocyclic group containing 1 or 2 oxygen atoms,
(7) unsaturated 3- to 8-membered, heteromonocyclic group containing 1 or 2 oxygen atoms and 1 to 3 nitrogen atoms,
(8) unsaturated condensed 7- to 13-membered, heterocyclic group containing 1 or 2 oxygen atoms,
(9) unsaturated condensed 7- to 13-membered, heterocyclic group containing 1 or 2 sulfur atoms,
(10) saturated 3- to 8-membered, heteromonocyclic group containing 1 or 2 oxygen atoms and 1 to 3 nitrogen atoms,
(11) unsaturated condensed 7- to 13-membered, heterocyclic group containing 1 or 2 oxygen atoms and 1 to 3 nitrogen atoms,
(12) unsaturated 3- to 8-membered, heteromonocyclic group containing 1 or 2 sulfur atoms and 1 to 3 nitrogen atoms,
(13) saturated 3- to 8-membered, heteromonocyclic group containing 1 or 2 sulfur atoms and 1 to 3 nitrogen atoms, and
(14) unsaturated condensed 7- to 13-membered, heterocyclic group containing 1 or 2 sulfur atoms and 1 to 3 nitrogen atoms, and

each of the above-mentioned heterocyclic group and ($C_6$-$C_{10}$) aryl group are optionally substituted by the group consisting of the following (A1) to (A35);

(A1) halogen,
(A2) ($C_1$-$C_6$) alkyl,
(A3) ($C_1$-$C_6$) alkoxy,
(A4) halo ($C_1$-$C_6$) alkyl,
(A5) halo ($C_1$-$C_6$) alkoxy,
(A6) ($C_2$-$C_6$) alkenyl,
(A7) acyl,
(A8) ($C_1$-$C_6$) alkylthio, ($C_1$-$C_6$) alkylsulfinyl, ($C_1$-$C_6$) alkylsulfonyl,
(A9) ($C_6$-$C_{10}$) aryl,
(A10) halo ($C_6$-$C_{10}$) aryl,
(A11) hydroxy,
(A12) hydroxy ($C_1$-$C_6$) alkyl, protected hydroxy ($C_1$-$C_6$) alkyl,
(A13) amino,
(A14) carboxy,
(A15) protected carboxy,
(A16) nitro ($C_2$-$C_6$) alkenyl,
(A17) ($C_1$-$C_6$) alkylenedioxy,
(A18) acylamino,
(A19) nitro.
(A20) ($C_6$-$C_{10}$) aryl ($C_1$-$C_6$) alkoxy,
(A21) carbamoyl ($C_2$-$C_6$) alkenyl optionally N-substituted by the group consisting of ($C_1$-$C_6$) alkyl, ($C_6$-$C_{10}$) aryl, ($C_1$-$C_6$) alkoxy ($C_6$-$C_{10}$)aryl, and halo ($C_6$-$C_{10}$) aryl,
(A22) ($C_1$-$C_6$) alkylaminocarbonyloxy,
(A23) ($C_1$-$C_6$) alkanoyloxy,
(A24) ($C_1$-$C_6$) alkoxy ($C_1$-$C_6$) alkanoyloxy,
(A25) ($C_1$-$C_6$) alkoxycarbonyloxy,

(A26) ($C_2$-$C_6$) alkenoyloxy optionally substituted by heterocyclic group of the above (1) to (14),
(A27) ($C_3$-$C_6$) cycloalkanecarbonyloxy,
(A28) ($C_1$-$C_6$) alkoxy substituted by the group consisting of carboxy, protected carboxy, ($C_1$-$C_6$) alkanoyl, ($C_3$-$C_7$) cycloalkanecarbamoyl, and ($C_1$-$C_6$) alkylcarbamoyl,
(A29) ($C_1$-$C_6$) alkylcarbamoyloxy ($C_1$-$C_6$) alkyl,
(A30) ($C_1$-$C_6$) alkoxycarbonylamino ($C_1$-$C_6$) alkyl,
(A31) amino ($C_1$-$C_6$) alkyl,
(A32) ($C_1$-$C_6$) alkylcarbamoyl ($C_1$-$C_6$) alkyl,
(A33) heterocyclic-carbonylamino, the heterocyclic group being selected from the above (1) to (14) and optionally being substituted N-protective group,
(A34) the above heterocyclic groups (1) to (14) being optionally substituted by ($C_1$-$C_6$) alkyl, and
(A35) oxo,

and its salt.

The object compounds of the present invention can be prepared by the following processes.

<u>Process 1</u>

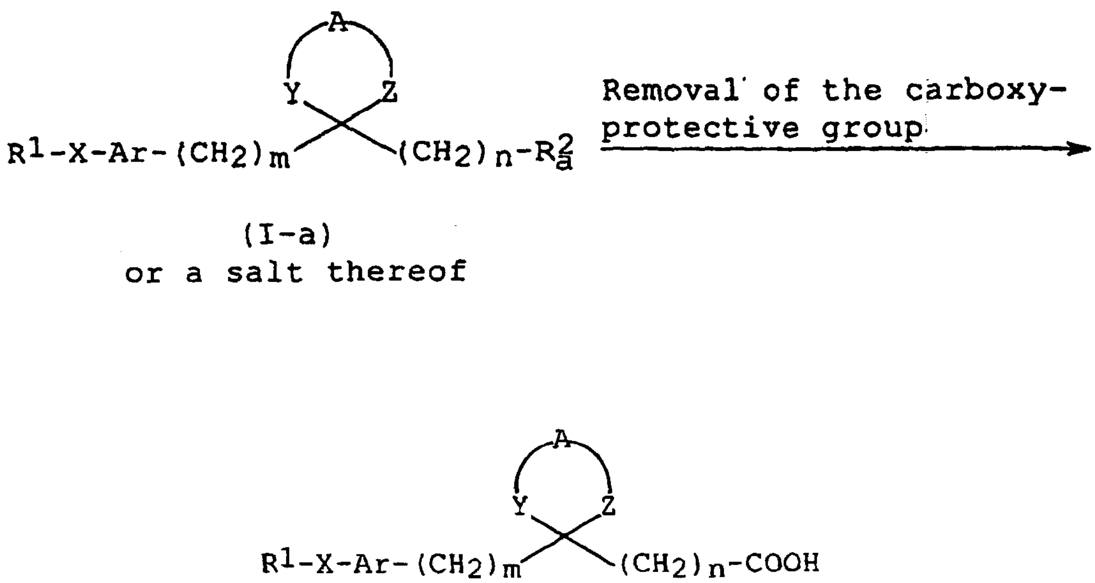


(I-a)
or a salt thereof

(I-b)
or a salt thereof

Process 2

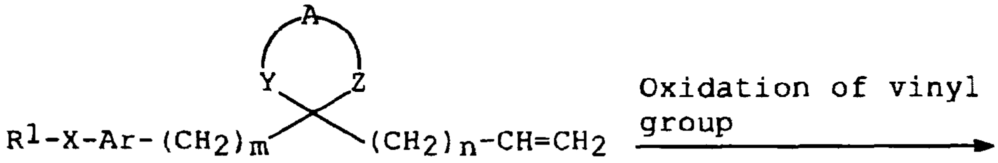


(II)
or a salt thereof

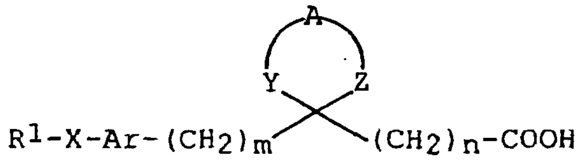

(I-b)
or a salt thereof

Process 3

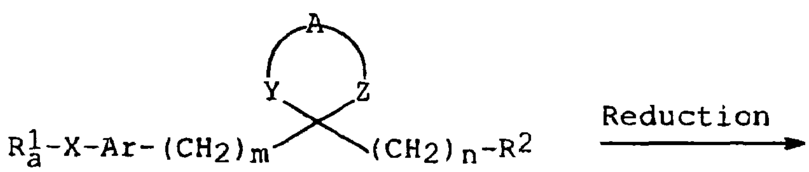


(I-c)
or a salt thereof

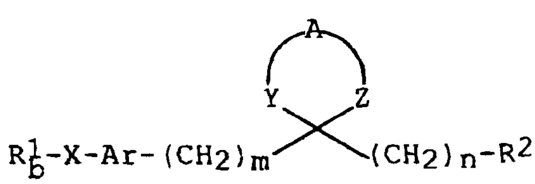

(I-d)
or a salt thereof

Process 4

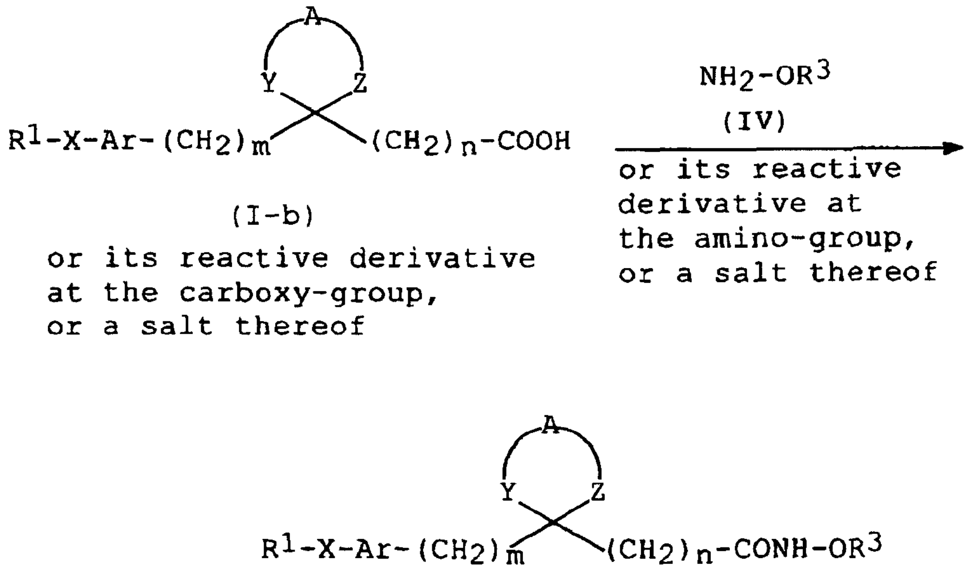


(I-b)
or its reactive derivative at the carboxy-group, or a salt thereof

(I-e)
or a salt thereof

Process 5

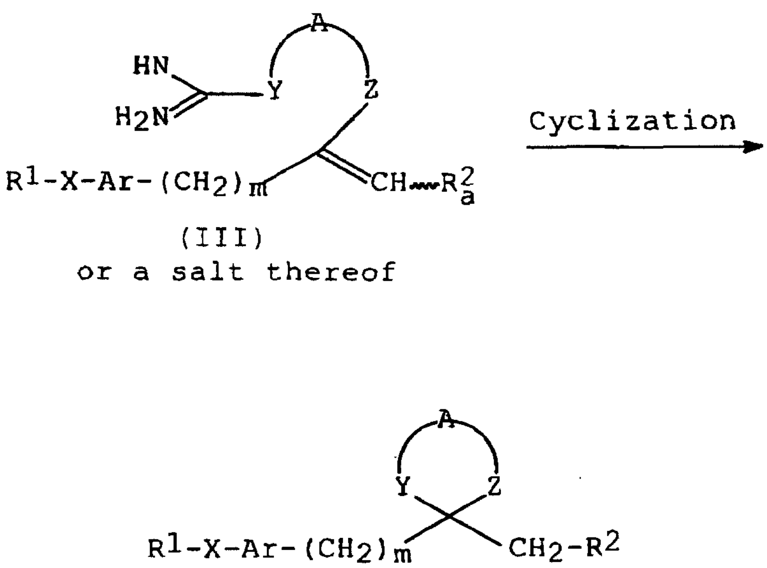


(III)
or a salt thereof

(I-f)
or a salt thereof

Process 6

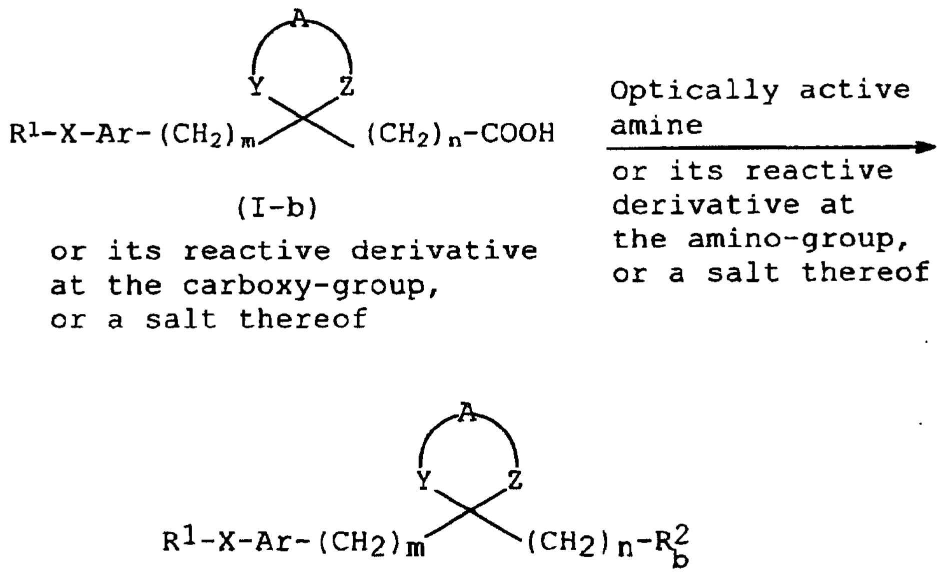


(I-b)

or its reactive derivative at the carboxy-group, or a salt thereof

(I-g)

or a salt thereof

Process 7

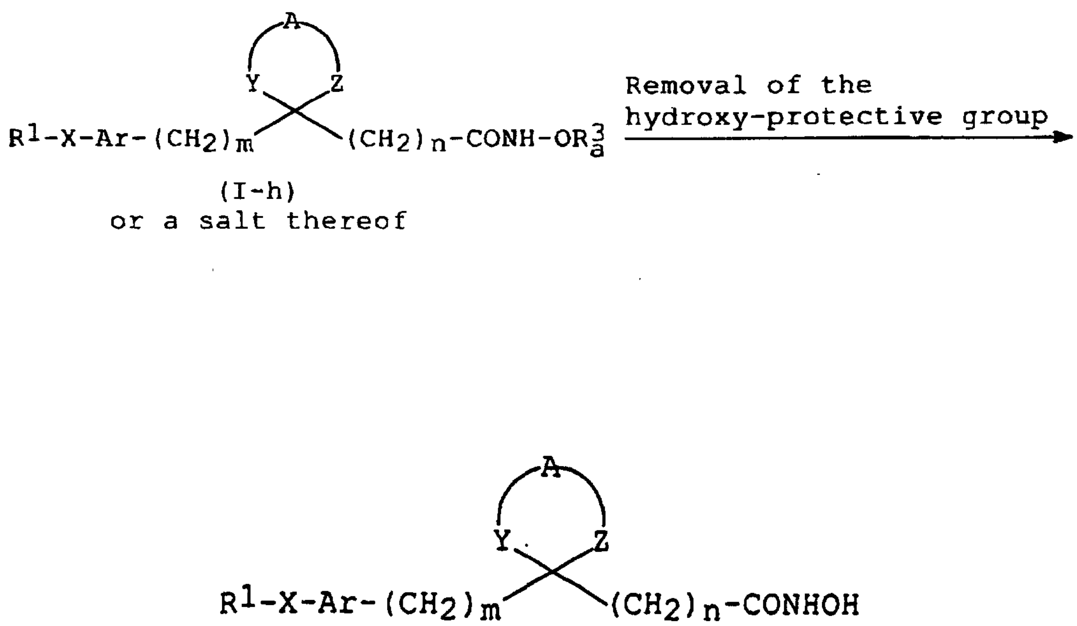


(I-h)

or a salt thereof

(I-i)

or a salt thereof

Process 8

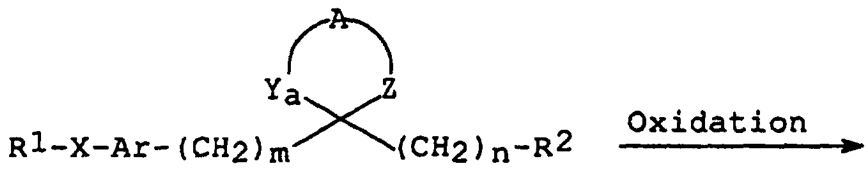


(I-j)
or a salt thereof

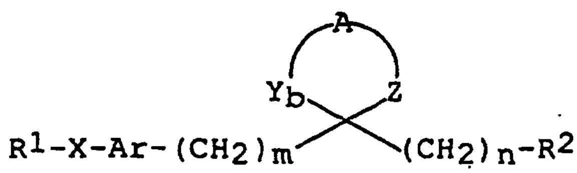

(I-k)
or a salt thereof

Process 9

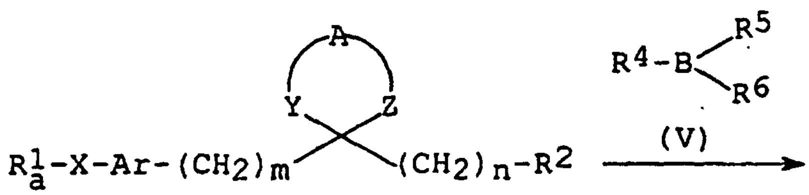


(I-c)
or a salt thereof

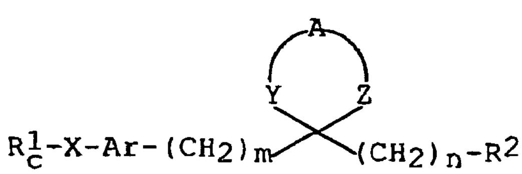

(I-l)
or a salt thereof

Process 10

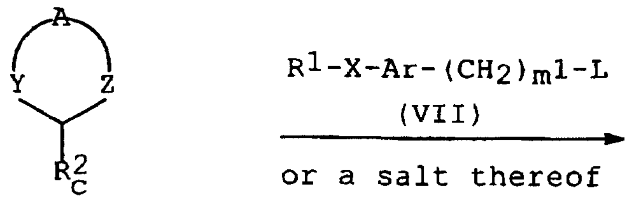


(VI)
or a salt thereof

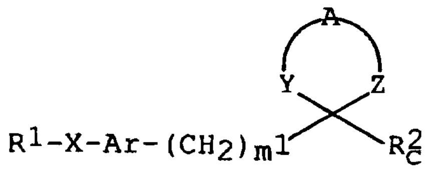

(I-m)
or a salt thereof

Process 11

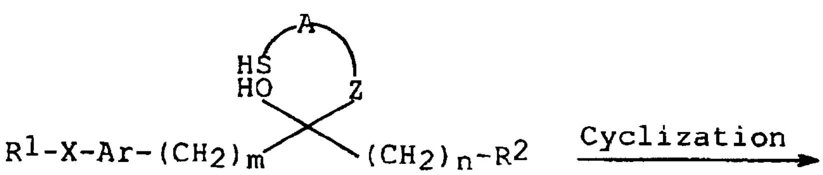


(VIII)
or a salt thereof

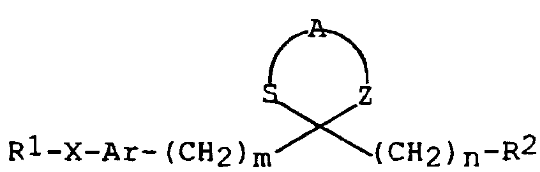

(I-n)
or a salt thereof

Process 12

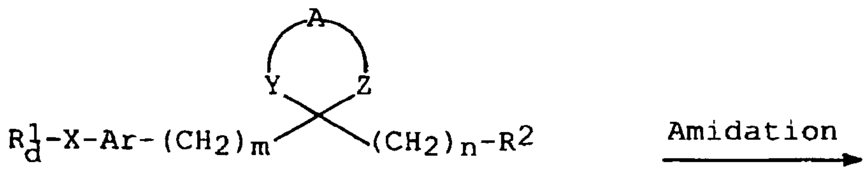


(I-p)

or its reactive derivative at the carboxy group, or a salt thereof

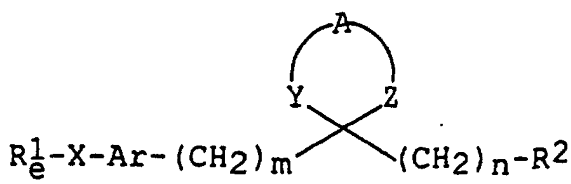

(I-q)

or a salt thereof

Process 13

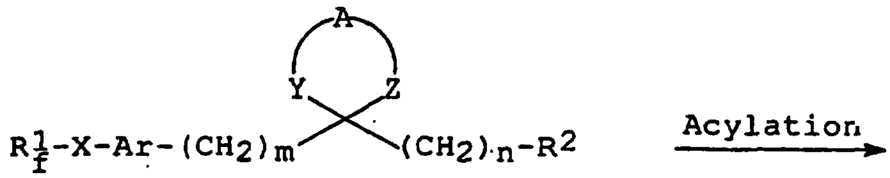


(I-r)
or its reactive derivative
at the amino group,
or a salt thereof

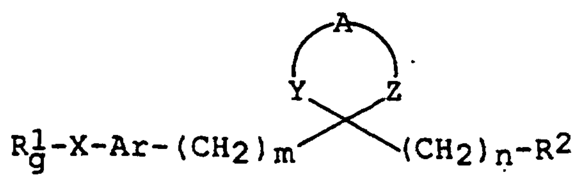

(I-s)
or a salt thereof

Process 14

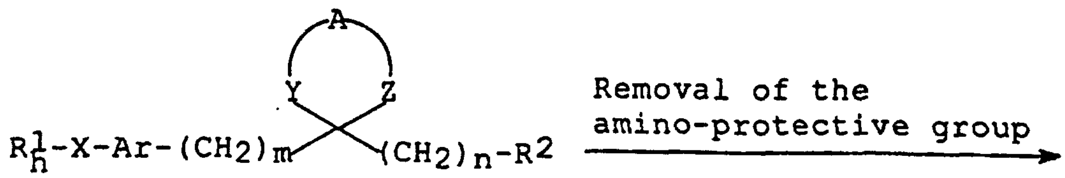


(I-t)
or a salt thereof

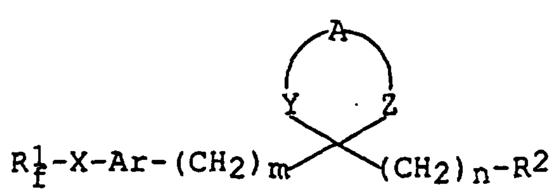

(I-r)
or a salt thereof

Psrocess 15

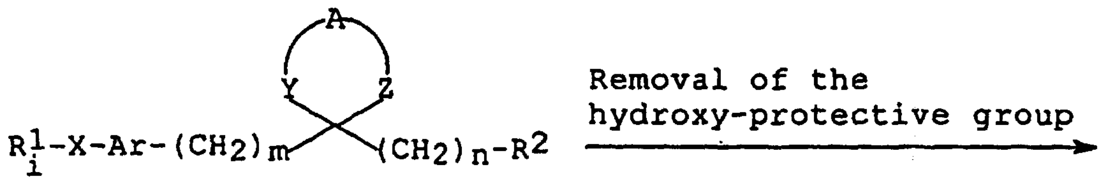


(I-u)
or a salt thereof

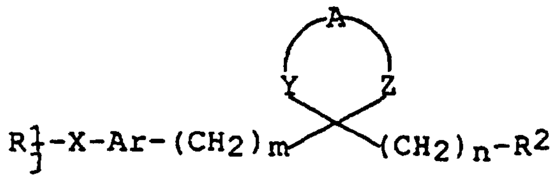

(I-v)
or a salt thereof

Process 16

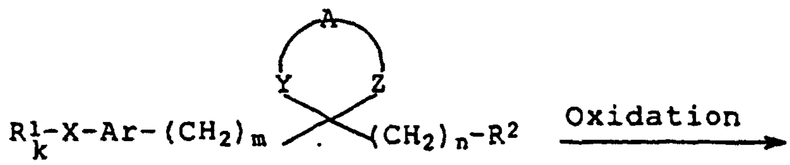


(I-w)
or a salt thereof

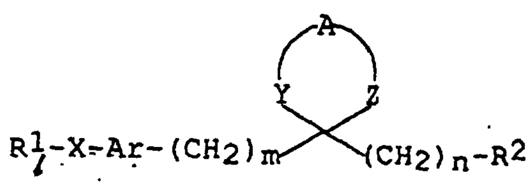

(I-x)
or a salt thereof

Process 17

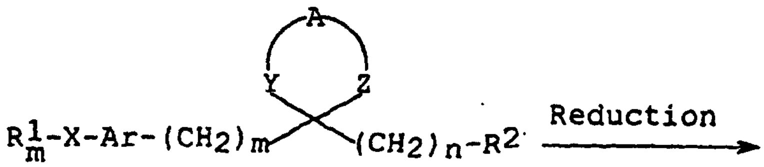


(I-y)
or a salt thereof

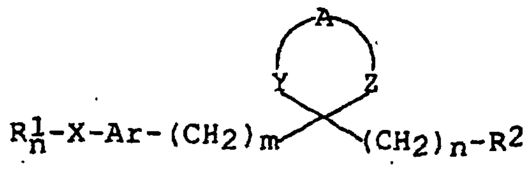

(I-z)
or a salt thereof

Process 18

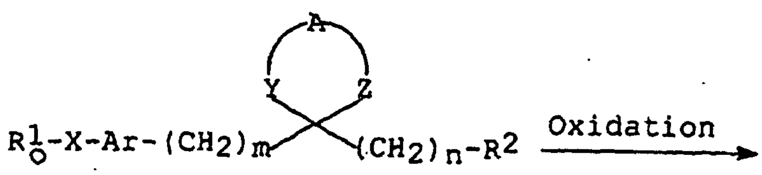


(I-aa)
or a salt thereof

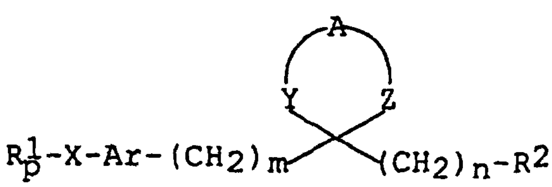

(I-ab)
or a salt thereof

Process 19

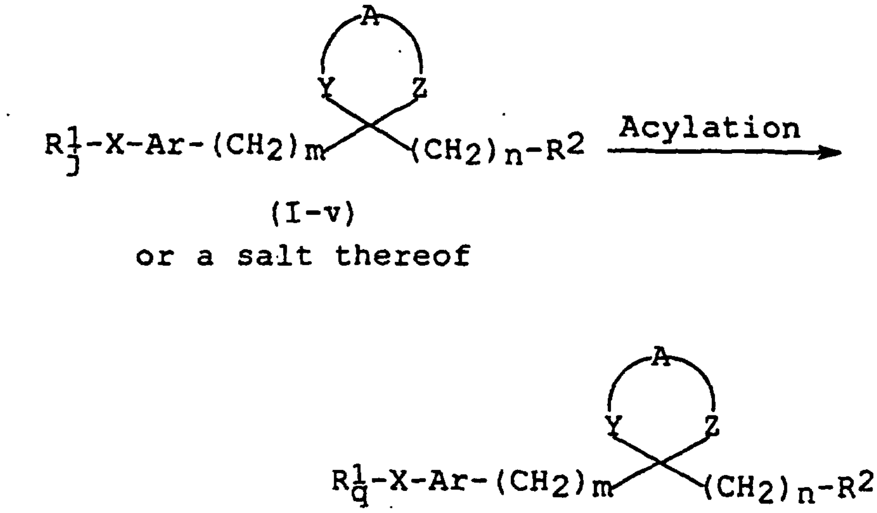


(I-v)
or a salt thereof

(I-ac)
or a salt thereof

Process 20

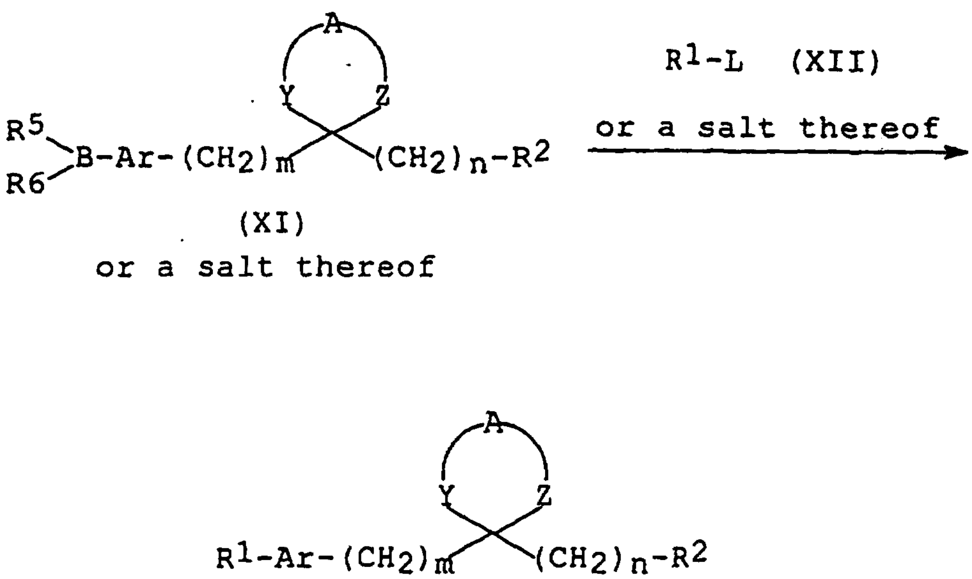


(XI)
or a salt thereof

(I-ad)
or a salt thereof

Process 21

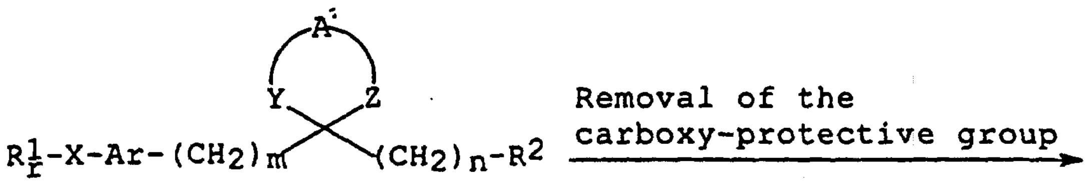


(I-ae)
or a salt thereof

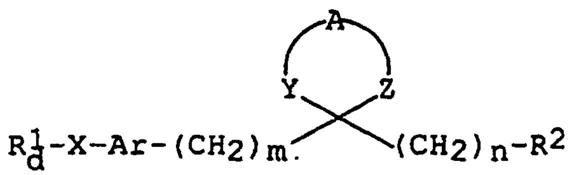

(I-p)
or a salt thereof

Process 22

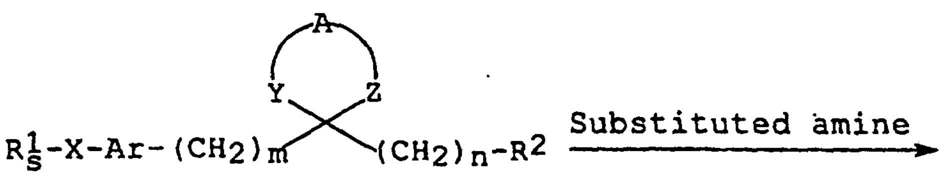


(I-af)
or a salt thereof

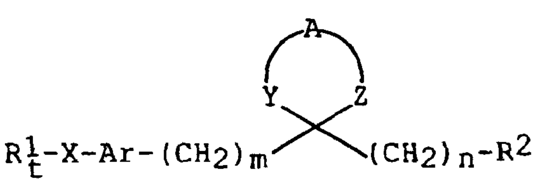

(I-ag)
or a salt thereof

in which

$R^1$, $R^2$, Ar, A, X, Y, Z, m and n are each as defined above,

| | |
|---|---|
| $R^1_a$ | is haloaryl or halo, |
| $R^1_b$ | is aryl, |
| $R^1_c$ | is aryl at least substituted by optionally substituted aryl, |
| $R^1_d$ | is aryl at least having carboxy moiety, |
| $R^1_e$ | is aryl at least having amido moiety, |
| $R^1_f$ | is aryl at least having amino moiety, |
| $R^1_g$ | is aryl at least having acylamino moiety, |
| $R^1_h$ | is aryl at least having protected amino moiety, |
| $R^1_i$ | is aryl at least having protected hydroxy moiety, |
| $R^1_j$ | is aryl at least having hydroxy moiety, |
| $R^1_k$ | is aryl at least having thia moiety, |
| $R^1_l$ | is aryl at least having sulfinyl or sulfonyl moiety, |
| $R^1_m$ | is aryl at least having formyl moiety, |
| $R^1_n$ | is aryl at least having hydroxymethyl moiety, |
| $R^1_o$ | is aryl at least having vinyl moiety, |
| $R^1_p$ | is aryl at least having 1,2-dihydroxyethyl moiety, |
| $R^1_q$ | is aryl at least having acyloxy moiety, |
| $R^1_r$ | is aryl at least having protected carboxy moiety, |
| $R^1_s$ | is aryl at least having halo(lower)alkanoyl moiety, |
| $R^1_t$ | is aryl at least having substituted amino(lower)alkanoyl moiety, |
| $R^2_a$ | is protected carboxy, |
| $R^2_b$ | is optically active amide, |
| $R^2_c$ | is protected carboxy, |
| $R^3$ | is hydrogen or hydroxy-protective group, |
| $R^3_a$ | is hydroxy-protective group, |
| $R^4$ | is optionally substituted aryl, |
| $R^5$ and $R^6$ | are each hydrogen or combined together to form lower alkylene, |
| $Y_a$ | is thia, or sulfinyl, |
| $Y_b$ | is sulfinyl or sulfonyl, |
| L | is a leaving group, and |
| $m^1$ | is an integer of 1 to 6. |

The starting compounds used in the above processes can be prepared according to the following Preparations or by a conventional method.

Suitable salts of the object compounds (I) to (I-ae) may be conventional non-toxic pharmaceutically acceptable salts and include an acid addition salt such as an organic acid salt (e.g., acetate, trifluoroacetate, maleate, tartrate, fumarate, methanesulfonate, benzenesulfonate, formate, toluenesulfonate), an inorganic acid salt (e.g., hydrochloride, hydrobromide, hydroiodide, sulfate, nitrate, phosphate), or a salt with a base such as an amino acid (e.g., arginine, aspartic acid, glutamic acid,), an alkali metal salt (e.g., sodium salt, potassium salt), an alkaline earth metal salt (e.g., calcium salt, magnesium salt), an ammonium salt, an organic base salt (e.g., trimethylamine salt, triethylamine salt, pyridine salt, picoline salt, dicyclohexylamine salt, N,N'-dibenzylethylenediamine salt).

The object compounds and pharmaceutically acceptable salts thereof may include solvates such as enclosure compounds (e.g., hydrate, etc.).

Suitable examples and illustrations of the various definitions, which the present invention includes within its scope and which are shown in the above and subsequent descriptions of the present specification, are as follows.

The term "lower" is intended to mean up to 6 carbon atoms, preferably up to 4 carbon atoms, unless otherwise indicated.

Suitable "aryl" and aryl moiety in the term "optionally substituted aryl" may include an aryl having 6 to 10 carbon atoms, such as phenyl, tolyl, xylyl, cumenyl, mesityl, naphthyl and the like, preferably phenyl and naphthyl.

Suitable "optionally substituted aryl" may include above-mentioned aryl, which is substituted by the group consisting of the following (A1) to (A35);

(A1) halogen,
(A2) lower alkyl,
(A3) lower alkoxy,
(A4) halo(lower)alkyl,
(A5) halo(lower)alkoxy,
(A6) lower alkenyl,

(A7) acyl,
(A8) lower alkylthio, lower alkylsulfinyl, lower alkylsulfonyl,
(A9) $C_6$-$C_{10}$ aryl
(A10) halo($C_6$-$C_{10}$)aryl,
(A11) hydroxy,
(A12) hydroxy(lower)alkyl, protected hydroxy(lower)alkyl,
(A13) amino,
(A14) carboxy,
(A15) protected carboxy,
(A16) nitro (lower) alkenyl,
(A17) lower alkylenedioxy,
(A18) acylamino,
(A19) nitro,
(A20) ($C_6$-$C_{10}$)aryl(lower)alkoxy,
(A21) carbamoyl(lower)alkenyl optionally N-substituted by the group consisting of lower alkyl, $C_6$-$C_{10}$ aryl lower alkoxy-($C_6$-$C_{10}$) aryl, and halo($C_6$-$C_{10}$)aryl,
(A22) lower alkylaminocarbonyloxy,
(A23) lower alkanoyloxy,
(A24) lower alkoxy(lower)alkanoyloxy,
(A25) lower alkoxycarbonyloxy,
(A26) lower alkenoyloxy optionally substituted by heterocyclic group of the following (1) to (14),
(A27) lower cycloalkanecarbonyloxy,
(A28) lower alkoxy substituted by the group consisting of carboxy, protected carboxy, lower alkanoyl, lower cycloalkanecarbamoyl, and lower alkylcarbamoyl,
(A29) lower alkylcarbamoyloxy(lower)alkyl,
(A30) lower alkoxycarbonylamino(lower)alkyl,
(A31) amino(lower)alkyl,
(A32) lower alkylcarbamoyl(lower)alkyl,
(A33) heterocyclic-carbonylamino, the heterocyclic group being selected from the following (1) to (14) and optionally being substituted N-protective group,
(A34) the following heterocyclic groups (1) to (14) being optionally substituted by lower alkyl, and
(A35) oxo.

Suitable "heterocyclic group" in the term "optionally substituted heterocyclic group" means saturated or unsaturated, monocyclic or polycyclic heterocyclic group containing at least one hetero atom such as oxygen atom, sulfur atom, nitrogen atom and the like.
Preferable heterocyclic groups are following (1) to (14):

(1) unsaturated 3- to 8-membered, preferably 5- or 6-membered, heteromonocyclic group containing 1 to 4 nitrogen atoms (pyrrolyl, pyrrolinyl, imidazolyl, pyrazolyl, pyridyl and its N-oxide, pyrimidyl, pyrazinyl, pyridazinyl, triazolyl (e.g., 4H-1,2,4-triazolyl, 1H-1,2,3-triazolyl, 2H-1,2,3-triazolyl, etc.), tetrazolyl (e.g., 1H-tetrazolyl, 2H-tetrazolyl, etc.), dihydrotriazinyl (e.g., 4,5-dihydro-1,2,4-triazinyl, 2,5-dihydro-1,2,4-triazinyl, etc.), and the like);
(2) saturated 3- to 8-membered, preferably 5- or 6-membered, heteromonocyclic group containing 1 to 4 nitrogen atoms (azetidinyl, pyrrolidinyl, imidazolidinyl, piperidinyl, piperidino, pyrazolidinyl, piperazinyl, and the like);
(3) unsaturated 3- to 8-membered, preferably 5- or 6-membered, heteromonocyclic group containing 1 or 2 sulfur atoms (thienyl, and the like);
(4) unsaturated condensed (preferably bicyclic) 7- to 13-membered, preferably 9- or 10-membered, heterocyclic group containing 1 to 5 nitrogen atoms
(indolyl, isoindolyl, indolizinyl, benzimidazolyl, quinolyl, isoquinolyl, indazolyl, benzotriazolyl, tetrazolopyridyl, tetrazolopyridazinyl (e.g., tetrazolo[1,5-b]pyridazinyl, etc.), dihydrotriazolopyridazinyl, and the like);
(5) unsaturated 3- to 8-membered, preferably 5- or 6-membered, heteromonocyclic group containing 1 or 2 oxygen atoms
(furyl, and the like);
(6) saturated 3- to 8-membered, preferably 5- or 6-membered, heteromonocyclic group containing 1 or 2 oxygen atoms
(oxolanyl, and the like);
(7) unsaturated 3- to 8-membered, preferably 5- or 6-membered, heteromonocyclic group containing 1 or 2 oxygen atoms and 1 to 3 nitrogen atoms

(oxazolyl, isoxazolyl, oxadiazolyl (e.g., 1,2,4-oxadiazolyl, 1,3,4-oxadiazolyl, 1,2,5-oxadiazolyl, etc.), and the like);
(8) unsaturated condensed (preferably bicyclic) 7- to 13-membered, preferably 9- or 10-membered, heterocyclic group containing 1 or 2 oxygen atoms (benzofuranyl, benzodihydrofuranyl, benzodioxolenyl, and the like);
(9) unsaturated condensed (preferably bicyclic) 7- to 13-membered, preferably 9- or 10-membered, heterocyclic group containing 1 or 2 sulfur atoms (benzothienyl, dihydrobenzothienyl, and the like);
(10) saturated 3- to 8-membered, preferably 5- or 6-membered, heteromonocyclic group containing 1 or 2 oxygen atoms and 1 to 3 nitrogen atoms (morpholinyl, morpholino, and the like);
(11) unsaturated condensed (preferably bicyclic) 7- to 13-membered, preferably 9- or 10-membered, heterocyclic group containing 1 or 2 oxygen atoms and 1 to 3 nitrogen atoms
(benzoxazolyl, benzoxadiazolyl, and the like);
(12) unsaturated 3- to 8-membered, preferably 5- or 6-membered, heteromonocyclic group containing 1 or 2 sulfur atoms and 1 to 3 nitrogen atoms
(thiazolyl, 1,2-thiazolyl, thiazolinyl, thiadiazolyl (e.g., 1,2,4-thiadiazolyl, 1,3,4-thiadiazolyl, 1,2,5-thiadiazolyl, 1,2,3-thiadiazolyl, etc.), and the like);
(13) saturated 3- to 8-membered, preferably 5- or 6-membered, heteromonocyclic group containing 1 or 2 sulfur atoms and 1 to 3 nitrogen atoms (thiazolidinyl, and the like);
(14) unsaturated condensed (preferably bicyclic) 7- to 13-membered, preferably 9- or 10-membered, heterocyclic group containing 1 or 2 sulfur atoms and 1 to 3 nitrogen atoms
(benzothiazolyl, benzothiadiazolyl, and the like); etc.

These heterocyclic groups may have one or more substituents. Examples of the substituents for substituted heterocyclic group may be the same as those for "optionally substituted aryl" (above-mentioned (A1) to (A35))

Suitable "lower alkyl" may include a straight or branched alkyl having 1 to 6 carbon atoms, and exemplified by methyl, ethyl, propyl, isopropyl, butyl, isobutyl, tert-butyl, pentyl, hexyl and the like, and the most preferably methyl for $R^1$.

Suitable "lower alkenyl" may include a straight or branched alkenyl having 2 to 6 carbon atoms, and exemplified by ethenyl, 1-propenyl, 2-propenyl, 1-butenyl, 2-butenyl, 1-pentenyl, 2-pentenyl and the like.

Suitable "lower alkoxy" may include a straight or branched alkenyl having 1 to 6 carbon atoms, and exemplified by methoxy, ethoxy, propoxy, isopropoxy, butoxy, isobutoxy, tert-butoxy, pentyloxy, tert-pentyloxy, hexyloxy and the like.

Suitable "hydroxy-protective group" may include a conventional protective group, for example, substituted lower alkyl such as lower alkoxy(lower)alkyl (e.g., methoxymethyl), lower alkoxy(lower)alkoxy(lower)alkyl (e.g., methoxyethoxymethyl) and substituted or unsubstituted aryl(lower)alkyl (e.g., benzyl, nitrobenzyl); acyl such as lower alkanoyl (e.g., acetyl, propionyl, pivaloyl), aroyl (e.g., benzoyl, fluorenecarbonyl), lower alkoxycarbonyl (e.g., methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, hexyloxycarbonyl), substituted or unsubstituted aryl(lower)alkoxycarbonyl (e.g., benzyloxycarbonyl, bromobenzyloxycarbonyl), arenesulfonyl (e.g., benzenesulfonyl, tosyl) and alkanesulfonyl (e.g., methanesulfonyl, ethanesulfonyl); tri(lower)alkylsilyl (e.g., trimethylsilyl); tetrahydropyranyl; and the like, preferably tetrahydropyranyl.

Suitable "halogen" includes fluorine, bromine, chlorine and iodine.

Suitable acyl and acyl moiety of "acylamino" includes acyl such as aliphatic acyl, aromatic acyl, heterocyclic acyl and aliphatic acyl substituted by aromatic or heterocyclic group(s) derived from carboxylic, carbonic, sulfonic and carbamic acids.

The aliphatic acyl includes saturated or unsaturated, acyclic or cyclic ones, for example, alkanoyl such as lower alkanoyl (e.g., formyl, acetyl, propionyl, butylyl, isobutylyl, valeryl, isovaleryl, pivaloyl, hexanoyl, etc.), alkylsulfonyl such as lower alkylsulfonyl (e.g., mesyl, ethylsulfonyl, propylsulfonyl, isopropylsulfonyl, butylsulfonyl, isobutylsulfonyl, pentylsulfonyl, hexylsulfonyl, etc.), carbamoyl, N-alkylcarbamoyl (e.g., methylcarbamoyl, ethylcarbamoyl, etc.), alkoxycarbonyl such as lower alkoxycarbonyl (e.g., methoxycarbonyl, ethoxycarbonyl, propoxycarbonyl, butoxycarbonyl, tert-butoxycarbonyl, etc.), alkenyloxycarbonyl such as lower alkenyloxycarbonyl (e.g., vinyloxycarbonyl, allyloxycarbonyl etc.), alkenoyl such as lower alkenoyl (e.g., acryloyl, methacryloyl, crotonoyl, etc.), cycloalkanecarbonyl such as cyclo(lower)alkanecarbonyl (e.g., cyclopropanecarbonyl, cyclopentanecarbonyl, cyclohexanecarbonyl, etc.), and the like.

The aromatic acyl may include $C_6$-$C_{10}$ aroyl (e.g., benzoyl, toluoyl, xyloyl, etc.), N-($C_6$-$C_{10}$)arylcarbamoyl (e.g., N-phenylcarbamoyl, N-tolylcarbamoyl, N-naphthylcarbamoyl, etc.), $C_6$-$C_{10}$ arenesulfonyl (e.g., benzenesulfonyl, tosyl, etc.), and the like.

The heterocyclic acyl may include heterocyclic-carbonyl (e.g., furoyl, thenoyl, nicotinoyl, isonicotinoyl, thiazolylcarbonyl, thiadiazolylcarbonyl, tetrazolylcarbonyl, etc.), and the like.

The aliphatic acyl substituted by aromatic group(s) may include aralkanoyl such as phenyl(lower)alkanoyl (e.g., phenylacetyl, phenylpropionyl, phenylhexanoyl, etc.), aralkoxycarbonyl such as phenyl(lower)alkoxycarbonyl (e.g.,

benzyloxycarbonyl, phenethyloxycarbonyl, etc.), aryloxyalkanoyl such as phenoxy(lower)alkanoyl (e.g., phenoxyacetyl, phenoxypropionyl, etc.), and the like.

The aliphatic acyl substituted by heterocyclic group(s) may include heterocyclic-alkanoyl such as heterocyclic-(lower)alkanoyl (e.g., thienylacetyl, imidazolylacetyl, furylacetyl, tetrazolylacetyl, thiazolylacetyl, thiadiazolylacetyl, thienylpropionyl, thiadiazolylpropionyl, etc.), and the like.

These acyl groups may be further substituted by one or more suitable substituents as those for "optionally substituted aryl" (above-mentioned (A1) to (A35)).

Suitable "protected carboxy" includes esterified carboxy wherein "esterified carboxy" is as defined below.

Suitable examples of the ester moiety of the esterified carboxy are lower alkyl ester (e.g., methyl ester, ethyl ester, propyl ester, isopropyl ester, butyl ester, isobutyl ester, tert-butyl ester, pentyl ester, hexyl ester, etc.) and the like, which may have at least one suitable substituent. Examples of the substituted lower alkyl ester are lower alkanoyloxy(lower)alkyl ester [e.g., acetoxymethyl ester, propionyloxymethyl ester, butyryloxymethyl ester, valeryloxymethyl ester, pivaloyloxymethyl ester, hexanoyloxymethyl ester, 1-(or 2-)acetoxyethyl ester, 1-(or 2- or 3-)acetoxypropyl ester, 1-(or 2- or 3- or 4-)-acetoxybutyl ester, 1-(or 2-)propionyloxyethyl ester, 1-(or 2- or 3-)propionyloxypropyl ester, 1-(or 2-)-butyryloxyethyl ester, 1-(or 2-)isobutyryloxyethyl ester, 1-(or 2-)pivaloyloxyethyl ester, 1-(or 2-)hexanoyloxyethyl ester, isobutyryloxymethyl ester, 2-ethylbutyryloxymethyl ester, 3,3-dimethylbutyryloxymethyl ester, 1-(or 2-)-pentanoyloxyethyl ester, etc.], lower alkanesulfonyl(lower)-alkyl ester (e.g., 2-mesylethyl ester, etc.), mono(or di or tri)halo(lower) alkyl ester (e.g., 2-iodoethyl ester, 2,2,2-trichloroethyl ester, etc.); lower alkoxycarbonyloxy(lower)alkyl ester [e.g., methoxycarbonyloxymethyl ester, ethoxycarbonyloxymethyl ester, propoxycarbonyloxymethyl ester, tert-butoxycarbonyloxymethyl ester, 1-(or 2-)methoxycarbonyloxyethyl ester, 1-(or 2-)ethoxycarbonyloxyethyl ester, 1-(or 2-)isopropoxycarbonyloxyethyl ester, etc.] phthalidylidene(lower)alkyl ester, (5-lower alkyl-2-oxo-1,3-dioxol-4-yl)(lower)alkyl ester [e.g., (5-methyl-2-oxo-1,3-dioxol-4-yl)methyl ester, (5-ethyl-2-oxo-1,3-dioxol-4-yl)methyl ester, (5-propyl-2-oxo-1,3-dioxol-4-yl)ethyl ester, etc.]; lower alkenyl ester (e.g., vinyl ester, allyl ester, etc.); lower alkynyl ester (e.g., ethynyl ester, propynyl ester, etc.); ar(lower)alkyl ester which may have at least one suitable substituent (e.g., benzyl ester, 4-methoxybenzyl ester, 4-nitrobenzyl ester, phenethyl ester, trityl ester, benzhydryl ester, bis(methoxyphenyl)methyl ester, 3,4-dimethoxybenzyl ester, 4-hydroxy-3,5-di-tert-butylbenzyl ester, etc.); aryl ester which may have at least one suitable substituent (e.g., phenyl ester, 4-chlorophenyl ester, tolyl ester, tert-butylphenyl ester, xylyl ester, mesityl ester, cumenyl ester, etc.); phthalidyl ester; and the like.

More preferable example of the protected carboxy 'thus defined may be $C_1$-$C_4$ alkoxycarbonyl, and the most preferable one may be methoxycarbonyl, ethoxycarbonyl, and t-butoxycarbonyl for $R^2$.

Suitable "leaving group" may include halogen as mentioned above, acyloxy such as sulfonyloxy (e.g., mesyloxy, tosyloxy, etc.), alkoxy (e.g., tert-butoxy, etc.), aralkoxy (e.g., benzyloxy, etc.), and the like, preferably halogen and the most preferably bromine.

Suitable "lower alkylene" may include straight or branched one such as methylene, ethylene, trimethylene, propylene, tetramethylene, ethylethylene, pentamethylene, hexamethylene, and the like, in which more preferable one may be $C_1$-$C_4$ alkylene, and the most preferable one may be ethylene, 1-methyltrimethylene, and trimethylene.

Suitable "halo(lower)alkyl" may be above-mentioned lower alkyl substituted by halogen as mentioned above, in which more preferable one may be halo($C_1$-$C_4$)alkyl.

Suitable "halo(lower)alkoxy" may be above-mentioned lower alkoxy substituted by halogen as mentioned above, in which more preferable one may be halo($C_1$-$C_4$)alkoxy.

Suitable "lower alkylthio" may be thio group substituted by above-mentioned lower alkyl, in which more preferable one may be $C_1$-$C_4$ alkylthio (e.g. methylthio, ethylthio, etc.).

Suitable "lower alkylsulfinyl" may include methylsulfinyl, ethylsulfinyl, and the like, in which more preferable one may be $C_1$-$C_4$ alkylsulfinyl (e.g. methylsulfinyl, etc.).

Suitable "lower alkylsulfonyl" may include methylsulfonyl, ethylsulfonyl, and the like, in which more preferable one may be $C_1$-$C_4$ alkylsulfonyl (e.g. methylsulfonyl, etc.).

Suitable "haloaryl may be aforementioned aryl substituted by halogen as mentioned above,.in which more preferable one may be halo($C_6$-$C_{10}$)aryl, and the most preferable one may be 4-fluorophenyl.

Suitable "hydroxy(lower)alkyl" may be above-mentioned lower alkyl substituted by hydroxy as mentioned above, in which more preferable one may be hydroxy($C_1$-$C_4$)alkyl.

Suitable "protected hydroxy(lower)alkyl" may be above-mentioned hydroxy(lower)alkyl group protected by a conventional hydroxy-protective group such as acyl, (lower alkyl)(diaryl)silyl group (e.g. (t-butyl)(diphenyl)silyl, etc.), and the like.

Suitable "aryl(lower)alkoxy" may include behzyloxy, phenylethoxy, and the like, in which more preferable one may be phenyl($C_1$-$C_4$)alkoxy (e.g. benzyloxy, etc.).

Suitable "carbamoyl(lower)alkenyl" may include carbamoylethenyl, carbamoylallyl, and the like, in which more preferable one may be carbamoyl($C_2$-$C_4$)alkenyl (e.g. carbamoylethenyl, etc.).

Suitable "lower alkoxyaryl" may include methoxyphenyl, ethoxyphenyl, and the like, in which more preferable

one may be $C_1$-$C_4$ alkoxyphenyl (e.g. methoxyphenyl, etc.).

Preferable examples of "carbamoyl(lower)alkenyl optionally N-substituted by the group consisting of lower alkyl, aryl, lower alkoxyaryl and haloaryl" may include lower alkylcarbamoyl(lower)alkenyl (e.g. carbamoylethenyl, methylcarbamoylethenyl, ethylcarbamoylethenyl, propylcarbamoylethenyl, isopropycarbamoylethenyl, dimethylcarbamoylethenyl, etc.), $C_6$-$C_{10}$ arylcarbamoyl(lower)alkenyl (e.g. phenylcarbamoylethenyl, etc.), lower alkoxy($C_6$-$C_{10}$) arylcarbamoyl(lower)alkenyl (e.g. methoxyphenylcarbamoylethenyl, etc.), haloarylcarbamoyl(lower)alkenyl (e.g. fluorophenylcarbamoylethenyl, etc.), and the like.

Suitable "lower alkylaminocarbonyloxy" may include methylaminocarbonyloxy, ethylaminocarbonyloxy, and the like, in which more preferable one may be $C_1$-$C_4$ alkylaminocarbonyloxy (e.g. methylaminocarbonyloxy, ethylaminocarbonyloxy, etc.).

Suitable "lower alkanoyloxy" may include acetyoxy, propanoyloxy, and the like, in which more preferable one may be $C_1$-$C_4$ alkanoyloxy (e.g. propanoyloxy, etc.).

Suitable "lower alkoxy(lower)alkanoyloxy" may include methoxyacetyloxy, ethoxyacetyloxy, and the like,:in which more preferable one may be $C_1$-$C_4$ alkoxy($C_1$-$C_4$)alkanoyloxy (e.g. methoxyacetyloxy, etc.).

Suitable "lower alkoxycarbonyloxy" may include methoxycarbonyloxy, ethoxycarbonyloxy, and the like, in which more preferable one may be $C_1$-$C_4$ alkoxycarbonyloxy (e.g. ethoxycarbonyloxy, etc.).

Suitable "lower alkenoyloxy" may include acryloyloxy, and the like, in which more preferable one may be $C_2$-$C_4$ alkenoyloxy (e.g. acryloyloxy, etc.).

Preferable examples of "lower alkenoyloxy optionally substituted by heterecyclic group" may include lower alkenoyloxy optionally substituted by the above-mentioned heterocyclic group (1) (e.g. pyridyl, etc.) such as pyridylacryloyloxy, and the like.

Suitable "lower cycloalkanecarbonyloxy" may include $C_3$-$C_7$ cycloalkanecarbonyloxy such as cyclopropanecarbonyloxy, cyclobutanecarbonyloxy, and the like, in which more preferable one may be $C_3$-$C_6$ cycloalkanecarbonyloxy (e.g. cyclopropanecarbonyloxy, etc.).

Suitable "lower cycloalkanecarbamoyl" may include $C_3$-$C_7$ cycloalkanecarbamoyl such as cyclopropanecarbamoyl, cyclobutanecarbamoyl, and the like, in which more preferable one may be $C_4$-$C_6$ cycloalkanecarbamoyl (e.g. cyclopropanecarbamoyl, etc.).

Suitable "lower alkylcarbamoyl" may include methylcarbamoyl, ethylcarbamoyl, propylcarbamoyl and the like, in which more preferable one may be $C_1$-$C_4$ alkylcarbamoyl (e.g. methylcarbamoyl, ethylcarbamoyl, propylcarbamoyl, etc.).

Preferable examples of "lower alkoxy substituted by the group consisting of carboxy, protected carboxy, lower alkanoyl, lower cycloalkanecarbamoyl, lower alkylcarbamoyl may include arboxy(lower)alokoxy (e.g. carboxymethoxy, etc.), lower alkoxycarbonyl(lower)alkoxy (e.g. ethoxycarbonylmethoxy, butoxycarbonylmethoxy, etc.), lower alkanoyl (lower)alkoxy (e.g. propanoylmethoxy, etc.), lower cycloalkanecarbamoyl(lower)alkoxy (e.g. cyclopropylcarbamoylmethoxy, etc.), loweralkylcarbamoyl(lower)alkoxy (e.g. methylcarbamoylmethoxy, ethylcarbamoylmethoxy, propylcarbamoylmethoxy, etc.), and the like.

Suitable "lower alkylcarbamoyloxy(lower)alkyl" may include methylcarbamoyloxymethyl, ethylcarbamoyloxymethyl, and the like, in which more preferable one may be $C_1$-$C_4$ alkylcarbamoyloxy($C_1$-$C_4$)alkyl (e.g. methylcarbamoyloxymethyl, etc.).

Suitable "lower alkoxycarbonylamino(lower)alkyl" may include methoxycarbonylaminomethyl, t-butoxycarbonylaminomethyl, and the like, in which more preferable one may be $C_1$-$C_4$ alkoxycarbonylamino($C_1$-$C_4$)alkyl (e.g. methoxycarbonylaminomethyl, t-butoxycarbonylaminomethyl, etc.).

Suitable "amino(lower)alkyl" may include aminomethyl, aminoethyl, and the like, in which more preferable one may be amino($C_1$-$C_4$)alkyl (e.g aminomethyl, etc.).

Suitable "lower alkylcarbamoyl(lower)alkyl" may include methylcarbamoylmethyl, methylcarbamoylethyl, ethylcarbamoylmethyl, ethylcarbamoylethyl, and the like, in which more preferable one may be $C_1$-$C_4$ alkylcarbamoyl-($C_1$-$C_4$)alkyl (e.g. methylcarbamoylmethyl, etc.).

Suitable "heterocyclic-carbonylamino" may include carbonylamino group substituted by the above-mentioned heterocyclic group (2),(4) and (5) (e.g. pyrrolidinyl, tetrahydroisoquinolyl, furyl, etc.) such as pyrrolidinylcarbonylamino, 1,2,3,4-tetrahydroisoquinolylcarbonylamino, furylcarbonylamino, and the like.

Preferable examples of "optionally substituted heterocyclic-carbonylamino" may include above-mentioned heterocyclic-carbonylamino optionally the heterocyclic group being substituted by N-protective group (e.g. acyl such as alkoxycarbonyl, etc.) such as pyrrolidinylcarbonylamino, 1,2,3,4-tetrahydroisoquinolylcarbonylamino, (N-(t-butoxycarbonyl)-1,2,3,4-tetrahydroisoquinolyl)-carbonylamino, furylcarbonylamino, and the like.

Suitable "nitro(lower)alkenyl" may be above-mentioned lower alkenyl substituted by.nitro, in which more preferable one may be nitro($C_2$-$C_4$)alkenyl.

Suitable "lower alkylenedioxy" may include straight or branched one such as methylenedioxy, ethylenedioxy, trimethylenedioxy, propylenedioxy, tetramethylenedioxy, ethylethylenedioxy, pentamethylenedioxy, hexamethylenedioxy, and the like, in which more preferable one may be $C_1$-$C_4$ alkylenedioxy.

Suitable "amido" may be the same as those for "amidated carboxy", and preferably lower alkylcarbamoyl, and the most preferably methylcarbamoyl.

Suitable "acylamino" may be amino substituted by acyl as mentioned above.

Suitable "substituted amine" may include amino group substituted by the above-mentioned substituent (A2) to (A12), (A14) to (A17), (A20) to (A32) and (A34).

Preferable examples may be hydroxy (lower)alkylamine (e.g. hydroxyethylamine, etc.), above-mentioned N-containing heterocyclic group (e.g. morpholino, etc.), lower alkenylamine (e.g. allylamine, etc.), $C_6$-$C_{10}$ aryl(lower) alkylamine e.g. benzylamine, etc.), lower alkylamine (e.g. t-butylamine, pentylamine, etc.), heterocyclic(lower) alkylamine such as pyridyl(lower)alkylamine (e.g. pyridylmethylamine, etc.), lower alkoxy(lower)alkylamine (e.g. ethoxyethylamine, etc.), and the like.

Preferable examples of the formula:

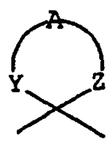

is one of the following formulae:

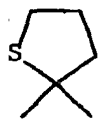 , 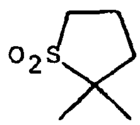 ,

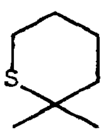 , 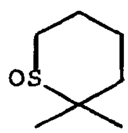 , 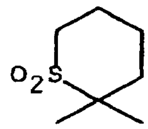 , 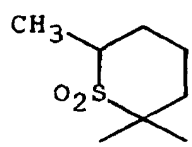 ,

and the like.

In the object compounds (I),

(i) the preferred one may be the compounds (I) wherein m and n are each 0 or 1, and
(ii) the more preferred one may be the compounds of the above item (i) wherein A is ethylene, 1-methyltrimethylene, or trimethylene.

The more preferred object compounds (I) are:

(iii) The compounds (I), having the following formula:

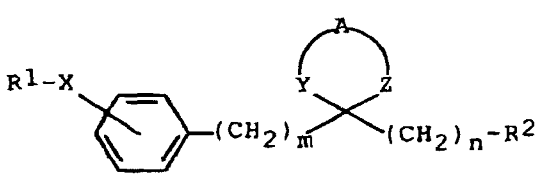 (IA)

wherein a group of the formula:

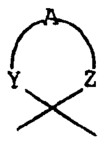

is one of the following formulae:

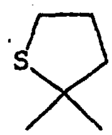 , 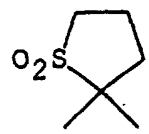 ,

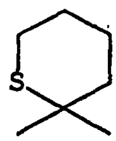 , 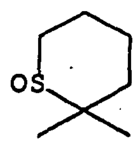 , 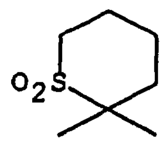 , or 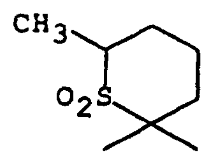 ,

$R^1$ is lower alkyl, phenyl, halophenyl, or (halo)(phenyl)phenyl,
$R^2$ is carboxy or hydroxyaminocarbonyl, and
m and n are each an integer of 0 or 1, and m+n=1.

(iV) The compounds (I), having the following formula:

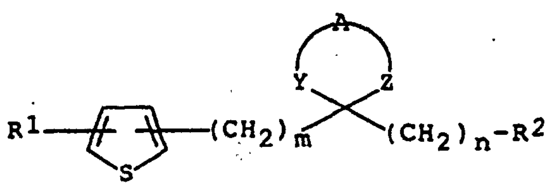 (IB)

wherein a group of the formula:

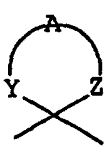

is one of the following formulae:

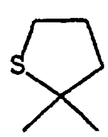 , 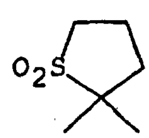 ,

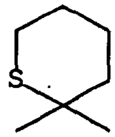 , 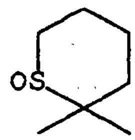 , 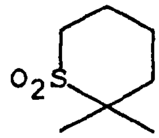 , or 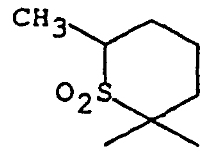 ,

$R^2$ is carboxy or hydroxyaminocarbonyl,

m and n are each an integer of 0 or 1, and m+n=1,

$R^1$ is halogen; heterocyclic group consisting of pyridyl, thienyl, furyl, benzofuranyl or benzothienyl, wherein the heterocyclic group is optionally substituted by the group consisting of lower alkanoyl, lower alkyl, lower alkoxy, lower alkoxycarbonylamino and lower alkylcarbamoyl; naphtyl or phenyl optionally substituted by the group consisting of the following (C1) to (C31);

(C1) halogen,
(C2) lower alkyl,
(C3) lower alkoxy,
(C4) halo(lower)alkyl,
(C5) halo (lower)alkoxy,
(C6) lower alkenyl,
(C7) lower alkylcarbamoyl, carbamoyl, phenyl(lower)alkylcarbamoyl, lower alkanoyl, (C8) lower alkylthio, lower alkylsulfinyl, lower alkylsulfonyl,
(C9) phenyl, naphthyl,
(C10) halophenyl,
(C11) hydroxy,
(C12) mono- or dihydroxy(lower)alkyl, phenoxycarbonyloxy(lower)alkyl
(C13) amino,
(C14) carboxy,
(C15) lower alkylenedioxy,
(C16) lower alkanoylamino, phenyl(lower)alkanoylamino, halophenyl(lower)alkanoylamino, lower alkoxy (lower) alkanoylamino, lower alkoxy(lower)alkanoylamino, phenoxy(lower)alkanoylamino, lower alkoxyphenoxy(lower)alkanoylamino, lower alkylphenoxy(lower)alkanoylamino, halophenoxy(lower)alkanoylamino, carboxy(lower)alkanoylamino, lower alkoxycarbonyl(lower)alkanoylamino, lower alkylcarbamoyl(lower)alkanoylamino, halo(lower)alkanoylamino, lower alkenyl(lower)alkanoylamino, lower alkoxy(lower)alkanoylamino, phenyl(lower)alkoxy(lower)alkanoylamino, piperidinyloxy(lower)alkanoylamino, N-lower alkoxycarbonylpiperidinyloxy(lower)alkanoylamino, pyridyloxy(lower)-alkanoylamino, hydroxy(lower)alkanoylamino, lower alkandyloxy (lower)alkanoylamino, lower alkylcarbamoyloxy(lower)alkanoylamino, N,N-di(lower alkyl)carbamoyloxy, piperidino-carbonyloxy(lower)alkanoylamino, phenyl(lower)alkylcarbamoyloxy(lower)-alkanoylamino, lower alkoxycarbonylamino(lower)alkanoylamino, amino(lower)alkanoylamino, lower alkoxycarbonylamino(lower)alkanoylamino, fluorenylmethoxycarbonylamino(lower)alkanoylamino, lower alkylamino(lower)alkanoylamino, [N,N-di(lower alkyl)amino](lower)alkanoylamino, [N-lower alkyl-N-(lower alkoxycarbonyl)amino](lower)alkanoylamino, [N-lower alkyl-N-(fluorenylmethoxycarbonyl)amino](lower)alkanoylamino, [N-lower alkyl-N-(mono- or di (lower)-alkylcarbamoyl)amino](lower)alkanoylamino, [N-(mono- or di(lower alkyl)carbamoyl)-amino](lower)alkanoylamino, benzoylamino(lower)alkanoylamino, lower alkanoylamino(lower)alkanoylamino, lower alkanesulfonylamino(lower)alkanoylamino, lower alkoxy(lower)alkanoylamino (lower)alkanoylamino, cyclo(lower)alkyloxycarbonylamino(lower)alkanoylamino, pyridylcarbonylamino(lower)alkanoylamino, morpholinocarbonylamino(lower)alklanoylamino, phenyl(lower) alkoxycarbonylamino(lower)alkanoylamino, lower alkoxyphenylsulfonylamino(lower)alkanoylamino, hydroxy(lower)alkylamino(lower)alkanoylamino, morpholino(lower)alkanoylamino, oxooxazolidinyl(lower)alkanoylamino, oxopyrrolidinyl(lower)alkanoylamino, trimethylhydantoinyl(lower)alkanoylamino, lower alkenylamino(lower)alkanoylamino, lower alkoxy(lower)alkylamino(lower)-alkanoylamino, phenyl(lower)alkylamino(lower)alkanoylamino, pyridyl(lower)alkylamino(lower)alkanoylamino, lower alkoxycarbonylamino, phenyl(lower)alkoxycarbonylamino, lower alkoxy(lower)alkoxycarbonylamino, halo(lower)alkoxycarbonylamino, amino(lower)alkoxycarbonylamino, phthalimido(lower)alkoxycarbonylamino, carbamoylamino, (mono- or di(lower alkyl)carbamoylamino, naphthylcarbamoylamino, halophenylcarbamoylamino, lower alkoxyphenylcarbamoylamino, lower alkenylcarbamoylamino, cyclo(lower)alkyl(lower)alkylcarbamoylamino, phenyl(lower)alkylcarbamoylamino, halo(lower)alkylcarbamoylamino, lower alkoxy(lower)alkylcarbamoylamino, hydroxy(lower)alkylcarbamoylamino, (lower alkyl)(diphenyl)silyloxy(lower)alkylcarbamoylamino, carboxy(lower)alkylcarbamoylamino, lower alkoxycarbonyl(lower)alkylcarbamoylamino, lower alkylcarbamoyl(lower)alkylcarbamoylamino, or pyridylcarbamoylamino, lower alkylsulfonylamino, lower alkenoylamino, lower cycloalkanecarbonylamino, lower alkenyloxycarbonylamino, phenoxycarbonylamino, lower alkylthiocarbonylamino,
(C17) phenyl(lower)alkoxy,
(C18) lower alkenyl, mono- or di(lower alkyl)carbamoyl(lower)alkenyl, (2-(methylcarbamoyl)ethenyl, 2-(ethylcarbamoyl)ethenyl, 2-(propylcarbamoyl)ethenyl, 2-(isopropylcarbamoyl)ethenyl, 2-(dimethylcarbamoyl)ethenyl,) phenylcarbamoyl(lower)alkenyl, lower alkoxycarbamoyl(lower) alkenyl, halophenylcarbamoyl(lower)alkenyl,
(C19) lower alkylaminocarbonyloxy,
(C20) lower alkanoyloxy,
(C21) lower alkoxy(lower)alkanoyloxy,
(C22) lower alkoxycarbonyloxy,
(C23) pyridyl(lower)alkenoyloxy
(C24) lower cycloalkanecarbonyloxy,
(C25) carboxy(lower)alkoxy, lower alkoxycarbonyl(lower)alkoxy; lower alkanoyl(lower)alkoxy, lower cycloalkanecarbamoyl(lower)alkoxy, lower alkylcarbamoyl(lower)alkoxy,
(C26) lower alkylcarbamoyloxy(lower)alkyl,
(C27) lower alkoxycarbonylamino(lower)alkyl,
(C28) amino(lower)alkyl,
(C29) lower alkylcarbamoyl(lower)alkyl,
(C30) furylcarbonylamino, tetrahydroisoquinolylcarbonylamino, N-lower alkoxycarbonyltetrahydroisoquinolylcarbonylamino, pyrrolidinylcarbonylamino,
(C31) oxazolyl, lower alkyloxadiazolyl.

(V) The compounds (I) of the above (iv), in which a group of the formula:

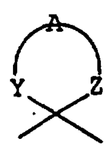

is one of the following formulae:

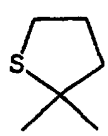 , 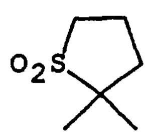 ,

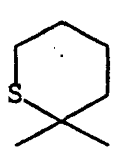 , 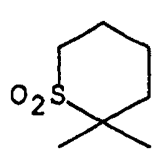 ,

$R^2$ is hydroxyaminocarbonyl,
m is 0 and n is 1,

a group of the formula:

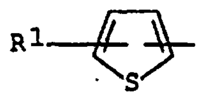

is one of the group of the following formulae (a) to (e);

(a)

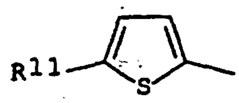

wherein

$R^{11}$ is halo (e.g. bromo, etc.), naphtyl (e.g. 2-naphthyl, etc.), phenyl (e.g. phenyl, etc.), mono- or dihalophenyl (e.g. 3(or 4)-chlorophenyl, 2(or 3 or 4)-fluorophenyl, 3,4-dichlorophenyl, 3,5-difluorophenyl, etc.), mono- or di(lower)alkylphenyl (e.g. 3(or 4)-methylphenyl, 4-ethylphenyl, 4-isopropylphenyl, 4-(t-butyl) phenyl, 3,4-dimethylphenyl, etc.), lower alkoxyphenyl (e.g. 4-methoxyphenyl, 4-ethoxyphenyl, etc.), trihalo(lower)alkylphenyl (e.g. 4-trifluoromethylphenyl, etc.), trihalo(lower)alkoxyphenyl (e.g. 4-trifluoromethoxyphenyl, etc.), lower alkenylphenyl (e.g. 4-ethenylphenyl, etc.), lower alkylcarbamoylphenyl (e.g. 4-methylcarbamoylphenyl, 4-ethylcarbamoylphenyl, etc.), carbamoylphenyl (e.g. 4-carbamoylphenyl, etc.), phenyl(lower)alkylcarbamoylphenyl (e.g. 4-benzylcarbamoylphenyl, etc.), lower alkanoylphenyl (e.g. 4-acetylphenyl, etc.), lower alkylthiophenyl, lower alkylsulfinylphenyl, lower alkylsulfonylphenyl (e.g. 4-methylthiophenyl, 4-ethylthiophenyl, 4-methylsulfinylphenyl, 4-methylsulfonylphenyl, etc.), phenylphenyl, (halo)(phenyl)phenyl (e.g. 4-phenyl-3-fluorophenyl etc.), halophenylphenyl (e.g. 4-(4-fluorophenyl)phenyl etc.) hydroxyphenyl (e.g. 3(or 4)-hydroxyphenyl, etc.), mono- or dihydroxy (lower)alkylphenyl, phenoxycarbonyloxy(lower)alkylphenyl, (e.g. 3(or 4)-hydroxymethylphenyl, 4-(1,2-dihydroxyethyl)phenyl, 4-(phenoxycarbonyloxymethyl)phenyl, etc.), aminophenyl(e.g. 3(or 4)-aminophenyl, etc.), carboxyphenyl (e.g. 4-carboxyphenyl, etc.), lower alkylendioxyphenyl (e.g. 3,4-methylendioxyphenyl, etc.), lower alkanesulfonylaminophenyl (e.g. 4-(methanesulfonylamino)phenyl etc.), lower alkenoylaminophenyl, (e.g. 3-(2-butenoylamino)phenyl, etc.), lower cycloalkanecarbonylaminophenyl (e.g. 3-(cyclopropanecarbonylamino)phenyl, 3-(cyclobutanecarbonylamino)phenyl, 3-(cyclopentanecarbonylamino)phenyl, etc.) phenyl(lower)alkoxyphenyl (e.g. 4-benzyloxyphenyl, etc.), carbamoyl(lower)alkenylphenyl, mono- or di(lower alkyl)carbamoyl(lower)alkenylphenyl (e.g. 4-(2-(methylcarbamoyl)ethenyl)phenyl, 4-(2-(ethylcarbamoyl)ethenyl)phenyl, 4-(2-(propylcarbamoyl) ethenyl)phenyl, 4-(2-(isopropycarbamoyl)ethenyl)phenyl, 4-2-(dimethylcarbamoyl)ethenyl)phenyl, etc.) phenylcarbamoyl(lower)alkenylphenyl (e.g. 4-(2-(phenylcarbamoyl)ethenyl)phenyl, etc.), lower alkoxycarbamoyl(lower)alkenylphenyl (e.g. 4-(2-(methoxyphenylcarbamoyl)ethenyl)phenyl, etc.), halophenylcarbamoyl(lower)alkenylphenyl (e.g. 4-(2-(4-fluorophenylcarbamoyl)ethenyl)phenyl, etc.) lower alkylcarbamoyloxyphenyl (e.g. 4-(methylaminocarbonyloxy)phenyl, 4-(ethylaminocarbonyloxy)phenyl, etc.), lower alkanoyloxyphenyl (e.g. 4-propanoyloxyphenyl, etc.) lower alkoxy(lower)alkanoyloxyphenyl (e.g. 4-(methoxyacetyloxy)phenyl, etc.) lower alkoxycarbonyloxyphenyl (e.g. 4-(ethoxycarbonyloxy)phenyl, etc.) pyridyl(lower)alkenoyloxyphenyl (e.g. 4-(3-(3-pyridyl)acryloyloxy)phenyl, etc.), cyclo (lower)alkylcarbonyloxyphenyl (e.g. 4-(cyclopropylcarbonyloxy)phenyl, etc.), carboxy(lower)alkoxyphenyl (e.g. 4-(carboxymethoxy)phenyl, etc.) lower alkoxycarbonyl(lower)alkoxyphenyl (e.g. 4-(ethoxycarbonylmethoxy)phenyl, 4-(t-butoxycarbonylmethoxy)phenyl, etc.), lower alkanoyl(lower)alkoxyphenyl (e.g. 4-(propanoylmethoxy)phenyl, etc.), lower cycloalkanecarbamoyl(lower)alkoxyphenyl (e.g. 4-(cyclopropylcarbamoylmethoxy)phenyl, etc.), lower alkylcarbamoyl(lower)alkoxyphenyl (e.g. 4-(methylcarbamoylmethoxy)phenyl, 4-(ethylcarbamoylmethoxy)phenyl, 4-(propylcarbamoylmethoxy)phenyl, etc.), lower alkylcarbamoyloxy(lower)alkylphenyl (e.g. 3(or 4)-(methylcarbamoyloxymethyl)phenyl, etc.), lower alkoxycarbonylamino(lower)alkylphenyl (e.g. 4-(methoxycarbonylaminomethyl)phenyl, 4-(t-butoxycarbonylaminomethyl)phenyl, etc.), amino(lower)alkylphenyl (e.g. 4-aminomethylphenyl, etc.), lower alkylcarbamoyl(lower)alkylphenyl (e.g. 4-(methylcarbamoylmethyl)phenyl, etc.), furylcarbonylaminophenyl, 1,2,3,4-tetrahydroisoquinolylcarbonylaminophenyl, N-Boc-1,2,3,4-tetrahydroisoquinolylcarbonylaminophenyl, pyrrolidinylcarbonylaminophenyl (e.g. 3-(2(or 3)-furylcarbonylamino)phenyl, 3-(1,2,3,4-tetrahydroisoquinolylcarbonylamino)phenyl, 3-(N-(t-butoxycarbonyl)-1,2,3,4-tetrahydroisoquinolylcarbonylamino)phenyl, 3-(pyrrolidinylcarbonylamino)phenyl, etc.), oxazolylphenyl (e.g. 4-(1,3-oxazolyl)phenyl, etc.), lower alkyloxadiazolylphenyl, (e.g. 4-(5-methyl-1,2,4-oxadiazol-3-yl)phenyl, etc.);

(b)

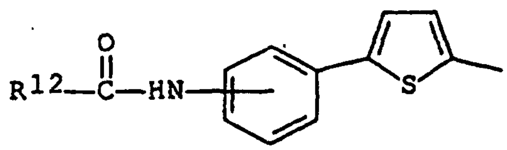

wherein

$R^{12}$ is lower alkyl (e.g. methyl, ethyl, propyl, isopropyl, butyl, isobutyl, t-butyl, neopentyl, etc.) optionally substituted by the group consisting of phenyl (e.g. phenyl, etc.), halophenyl (e.g. 4-chlorophenyl, etc.), lower alkoxyphenyl (e.g. 4-methoxyphenyl, etc.), lower alkoxy (e.g. methoxy, ethoxy, propoxy, butoxy, isopropyloxy, etc.), phenoxy (e.g. phenoxy, etc.), lower alkoxyphenoxy (e.g. 3(or 4)-methoxyphenoxy, etc.), halophenoxy (e.g. 4-fluoro(or chloro)phenoxy, etc.), lower alkylphenoxy (e.g. 3(or 4)-methylphenoxy, etc.), carboxy (e.g. carboxy, etc.), lower alkoxycarbonyl (e.g. methoxycarbonyl, t-butoxycarbonyl, etc.), lower alkylcarbamoyl (e.g. methylcarbamoyl, etc.), halo (e.g. chloro, etc.), lower alkenyloxy (e.g. allyloxy etc.), lower alkoxy(lower)alkoxy (e.g. 2-ethoxyethoxy, etc.), phenyl(lower)alkoxy (e.g. benzyloxy, etc.), piperidinyloxy (e.g. 4-piperidinyloxy, etc.), N-t-butoxycarbonylpiperidinyloxy (e.g. N-t-butoxycarbonyl-4-piperidinyloxy, etc.), pyridyloxy (e.g. 3(or 4)-pyridyloxy, etc.), hydroxy (e.g. hydroxy, etc.), lower alkanoyloxy (e.g. acetoxy etc.), mono- or di(lower)alkylcarbamoyloxy (e.g. methylcarbamoyloxy, N-methyl-N-ethylcarbamoyloxy, etc.), piperidinylcarbonyloxy (e.g. piperidinocarbonyloxy, etc.), phenyl (lower)alkylcarbamoyloxy (e.g. benzylcarbamoyloxy, etc.), lower alkoxycarbonylamino (e.g. methoxycarbonylamino, ethoxycarbonylamino, propoxycarbonylamino, t-butoxycarbonylamino, etc.), amino (e. g. amino, etc.), lower alkoxycarbonylamino (e.g. methoxycarbonylamino, t-butoxycarbonylamino, etc.), fluorenylmethoxycarbonylamino (e.g. fluorenylmethoxycarbonylamino etc.), mono- or di(lower) alkylamino (e.g. methylamino ethylamino dimethylamino, N-methyl-N-ethylamino t-butylamino, pentylamino etc.), N-lower alkyl-N-(lower alkoxycarbonyl)amino (e.g. N-methyl-N-methoxycarbonylamino, N-methyl-N-t-butoxycarbonylamino N-ethyl-N-t-butoxycarbonylamino, etc.), N-lower alkyl-N-(fluorenylmethoxycarbonyl)amino (e.g. N-methyl-N-(fluorenylmethoxycarbonyl)amino etc.), N-lower alkyl-N-(mono- or di(lower)alkylcarbamoyl)amino (e.g. N-methyl-N-(dimethylcarbamoyl)amino, etc.), N-(mono- or di(lower alkyl)carbamoyl)amino (e.g. dimethylcarbamoylamino N-(ethylcarbamoyl) amino, etc.), benzoylamino (e.g. benzoylamino, etc.), lower alkanoylamino (e.g. acetylamino, isobutyrylamino, pivaloylamino, etc.), lower alkanesulfonylamino (e.g. methanesulfonylamino, etc.), lower alkoxy(lower)alkanoylamino (e.g. methoxyacetylamino, etc.), cycle(lower)alkyloxycarbonylamino (e.g. cyclopentyloxycarbonylamino, etc.), pyridylcarbonylamino (e.g. pyridylcarbonylamino, etc.), morpholinocarbonylamino (e.g. morpholinocarbonylamino, etc.), phenyl(lower)alkoxycarbonylamino (e.g. benzyloxycarbonylamino, etc.), lower alkoxyphenylsulfonylamino (e.g. 4-methoxyphenylsulfonylamino, etc.), hydroxy(lower)alkylamino (e.g. 2-hydroxyethylamino, etc.), morpholino (e.g. morpholino, etc.), oxooxazolidinyl (e.g. 2-oxo-1,3-oxazolidin-1-yl, etc.), oxopyrrolidinyl (e.g. 2-oxopyrrolidin-1-yl, etc.), trimethylhydantoinyl (e.g. 3,4,4-trimethylhydantoin-1-yl, etc.), pyridyl (e.g. 3(or 4)-pyridyl, etc.), lower alkenylamino (e.g. allylamino, etc.), lower alkoxy(lower)alkylamino (e.g. 2-ethoxyethylamino, etc.), phenyl(lower)alkylamino (e.g. benzylamino, etc.), pyridyl(lower)alkylamino (e.g. 3-pyridylmethylamino, etc.), and cycle(lower)alkyl cyclohexyl, etc.),

(c)

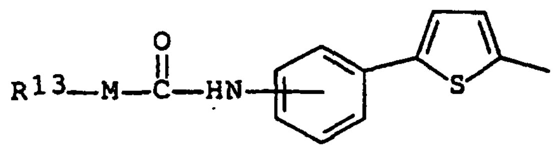

wherein

M is oxygen or sulfur,

$R^{13}$ is lower alkyl (e.g. methyl, ethyl, propyl, isopropyl, etc.), phenyl(lower)alkyl (e.g. benzyl, etc.), lower alkoxy(lower)alkyl (e.g. 2-methoxyethyl, etc.), halo(lower)alkyl (e.g. 2-choloroethyl, etc.), amino(lower) alkyl (e.g. 2-aminoethyl), phthalimido(lower)alkoxycarbonylamino (e.g. 2-phthalimidoethyl, etc.), lower alkenyl (e.g. allyl, etc.), phenyl (e.g. phenyl, etc.),

(d)

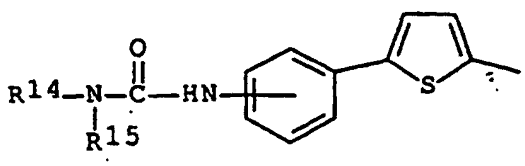

wherein

$R^{15}$ is hydrogen, or lower alkyl (e.g. methyl, etc.),

$R^{14}$ is hydrogen, lower alkyl (e.g. methyl, ethyl, propyl, isopropyl, butyl, isobutyl, pentyl, hexyl, etc.), naphthyl (e.g. 1-naphthyl, etc.), halophenyl (e.g. 3(or 4)-chlorophenyl etc.), lower alkoxyphenyl (e.g. 4-methoxyphenyl, etc.), lower alkenyl (e.g. allyl, etc.), cycle(lower)alkyl(lower)alkyl (e.g. cyclohexylmethyl, etc.), phenyl(lower)alkyl (e.g. benzyl, etc.), halo(lower)alkyl (e.g. 2-chloroethyl, etc.), lower alkoxy(lower)alkyl (e.g. methoxymethyl, 2-methoxyethyl, etc.), hydroxy(lower)alkyl (e.g. 2-hydroxyethyl, etc.), (lower alkyl) (diphenyl)silyloxy(lower)alkyl (e.g. 2-((t-butyl)(diphenyl)silyloxy)ethyl, etc.), carboxy(lower)alkyl (e.g. carboxymethyl, etc.), lower alkoxycarbonyl(lower)alkyl (e.g. ethoxycarbonylmethyl, etc.), lower alkylcarbamoyl(lower)alkyl (e.g. methylcarbamoylmethyl, etc.), or pyridyl (e.g. 3-(e) pyridyl, etc.),

(e)

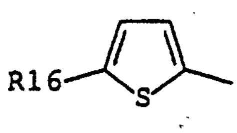

wherein

$R^{16}$ is benzothienyl (e.g. 2-benzothienyl, etc.), benzofuranyl (e.g. 2-benzofuranyl, etc.), thienyl (e.g. 2 (or 3)-thienyl, etc.), furyl (e.g. 2-furyl, etc.), pyridyl (e.g. 3-pyridyl, etc.), lower alkylpyridyl (e.g. 1-methyl-4-pyridyl, 6-methyl-3-pyridyl, etc.), lower alkoxypyridyl (e.g. 6-methoxy-3-pyridyl, etc.), lower alkoxycarbonylaminopyridyl (e.g. 5-methoxycarbonylamino-3-pyridyl, etc.), lower alkanoylthienyl (e.g. 5-acetyl-2-thienyl, etc.), lower alkylcarbamoylbenzofuranyl (e.g. 2-methylcarbamoyl-5-benzofuranyl, etc.).

(vi) The compounds (I) of the above (v), wherein a group of the formula:

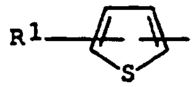

is the same group as (a), (c), (d) and (e) of claim 7, and the following formula (b):

(b)

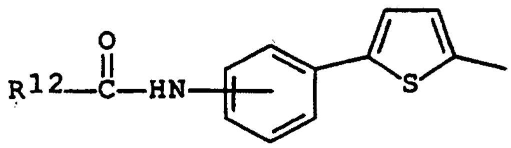

wherein

$R^{12}$ is lower alkyl, phenyl(lower)alkyl, halophenyl(lower)alkyl, lower alkoxyphenyl(lower)alkyl, lower alkoxy (lower)alkyl, phenoxy(lower)alkyl, lower alkoxyphenoxy(lower)alkyl, halophenoxy(lower)alkyl, lower alkylphenoxy(lower)alkyl, carboxy(lower)alkyl, lower alkoxycarbonyl(lower)alkyl, lower alkylcarbamoyl (lower)alkyl, halo(lower)alkyl, lower alkenyloxy(lower)alkyl, lower alkoxy(lower)alkoxy(lower)alkyl, phenyl(lower)alkoxy(lower)alkyl, piperidinyloxy(lower)alkyl, N-t-butoxycarbonylpiperidinyloxy(lower)alkyl, pyridyloxy(lower)alkyl, hydroxy(lower)alkyl, lower alkanoyloxy(lower)alkyl, mono- or di(lower)alkylcarbamoyloxy(lower)alkyl, piperidinylcarbonyloxy(lower)alkyl, phenyl(lower)alkylcarbamoyloxy(lower) alkyl, amino(lower)alkyl, lower alkoxycarbonylamino(lower)alkyl, fluorenylmethoxycarbonylamino(lower)alkyl, mono- or di(lower)alkylamino(lower)alkyl, N-lower alkyl-N-(lower alkoxycarbonyl)amino(lower) alkyl, N-lower alkyl-N-(fluorenylmethoxycarbonyl)amino(lower)-alkyl, N-lower alkyl-N-(mono- or di(lower)-alkylcarbamoyl)amino(lower)alkyl, N-(mono- or di(lower alkyl)carbarnoyl)amino(lower)alkyl, benzoylamino(lower)alkyl, lower alkanoylamino(lower)alkyl, lower alkanesulfonylamino(lower)alkyl, lower alkoxy(lower)alkanoylamino(lower)alkyl, cyclo(lower)alkyloxycarbonylamino(lower)alkyl, pyridylcarbonylamino(lower)alkyl, morpholinocarbonylamino(lower)alkyl, phenyl(lower)alkoxycarbonylamino(lower)alkyl, lower alkoxyphenylsulfonylamino(lower)alkyl, hydroxy(lower)alkylamino(lower)alkyl, morpholino(lower)alkyl, oxooxazolidinyl(lower)alkyl, oxopyrrolidinyl(lower)alkyl, trimethylhydantoinyl(lower)alkyl, pyridyl(lower)alkyl, lower alkenylamino(lower)alkyl, lower alkoxy(lower)alkylamino(lower)alkyl, phenyl(lower)alkylamino(lower)alkyl, pyridyl(lower)alkylamino(lower)alkyl, cycl(lower)oalkyl, (amino) (phenyl)(lower)alkyl (e.g. 2-phenyl-1-aminoethyl, etc.), (lower alkoxycarbonylamino)(phenyl)(lower) alkyl (e.g. 1-amino-1-phenylmethyl, 1-t-butoxycarbonylamino-1-phenylmethyl, 1-amino-2-phenylethyl, 1-t-butoxycarbonylamino-2-phenylethyl, etc.), (amino)(lower alkoxy)(lower)alkyl (e.g. 1-amino-2-methoxyethyl, etc.), (lower alkoxycarbonylamino)-(lower alkoxy)(lower)alkyl (e.g. 1-t-butoxycarbonylamino-2-methoxyethyl, etc.), (amino)(carboxy)(lower)alkyl, (lower alkoxycarbonylamino)(carboxy)(lower) alkyl, (amino)(lower alkoxycarbonyl)(lower)alkyl, (lower alkoxycarbonylamino)(lower alkoxycarbonyl) (lower)alkyl (e.g. 1-amino-3-carboxypropyl, 1-t-butoxycarbonylamino-3-carboxypropyl, 1-amino-3-(t-butoxycarbonyl)propyl, 1-t-butoxycarbonylamino-3-t-butoxycarbonylpropyl, etc.) (amino)(phenyl(lower)alkoxy)(lower)alkyl, (lower alkoxycarbonylamino)(phenyl(lower)alkoxy)-(lower)alkyl (e.g. 1-amino-2-benzyloxyethyl, 1-t-butoxycarbonylamino-2-benzyloxyaminoethyl, etc.), (amino)(pyridyl)(lower)alkyl, (lower alkoxycarbonylamino)(pyridyl)(lower)alkyl (e.g. 1-amino-2-(3-pyridyl)ethyl, 1-t-butoxycarbonylamino-2-(3-pyridyl)ethyl, 1-amino-2-(9-pyridyl)ethyl, 1-t-butoxycarbonylamino-2-(4-pyridyl)ethyl, etc.), (amino)(hydroxy)(lower)alkyl, (lower alkoxycarbonylamino)(hydroxy)(lower)alkyl (e.g. 1-amino-2-hydroxyethyl, 1-t-butoxycarbonylamino-2-hydroxyethyl, etc.), (amino)(amino)(lower)alkyl, (lower alkoxycarbonylamino) (amino) (lower)alkyl, (amino) (lower alkoxycarbonylamino) (lower)alkyl, (lower alkoxycarbonylamino)(lower alkoxycarbonylamino)(lower)alkyl (e.g. 1,5-diaminopentyl, 1-t-butoxycarbonylamino-5-aminopentyl, 1,5-bis(t-butoxycarbonylamino)pentyl, 1-amino-5-(t-butoxycarbonylamino)pentyl, etc.), (amino)(lower cycloalkane)(lower)alkyl, (lower alkoxycarbonylamino)(lower cycloalkane)(lower)alkyl (e.g. 1-amino-2-cyclohexylethyl, 1-t-butoxycarbonylamino-2-cyclohexylethyl, etc.).

(vii) The compounds (I) of the above (vi), wherein a group of the formula:

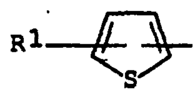

is the group of the following formula (a) to (e) :

(a)

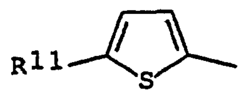

wherein

$R^{11}$ is 2-naphthyl, phenyl, 3(or 4)-chlorophenyl, 2(or 3 or 4)-fluorophenyl, 3,4-dichloropheny, 3,5-difluorophenyl, 3(or 4)-methylphenyl, 4-ethylphenyl, 4-isopropylphenyl, 4-(t-butyl)phenyl, 3,4-dimethylphenyl, 4-methoxyphenyl, 4-ethoxyphenyl, 4-trifluoromethylphenyl, 4-trifluoromethoxyphenyl, 4-ethenylphenyl, - 4-methylcarbamoylphenyl, 4-ethylcarbamoylphenyl, 4-carbamoylphenyl, 4-benzylcarbamoylphenyl, 4-acetylphenyl, 4-methylthiophenyl, 4-ethylthiophenyl, 4-methylsulfinylphenyl, 4-methylsulfonylphenyl, phenylphenyl, 4-phenyl-3-fluorophenyl, 4-(4-fluorophenyl)phenyl, 3(or 4)-hydroxyphenyl, 3(or 4)-hydroxymethylphenyl, 4-(1,2-dihydroxyethyl)phenyl, 4-(phenoxycarbonyloxymethyl)phenyl, 3(or 4)-aminophenyl, 4-carboxyphenyl, 3,4-methylendioxyphenyl, 4-(methanesulfonylamino)phenyl, 3-(2-butenoylamino)phenyl, 3-(cyclopropanecarbonylamino)phenyl, 3-(cyclobutanecarbonylamino)phenyl, 3-(cyclopentanecarbonylamino)phenyl, 4-benzyloxyphenyl, 4-(2-(methylcarbamoyl)ethenyl)phenyl, 4-(2-(ethylcarbamoyl)ethenyl)phenyl, 4-(2-(propylcarbamoyl)ethenyl)phenyl, 4-(2-(isopropylcarbamoyl)ethenyl)phenyl, 4-2-(dimethylcarbamoyl)ethenyl)phenyl, 4-(2-(phenylcarbamoyl)ethenyl)phenyl, 4-(2-(methoxyphenylcarbamoyl)ethenyl)phenyl, 4-(2-(4-fluorophenylcarbamoyl)ethenyl)phenyl, 4-(methylaminocarbonyloxy)phenyl, 4-(ethylaminocarbonyloxy)phenyl, 4-propanoyloxyphenyl, 4-(methoxyacetyloxy)phenyl, 4-(ethoxycarbonyloxy)phenyl, 4-(3-(3-pyridyl)acryloyloxy)phenyl, 4-(cyclopropylcarbonyloxy)phenyl, 4-(carboxymethoxy)phenyl, 4-(ethoxycarbonylmethoxy)phenyl, 4-(t-butoxycarbonylmethoxy)phenyl, 4-(propanoylmethoxy)phenyl, 4-(cyclopropylcarbamoylmethoxy)phenyl, 3(or 4)-(methylcarbamoylmethoxy)phenyl, 4-(ethylcarbamoylmethoxy)phenyl, 4-(propylcarbamoylmethoxy)phenyl, 3 (or 4)-(methylcarbamoyloxymethyl)phenyl, 4-(methoxycarbonylaminomethyl)phenyl, 9-(t-butoxycarbonylaminomethyl)phenyl, 4-aminomethylphenyl, 4-(methylcarbamoylmethyl)phenyl, 3-(2(or 3)-furylcarbonylamino)phenyl, 3-(1,2,3,4-tetrahydroisoquinolylcarbonylamino)phenyl, 3-(N-(t-butoxycarbonyl)-1,2,3,4-tetrahydroisoquinolylcarbonylamino)phenyl, 3-(pyrrolidinylcarbonylamino)phenyl, 4-(1,3-oxazolyl)phenyl, 4-(5-methyl-1,2,4-oxadiazol-3-yl)phenyl,

(b)

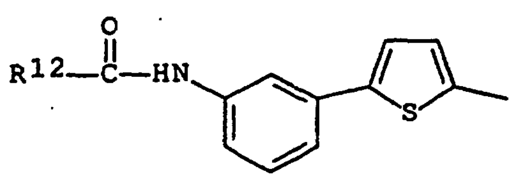

wherein

$R^{12}$ is methyl, ethyl, propyl, isopropyl, butyl, isobutyl, t-butyl, neopentyl, phenylmethyl, 4-chlorophenylmethyl, 4-methoxyphenylmethyl, methoxymethyl, ethoxymethyl, propoxymethyl, butoxymethyl, isopropyloxymethyl, 1-methoxyethyl, 2-methoxyethyl, phenoxymethyl, 2-phenoxyethyl, 3(or 4)-methoxyphenoxymethyl, 4-fluoro(or chloro)phenoxymethyl, 3(or 4)-methylphenoxymethyl, 2-carboxyethyl, 2-methoxycarbonylethyl, 2-t-butoxycarbonylethyl, 2-methylcarbamoylethyl, 2-chloroethyl, chloromethyl, allyloxymethyl, (2-ethoxyethoxy)methyl, benzyloxymethyl, 4-piperidinyloxymethyl, (N-t-butoxycarbonyl-4-piperidinyl)oxymethyl, 3(or 4)-pyridyloxymethyl, hydroxymethyl, 2-hydroxyethyl, acetoxymethyl, 1-acetoxyethyl, methylcarbamoyloxymethyl, 1-(N-methyl-N-ethylcarbamoyloxy)methyl, (piperidinocarbonyloxy)methyl, (benzylcarbamoyloxy)methyl, (t-butoxycarbonylamino)methyl, aminomethyl, 1-aminoethyl, 1-(t-butoxycarbonylamino)ethyl, 2-aminoethyl, methoxycarbonylaminomethyl, 2-(methoxycarbonylamino)ethyl, ethoxycarbonylaminomethyl, propoxycarbonylaminomethyl, 1-(fluorenylmethoxycarbonylamino)methyl, 2-(t-butoxycarbonylamino)ethyl, 2-(fluorenylmethoxycarbonylamino)ethyl, 1-aminoisopropyl, 1-aminopropyl, 1-(t-butoxycarbonylamino)propyl, 1-(t-butoxycarbonylamino)isopropyl, 1,5-diaminopentyl, 1,5-bis(t-butoxycarblnylamino)-pentyl, methylaminomethyl, ethylaminomethyl, dimethylaminomethyl, pentylaminomethyl, t-butylaminomethyl, (N-methyl-N-methoxycarbonylamino) methyl, 1-(N-methyl-N-t-butoxycarbonylamino)methyl, 1-(N-ethyl-N-t-butoxycarbonylamino)methyl, 2-(N-methyl-N-(fluorenylmethoxycarbonyl)amino)-ethyl, 2-(N-methyl-N-(t-butoxycarbonyl)amino) ethyl, 1-(N-methyl-N-(dimethylcarbamoyl)amino)methyl, 1-(dimethylcarbamoylamino)methyl, 1-(N-(ethylcarbamoyl)amino)methyl, 2-(N-(ethylcarbamoyl)amino)ethyl, benzoylaminomethyl, 2-benzoylaminoethyl, acetylaminomethyl, isobutyrylaminomethyl, pivaloylaminomethyl, 1-(methanesulfonylamino)methyl, 2-(methanesulfonylamino)ethyl, methoxyacetylaminomethyl, cyclopentyloxycarbonylaminomethyl, pyridylcarbonylaminomethyl, morpholinocarbonylaminomethyl, benzyloxycarbonylaminomethyl, 1-(4-methoxyphenylsulfonylamino)methyl, 1-(2-hydroxyethylamino)methyl, morpholinomethyl, 1-(2-oxo-1,3-oxazolidin-1-yl)methyl, 1-(2-oxopyrrolidin-1-yl)methyl, 1-(3,4,4-trimethylhydantoin-1-yl)methyl, allylaminomethyl, 1-(2-ethoxyethylamino)methyl, benzylaminomethyl, 1-(3-pyridylmethylamino)methyl, 2-phenyl-1-aminoethyl, 1-amino-1-phenylmethyl, 1-t-butoxycarbonylamino-1-phenylmethyl, 1-amino-2-phenylethyl, 1-t-butoxycarbonylamino-2-phenylethyl, 1-amino-2-methoxyethyl, 1-t-butoxycarbonylamino-2-methoxyethyl, 1-amino-3-carboxypropyl, 1-t-butoxycarbonylamino-3-carboxypropyl, 1-amino-3-(t-butoxycarbonyl)propyl, 1-t-butoxycarbonylamino-3-t-butoxycarbonylpropyl, etc.), 1-amino-2-benzyloxyethyl, 1-t-butoxycarbonylamino-2-benzyloxyaminoethyl, 1-amino-2-(3-pyridyl)ethyl, 1-t-butoxycarbonylamino-2-(3-pyridyl)ethyl, 1-amino-2-(4-pyridyl)ethyl, 1-t-butoxycarbonylamino-2-(4-pyridyl)ethyl, 1-amino-2-hydroxyethyl, 1-t-butoxycarbonylamino-2-hydroxyethyl, (1,5-diaminopentyl, 1-t-butoxycarbonylamino-5-aminopentyl, 1,5-bis(t-butoxycarbonylamino)pentyl, 1-amino-5-(t-butoxycarbonylamino)pentyl, 1-amino-2-cyclohexylethyl, 1-t-butoxycarbonylamino-2-cyclohexylethyl,

(c)

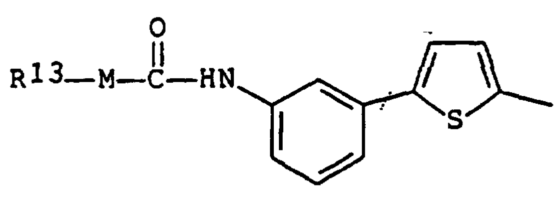

wherein

M=O and $R^{13}$ is methyl, ethyl, propyl, isopropyl, benzyl, 2-methoxyethyl, 2-choloroethyl, 2-aminoethyl, 2-phthalimidoethyl, allyl, phenyl, or

M=S and $R^{13}$ is methyl, ethyl,

(d)

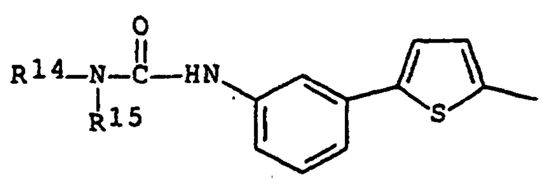

wherein

$R^{15}$ is hydrogen and
$R^{14}$ is hydrogen, methyl, ethyl, propyl, isopropyl, butyl, isobutyl, pentyl, hexyl, 1-naphthyl, 3(or 4)-chlorophenyl, 3-methoxyphenyl, allyl, cyclohexylmethyl, benzyl, 2-chloroethyl, methoxymethyl, 2-methoxyethyl, 2-hydroxyethyl, 2-((t-butyl)(diphenyl)silyloxy)ethyl, carboxymethyl, ethoxycarbonylmethyl, methylcarbamoylmethyl, or 3-pyridyl,
$R^{14}$ is ethyl and $R^{15}$ is methyl,

(e)

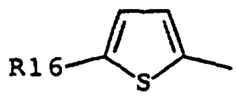

wherein

$R^{16}$ is 2-benzothienyl, 2-benzofuranyl, 2(or 3)-thienyl, 2-furyl, 3-pyridyl, 1-methyl-4-pyridyl, 6-methyl-3-pyridyl, 6-methoxy-3-pyridyl,

5-methoxycarbonylamino-3-pyridyl, 5-acetyl-2-thienyl, 2-methylcarbamoyl-5-benzofuranyl.

The processes for preparing the object compounds are explained in detail in the following.

Process 1

The object compound (I-b) or a salt thereof can be prepared by subjecting a compound (I-a) or a salt thereof to removal reaction of the carboxy-protective group.

Suitable salts of the compounds (I-a) and (I-b) can be referred to the ones as exemplified for the compound (I).

This reaction is carried out in accordance with a conventional method such as solvolysis including hydrolysis, reduction or the like.

The solvolysis is preferably carried out in'the presence of a base or an acid including Lewis acid.

Suitable base may include an inorganic base'and an organic base such as an alkali metal [e.g. sodium, lithium, potassium, etc.], an alkaline earth metal [e.g. magnesium, calcium, etc.], the hydroxide or carbonate or bicarbonate thereof, hydrazine, trialkylamine [e.g. trimethylamine, triethylamine, etc.], picoline, 1,5-diazabicyclo[4.3.0]-non-5-ene, 1,4-diazabicyclo[2.2.2]octane, 1,8-diazabicyclo[5.4.0]undec-7-ene, or the like.

Suitable acid may include and organic acid [e.g. formic acid, acetic acid, propionic acid, trichloroacetic acid, trifluoroacetic acid, etc.], an inorganic acid [e.g. hydrochloric acid, hydrobromic acid, sulfuric acid, hydrogen chloride, hydrogen bromide, hydrogen fluoride, boron trifluoride diethyl etherate, hydrogen iodide, etc.].

The removal reaction using Lewis acid such as trihaloacetic acid [e.g. trichloroacetic acid, trifluoroacetic acid, etc.] or the like, is preferably carried out in the presence of cation trapping agents [e.g. anisole, phenol, etc.].

The reaction is usually carried out in a solvent such as water, an alcohol [e.g. methanol, ethanol, etc.], methylene chloride, chloroform, carbon tetrachloride, dioxane, tetrahydrofuran, N,N-dimethylformamide, a mixture thereof or any other solvent which does not adversely influence the reaction. A liquid base or acid can be also used as the solvent. The reaction temperature is not critical and the reaction is usually carried out under cooling to heating.

The reduction method applicable for the removal reaction may include chemical reduction and catalytic reduction.

Suitable reducing agents to be used in chemical reduction may include a combination of metal [e.g. tin, zinc, iron, etc.] or metallic compound [e.g. chromium chloride, chromium acetate, etc.] and an organic or inorganic acid [e.g. formic acid, acetic acid, propionic acid, trifluoroacetic acid, p-toluenesulfonic acid, hydrochloric acid, hydrobromic acid, etc.].

Suitable catalysts to be used in catalytic reduction are conventional ones such as platinum catalysts [e.g. platinum plate, spongy platinum, platinum black, colloidal platinum, platinum oxide, platinum wire, etc.], palladium catalysts [e.g. spongy palladium, palladium black, palladium oxide, palladium on carbon, colloidal palladium, palladium on barium sulfate, palladium on barium carbonate, etc.], nickel catalysts [e.g. reduced nickel, nickel oxide, Raney nickel, etc.], cobalt catalysts [e.g. reduced cobalt, Raney cobalt, etc.], iron catalysts [e.g. reduced iron, Raney iron, etc.], copper catalysts [e.g. reduced copper, Raney copper, Ullman copper, etc.] and the like, and these catalysts may

be used in a combination with ammonium formate (e.g. a combination of palladium on carbon and ammonium formate, etc.).

The reduction is usually carried out in a conventional solvent which does not adversely influence the reaction such as water, methanol, ethanol, propanol, N,N-dimethylformamide, or a mixture thereof. Additionally, in case that the above-mentioned acids to be used in chemical reduction are in liquid form, they can also be used as a solvent. Further, a suitable solvent to be used in catalytic reduction may be the above-mentioned solvent, and other conventional solvent such as diethyl ether, dioxane, tetrahydrofuran, etc., or a mixture thereof.

The reaction temperature of this reduction is not critical and the reaction is usually carried out under cooling to heating.

Process 2

The compound (I-b) or a salt thereof can be prepared by oxidating the compound (II) or a salt thereof.

Suitable salts of the compound (II) may be the same as those for the compound (I).

Oxidation is carried out in a conventional manner, which is capable of oxidating a vinyl group to a carboxy group, and suitable oxidizing reagent may be oxygen acid such as periodate (e.g. sodium periodate, potassium periodate, etc.), peroxy acid such as perbenzoic acid (e.g., perbenzoic acid, m-chloroperbenzoic acid, etc.), OXONE ($2KHSO_5 \cdot KHSO_4 \cdot K_2SO_4$), potassium permanganate, a combination of titanium (III) chloride and hydrogen peroxide, a combination thereof (e.g. a combination of potassium permanganate and sodium periodate, etc.), and the like.

This reaction can be carried out in the presence of a suitable base as mentioned above (e.g. potassium carbonate, etc.).

The reaction is usually carried out in a conventional solvent such as water, alcohol (e.g., methanol, ethanol, isopropyl alcohol, t-butyl alcohol, etc.), tetrahydrofuran, dioxane, dichloromethane, ethylene dichloride, chloroform, N, N-dimethylformamide, N,N-dimethylacetamide, or any other organic solvent which does not adversely affect the reaction.

Among these solvents, hydrophilic solvents may be used in a mixture with water.

The reaction temperature is not critical and the reaction is usually carried out under cooling to heating.

Process 3

The compound (I-d) or a salt thereof can be prepared by subjecting the compound (I-c) or a salt thereof to a reduction reaction.

Suitable salts of the compounds (I-c) and (I-d) may be the same as those for the compound (I).

The reduction method applicable for this reaction may be the same as Process 1, which is capable of converting haloaryl group to aryl group (e.g. a combination of palladium on carbon and ammonium formate, etc.).

Process 4

The compound (I-e) or a salt thereof can be prepared by reacting the compound (I-b) or its reactive derivative at the carboxy group, or a salt thereof with compound (IV) or its reactive derivative at the amino group, or a!salt thereof.

Suitable reactive derivative at the amino group of the compound (IV) may include Schiff's base type imino or its tautomeric enamine type isomer formed by the reaction of the compound (IV) with a carbonyl compound such as aldehyde, ketone or the like; a silyl derivative formed by the reaction of the compound (IV) with a silyl compound such as bis(trimethylsilyl)acetamide, mono(trimethylsilyl)acetamide, bis(trimethylsilyl)urea or the like; a derivative formed by reaction of the compound (IV) with phosphorus trichloride or phosgene, and the like.

Suitable salts of the compound (IV) and its reactive derivative can be referred to the acid addition salts as exemplified for the compound (I).

Suitable salts of the compound (I-e) may be the same as those for the compound (I).

Suitable reactive derivative at the carboxy group of the compound (I-b) may include an acid halide, an acid anhydride, an activated amide, an activated ester, and the like. Suitable examples of the reactive derivatives may be an acid chloride; an acid azide; a mixed acid anhydride with an acid such as substituted phosphoric acid [e.g. dialkylphosphoric acid, phenylphosphoric acid, diphenylphosphoric acid, dibenzylphosphoric acid, halogenated phosphoric acid, etc.], dialkylphosphorous acid, sulfurous acid, thiosulfuric acid, sulfuric acid, sulfonic acid [e.g. methanesulfonic acid, etc.], aliphatic carboxylic acid [e.g. acetic acid, propionic acid, butyric acid, isobutyric acid, pivalic acid, pentanoic acid, isopentanoic acid, 2-ethylbutyric acid, trichloroacetic acid, etc.] or aromatic carboxylic acid [e.g. benzoic acid, etc.]; a symmetrical acid anhydride; an activated amide with imidazole, 4-substituted imidazole, dimethylpyrazole, triazole or tetrazole; or an activated ester [e.g. cyanomethyl ester, methoxymethyl ester, dimethyliminomethyl

$$[(CH_3)_2\overset{+}{N}=CH-]$$

ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, etc.], or an ester with a N-hydroxy compound [e.g. N,N-dimethylhydroxylamine, 1-hydroxy-2-(1H)-pyridone, N-hydroxysuccinimide, N-hydroxyphthalimide, 1-hydroxy-1H-benzotriazole, etc.], and the like. These reactive derivatives can optionally be selected from them according to the kind of the compound (I-b) to be used.

The reaction is usually carried out in a conventional solvent such as water, alcohol [e.g. methanol, ethanol, etc.], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvent may also be used in a mixture with water.

In this reaction, when the compound (I-b) is: used in a free acid form or its salt form, the reaction is preferably carried out in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)carbodiimide; N,N'-diethylcarbodiimide, N,N'-diisopropylcarbodiimide; 1-ethyl-3-(3-dimethylaminopropyl)carbodiimide (WSCD); N,N'-carbonylbis (2-methylimidazole); pentamethyleneketene-N-cyclohexylimine; diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus trichloride; diphenyl phosphorylazide; thionyl chloride; oxalyl chloride; lower alkyl haloformate [e.g. ethyl chloroformate, isopropyl chloroformate, etc.]; triphenylphosphine; 2-ethyl-7-hydroxybenzisoxazolium salt; 2-ethyl-5-(m-sulfophenyl)-isoxazolium hydroxide intramolecular salt; N-hydroxybenzotriazole; 1-(p-chlorobenzenesulfonyloxy)-6-chloro-1H-benzotriazole; so-called Vilsmeier reagent prepared by the reaction of N,N-dimethylformamide with thionyl chloride, phosgene, trichloromethyl chloroformate, phosphorus oxychloride, etc.; or the like.

The reaction may also be carried out in the presence of an inorganic or organic base as mentioned above such as an alkali metal bicarbonate, tri(lower)alkylamine, pyridine, N-(lower)alkylmorpholine, N,N-di(lower)alkylbenzylamine, alkali metal hydroxide, or the like.

The reaction temperature is not critical, and the reaction is usually carried out under cooling to warming.

Process 5

The object compound (I-f) or a salt thereof can be prepared by cyclizing the compound (III) or a salt thereof.

Suitable salts of the compounds (I-f) and (III) may be the same as those for the compound (I).

This reaction is preferably carried out in the presence of hydrogen halide (e.g. hydrogen iodide, etc.) or alkali metal halide (e.g. sodium iodide, etc.).

This reaction can be carried out in the presence of a suitable base as mentioned above such as alkali metal hydroxide.

The reaction can be carried out in a conventional solvent, which does not adversely influence the reaction as mentioned above such as water, tetrahydrofuran, alcohol (e.g. methanol, ethanol, etc.), a mixture thereof, and the like.

The reaction temperature is not critical and the reaction can be carried out under from warming to heating.

Process 6

The compound (I-g) or a salt thereof can be prepared by reacting the compound (I-b) or its reactive derivative at the carboxy group, or a salt thereof, with an optically active amine or its reactive derivative at the amino group, or a salt thereof.

Suitable "optically active amine" may include a conventional one which is capable of separating the starting racemic compound into each optically active compound such as (R)-(+)-$\alpha$-methylbenzylamine, and the like.

Suitable salts of the compound (I-g) may be the same as those exemplified for the compound (I).

Suitable salts of the optically active amine may be acid addition salts as mentioned for the compound (I).

The reaction is usually carried out in a conventional solvent such as water, acetone, dioxane, acetonitlrile, chloroform, ethylene chloride, tetrahydrofuran, ethyl acetate, N,N-dimethylformamide, pyridine and dichloromethane, a mixture thereof, or any other organic solvents which do not adversely affect the reaction.

This reaction can be carried out in the presence of an organic or inorganic base such as alkali metal (e.g. lithium, sodium, potassium, etc.), alkaline earth metal (e.g., calcium, etc.), alkali metal hydride (e.g., sodium hydride, etc.), alkaline earth metal hydride (e.g., calcium hydride, etc.), alkali metal hydroxide (e.g., sodium hydroxide, potassium hydroxide, etc.), alkali metal carbonate (e.g., sodium carbonate, potassium carbonate, etc.), alkali metal bicarbonate

(e.g., sodium bicarbonate, potassium bicarbonate, etc.), alkali metal alkoxide (e.g., sodium methoxide, sodium ethoxide, potassium tert-butoxide, etc.), alkali metal alkanoic acid (e.g., sodium acetate, etc.), trialkylamine (e.g., triethylamine, etc.), pyridine compound (e.g., pyridine, lutidine, picoline, 4-dimethylaminopyridine, etc.), quinoline, lithium diisopropylamide, and the like.

Suitable reactive derivative at the amino group of optically active amine may include Schiff's base type imino or its tautomeric enamine type isomer formed by the reaction of the said amine with a carbonyl compound such as aldehyde, ketone or the like; a silyl derivative formed by the reaction of the said amine with a silyl compound such as bis(trimethylsilyl)acetamide, mono(trimethylsilyl)acetamide, bis(trimethylsilyl)urea or the like; a derivative formed by the reaction of the said amine with phosphorus trichloride or phosgene, and the like.

Suitable reactive derivative at the carboxy group and salts of the compound (I-b) may be the same as mentioned above.

The reaction is preferably carried out in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)carbodiimide; N,N'-diethylcarbodiimide; N,N'-diisopropylcarbodiimide; 1-ethyl-3-(3-dimethylaminopropyl)carbodiimide; N,N'-carbonylbis-(2-methylimidazole); pentamethyleneketene-N-cyclohexylimine; diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus trichloride; diphenyl phosphorylazide; thionyl chloride; oxalyl chloride; lower alkyl haloformate (e.g., ethyl chloroformate, isopropyl chloroformate); triphenylphosphine; 2-ethyl-7-hydroxybenzisoxazolium salt; 2-ethyl-5-(m-sulfophenyl)isoxazolium hydroxide intramolecular salt; 1-(p-chlorobenzenesulfonyloxy)-6-chloro-1H-benzotriazole; 1-hydroxybenzotriazole; or so-called Vilsmeier reagent prepared by the reaction of N,N-dimethylformamide with thionyl chloride, phosgene, trichloromethyl chloroformate, phosphorus oxychloride or oxalyl chloride.

The reaction temperature is not critical, and the reaction is usually carried out under cooling to warming.

<u>Process 7</u>

The compound (I-i) or a salt thereof can be prepared by subjecting the compound (I-h) or a salt thereof to a removal reaction of the hydroxy protective group.

Suitable salts of the compounds (I-h) and (I-i) may be the same as those exemplified for the compound (I).

The reaction of this process can be carried out in a manner similar to that in <u>Process 1</u>.

<u>Process 8</u>

The compound (I-k) and a salt thereof can be prepared by oxidating the compound (I-j) or a salt thereof.

Suitable salts of the compounds (I-j) and (I-k) may be the same as those exemplified above with regard to the compound (I).

Suitable method of this oxidation includes conventional ones, which can convert thia group to sulfinyl or sulfonyl group, or sulfinyl to sulfonyl group, such as exemplified for Process 2.

<u>Process 9</u>

The compound (I-*l*) or a salt thereof can be prepared by reacting the compound (I-c) or a salt thereof with the compound (V).

Suitable salts of the compound (I-*l*) may be the same as those exemplified for the compound (I).

The reaction is usually carried out in a conventional solvent such as water, acetone, dioxane, acetonitrile, chloroform, ethylene chloride, tetrahydrofuran, ethyl acetate, N,N-dimethylformamide, pyridine and dichloromethane, a mixture thereof, or any other organic solvents which do not adversely affect the reaction.

This reaction can be carried out in the presence of an organic or inorganic base such as alkali metal (e.g., lithium, sodium, potassium, etc.), alkaline earth metal (e.g., calcium, etc.), alkali metal hydride (e.g., sodium hydride, etc.), alkaline earth metal hydride (e.g., calcium hydride, etc.), alkali metal hydroxide (e.g., sodium hydroxide, potassium hydroxide, etc.), alkali metal carbonate (e.g., sodium carbonate, potassium carbonate, etc.), alkali metal bicarbonate (e.g., sodium bicarbonate, potassium bicarbonate, etc.), alkali metal alkoxide (e.g., sodium methoxide, sodium ethoxide, potassium tert-butoxide, etc.), alkali metal alkanoic acid (e.g., sodium acetate, etc.), trialkylamine (e.g., triethylamine, etc.), pyridine compound (e.g., pyridine, lutidine, picoline, 4-dimethylaminopyridine, etc.), quinoline, lithium diisopropylamide, alkali metal halide (e.g., sodium iodide, potassium iodide, etc.), alkali metal thiocyanate (e.g., sodium thiocyanate, potassium thiocyanate, etc.), di(lower)alkyl azodicarboxylate (e.g., diethyl azodicarboxylate, diisopropyl azodicarboxylate, etc.), and the like.

The reaction is preferably carried out in the presence of a conventional condensing agent such as

N,N'-dicyclohexylcarbodiimide; N-cyclohexyl-N'-morpholinoethylcarbodiimide; N-cyclohexyl-N'-(4-diethylaminocyclohexyl)carbodiimide; N,N'-diethylcarbodiimide; N,N'-diisopropylcarbodiimide; N-ethyl-N'-(3-dimethylaminopropyl) carbodiimide; N,N'-carbonylbis-(2-methylimidazole); pentamethyleneketene-N-cyclohexylimine; diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus trichloride; diphenyl phosphorylazide; thionyl chloride; oxalyl chloride; lower alkyl haloformate (e.g., ethyl chloroformate, isopropyl chloroformate); triphenylphosphine; etrakis(triphenylphosphine)palladium(0); 2-ethyl-7-hydroxybenzisoxazolium salt; 2-ethyl-5-(m-sulfophenyl)isoxazolium hydroxide intramolecular salt; 1-(p-chlorobenzenesulfonyloxy)-6-chloro-1H-benzotriazole; 1-hydroxybenzotriazole; or so-called Vilsmeier reagent prepared by the reaction of N,N-dimethylformamide with thionyl chloride, phosgene, trichloromethyl chloroformate, phosphorus oxychloride or oxalyl chloride.

The reaction temperature is not critical, and the reaction is usually carried out under from warming to heating.

Process 10

The compound (I-m) or a salt thereof can be prepared by reacting the compound (VI) or a salt thereof with the compound (VII) or a salt thereof.

Suitable salts of the compound (I-m) may be the same as those exemplified for the compound (I).

The reaction of this process can be carried out in a manner similar to that in Process 9.

Process 11

The compound (I-n) or a salt thereof can be prepared by cyclizing the compound (VIII) or a salt thereof.

Suitable salts of the compounds (I-n) and (VIII) may be the same as those exemplified for the compound (I).

This reaction can be carried out in the presence of a suitable acid as exemplified for Process 1, wherein preferable one may be trifluoroacetic acid, and the like.

The reaction can be carried out in a conventional solvent, which does not adversely influence the,reaction as mentioned above such as water, tetrahydrofuran, alcohol (e.g. methanol, ethanol, etc.), a mixture thereof, and the like.

The reaction temperature is not critical and the reaction can be carried out under cooling to warming.

Process 12

The compound (I-q) or a salt thereof can be prepared by amidating the compound (I-p) or its reactive derivative at the carboxy group, or a salt thereof.

Suitable salts of the compounds (I-p) and (I-q) may be the same as those for the compound (I).

Suitable reactive derivative of the compound (I-p) may be the same as those for the compound (I-b).

The amidation reaction applicable to this process may include a conventional amidation reaction which can convert a carboxy-group to an amido-group, for example, reaction with an optionally substituted amines such as mono- or di(lower)alkylamine (e.g.'methylamine, etc.), and the like.

And the reaction can be carried out in substantially the same manner as described in Process 4.

Process 13

The compound (I-s) or a salt thereof can be prepared by acylating the compound (I-r) or its reactive derivative at the amino group, or a salt thereof.

Suitable salts of the compounds (I-r) and (I-s) may be the same as those for the compound (I).

Suitable acylating agent used in this reaction may be a conventional acylating agent which is capable of introducing the acyl group as mentioned before such as carboxylic acid, carbonic acid, sulfonic acid and their reactive derivative, for example, an acid halide, an acid anhydride, an activated amide, an activated ester, and the like. Preferable example of such reactive derivative may include acid chloride, acid bromide, a mixed acid anhydride with an acid such as substituted phosphoric acid (e.g. dialkylphosphoric acid, phenylphosphoric acid, diphenylphosphoric acid, dibenzylphosphoric acid, halogenated phosphoric acid, etc.), dialkylphosphorous acid, sulfurous acid, thiosulfuric acid, sulfuric acid, alkyl carbonate (e.g. methyl carbonate, ethyl carbonate, propyl carbonate, etc.), aliphatic carboxylic acid (e.g. pivalic acid, pentanoic acid, isopentanoic acid, 2-ethylbutyric acid, trichloroacetic acid, etc.), aromatic carboxylic acid (e.g. benzoic acid, etc.), a symmetrical acid anhydride, an activated acid amide with a heterocyclic compound containing imino function such as imidazole, 4-substituted imidazole, dimethylpyrazole, triazole and tetrazole, an activated ester (e.g. p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyridyl ester, piperidinyl ester, 8-quinolyl thioester, or an ester with a N-hydroxy compound such as N,N-dimethylhydroxylamine, 1-hydroxy-2-(1H)-pyridone, N-hydroxysuccinimide, N-hydroxyphthalimide, 1-hydroxybenzotriazole, 1-hydroxy-6-chlorobenzotriazole, etc.), isocyanic acid or a salt thereof (e.g. sodium isocyanate, etc.), lower alkylisocyanate (e.g. methylisocyanate, ethylisocyanate, etc.), and the like.

This reaction can be carried out in the presence of an organic or inorganic base such as alkali metal (e.g. lithium, sodium potassium, etc.), alkaline earth metal (e.g. calcium, etc.), alkali metal hydride (e.g. sodium hydride, etc.), alkaline earth metal hydride (e.g. calcium hydride, etc.), alkali metal hydroxide (e.g. sodium hydroxide, potassium hydroxide, etc.), alkali metal carbonate (e.g. sodium carbonate, potassium carbonate, etc.), alkali metal bicarbonate (e.g. sodium bicarbonate, potassium bicarbonate, etc.), alkali metal alkoxide (e.g. sodium methoxide, sodium ethoxide, potassium tert-butoxide, etc.), alkali metal alkanoic acid (e.g. sodium acetate, etc.), trialkylamine (e.g. triethylamine, etc.), pyridine compound (e.g. pyridine, lutidine, picoline, 4-dimethylaminopyridine, etc.), quinoline, and the like.

In case that the acylating agent is used in a free form or its salt in this reaction, the reaction is preferably carried out in the presence of a condensing agent such as a carbodiimide compound [e.g. N,N'-dicyclohexylcarbodiimide, N-cyclohexyl-N'-(4-diethylaminocyclohexyl)carbodiimide, N,N'-diethylcarbodiimide, N,N'-diisopropylcarbodiimide, N-ethyl-N'-(3-dimethylaminopropyl)carbodiimide, etc.], a ketenimine compound (e.g. N,N'-carbonylbis(2-methylimidazole), pentamethyleneketene-N-cyclohexylimine, diphenylketene-N-cyclohexylimine, etc.); an olefinic or acetylenic ether compounds (e.g. ethoxyacetylene, β-chlorovinylethyl ether), a sulfonic acid ester of N-hydroxybenzotriazole derivative [e.g. 1-(4-chlorobenzenesulfonyloxy)-6-chloro-1H-benzotriazole, etc.], a combination of trialkylphosphite or triphenylphosphine and carbon tetrachloride, disulfide or diazenedicarboxylate (e.g. diethyl diazenedicarboxylate, etc.), a phosphorus compound (e.g. ethyl polyphosphate, isopropyl polyphosphate, phosphoryl chloride, phosphorus trichloride, etc.), thionyl chloride, oxalyl chloride, N-ethylbenzisoxazoliun salt, N-ethyl-5-phenylisoxazolium-3-sulfonate, a reagent (referred to a so-called "Vilsmeier reagent") formed by the reaction of an amide compound such as N,N-di (lower)alkylformamide (e.g. dimethylformamide, etc.), N-methylformamide or the like with a halogen compound such as thionyl chloride, phosphoryl chloride, phosgene or the like, and the like.

The reaction is usually carried out in a conventional solvent which does not adversely influence the reaction such as water, acetone, dichloromethane, alcohol (e.g. methanol, ethanol, etc.), tetrahydrofuran, pyridine, N,N-dimethylformamide, etc., or a mixture thereof.

The reaction temperature is not critical and the reaction is usually carried out under from cooling to heating.

Process 14

The compound (I-r) or a salt thereof can be prepared by subjecting the compound (I-t) or a salt thereof to a removal reaction of the amino-protective group.

Suitable salts of the compound (I-t) may be the same as those for the compound (I).

The reaction of this process can be carried out in a manner similar to that in Process 1.

Process 15

The compound (I-v) or a salt thereof can be prepared by subjecting the compound (I-u) or a salt thereof to a removal reaction of the hydroxy-protective group.

Suitable salts of the compounds (I-u) and (I-v) may be the same as those for the compound (I).

The reaction of this process can be carried out in a manner similar to that in Process 1.

Process 16

The compound (I-x) or a salt thereof can be prepared by oxidating the compound (I-w) or a salt thereof.

Suitable salts of the compounds (I-w) and (I-x) may be the same as those for the compound (I).

The reaction of this process can be carried out in a manner similar to that in Process 8.

Process 17

The compound (I-z) or a salt thereof can be prepared by reducing the compound (I-y) or a salt thereof.

Suitable salts of the compounds (I-y) and (I-z) may be the same as those for the compound (I).

The reaction of this process can be carried out in a manner similar to that in Process 3.

Process 18

The compound (I-ab) or a salt thereof can be prepared by oxidating the compound (I-aa) or a salt thereof.

Suitable salts of the compounds (I-aa) and (I-ab) may be the same as those for the compound (I).

The reaction of this process can be carried out in a manner similar to that in Process 2.

Process 19

The compound (I-ac) or a salt thereof can be prepared by acylating the compound (I-v) or a salt thereof.
Suitable salts of the compound (I-ac) may be the same as those for the compound (I).
The reaction of this process can be carried out in a manner similar to that in Process 13.

Process 20

The compound (I-ad) or a salt thereof can be prepared by reacting the compound (XI) or a salt thereof with the compound (XII) or a salt thereof.
Suitable salts of the compounds (I-ad), (XI) and (XII) may be the same as those for the compound (I).
The reaction of this process can be carried lout in a manner similar to that in Process 9.

Process 21

The compound (I-p) or a salt thereof can be prepared by subjecting the compound (I-ae) or a salt thereof to a removal reaction of the carboxy-protective group.
Suitable salts of the compound (I-ae) may be the same as those for the compound (I).
The reaction.of this process can be carried out in a manner similar to that in Process 1.

Process 22

The compound (I-ag) or a salt thereof can be prepared by reacting the compound (I-af) or a salt thereof with a substituted amine.
Suitable salts of the compounds (I-af) and (I-ag) may be the same as those for the compound (I).
The reaction of this process can be carried out in a manner similar to that in Process 4.
The compounds obtained above can be isolated and purified by a conventional method such as pulverization, recrystallization, column chromatography, reprecipitation and the like.
The object compounds can be transformed into their salts in a conventional manner.
It is to be noted that the object compounds may include one or more stereoisomers or optical isomers due to asymmetric carbon atoms, and all of such isomers and mixture thereof are included within the scope of this invention.
Collagenases initiate the degradation of collagen in vertebrates and, in addition to their normal function in the metabolism of connective tissue and wound healing, they have been implicated to be involved in a number of pathological conditions such as joint destruction in rheumatoid arthritis, periodontal disease, corneal ulceration, tumor metastasis, osteoarthritis, decubitus, restenosis after percutaneous transluminal coronary angioplasty, osteoporosis, psoriasis, chronic active hepatitis, autoimmune keratitis, and the like, and therefore the compounds of the present invention are useful for the preparation of medicaments for treating and/or preventing such pathological conditions.
Inhibitory activity of MMP can be assayed by a conventional test method as mentioned below.

Test Methods:

Test Method 1:

Inhibitory activity of human MMP-1

Human collagenase was prepared from the culture medium of human skin fibroblast stimulated with interleukin-1β (1 ng/ml). Latent collagenase was activated by incubation with trypsin (200 μg/ml) at 37°C for 60 minutes and the reaction was stopped by adding soybean trypsin inhibitor (800 μg/ml). Collagenase activity was determined using FITC-labeled calf skin type I collagen. FITC-collagen (2.5 mg/ml) was incubated at 37°C for 120 minutes with the activated collagenase and test compound in 50 mM Tris buffer (containing 5 mM $CaCl_2$, 200 mM NaCl and 0.02% $NaN_3$, pH 7.5). After stopping the enzyme reaction by adding the equal volume of 70% ethanol-200 mM Tris buffer (pH 9.5), the reaction mixture was centrifuged, and collagenase activity was estimated by measuring the fluorescence intensity of supernatant at 495 nm (excitation) and 520 nm (emission).

Test Method 2:

Inhibitory activity of human MMP-9

The inhibitory activity of test compounds against human MMP-9 were measured by using commercial kits (Yagai, Japan). Gelatinolytic activity was determined by monitoring the degradation of FITC-labeled bovine type IV collagen after incubation for 4 hours at 42°C. The amount of degraded collagen was estimated by measuring the fluorescence intensity at 495 nm (excitation) and 520 nm (emission).

Test Method 3:

Inhibitory activity of human MMP-13

The inhibitory potential of test compounds against human MMP-13 were assayed by using commercial kit (Chondrex, USA) contained truncated form of human recombinant MMP-13 and fluorogenic peptide substrate. Activity of human MMP-13 was determined by monitoring the degradation of fluorogenic peptide substrate after incubation for 1 hour at 35°C and estimated by measuring the fluorescence intensity of degraded peptide substrate at 495 nm (excitation) and 520 nm (emission).

Test Method 4:

Inhibitory activity of human MMP-8

The inhibitory potential of test compounds against human MMP-8 were assayed by using commercial kit (Chondrex, USA) contained recombinant human pro-MMP-8 and FITC-labeled telopeptide-free soluble bovine type I collagen as a substrate. Recombinant human pro-MMP-8 was activated by a sequential incubation with mercury compound and proteinase at 35°C for 1 hour. Reaction mixture containing the activated MMP-8, substrate and test compounds were incubated at 35°C for 2 hours. After stopping the enzyme reaction by adding the stop solution (o-phenathroline), the reaction mixture was centrifuged and MMP-8 activity was estimated by measuring the fluorescence intensity of supernatant at 490 nm (excitation) and 520 nm (emission).

For therapeutic purposes, the compounds and pharmaceutically acceptable salts thereof of the present invention can be used in the form of a pharmaceutical preparation containing, as an active ingredient, one of said compounds in admixture with a pharmaceutically acceptable carrier such as an organic or inorganic solid or liquid excipient suitable for oral, parenteral or external administration. The pharmaceutical preparations may be capsules, tablets, dragees, granules, solutions, suspensions, emulsions, sublingual tablets, suppositories, ointments, and the like. If desired, there may be included, in these preparations, auxiliary substances, stabilizing agents, wetting agents, emulsifying agents, buffers and other commonly used additives.

While the dose of the compound will vary depending upon the age and condition of patient and the like, in the case of intravenous administration, a daily dose of 0.01 - 100 mg of the active ingredient per kg weight of a human being, and in the case of intramuscular administration, a daily dose of 0.05 - 100 mg of the same per kg weight of a human being, or in the case of oral administration, a daily dose of 0.1 - 100 mg of the same per kg weight of a human being, is generally given for the treatment of MMP or TNFα-mediated diseases.

In order to illustrate the usefulness of the object compound, the pharmacological test data of a representative compound of the compounds are shown in the following.

Inhibitory activity of MMP

1. Test Method

Inhibitory activity of human MMP-13 as mentioned above.

2. Test Compound

Compound of Example 15

3. Test Resut

| Test Compound | Inhibitory activity [$IC_{50}$ (nM)] |
|---|---|
| Example 15 | 2.2 |

The following Preparations and Examples are given for the purpose of illustrating the present invention in detail.

The abbreviations used in this description are, for examplee, as follows.

Aib: aminoisobutyric acid
Abu: aminobutyric acid
4PyAla: 4-pyridylalanine

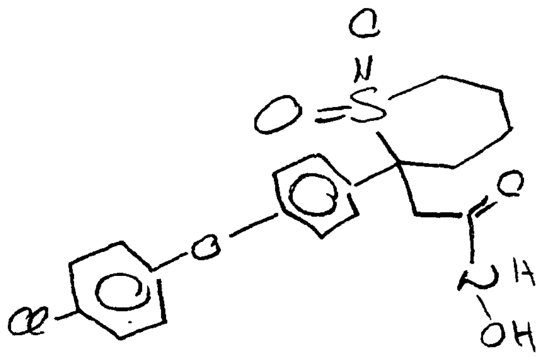

Preparation 1-1)

N-Chlorosuccinimide (2.67 g) was added gradually over 30 minutes to a stirred solution of tetrahydro-2H-thiopyran (2.04 g) in benzene (20 ml). The temperature was maintained at 20-30°C by intermittent external cooling. The mixture was stirred for 1 hour and rapidly filtered to remove succinimide. The filtrate was added to a solution of 4-anisylmagnesium bromide in diethyl ether which was prepared from 4-anisyl bromide (7.48 g) and magnesium turnings (0.875 g) in diethyl ether (36 ml) in a usual manner. The rate of addition was such that the temperature of the reaction was maintained between 10-15°C. The resultant mixture was stirred at room temperature for 17 hours and decomposed by the addition of ice and a 20% aqueous solution of sulfuric acid. The organic layer was separated, washed twice with water, once with 1N sodium hydroxide solution, twice with water, then once with brine, and dried over magnesium sulfate. Removal of the solvent, followed by washing with methanol, gave 3,4,5,6-tetrahydro-2-(4-methoxyphenyl)-2H-thiopyran (1.22 g) as a colorless powder.

mp: 82-85°C

IR (KBr): 1610, 1514, 1252 $cm^{-1}$

NMR (DMSO-$d_6$, δ) : 1.35-1.65 (2H, m), 1.7-2.1 (4H, m), 2.56-2.64 (1H, m), 2.73-2.86 (1H, m), 3.72 (3H, s), 3.84 (1H, dd, J=11.0, 2.7Hz), 6.87 (2H, d, J=8.7Hz), 7.24 (2H, d, J=8.7Hz)

| | | |
|---|---|---|
| Anal. Calcd. for $C_{12}H_{16}OS$ | C 69.19, | H 7.74 |
| Found | C 69.59, | H 7.68 |

Preparation 1-2)

1.0M Solution of boron tribromide in dichloromethane (8.27 ml) was added dropwise to a stirred solution of 3,4,5,6-tetrahydro-2-(4-methoxyphenyl)-2H-thiopyran (718 mg) in dichloromethane (10 ml) under ice cooling and the resulting mixture was stirred for 3 hours while the temperature was allowed to rise to room temperature. The reaction mixture was evaporated in vacuo and the residue was partitioned between ethyl acetate and water. The organic layer

was separated, washed with brine, dried over sodium sulfate, and evaporated in vacuo. The residue was chromatographed (eluent: toluene - ethyl acetate) over silica gel (15.4 g) to afford 3,4,5,6-tetrahydro-2-(4-hydroxyphenyl)-2H-thiopyran (600 mg) as a colorless powder.

mp: 138-140.5°C

IR (KBr): 3421 (br), 1241 $cm^{-1}$

NMR (DMSO-$d_6$, δ): 1.35-1.65 (2H, m), 1.7-2.05 (4H, m), 2.58 (1H, br d, J=13.3Hz), 2.71-2.85 (1H, m), 3.78 (1H, dd, J=10.8, 2.5Hz), 6.68 (2H, d, J=8.5Hz), 7.11 (2H, d, J=8.5Hz), 9.32 (1H, s)

Preparation 1-3)

A mixture of 3,4,5,6-tetrahydro-2-(4-hydroxyphenyl)-2H-thiopyran (578 mg), 4-bromochlorobenzene (683 mg), 8-hydroxyquinoline (17.3 mg), potassium carbonate (247 mg), and copper (I) chloride (11.8 mg) in 1,3-dimethyl-2-imidazolidinone (1.73 g) was stirred at 150°C under a nitrogen atmosphere for 21 hours and cooled to room temperature. The reaction mixture was partitioned between ethyl acetate and water. The organic layer was separated, washed with a 1N aqueous solution of sodium hydroxide and brine, dried over magnesium sulfate, and evaporated in vacuo. The residue was chromatographed (eluent: n-hexane-toluene) over silica gel to afford 2-[4-(4-chlorophenoxy) phenyl]-3,4,5,6-tetrahydro-2H-thiopyran (417 mg) as a colorless powder.

mp: 69.5-70.5°C

IR (KBr): 1274 $cm^{-1}$

NMR ($CDCl_3$, δ): 1.45-1.77 (2H, m), 1.83-2.2 (4H, m), 2.66 (1H, m), 2.81-2.95 (1H, m), 3.84 (1H, dd, J=11.6, 2.6Hz), 6.88-6.97 (4H, m), 7.23-7.36 (4H, m)

Preparation 1-4)

An aqueous solution (23 ml) of OXONE ($2KHSO_5 \cdot KHSO_4 \cdot K_2SO_4$, 1.82 g) was added dropwise to a suspension of 2-[4-(4-chlorophenoxy)phenyl]-3,4,5,6-tetrahydro-2H-thiopyran (600 mg) in methanol (23 ml) under ice cooling and the resulting mixture was stirred at room temperature for 17 hours. The reaction mixture was mixed with an aqueous solution (10 ml) of sodium sulfite (746 mg) at room temperature, stirred at the same temperature for a while, and concentrated in vacuo. The residue was partitioned between ethyl acetate and water. The organic layer was separated, washed with brine, dried over sodium sulfate, and evaporated in vacuo. The residue was washed with n-hexane to afford 2-(4-(4-chlorophenoxy)-phenyl]-3,4,5,6-tetrahydro-2H-thiopyran 1,1-dioxide (636 mg) as a colorless powder.

mp: 151.5-152°C

IR (KBr): 1313, 1247, 1120 $cm^{-1}$

NMR ($CDCl_3$, δ): 1.65 (1H, m), 2.04-2.26 (4H, m), 2.37-2.56 (1H, m), 2.96-3.13 (1H, m), 3.24 (1H, m), 4.01 (1H, dd, J=12.8, 3.1Hz), 6.92-7.03 (4H, m), 7.28-7.42 (4H, m)

(+) APCI MS m/z: 336 ($M^+$+H)

Preparation 1-5)

1.5M Solution of lithium diisopropylamide mono tetrahydrofuran in cyclohexane (1.47 ml) was added dropwise to a stirred suspension of 2-[4-(4-chlorophenoxy)phenyl]-3,4,5,6-tetrahydro-2H-thiopyran 1,1-dioxide (621 mg) in tetrahydrofuran (9 ml) under a nitrogen atmosphere and dry ice - acetone cooling and the resultant suspension was stirred under the same conditions for 25 minutes. A solution of allyl bromide (491 mg) in tetrahydrofuran (2.5 ml) was added dropwise therein and the resultant mixture was stirred under the same conditions for 2 hours and 30 minutes. After addition of a saturated aqueous solution of ammonium chloride under the same conditions, the reaction mixture was extracted with ethyl acetate. The extract was washed successively with 1N hydrochloric acid, brine, and a 'saturated aqueous solution of sodium bicarbonate, dried over magnesium sulfate, and evaporated in vacuo. The residue was chromatographed (eluent: toluene - ethyl acetate) over silica gel to afford 2-allyl-2-[4-(4-chlorophenoxy)phenyl]-3,4,5,6-tetrahydro-2H-thiopyran 1,1-dioxide (419 mg) as a colorless oil.

IR (Film): 1639, 1311, 1243, 1126 $cm^{-1}$

NMR ($CDCl_3$, δ): 1.81-1.86 (2H, m), 2.09-2.21 (3H, m), 2.53-2.68 (1H, m), 2.86-3.09 (2H, m), 3.17-3.35 (2H, m), 5.02-5.09 (1H, m), 5.14-5.31 (2H, m), 6.94-7.05 (4H, m), 7.31 (2H, d, J=9.0Hz), 7.61 (2H, d, J=9.0Hz)

(+) API-ES MS m/z: 399 and 401 ($M^+$+Na)

Preparation 1-6)

1.5M Solution of lithium diisopropylamide mono tetrahydrofuran in cyclohexane (0.34 ml) was added dropwise to a stirred solution of 2-allyl-2-[4-(4-chlorophenoxy)phenyl]-3,4,5,6-tetrahydro-2H-thiopyran 1,1-dioxide (162 mg) in

tetrahydrofuran (2.5 ml) under a nitrogen atmosphere and dry ice - acetone cooling and the resultant solution was stirred under the same conditions for 35 minutes. A solution of methyl iodide (134 mg) in tetrahydrofuran (0.5 ml) was added dropwise therein and the resultant mixture was stirred under the same condition for 1 hour and 20 minutes. After addition of a saturated aqueous solution of ammonium chloride under the same conditions, the reaction mixture was extracted with ethyl acetate. The extract was washed successively with 1N hydrochloric acid and brine, dried over sodium sulfate, and evaporated in vacuo. The residue was chromatographed (eluent: toluene - ethyl acetate) over silica gel (8.1 g) to afford 2-allyl-2-[4-(4-chlorophenoxy)phenyl]-3,4,5,6-tetrahydro-6-methyl-2H-thiopyran 1,1-dioxide (78 mg) as a paste.

IR (KBr): 1639, 1284, 1244, 1126 $cm^{-1}$

NMR ($CDCl_3$, δ): 1.38 (3H, d, J=6.7Hz), 1.82-2.25 (5H, m), 2.61 (1H, m), 2.99 (1H, dd,=14.3, 7.7Hz), 3.32-3.39 (2H, m), 5.02-5.08 (1H, m), 5.16-5.29 (2H, m), 6.95-7.03 (4H, m), 7.28-7.33 (2H, m), 7.57-7.64 (2H, m)

(+) APCI MS m/z: 391 and 393 ($M^+$+H)

Preparation 2

2-[4-(4-Chlorophenoxy)phenyl]-3,4,5,6-tetrahydro-2H-thiopyran (148 mg) was prepared from 3,4,5,6-tetrahydro-2-(4-hydroxyphenyl)-2H-thiopyran (292 mg) and 4-chloroiodobenzene (430 mg) in a similar manner to that of Preparation 1-3).

mp: 69.5-70.5°C

Preparation 3

2-[4-(4-Chlorophenoxy)phenyl]-3,4,5,6-tetrahydro-2H-thiopyran (97 mg) was prepared from tetrahydro-2H-thiopyran (510 mg) and 4-bromo-4'-chlorodiphenyl ether (2.12 g) in a similar manner to that of Preparation 1-1).

mp: 69.5-70.5°C

Preparation 4-1)

5-Chlorovaleryl chloride (17.1 g) was added dropwise to a stirred suspension of aluminum chloride (14.7 g) in dichloromethane (125 ml) under a nitrogen atmosphere and ice cooling over 5 minutes and the resulting solution was stirred under the same conditions for 10 minutes, then therein a solution of 4-chlorodiphenyl ether (20.5 g) in dichloromethane (115 ml) was added dropwise over 20 minutes. The resulting mixture was stirred under the same conditions for 1 hour and 15 minutes, and poured into a mixture of 7% hydrochloric acid and ice. The organic layer was separated, washed with brine, dried over sodium sulfate, and evaporated in vacuo. The oily residue was powdered from n-hexane to afford 4-(5-chlorovaleryl)-4'-chlorodiphenyl ether (30.9 g) as a colorless powder.

mp: 59.5-60.5°C

IR (KBr): 1672, 1250 $cm^{-1}$

NMR ($CDCl_3$, δ): 1.85-1.91 (4H, m), 2.94-3.02 (2H, m), 3.55-3.62 (2H, m), 6.96-7.05 (4H, m), 7.36 (2H, d, J=9.0Hz), 7.95 (2H, d, J=8.9Hz)

(+) API-ES MS m/z: 345, 347 and 349 ($M^+$+Na)

Preparation 4-2)

A solution of sodium borohydride (2.16 g) in water (59 ml) was added dropwise to a stirred suspension of 4-(5-chlorovaleryl)-4'-chlorodiphenyl ether (30.8 g) and sodium bicarbonate (9.61 g) in ethanol (480 ml) under a nitrogen atmosphere at room temperature over 10 minutes and the resulting mixture was stirred under the same conditions for 3 hours. After removal of ethanol, the reaction mixture was acidified with 3N hydrochloric acid (70 ml) and extracted with toluene. The extract was washed successively with water, a saturated aqueous solution of sodium bicarbonate, and brine, dried over sodium sulfate, and evaporated in vacuo to afford 4-(5-chloro-1-hydroxypentyl)-4'-chlorodiphenyl ether (31.1 g) as a yellow oil.

IR (Film): 3383 (br), 1242 $cm^{-1}$

NMR ($CDCl_3$, δ): 1.43-1.89 (7H, m), 3.53 (2H, t, J=6.6Hz), 4.67 (1H, m), 6.89-7.01 (4H, m), 7.24-7.34 (4H, m)

(+) API-ES MS m/z: 347, 349 and 351 ($M^+$+Na), 311 and 313 ($M^+$-HCl+Na)

Preparation 4-3)

Thionyl chloride (31.2 g) was added dropwise to a stirred solution of 4-(5-chloro-1-hydroxypentyl)-4'-chlorodiphenyl ether (31.0 g) in chloroform (383 ml) under ice cooling and the resulting solution was stirred under reflux

for 4 hours. The reaction mixture was cooled to room temperature and evaporated in vacuo. The residue was partitioned between toluene and water. The organic layer was separated, washed with a saturated aqueous solution of sodium bicarbonate (twice) and brine, dried over sodium sulfate, and evaporated in vacuo to afford 4-chloro-4'-(1,5-dichloropentyl)diphenyl ether (33.7 g) as a pale brown oil.

IR (Film): 1242 $cm^{-1}$

NMR ($CDCl_3$, δ): 1.47-1.85 (4H, m), 2.02-2.19 (2H, m), 3.53 (2H, t, J=6.5Hz), 4.85 (1H, dd, J=7.9, 6.6Hz), 6.91-7.00 (4H, m), 7.27-7.38 (4H, m)

Preparation 4-4)

Sodium sulfide nonahydrate (21.8 g) was added gradually to a stirred solution of 4-chloro-4'-(1,5-dichloropentyl)-diphenyl ether (24.0 g) in N,N-dimethylformamide (DMF, 240 ml) under ice cooling and a nitrogen atmosphere, and the resulting mixture was stirred under the same conditions for 2 hours and at room temperature for 3 days, then the reaction mixture was filtered. The filtrate was concentrated in vacuo and partitioned between water and toluene. The organic layer was separated, washed twice with brine, dried over magnesium sulfate, and evaporated in vacuo. The residue was chromatographed (eluent: n-hexane - toluene) over silica gel to afford 2-[4-(4-chlorophenoxy)phenyl]-3,4,5,6-tetrahydro-2H-thiopyran (13.8 g) as a colorless powder.

mp: 69.5-70.5°C

Preparation 5-1)

5-(4-Chlorophenyl)-2-(5-chlorovaleryl)thiophene (3.75 g) was obtained in a similar manner to that of Preparation 4-1).

NMR ($CDCl_3$, δ): 1.86-1.95 (4H, m), 2.95 (2H, dd, J=6.9, 6.9Hz), 3.59 (2H, dd, J=6.9, 6.9Hz), 7.30 (1H, d, J=3.9Hz), 7.39 (2H, d, J=8.4Hz), 7.58 (2H, d, J=8.4Hz), 7.67 (1H, d, J=3.9Hz)

Preparation 5-2)

Ethyl 7-chloro-3-[5-(4-chlorophenyl)-2-thienyl]hept-2-enoate (4.2 g) was obtained in a similar manner to that of Preparation 8-2).

NMR ($CDCl_3$, δ): 1.21-1.34 (3H, m), 1.61-1.93 (4H, m), 2.53-2.57 (1H, m), 3.06-3.11 (1H, m), 3.52-3.60 (2H, m), 4.12-4.23 (2H, m), 5.88 (0.5H, s), 6.23 (0.5H, s), 7.21-7.34 (4H, m), 7.51-7.53 (2H, m)

Preparation 6-1)

Potassium tert-butoxide (1.34 g) was gradually added to a stirred solution of 4-chloro-4'-(1,5-dichloropentyl)-diphenyl ether (3.44 g) and thiobenzoic acid (1.66 g) in N,N-dimethylformamide (48 ml) under ice cooling and a nitrogen atmosphere over 5 minutes, and the resulting mixture was stirred at the same temperature for 2 hours and at room temperature for 16 hours. The reaction mixture was partitioned between ethyl acetate and an aqueous solution of sodium bicarbonate. The organic layer was separated, washed with a saturated aqueous solution of sodium bicarbonate and brine, dried over magnesium sulfate, and evaporated in vacuo. The oily residue (4.63 g) was chromatographed (eluent: n-hexane - toluene) over silica gel to afford 4-(1-benzoylthio-5-chloropentyl)-4'-chlorodiphenyl ether (1.16 g) as a pink oil.

IR (Film): 1660, 1242 $cm^{-1}$

NMR ($CDCl_3$, δ): 1.36-1.65 (2H, m), 1.75-1.90 (2H, m), 1.99-2.12 (2H, m), 3.52 (2H, t, J=6.6Hz), 4.77 (1H, t, J=7.8Hz), 6.90-6.98 (4H, m), 7.25-7.57 (7H, m), 7.90-7.96 (2H, m)

(+) API-ES MS m/z: 467, 469 and 471 ($M^+$+Na)

Preparation 6-2)

28% Solution of sodium methoxide in methanol (96.5 mg) was added dropwise to a stirred solution of 4-(1-benzoylthio-5-chloropentyl)-4'-chlorodiphenyl ether (223 mg) in methanol (1.1 ml) and acetonitrile (1.1 ml) under ice cooling and the resulting mixture was stirred at the same temperature for 2 hours, then additional 28% solution of sodium methoxide in methanol (96.5 mg), methanol (1.0 ml) and sodium iodide (7.5 mg) were added therein and the mixture was stirred at room temperature for 15 hours. The reaction mixture was acidified with 3N hydrochloric acid (0.5 ml) under ice cooling. The acidic mixture was extracted with toluene. The extract was washed with water and brine, dried over magnesium sulfate, and evaporated in vacuo. The residue (197 mg) was chromatographed (eluent: n-hexane - toluene) over silica gel (3.9 g) to afford 2-[4-(4-chlorophenoxy)phenyl]-3,4,5,6-tetrahydro-2H-thiopyran (114 mg) as a

colorless powder.
mp: 69.5-70.5°C

Preparation 7-1)

4-(4-Chlorobutyryl)-4'-chlorodiphenyl ether (6.12 g) was prepared from 4-chlorobutyryl chloride (3.10 g) and 4-chlorodiphenyl ether (4.09 g) in a similar manner to that of Preparation 4-1).
IR (Film): 1680, 1250 $cm^{-1}$
NMR ($CDCl_3$, δ): 2.22 (2H, m), 3.14 (2H, t, J=7.0Hz), 3.68 (2H, t, J=6.2Hz), 6.96-7.05 (4H, m), 7.31-7.40 (2H, m), 7.93-8.01 (2H, m)

Preparation 7-2)

4-(4-Chloro-1-hydroxybutyl)-4'-chlorodiphenyl ether (6.14 g) was prepared in a similar manner to that of Preparation 4-2).
NMR ($CDCl_3$, δ): 1.76-2.01 (4H, m), 3.51-3.64 (2H, m), 4.70 (1H, m), 6.90-7.00 (4H, m), 7.24-7.34 (4H, m)
(+) API-ES MS m/z: 333, 335 and 337 ($M^+$+Na)

Preparation 7-3)

4-Chloro-4'-(1,4-dichlorobutyl)diphenyl ether (5.75 g) was prepared in a similar manner to that of Preparation 4-3).
IR (Film): 1244 $cm^{-1}$
NMR ($CDCl_3$, δ): 1.72-2.30 (4H, m), 3.57 (2H, t, J=6.4Hz), 4.88 (1H, t, J=7.2Hz), 6.89-7.00 (4H, m), 7.24-7.39 (4H, m)
(+) API-ES MS m/z: 293 and 295 ($M^+$-Cl)

Preparation 7-4)

2-[4-(4-Chlorophenoxy)phenyl]-2,3,4,5-tetrahydrothiophene (3.63 g) was prepared in a similar manner to that of Preparation 4-4).
IR (Film): 1238 $cm^{-1}$
NMR ($CDCl_3$, δ): 1.87-2.02 (2H, m), 2.23-2.44 (2H, m), 2.99-3.17 (2H, m), 4.50 (1H, dd, J=8.4, 6.0Hz), 6.88-6.97 (4H, m), 7.23-7.31 (2H, m), 7.38 (2H, d, J=8.5Hz)

Preparation 7-5)

2-[4-(4-Chlorophenoxy)phenyl]-2,3,4,5-tetrahydrothiophene 1,1-dioxide (3.05 g) was prepared in a similar manner to that of Preparation 1-4).
mp: 74.5-78.5°C
IR (Film): 1315, 1234, 1169, 1126 $cm^{-1}$
NMR ($CDCl_3$, δ): 2.18-2.55 (4H, m), 3.12-3.36 (2H, m), 4.14 (1H, dd, J=11.7, 7.3Hz), 6.92-7.05 (4H, m), 7.28-7.39 (4H, m)
(+) APCI MS m/z: 323 and 325 ($M^+$+H)

Preparation 8-1)

To a suspension of aluminum chloride (3.58 g) in methylene chloride (20 ml) was added a solution of 5-chlorovaleryl chloride (4.17 g) in methylene chloride (5 ml) dropwise at 0°C. After being stirred for 30 minutes at the same temperature, a solution of 4-chlorodiphenyl ether (6 g) in methylene chloride (5 ml) was added therein and the mixture was stirred under ice-bath cooling for 2 hours. After 4N hydrochloric acid was added carefully to decompose excess aluminum chloride, the organic layer was separated and the aqueous layer was extracted with chloroform (20 ml x 2). The combined organic layer was washed with water and brine, and concentrated under reduced pressure. The resulting residue was washed with hexane to give 4-(5-chlorovaleryl)-4'-chlorodiphenyl ether (6.89 g) as a slightly yellow solid.
NMR (DMSO-$d_6$, δ): 1.84-1.95 (4H, m), 2.99 (2H, t, J=7Hz), 3.42 (2H, t, J=7Hz), 6.99 (2H, d, J=9Hz), 7.00 (2H, d, J=9Hz), 7.37 (2H, d, J=9Hz), 7.96 (2H, d, J=9Hz)

Preparation 8-2)

To a suspension of sodium hydride (60% oil dispersion, 3.14 g) in tetrahydrofuran (160 ml) was added a solution of triethyl phosphonoacetate (5.23 ml) in tetrahydrofuran (20 ml) at 0°C. After being stirred 30 minutes at the same temperature, a solution of 4-(5-chlorovaleryl)-4'-chlorodiphenyl ether (48 g) in tetrahydrofuran (60 ml) was added therein, and the reaction mixture was refluxed overnight. The mixture was cooled to room temperature, poured into water, and concentrated under reduced pressure. The residue was extracted with ethyl acetate (200 ml x 3). The combined extract was washed with brine, dried over magnesium sulfate and concentrated to give ethyl 7-chloro-3-[4-(4-chlorophenoxy)phenyl]hept-2-enoate (E:Z = 1:1 mixture) (67.4 g) as a yellow oil.

NMR ($CDCl_3$, δ): 1.14 (1.5H, t, J=7Hz), 1.26 (1.5H, t, J=7Hz), 1.29-1.40 (2H, m), 1.50-1.67 (2H, m), 1.74-1.89 (2H, m), 2.45 (1.5H, t, J=7Hz), 3.09 (1.5H, t, J=7Hz), 3.51 (1H, t, J=7Hz), 3.53 (1H, t, J=7Hz), 4.03 (1H, q, J=7Hz), 4.14 (1H, q, J=7Hz), 5.89 (0.5H, s), 6.04 (0.5H, s), 6.90-7.01 (4H, m), 7.14 (2H, d, J=8Hz), 7.28 (1H, d, J=8Hz), 7.30 (1H, d, J=8Hz), 7.42 (2H, d, J=8Hz)

Preparation 8-3)

A solution of sodium iodide (128 g) and ethyl 7-chloro-3-[4-(4-chlorophenoxy)phenyl]hept-2-enoate (67.4 g) in acetone (200 ml) was refluxed for 24 hours. The resulting mixture was poured into water (300 ml) and extract with ethyl acetate (100 ml x 2). The combined organic layer was washed with water and brine, and dried over magnesium sulfate to give ethyl 3-[4-(4-chlorophenoxy)phenyl]-7-iodo-hept-2-enoate (67.8 g) (E:Z = 1:1 mixture) as a yellow oil.

NMR ($CDCl_3$, δ): 1.14 (1.5H, t, J=7Hz), 1.26 (1.5H, t, J=7Hz), 1.29-1.37 (2H, m), 1.46-1.62 (2H, m), 1.78-1.94 (2H, m), 2.44 (1.5H, t, J=7Hz), 3.12 (1.5H, t, J=7Hz), 3.18 (3H, t, J=7Hz), 4.04 (1H, q, J=7Hz), 4.20 (1H, q, J=7Hz), 5.88 (0.5H, s), 6.04 (0.5H, s), 6.89-7.00 (4H, m), 7.14 (2H, d, J=8Hz), 7.26-7.36 (3H, m), 7.42 (2H, d, J=8Hz)

Preparation 8-4)

A mixture of ethyl 3-[4-(4-chlorophenoxy)phenyl)-7-iodo-hept-2-enoate (59.8 g) and thiourea (9.39 g) in ethanol (123 ml) was refluxed for 24 hours. The resulting mixture was cooled and evaporated to give ethyl 7-amidinothio-3-[4-(4-chlorophenoxy)phenyl]hept-2-enoate hydroiodide (70.2 g) (E:Z = 1:1 mixture) as slightly yellow oil.

NMR (DMSO-$d_6$, δ): 1.05 (1.5H, t, J=7Hz), 1.24 (1.5H, t, J=7Hz), 1.33-1.68 (4H, m), 2.48 (1H, t, J=7Hz), 3.05-3.16 (3H, m), 3.96 (1H, q, J=7Hz), 4.12 (1H, q, J=7Hz), 5.93 (0.5H, s), 6.07 (0.5H, s), 6.96-7.13 (4H, m), 7.20 (1H, d, J=8Hz), 7.46 (1H, d, J=8Hz), 7.48 (1H, d, J=8Hz), 7.60 (1H, d, J=8Hz)

MS (ES-) m/z: 433 (M-H)

Preparation 9-1)

4-(5-Chlorovaleryl)-4'-fluorodiphenyl ether (3.86 g) was obtained in a similar manner to that of Preparation 4-1).

NMR ($CDCl_3$, δ): 1.81-1.92 (4H, m), 2.97 (2H, dd, J=7, 7Hz), 3.53-3.6 (2H, m), 6.94-7.12 (6H, m), 7.92-7.95 (2H, m)

Preparation 9-2)

Ethyl 7-chloro-3-[4-(4-fluorophenoxy)phenyl]hept-2-enoate (1.90 g) was obtained in a similar manner to that of Preparation 8-2).

NMR ($CDCl_3$, δ): 1.31 (3H, dd, J=7, 7Hz), 1.54-1.64 (2H, m), 1.78-1.88 (2H, m), 3.12 (2H, dd, J=7.5, 7.5Hz), 3.53 (2H, dd, J=7, 7Hz), 4.20 (2H, ddd, J=7, 7, 7Hz), 6.03 (1H, s), 6.93-7.09 (6H, m), 7.39-7.42 (2H, m)

Preparation 10-1)

4-(5-Chlorovaleryl)-4'-bromodiphenyl ether (6.71 g) was obtained in a similar manner to that of Preparation 4-1).

NMR ($CDCl_3$, δ): 1.82-1.94 (4H, m), 2.99 (2H, t, J=6.5Hz), 3.60 (2H, t, J=6.5Hz), 6.95 (2H, d, J=9Hz), 7.00 (2H, d, J=9Hz), 7.51 (2H, d, J=9Hz), 7.95 (2H, d, J=9Hz)

Preparation 10-2

Ethyl 3-[4-(4-bromophenoxy)phenyl]-7-chlorohept-2-enoate (7.55 g) was obtained in a similar manner to that

of Preparation 8-2).

NMR ($CDCl_3$, δ): 1.14 (1.5H, t, J=7Hz), 1.21-1.39 (1.5H, m), 1.49-1.65 (2H, m), 1.74-1.88 (2H, m), 2.47 (1H, t, J=7Hz), 3.12 (1H, t, J=7Hz), 3.47-3.56 (2H, m), 4.03 (1H, q, J=7Hz), 4.20 (1H, q, J=7Hz), 5.89 (0.5H, s), 6.04 (0.5H, s), 6.86-6.99 (4H, m), 7.39-7.50 (4H, m)

Preparation 10-3)

Ethyl 3-[4-(4-bromophenoxy)phenyl]-7-iodohept-2-enoate (7.85 g) was obtained in a similar manner to that of Preparation 8-3).

NMR ($CDCl_3$, δ): 1.14 (1.5H, t, J=7Hz), 1.21-1.39 (1.5H, m), 1.49-1.65 (2H, m), 1.74-1.88 (2H, m), 2.47 (1H, t, J=7Hz), 3.12 (1H, t, J=7Hz), 3.47-3.56 (2H, m), 4.03 (1H, q, J=7Hz), 4.20 (1H, q, J=7Hz), 5.89 (0.5H, s), 6.04 (0.5H, s), 6.86-6.99 (4H, m), 7.39-7.50 (4H, m)

Preparation 10-4)

Ethyl 7-amidinothio-3-[4-(4-bromophenoxy)phenyl]hept-2-enoate hydroiodide (10.4 g) was obtained in a similar manner to that of Preparation 8-4).

NMR (DMSO-$d_6$, δ): 1.06 (1.5H, t, J=7Hz), 1.24 (1.5H, t, J=7Hz), 1.35-1.52 (2H, m), 1.54-1.75 (2H, m), 3.06-3.17 (2H, m), 3.39-3.49 (2H, m), 3.94 (1H, q, J=7Hz), 4.15 (1H, q, J=7Hz), 5.94 (0.5H, s), 6.07 (0.5H, s), 6.96-7.11 (4H, m), 7.56-7.64 (4H, m), 9.01-9.15 (4H, m)

MS (ESI+) m/z: 479, 563 (+TFA)

Preparation 11-1)

Methyl 4-(5-chlorovaleryl)phenyl ether (8.77 g) was obtained in a similar manner to that of Preparation 4-1).

NMR ($CDCl_3$, δ): 1.84-1.94 (4H, m), 2.97 (2H, t, J=7Hz), 3.59 (2H, t, J=6.5Hz), 3.88 (3H, s), 6.94 (2H, d, J=9Hz), 7.95 (2H, d, J=9Hz)

Preparation 11-2)

To a stirred solution of lithium diisopropylamide in tetrahydrofuran (prepared from diisopropylamine (2.32 g) and n-butyl lithium (14.3 ml, 1.6M in n-hexane) in tetrahydrofuran (14 ml) was added dropwise ethyl acetate (2.80 g) while maintaining -60°C on a dry-ice acetone bath. The mixture was stirred for 1 hour at -60°C and quenched by addition of saturated aqueous ammonium chloride. The mixture was extracted with ethyl acetate. The obtained organic phase was washed with saturated aqueous ammonium chloride three times and brine, dried over sodium sulfate and evaporated in vacuo. The residue was purified by column chromatography on silica gel (eluted with 5 to 10% ethyl acetate in n-hexane) to give ethyl 7-chloro-3-hydroxy-3-(4-methoxyphenyl)-heptanoate (3.56 g) as an oil.

NMR ($CDCl_3$, δ): 1.12 (3H, t, J=7.5Hz), 1.16-1.30 (1H, m), 1.39-1.56 (1H, m), 1.62-1.82 (4H, m), 2.77 (1H, d, J=16Hz), 2.94 (1H, d, J=16Hz), 3.45 (2H, t, J=6.5Hz), 3.80 (3H, s), 4.04 (2H, q, J=7.5Hz), 4.38 (1H, s), 6.86 (2H, d, J=9Hz), 7.31 (2H, d, J=9Hz)

Preparation 11-3)

A mixture of ethyl 7-chloro-3-hydroxy-3-(4-methoxyphenyl)heptanoate (3.55 g), potassium thioacetate (1.42 g) and catalytic amount of tetrabutyl ammonium iodide (n-$Bu_4NI$) (150 mg) in N,N-dimethylformamide (30 ml) was stirred for 6 hours at room temperature. The mixture was poured into saturated aqueous ammonium chloride and extracted with ethyl acetate. The organic phase was washed with saturated aqueous ammonium chloride three times and brine, dried over sodium sulfate and evaporated in vacuo to give ethyl 7-acetylthio-3-hydroxy-3-(4-methoxyphenyl) heptanoate (3.61 g) as an oil.

NMR ($CDCl_3$, δ): 1.12 (3H, t, J=7Hz), 1.33-1.55 (4H, m), 1.66-1.80 (2H, m), 2.30 (3H, s), 2.72-2.81 (3H, m), 2.93 (1H, d, J=15.5Hz), 3.80 (3H, s), 4.03 (2H, q, J=7Hz), 4.36 (1H, s), 6.85 (2H, d, J=9Hz), 7.30 (2H, d, J=9Hz)

Preparation 11-4)

A mixture of ethyl 7-acetylthio-3-hydroxy-3-(4-methoxyphenyl)heptanoate (3.60 g) and potassium carbonate (1.40 g) in ethanol (54 ml) was stirred for 6 hours at room temperature and concentrated in vacuo. The residue was partitioned between ethyl acetate and 1% aqueous citric acid. The separated organic phase was washed with brine, dried over sodium sulfate and evaporated in vacuo to give ethyl 3-hydroxy-3-(4-methoxyphenyl)-7-mercaptoheptanoate

(3.01 g) as an oil.

NMR ($CDCl_3$, δ): 1.12 (3H, t, J=7.5Hz), 1.26 (1H, t, J=7.5Hz), 1.35-1.60 (4H, m), 1.65-1.82 (2H, m), 2.40-2.57 (2H, m), 2.77 (1H, d, J=16Hz), 2.94 (1H, d, J=16Hz), 3.80 (3H, s), 4.04 (2H; q, J=7.5Hz), 4.36 (1H, s), 6.86 (2H, d, J=9Hz), 7.30 (2H, d, J=9Hz)

Preparation 12-1)

5-(4-Fluorophenyl)-2-(5-chlorovaleryl)thiophene (2.03 g) was obtained in a similar manner to that of Preparation 4-1).

NMR ($CDCl_3$, δ): 1.79-1.99 (4H, m), 2.95 (2H, t, J=7Hz), 3.59 (2H, t, J=7Hz), 7.12 (2H, dd, J=8, 8Hz), 7.59-7.68 (4H, m)

Preparation 12-2)

Ethyl 7-chloro-3-[5-(4-fluorophenyl)-2-thienyl]hept-2-enoate (1.71 g) (E:Z = 1:1 mixture) was obtained in a similar manner to that of Preparation 8-2).

NMR ($CDCl_3$, δ): 1.24 (1.5H, t), 1.37 (1.5H, t), 1.63-1.98 (4H, m), 2.56 (0.5H, t, J=7Hz), 2.87-3.00 (1H, m), 3.08 (0.5H, t, J=7Hz), 3.53 (1H, t, J=7Hz), 3.61 (1H, t, J=7Hz), 4.10-4.24 (2H, m), 5.87 (0.5H, s), 6.22 (0.5H, s), 7.04-7.31 (3H, m), 7.55-7.69 (3H, m)

Preparation 12-3)

Ethyl 3-[5-(4-fluorophenyl)-2-thienyl]-7-iodohept-2-enoate (1.94 g) was obtained in a similar manner to that of Preparation 8-3).

NMR ($CDCl_3$, δ): 1.06 (1.5H, t, J=7Hz), 1.32 (1.5H, t, J=7Hz), 1.67-2.08 (4H, m), 3.24 (2H, t, J=7Hz), 4.18 (2H, q, J=7Hz), 6.22 (1H, s), 6.92-7.30 (4H, m), 7.48-7.60 (2H, m)

Preparation 12-4)

Ethyl 7-amidinothio-3-[5-(4-fluorophenyl)-2-thienyl]hept-2-enoate hydroiodide (1.57 g) was obtained in a similar manner to that of Preparation 8-4).

NMR ($CDCl_3$, δ): 1.05 (3H, t, J=7Hz), 1.57-1.82 (4H, m), 3.18 (2H, t, J=7Hz), 4.14 (2H, q, J=7Hz), 4.36 (2H, t, J=7Hz), 6.19 (1H, s), 7.24-7.69 (6H, m)

MS (ESI+) m/z = 407 (M+H)

Preparation 13-1)

4-(5-Chlorovaleryl)biphenyl (1.35 g) was obtained in a similar manner to that of Preparation 4-1).

NMR ($CDCl_3$, δ): 1.90-1.93 (4H, m), 3.06 (2H, dd, J=6, 6Hz), 3.61 (2H, dd, J=7, 7Hz), 7.40-7.50 (3H, m), 7.63 (2H, d, J=8Hz), 7.69 (2H, d, J=8Hz), 8.03 (2H, d, J=8Hz)

Preparation 13-2)

Ethyl 3-(4-biphenylyl)-7-chlorohept-2-enoate (1 g) was obtained in a similar manner to that of Preparation 8-2).

NMR ($CDCl_3$, δ): 1.10 (1.5H, dd, J=7, 7Hz), 1.33 (1.5H, dd, J=7, 7Hz), 1.55-1.68 (1H, m), 1.76-1.90 (1H, m), 2.51 (1H, dd, J=7.5, 7.5Hz), 3.18 (1H, dd, J=7.5, 7.5Hz), 3.52 (1H, dd, J=6.5, 6.5Hz), 3.54 (1H, dd, J=6.5, 6.5Hz), 4.02 (1H, ddd, J=7, 7, 7Hz), 4.22 (1H, ddd, J=7, 7, 7Hz), 5.92 (0.5H, s), 6.13 (0.5H, s), 7.24-7.65 (9H, m)

Preparation 14-1)

4'-Chloro-4-(5-chlorovaleryl)biphenylyl (3.29 g) was obtained in a similar manner to that of Preparation 4-1).

NMR ($CDCl_3$, δ): 1.90-1.93 (4H, m), 3.05 (2H, dd, J=6, 6Hz), 3.60 (2H, dd, J=6, 6Hz), 7.44 (2H, d, J=8Hz), 7.56 (2H, d, J=8Hz), 7.65 (2H, d, J=8Hz), 8.03 (2H, d, J=8Hz)

Preparation 14-2)

Ethyl 7-chloro-3-(4'-chloro-4-biphenylyl)hept-2-enoate (0.70 g) was obtained in a similar manner to that of Preparation 8-2).

NMR ($CDCl_3$, δ): 1.11 (1.5H, dd, J=7, 7Hz), 1.32 (1.5H, dd, J=7, 7Hz), 1.50-1.58 (1H, m), 1.58-1.65 (1H, m), 2.49 (1H, dd, J=7, 7Hz), 3.17 (1H, dd, J=7, 7Hz), 3.50 (1H, dd, J=6, 6Hz), 3.54 (1H, dd, J=6, 6Hz), 4.01 (1H, ddd, J=7, 7, 7Hz), 4.22 (1H, ddd, J=7, 7, 7Hz), 5.93 (0.5H, s), 6.12 (0.5H, s), 7.40-7.58 (8H, m)

Preparation 15-1)

4'-Bromo-4-(5-chlorovaleryl)biphenyl (1.97 g) was obtained in a similar manner to that of Preparation 4-1).

NMR ($CDCl_3$, δ): 1.90-1.93 (4H, m), 3.05 (2H, dd, J=7, 7Hz), 3.60 (2H, dd, J=6, 6Hz), 7.49 (2H, d, J=8Hz), 7.60 (2H, d, J=8Hz), 7.65 (2H, d, J=8Hz), 8.03 (2H, d, J=8Hz)

Preparation 15-2)

Ethyl 7-chloro-3-(4'-bromo-4-biphenylyl)hept-2-enoate (0.64 g) was obtained in a similar manner to that of Preparation 8-2).

NMR ($CDCl_3$, δ): 1.11 (1.5H, dd, J=7, 7Hz), 1.33 (1.5H, dd, J=7, 7Hz), 1.52-1.67 (2H, m), 1.75-1.89 (2H, m), 2.50 (1H, dd, J=7, 7Hz), 3.17 (1H, dd, J=8, 8Hz), 3.51 (1H, dd, J=7, 7Hz), 3.54 (1H, dd, J=7, 7Hz), 4.02 (1H, ddd, J=7, 7, 7Hz), 4.22 (1H, ddd, J=7, 7, 7Hz), 5.93 (0.5H, s), 6.11 (0.5H, s), 7.24-7.60 (8H, m)

Preparation 16-1)

4'-Fluoro-4-(5-chlorovaleryl)biphenyl (2.95 g) was obtained in a similar manner to that of Preparation 4-1).

NMR ($CDCl_3$, δ): 1.89-1.95 (4H, m), 3.05 (2H, dd, J=7, 7Hz), 3.60 (2H, dd, J=6, 6Hz), 7.13-7.19 (2H, m), 7.57-7.65 (2H, m), 8.03 (2H, d, J=8.4Hz)

Preparation 16-2)

Ethyl 7-chloro-3-(4'-fluoro-4-biphenylyl)hept-2-enoate (1.07 g) was obtained in a similar manner to that of Preparation 8-2).

NMR ($CDCl_3$, δ): 1.33 (3H, dd, J=7, 7Hz), 1.59-1.67 (2H, m), 1.81-1.90 (2H, m), 3.17 (2H, dd, J=7.5, 7.5Hz), 3.54 (2H, dd, J=7, 7Hz), 4.22 (2H, ddd, J=7, 7, 7Hz), 6.12 (1H, s), 7.14 (2H, dd, J=8, 8Hz), 7.50-7.59 (6H, m)

Preparation 17)

Methyl 3,4,5,6-tetrahydro-2H-thiopyran-2-carboxylate 1,1-dioxide (844 mg) was obtained in a similar manner to that of Preparation 1-4).

mp: 78-82°C

NMR ($CDCl_3$, δ): 1.44-1.80 (3H, m), 1.83-1.96 (1H, m), 2.10-2.42 (2H, m), 2.72-2.85 (1H, m), 3.14-3.78 (1H, m), 5.57 (1H, dd, J=3, 8Hz), 3.82 (3H, s)

MS (ESI-) m/z: 191 (M-H)

Preparation 18)

To a solution of potassium ethyl malonate (16.7 g) in acetonitrile (4 ml) was added triethylamine (15.1 g) and magnesium chloride (11.1 g) at 0°C and the reaction mixture was stirred at ambient temperature for 2.5 hours. To the resulting slurry was added phenoxybenzoyl chloride (10.86 g) (prepared from 4-phenoxybenzoic acid (10 g) and thionyl chloride (20 ml)] dropwise over 25 minutes at 0°C and the reaction mixture was stirred at ambient temperature for 5 hours. After the reaction mixture was concentrated in vacuo, toluene and 13% aqueous hydrochloric acid (60 ml) was added therein cautiously while keeping the temperature below 25°C. The organic layer was washed with 13% aqueous hydrochloric acid and concentrated in vacuo to give ethyl 3-oxo-3-(4-phenoxyphenyl)propanoate as a yellow oil (14 g).

NMR ($CDCl_3$, δ): 1.27 (3H, t, J=7.0Hz), 3.95 (2H, s), 4.24 (2H, q, J=7.0Hz), 6.99 (2H, d, J=8.0Hz), 7.07 (2H, d, J=8.0Hz), 7.18 (1H, dd, J=8.0, 8.0Hz), 7.41 (2H, dd, J=8.0, 8.0Hz), 7.92 (2H, d, J=8.0Hz)

MS (ESI-) m/z: 283.1 (M-H)

Preparation 19-1)

5-Bromo-2-(5-chlorovaleryl)thiophene (13.4 g) was obtained in a similar manner to that of Preparation 4-1).

NMR ($CDCl_3$, δ): 1.86-1.91 (4H, m), 2.88 (2H, dd, J=6.9, 6.9Hz), 3.55 (2H, dd, J=6.6, 6.6Hz), 7.11 (1H, d, J=4.2Hz), 7.45 (1H, d, J=4.2Hz)

Preparation 19-2)

Ethyl 7-chloro-3-(5-bromo-2-thienyl)hept-2-enoate (12.5 g) was obtained in a similar manner to that of Preparation 8-2).

NMR ($CDCl_3$, δ): 1.22-1.59 (3H, m), 1.65-1.92 (4H, m), 3.01-3.06 (1H, m), 3.50-3.59 (2H, m), 4.10-4.23 (2H, m), 5.85 (0.5H, s), 6.09 (0.5Hz, s), 6.98-7.07 (2H, m)

Preparation 20-1)

tert-Butyl 7-chloro-3-hydroxy-3-(5-bromo-2-thienyl)heptanoate (244 g) was obtained in substantially the same manner as that of Preparation 11-2).

NMR ($CDCl_3$, δ): 1.36 (9H, s), 1.38-1.57 (2H, m), 1.68-1.80 (4H, m), 2.70 (1H, d, J=15.6Hz), 2.79 (1H, d, J=15.6Hz), 3.49 (2H, t, J=6.9Hz), 5.00 (1H, s), 6.59 (1H, d, J=3.9Hz), 6.89 (1H, d, J=3.9Hz)

Preparation 20-2)

tert-Butyl 7-acetylthio-3-hydroxy-3-(5-bromo-2-thienyl)heptanoate (267 g) was obtained in substantially the same manner as that of Preparation 11-3).

NMR ($CaCl_3$, δ): 1.35 (9H, s), 1.39-1.57 (4H, m), 1.66-1.80 (2H, m), 2.31 (3H, s), 2.67 (1H, d, J=15.9Hz), 2.76 (1H, d, J=15.9Hz), 2.82 (2H, t, J=7.5Hz), 4.96 (1H, s), 6.58 (1H, d, J=3.9Hz), 6.88 (1H, d, J=3.9Hz)

Preparation 20-3)

tert-Butyl 3-hydroxy-3-(5-bromo-2-thienyl)-7-mercaptoheptanoate (277 g) was obtained in substantially the same manner as that of Preparation 11-4).

NMR ($CDCl_3$, δ): 1.30 (1H, t, J=7.8Hz), 1.31-1.39 (9H, m), 1.40-1.65 (4H, m), 1.67-1.83 (2H, m), 2.49 (2H, dd, J=7.8, 15Hz), 2.70 (1H, d, J=16H), 2.79 (1H, d, J=16Hz), 4.98 (1H, s), 6.59 (1H, d, J=4.2Hz), 6.89 (1H, d, J=4.2Hz)

Preparation 21-1)

To a solution of 4-bromophenol (300 mg) in acetone was added potassium carbonate (264 mg) and bromoacetic acid t-butyl ester (0.28 ml) at room temperature. After being stirred at the same temperature overnight, the reaction mixture was concentrated in vacuo. The resulting residue was diluted with ethyl acetate, washed with aqueous saturated sodium bicarbonate and brine, dried over sodium sulfate and concentrated in vacuo to give 4-bromophenoxy acetic acid t-butyl ester (450 mg) as an oil.

NMR (DMSO-$d_6$, δ): 1.42 (9H, s), 4.66 (2H, s), 6.88 (2H, d, J=4.5Hz), 7.45 (2H, d, J=4.5Hz)

Preparation 21-2)

To a solution of 4-bromophenoxyacetic acid t-butyl ester (4 g) in dichloromethane (10 ml) was added trifluoroacetic acid (30 ml) at room temperature. After being stirred at the same temperature for 3 hours, the reaction mixture was concentrated in vacuo. The resulting residue was diluted with ethyl acetate, washed with water and brine, dried over sodium sulfate and concentrated in vacuo to give 4-bromophenoxy acetic acid (2.1 g) as a power.

NMR (DMSO-$d_6$, δ): 4.67 (2H, s), 6.89 (2H, d, J=9Hz), 7.45 (2H, d, J=9Hz)

MS (ESI-): 230 (M-H)

Preparation 21-3)

N-Ethyl-2-(4-bromophenoxy)acetamide (110 mg) was obtained in substantially the same manner as that of Example 32.

NMR (DMSO-$d_6$, δ): 1.03 (3H, t, J=7.2Hz), 3.10-3.19 (2H, m), 4.45 (2H, s), 6.93 (2H, d, J=9Hz), 7.47 (2H, d, J=9Hz), 8.11 (1H, br)

Preparation 21-4)

4-(Ethylaminocarbonylmethoxy)benzeneboronic acid pinacol cyclic ester (130 mg) was obtained in substantially the same manner as that of Preparation 24-2).

The product was used for the next reaction without further purification.

Preparation 22

4-(t-Butyloxycarbonylmethoxy)benzeneboronic acid (200 mg) was obtained in substantially the same manner as that of Preparation 21-1).

NMR (DMSO-$d_6$, δ): 1.42 (9H, s), 4.65 (2H, s), 6.84 (2H, d, J=9.0Hz), 7.71 (2H, d, J=9.0Hz), 7.88 (2H, s)

Preparation 23-1)

4-Chloro-1-(5-bromo-2-thienyl)butan-1-one (8.0 g) was obtained in substantially the same manner as that of Preparation 8-1).

NMR ($CDCl_3$, δ): 2.21 (2H, quintet, J=7Hz), 3.05 (2H, t, J=7Hz), 3.66 (2H, t, J=7Hz), 7.11 (1H, d, J=4Hz), 7.50 (1H, d, J=4Hz)

Preparation 23-2)

tert-Butyl 6-chloro-3-hydroxy-3-(5-bromo-2-thienyl)hexanoate (4.46 g) was obtained in substantially the same manner as that of Preparation 11-2).

NMR ($CDCl_3$, δ): 1.37 (9H, s), 1.64-1.80 (1H, m), 1.82-1.98 (3H, m), 2.70 (1H, d, J=16Hz), 2.80 (1H, d, J=16Hz), 3.48-3.55 (2H, m), 5.02 (1H, s), 6.61 (1H, d, J=4Hz), 6.90 (1H, d, J=4Hz)

Preparation 23-3)

tert-Butyl 6-acetylthio-3-hydroxy-3-(5-bromo-2-thienyl)hexanoate (4.77 g) was obtained in substantially the same manner as that of Preparation 11-3).

NMR ($CDCl_3$, δ): 1.36 (9H, s), 1.45-1.60 (1H, m), 1.62-1.94 (3H, m), 2.31 (3H, s), 2.68 (1H, d, J=16Hz), 2.77 (1H, d, J=16Hz), 2.85 (2H, t, J=7Hz), 4.98 (1H, s), 6.59 (1H, d, J=4Hz), 6.88 (1H, d, J=4Hz)

Preparation 23-4)

tert-Butyl 3-hydroxy-3-(5-bromo-2-thienyl)-6-mercaptohexanoate (4.0 g) was obtained in substantially the same manner as that of Preparation 11-4).

NMR ($CDCl_3$, δ): 1.30 (1H, t, J=8Hz), 1.36 (9H, s), 1.44-1.62 (1H, m), 1.66-1.93 (3H, m), 2.44-2.55 (2H, m), 2.70 (1H, d, J=16Hz), 2.79 (1H, d, J=16Hz), 4.99 (1H, s), 6.60 (1H, d, J=4Hz), 6.89 (1H, d, J=4Hz)

Preparation 24-1)

A mixture of 4-bromobenzaldehyde (5.00 g), tosylmethyl isocyanide (5.43 g) and potassium carbonate (5.60 g) in methanol (50 ml) was refluxed for 2 hours and concentrated. The residue was taken up between ethyl acetate and saturated aqueous ammonium hydrochloride. The separated organic layer was washed with water and brine, dried over sodium sulfate and filtered. The filtrate was treated with silica gel and the obtained residue was triturated with n-hexane to give 5-(4-bromophenyl)oxazole (4.06 g) as a solid.

NMR ($CDCl_3$, δ): 7.37 (1H, s), 7.51-7.58 (4H, m), 7.93 (1H, s)

MS (ESI+): 224 (M+H)

Preparation 24-2)

A mixture of 5-(4-bromophenyl)oxazole (672 mg), bis(pinacolato)diborane (762 mg) dichlorobis(triphenylphosphine)palladium(II) (42.1 mg) and potassium acetate (883 mg) in dioxane (15 ml) was stirred for 14 hours at 80°C to form 4-(5-oxazolyl)benzeneboronic acid pinacol cyclic ester. After cooling to room temperature, the mixture was used to next reaction without further purification.

Preparation 25

(4-(Methoxycarbonylaminomethyl)phenyl)boronic acid (70-mg) was obtained from (4-aminomethylphenyl)boronic acid hydrochloride in a similar manner to that of Example 130.

NMR (DMSO-$d_6$, δ): 3.53 (3H, s), 4.18 (2H, d, J=7.5Hz), 7.20 (2H, d, J=8.5Hz), 7.66-7.75 (3H, m), 8.00 (2H, s)

Preparation 26

(4-(Cyclopropylcarbonyloxy)phenyl)boronic acid (77 mg) was obtained from (4-hydroxyphenyl)boronic acid in a similar manner to that of Preparation 21-1). NMR (DMSO-$d_6$, δ): 1.00-1.07 (4H, m), 1.85-1.94 (1H, m), 7.08 (2H, d, J=8.5Hz), 7.81 (2H, d, J=8.5Hz), 8.09 (2H, s)

Preparation 27

(4-(Ethoxycarbonylmethoxy)phenyl)boronic acid (100 mg) was obtained in a similar manner to that of Preparation 21-1).

NMR (DMSO-$d_6$, δ): 1.21 (3H, dd, J=7.2, 7.2Hz), 4.17 (2H, ddd, J=7.2, 7.2, 7.2Hz), 4.78 (2H, s), 6.87 (2H, d, J=8.5Hz), 7.72 (2H, d, J=8.5Hz), 7.88 (2H, s)

Preparation 28

(4-(Ethylcarbonylmethoxy)phenyl)boronic acid (95 mg) was obtained in a similar manner to that of Preparation 21-1).

NMR (DMSO-$d_6$, δ): 0.97 (3H, dd, J=7.2, 7.2Hz), 2.49-2.54 (2H, m), 4.82 (2H, s), 6.84 (2H, d, J=8.5Hz), 7.71 (2H, d, J=8.5Hz), 7.87 (2H, s)

Preparation 29

(4-Cyclopropylaminocarbonylmethoxyphenyl)boronic acid (160 mg) was obtained in a similar manner to that of Example 32.

NMR (DMSO-$d_6$, δ): 0.46-0.50 (2H, m), 0.61-0.64 (2H, m),-2.65-2.72 (1H, m), 4.43 (2H, s), 6.88 (2H, d, J=8.5Hz), 7.72 (2H, d, J=8.5Hz), 7.88 (2H, s), 8.13 (1H, br)

Preparation 30

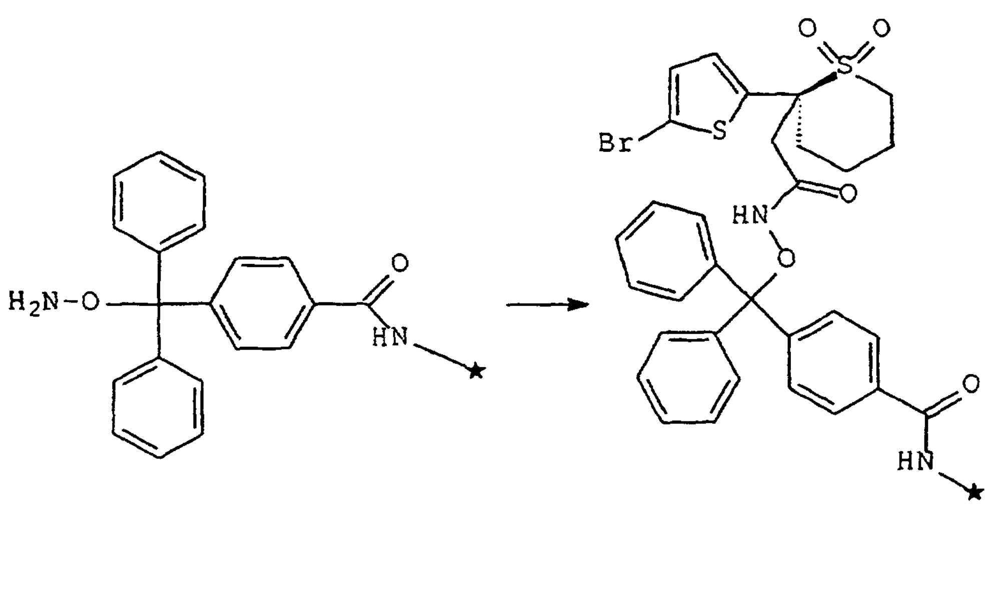

(a) (b)

To a solution of (2S)-2-(5-bromo-2-thienyl)-3,4,5,6-tetrahydro-2H-thiopyran-2-acetic acid 1,1-dioxide (4.24 g) in N,N-dimethylformamide (60 ml) was added 1-hydroxybenztriazole (1.62 g) and diisopropylcarbodiimide (1.88 ml) at ambient temperature. After 1 minute, the solution was added to hydroxylamine trityl crowns (a) (14.4 μmol/crown x 100), the reaction mixture was left overnight at ambient temperature. The crowns were washed with N,N-dimethylformamide, methanol and dichloromethane, successively and air dried to give (2S)-N-[2-[2-(5-bromo-2-thienyl)-1,1-dioxo-3,4,5,6-tetrahydro-2H-thiopyran-2-yl]acetyl]-hydroxylamine trityl crowns (b) (10.0 μmol/crown x 100).

Preparation 31-1)

To a suspension of 3-nitrophenylboronic acid (2.33 g) and tetrakis(triphenylphosphine)palladium (1.29 g) in degassed N,N-dimethylformamide (50 ml) was added a solution of sodium carbonate (8.5 g) in degassed water (20 ml) and (2S)-N-[2-[2-(5-brorno-2-thienyl)-1,1-dioxo-3,4,5,6-tetrahydro-2H-thiopyran-2-yl]acetyl]hydroxylamine trityl crowns (202 μmol, 10.1 μmol/crown x 20) in an atmosphere of nitrogen. After the resulting mixture was heated for 48 hours at 60°C, the crowns were washed with degassed N,N-dimethylformamide, a solution of sodium diethylditiocarbamate (1.0 g) and diisopropylethylamine (1.0 ml) in N,N-dimethylformamide (200 ml), N,N-dimethylformamide, methyl sulfoxide, water, methanol and dichloromethane, successively to give (2S)-N-[2-[2-(5-(3-nitrophenyl)-2-thienyl)-1,1-dioxo-3,4,5,6-tetrahydro-2H-thiopyran-2-yl]acetyl]hydroxylamine trityl crowns (202 μmol, 10.1 μmol/crown x 20).

Preparation 31-2)

To a solution of 2M tin (II) chloride dihydrate (7.67 g) in N,N-dimethylformamide (17 ml) was added (2S)-N-[2-[2-(5-(3-nitrophenyl)-2-thienyl)-1,1,-dioxo-3,4,5,6-tetrahydro-2H-thiopyran-2-yl]acetyl]hydroxylamine trityl crowns (304 μmol, 13.2 μmol/crown x 23). The reaction mixture was left overnight at ambient temperature. The crowns were washed with N,N-dimethylformamide, water, methanol and dichloromethane, successively and air dried to give (2S)-N-[2-[2-(5-(3-aminophenyl)-2-thienyl)-1,1-dioxo-3,9,5,6-tetrahydro-2H-thiopyran-2-yl]acetyl]hydroxylamine trityl crowns (13.2 μmol/crown x 23).

The following compounds were obtained from 6-methyl-3-(trifluoromethanesulfonyloxy)pyridine in a similar manner to that of Preparation 24-2).

Preparation 32

6-Methylpyridine-3-boronic acid pinacol cyclic ester

Preparation 33

6-Methoxypyridine-3-boronic acid pinacol cyclic ester

Preparation 34

4-(5-Methyl-1,2,4-oxadiazol-3-yl)benzeneboronic acid pinacol cyclic ester

Preparation 35

5-(Methoxycarbonylamino)pyridine-3-boronic acid pinacol cyclic ester

Preparation 36

(2S)-N-(2-Tetrahydropyranyloxy)-2-[5-(pinacolatoboryl)-2-thienyl]-3,4,5,6-tetrahydro-2H-thiopyran-2-acetamide 1,1-dioxide

Preparation 37

4-(Methylaminocarbonylmethyl)benzeneboronic acid pinacol cyclic ester

Preparation 38

2-(Methylaminocarbonyl)benzofuran-5-boronic acid pinacol cyclic ester

Example 1

1.5M Lithium diisopropylamide mono tetrahydrofuran in cyclohexane (0.28 ml) was added dropwise to a stirred suspension of 2-[4-(4-chlorophenoxy)phenyl]-3,4,5,6-tetrahydro-2H-thiopyran 1,1-dioxide (117 mg) in tetrahydrofuran

(2.4 ml) under dry ice - acetone cooling and a nitrogen atmosphere, and the mixture was stirred under the same conditions for 15 minutes, then a solution of tert-butyl bromoacetate (75 mg) in tetrahydrofuran (0.2 ml) was added dropwise therein and the resulting mixture was stirred under the same conditions for 2 hours. A saturated aqueous solution of ammonium chloride was added to the stirred reaction mixture and the resulting mixture was extracted with diethyl ether. The extract was washed with brine, dried over sodium sulfate, and evaporated in vacuo. The residue was chromatographed (eluent: toluene - ethyl acetate) over silica gel to afford a mixture (86 mg) of t-butyl 2-[4-(4-chlorophenoxy)phenyl]-3,4,5,6-tetrahydro-2H-thiopyran-2-acetate 1,1-dioxide and the starting material.

A solution of trifluoroacetic acid (560 mg) and the obtained mixture (79 mg) in dichloromethane (3.0 ml) was allowed to stand at room temperature for 3 days and evaporated in vacuo. The residue was dissolved in ethyl acetate and extracted five times with a saturated aqueous solution of sodium bicarbonate. The aqueous extracts were combined, acidified with hydrochloric acid, and extracted with ethyl acetate. The organic extract was washed with brine, dried over sodium sulfate, and evaporated in vacuo to afford 2-[4-(4-chlorophenoxy)phenyl]-3,4,5,6-tetrahydro-2H-thiopyran-2-acetic acid 1,1-dioxide (44 mg) as a colorless powder.

mp: 191-193°C

IR (KBr): 1711, 1290, 1244, 1124 $cm^{-1}$

NMR ($CDCl_3$, δ): 1.75-2.05 (2H, m), 2.15 (2H, m), 2.6-2.85 (2H, m), 3.08-3.15 (2H, m), 3.21 (1H, d, J=15.6Hz), 3.60 (1H, d, J=15.6Hz), 6.94-7.01 (4H, m), 7.31 (2H, dd, J=6.7, 2.1Hz), 7.59 (2H, d, J=9.0Hz)

(-) API-ES MS m/z: 393 ($M^+$-H)

| | | |
|---|---|---|
| Anal. Calcd. for $C_{19}H_{19}ClO_5S$ | C 57.79, | H 4.85 |
| Found | C 57.88, | H 4.83 |

Example 2

A mixture of ethyl 2-(4-methoxyphenyl)-3,4,5,6-tetrahydro-2H-thiopyran-2-acetate (100 mg) and lithium hydroxide monohydrate (42.8 mg) in a mixture of methanol and water was stirred for 4 hours at 60°C. After cooling to room temperature, the mixture was acidified with 4N hydrochloric acid and concentrated in vacuo. The residue was partitioned between ethyl acetate and 1N hydrochloric acid. The separated organic layer was washed with brine, dried over sodium sulfate and evaporated in vacuo to give 2-(4-methoxyphenyl)-3,4,5,6-tetrahydro-2H-thiopyran-2-acetic acid (93 mg) as a crystalline solid.

NMR (DMSO-$d_6$, δ): 1.44-1.80 (4H, m), 2.26-2.54 (3H, m), 2.62-2.79 (2H, m), 3.00 (1H, d, J=14.5Hz), 3.75 (3H, s), 6.89 (2H, d, J=9Hz), 7.48 (2H, d, J=9Hz)

MS (ESI-) m/z: 265 (M-H)

Example 3

To a solution of ethyl 2-[5-(4-fluorophenyl)-2-thienyl]-3,4,5,6-tetrahydro-2H-thiopyran-2-acetate (315 mg) in methanol (4 ml) was added 1N sodium hydroxide aqueous solution (1.3 ml) at 0°C and the mixture was stirred for 5 hours at room temperature. The resulting mixture was evaporated to remove methanol. The residue was acidified with 1N hydrochloric acid (HCl) and extracted with ethyl acetate (x3). The combined organic layer was washed with brine, dried over magnesium sulfate and concentrated to give 2-[5-(4-fluorophenyl)-2-thienyl]-3,4,5,6-tetrahydro-2H-thiopyran-2-acetic acid (296 mg) as white solid.

NMR ($CDCl_3$, δ): 1.58-1.92 (4H, m), 2.20-2.32 (1H, m), 2.57-2.68 (2H, m), 2.70-2.81 (1H, m), 2.89 (1H, d, J=14Hz), 2.97 (1H, d, J=14Hz), 6.97-7.08 (4H, m), 7.48-7.56 (2H, m)

MS (ESI-) m/z: 335 (M-H)

Example 4

To a solution of methyl 2-(4-phenoxybenzyl)-3,4,5,6-tetrahydro-2H-thiopyran-2-carboxylate 1,1-dioxide in methanol (MeOH) (5 ml) was added solution of lithium hydroxide monohydrate (375 mg) in water ($H_2O$) (5 ml) at room temperature. After being stirred at 60°C for 2 hours, the mixture was concentrated in vacuo to remove MeOH. The residual solution was acidified by 1M hydrochloric acid and extracted with ethyl acetate (AcOEt) (20 ml x 2). The combined extract was washed with brine, dried over magnesium sulfate, and concentrated in vacuo to give 2-(4-phenoxybenzyl)-3,4,5,6-tetrahydro-2H-thiopyran-2-carboxylic acid 1,1-dioxide (320 mg) as an amorphous powder.

NMR ($CDCl_3$, δ): 1.70-1.84 (2H, m), 1.95-2.20 (3H, m), 2.25-2.37 (1H, m), 3.11-3.25 (3H, m), 3.16 (1H, d, J=14Hz), 6.91 (2H, d, J=8Hz), 7.00 (2H, d, J=8Hz), 7.11 (1H, t, J=8Hz), 7.20 (2H, d, J=8Hz), 7.33 (2H, t, J=8Hz)

MS (ESI-) m/z: 359 (M-H)

Example 5

2-(4-Phenoxybenzyl)-3,4,5,6-tetrahydro-2H-thiopyran-2-carboxylic acid (280 mg) was obtained in a similar manner to that of Example 4.

NMR ($CDCl_3$, δ): 1.45-2.02 (5H, m), 2.35-2.46 (1H, m), 2.52-2.65 (1H, m), 2.68-2.85 (1H, m), 3.07 (2H, dd, J=3, 14Hz), 6.90 (2H, d, J=8Hz), 6.99 (2H, d, J=8Hz), 7.09 (1H, t, J=8Hz), 7.13 (2H, d, J=8Hz), 7.32 (2H, t, J=8Hz)

MS (ESI-) m/z: 327 (M-H)

Example 6

1.5M Solution of lithium diisopropylamide mono-tetrahydrofuran in cyclohexane (1.60 ml) was added dropwise to a stirred solution of 2-[4-(4-chlorophenoxy)phenyl]-2,3,4,5-tetrahydrothiophene 1,1-dioxide (646 mg) in tetrahydrofuran (6.5 ml) under a nitrogen atmosphere and dry ice - acetone cooling, and the'resultant solution was stirred under the same conditions for 35 minutes. A solution of allyl bromide (532 mg) in tetrahydrofuran (1.9 ml) was added dropwise therein and the resultant mixture was stirred under the same conditions for 1 hour and 30 minutes. After addition of a saturated aqueous solution of ammonium chloride (10 ml) under the same conditions, the reaction mixture was extracted with ethyl acetate. The extract was washed successively with 1N hydrochloric acid and a saturated aqueous solution of sodium bicarbonate, dried over magnesium sulfate, and evaporated in vacuo to afford an oil (0.68 g).

Potassium permanganate (259 mg), sodium periodate (1.75 g), and potassium carbonate (618 mg) was successively added to a stirred emulsion of the obtained oil in tert-butyl alcohol (22 ml) and water (38 ml) at room temperature and the resulting mixture was stirred at the same temperature for 1 hour and 40 minutes. The reaction mixture was acidified to pH c.a. 1.0 with 1N hydrochloric acid (10 ml) under ice cooling, and then sodium bisulfite was added portionwise therein under the same condition till the mixture became yellow. The yellow mixture was extracted with ethyl acetate. The extract was washed with an aqueous solution of sodium bisulfite and brine, dried over sodium sulfate, and evaporated in vacuo. The powdery residue was washed with a mixture of diisopropyl ether - diethyl ether to afford a colorless powder (401 mg), 388 mg of which was chromatographed (eluent: toluene - ethyl acetate - acetic acid) over silica gel to afford an oil. The obtained oil was powdered from n-hexane to afford 2-[4-(4-chlorophenoxy)phenyl]-2,3,4,5-tetrahydrothiophene-2-acetic acid 1,1-dioxide (306 mg) as a colorless amorphous powder.

mp: 45-50°C

IR (KBr): 2750-2400, 1734, 1716, 1300, 1244, 1126 $cm^{-1}$

NMR (DMSO-$d_6$, δ): 2.16-2.25 (2H, m), 2.65-2.77 (2H, m), 3.08-3.41 (4H, m), 7.00-7.10 (4H, m), 7.41-7.51 (4H, m), 12.38 (1H, br)

(-) API-ES MS m/z: 379 and 381 ($M^+$-H)

| | | |
|---|---|---|
| Anal. Calcd. for $C_{18}H_{17}ClO_5S$ | C 56.76, | H 4.50 |
| Found | C 57.32, | H 5.04 |

Example 7

Potassium permanganate (172 mg), sodium periodate (1.28 g), and potassium carbonate (409 mg) was successively added to a stirred emulsion of 2-allyl-2-[4-(4-chlorophenoxy)-phenyl]-3,4,5,6-tetrahydro-2H-thiopyran 1,1-dioxide (372 mg) in tert-butyl alcohol (15 ml) and water (26 ml) at room temperature and the resulting mixture was stirred at the same temperature for 1 hour and 30 minutes. The reaction mixture was acidified to pH c.a. 1.0 with conc. hydrochloric acid under ice cooling, and then sodium bisulfite was added portionwise therein under the same condition till the mixture became yellow. The yellow mixture was extracted with ethyl acetate. The extract was washed with brine, dried over sodium sulfate, and evaporated in vacuo. The residue was chromatogrphed (eluent: toluene - ethyl acetate - acetic acid) over silica gel to afford a colorless powder (277 mg), which was washed with n-hexane to afford 2-[4-(4-chlorophenoxy)phenyl]-3,4,5,6-tetrahydro-2H-thiopyran-2-acetic acid 1,1-dioxide (250 mg) as a colorless powder.

mp: 191-193°C

Example 8

2-[4-(4-Chlorophenoxy)phenyl]-3,4,5,6-tetrahydro-6-methyl-2H-thiopyran-2-acetic acid 1,1-dioxide (59 mg) was prepared in a similar manner to that of Example 7.

IR (KBr): 3442, 1735, 1714, 1284, 1245, 1124 $cm^{-1}$

NMR (DMSO-$d_6$, δ): 1.16 (3H, d, J=6.6Hz), 1.62-1.98 (4H, m), 2.47-2.63 (2H, m), 3.2-3.59 (3H, m), 6.98-7.10 (4H, m), 7.46 (2H, d, J=9.0Hz), 7.55 (2H, d, J=9.0Hz)

(-) API-ES MS m/z: 407 and 409 ($M^+$-H)

Example 9

A suspension of (2R or 2S)-2-[4-(4-chlorophenoxy)-phenyl]-3,4,5,6-tetrahydro-N-((1R)-1-phenylethyl)-2H-thiopyran-2-acetamide 1,1-dioxide (diastereomer A, 149 mg) obtained in Example 30 in a mixture of 14N sulfuric acid (7.0 ml) and 1,4-dioxane (4.2 ml) was stirred under reflux for 21 hours and cooled to room temperature. The reaction mixture was partitioned between ethyl acetate and water. The organic layer was washed with water and brine (twice), dried over magnesium sulfate, and evaporated in vacuo. The residue was chromatographed (eluent: dichloromethane - methanol) over silica gel to afford (2R or 2S)-(-)-2-[4-(4-chlorophenoxy)-phenyl]-3,4,5,6-tetrahydro-2H-thiopyran-2-acetic acid 1,1-dioxide (an optical isomer A, 109 mg) as a crude brown gum.

IR (KBr): 1734, 1716, 1284, 1244, 1122 $cm^{-1}$

NMR ($CDCl_3$, δ): 1.93 (2H, m), 2.14 (2H, m), 2.6-2.85 (2H, m), 3.08-3.16 (2H, m), 3.22 (1H, d, J=15.6Hz), 3.62 (1H, d, J=15.6Hz), 6.95-7.04 (4H, m), 7.28-7.35 (2H, m), 7.60.(2H, d, J=9.0Hz)

(-) API-ES MS m/z: 393 and 395 ($M^+$-H)

$[\alpha]_D^{25}$: -32.3° (C=1.0, MeOH)

Analytical chiral HPLC:

column: Chiralpak AS (4.6 x 250 mm,
Daicel Chemical Industries, Ltd.)
eluent: n-hexane-ethanol-TFA (700:300:1)
flow rate: 1.0 ml/minute
detection: 220 nm
retention time: 50.0 minutes

Example 10

(2R or 2S)-2-[4-(4-Chlorophenoxy)phenyl]-3,4,5,6-tetrahydro-2H-thiopyran-2-acetic acid 1,1-dioxide (an optical isomer B, 77 mg) was prepared from (2R or 2S)-(+)-2-[4-(4-chlorophenoxy)phenyl]-3,4,5,6-tetrahydro-N-((1R)-1-phenylethyl)-2H-thiopyran-2-acetamide 1,1-dioxide (diastereomer B, 133 mg) obtained in Example 30 in a similar manner to that of Example 9.

IR (KBr): 1711, 1290, 1242, 1122 $cm^{-1}$

NMR ($CDCl_3$, δ): 1.82-1.95 (2H, m), 2.10-2.15 (2H, m), 2.62-2.80 (2H, m), 3.03-3.11 (2H, m), 3.21 (1H, d, J=15.6Hz), 3.60 (1H, d, J=15.6Hz), 6.92-7.01 (4H, m), 7.28-7.34 (2H, m), 7.59 (2H, d, J=9.0Hz)

(-) API-ES MS m/z: 393 ($M^+$-H)

Analytical chiral HPLC:

column: Chiralpak AS (4.6 x 250 mm,
Daicel Chemical Industries, Ltd.)
eluent: n-hexane-ethanol-TFA (700:300:1)
flow rate: 1.0 ml/minute
detection: 220 nm
retention time: 8.96 minutes

Example 11

To a solution of 2-[4-(4-chlorophenoxy)phenyl]-3,4,5,6-tetrahydro-2H-thiopyran-2-acetic acid 1,1-dioxide (16.8 g) in ethyl acetate (420 ml) was added (R)-(+)-α-methylbenzylamine (2.84 g) at room temperature. After being stirred overnight at the same temperature, the resulting crystal was filtrated and washed with ethyl acetate to give (2R or 2S)-2-[4-(4-chlorophenoxy)phenyl]-3,4,5,6-tetrahydro-2H-thiopyran-2-acetic acid 1,1-dioxide (R)-(+)-α-methylbenzylamine salt (9.43 g). A suspension of resulting salt in ethyl acetate (200 ml) was washed with 1N hydrochloric acid (100 ml x 2), water and brine, and concentrated to give the free acid. This procedure was repeated three times (second: amine 0.75 eq; third:0.85 eq) to give the optically resoluted (2R or 2S)-(-)-2-[4-(4-chlorophenoxy)phenyl]-3,4,5,6-tetrahydro-2H-thiopyran-2-acetic acid (an optical isomer A) (4.75 g) as a white solid.

$[\alpha]_D^{25}$: -32.3° (C=1.0, MeOH)

Optical purity: 91% ee

Analytical chiral HPLC:

column: Chiralpak AS (4.6 x 250 mm, Daicel Chemical Industries, Ltd.)
eluent: n-hexane-ethanol-TFA (700:300:1)
flow rate: 1.0 ml/minutes
detection: 220 nm
retention time: 50.0 minutes

Example 12

To a solution of 2-(5-bromo-2-thienyl)-3,4,5,6-tetrahydro-2H-thiopyran-2-acetic acid 1,1-dioxide (1 g) in ethanol (8 ml) was added (R)-(+)-α-methylbenzylamine (185 mg) at room temperature. After being stirred overnight at the room temperature, the resulting crystal was filtrated and washed with ethanol to give 2-(5-bromo-2-thienyl)-3,4,5,6-tetrahydro-2H-thiopyran-2-acetic acid 1,1-dioxide (R)-(+)-α-methylbenzylamine salt. A suspension of resulting salt in ethyl acetate was washed with 1N hydrochloric acid and brine, and concentrated to give the free acid. This procedure was repeated two times to give the optically resoluted (2R or 2S)-2-(5-bromo-2-thienyl)-3,4,5,6-tetrahydro-2H-thiopyran-2-acetic acid 1,1-dioxide (300 mg) as a white solid.

NMR (DMSO-$d_6$, δ): 1.74-1.87 (4H, m), 2.30-2.37 (1H, m), 3.07-3.56 (5H, m), 7.02 (1H, d, J=4.2Hz), 7.21 (1H, d, J=4.2Hz)

MS (ESI-) m/z: 351 (M-H)

$[\alpha]_D^{25}$: -25.3° (C=1.0, MeOH)

Optical purity: 95% ee

Analytical chiral HPLC:

column: Chiralpak AS (4.6 x 250 mm, Daicel Chemical Industries, Ltd.)
eluent: n-hexane-ethanol-trifluoroacetic acid (TFA) (700:300:1)
flow rate: 1.0 ml/minute
detection: 220 nm
retention time: 20.2 minutes

Example 13

A mixture of 2-[4-(4-chlorophenoxy)phenyl]-3,4,5,6-tetrahydro-2H-thiopyran-2-acetic acid 1,1-dioxide (98.7 mg), ammonium formate (78.8 mg), and 10% palladium - carbon (50% wet, 60 mg) in ethanol (5 ml) was stirred under reflux for 3 hours and 20 minutes, and filtered. The filtrate was evaporated in vacuo and the residue was partitioned between ethyl acetate and 0.1N hydrochloric acid. The organic layer was washed with brine, dried over sodium sulfate, and evaporated in vacuo. The residue was powdered from diisopropyl ether to afford 3,4,5,6-tetrahydro-2-(4-phenoxyphenyl)-2H-thiopyran-2-acetic acid 1,1-dioxide (87 mg) as a colorless powder.

mp: 208.5-209.5°C

IR (KBr): 2750-2550, 1707, 1290, 1246, 1124 $cm^{-1}$

NMR ($CDCl_3$, δ): 1.75-2.25 (4H, m), 2.74 (2H, m), 3.11 (2H, m), 3.21 (1H, d, J=15.6Hz), 3.61 (1H, d, J=15.6Hz), 6.97-7.07 (4H, m), 7.14 (1H, t, J=7.4Hz), 7.36 (2H, t, J=7.7Hz), 7.59 (2H, d, J=9.0Hz)

(-) API-ES MS m/z: 359 ($M^+$-H)

Example 14

A solution of oxalyl chloride (76.2 mg) in dichloromethane (0.7 ml) was added dropwise to a stirred suspension of 2-[4-(4-chlorophenoxy)phenyl]-3,4,5,6-tetrahydro-2H-thiopyran-2-acetic acid 1,1-dioxide (118 mg) and N,N-dimethylformamide (1.10 mg) in dichloromethane (1.2 ml) under ice cooling and a nitrogen atmosphere, then the resulting mixture was stirred at room temperature under a nitrogen atmosphere for 2 hours and evaporated in vacuo. The residue was dissolved in dichloromethane (1.6 ml) and the solution was added dropwise to a stirred mixture of hydroxylammonium chloride (125 mg), 1N aqueous solution of sodium hydroxide (1.8 ml), tetrahydrofuran (3. 6 ml), and tert-butyl alcohol (1.8 ml) at room temperature and the resulting mixture was stirred at room temperature for 2 hours and 30 minutes. The reaction mixture was partitioned between water and ethyl acetate. The organic layer was separated, dried over sodium sulfate, and evaporated in vacuo. The residue was chromatographed (eluent: toluene - ethyl acetate - acetic acid) over silica gel (2.6 g) to afford a colorless powder, which was washed with diisopropyl ether to afford 2-[4-(4-chlorophenoxy)phenyl]-3,4,5,6-tetrahydro-N-hydroxy-2H-thiopyran-2-acetamide 1,1-dioxide (88 mg) as a

colorless powder.

mp: 179-180°C (dec.)

IR (KBr): 3421, 3315, 3220, 1652, 1284, 1248, 1119 $cm^{-1}$

NMR (DMSO-$d_6$, δ): 1.74-1.99 (4H, m), 2.5 (1H, m), 2.87 (1H, br d, J=13.8Hz), 3.01-3.55 (4H, m), 6.87-7.12 (4H, m), 7.42-7.59 (4H, m), 8.73 (1H, s), 10.48 (1H, s)

(+) API-ES MS m/z: 432 ($M^+$+Na)

| | | | |
|---|---|---|---|
| Anal. Calcd. for $C_{19}H_{20}ClNO_5S$ | C 55.68, | H 4.92, | N 3.42 |
| Found | C 55.67, | H 5.28, | N 3.23 |

Example 15

A solution of oxalyl chloride (55.8 mg) in dichloromethane (0.5 ml) was added dropwise to a stirred solution of crude (2R or 2S)-[4-(4-chlorophenoxy)phenyl]-3,4,5,6-tetrahydro-2H-thiopyran-2-acetic acid 1,1-dioxide (an optical isomer A, 87 mg) obtained in Example 11 and N,N-dimethylformamide (0.80 mg) in dichloromethane (0.88 ml) under ice cooling and a nitrogen atmosphere, then the resulting mixture was stirred at room temperature under a nitrogen atmosphere for 2 hours and 30 minutes, and evaporated in vacuo. The residue was dissolved in dichloromethane (0.9 ml) and the solution was added dropwise to a stirred mixture of hydroxylammonium chloride (91.7 mg), 1N aqueous solution of sodium hydroxide (1.3 ml), tetrahydrofuran (2.6 ml), and tert-butyl alcohol (1.3 ml) at room temperature and the resulting mixture was stirred at the same temperature for 1 hour and 30 minutes. The reaction mixture was partitioned between water and ethyl acetate. The organic layer was separated, dried over sodium sulfate, and evaporated in vacuo. The residue was chromatographed (eluent: toluene - ethyl acetate - acetic acid) over silica gel (1.9 g) to afford a pale brown powder (67 mg), which was washed with diisopropyl ether to afford (R or S)-(-)-2-[4-(4-chlorophenoxy)phenyl]-3,4,5,6-tetrahydro-N-hydroxy-2H-thiopyran-2-acetamide 1,1-dioxide (optical isomer A, 55 mg) as a colorless powder.

mp: 183.5-187°C (dec.)

$[\alpha]_D^{28}$ : -9.3° (C=0.71, MeOH)

IR (KBr) : 3446, 3423, 1653, 1284, 1250, 1119 $cm^{-1}$

NMR (DMSO-$d_6$, δ): 1.75-2.05 (4H, m), 2.5 (1H, m), 2.87 (1H, br d, J=12.9Hz), 3.01-3.5 (4H, m), 7.00 (2H, d, J=8.9Hz), 7.08 (2H, d, J=8.9Hz), 7.46 (2H, d, J=8.9Hz), 7.56 (2H, d, J=8.9Hz), 8.73 (1H, s), 10.48 (1H, s)

(+) API-ES MS m/z: 432 and 434 ($M^+$+Na)

(-) API-ES MS m/z: 408 and 410 ($M^+$-H)

Analytical chiral HPLC:

column: Chiralpak AS (4.6 x 250 mm,
Daicel Chemical Industries, Ltd.)
eluent: n-hexane-ethanol-TFA (700:300:1)
flow rate: 1.0 ml/minute
detection: 220 nm
retention time: 18.2 minutes

Example 16

(R or S)-(+)-2-[4-(4-Chlorophenoxy)phenyl]-3,4,5,6-tetrahydro-N-hydroxy-2H-thiopyran-2-acetamide 1,1-dioxide (41 mg) was prepared from crude (2R or 2S)-2-[4-(4-chlorophenoxy)phenyl]-3,4,5,6-tetrahydro-2H-thiopyran-2-acetic acid 1,1-dioxide (an optical isomer B, 87 mg) obtained in Example 11 in a similar manner to that of Example 15.

mp: 187-188°C (dec.)

$[\alpha]_D^{28}$: 10.5° (C=0.56, MeOH)

IR (KBr): 3444, 3423, 3317, 3224, 1655, 1284, 1250, 1122 $cm^{-1}$

NMR (DMSO-$d_6$, δ): 1.75-2.05 (4H, m), 2.5 (1H, m), 2.87 (1H, br d, J=13.6Hz), 3.01-3.52 (4H, m), 7.00 (2H, d, J=8.9Hz), 7.08 (2H, d, J=8.9Hz), 7.46 (2H, d, J=8.9Hz), 7.56 (2H, d, J=8.9Hz), 8.73 (1H, s), 10.48 (1H, s)

(+) API-ES MS m/z: 432 and 434 ($M^+$+Na)

(-) API-ES MS m/z: 408 and 410 ($M^+$-H)

Analytical chiral HPLC:

column: Chiralpak AS (4.6 x 250 mm,
Daicel Chemical Industries, Ltd.)

eluent: n-hexane-ethanol-TFA (700:300:1)
flow rate: 1.0 ml/minute
detection: 220 nm
retention time: 27.9 minutes

Example 17

2-[4-(4-Chlorophenoxy)phenyl]-3,4,5,6-tetrahydro-N-hydroxy-6-methyl-2H-thiopyran-2-acetamide 1,1-dioxide (30 mg) was prepared from 2-[4-(4-chlorophenoxy)phenyl]-3,4,5,6-tetrahydro-6-methyl-2H-thiopyran-2-acetic acid 1,1-dioxide (55 mg) in a similar manner to that of Example 14.

mp: 102°C (dec.)

IR (KBr): 3446 and 3421 (br), 1668, 1282, 1246, 1124 $cm^{-1}$

NMR (DMSO-$d_6$, δ): 1.17 (3H, d, J=6.6Hz), 1.5-2.05 (4H, m), 2.4 (1H, m), 2.8-2.95 (1H, br d), 3.09 (1H, d, J=15.0Hz), 3.23 (1H, d, J=15.0Hz), 3.54 (1H, m), 7.00 (2H, d, J=8.8Hz), 7.08 (2H, d, J=8.9Hz), 7.42-7.49 (2H, m), 7.56 (2H, d, J=8.9Hz), 8.73 (1H, s), 10.49 (1H, s)

(+) API-ES MS m/z: 446 and 448 ($M^+$+Na)

Example 18

3,9,5,6-Tetrahydro-N-hydroxy-2-(4-phenoxyphenyl)-2H-thiopyran-2-acetamide 1,1-dioxide (41 mg) was prepared from 3,4,5,6-tetrahydro-2-(4-phenoxyphenyl)-2H-thiopyrin-2-acetic acid 1,1-dioxide (66 mg) in a similar manner to that of Example 14.

mp: 182-183°C (dec.)

IR (KBr): 3446 and 3423 (br), 1655, 1288, 1246, 1115 $cm^{-1}$

NMR (DMSO-$d_6$, δ): 1.75-2.05 (4H, m), 2.4 (1H, m), 2.87 (1H, br d, J=13.0Hz), 3.04-3.55 (4H, m), 6.96 (2H, d, J=8.9Hz), 7.06 (2H, d, J=7.5Hz), 7.19 (1H, t, J=7.3Hz), 7.43 (2H, t, J=7.4Hz), 7.54 (2H, d, J=8.9Hz), 8.74 (1H, s), 10.48 (1H, s)

(+) APCI MS m/z: 376 ($M^+$+H), 343 ($M^+$-NHOH)

(-) API-ES MS m/z: 374 ($M^+$-H)

Example 19

2-[4-(4-Chlorophenoxy)phenyl]-2,3,4,5-tetrahydro-N-hydroxy-2-thiophene-2-acetamide 1,1-dioxide (88 mg) was prepared from 2-[4-(4-chlorophenoxy)phenyl]-2,3,4,5-tetrahydrothiophene-2-acetic acid 1,1-dioxide (122 mg) in a similar manner to that of Example 14.

mp: 63-68°C (dec.)

IR (KBr): 3423 (br), 1662, 1296, 1244, 1126 $cm^{-1}$

NMR (DMSO-$d_6$, δ): 2.19-2.23 (2H, m), 2.56-3.28 (6H, m), 6.99-7.10 (4H, m), 7.38-7.48 (4H, m), 8.73 (1H, s), 10.40 (1H, s)

(+) APCI MS m/z: 396 and 398 ($M^+$+H),
363 and 365 ($M^+$-NHOH)

| | | | |
|---|---|---|---|
| Anal. Calcd. for $C_{18}H_{18}ClNO_5S$ | C 54.61, | H 4.58, | N 3.54 |
| Found | C 55.20, | H 5.06, | N 3.26 |

Example 20

To a solution of potassium hydroxide (400 g) in water (200 ml) was added a solution of ethyl 7-amidinothio-3-[4-(4-chlorophenoxy)phenyl]hept-2-enoate hydroiodide (70.1 g) in tetrahydrofuran (100 ml), and the mixture was refluxed overnight. After the solution was cooled and acidified with 1N HCl, the mixture was extracted with ethyl acetate (100 ml x 3). The combined organic layer was washed with water and brine, dried over magnesium sulfate and concentrated under reduced pressure to give 2-[4-(4-chloxophenoxy)phenyl]-3,4,5,6-tetrahydro-2H-thiopyran-2-acetic acid (60 g) as an yellow oil.

NMR ($CDCl_3$, δ): 1.51-1.90 (4H, m), 2.26-2.38 (1H, m), 2.48-2.68 (3H, m), 2.90 (2H, d, J=14Hz), 2.98 (2H, d, J=14Hz), 6.94 (4H, d, J=8Hz), 7.27 (2H, d, J=8Hz), 7.57 (2H, d, J=8Hz)

MS (ES-) m/z: 361 (M-H)

Example 21

2-[4-(4-Bromophenoxy)phenyl]-3,4,5,6-tetrahydro-2H-thiopyran-2-acetic acid (3.99 g) was obtained in a similar manner to that of Example 20.

NMR ($CDCl_3$, δ): 1.50-1.87 (4H, m), 2.27-2.49 (1H, m), 2.47-2.70 (3H, m), 2.90 (1H, d, J=15Hz), 3.00 (1H, d, J=15Hz), 6.96 (4H, d, J=9Hz), 7.44 (4H, d, J=9Hz)

MS (ESI-) m/z: 407 (M-H)

Example 22

Ethyl 2-[5-(4-fluorophenyl)-2-thienyl]-3,4,5,6-tetrahydro-2H-thiopyran-2-acetate (117 mg) was obtained from 7-amidinothio-3-[5-(4-fluorophenyl)-2-thienyl]hept-2-enoate hydroiodide in a similar manner to that of Example 20.

NMR ($CDCl_3$, δ): 1.12 (3H, t, J=7Hz), 1.49-1.92 (4H, m), 2.18-2.30 (1H, m), 2.55-2.68 (2H, m), 2.73-2.82 (1H, m), 2.81 (1H, d, J=14Hz), 2.89 (1H, d, J=14Hz), 4.01 (2H, q, J=7Hz), 6.93-7.11 (4H, m), 7.49-7.56 (2H, m)

Example 23

To a solution of ethyl 3-(4-biphenylyl)-7-chlorohept-2-enoate (0.90 g) in acetone (15 ml) was added NaI (1.55 g) at 60°C. After being stirred overnight, the reaction mixture was cooled, concentrated in vacuo, diluted with water, and extracted with ether. The ether extract was washed with brine, dried over $MgSO_4$ and concentrated in vacuo. A mixture of this residue and thiourea (158 mg) in ethanol (EtOH) (15 ml) was refluxed with stirring for 24 hours. The resulting mixture was cooled and concentrated in vacuo to yield the isothiouronium salt. To a solution of potassium hydroxide (KOH) (1.74 g) in water was added this isothiouronium salt, and the mixture was refluxed with stirring for 8 hours. The reaction mixture was cooled at 0°C and was quenched by cautions dropwise addition of a solution of 50% aqueous sulfuric acid ($H_2SO_4$) until acidic. The mixture was extracted with ether, and the organic layers were washed with water and brine, dried over magnesium sulfate ($MgSO_4$) and concentrated in vacuo. The residue was purified by silica gel column chromatography (eluent: 5% methanol (MeOH) in chloroform ($CHCl_3$) to give 2-(4-biphenylyl)-3,4,5,6-tetrahydro-2H-thiopyran-2-acetic acid (335 mg) as an amorphous.

NMR ($CDCl_3$, δ): 1.60-1.85 (4H, m), 2.30-2.39 (1H, m), 2.50-2.70 (3H, m), 2.94 (1H, d, J=14.7Hz), 3.01 (1H, d, J=14.7Hz), 7.34 (1H, dd, J=7, 7Hz), 7.56-7.61 (4H, m), 7.68-7.71 (2H, m)

MS (ESI-) m/z: 311 (M-H)

Example 24

2-[4-(4-Fluorophenoxy)phenyl]-3,4,5,6-tetrahydro-2H-thiopyran-2-acetic acid (200 mg) was obtained in a similar manner to that of Example 23.

NMR ($CDCl_3$, δ): 1.60-1.84 (4H, m), 2.27-2.36 (1H, m), 2.50-2.67 (3H, m), 2.88 (1H, d, J=15Hz), 2.97 (1H, d, J=16Hz), 6.91 (2H, d, J=9Hz), 6.99-7.06 (4H, m), 7.56 (2H, d, J=9Hz)

MS (ESI-) m/z: 345 (M-H)

Example 25

2-[4-(4-Chlorophenyl)phenyl]-3,4,5,6-tetrahydro-2H-thiopyran-2-acetic acid (310 mg) was obtained in a similar manner to that of Example 23.

NMR ($CDCl_3$, δ): 1.60-1.85 (4H, m), 2.29-2.38 (1H, m), 2.52-2.69 (3H, m), 2.94 (1H, d, J=14.7Hz), 3.01 (1H, d, J=14.7Hz), 7.39 (2H, d, J=8Hz), 7.50-7.54 (4H, m), 7.69 (2H, d, J=8Hz)

MS (ESI-) m/z: 345 (M-H)

Example 26

2-[4-(4-Bromophenyl)phenyl]-3,4,5,6-tetrahydro-2H-thiopyran-2-acetic acid (120 mg) was obtained in a similar manner to that of Example 23.

NMR ($CDCl_3$, δ): 1.61-1.85 (4H, m), 2.29-2.36 (1H, m), 2.51-2.69 (3H, m), 2.94 (1H, d, J=14.7Hz), 3.01 (1H, d, J=14.7Hz), 7.45 (2H, d, J=9Hz), 7.51-7.57 (4H, m), 7.69 (2H, d, J=9Hz)

MS (ESI-) m/z: 389 (M-H)

Example 27

2-[4-(4-Fluorophenyl)phenyl]-3,4,5,6-tetrahydro-2H-thiopyran-2-acetic acid (200 mg) was obtained in a similar manner to that of Example 23.

NMR ($CDCl_3$, δ): 1.60-1.85 (4H, m), 2.29-2.38 (1H, m), 2.53-2.67 (2H, m), 2.94 (1H, d, J=15Hz), 3.01 (1H, d, J=16Hz), 7.07-7.12 (2H, m), 7.51-7.56 (4H, m) , 7.69 (2H, d, J=8.5Hz)

MS (ESI-) m/z: 329 (M-H)

Example 28

2-[5-(4-Chlorophenyl)-2-thienyl]-3,4,5,6-tetrahydro-2H-thiopyran-2-acetic acid (2.5 g) was obtained in a similar manner to that of Example 23.

NMR (DMSO-$d_6$, δ): 1.55-1.75 (4H, m), 2.25-2.32 (1H, m), 2.45-2.63 (3H, m), 2.74 (1H, d, J=14Hz), 3.97 (1H, d, J=14Hz), 7.02 (1H, d, J=3.6Hz), 7.40 (1H, d, J=3.6Hz), 7.45 (2H, d, J=8.7Hz), 7.64 (2H, d, J=8.7Hz)

MS (ESI-) m/z: 351 (M-H)

Example 29

2-(5-Bromo-2-thienyl)-3,4,5,6-tetrahydro-2H-thiopyran-2-acetic acid (8.5 g) was obtained in a similar manner to that of Example 23.

NMR (DMSO-$d_6$, δ): 1.45-1.73 (4H, m), 2.12-2.20 (1H, m), 2.45-2.70 (4H, m), 2.87 (1H, d, J=14.4Hz), 6.84 (1H, d, J=4.2Hz), 7.08 (1H, d, J=4.2Hz)

Example 30

1-Ethyl-3-(3-dimethylaminopropyl)carbodiimide (WSCD) hydrochloride (205 mg) was added to a stirred mixture of 2-[4-(4-chlorophenoxy)phenyl]-3,4,5,6-tetrahydro-2H-thiopyran-2-acetic acid 1,1-dioxide (326 mg), (R)-(+)-α-methylbenzylamine (105 mg), and 1-hydroxybenzotriazole (123 mg) in dichloromethane (8 ml) under ice cooling, and then the resulting mixture was stirred at the same temperature for 2 hours and at room temperature for 2 hours and extracted with dichloromethane. The extract was washed successively with water, 0.1N hydrochloric acid, water, and a saturated aqueous solution of sodium bicarbonate, dried over magnesium sulfate, and evaporated in vacuo. The residue was chromatographed (eluent: toluene - ethyl acetate) over silica gel to afford diastereomer A (180 mg) and diastereomer B (181 mg) of 2-[4-(4-chlorophenoxy)phenyl]-3,4,5,6-tetrahydro-2H-thiopyran-2-N-[(1R)-1-phenylethyl] acetamide 1,1-dioxide as a colorless solid and colorless crystals, respectively.

Diastereomer A:

mp: 138-160.5°C
$[\alpha]_D^{27}$: 31.0° (C=0.52, MeOH)
IR (KBr): 3421 (br), 1651, 1288, 1244, 1124 $cm^{-1}$
NMR ($CDCl_3$, δ): 1.06 (3H, d, J=6.8Hz), 1.94-2.15 (4H, m), 2.68-2.74 (2H, m), 2.99-3.26 (4H, m), 4.72-4.87 (1H, m), 5.21 (1H, br d, J=8.0Hz), 6.97 (2H, d, J=9.0Hz), 7.00-7.11 (4H, m), 7.19-7.37 (5H, m), 7.70 (2H, d, J=9.0Hz) (+) APCI MS m/z: 498 and 500 ($M^+$+H)

Diastereomer B:

mp: 82.5-89°C
$[\alpha]_D^{27}$: 53.4° (C=0.50, MeOH)
IR (KBr): 3365 (br), 1651, 1288, 1246, 1122 $cm^{-1}$
NMR ($CDCl_3$, δ): 1.32 (3H, d, J=6.9Hz), 1.91 (2H, m), 2.13 (2H, m), 2.62 (2H, m), 2.99-3.30 4H, m), 4.85 (1H, m), 5.31 (1H, br d, J=8.0Hz) 6.74-6.80 (2H, m), 6.90-6.99 (4H, m), 7.15-7.36 (5H, m), 7.58 (2H, d, J=9.0Hz)
(+) APCI MS m/z: 498 and 500 ($M^+$+H)

Example 31

To a solution of (2R or 2S)-2-[4-(4-chlorophenoxy)-phenyl]-3,4,5,6-tetrahydro-2H-thiopyran-2-acetic acid 1,1-dioxide (4.75 g) obtained in Example 10, 0-(2-tetrahydropyranyl)hydrdxylamine (2.11 g), and 1-hydroxybenzotriazole (1.95 g) in N,N-dimethylformamide (60 ml) was added WSCD hydrochioride (2.77 g). After being stirred for 4 hours

at room temperature, the solvent was evaporated in vacuo, and the resulting residue was dissolved in ethyl acetate (60 ml). The solution was washed with 5% aqueous citric acid solution, saturated sodium bicarbonate solution and brine, and dried over magnesium sulfate. The solution was concentrated under reduced pressure to give (2R or 2S) -N-(2-tetrahydropyranyloxy)-2-[4-(4-chlorophenoxy)-phenyl]-3,4,5,6-tetrahydro-2H-thiopyran-2-acetamide 1,1-dioxide (6.52 g) as a slightly yellow oil.

NMR ($CDCl_3$, δ): 1.46-2.22 (10H, m), 2.77 (1H, br), 3.02-3.28 (4H, m), 3.38-3.55 (1H, m), 3.65-3.78 (1H, m), 4.37 (0.5H, s), 4.77 (0.5H, s), 6.95 (2H, d, J=8Hz), 7.00 (2H, d, J=8Hz), 7.29 (2H, d, J=8Hz), 7.65 (2H, d, J=8Hz), 8.28 (1H, br)

MS (ESI-) m/z: 493 (M-H)

The following compounds were obtained in a similar manner to that of Example 31.

Example 32

2-[4-(4-Chlorophenoxy)phenyl]-3,9,5,6-tetrahydro-2H-thiopyran-2-acetamide (140 mg)

NMR ($CDCl_3$, δ): 1.42-1.91 (10H, m), 2.23-2.36 (1H, m), 2.49-2.72 (4H, m), 2.74-2.83 (1H, m), 3.45-3.58 (1H, m), 3.71-3.83 (1H, m), 4.55 (1H, s), 4.79 (0.5H, s), 6.89-7.03 (4H, m), 7.27 (2H, d, J=8Hz), 7.60 (2H, d, J=8Hz), 7.96 (0.5H, s), 8.16 (0.5H, s)

MS (ESI-) m/z: 460 (M-H)

Example 33

N-(2-Tetrahydropyranyloxy)-2-[4-(4-fluorophenoxy)-phenyl]-3,4,5,6-tetrahydro-2H-thiopyran-2-acetamide 1/1-dioxide (307 mg)

NMR ($CDCl_3$, δ): 1.53-1.70 (10H, m), 2.08-2.17 (2H, m), 2.73-2.76 (2H, m), 3.02-3.23 (4H, m), 3.42-3.52 (1H, m), 6.98-7.05 (6H, m), 7.64 (2H, d, J=9Hz)

MS (ESI-) m/z: 476 (M-H)

Example 34

N-(2-Tetrahydropyranyloxy)-2-[4-(4-fluorophenoxy)-phenyl]-3,4,5,6-tetrahydro-2H-thiopyran-2-acetamide (250 mg)

NMR ($CDCl_3$, δ): 1.50-1.64 (6H, m), 1.74-1.78 (4H, m), 2.25-2.34 (2H, m), 2.52-2.70 (4H, m), 2.76-2.82 (1H, m), 3.49-3.57 (1H, m), 3.72-3.83 (1H, m) , 6.95-7.04 (6H, m), 7.59 (2H, d, J=9Hz)

MS (ESI-) m/z: 444 (M-H)

Example 35

N-(2-Tetrahydropyranyloxy)-2-[4-(4-fluorophenoxy)-phenyl]-3,4,5,6-tetrahydro-2H-thiopyran-2-acetamide 1-oxide (152 mg)

NMR ($CDCl_3$, δ): 1.54-1.81 (10H, m), 2.45-2.56 (2H, m), 2.75-2.96 (5H, m), 3.53-3.59 (1H, m), 3.81-3.87 (1H, m), 6.95-7.04 (6H, m), 7.39 (2H, d, J=9Hz)

MS (ESI-) m/z: 460 (M-H)

Example 36

N-(2-Tetrahydropyranyloxy)-2-[4-(4-fluorophenoxy)-phenyl]-3,4,5,6-tetrahydro-2H-thiopyran-2-acetamide 1,1-dioxide (173 mg)

NMR ($CDCl_3$, δ): 1.45-1.75 (6H, m), 1.89-2.01 (2H, m), 2.06-2.20 (2H, m), 2.68-2.80 (2H, m), 3.00-3.24 (4H, m), 3.40-3.55 (1H, m), 3.64-3.75 (1H, m), 4.49 (0.5H, br s), 4.75 (0.5H, br s), 6.93 (2H, d, J=9Hz), 7.04 (2H, d, J=9Hz), 7.45 (2H, d, J=9Hz), 7.67 (2H, d, J=9Hz), 7.77 (2H, d, J=9Hz), 7.77 (0.5H, br s), 7.90 (0.5H, br s)

MS (ESI-) m/z: 536 (M-H)

Example 37

N-(2-Tetrahydropyranyloxy)-2-[4-[4-(4-fluorophenyl)-phenoxy]phenyl]-3,4,5,6-tetrahydro-2H-thiopyran-2-acetamide 1,1-dioxide (122 mg)

NMR ($CDCl_3$, δ): 1.45-1.79 (6H, m), 1.88-2.02 (2H, m), 2.06-2.24 (2H, m), 2.66-2.82 (2H, m), 3.00-3.25 (4H, m), 3.40-3.56 (1H, m), 3.64-3.77 (1H, m), 4.41 (0.5H, br s), 4.75 (0.5H, br s), 7.00-7.18 (6H, m), 7.42-7.56 (4H, m), 7.67 (2H, d, J=9Hz), 7.75 (0.5H, br s), 7.89 (0.5H, br s)

MS (ESI-) m/z: 552 (M-H)

Example 38

N-(2-Tetrahydropyranyloxy)-2-(4-methoxyphenyl)-3,4,5,6-tetrahydro-2H-thiopyran-2-acetamide 1,1-dioxide (113 mg)

NMR ($CDCl_3$, δ): 1.41-1.72 (6H, m), 1.87-2.01 (2H, m), 2.06-2.21 (2H, m), 2.59-2.81 (2H, m), 3.00-3.25 (4H, m), 3.32-3.73 (2H, m), 3.81 (3H, s), 4.37-4.44 (0.5H, m), 4.69-4.79 (0.5H, m), 6.91-7.03 (2H, m), 7.53-7.69 (2H, m), 7.70-7.91 (1H, m)

MS (ESI-) m/z: 396 (M-H)

Example 39

N-(2-Tetrahydropyranyloxy)-2-[5-(4-fluorophenyl)-2-thienyl]-3,4,5,6-tetrahydro-2H-thiopyran-2-acetamide (145 mg)

NMR ($CDCl_3$, δ): 1.45-1.99 (10H, m), 2.17-2.32 (1H, m), 2.50-2.97 (3H, m), 3.40-3.51 (1H, m), 3.71-3.86 (1H, m), 4.70 (0.5H, s), 4.86 (0.5H, s), 6.93-7.19 (4H, m), 7.52-7.67 (2H, m), 8.09 (0.5H, s), 8.22 (0.5H, s)

MS (ESI-) m/z: 434 (M-H)

Example 40

N-(2-Tetrahydropyranyloxy)-2-[5-(4-fluorophenyl)-2-thienyl]-3,4,5,6-tetrahydro-2H-thiopyran-2-acetamide 1,1-dioxide (345 mg)

NMR ($CDCl_3$, δ): 1.39-1.79 (6H, m), 1.89-2.00 (2H, m), 2.05-2.27 (2H, m), 2.64-2.92 (2H, m), 3.06 (1H, s), 3.09-3.16 (1H, m), 3.27-3.50 (1H, m), 3.61-3.73 (1H, m), 4.53 (0.5H, br s), 4.82 (1H, br s), 7.02-7.11 (2H, m), 7.16-7.27 (2H, m), 7.55 (1H, d, J=8Hz), 7.57 (1H, d, J=8Hz), 7.97 (0.5H, s), 8.13 (1H, br s)

Example 41

N-(2-Tetrahydropyranyloxy)-2-(4-biphenylyl)-3,4,5,6-tetrahydro-2H-thiopyran-2-acetamide (330 mg)

NMR ($CDCl_3$, δ): 1.45-1.81 (10H, m), 2.29-2.38 (1H, m), 2.52-2.96 (7H, m), 3.58-3.75 (1H, m), 7.35-7.37 (1H, m), 7.42-7.47 (2H, m), 7.56-7.64 (4H, m), 7.71-7.75 (2H, m)

MS (ESI-) m/z: 410 (M-H)

Example 42

N-(2-Tetrahydropyranyloxy)-2-(4-biphenylyl)-3,4,5,6-tetrahydro-2H-thiopyran-2-acetamide 1,1-dioxide (390 mg)

NMR ($CDCl_3$, δ): 1.38-1.66 (6H, m), 1.95-2.03 (2H, m), 2.12-2.21 (2H, m), 2.73-2.84 (2H, m), 2.89 (1H, s), 2.96 (1H, s), 3.04-3.31 (4H, m), 3.50-3.62 (1H, m), 7.35-7.47 (3H, m), 7.58-7.60 (2H, m), 7.65-7.70 (2H, m), 7.77-7.81 (2H, m)

MS (ESI-) m/z: 442 (M-H)

Example 43

N-(2-Tetrahydropyranyloxy)-2-[4-(4-chlorophenyl)phenyl]-3,4,5,6-tetrahydro-2H-thiopyran-2-acetamide 1,1-dioxide (300 mg)

NMR ($CDCl_3$, δ): 1.60-1.66 (6H, m), 1.94-2.03 (2H, m), 2.13-2.21 (2H, m), 2.76-2.84 (2H, m), 2.89-2.96 (2H, m), 3.07-3.27 (4H, m), 3.53-3.65 (1H, m), 7.41 (2H, d, J=8.5Hz), 7.51 (2H, d, J=8.5Hz), 7.60-7.64 (2H, m), 7.76-7.80 (2H, m)

MS (ESI-) m/z: 476 (M-H)

Example 44

N-(2-Tetrahydropyranyloxy)-2-[4-(4-bromophenyl)phenyl]-3,4,5,6-tetrahydro-2H-thiopyran-2-acetamide 1,1-dioxide (100 mg)

NMR ($CDCl_3$, δ): 1.44-1.62 (10H, m), 2.10-2.20 (2H, m), 2.76-2.84 (2H, m), 3.05-3.31 (4H, m), 3.53-3.65 (1H, m), 7.45 (2H, d, J=9Hz), 7.56-7.64 (4H, m), 7.70-7.77 (2H, m)

MS (ESI-) m/z: 520 (M-H)

Example 45

N-(2-Tetrahydropyranyloxy)-2-[4-(4-fluorophenyl)phenyl]-3,4,5,6-tetrahydro-2H-thiopyran-2-acetamide 1,1-dioxide (230 mg)

NMR ($CDCl_3$, δ): 1.45-1.70 (10H, m), 2.12-2.19 (2H, m), 2.79-2.81 (2H, m), 3.05-3.32 (4H, m), 3.55-3.64 (1H, m), 7.13 (2H, dd, J=9, 9Hz), 7.52-7.61 (4H, m), 7.73-7.78 (2H, m)

MS (ESI-) m/z: 460 (M-H)

Example 46

N-(2-Tetrahydropyranyloxy)-2-(4-phenoxybenzyl)-3,4,5,6-tetrahydro-2H-thiopyran-2-carboxamide 1,1-dioxide (360 mg)

NMR ($CDCl_3$, δ): 1.45-2.31 (12H, m), 2.42-2.63 (1H, m), 3.03-3.20 (3H, m), 3.43 (1H, d, J=14Hz), 3.57-3.70 (1H, m), 3.84-4.02 (1H, m), 4.91, 5.11 (1H, s), 6.91 (2H, d, J=8Hz), 6.97-7.14 (3H, m), 7.19 (2H, t, J=8Hz), 7.28-7.48 (2H, m), 9.99, 10.07 (1H, s)

MS (ESI-) m/z: 458 (M-H)

Example 47

N-(2-Tetrahydropyranyloxy)-2-(4-phenoxybenzyl)-3,4,5,6-tetrahydro-2H-thiopyran-2-carboxamide (320 mg)

NMR ($CDCl_3$, δ): 1.33-2.00 (12H, m), 2.47-2.59 (1H, m), 2.64-2.90 (3H, m), 3.08-3.22 (1H, m), 3.50-3.65 (1H, m), 3.75-4.00 (1H, m), 4.70, 4.98 (1H, s), 6.87-7.20 (7H, m), 7.32 (2H, t, J=8Hz), 9.70 (1H, s)

MS (ESI-) m/z: 426 (M-H)

Example 48

N-(2-Tetrahydropyranyloxy)-2-(4-biphenylylmethyl)-3,4,5,6-tetrahydro-2H-thiopyran-2-carboxamide 1,1-dioxide (152 mg)

NMR ($CDCl_3$, δ): 1.54-2.35 (12H, m), 2.45-2.66 (1H, m), 3.08-3.25 (3H, m), 3.33-3.55 (1H, m), 3.56-3.70 (1H, m), 3.82-4.08 (1H, m), 4.95, 5.14 (1H, s), 7.23-7.36 (3H, m), 7.42 (2H, t, J=8Hz), 7.47-7.67 (4H, m), 10.01, 10.08 (1H, s)

MS (ESI-) m/z: 442 (M-H)

Example 49

N-(2-Tetrahydropyranyloxy)-2-[5-(4-chlorophenyl)-2-thienyl]-3,4,5,6-tetrahydro-2H-thiopyran-2-acetamide 1,1-dioxide (250 mg)

NMR ($CDCl_3$, δ): 1.45-1.67 (10H, m) , 1.90-1.97 (2H, m), 2.67-3.12 (6H, m), 3.62-3.68 (1H, m), 7.24-7.26 (2H, m), 7.35 (2H, d, J=8.7Hz), 7.52 (2H, d, J=8.7Hz)

MS (ESI-) m/z: 482 (M-H)

Example 50

N-(2-Tetrahydropyranyloxy)-2-(5-bromo-2-thienyl)-3,4,5,6-tetrahydro-2H-thiopyran-2-acetamide 1,1-dioxide (190 mg)

NMR ($CDCl_3$, δ): 1.50-2.17 (11H, m), 2.65-3.10 (6H, m) , 3.47-3.58 (1H, m), 3.72-3.81 (1H, m), 7.00-7.05 (2H, m)

MS (ESI-) m/z: 450 (M-H)

Example 51

(2R or 2S)-N-(2-Tetrahydropyranyloxy)-2-(5-bromo-2-thienyl)-3,4,5,6-tetrahydro-2H-thiopyran-2-acetamide 1,1-dioxide (80 mg) from (2R or 2S) 2-(5-bromo-2-thienyl)-3,4,5,6-tetrahydro-2H-thiopyran-2-acetic acid 1,1-dioxide (63 mg) obtained in Example 12

NMR ($CDCl_3$, δ): 1.50-2.17 (11H, m), 2.65-3.10 (6H, m), 3.47-3.58 (1H, m), 3.72-3.81 (1H, m), 7.00-7.05 (2H, m)

MS (ESI-) m/z: 450 (M-H)

Example 52

To a mixture of (2R or 2S)-N-(2-tetrahydropyranyloxy)-2-[4-(4-chlorophenoxy)phenyl]-3,4,5,6-tetrahydro-2H-thiopyran-2-acetamide 1,1-dioxide (6.51 g) obtaihed in Example 31 in methanol (40 ml) was added 10% hydrogen chloride in methanol (10 ml) at room temperature. After being stirred for 30 minutes, the solution was concentrated.

The residue was purified with silica gel column chromatography (eluent: hexane-EtOAc 1:1) to give (2R or 2S)-(-)-N-hydroxy-2-[4-(4-chlorophenoxy)phenyl]-3,4,5,6-tetrahydro-2H-thiopyran-2-acetamide 1,1-dioxide (optical isomer A, 4.72 g) as white crystalline.

$[\alpha]_D^{25}$: -13.7°C (C=0.98, MeOH)

Analytical chiral HPLC:

column: Chiralpak AS (4.6 x 250 mm, Daicel Chemical Industries, Ltd.)
eluent: n-hexane-ethanol-TFA (700:300:1)
flow rate: 1.0 ml/minute
detection: 220 nm
retention time: 18.2 minutes

The following compounds were obtained in a similar manner to that of Example 52.

Example 53

N-Hydroxy-2-[4-(4-chlorophenoxy)phenyl]-3,4,5,6-tetrahydro-2H-thiopyran-2-acetamide (61 mg)

NMR (DMSO-$d_6$, δ): 1.32-1.78 (4H, m), 2.30-2.58 (4H, m), 2.60-2.73 (2H, m), 6.96-7.08 (4H, m), 7.43 (2H, d, J=8Hz), 7.55 (2H, d, J=8Hz), 10.19 (1H, s)

MS (ESI-) m/z: 376 (M-H)

Example 54

N-Hydroxy-2-[4-(4-fluorophenoxy)phenyl]-3,4,5,6-tetrahydro-2H-thiopyran-2-acetamide 1,1-dioxide (220 mg)

NMR (DMSO-$d_6$, δ): 1.75-2.03 (4H, m), 2.99 (1H, d, J=14Hz), 3.19 (1H, d, J=14Hz), 3.34-3.50 (4H, m), 6.94 (2H, d, J=9Hz), 7.09-7.14 (2H, m), 7.23-7.29 (2H, m), 7.53 (2H, d, J=9Hz), 8.74 (1H, s)

MS (ESI-) m/z: 392 (M-H)

Example 55

N-Hydroxy-2-[4-(4-fluorophenoxy)phenyl]-3,4,5,6-tetrahydro-2H-thiopyran-2-acetamide (143 mg)

NMR (DMSO-$d_6$, δ): 1.46-1.73 (4H, m), 2.35-2.49 (4H, m), 2.64-2.71 (2H, m), 6.93 (2H, d, J=9Hz), 7.05-7.09 (2H, m), 7.20-7.26 (2H, m), 7.53 (2H, d, J=9Hz), 8.62 (1H, s), 10.18 (1H, s)

MS (ESI-) m/z: 360 (M-H)

Example 56

N-Hydroxy-2-[4-(4-fluorophenoxy)phenyl]-3,4,5,6-tetrahydro-2H-thiopyran-2-acetamide 1-oxide (80 mg)

NMR (DMSO-$d_6$, δ): 1.47-1.65 (4H, m), 1.95-2.02 (1H, m), 2.25-2.44 (3H, m), 2.57 (1H, d, J=14Hz), 2.70 (1H, d, J=14Hz), 6.96 (2H, d, J=9Hz), 7.07-7.12 (2H, m), 7.22-7.28 (2H, m), 7.40 (2H, d, J=9Hz), 8.64 (1H, s), 10.31 (1H, s)

MS (ESI-) m/z: 376 (M-H)

Example 57

N-Hydroxy-2-[4-(4-bromophenoxy)phenyl]-3,4,5,6-tetrahydro-2H-thiopyran-2-acetamide 1,1-dioxide (104 mg)

NMR ($CDCl_3$, δ): 1.86-1.97 (2H, m), 2.02-2.23 (2H, m), 2.60-2.80 (2H, m), 3.01-3.24 (4H, m), 6.93 (2H, d, J=9Hz), 7.01 (2H, d, J=9Hz), 7.46 (2H, d, J=9Hz), 7.64 (2H, d, J=9Hz)

MS (ESI-) m/z: 452 (M-H)

Example 58

N-Hydroxy-2-[4-[4[(4-fluorophenyl)phenoxy)phenyl]-3,4,5,6-tetrahydro-2H-thiopyran-2-acetamide 1,1-dioxide (63 mg)

NMR ($CDCl_3$, δ): 1.87-2.00 (2H, m), 2.04-2.25 (2H, m), 2.60-2.82 (2H, m), 3.01-3.25 (4H, m), 7.00-7.19 (6H, m), 7.47-7.57 (4H, m), 7.64 (2H, d, J=9Hz)

MS (ESI-) m/z: 468 (M-H)

Example 59

N-Hydroxy-2-(4-methoxyphenyl)-3,4,5,6-tetrahydro-2H-thiopyran-2-acetamide 1,1-dioxide (21 mg)

NMR (DMSO-$d_6$, δ): 1.70-2.05 (4H, m), 2.39-2.52 (1H, m) , 2.81-2.91 (1H, m) , 2.95-3.22 (4H, m), 3.26 (3H, s), 6.91 (2H, d, J=9Hz), 7.45 (2H, d, J=9Hz), 8.70 (1H, s), 10.46 (1H, s)

MS (ESI-) m/z: 312 (M-H)

Example 60

N-Hydroxy-2-[5-(4-fluorophenyl)-2-thienyl]-3,4,5,6-tetrahydro-2H-thiopyran-2-acetamide (87 mg)

NMR ($CDCl_3$, δ): 1.45-1.85 (4H, m), 2.35-2.74 (4H, m), 2.46 (1H, d, J=14Hz), 2.66 (1H, d, J=14Hz), 7.17-7.33 (3H, m), 7.45 (1H, d, J=3Hz), 7.64-7.77 (2H, m), 8.74 (1H, s)

MS (ESI-) m/z: 350 (M-H)

Example 61

N-Hydroxy-2-[5-(4-fluorophenyl)-2-thienyl]-3,4,5,6-tetrahydro-2H-thiopyran-2-acetamide 1,1-dioxide (286 mg)

NMR ($CDCl_3$, δ): 1.69-2.07 (4H, m), 2.35-2.48 (2H, m), 2.94-3.54 (4H, m), 7.17-7.33 (3H, m), 7.45 (1H, d, J=3Hz), 7.64-7.77 (2H, m), 8.74 (1H, s)

MS (ESI-) m/z: 382 (M-H)

Example 62

N-Hydroxy-2-(4-biphenylyl)-3,4,5,6-tetrahydxo-2H-thiopyran-2-acetamide (230 mg)

NMR (DMSO-$d_6$, δ): 1.61-1.79 (4H, m), 2.39-2.56 (2H, m), 2.65-2.75 (3H, m), 2.89 (1H, s), 7.36 (1H, dd, J=7, 7Hz), 7.47 (2H, dd, J=7, 7Hz), 7.64-7.69 (6H, m), 8.63 (1H, s)

MS (ESI-) m/z: 326 (M-H)

Example 63

N-Hydroxy-2-(4-biphenylyl)-3,4,5,6-tetrahydro-2H-thiopyran-2-acetamide 1,1-dioxide (230 mg)

NMR (DMSO-$d_6$, 6): 1.77-2.05 (4H, m), 2.49-2.511 (2H, m) , 3.19-3.41 (4H, m), 7.41 (1H, dd, J=7.5, 7.5Hz), 7.49 (2H, dd, J=7.5, 7.5Hz), 7.65-7.71 (6H, m), 8.74 (1H, s)

MS (ESI-) m/z: 358 (M-H)

Example 64

N-Hydroxy-2-[4-(4-chlorophenyl)phenyl]-3,4,5,6-tetrahydro-2H-thiopyran-2-acetamide 1,1-dioxide (150 mg)

NMR (DMSO-$d_6$, δ): 1.78-2.04 (4H, m), 2.91-3.10 (2H, m), 3.17-3.36 (4H, m), 7.54 (2H, d, J=9Hz), 7.61-7.69 (4H, m), 7.73 (2H, d, J=9Hz), 8.72 (1H, s)

Example 65

N-Hydroxy-2-[4-(4-bromophenyl)phenyl]-3,4,5,6-tetrahydro-2H-thiopyran-2-acetamide 1,1-dioxide (70 mg)

NMR (DMSO-$d_6$, δ): 1.77-2.06 (4H, m), 2.93-3.08 (2H, m), 3.19-3.36 (4H, m), 7.61-7.70 (8H, m), 8.74 (1H, s)

MS (ESI-) m/z: 436 (M-H)

Example 66

N-Hydroxy-2-[4-(4-fluorophenyl)phenyl)-3,4,5,6-tetrahydro-2H-thiopyran-2-acetamide 1,1-dioxide (160 mg)

NMR (DMSO-$d_6$, δ): 1.77-2.05 (4H, m), 2.93-3.54 (6H, m), 7.31 (2H, dd, J=9, 9Hz), 7.63-7.77 (6H, m), 8.73 (1H, s), 10.53 (1H, s)

MS (ESI-) m/z: 376 (M-H)

Example 67

N-Hydroxy-2-(4-phenoxybenzyl)-3,4,5,6-tetrahydro-2H-thiopyran-2-carboxamide 1,1-dioxide (199 mg)

NMR ($CDCl_3$, δ): 1.47-2.20 (5H, m), 2.46-2.62 (1H, m), 2.96-3.20 (3H, m), 3.43 (1H, d, J=14Hz), 6.89 (2H, d, J=8Hz), 6.98 (2H, d, J=8Hz), 7.05-7.18 (3H, m), 7.33 (2H, d, J=8Hz), 7.96 (1H, br s), 9.98 (1H, br s)

MS (ESI-) m/z: 374 (M-H)

Example 68

N-Hydroxy-2-(4-phenoxybenzyl)-3,4,5,6-tetrahydro-2H-thiopyran-2-carboxamide (203 mg)

mp: 124-126°C

NMR ($CDCl_3$, δ): 1.22-1.38 (1H, m), 1.45-1.66 (2H, m), 1.70-1.83 (1H, m), 1.88-1.98 (1H, m), 2.43-2.55 (1H, m), 2.60-2.75 (2H, m), 2.80 (1H, d, J=14Hz), 3.15 (1H, d, J=14Hz), 6.90 (2H, d, J=8Hz), 7.00 (2H, d, J=8Hz), 7.05-7.15 (3H, m), 7.33 (2H, t, J=8Hz), 9.47 (1H, s)

MS (ESI-) m/z: 342 (M-H)

Example 69

N-Hydroxy-2-(4-biphenylylmethyl)-3,4,5,6-tetrahydro-2H-thiopyran-2-carboxamide 1,1-dioxide (78 mg)

mp: 101-104°C

NMR (DMSO-$d_6$, δ): 1.58-2.18 (6H, m), 3.06-3.17 (1H, m), 3.39 (1H, d, J=14Hz), 3.49-3.64 (1H, m), 3.65 (1H, d, J=14Hz), 7.31 (2H, d, J=8Hz), 7.37 (1H, d, J=8Hz), 7.46 (2H, t, J=8Hz), 7.59 (2H, d, J=8Hz), 7.65 (2H, d, J=8Hz), 9.20 (1H, s)

MS (ESI-) m/z: 358 (M-H)

Example 70

N-Hydroxy-2-[5-(4-chlorophenyl)-2-thienyl]-3,4,5,6-tetrahydro-2H-thiopyran-2-acetamide 1,1-dioxide (200 mg)

NMR (DMSO-$d_6$, δ): 1.74-2.02 (4H, m), 2.96-3.52 (6H, m), 7.22 (1H, d, J=3.6Hz), 7.47-7.53 (3H, m), 7.67 (2H, d, J=8.7Hz), 8.85 (1H, s), 10.59 (1H, s)

MS (ESI-) m/z: 398 (M-H)

Example 71

N-Hydroxy-2-(5-bromo-2-thienyl)-3,4,5,6-tetxahydro-2H-thiopyran-2-acetamide 1,1-dioxide (140 mg)

NMR (DMSO-$d_6$, δ): 1.73-2.00 (4H, m), 2.26-2.33 (1H, m), 2.86-2.96 (2H, m), 3.11-3.23 (2H, m), 3.40-3.45 (1H, m), 7.03 (1H, d, J=3.9Hz), 7.22 (1H, d, J=3.9Hz), 8.86 (1H, s), 10.57 (1H, s)

MS (ESI-) m/z: 366 (M-H)

Example 72

(2R or 2S)-N-Hydroxy-2-(5-bromo-2-thienyl)-3,4,5,6-tetrahydro-2H-thiopyran-2-acetamide 1,1-dioxide (65 mg) from (2R or 2S)-N-(2-tetrahydropyranyloxy)-2-(5-bromo-2-thienyl)-3,4,5,6-tetrahydro-2H-thiopyran-2-acetamide 1,1-dioxide (80 mg) obtained in Example 53

$[\alpha]_D^{25}$: -17.1°C (C=0.485, DMF)

NMR (DMSO-$d_6$, δ): 1.73-2.00 (4H, m), 2.26-2.33 (1H, m), 2.86-2.96 (2H, m), 3.11-3.23 (2H, m), 3.40-3.45 (2H, m), 7.03 (1H, d, J=3.9Hz), 7.22 (1H, d, J=3.9Hz), 8.86 (1H, s), 10.57 (1H, s)

MS (ESI-) m/z: 366 (M-H)

Optical purity: 95% ee

Analytical chiral HPLC:

column: Chiralpak AS (4.6 x 250 mm,
Daicel Chemical Industries, Ltd.)
eluent: n-hexane-ethanol-TFA (700:300:1)
flow rate: 1.0 ml/minute
detection: 220 nm

The following compounds were obtained in a similar manner to that of Preparation 1-4).

Example 73

2-[4-(4-Fluorophenoxy)phenyl]-3,4,5,6-tetrahydro-2H-thiopyran-2-acetic acid 1,1-dioxide (450 mg)
NMR ($CDCl_3$, δ): 1.87-2.21 (4H, m), 2.67-2.85 (2H, m), 3.03-3.15 (2H, m), 3.21 (1H, d, J=16Hz), 3.62 (1H, d, J=16Hz), 6.94-7.04 (6H, m), 7.59 (2H, d, J=9Hz)
MS (ESI-) m/z: 377 (M-H)

Example 74

2-[4-(4-Bromophenoxy)phenyl]-3,4,5,6-tetrahydro-2H-thiopyran-2-acetic acid 1,1-dioxide
NMR ($CDCl_3$, δ): 1.75-2.02 (4H, m), 2.08-2.21 (2H, m), 2.63-2.85 (2H, m), 3.02-3.16 (2H, m), 3.21 (1H, d, J=16Hz), 3.60 (1H, d, J=16Hz), 6.92 (2H, d, J=9Hz), 7.00 (2H, d, J=9Hz), 7.46 (2H, d, J=9Hz), 7.60 (2H, d, J=9Hz)
MS (ESI-) m/z: 439 (M-H)

Example 75

2-(4-Methoxyphenyl)-3,4,5,6-tetrahydro-2H-thiopyran-2-acetic acid
NMR ($CDCl_3$, δ): 1.71-2.00 (4H, m), 2.07-2.20 (2H, m), 2.60-2.86 (2H, m), 2.99-3.14 (2H, m), 3.19 (1H, d, J=15.5Hz), 3.60 (1H, d, J=15.5Hz), 3.81 (3H, s), 6.82 (2H, d, J=9Hz), 7.56 (2H, d, J=9Hz)
MS (ESI-) m/z: 297 (M-H)

Example 76

2-[5-(4-Fluorophenyl)-2-thienyl]-3,4,5,6-tetrahydro-2H-thiopyran-2-acetic acid
NMR ($CDCl_3$, δ): 1.70-2.02 (2H, m), 2.09-2.241(2H, m), 2.67-2.86 (2H, m), 3.02-3.20 (2H, m), 3.19 (1H, d, J=15Hz), 3.46 (1H, d, J=15Hz), 7.06 (2H, dd, J=8Hz, 8Hz), 7.15 (1H, d, J=3Hz), 7.21 (1H, d, J=3Hz), 7.46-7.62 (2H, m)
MS (ESI-) m/z: 367 (M-H)

Example 77

2-(4-Biphenylyl)-3,4,5,6-tetrahydro-2H-thiopyran-2-acetic acid 1,1-dioxide (0.33 g)
NMR ($CDCl_3$, δ): 1.80-2.01 (2H, m), 2.11-2.19 (2H, m), 2.69-2.76 (1H, m), 2.82-2.92 (1H, m), 3.02-3.16 (2H, m), 3.24 (1H, d, J=15.6Hz), 3.67 (1H, d, J=15.6Hz), 7.37-7.46 (2H, m), 7.58-7.63 (4H, m), 7.71 (2H, d, J=9Hz)
MS (ESI-) m/z: 343 (M-H)

Example 78

2-[4-(4-Chlorophenyl)phenyl]-3,4,5,6-tetrahydro-2H-thiopyran-2-acetic acid 1,1-dioxide (275 mg)
NMR ($CDCl_3$, δ): 1.79-2.00 (2H, m), 2.10-2.18 (2H, m), 2.66-2.89 (2H, m), 3.02-3.14 (2H, m), 3.23 (1H, d, J=16Hz), 3.65 (1H, d, J=16Hz), 7.40 (2H, d, J=9Hz), 7.50 (2N, d, J=8.5Hz), 7.57 (2H, d, J=8.5Hz), 7.70 (2H, d, J=9Hz)
MS (ESI-) m/z: 377 (M-H)

Example 79

2-[4-(4-Bromophenyl)phenyl]-3,4,5,6-tetrahydro-2H-thiopyran-2-acetic acid 1,1-dioxide (105 mg)
NMR ($CDCl_3$, δ): 1.70-2.20 (4H, m), 2.72-2.92 (2H, m), 3.05-3.16 (2H, m), 3.25 (1H, d, J=16Hz), 3.67 (1H, d, J=16Hz), 7.44 (2H, d, J=9Hz), 7.55-7.58 (4H, m) 7.71 (2H, d, J=9Hz)
MS (ESI-) m/z: 421 (M-H)

Example 80

2-[4-(4-Fluorophenyl)phenyl]-3,4,5,6-tetrahydro-2H-thiopyran-2-acetic acid 1,1-dioxide (220 mg)
NMR ($CDCl_3$, δ): 1.80-2.18 (4H, m), 2.69-2.91 (1H, m), 3.04-3.16 (3H, m), 3.24 (1H, d, J=16Hz), 3.66 (1H, d, J=16Hz), 7.12 (2H, dd, J=9, 9Hz), 7.52-7.58 (4H m), 7.70 (2H, d, J=8.5Hz)
MS (ESI-) m/z: 361 (M-H)

Example 81

A mixture of 2-[4-(4-chlorophenoxy)phenyl]-3,4,5,6-tetrahydro-2H-thiopyran-2-acetic acid (56 g), potassium permanganate (48.8 g) and benzyltrimethylammonium chloride (2.87 g) in water-methylene chloride (2:1, 1.5 ℓ) was stirred for 3 hours at room temperature. After the reaction mixture was poured into saturated sodium sulfite solution (500 ml), the solution was acidified with 4N hydrochloric acid. The organic layer was separated and the aqueous layer was extracted with chloroform (400 ml x 2). The combined organic layer was washed with brine, dried over magnesium sulfate and concentrated in vacuo. The resulting residue was purified with silica gel column chromatography (eluent: chloroform-methanol 10:1) to give 2-[4-(4-chlorophenoxy)phenyl]-3,4,5,6-tetrahydro-2H-thiopyran-2-acetic acid 1,1-dioxide (17.2 g) as colorless crystal.

mp: 191-193°C

Example 82

To a solution of 2-[4-(4-fluorophenoxy)phenyl]-3,4,5,6-tetrahydro-2H-thiopyran-2-acetic acid (300 mg) in MeOH was added dropwise titanium (III) chloride (2.67 ml) (10 wt. % solution in hydrochloric acid) in MeOH and hydrogen peroxide (0.69 ml) (30% aqueous solution) at room temperature. After being stirred for 15 minutes, the reaction is stopped by adding water. The reaction mixture is extracted with EtOAc and the solution was washed with brine, dried over $MgSO_4$ and concentrated in vacuo. The residue was purifiedjby silica gel column chromatography (eluent: 5% MeOH in $CHCl_3$) to give 2-[4-(4-fluorophenoxy)phenyl]-3,4,5,6-tetrahydro-2H-thiopyran-2-acetic acid 1-oxide (150 mg) as an amorphous.

NMR ($CDCl_3$, δ): 1.55-1.76 (4H, m), 2.45-2.62 (4H, m), 3.05-3.07 (2H, m), 6.95-7.07 (6H, m), 7.39 (2H, d, J=9Hz)

MS (ESI-) m/z: 361 (M-H)

Example 83

2-[5-(4-Chlorophenyl)-2-thienyl]-3,4,5,6-tetrahydro-2H-thiopyran-2-acetic acid 1,1-dioxide (1.95 g) was obtained in a similar manner to that of Preparation 1-4).

NMR (DMSO-$d_6$, δ): 1.76-1.90 (4H, m), 3.16-3.55 (6H, m), 7.19 (1H, d, J=3.6Hz), 7.47-7.52 (3H, m), 7.68 (2H, d, J=8.4Hz)

Example 84

2-(5-Bromo-2-thienyl)-3,4,5,6-tetrahydro-2H-thiopyran-2-acetic acid 1,1-dioxide (6.0 g) was obtained in a similar manner to that of Preparation 1-4).

NMR (DMSO-$d_6$, δ): 1.74-1.87 (4H, m), 2.30-2.37 (1H, m), 3.07-3.56 (5H, m), 7.02 (1H, d, J=4.2Hz), 7.21 (1H, d, J=4.2Hz)

MS (ESI-) m/z: 351 (M-H)

Example 85

A mixture of 2-[4-(4-bromophenoxy)phenyl]-3,4,5,6-tetrahydro-2H-thiopyran-2-acetic acid 1,1-dioxide (130 mg), 4-fluorobenzeneboronic acid (49.7 mg) and tetrakis(triphenylphosphine)palladium(0) (3.4 mg) in a mixture of 1,2-dimethoxyethane (0.5 ml) and 2M aqueous sodium carbonate (0.5 ml) was refluxed for 6 hours. The mixture was acidified with 4N hydrochloric acid to pH 3 and extracted with ethyl acetate. The separated organic phase was washed with brine, dried over sodium sulfate and evaporated in vacuo to give 2-[4-[4-(4-fluorophenyl)phenoxy]phenyl]-3,4,5,6-tetrahydro-2H-thiopyran-2-acetic acid 1,1-dioxide (116 mg) as an oil.

NMR ($CDCl_3$, δ): 1.75-2.02 (4H, m), 2.08-2.21 (2H, m), 2.64-2.84 (2H, m), 3.01-3.17 (2H, m), 3.23 (1H, d, J=15Hz), 3.62 (1H, d, J=15Hz), 7.04 (2H, d, J=9Hz), 7.07-7.16 (4H, m), 7.41-7.56 (4H, m), 7.61 (2H, d, J=9Hz)

MS (ESI-) m/z: 453 (M-H)

Example 86

1.6M n-Butyl lithium in hexane (1.63 ml) was added dropwise to a solution of diisopropylamine (261 mg) in THF (10 ml) under ice-bath cooling and a nitrogen atmosphere. After being stirred under the same condition for 30 minutes, a solution of methyl 3,4,5,6-tetrahydro-2H-thiopyran-2-carboxylate 1,1-dioxide (450 mg) in THF (8 ml) was added therein and the mixture was stirred for 45 minutes under dry ice-acetone cooling. A solution of 4-phenoxybenzyl bromide (719 mg) in THF (8 ml) was added to this mixture under the same condition. After the mixture was stirred for

2 hours under the same temperature for 2 hours under ice-bath cooling and for 2 hours at room temperature, a saturated aqueous solution of ammonium chloride was added to the reaction mixture. The resulting mixture was extracted with AcOEt. The extract was washed with 5% hydrochloric acid, 1M sodium bicarbonate solution and brine, dried over magnesium sulfate, and concentrated in vacuo. The residue was purified by silica gel ($SiO_2$) column chromatography (eluent: hexane-AcOEt, 6:1) to give methyl 2-(4-phenoxybenzyl)-3,4,5,6-tetrahydro-2H-thiopyran-2-carboxylate 1,1-dioxide (745 mg) as an oil.

NMR ($CDCl_3$, δ): 1.68-1.92 (2H, m), 2.02-2.34 (4H, m), 3.10-3.30 (2H, m), 3.17 (1H, d, J=14Hz), 3.74 (1H, d, J=14Hz), 3.83 (3H, s), 6.91 (2H, d, J=8Hz), 7.00 (2H, d, J=8Hz), 7.06-7.18 (3H, m), 7.34 (2H, t, J=8Hz)

Example 87

Methyl 2-(4-phenoxybenzyl)-3,4,5,6-tetrahydro-2H-thiopyran-2-carboxylate (605 mg) was obtained in a similar manner to that of Example 86.

NMR ($CDCl_3$, δ): 1.46-1.82 (4H, m), 1.86-1.96 (1H, m), 2.28-2.40 (1H, m), 2.52-2.62 (1H, m), 2.69-2.80 (1H, m), 3.07 (2H, s), 3.71 (3H, s), 6.89 (2H, d, J=8Hz), 7.00 (2H, d, J=8Hz), 7.06-7.14 (3H, m), 7.33 (2H, t, J=8Hz)

Example 88

Methyl 2-(4-biphenylylmethyl)-3,4,5,6-tetrahydro-2H-thiopyran-2-carboxylate 1,1-dioxide (250 mg) was obtained in a similar manner to.that of Preparation 86.

NMR ($CDCl_3$, δ): 1.72-1.93 (2H, m), 2.06-2.20 (3H, m), 2.25-2.37 (1H, m), 3.12-3.30 (2H, m), 3.24 (1H, d, J=14Hz), 3.81 (1H, d, J=14Hz), 3.86 (3H, s), 7.23 (2H, d, J=8Hz), 7.30-7.38 (1H, m), 7.43 (2H, t, J=8Hz), 7.49-7.58 (4H, m)

Example 89

To a stirred solution of ethyl 3-hydroxy-3-(4-methoxyphenyl)-7-mercaptoheptanoate (3.00 g) in dichloromethane (20 ml) was added trifluoroacetic acid (1 ml) at room temperature under nitrogen. After 1 hour, the mixture was quenched by the addition of triethylamine (1 ml) with ice cooling and concentrated in vacuo. The residue was partitioned between ethyl acetate and saturated aqueous sodium bicarbonate. The separated organic phase was washed with brine, dried over sodium sulfate and evaporated in vacuo. The obtained oil was purified by column chromatography on silica gel (eluted with 5 to 10% ethyl acetate in n-hexane) to give ethyl 2-(4-methoxyphenyl)-3,4,5,6-tetrahydro-2H-thiopyran-2-acetate (2.216 g) as an oil.

NMR ($CDCl_3$, δ): 1.05 (3H, t, J=7Hz), 1.50-1.84 (5H, m), 2.25-2.36 (1H, m), 2.45-2.71 (2H, m), 2.79 (1H, d, J=14Hz), 2.91 (1H, d, J=14Hz), 3.81 (3H, s), 3.93 (2H, q, J=7Hz), 6.88 (2H, d, J=9Hz), 7.55 (2H, d, J=9Hz)

Example 90

A mixture of tert-Butyl 2-(5-bromo-2-thienyl)-3,4,5,6-tetrahydro-2H-thiopyran-2-acetate (199 g) and aqueous 90% trifluoroacetic acid (1.0 1) was stirred for 2 hours at room temperature. The mixture was diluted with water (1.5 1) and stirred for 1 hour with ice cooling. The separated solid was collected and washed with water (500 ml) to give 2-(5-bromo-2-thienyl)-3,4,5,6-tetrahydro-2H-thiopyran-2-acetic acid (166.2 g).

NMR (DMSO-$d_6$, δ): 1.45-1.73 (4H, m), 2.12-2.20 (1H, m), 2.45-2.70 (5H, m), 2.87 (1H, d, J=14.4Hz), 6.84 (1H, d, J=4.2Hz), 7.08 (1H, d, J=4.2Hz)

MS (ESI-): 319 (M-H)

Example 91

To a solution of (2S)-N-(2-tetrahydropyranyloxy)-2-[5-(3-(2-(tert-butyl)(diphenyl)silyloxy)ethylaminocarbonylamino)phenyl)-2-thienyl]-3,4,5,6-tetrahydro-2H-thiopyran-2-acetamide 1,1-dioxide (500 mg) in tetrahydrofuran (3 ml) was added 1M solution of tetrabutylammonium fluoride in tetrahydrofuran (1.0 ml) at 0°C and the reaction mixture was stirred at ambient temperature for 3 hours. The resulting mixture was extracted with ethyl acetate. The organic layer was washed with water, 10% aqueous citric acid solution, saturated aqueous sodium hydrogencarbonate and brine, dried over anhydrous magnesium sulfate and concentrated in vacuo. The residue was purified by flash column chromatography on silica gel 60 (elueht: 6% methanol-chloroform) to give (2S)-N-(2-tetrahydropyranyloxy)-2-[5-{3-((2-hydroxyethyl)-aminocarbonylamino)phenyl)-2-thienyl]-3,4,5,6-tetrahydro-2H-thiopyran-2-acetamide 1,1-dioxide (70 mg) as a white amorphous.

NMR ($CDCl_3$, δ): 1.46 (2H, br), 1.52-1.68 (4H, m), 1.94 (2H, br), 2.04-2.24 (2H, m), 2.67-2.88 (2H, m), 3.00-3.06 (2H, m), 3.11 (2H, m), 3.30-3.47 (2H, m), 3.72 (2H, td, J=7.0, 7.0Hz), 4.24 (2H, td, J=7.0, 7.0Hz), 4.52 (1/2H, br), 4.82

(1/2H, br), 6.80 (1H, s), 7.18-7.46 (6H, m), 7.60 (1H, s), 8.32 (1/2H, s), 8.46 (1/2H, s)

MS (ESI-): 550.5 (M-H)

Example 92

To a solution of 2-(5-bromo-2-thienyl)-3,4,5,6-tetrahydro-2H-thiopyran-2-acetic acid 1,1-dioxide (140.0 g) in a mixture of acetonitrile (560 ml) and ethandl (800 ml) was added a solution of R-(+)-α-methylbenzylamine (28.8 g) in ethanol (40 ml) at 50°C. After been allowed to cool to ambient temperature over the time of 2 hours, the mixture was stirred for 2 hours at ambient temperature and for 2 hours with ice cooling additionally. The separated solid was collected and washed with acetonitrile (140 ml) to give (2S)-2-(5-bromo-2-thienyl)-3,4,5,6-tetrahydro-2H-thiopyran-2-N-[(R)-1-phenylethyl]acetamide 1,1-dioxide (66.2 g).

The absolute configuration was determined by X ray crystallography analysis.

NMR (DMSO-$d_6$, δ): 1.35 (3H, d, J=6.6Hz), 1.60-2.04 (4H, m), 2.18-2.32 (1H, m), 2.86-3.10 (3H, m), 3.24 (1H, d, J=15.6Hz), 3.36-3.52 (2H, m), 4.15 (1H, q, J=6.6Hz), 6.96 (1H, d, J=4.2Hz), 7.14 (1H, d, J=4.2Hz), 7.24-7.43 (5H, m)

The following compounds were obtained in substantially the same manner as that of Example 52.

Example 93

(2S)-N-Hydroxy-2-[5-(3-methylphenyl)-2-thienyl]-3,4,5,6-tetrahydro-2H-thiopyran-2-acetamide 1,1-dioxide (120 mg)

NMR (DMSO-$d_6$, δ): 1.75-2.06 (4H, m), 2.35 (3H, s), 2.95-3.52 (6H, m), 7.13 (1H, d, J=7.5Hz), 7.20 (1H, d, J=5Hz), 7.30 (1H, dd, J=7.5, 7.5Hz), 7.42-7.45 (3H, m), 8.84 (1H, s)

MS (ESI-): 378 (M-H)

Example 94

(2S)-N-Hydroxy-2-[5-(4-methylphenyl)-2-thienyl]-3,4,5,6-tetrahydro-2H-thiopyran-2-acetamide 1,1-dioxide (82 mg)

NMR (DMSO-$d_6$, δ): 1.74-2.04 (4H, m), 2.32 (3H, s), 2.47-2.53 (1H, m), 2.95-3.25 (4H, m), 3.43-3.53 (1H, m), 7.16 (1H, d, J=3.0Hz), 7.23 (2H, d, J=7.0Hz), 7.40 (1H, d, J=3.0Hz), 7.53 (2H, d, J=7.0Hz), 8.85 (1H, s)

MS (ESI-): 378.0 (M-H)

Example 95

(2S)-N-Hydroxy-2-[5-(4-ethylphenyl)-2-thienyl]-3,4,5,6-tetrahydro-2H-thiopyran-2-acetamide 1,1-dioxide (115 mg)

NMR ($CDCl_3$, δ): 1.24 (3H, t, J=7Hz), 1.74-1.93 (2H, m), 1.96-2.16 (2H, m), 2.57-2.73 (2H, m), 2.65 (2H, q, J=7Hz), 2.94-3.15 (4H, m), 7.14-7.24 (4H, m), 7.50 (2H, d, J=8Hz), 8.52 (1H, s)

MS (ESI-) : 392 (M-H)

Example 96

(2S)-N-Hydroxy-2-[5-(4-methoxyphenyl)-2-thienyl]-3,4,5,6-tetrahydro-2H-thiopyran-2-acetamide 1,1-dioxide (970 mg)

NMR (DMSO-$d_6$, δ): 1.72-2.06 (4H, m), 2.34-2.45 (1H, m), 2.94-3.26 (4H, m), 3.39-3.56 (1H, m), 3.78 (3H, s), 6.98 (2H, d, J=7Hz), 7.16 (1H, d, J=3Hz), 7.34 (1H, d, J=3Hz), 7.57 (2H, d, J=7Hz), 8.85 (1H, s), 10.58 (1H, s)

MS (ESI-): 394 (M-H)

Example 97

(2S)-N-Hydroxy-2-[5-(4-hydroxymethylphenyl)-2-thienyl]-3,4,5,6-tetrahydro-2H-thiopyran-2-acetamide 1,1-dioxide (75 mg)

NMR (DMSO-$d_6$, δ): 1.67-2.08 (4H, m), 2.33-2.48 (1H, m), 2.92-3.28 (4H, m), 3.39-3.54 (1H, m), 4.50 (2H, d, J=6Hz), 5.24 (1H, t, J=6Hz), 7.20 (1H, d, J=3Hz), 7.36 (2H, d, J=7Hz), 7.44 (1H, d, J=3Hz), 7.60 (2H, d, J=7Hz), 8.86 (1H, s)

MS (ESI-): 394 (M-H)

Example 98

(2S)-N-Hydroxy-2-[5-(2-thienyl)-2-thienyl]-3,4,5,6-tetrahydlro-2H-thiopyran-2-acetamide 1,1-dioxide (130 mg)
NMR (DMSO-$d_6$, δ): 1.72-2.04 (4H, m), 2.93-3,-51 (6H, m), 7.10 (1H, dd, J=4.5, 4.5Hz), 7.15 (1H, d, J=4.0Hz), 7.26 (1H, d, J=4.0Hz), 7.29 (1H, d, J=4.5Hz), 7.53 (1H, d, J=4.5Hz), 8.32 (1H, s)
MS (ESI-): 370 (M-H)

Example 99

(2S)-N-Hydroxy-2-[5-(2-furyl)-2-thienyl]-3,4,5,6-tetrahydro-2H-thiopyran-2-acetamide 1,1-dioxide (12 mg)
NMR ($CDCl_3$, δ): 1.80-1.97 (2H, m), 2.00-2.25 (2H, m), 2.56-2.69 (1H, m), 2.74-2.89 (1H, m), 2.96-3.20 (4H, m), 6.43-6.47 (1H, m), 6.52-6.56 (1H, m), 7.15-7.22 (2H, m), 7.39-7.44 (1H, m), 8.12 (1H, br s)
MS (ESI-): 354 (M-H)

Example 100

(2S)-N-Hydroxy-2-[5-(4-methylcarbamoylphenyl)-2-thienyl]-3,4,5,6-tetrahydro-2H-thiopyran-2-acetamide 1,1-dioxide (46 mg)
NMR (DMSO-$d_6$, δ): 1.68-2.10 (4H, m), 2.33-2.48 (1H, m), 2.79 (3H, d, J=4Hz), 2.92-3.32 (4H, m), 3.40-3.57 (1H, m), 7.26 (1H, d, J=3Hz), 7.60 (1H, d, J=3Hz), 7.74 (2H, d, J=8Hz), 7.88 (2H, d, J=8Hz), 8.50 (1H, m)
MS (ESI-): 421 (M-H)

Example 101

(2S)-N-Hydraxy-2-[5-(4-ethylcarbamoylphenyl)-2-thienyl]-3,4,5,6-tetrahydro-2H-thiopyran-2-acetamide 1,1-dioxide (740 mg)
NMR (DMSO-$d_6$, δ): 1.13 (3H, t, J=7.5Hz), 1.75-2.05 (4H, m), 2.36-2.45 (1H, m), 2.95-3.03 (2H, m), 3.13-3.47 (5H, m), 7.24 (1H, d, J=5.0Hz), 7.60 (1H, d, J=5.0Hz), 7.73 (2H, d, J=9.0Hz), 7.87 (2H, d, J=9.0Hz), 8.51 (1H, t, J=3.0Hz)
MS (ESI-): 435.2 (M-H)

Example 102

(2S)-N-Hydroxy-2-[5-(3-acetylaminophenyl)-2-thienyl]-3,4,5,6-tetrahydro-2H-thiopyran-2-acetamide, 1,1-dioxide (2 g)
NMR (DMSO-$d_6$, δ): 1.74-2.01 (4H, m), 2.06 (3H, s), 2.35-2.46 (1H, m), 2.94-3.50 (5H, m), 7.20 (1H, d, J=3.0Hz), 7.33 (2H, d, J=5.0Hz), 7.39 (1H, d, J=3.0Hz), 7.44-7.49 (1H, m), 7.94 (1H, s)
MS (ESI-): 421.1 (M-H)

Example 103

(2S)-N-Hydroxy-2-[5-(3-aminophenyl)-2-thienyl]-3,4,5,6-tetrahydro-2H-thiopyran-2-acetamide 1,1-dioxide hydrochloride (87 mg)
NMR (DMSO-$d_6$, δ): 1.69-2.09 (4H, m), 2.34-2.56 (1H, m), 2.95-3.07 (2H, m), 3.11-3.32 (2H, m), 3.40-3.57 (1H, m), 7.17 (1H, d, J=8Hz), 7.24 (1H, d, J=3Hz), 7.42-7.60 (4H, m), 10.63 (1H, s)
MS (ESI-): 379 (M-H)

Example 104

(2S)-N-Hydroxy-2-[5-(3-ethylcarbamoylaminophenyl)-2-thienyl]-3,4,5,6-tetrahydro-2H-thiopyran-2-acetamide 1,1-dioxide
NMR (DMSO-$d_6$, δ): 1.06 (3H, t, J=7.2Hz), 1.69-2.09 (4H, m), 2.31-2.53 (1H, m), 2.93-3.55 (7H, m), 6.14 (1H, t, J=4.5Hz), 7.12-7.30 (4H, m), 7.38 (1H, d, J=4.5Hz), 7.84 (1H, s), 8.56 (1H, s), 8.72-8.95 (1H, m), 10.60 (1H, s)
MS (ESI-): 450 (M-H)

Example 105

(2S)-N-Hydroxy-2-[5-(3-methoxycarbonylaminophenyl)-2-thienyl]-3,4,5,6-tetrahydro-2H-thiopyran-2-acetamide 1,1-dioxide (82 mg)

NMR (DMSO-$d_6$, δ): 1.70-2.07 (4H, m), 2.34-2.53 (1H, m), 2.94-3.08 (2H, m), 3.10-3.27 (2H, m), 3.30-3.55 (1H, m), 3.69 (3H, s), 7.20 (1H, d, J=3.5Hz), 7.27-7.41 (4H, m), 7.83 (1H, s), 9.76 (1H, s)

MS (ESI+): 456 (M+H+$NH_3$)

Example 106

(2S)-N-Hydroxy-2-[5-(3-carbamoylaminophenyl)-2-thienyl]-3,4,5,6-tetrahydro-2H-thiopyran-2-acetamide 1,1-dioxide (45 mg)

NMR (DMSO-$d_6$, δ): 1.70-2.08 (4H, m), 2.32-2.55 (1H, m), 2.93-3.07 (2H, m), 3.09-3.30 (2H, m), 3.39-3.55 (1H, m), 5.90 (2H, s), 7.16-7.23 (2H, m), 7.24-7.39 (2H, m), 7.37 (1H, d, J=3.5Hz), 7.78 (1H, s), 8.66 (1H, s), 8.84 (1H, s)

MS (ESI-): 422 (M-H)

Example 107

(2S)-N-Hydroxy-2-[5-(3-methylcarbamoylaminophenyl)-2-thienyl]-3,4,5,6-tetrahydro-2H-thiopyran-2-acetamide 1,1-dioxide (77 mg)

NMR (DMSO-$d_6$, δ): 1.74-2.05 (4H, m), 2.38-2.46 (1H, m), 2.66 (3H, d, J=4.0Hz), 2.95-3.26 (4H, m), 3.42-3.52 (1H, m), 6.06 (1H, q, J=4.0Hz), 7.17-7.20 (2H, m), 7.22-7.26 (2H, m), 7.36 (1H, d, J=3.5Hz), 7.82 (1H, s), 8.65 (1H, s), 8.83 (1H, s)

MS (ESI-): 436.2 (M-H)

Example 108

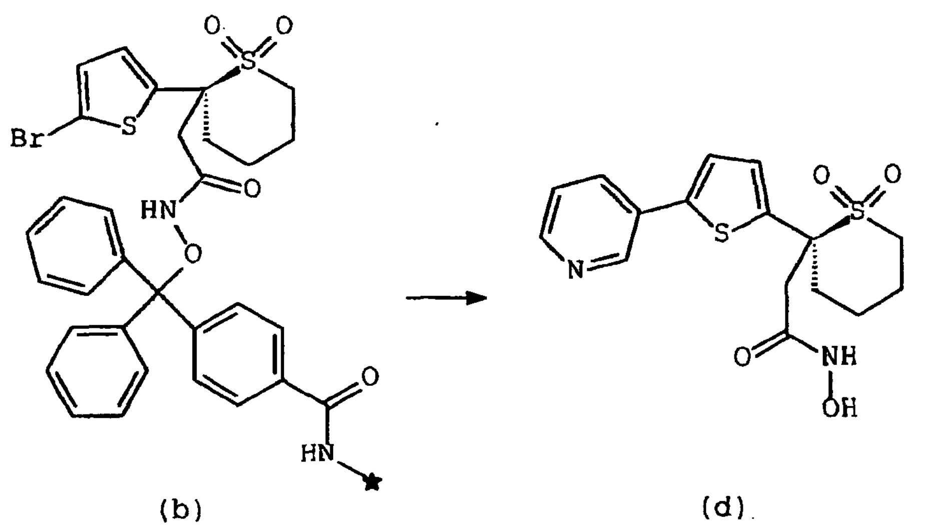

(b) (d)

To a solution of pyridine-3-boronic acid 1,3-propanediol cyclic ester (130 mg) in degassed N,N-dimethylformamide (0.5 ml) were added a suspension of tetrakis(triphenylphosphine)-palladium (103 mg) in degassed N,N-dimethylformamide (2.5 ml), a solution of sodium carbonate (424 mg) in degassed water (1 ml) and (2S)-N-[2-[2-(5-bromo-2-thienyl)-1,1-dioxo-3,4,5,6-tetrahydro-2H-thiopyran-2-yl]acetyl]hydroxylamine trityl crowns (b) (4 mmol, 10.0 μmol/crown) in an atmosphere of nitrogen. After resulting mixture was heated for 48 hours at 60°C, the crowns were washed with degassed N,N-dimethylformamide, a solution of sodium diethyldithiocarbamate (500 mg) and diisopropylethylamine (0.5 ml) in N,N-dimethylformamide (100 ml), N,N-dimethylformamide, methyl sulfoxide, water, methanol and dichloromethane, successively. The crowns were treated with 5% trifluoroacetic acid in dichloromethane for 1 hour at ambient temperature and removed from the solution. After the solution was evaporated under a stream of nitrogen, the residue was purified by reverse phase HPLC (0.1% trifluoroacetic acid in acetonitrile, 0-20% gradient) to give (2S)-N-hydroxy-2-[5-(3-pyridyl)-2-thienyl]-3,4,5,6-tetrahydro-2H-thiopyran-2-acetamide 1,1-dioxide (10 mg) as a powder.

NMR (DMSO-$d_6$, δ): 1.25-2.05 (4H, m), 2.87-2.93 (1H, m), 2.97-3.50 (5H, m), 7.28 (1H, d, J=4.0Hz), 7.53-7.58

(1H, m), 7.65 (1H, d, J=4.0Hz), 8.16 (1H, d, J=7.5Hz), 8.56 (1H, br), 8.95 (1H, br)

MS (ESI-): 365.0(M-H)

Example 109

(2S)-N-(2-Tetrahydropyranyloxy)-2-[5-(3-methylcarbamoylaminophenyl)-2-thienyl]-3,4,5,6-tetrahydro-2H-thiopyran-2-acetamide 1,1-dioxide (132 mg)

NMR ($CDCl_3$, δ): 1.45 (4H, br), 1.62-1.66 (2H, m), 1.93 (2H, br), 2.07-2.17 (2H, m), 2.77-2.81 (5H, m), 2.98-3.03 (1H, m), 3.10-3.15 (3H, m), 3.32-3.50 (1H, m), 3.70-3.78 (1H, m), 4.59 (1Hx1/2, s), 4.83 (1Hx1/2, s), 7.00 (1H, d, J=3.5Hz), 7.07-7.08 (1H, m), 7.12-7.21 (4H, m), 7.30-7.40 (2H, m), 9.16 (1Hx1/2, s), 9.35 (1Hx1/2, s)

MS (ESI-): 520.2 (M-H)

The following compounds were obtained in substantially the same manner as that of Example 85.

Example 110

(2S)-N-(2-Tetrahydropyranyloxy)-2-[5-(3-methylphenyl)-2-thienyl]-3,4,5,6-tetrahydro-2H-thiopyran-2-acetamide 1,1-dioxide (200 mg)

NMR ($CDCl_3$, δ): 1.40-1.73 (10H, m), 2.37 (3H, s), 2.69-2.88 (2H, m), 3.06-3.16 (4H, m), 3.30-3.45 (1H, m), 3.61-3.75 (2H, m), 7.11 (1H, d, J=7.5Hz), 7.24-7.28 (3H, m), 7.38-7.41 (2H, m)

Example 111

(2S)-N-(2-Tetrahydropyranyloxy)-2-[5-(4-methylphenyl)-2-thienyl]-3,4,5,6-tetrahydro-2H-thiopyran-2-acetamide 1,1-dioxide (142 mg)

NMR ($CDCl_3$, δ): 1.43 (2H, br), 1.65-1.69 (2H, m), 1.95 (2H, br), 2.07-2.18 (2H, m), 2.37 (3H, s), 2.63-2.80 (2H, m), 3.06 (2H, br s), 3.10-3.16 (2H, m), 3.40-3.50 (2H, m), 3.58-3.76 (2H, m), 4.53 (1Hx1/2, s), 4.80 (1Hx1/2, s), 7.15-7.24 (4H, m), 7.46 (2H, d, J=8.0Hz), 7.39 (1Hx1/2, s), 8.06 (1Hx1/2, s)

MS (ESI-): 462.1 (M-H)

Example 112

(2S)-N-(2-Tetrahydropyranyloxy)-2-[5-(4-ethylphenyl)-2-thienyl)-3,4,5,6-tetrahydro-2H-thiopyran-2-acetamide 1,1-dioxide

NMR ($CDCl_3$, δ): 1.25 (3H, t, J=8Hz), 1.35-1.76 (6H, m), 1.87-2.00 (2H, m), 2.04-2.23 (2H, m), 2.60-2.91 (4H, m), 3.00-3.19 (4H, m), 3.27-3.50 (1H, m), 3.60-3.77 (1H, m), 4.53, 4.71 (1H, s), 7.15-7.28 (4H, m), 7.51 (2H, d, J=8Hz), 8.10, 8.25 (1H, s)

MS (ESI+): 478 (M+H)

Example 113

(2S)-N-(2-Tetrahydropyranyloxy)-2-[5-(4-methoxyphenyl)-2-thienyl]-3,4,5,6-tetrahydro-2H-thiopyran-2-acetamide 1,1-dioxide (1.85 g)

NMR ($CDCl_3$, δ): 1.36-1.82 (6H, m), 1.98-2.02 (2H, m), 2.10-2.25 (2H, m), 2.62-2.88 (2H, m), 2.98-3.20 (4H, m), 3.26-3.50 (1H, m), 3.57-3.72 (1H, m), 3.83 (3H, s), 6.90 (2H, d, J=8Hz), 7.13, 7.15 (1H, d, J=3Hz), 7.22, 7.24 (1H, d, J=3Hz), 7.52 (2H, d, J=8Hz), 8.11, 8.25 (1H, s)

MS (ESI-): 478(M-H)

Example 114

(2S)-N-(2-Tetrahydropyranyloxy)-2-[5-(4-hydroxymethylphenyl)-2-thienyl]-3,4,5,6-tetrahydro-2H-thiopyran-2-acetamide 1,1-dioxide (181 mg)

NMR ($CDCl_3$, δ): 1.36-1.78 (6H, m), 1.85-2.03 (2H, m), 2.06-2.55 (2H, m), 2.66-2.93 (2H, m), 2.98-3.21 (4H, m), 3.26-3.50 (1H, m), 3.62-3.73 (1H, m), 4.52, 4.82 (1H, m), 4.70 (1H, s), 7.22-7.30 (1H, m), 7.35 (2H, d, J=8Hz), 7.42-7.62 (1H, m), 7.57 (2H, d, J=8Hz), 8.25, 8.35 (1H, s)

MS (ESI-): 478 (M-H)

Example 115

(2S)-N-(2-Tetrahydropyranyloxy)-2-[5-(2-thienyl)-2-thienyl]-3,4,5,6-tetrahydro-2H-thiopyran-2-acetamide 1,1-dioxide (200 mg)

NMR ($CDCl_3$, δ): 1.41-1.75 (10H, m), 2.65-2.82 (2H, m), 3.04-3.17 (4H, m), 3.30-3.74 (3H, m), 7.00-7.02 (1H, m), 7.11-7.13 (1H, m), 7.17-7.19 (3H, m)

MS (ESI-): 454 (M-H)

Example 116

(2S)-N-(2-Tetrahydropyranyloxy)-2-(5-(2-furyl)-2-thienyl]-3,4,5,6-tetrahydro-2H-thiopyran-2-acetamide 1,1-dioxide (95 mg)

NMR ($CDCl_3$, δ): 1.41-1.77 (6H, m), 1.85-2.00 (2H, m), 2.03-2.25 (2H, m), 2.61-2.91 (2H, m), 3.00-3.06 (2H, m), 3.07-3.16 (2H, m), 3.28-3.53 (1H, m), 3.61-3.75 (1H, m), 4.51 (0.5H, s), 4.80 (0.5H, s), 6.42-6.47 (1H, m), 6.50-6.56 (1H, m), 7.17-7.24 (2H, m), 7.38-7.42 (1H, m), 7.93 (0.5H, s), 8.09 (0.5H, s)

MS (ESI-): 438 (M-H)

Example 117

(2S)-N-(2-Tetrahydropyranyloxy)-2-[5-(4-carboxyphenyl)-2-thienyl]-3,4,5,6-tetrahydro-2H-thiopyran-2-acetamide 1,1-dioxide (910 mg)

NMR ($CDCl_3$, δ): 1.36-1.73 (6H, m), 1.85-2.25 (4H, m), 2.76-2.88 (2H, m), 3.02-3.25 (4H, m), 3.28-3.53 (1H, m), 3.69-3.81 (1H, m), 4.56, 4.87 (1H, s), 7.57 (1H, d, J=8Hz), 7.36-7.44 (1H, m), 7.72-7.83 (2H, m), 8.00 (2H, d, J=8Hz)

MS (ESI-): 492 (M-H)

Example 118

(2S)-N-(2-Tetrahydropyranyloxy)-2-[5-(4-ethylcarbamoylphenyl)-2-thienyl]-3,4,5,6-tetrahydro-2H-thiopyran-2-acetamide 1,1-dioxide (984 mg)

NMR ($CDCl_3$, δ): 1.28 (3H, t, J=7.0Hz), 1.44-1.55 (4H, m), 1.64-1.68 (2H, m), 1.95 (2H, br), 2.06-2.23 (2H, m), 2.67-2.91 (2H, m), 3.01-3.16 (4H, m), 3.27-3.33 (1H, m), 3.46-3.55 (2H, m), 3.62-3.65 (1H, m), 4.50 (1Hx1/2, s), 4.82 (1Hx1/2, s), 7.28 (1H, d, J=4.0Hz), 7.33 (1H, d, J=4.0Hz), 7.62 (2H, d, J=8.0Hz), 7.75 (2H, d, J=8.0Hz), 8.19 (1Hx1/2, s), 8.27 (1Hx1/2, s)

MS (ESI-): 519.2 (M-H)

Example 119

(2S)-N-(2-Tetrahydropyranyloxy)-2-[5-(3-acetylaminophenyl)-2-thienyl]-3,4,5,6-tetrahydro-2H-thiopyran-2-acetamide 1,1-dioxide (2.63 g)

NMR ($CDCl_3$, δ): 1.43 (2H, br), 1.55-1.64 (4H, m), 1.93 (2H, br), 2.04-2.15 (2H, m), 2.19 (3H, s), 2.80-2.85 (2H, m), 3.02-3.16 (4H, m), 3.44-3.48 (1H, m), 3.66-3.73 (1H, m), 4.55 (1Hx1/3, s), 4.85 (1Hx2/3, s), 7.08-7.11 (1H, m), 7.16-7.23 (3H, m), 7.38 (1H, br s), 7.57 (1H, d, J=7.0Hz), 7.94 (1H, s), 8.93 (1Hx1/3, s), 9.02 (1Hx2/3, s)

MS (ESI-): 505.4 (M-H)

Example 120

(2S)-N-(2-Tetrahydropyranyloxy)-2-[5-(3-aminophenyl)-2-thienyl]-3,4,5,6-tetrahydro-2H-thiopyran-2-acetamide 1,1-dioxide (214 mg)

NMR ($CDCl_3$, δ): 1.36-1.80 (6H, m), 1.84-2.25 (4H, m), 2.61-2.92 (2H, m), 2.98-3.20 (4H, m), 3.27-3.89 (4H, m), 4.54 (0.5H, s), 4.81 (0.5H, s), 6.62 (1H, dd, J=2.3, 8Hz), 6.91 (1H, s), 6.99 (1H, d, J=8Hz), 7.15 (1H, t, J=8Hz), 7.20-7.29 (2H, m), 7.98 (0.5H, s), 8.15 (0.5H, s)

MS (ESI-): 463 (M-H)

Example 121

tert-Butyl2-(5-bromo-2-thienyl)-3,4,5,6-tetrahydro-2H-thiopyran-2-acetate (199 g) was obtained in substantially the same manner as that of Example 89.

NMR ($CDCl_3$, δ): 1,34 (9H, s), 1.46-1.91 (5H, m), 2.10-2.22 (1H, m), 2.49-2.62 (2H, m), 2.66 (1H, d, J=13.2Hz),

2.75 (1H, d, J=13.2Hz), 6.74 (1H, d, J=3.9Hz), 7.45 (1H, d, J=3.9Hz)

Example 122

(2S)-2-(5-Bromo-2-thienyl)-3,4,5,6-tetrahydro-2H-thiopyran-2-N-[(R)-1-phenylethyl]acetamide 1,1-dioxide (78 g) was partitioned between ethyl acetate (500 ml) and aqueous 1N hydrochloric acid (300 ml). The separated organic phase was washed with aqueous 1N hydrochloric acid (100 ml) and brine (100 ml), dried over sodium sulfate and concentrated in vacuo to give (2S)-2-(5-bromo-2-thienyl)-3,4,5,6-tetrahydro-2H-thiopyran-2-acetic acid 1,1-dioxide (57.5 g) as an solid.

mp: 189°C (dec.)

NMR (DMSO-$d_6$, δ): 1.74-1.87 (4H, m), 2.30-2.37 (1H, m), 3.07-3.56 (5H, m), 7.02 (1H, d, J=4.2Hz), 7.21 (1H, d, J=4.2Hz)

Example 123

(2S)-N-(2-Tetrahydropyranyloxy)-2-[5-(4-methylcarbamoylphenyl)-2-thienyl]-3,4,5,6-tetrahydro-2H-thiopyran-2-acetamide 1,1-dioxide (68 mg) was obtained in substantially the same manner as that of Example 31.

NMR (DMSO-$d_6$, δ): 1.35-1.64 (6H, m), 1.71-2.08 (4H, m), 2.36-2.53 (1H, m), 2.79 (3H, d, J=4Hz), 2.88-3.32 (4H, m), 3.40-3.53 (2H, m), 3.74-3.92 (1H, m), 4.45, 4.75 (1H, s), 7.22-7.30 (1H, m), 7.55-7.64 (1H, m), 7.74 (2H, d, J=8Hz), 7.88 (2H, d, J=8Hz), 8.50 (1H, d, J=4Hz), 11.25 (1H, s)

MS (ESI-): 505 (M-H)

Example 124

To a mixture of (2S)-N-(2-tetrahydropyranyloxy)-2-[5-(3-aminophenyl)-2-thienyl]-3,4,5,6-tetrahydro-2H-thiopyran-2-acetamide 1,1-dioxide (110 mg) in dichloromethane (1.5 ml) was added a solution of ethylisocyanate (21.9 mg) in dichloromethane (0.5 ml) with ice cooling. The mixture was allowed to warm to room temperature and stirred for 3 hours. The resulted mixture was purified by chromatography on silica gel (methanol in chloroform, 0.5 to 3% gradient) to give (2S)-N-(2-tetrahydropyranyloxy)-2-(5-(3-ethylcarbamoylaminophenyl)-2-thienyl]-3,4,5,6-tetrahydro-2H-thiopyran-2-acetamide 1,1-dioxide (100 mg) as an amorphous solid.

NMR ($CDCl_3$, δ): 1.08-1.18 (3H, m), 1.38-1.75 (6H, m), 1.86-2.00 (2H, m), 2.02-2.24 (2H, m), 2.76-2.90 (2H, m), 2.96-3.17 (4H, m), 3.20-3.55 (3H, m), 3.70-3.85 (1H, m), 4.61 (0.5H, s), 4.84 (0.5H, s), 5.25-5.40 (1H, m), 6.95-7.34 (5H, m), 7.36-7.50 (1H, m), 9.13 (0.5H, s), 9.35 (0.5H, s)

MS (ESI-): 534 (M-H)

Example 125

To a mixture of (2S)-N-(2-tetrahydropyranyloxy)-2-[5-(3-aminophenyl)-2-thienyl]-3,4,5,6-tetrahydro-2H-thiopyran-2-acetamide 1,1-dioxide (110 mg) and pyridine (28.1 mg) in dichloromethane (1.5 ml) was added a solution of methyl chloroformate (26.8 mg) in dichloromethane (0.5 ml) with ice cooling. The mixture was allowed to warm to room temperature and stirred for 3 hours. The resulted mixture was washed with aqueous 0.5% citric acid and brine, dried over sodium sulfate and concentrated in vacuo. The obtained residue was purified by chromatography on silica gel (methanol in chloroform, 0.5 to 3% gradient) to give (2S)-N-(2-tetrahydropyranyloxy)-2-[5-(3-methoxycarbonylaminophenyl)-2-thienyl]-3,4,5,6-tetrahydro-2H-thiopyran-2-acetamide 1,1-dioxide (101 mg) as an amorphous solid.

NMR ($CDCl_3$, δ): 1.35-1.56 (4H, m), 1.59-1.75 (2H, m), 1.86-2.00 (2H, m), 2.05-2.26 (2H, m), 2.63-2.93 (2H, m), 3.01-3.17 (4H, m), 3.27-3.51 (1H, m), 3.58-3.74 (1H, m), 3.79 (3H, s), 4.54 (0.5H, s), 4.82 (0.5H, s), 6.74 (1H, br s), 7.15-7.21 (1H, m), 7.23-7.37 (4H, m), 7.62 (1H, br s), 8.10 (0.5H, s), 8.24 (0.5H, s)

MS (ESI-): 521 (M-H)

The following compound was obtained in substantially the same manner as that of Example 124.

Example 126

(2S)-N-(2-Tetrahydropyranyloxy)-2-[5-(3-aminocarbamoylphenyl)-2-thienyl]-3,4,5,6-tetrahyaro-2H-thiopyran-2-acetamide 1,1-dioxide (170 mg)

NMR (DMSO-$d_6$, δ): 1.36-1.65 (6H, m), 1.69-2.10 (4H, m), 2.34-2.48 (1H, m), 2.89-3.32 (4H, m), 3.39-3.54 (2H, m), 3.72-3.91 (1H, m), 4.43 (0.5H, s), 4.75 (0.5H, s), 5.90 (2H, s), 7.16-7.30 (4H, m), 7.32-7.49 (1H, m), 7.79 (1H, s), 8.66 (1H, s)

MS (ESI-): 506 (M-H)

Example 127

(2S)-N-(2-Tetrahydropyranyloxy)-2-[5-(4-fluorophenyl)-2-thienyl]-2,3,4,5-tetrahydrothiophene-2-acetamide 1,1-dioxide (272 mg) was obtained in a similar manner to that of Example 31.

NMR ($CDCl_3$, δ): 1.39-1.84 (6H, m), 2.27-2.41 (2H, m), 2.82-3.00 (4H, m), 3.13-3.25 (2H, m), 3.35-3.63 (1H, m), 3.68-3.80 (1H, m), 4.55-4.64 (0.5H, m), 4.81-4.89 (0.5H, m), 7.03-7.11 (2H, m), 7.13-7.40 (2H, m), 7.49-7.58 (2H, m), 8.20 (0.5H, s), 8.30 (0.5H, s)

MS (ESI-): 452 (M-H)

The following compounds were obtained in substantially the same manner as that of Example 52.

Example 128

(2S)-N-Hydroxy-2-[5-(3 -ethoxyacetylaminophenyl)-2-thienyl]-3,4,5,6-tetrahydro-2H-thiopyran-2-acetamide 1,1-dioxide (4.62 g)

NMR (DMSO-$d_6$, δ): 1.20 (3H, t, J=7Hz), 2.35-2.48 (1H, m), 2.94-3.28 (4H, m), 3.42-3.53 (1H, m), 3.58 (2H, q, J=7Hz), 4.04 (2H, s), 7.21 (1H, d, J=3Hz), 7.32-7.44 (3H, m), 7.57-7.63 (1H, m), 8.03 (1H, s), 8.85 (1H, br), 9.81 (1H, s)

MS (ESI-): 465 (M-H)

Example 129

(2S)-N-Hydroxy-2-[5-(3-propionylaminophenyl)-2-thienyl]-3,4,5,6-tetrahydro-2H-thiopyran-2-acetamide 1,1-dioxide (76 mg)

NMR (DMSO-$d_6$, δ): 1.09 (3H, t, J=7Hz), 2.35 (2H, q, J=7Hz), 2.37-2.48 (1H, m), 2.90-3.52 (5H, m), 7.21 (1H, d, J=3Hz), 7.28-7.53 (5H, m), 8.00 (1H, s), 9.98 (1H, s), 10.61 (1H, s)

MS (ESI-): 435 (M-H)

Example 130

(2S)-N-Hydroxy-2-[5-(3-propylaminocarbonylaminophenyl)-2-thienyl]-3,4,5,6-tetrahydro-2H-thiopyran-2-acetamide 1,1-dioxide (75 mg)

NMR (DMSO-$d_6$, δ): 0.88 (3H, t, J=7Hz), 1.44 (2H, q, J=7Hz), 1.68-2.10 (4H, m), 2.34-2.48 (1H, m), 2.90-3.58 (7H, m), 6.22 (1H, br), 7.11-7.30 (4H, m), 7.48 (1H, d, J=3Hz), 7.84 (1H, s), 8.60 (1H, s), 10.61 (1H, s)

MS (ESI-): 464 (M-H)

Example 131

(2S)-N-Hydroxy-2-[5-(3-butyrylaminophenyl)-2-thienyl]-3,4,5,6-tetrahydro-2H-thiopyran-2-acetamide 1,1-dioxide (79 mg)

NMR (DMSO-$d_6$, δ): 0.92 (3H, t, J=7Hz), 1.55-1.68 (2H, m), 1.69-2.10 (4H, m), 2.30 (2H, t, J=7Hz), 2.35-2.48 (1H, m), 2.92-3.65 (5H, m), 7.20 (1H, d, J=3Hz), 7.34 (2H, d, J=3Hz), 7.40 (1H, d, J=3Hz), 7.44-7.53 (1H, m), 8.00 (1H, s)

MS (ESI-): 449 (M-H)

Example 132

(2S)-N-Hydroxy-2-[5-(3-(2-methoxyethoxycarbonylamino)-phenyl)-2-thienyl]-3,4,5,6-tetrahydro-2H-thiopyran-2-acetamide 1,1-dioxide (64 mg)

NMR (DMSO-$d_6$, δ): 1.70-2.11 (4H, m), 2.34-2.50 (1H, m), 2.92-3.26 (4H, m), 3.29 (3H, s), 3.40-3.66 (3H, m), 4.15-4.28 (2H, m), 7.20 (1H, d, J=3Hz), 7.26-7.42 (4H, m), 7.85 (1H, s), 8.84 (1H, s), 9.87 (1H, s), 10.60 (1H, s)

MS (ESI-) : 481 (M-H)

Example 133

(2S)-N-Hydroxy-2-[5-(3-(2-methoxycarbonylaminoacetylamino)phenyl)-2-thienyl]-3,4,5,6-tetrahydro-2H-thiopyran-2-acetamide 1,1-dioxide (58 mg)

NMR (DMSO-$d_6$, δ): 1.69-2.07 (4H, m), 2.32-2.48 (1H, m), 2.92-3.50 (5H, m), 3.56 (3H, s), 3.80 (2H, d, J=8Hz), 7.19 (1H, d, J=3Hz), 7.30-7.52 (5H, m), 7.97 (1H, s), 8.82 (1H, s)

MS (ESI-) : 494 (M-H)

Example 134

(2S)-N-Hydroxy-2-[5-(3-(phenoxyacetylamino)phenyl)-2-thienyl]-3,4,5,6-tetrahydro-2H-thiopyran-2-acetamide 1,1-dioxide (3.8 g)

NMR (DMSO-$d_6$, δ): 1.70-2.09 (4H, m), 2.35-2.50 (1H, m), 2.92-3.55 (5H, m), 4.72 (2H, s), 6.94-7.05 (3H, m), 7.21 (1H, d, J=3Hz), 7.28-7.44 (5H, m), 7.52-7.60 (1H, m), 8.03 (1H, s), 8.84 (1H, s), 10.21 (1H, s), 10.60 (1H, s)

MS (ESI-): 513 (M-H)

Example 135

(2S)-N-Hydroxy-2-[5-(3-(propoxyacetylamino)phenyl)-2-thienyl]-3,4,5,6-tetrahydro-2H-thiopyran-2-acetamide 1,1-dioxide (2.28 g)

NMR (DMSO-$d_6$, δ): 0.91 (3H, t, J=8Hz), 1.54-1.67 (2H, m), 1.70-2.11 (4H, m), 2.36-2.49 (1H, m), 2.94-3.29 (4H, m), 3.43-3.58 (1H, m), 3.48 (2H, t, J=8Hz), 4.05 (2H, s), 7.21 (1H, d, J=3Hz), 7.32-7.45 (3H, m), 7.54-7.62 (1H, m), 8.03 (1H, s), 8.85 (1H, s), 9.81 (1H, s), 10.60 (1H, s)

MS (ESI-): 479 (M-H)

Example 136

(2S)-N-Hydroxy-2-[5-[3-(2-propen-1-yloxy)acetylamino]phenyl]-2-thienyl]-3,4,5,6-tetrahydro-2H-thiopyran-2-acetamide 1,1-dioxide (9.32 g)

NMR (DMSO-$d_6$, δ): 1.70-2.09 (4H, m), 2.35-2.56 (1H, m), 2.94-3.08 (2H, m), 3.10-3.29 (2H, m), 3.30-3.55 (1H, m), 4.07 (2H, s), 4.10 (2H, d, J=6Hz), 5.22 (1H, d, J=9Hz), 5.34 (1H, d, J=15Hz), 5.89-6.04 (1H, m), 7.21 (1H, d, J=3.5Hz), 7.31-7.44 (3H, m), 7.55-7.61 (1H, m), 8.03 (1H, s), 8.85 (1H, br s), 9.86 (1H, s), 10.6 (1H, br s)

MS (ESI-): 477 (M-H)

Example 137

(2S)-N-Hydroxy-2-[5-[4-(5-oxazolyl)phenyl]-2-thienyl]-3,4,5,6-tetrahydro-2H-thiopyran-2-acetamide 1,1-dioxide (331 mg)

NMR (DMSO-$d_6$, δ): 1.72-2.10 (4H, m), 2.36-2.49 (1H, m), 2.95-3.09 (2H, m), 3.11-3.30 (2H, m), 3.42-3.56 (1H, m), 7.24 (1H, d, J=3.9Hz), 7.57 (1H, d, J=3.9Hz), 7.76 (1H, s), 7.77 (4H, s), 8.48 (1H, s)

MS (ESI-): 431 (M-H)

Example 138

(2S)-N-Hydroxy-2-[5-[3-(n-butyloxyacetylamino)phenyl)-2-thienyl]-3,4,5,6-tetrahydro-2H-thiopyran-2-acetamide 1,1-dioxide (90 mg)

NMR (DMSO-$d_6$, δ): 0.92 (3H, t, J=7.0Hz), 1.47 (2H, tq, J=7.0, 7.0Hz), 1.60 (2H, dd, J=7.0, 7.0Hz), 1.74-2.06 (4H, m), 2.37-2.47 (1H, m), 2.96-3.30 (4H, m), 3.38-3.45 (1H, m), 3.52 (2H, t, J=7.0Hz), 4.05 (2H, s), 7.22 (1H, d, J=4.0Hz), 7.32-7.37 (2H, m), 7.41 (1H, d, J=4.0Hz), 7.55-7.60 (1H, m), 8.02 (1H, s), 8.84 (1H, s), 9.80 (1H, s), 10.59 (1H, s)

MS (ESI-): 493.2 (M-H)

Example 139

(2S)-N-Hydroxy-2-[5-{3-ethoxycarbonylaminoacetylamino)phenyl}-2-thienyl]-3,4,5,6-tetrahydro-2H-thiopyran-2-acetamide 1,1-dioxide (2.3 g)

NMR (DMSO-$d_6$, δ): 1.18 (3H, t, J=7.0Hz), 1.74-2.05 (4H, m), 2.36-2.46 (1H, m), 2.95-3.26 (4H, m), 3.40-3.53 (1H, m), 3.79 (2H, d, J=6.0Hz), 4.02 (2H, q, J=7.0Hz), 7.21 (1H, d, J=4.0Hz), 7.35-7.40 (3H, m), 7.40 (1H, d, J=4.0Hz), 7.45-7.49 (1H, m), 7.97 (1H, s), 8.84 (1H, s), 10.08 (1H, s)

MS (ESI-): 508.3 (M-H)

Example 140

(2S)-N-Hydroxy-2-[5-{3-(2-chloroethylaminocarbonylamino)phenyl)-2-thienyl]-3,4,5,6-tetrahydro-2H-thiopyran-2-acetamide 1,1-dioxide (72 mg)

NMR (DMSO-$d_6$, δ): 1.72-2.04 (4H, m), 2.36-2.45 (1H, m), 2.93-3.23 (4H, m), 3.39-3.46 (3H, m), 3.67 (2H, t,

J=6.0Hz), 6.44 (1H, t, J=6.0Hz), 7.17-7.30 (4H, m), 7.38 (1H, d, J=4.0Hz), 7.84 (1H, s), 8.81 (1H, s), 8.84 (1H, s)

MS (ESI-): 484.3 (M-H)

Example 141

(2S)-N-Hydroxy-2-[5-{3-(3-methoxypropionylamino)-phenyl}-2-thienyl]-3,4,5,6-tetrahydro-2H-thiopyran-2-acetamide 1,1-dioxide (3.4 g)

NMR (DMSO-$d_6$, δ): 1.72-2.05 (4H, m), 2.38-2.47 (1H, m), 2.56 (2H, t, J=6.0Hz), 2.95-3.21 (4H, m), 3.27 (3H, s), 3.36-3.52 (1H, m), 3.64 (2H, t, J=6.0Hz), 7.20 (1H, d, J=4.0Hz), 7.31-7.36 (2H, m), 7.40 (1H, d, J=4.0Hz), 7.45-7.49 (1H, m), 8.01 (1H, s), 10.07 (1H, s), 10.60 (1H, s)

MS (ESI-): 479.2 (M-H+Na)

Example 142

(2S)-N-Hydroxy-2-[5-{3-(rnethoxyacetylamino)pienyl}-2-thienyl]-3,4,5,6-tetrahydro-2H-thiopyran-2-acetamide 1,1-dioxide (2.5 g)

NMR (DMSO-$d_6$, δ): 1.74-2.06 (4H, m), 2.37-2.48 (1H, m), 2.95-3.26 (4H, m), 3.41 (3H, s), 3.43-3.53 (1H, m) , 4.02 (2H, s), 7.20 (1H, d, J=4.0Hz), 7132-7.38 (2H, m), 7.42 (1H, d, J=4.0Hz), 7.58-7.63 (1H, m), 8.04 (1H, s), 8.84 (1H, s), 9.87 (1H, s), 10.60 (1H, s)

MS (ESI-): 451.2 (M-H)

Example 143

(2S)-N-Hydroxy-2-[5-(3-hydroxymethylphenyl)-2-thienyl]-3,4,5,6-tetrahydro-2H-thiopyran-2-acetamide 1,1-dioxide (60 mg)

NMR (DMSO-$d_6$, δ): 1.71-2.08 (4H, m), 2.37-2.46 (1H, m), 2.95-3.54 (5H, m), 4.54 (2H, d, J=5.5Hz), 5.29 (1H, dd, J=5.5, 5.5Hz), 7.21 (1H, d, J=4.0Hz), 7.25 (1H, d, J=8.0Hz), 7.38 (1H, dd, J=8.0, 8.0Hz), 7.46 (1H, d, J=4.0Hz), 7.53 (1H, d, J=8.0Hz), 7.59 (1H, s), 8.85 (1H, s), 10.6 (1H, s)

MS (ESI-): 394 (M-H)

Example 144

(2S)-N-Hydroxy-2-[5-(4-(cis-1,2-dihydroxyethyl)-phenyl)-2-thienyl]-3,4,5,6-tetrahydro-2H-thiopyran-2-acetamide 1,1-dioxide (100 mg)

NMR (DMSO-$d_6$, δ): 1.73-2.07 (4H, m), 2.35-2.46 (1H, m), 2.95-3.50 (5H, m), 4.51-4.56 (1H, m), 4.70-4.80 (1H, br), 5.24-5.35 (1H, br), 7.20 (1H, d, J=4.0Hz), 7.39 (2H, d, J=8.0Hz), 7.44 (1H, d, J=4.0Hz), 7.58 (2H, d, J=8.0Hz), 8.83 (1H, s), 10.58 (1H, s)

MS (ESI-): 424 (M-H)

Example 145

(2S)-N-Hydroxy-2-[5-(3-(methylaminocarbonyloxymethyl)-phenyl)-2-thienyl]-3,4,5,6-tetrahydro-2H-thiopyran-2-acetamide 1,1-dioxide (100 mg)

NMR (DMSO-$d_6$, δ): 1.70-2.06 (4H, m), 2.35-2.46 (1H, m), 2.59 (3H, d, J=5.0Hz), 2.96-3.54 (5H, m), 5.05 (2H, m) , 7.22 (1H, d, J=4.0Hz), 7.29 (1H, d, J=8.0Hz), 7.42 (1H, dd, J=8.0, 8.0Hz), 7.49 (1H, d, J=4.0Hz), 7.59-7.61 (2H, m), 8.84 (1H, br), 10.59 (1H, br)

MS (ESI-): 45i (M-H)

Example 146

(2S)-N-Hydroxy-2-[5-(4-(2-methylaminocarbonylethenyl)phenyl)-2-thienyl]-3,4,5,6-tetrahydro-2H-thiopyran-2-acetamide 1,1-dioxide (170 mg)

NMR (DMSO-$d_6$, δ): 1.72-2.09 (4H, m), 2.38-2.48 (1H, m), 2.71 (3H, d, J=5Hz), 2.98-3.51 (5H, m), 6.63 (1H, d, J=15Hz), 7.23 (1H, d, J=4Hz), 7.42 (1H, d, J=15Hz), 7.55 (1H, d, J=4Hz), 7.60 (2H, d, J=8.4Hz), 7.69 (2H, d, J=8.4Hz), 8.05 (1H, d, J=5Hz), 10.60 (1H, s)

MS (ESI-): 447 (M-H)

Example 147

(2S)-N-Hydroxy-2-[5-(4-(2-ethylaminocarbonylethyl)-phenyl)-2-thienyl]-3,4,5,6-tetrahydro-2H-thiopyran-2-acetamide 1,1-dioxide (200 mg)

NMR (DMSO-$d_6$, δ): 1.08 (3H, t, J=7.2Hz), 1.73-2.12 (4H, m), 2.39-2.48 (1H, m), 2.96-3.05 (2H, m), 3.11-3.54 (5H, m), 6.63 (1H, d, J=15Hz), 7.24 (1H, d, J=4Hz), 7.41 (1H, d, J=15Hz), 7.55 (1H, d, J=4Hz), 7.60 (2H, d, J=8.4Hz), 7.69 (2H, d, J=8.4Hz), 8.10 (1H, dd, J=7.2, 7.2Hz), 10.6 (1H, s)

MS (ESI-): 461 (M-H)

Example 148

(2S)-N-Hydroxy-2-[5-(3-(isopropylaminocarbonylamino)-phenyl)-2-thienyl]-3,4,5,6-tetrahydro-2H-thiopyran-2-acetamide 1,1-dioxide (82 mg)

NMR (DMSO-$d_6$, δ): 1.10 (6H, d, J=8Hz), 1.65-2.11 (4H, m), 2.30-2.45 (1H, m), 2.85-3.26 (4H, m), 3.36-3.56 (1H, m), 3.65-3.88 (1H, m), 6.08 (1H, br s), 7.07-7.45 (6H, m), 7.84 (1H, s), 8.50 (1H, s), 10.62 (1H, s)

MS (ESI-): 464 (M-H)

Example 149

(2S)-N-Hydroxy-2-[5-[3-[(2-hydroxyethylamino)-acetylamino]phenyl]-2-thienyl]-3,4,5,6-tetrahydro-2H-thiopyran-2-acetamide 1,1-dioxide hydrochloride (82 mg)

NMR (DMSO-$d_6$, δ): 1.70-2.10 (4H, m) , 2.32-2.53 (1H, m), 2.94-3.06 (2H, m), 3.09-3.20 (2H, m), 3.21-3.30 (2H, m), 3.40-3.88 (3H, m), 3.98-4.05 (2H, m), 7.23 (1H, d, J=3.5Hz), 7.37-7.53 (4H, m), 7.93-8.00 (2H, m), 8.93-9.07 (2H, s)

MS (ESI+): 482 (M+H)

Example 150

(2S)-N-Hydroxy-2-[S-[3-[(4-morpholino)acetylamino]-phenyl)-2-thienyl]-3,4,5,6-tetrahydro-2H-thiopyrin-2-acetamide 1,1-dioxide hydrochloride (75 mg)

NMR (DMSO-$d_6$, δ): 1.70-2.10 (4H, m), 2.32-2.52 (1H, m), 2.90-3.06 (2H, m), 3.09-3.37 (4H, m), 3.41-3.58 (5H, m), 3.62-4.03 (2H, m), 4.25 (2H, s), 7.23 (1H, d, J=3.5Hz), 7.40-7.54 (5H, m), 8.03 (1H, s)

MS (ESI+): 508 (M+H)

Example 151

(2S)-N-Hydroxy-2-[5-(3-(4-methoxyphenyl)acetylamino)-phenyl)-2-thienyl]-3,4,5,6-tetrahydro-2H-thiopyran-2-acetamide 1,1-dioxide (105 mg)

NMR (DMSO-$d_6$, δ): 1.70-2.08 (4H, m), 2.34-2.48 (1H, m), 2.92-3.30 (4H, m), 3.42-3.54 (1H, m), 3.58 (2H, s), 3.73 (3H, s), 6.90 (2H, d, J=9Hz), 7.20 (1H, d, J=3Hz), 7.26 (2H, d, J=9Hz), 7.32-7.38 (2H, m), 7.40 (1H, d, J=3Hz), 7.44-7.53 (1H, m), 7.99 (1H, s), 8.84 (1H, br s), 10.24 (1H, s), 10.60 (1H, s)

MS (ESI-): 527 (M-H)

Example 152

(2S)-N-Hydroxy-2-[5-(3-(3-methoxyphenoxy)acetylamino)-phenyl)-2-thienyl]-3,4,5,6-tetrahydro-2H-thiopyran-2-acetamide 1,1-dioxide (110 mg)

NMR (DMSO-$d_6$, δ): 1.68-2.11 (4H, m), 2.32-2.50 (1H, m), 2.90-3.66 (5H, m), 4.71 (2H, s), 6.49-6.66 (2H, m), 7.13-7.26 (2H, m), 7.31-7.47 (2H, m), 7.52-7.72 (2H, m), 7.94-8.14 (1H, m), 8.70 (2H, br s), 10.23 (1H, s), 10.62 (1H, s)

MS (ESI-): 543 (M-H)

Example 153

(2S)-N-Hydroxy-2-[5-(3-(3-phenoxypropionylamino)-phenyl)-2-thienyl]-3,4,5,6-tetrahydro-2H-thiopyran-2-acetamide 1,1-dioxide (78 mg)

NMR (DMSO-$d_6$, δ): 1.69-2.12 (4H, m), 2.35-2.50 (1H, m), 2.82 (2H, t, J=7Hz), 2.92-3.56 (5H, m), 4.28 (2H, t, J=7Hz), 6.95 (3H, d, J=9Hz), 7.21 (1H, d, J=3Hz), 7.29 (2H, t, J=8Hz), 7.34-7.55 (4H, m), 8.03 (1H, s), 8.84 (1H, s), 10.21 (1H, s), 10.60 (1H, s)

MS (ESI-): 527 (M-H)

Example 154

(2S)-N-Hydroxy-2-[5-(3-(4-fluorophenoxy)acetylamino)phenyl)-2-thienyl]-3,4,5,6-tetrahydro-2H-thiopyran-2-acetamide 1,1-dioxide (75 mg)

NMR (DMSO-$d_6$, δ): 1.69-2.11 (4H, m), 2.34-2.49 (1H, m), 2.92-3.56 (5H, m), 4.71 (2H, s), 6.97-7.36 (5H, m), 7.83-7.46 (3H, m), 7.52-7.62 (1H, m), 8.02 (1H, s), 8.84 (1H, s), 10.20 (1H, s), 10.60 (1H, s)

MS (ESI-): 531 (M-H)

Example 155

(2S)-N-Hydroxy-2-[5-(3-(4-methoxyphenoxy)acetylamino)-phenyl)-2-thienyl]-3,4,5,6-tetrahydro-2H-thiopyran-2-acetamide 1,1-dioxide (89 mg)

NMR (DMSO-$d_6$, δ): 1.68-2.08 (4H, m), 2.35-2.50 (1H, m), 2.94-3.30 (4H, m), 3.41-3.54 (1H, m), 3.70 (3H, s), 4.65 (2H, s), 6.90 (2H, d, J=10Hz), 6.98 (2H, d, J=10Hz), 7.21 (1H, d, J=3Hz), 7.33-7.45 (3H, m), 7.54-7.62 (1H, m), 8.03 (1H, s), 8.84 (1H, s), 10.17 (1H, s), 10.60 (1H, s)

MS (ESI-): 543 (M-H)

Example 156

(2S)-N-Hydroxy-2-[5-(3-(2-(methylaminocarbonyloxy)-acetylamino)phenyl)-2-thienyl]-3,4,5,6-tetrahydro-2H-thiopyran-2-acetamide 1,1-dioxide (68 mg)

NMR (DMSO-$d_6$, δ): 1.64-2.08 (4H, m), 2.32-2.51 (1H, m), 2.61 (3H, s), 2.92-3.56 (5H, m), 4.57 (2H, s), 7.12-7.54 (7H, m), 7.98 (1H, s), 10.14 (1H, s), 10.61 (1H, s)

MS (ESI-): 494 (M-H)

Example 157

(2S)-N-Hydroxy-2-(5-phenyl-2-thienyl)-3,4,5,6-tetrahydro-2H-thiopyran-2-acetamide 1,1-dioxide (51 mg)

NMR (DMSO-$d_6$, δ): 1.70-2.07 (4H, m), 2.34-2.52 (1H, m), 2.94-3.07 (2H, m), 3.10-3.27 (2H, m), 3.30-3.50 (1H, m), 7.21 (1H, d, J=3.5Hz), 7.29-7.36 (1H, m), 7.43 (2H, t, J=8Hz), 7.48 (1H, d, J=3.5Hz), 7.65 (2H, d, J=8Hz) , 8.85 (1H, br s)

MS (ESI-): 364 (M-H)

Example 158

(2S)-N-Hydroxy-2-[5-(4-ethylaminocarbonylmethoxy)-phenyl)-2-thienyl]-3,4,5,6-tetrahydro-2H-thiopyran-2-acetamide 1,1-dioxide (35 mg)

NMR (BMSO-$d_6$, δ): 1.04 (3H, t, J=7.2Hz), 1.71-2.08 (4H, m), 1.35-1.45 (1H, m), 2.95-3.50 (7H, m), 4.89 (2H, s), 7.00 (2H, d, J=9.0Hz), 7.17 (1H, d, J=4.0Hz), 7.35 (1H, d, J=4.0Hz), 7.58 (2H, d, J=9.0Hz), 8.12 (1H, br), 8.84 (1H, s), 10.59 (1H, s)

Example 159

(2S)-N-Hydroxy-2-[5-(4-(methylaminocarbonylmethoxy)phenyl)-2-thienyl]-3,4,5,6-tetrahydro-2H-thiopyran-2-acetamide 1,1-dioxide (450 mg)

NMR (DMSO-$d_6$, δ): 1.70-2.07 (4H, m), 2.35-2.45 (1H, m), 2.65 (3H, d, J=4.5Hz), 2.95-3.50 (5H, m), 4.50 (2H, s), 7.01 (2H, d, J=9.0Hz), 7.16 (1H, d, J=4.0Hz), 7.35 (1H, d, J=4.0Hz), 7.57 (2H, d, J=9.0Hz), 8.06 (1H, br), 8.84 (1H, s), 10.57 (1H, s)

MS (ESI-): 451 (M-H)

Example 160

(2S)-N-Hydroxy-2-[5-(4-fluorophenyl)-2-thienyl]-2,3,4,5-tetrahydrothiophene-2-acetamide 1,1-dioxide (200 mg)

NMR (DMSO-$d_6$, δ): 2.14-2.35 (2H, m), 2.55-2.68 (1H, m), 2.80 (1H, d, J=15Hz), 2.90 (1H, d, J=15Hz), 3.05-3.40 (3H, m) , 7.17 (1H, d, J=4Hz), 7.26 (2H, d, J=9Hz), 7.45 (1H, d, J=4Hz), 7.70 (2H, dd, J=5, 9Hz), 8.88 (1H, s), 10.60 (1H, s)

MS (ESI-): 368 (M-H)

Example 161

(2S)-N-(2-Tetrahydropyranyloxy)-2-[5-(3-(acetoxyacetylamino)phenyl)-2-thienyl]-3,4,5,6-tetrahydro-2H-thiopyran-2-acetamide 1,1-dioxide (110 mg) was dissolved in 50% trifluoroacetic acid in dichloromethane (10 ml) and the reaction mixture was stirred at room temperature for 1 hour. After the mixture was concentrated in vacuo, the residue was purified by $SiO_2$ column chromatography (eluent: 2% MeOH in $CHCl_3$) to afford (2S)-N-hydroxy-2-[5-(3-(acetoxyacetylamino)phenyl)-2-thienyl]-3,4,5,6-tetrahydro-2H-thiopyran-2-acetamide 1,1-dioxide (65 mg) as a powder.

NMR (DMSO-$d_6$, δ): 1.69-2.07 (4H, m), 2.14 (3H, s), 2.32-2.47 (1H, m), 2.92-3.55 (5H, m), 4.67 (2H, s), 7.22 (1H, d, J=3Hz), 7.32-7.52 (3H, m), 7.97 (1H, s)

MS (ESI-): 479 (M-H)

Example 162

(2S)-N-Hydroxy-2-[5-(3-((2S)-2-acetoxypropionylamino)phenyl)-2-thienyl]-3,4,5,6-tetrahydro-2H-thiopyran-2-acetamide 1,1-dioxide (50 mg)

NMR (DMSO-$d_6$, δ): 1.44 (3H, d, J=8Hz), 1.72-2.08 (4H, m), 2.33-2.49 (1H, m), 2.93-3.32 (4H, m), 3.40-3.56 (1H, m), 5.04 (1H, q, J=8Hz), 7.22 (1H, d, J=3Hz), 7.32-7.53 (4H, m), 7.97 (1H, s), 8.84 (1H, s), 10.19 (1H, s), 10.59 (1H, s)

MS (ESI-): 493 (M-H)

Example 163

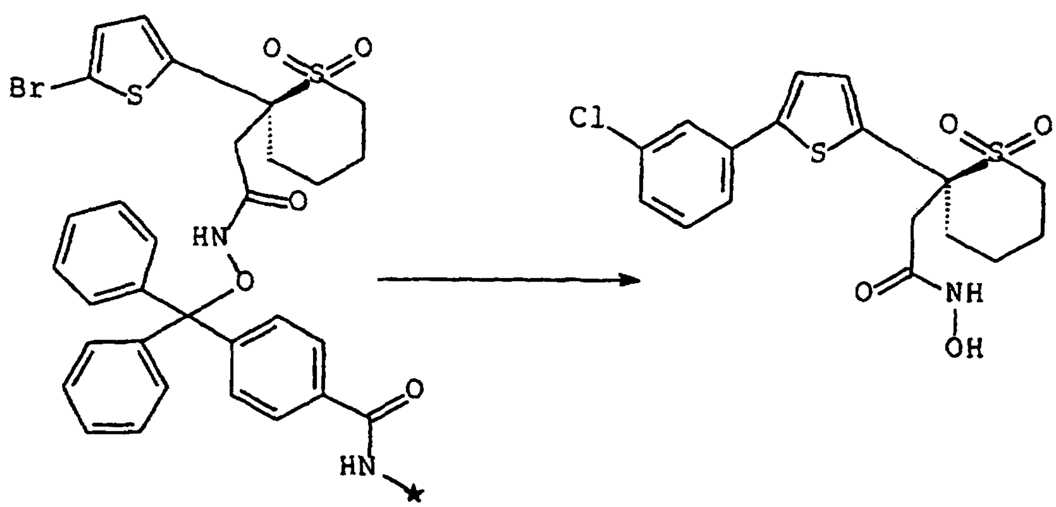

To a solution of 3-chlorophenylboronic acid (125 mg) in degassed N,N-dimethylformamide (0.5 ml) was added a suspension of tetrakis(triphenylphosphine)palladium (103 mg) in degassed N,N-dimethylformamide (2.5 ml), a solution of sodium carbonate (424 mg) in degassed water (1 ml) and N-[2-[2-(5-bromo-2-thienyl)-1,1-dioxo-3,4,5,6-tetrahydro-2H-thiopyran-2-yl]acetyl]hydroxylamine trityl crowns (59.2 µmol, 14.8 µmol/crown) in an atmosphere of nitrogen. After resulting mixture was heated for 48 hours at 60°C, the crowns were washed with degassed N,N-dimethylformamide, a solution of sodium diethylditiocarbamate (500 mg) and diisopropylethylamine (0.5 ml) in N,N-dimethylformamide (100 ml), N,N-dimethylformamide, methyl sulfoxide, water, methanol and dichloromethane, successively. The crowns were treated with 5% trifluoroacetic acid in dichloromethane for 1 hour at ambient temperature and removed from the solution. After the solution was evaporated under a stream of nitrogen, the residue was purified by HPLC (0.1% trifluoroacetic acid in 30% ethanol-hexane) to give (2S)-N-hydroxy-2-[5-(3-chlorophenyl)-2-thienyl]-3,4,5,6-tetrahydro-2H-thiopyran-2-acetamide 1,1-dioxide (4.5 mg) as a powder.

MS (ESI+): 400.2 (M+H)

The Object Compounds listed in the Table were obtained from the Starting Compounds 1 and 2 in a similar manner to that of Example 163 according to the following reaction scheme.

Reaction Scheme: (Examples 164 to 185 )

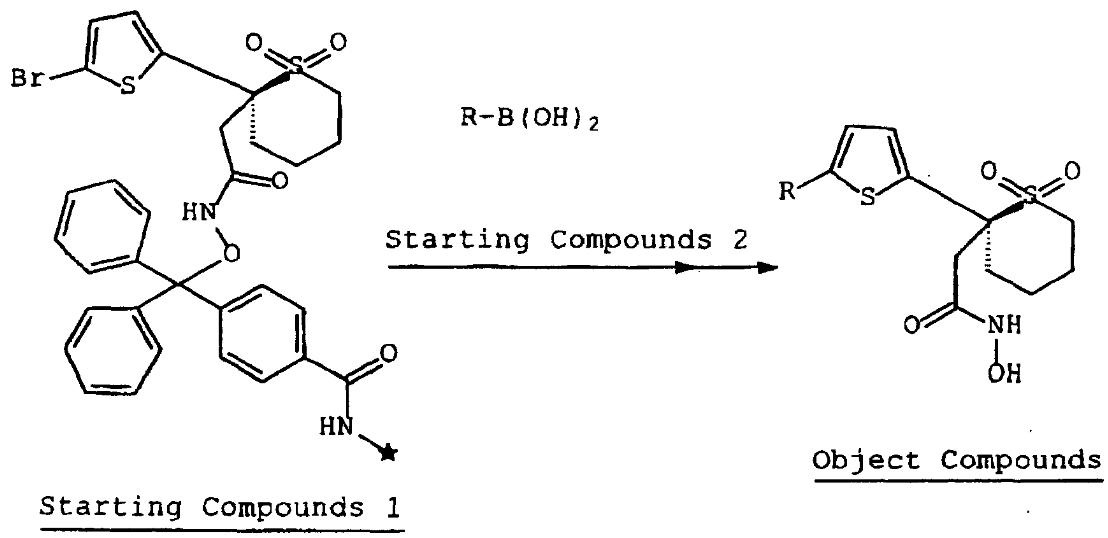

Table

| Example Nos. | Object Compounds R | Physicochemical Data |
|---|---|---|
| 164 | 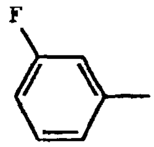 | MS (ESI+): 384.3 (M+H) |
| 165 | 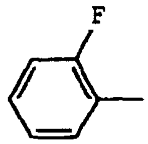 | MS (ESI+): 384.3 (M+H) |
| 166 | 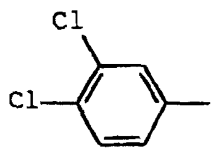 | MS (ESI+): 434.2 (M+H) |
| 167 | 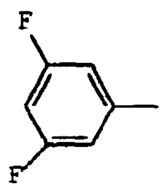 | MS (ESI+): 402.2 (M+H) |
| 168 | 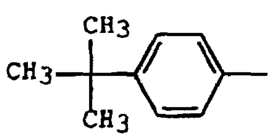 | MS (ESI+): 422.4 (M+H) |
| 169 | 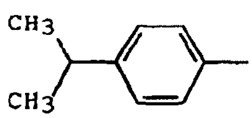 | MS (ESI+): 408.2 (M+H) |

| Example Nos. | Object Compounds | |
|---|---|---|
| | R | Physicochemical Data |
| 170 | 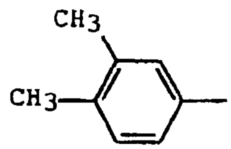 | MS (ESI+): 394.2 (M+H) |
| 171 | 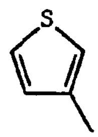 | MS (ESI+) 372.2 (M+H) |
| 172 | 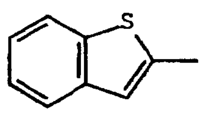 | MS (ESI+): 422.2 (M+H) |
| 173 | 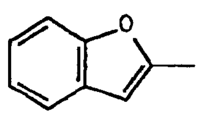 | MS (ESI-): 568.4 (M-H) |
| 174 | 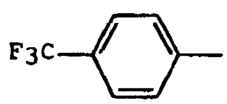 | MS (ESI+): 434.3 (M+H) |
| 175 | 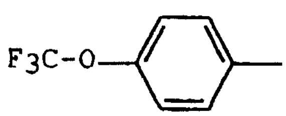 | MS (ESI+): 450.2 (M+H) |
| 176 | 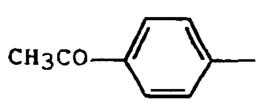 | MS (ESI+): 408.3 (M+H)<br>(2R or 2S) |

| Example Nos. | Object Compounds | |
|---|---|---|
| | R | Physicochemical Data |
| 177 | 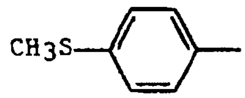 | MS (ESI+): 412.3 (M+H) |
| 178 | 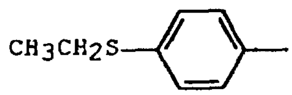 | MS (ESI+): 426.2 (M+H) |
| 179 | 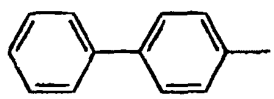 | MS (ESI+): 442.3 (M+H) |
| 180 | 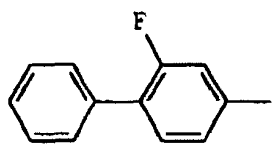 | MS (ESI+): 501.2 (M+H) |
| 181 | 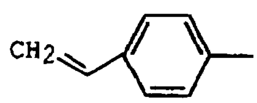 | MS (ESI-): 568.4 (M-H) |
| 182 | 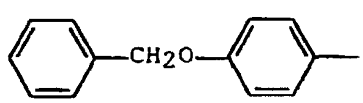 | MS (ESI+): 513.3 ($M+H+CH_3CN$) |
| 183 | 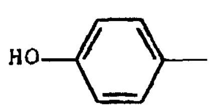 | MS (ESI+): 382.3 (M+H) (2R or 2S) |

| Example Nos. | Object Compounds | |
|---|---|---|
| | R | Physicochemical Data |
| 184 | 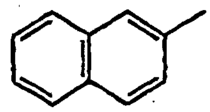 | MS (ESI+): 416.3 (M+H) |
| 185 | 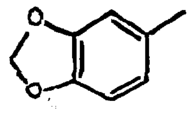 | MS (ESI+): 410.3 (M+H) |

The following compounds were obtained in a similar manner to that of Example 52.

Example 186

(2S)-N-Hydroxy-2-[5-(3-aminoacetylamino)phenyl)-2-thienyl]-3,4,5,6-tetrahydro-2H-thiopyran-2-acetamide 1,1-dioxide hydrochloride (3.0 g) from (2S)-N-(2-tetrahydropyranyloxy)-2-[5-(3-(2-(t-butoxycarbonylamino)-acetylamino)phenyl)-2-thienyl]-3,4,5,6-tetrahydro-2H-thiopyran-2-acetamide 1,1-dioxide

NMR (DMSO-$d_6$, δ): 1.68-2.10 (4H, m), 2.34-2.48 (1H, m), 2.92-3.62 (5H, m), 3.76-3.88 (2H, m), 7.23 (1H, d, J=3Hz), 7.36-7.46 (3H, m), 7.49-7.56 (1H, m), 7.99 (1H, s), 10.69 (1H, s), 10.92 (1H, s)

MS (ESI+): 438 (M+H)

Example 187

(2S)-N-Hydroxy-2-[5-(3-(3-(N-methylamino)-propionylamino)phenyl)-2-thienyl]-3,4,5,6-tetrahydro-2H-thiopyran-2-acetamide 1,1-dioxide hydrochloride (112 mg) from (2S)-N-(2-tetrahydropyranyloxy)-2-[5-(3-(3-(N-t-butoxycarbonyl-N-methylamino)propionylamino)phenyl)-2-thienyl]-3,4,5,6-tetrahydro-2H-thiopyran-2-acetamide 1,1-dioxide

NMR (DMSO-$d_6$, δ): 1.68-2.11 (4H, m) , 2.32-2.47 (1H, m), 2.61 (3H, t, J=4Hz), 2.78 (2H; t, J=7Hz), 2.90-3.30 (6H, m), 3.38-3.62 (1H, m), 7.22 (1H, d, J=3Hz), 7.32-7.50 (4H, m), 8.04 (1H, s), 8.36-8.58 (2H, m) , 10.33 (1H, s) , 10.61 (1H, s)

MS (ESI+): 466 (M+H)

Example 188

(2S)-N-Hydroxy-2-[5-{3-(((2S)-2-amino-3-(3-pyridyl)propionyl)amino)phenyl}-2-thienyl]-3,4,5,6-tetrahydro-2H-thiopyran-2-acetamide 1,1-dioxide hydrochloride (500 mg) from (2S)-N-(2-tetrahydropyranyloxy)-2-[5-(3-((2S)-2-(t-butoxycarbonylamino)-3-(3-pyridyl)propionylamino)phenyl}-2-thienyl]-3,4,5,6-tetrahydro-2H-thiopyran-2-acetamide 1,1-dioxide

NMR (DMSO-$d_6$, δ): 1.74-2.06 (4H, m), 2.35-2.46 (1H, m), 2.97-3.34 (4H, m), 3.46-3.55 (3H, m), 4.24 (1H, br), 7.23 (1H, d, J=4.0Hz), 7.41-7.44 (3H, m), 7.56 (1H, d, J=7.0Hz), 7.88 (1H, dd, J=7.0, 7.0Hz), 7.95 (1H, s), 8.38 (1H, d, J=7.0Hz), 8.48 (2H, br), 8.77 (1H, br), 8.87 (1H, br s)

MS (ESI+): 529.1 (M+H)

Example 189

(2S)-N-Hydroxy-2-[5-(3-(2-(N-methylamino)acetylamino)-phenyl)-2-thienyl]-3,4,5,6-tetrahydro-2H-thiopyran-2-acetamide 1,1-dioxide hydrochloride (78 mg) from (2S)-N-(2-tetrahydropyranyloxy)-2-[5-(3-(2-(N-t-butoxycarbonylamino)-acetylamino)phenyl)-2-thienyl]-3,4,5,6-tetrahydro-2H-thiopyran-2-acetamide 1,1-dioxide

NMR (DMSO-$d_6$, δ): 1.65-2.08 (4H, m), 2.31-2.49 (1H, m), 2.63 (2H, t, J=7Hz), 2.92-3.52 (5H, m), 3.92-4.00 (2H, m), 7.23 (1H, d, J=3Hz), 7.34-7.45 (3H, m), 7.49-7.57 (1H, m), 7.98 (1H, s), 8.97-9.14 (2H, m), 10.67 (1H, s), 10.94 (1H, s)

MS (ESI+): 454 (M+H)

Example 190

(2S)-N-Hydroxy-2-[5-(3-(3-aminopropionylamino)phenyl)-2-thienyl]-3,4,5,6-tetrahydro-2H-thiopyran-2-acetamide 1,1-dioxide hydrochloride (78 mg) from (2S)-N-(2-tetrahydropyranyloxy)-2-[5-(3-(3-(t-butoxycarbonylamino)-propionylamino)phenyl)-2-thienyl]-3,4,5,6-tetrahydro-2H-thiopyran-2-acetamide 1,1-dioxide

NMR (DMSO-$d_6$, δ): 1.68-2.09 (4H, m), 2.32-2.50 (1H, m), 2.78 (2H, d, J=8Hz), 2.92-3.62 (7H, m), 7.23 (1H, d, J=3Hz), 7.32-7.57 (4H, m), 7.92-8.16 (4H, m), 10.45 (1H, s), 10.67 (1H, s)

MS (ESI+): 454 (M+H)

Example 191

(2S)-N-Hydroxy-2-[5-[3-(((2S)-2-amino-3-methoxypropionyl)amino)phenyl]-2-thienyl]-3,4,5,6-tetrahydro-2H-thiopyran-2-acetamide 1,1-dioxide hydrochloride (1.64 g) from (2S)-N-(2-tetrahydropyranyloxy)-2-[5-[3-(((2S)-2-(t-butoxycarbonylamino)-3-methoxypropionyl)amino)phenyl]-2-thienyl]-3,4,5,6-tetrahydro-2H-thiopyran-2-acetamide 1,1-dioxide

NMR (DMSO-$d_6$, δ): 1.70-2.10 (4H, m), 2.34-2.53 (1H, m), 2.94-3.06 (2H, m), 3.10-3.56 (6H, m), 3.74-3.86 (2H, m), 4.17-4.29 (1H, m), 7.22 (1H, d, J=3.5Hz), 7.38-7.46 (3H, m), 7.53-7.59 (1H, m), 7.95 (1H, s), 8.30-8.44 (2H, m), 8.84 (1H, br s), 10.63 (1H, s), 10.85 (1H, br s)

MS (ESI+): 482 (M+H)

Example 192

(2S)-N-Hydroxy-2-[5-(3-(2-hydroxyacetylamino)phenyl)-2-thienyl]-3,4,5,6-tetrahydro-2H-thiopyran-2-acetamide 1,1-dioxide (58 mg) from (2S)-N-(2-tetrahydropyranyloxy)-2-[5-(3-(2-methoxycarbonyloxyacetylamino)phenyl)-2-thienyl]-3,4,5,6-tetrahydro-2H-thiopyran-2-acetamide 1,1-dioxide

NMR (DMSO-$d_6$, δ): 1.70-2.12 (4H, m), 2.32-2.49 (1H, m), 2.91-3.28 (4H, m), 3.39-3.55 (1H, m), 4.01 (2H, d, J=7Hz), 5.71 (1H, t, J=7Hz), 7.22 (1H, d, J=3Hz), 7.38-7.40 (2H, m), 7.40 (1H, d, J=3Hz), 7.60-7.72 (1H, m), 8.08 (1H, s), 8.85 (1H, s), 9.79 (1H, s), 10.61 (1H, s)

MS (ESI-): 437 (M-H).

Example 193

(2S)-N-Hydroxy-2-[5-(3-(((2S)-2-hydroxypropionyl)-amino)phenyl)-2-thienyl]-3,4,5,6-tetrahydro-2H-thiopyran-2-acetamide 1,1-dioxide (65 mg) from (2S)-N-(2-tetrahydropyranyloxy)-2-[5-(3-(((2S)-2-acetoxypropionyl)amino)phenyl)-2-thienyl]-3,4,5,6-tetrahydro-2H-tbiopyran-2-acetamide 1,1-dioxide

NMR (DMSO-$d_6$, δ): 1.33 (3H, br s), 1.67-2.12 (4H, m), 2.30-2.48 (1H, m), 2.90-3.71 (5H, m), 4.09-4.24 (1H, m), 7.21 (1H, br s), 7.38-7.48 (3H, m), 7.68-7.75 (1H, m), 8.11 (1H, s), 9.26 (1H, s), 10.60 (1H, s)

MS (ESI-): 451 (M-H)

Example 194

To a solution of (2S)-N-2-(tetrahydropyranyloxy)-2-[5-(3-(3-aminopropionylamino)phenyl)-2-thienyl]-3,4,5,6-tetrahydro-2H-thiopyran-2-acetamide 1,1-dioxide (180 mg) in chloroform (5 ml) and pyridine (1 ml) was added a solution of methoxycarbonyl chloride (38.1 mg) at room temperature. After being stirred at the same temperature overnight, the mixture was concentrated in vacuo. The residue was dissolved in ethyl acetate (10 ml) and the solution was washed successively with a 5% citric acid solution, a saturated sodium bicarbonate solution and brine, dried over magnesium sulfate, and concentrated in vacuo. After the residue was dissolved in 1% hydrogen chloride in methanol (5 ml), the mixture was stirred at room temperature for 15 minutes and concentrated in vacuo. The residue was purified by $SiO_2$ column chromatography (eluent: 2% MeOH in $CHCl_3$) to afford (2S)-N-hydroxy-2-[5-(3-(3-(methoxycarbonylamino)propionylamino)phenyl)-2-thienyl]-3,4,5,6-tetrahydro-2H-thiopyran-2-acetamide 1,1-dioxide (63 mg) as a powder.

NMR (DMSO-$d_6$, δ): 1.22-2.11 (4H, m), 2.35-2.56 (3H, m), 2.94-3.33 (6H, m), 3.42-3.58 (1H, m), 7.20 (1H, d, J=3Hz), 7.21-7.38 (1H, m), 7.32-7.38 (2N, m), 7.40 (1H, d, J=3Hz), 7.42-7.50 (1H, m), 8.01 (1H, s), 8.85 (1H, m)

MS (ESI-): 508 (M-H)

Example 195

To a solution of (2S)-N-(2-tetrahydropyranyloxy)-2-[5-(4-(t-butyloxycarbonylmethoxy)phenyl)-2-thienyl]-3,4,5,6-tetrahydro-2H-thiopyran-2-acetamide 1,1-dioxide (220 mg) in tetrahydrofuran (THF):$H_2O$= 2:1 (3 ml) was added lithium hydroxide monohydrate (23.9 mg) at room temperature. After being stirred at the same temperature overnight, the reaction mixture was concentrated in vacuo. The resulting residue was diluted with ethyl acetate, washed with 1% citric acid solution and brine, dried over sodium sulfate and concentrateed in vacuo to give (2S)-N-(2-tetrahydropyranyloxy)-2-[5-(4-carboxymethoxyphenyl)-2-thienyl]-3,4,5,6-tetrahydro-2H-thiopyran-2-acetamide 1,1-dioxide (190 mg) as an amorphous solid.

NMR (DMSO-$d_6$, δ): 1.35-1.60 (6H, m), 1.66-1.71 (2H, m), 1.73-2.05 (2H, m), 2.34-2.46 (1H, m), 2.90-3.50 (6H, m), 3.72-3.90 (1H, m), 4.45, 4.75 (1H, s), 4.72 (2H, s), 6.96 (2H, d, J=9.0Hz), 7.16 (1H, d, J=4.0Hz), 7.32 (1H, d, J=4.0Hz), 7.56 (2H, d, J=9.0Hz), 11.2 (1H, s)

MS (ESI-): 522 (M-H)

Example 196

To the reaction mixture of 4-(5-oxazolyl)-benzeneboronic acid pinacol cyclic ester obtained in Preparation 24-2) was added (2S)-N-(2-tetrahydropyranyloxy)-2-(5-bromo-2-thienyl)-3,4,5,6-tetrahydro-2H-thiopyran-2-acetamide 1,1-dioxide (679 mg), tetrakis(triphenylphosphine)palladium(0) (8.67 mg) and aqueous 2M sodium carbonate (7.5 ml) at room temperature. The mixture was stirred for 3 hours at 80°C and taken up between ethyl acetate and 3% aqueous sodium bicarbonate. The separated organic layer was washed with brine, dried over sodium sulfate and evaporated in vacuo. The residue was purified by chromatography on silica gel (eluted with 0.5 to 3% methanol in chloroform) to give (2S)-N-(2-tetrahydropyranyloxy)-2-[5-[4-(5-oxazolyl)phenyl]-2-thienyl]-3,4,5,6-tetrahydro-2H-thiopyran-2-acetamide 1,1-dioxide (795 mg) as an amorphous solid.

NMR ($CDCl_3$, δ): 1.40-1.55 (4H, m), 1.62-1.75 (2H, m), 1.91-2.02 (2H, m), 2.05-2.25 (2H, m), 2.70-2.94 (2H, m), 3.01-3.17 (4H, m), 3.29-3.51 (1H, m), 3.64-3.74 (1H, m), 4.53 (0.5Hz, s), 4.82 (0.5H, s), 7.26-7.34 (2H, m), 7.39 (1H, s), 7.65 (4H, s), 7.94 (1H, s), 8.25 (0.5H, s), 8.37 (0.5H, s)

MS (ESI-): 515 (M-H)

The following compounds were obtained in a similar manner to that of Example 85.

Example 197

(2S)-N-(2-Tetrahydropyranyloxy)-2-[5-(3-formylphenyl)-2-thienyl]-3,4,5,6-tetrahydro-2H-thiopyran-2-acetamide 1,1-dioxide (0.9 g)

NMR ($CDCl_3$, δ): 1.40-1.75 (6H, m), 1.90-2.02 (2H, m), 2.05-2.26 (2H, m), 2.70-2.93 (2H, m), 3.01-3.19 (2H, m), 3.28-3.53 (1H, m), 3.62-3.75 (1H, m), 4.53, 4.83 (1H, s), 7.26-7.37 (2H, m), 7.56 (1H, dd, J=8.0, 8.0Hz), 7.80-7.85 (2H, m), 8.09 (1H, s), 10.05 (1H, s)

MS (ESI-): 476 (M-H)

Example 198

(2S)-N-(2-Tetrahydropyranyloxy)-2-[5-(4-ethenylphenyl)-2-thienyl]-3,4,5,6-tetrahydro-2H-thiopyran-2-acetamide 1,1-dioxide (2.1 g)

NMR ($CDCl_3$, δ) : 1.37-1.75 (6H, m) , 1.86-2.00 (2H, m), 2.05-2.25 (2H, m), 2.65-2.91 (2H, m), 3.00-3.17 (4H, m), 3.26-3.49 (1H, m), 3.59-3.70 (1H, m), 4.51, 4.81 (1H, s), 5.28 (1H, d, J=11Hz), 5.78 (1H, d, J=17.7Hz), 6.66-6.75 (1H, m), 7.41 (2H, d, J=8.0Hz), 7.55 (2H, d, J=8.0Hz)

MS (ESI-): 474 (M-H)

Example 199

(2S)-N-(2-Tetrahydropyranyloxy)-2-[5-(4-(2-carboxyethenyl)phenyl)-2-thienyl]-3,4,5,6-tetrahydro-2H-thiopyran-2-acetamide 1,1-dioxide (1.1 g)

NMR ($CDCl_3$, δ): 1.41-1.71 (6H, m), 1.92-2.25 (4H, m), 2.76-2.88 (2H, m), 3.05-3.19 (4H, m), 3.30-3.51 (1H, m), 3.69-3.76 (1H, m), 4.54, 4.84 (1H, s), 6.40 (1H, d, J=15Hz), 7.45-7.74 (7H, m)

MS (ESI-): 518 (M-H)

Example 200

(2S)-N-(2-Tetrahydropyranyloxy)-2-(5-phenyl-2-thienyl)-3,4,5,6-tetrahydro-2H-thiopyran-2-acetamide 1,1-dioxide (93 mg)

NMR ($CDCl_3$, δ): 1.39-1.75 (6H, m) , 1.90-2.00 (2H, m), 2.04-2.24 (2H, m), 2.63-2.91 (2H, m), 3.03-3.18 (4H, m), 3.27-3.50 (1H, m), 3.59-3.70 (1H, m), 4.53 (0.5H, s), 4.80 (0.5H, s), 7.29-7.41 (5H, m), 7.56-7.63 (2H, m), 7.95 (0.5H, s), 8.12 (0.5H, s)

MS (ESI-): 448 .(M-H)

Example 201

(2S)-N-(2-Tetrahydropyranyloxy)-2-[5-(4-(t-butyloxycarbonylmethoxy)phenyl)-2-thienyl]-3,4,5,6-tetrahydro-2H-thiopyran-2-acetamide 1,1-dioxide (3.8 g)

NMR (DMSO-$d_6$, δ): 1.35-1.60 (15H, m), 1.65-1.71 (2H, m), 1.73-2.04 (2H, m), 2.34-2.45 (1H, m), 2.90-3.50 (6H, m), 3.72-3.90 (1H, m), 4.45, 4.75 (1H, s), 4.73 (2H, s), 7.01 (2H, d, J=9.0Hz), 7.15 (1H, d, J=4.0Hz), 7.33 (1H, d, J=4.0Hz), 7.55 (2H, d, J=9.0Hz)

Example 202

(2S)-N-(2-Tetrahydropyranyloxy)-2-[5-(4-(ethylaminocarbonylmethoxy)phenyl)-2-thienyl]-3,4,5,6-tetrahydro-2H-thiopyran-2-acetamide 1,1-dioxide (65 mg) was obtained in a similar manner to that of Example 201.

NMR (DMSO-$d_6$, δ): 1.04 (3H, t, J=7.2Hz), 1.37-1.63 (6H, m), 1.69-1.81 (2H, m), 1.83-2.04 (2H, m), 2.35-2.47 (1H, m), 2.87-3.51 (8H, m), 3.74-3.90 (1H, m), 3.95, 4.75 (1H, s), 4.49 (2H, s), 7.00 (2H, d, J=9.0Hz), 7.15-7.20 (1H, m), 7.33-7.36 (1H, m), 7.58 (2H, d, J=9.0Hz), 8.13 (1H, br)

Example 203

(2S)-N-(2-Tetrahydropyranyloxy)-2-[5-(3-(propylaminocarbonylamino)phenyl)-2-thienyl]-3,4,5,6-tetrahydro-2H-thiopyran-2-acetamide 1,1-dioxide (140 mg)

NMR (DMSO-$d_6$, δ): 0.88 (3H, t, J=7Hz), 1.26-2.09 (12H, m), 2.43-2.46 (1H, m), 2.78-3.56 (6H, m), 3.72-3.92 (1H, m), 4.44, 4.75 (1H, s), 6.11-6.24 (1H, m), 7.09-7.42 (6H, m), 7.85 (1H, s), 8.55 (1H, s)

MS (ESI-): 548 (M-H)

The following compounds were obtained in a similar manner to that of Example 124.

Example 204

(2S)-N-(2-Tetrahydropyranyloxy)-2-[5-(3-(isopropylaminocarbonylamino)phenyl)-2-thienyl]-3,4,5,6-tetrahydro-2H-thiopyran-2-acetamide 1,1-dioxide (160 mg)

NMR (DMSO-$d_6$, δ): 1.10 (6H, m), 1.30-2.10 (10H, m), 2.34-2.47 (1H, m), 2.83-3.30 (5H, m), 3.40-3.53 (1H, m), 3.18-3.90 (2H, m), 4.36, 4.44 (1H, s), 6.04 (1H, d, J=8Hz), 7.09-7.40 (6H, m), 7.85 (1H, s), 8.44 (1H, s)

MS (ESI-): 548 (M-H)

Example 205

(2S)-N-(2-Tetrahydropyranyloxy)-2-[5-{3-(2-chloroethylaminocarbonylamino)phenyl}-2-thienyl]-3,4,5,6-tetrahydro-2H-thiopyran-2-acetamide 1,1-dioxide (335 mg)

NMR ($CDCl_3$, δ): 1.46 (2H, br), 1.62-1.67 (4H, m), 1.84-1.96 (2H, m), 2.74-2.88 (2H, m), 2.96-3.06 (2H, m), 3.08-3.14 (2H, m), 3.31-3.41 (2H, m), 3.49-3.54 (1H, m), 3.55-3.60 (2H, m), 3.66 (2H, t, J=6.0Hz), 3.70-3.80 (1H, m), 4.48 (1/2H, br), 4.84 (1/2H, br), 7.17-7.24 (4H, m), 7.30-7.50 (5H, m)

MS (ESI-): 568.4 (M-H)

Example 206

To a solution of (2S)-N-(2-tetrahydropyranyloxy)-2-[5-(3-aminophenyl)-2-thienyl]-3,4,5,6-tetrahydro-2H-thiopyran-2-acetamide 1,1-dioxide (7.00 g), ethoxyacetic acid (2.04 g) and 1-hydroxybenzotriazole (2.65 g) in N,N-dimethylformamide (80 ml) was added 1-ethyl-3-(3-dimethylaminopropyl)-carbodiimide hydrochloride (WSCD HCl) (3.75 g) at room temperature. After being stirred at the same temperature overnight, the mixture was concentrated in vacuo. The residue was dissolved in ethyl acetate (200 ml) and the solution was washed successively with 5% citric acid

solution, a saturated.aqueous sodium bicarbonate solution and brine, dried over magnesium sulfate, and concentrated in vacuo. The residue was purified by $SiO_2$ column chromatography (eluent 1% MeOH in $CHCl_3$) to afford (2S)-N-(2-tetrahydropyranyloxy)-2-[5-(3-(2-ethoxyacetylamino)phenyl)-2-thienyl]-3,4,5,6-tetrahydro-2H-thiopyran-2-acetamide 1,1-dioxide (7.00 g).

NMR (DMSO-$d_6$, δ): 1.20 (3H, t, J=7Hz), 1.33-1.62 (6H,. m), 1.70-2.12 (4H, m), 2.35-2.50 (1H, m), 2.88-3.22 (5H, m), 3.38-3.52 (1H, m), 3.58 (2H, q, J=7Hz), 3.75-3.92 (1H, m), 4.44, 4.75 (1H, s), 7.18-7.25 (1H, m), 7.34-7.45 (3H, m), 7.55-7.64 (1H, m), 8.03 (1H, s), 9.81 (1H, s), 11.24 (1H, s)

MS (ESI-): 549 (M-H)

The following compounds were obtained in substantially the same manner as that of Example 206.

Example 207

(2S)-N-(2-Tetrahydropyranyloxy)-2-[5-(3-(2-(methylaminocarbonyloxy)acetylamino)phenyl)-2-thienyl]-3,4,5,6-tetrahydro-2H-thiopyran-2-acetamide 1,1-dioxide (144 mg)

NMR (DMSO-$d_6$, δ): 1.36-2.10 (10H, m), 2.36-2.51 (1H, m), 2.60 (3H, d, J=6Hz), 2.87-3.30 (5H, m), 3.40-3.54 (1H, m), 3.72-3.90 (1H, m), 4.43, 4.76 (1H, m), 4.57 (2H, s), 7.18-7.30 (2H, m), 7.35-7.52 (4H, m), 7.99 (1H, s), 10.14 (1H, s), 11.25 (1H, s)

MS (ESI-): 578 (M-H)

Example 208

(2S)-N-(2-Tetrahydropyranyloxy)-2-[5-[3-((2S)-2-(tert-butoxycarbonylamino)-3-methoxypropionylamino)phenyl]-2-thienyl]-3,4,5,6-tetrahydro-2H-thiopyran-2-acetamide 1,1-dioxide (2.90 g)

NMR ($CDCl_3$, δ): 1.37-1.51 (11H, m), 1.59-2.01 (6H, m), 2.04-2.25 (2H, m), 2.64-2.91 (2H, m), 3.00-3.17 (4H, m), 3.26-3.50 (4H, m), 3.53-3.72 (2H, m), 3.80-3.94 (1H, m), 4.34-4.45 (1H, m), 4.53 (0.5H, s), 4.82 (0.5H, s), 5.45-5.59 (1H, m), 7.20-7.36 (5H, m), 7.43-7.51 (1H, m), 7.75-7.83 (1H, m), 8.18 (0.5H, s), 8.31 (0.5H, s), 8.49 (1H, br s)

MS (ESI+): 683 (M+H+$NH_3$)

Example 209

(2S)-N-(2-Tetrahydropyranyloxy)-2-[5-(3-(2-(4-methoxyphenyl)acetylamino)phenyl)-2-thienyl]-3,4,5,6-tetrahydro-2H-thiopyran-2-acetamide 1,1-dioxide (160 mg)

NMR ($CDCl_3$, δ): 1.36-1.76 (8H, m), 1.86-1.98 (2H, m), 2.04-2.22 (2H, m), 2.69-2.92 (2H, m), 3.02-3.17 (2H, m), 3.26-3.51 (1H, m), 3.62-3.73 (1H, m), 3.68 (2H, s), 3.82 (3H, s), 4.52, 4.72 (1H, s), 6.87-6.95 (2H, m), 7.13-7.32 (6H, m), 1.45-7.56 (3H, m), 8.56, 8.59 (1H, s)

MS (ESI-): 611 (M-H)

Example 210

(2S)-N-(2-Tetrahydropyranyloxy)-2-[5-(3-(2-(tert-butoxycarbonylamino)acetyl)amino)phenyl)-2-thienyl]-3,4,5,6-tetrahydro-2H-thiopyran-2-acetamide 1,1-dioxide (246 mg)

NMR (DMSO-$d_6$, δ): 1.26-1.63 (6H, m), 1.40 (9H, s), 1.68-2.06 (4H, m), 2.35-2.48 (1H, m), 2.88-3.30 (5H, m), 3.36-3.53 (1H, m), 3.74 (2H, d, J=7Hz), 3.72-3.92 (1H, m), 4.43, 4.75 (1H, s), 7.03-7.12 (1H, m), 7.18-7.24 (1H, m), 7.40-7.53 (4H, m), 7.98 (1H, s), 10.05 (1H, s), 11.25 (1H, s)

MS (ESI-): 620 (M-H)

Example 211

(2S)-N-(2-Tetrahydropyranyloxy)-2-[5-(3-propoxyacetylamino)phenyl)-2-thienyl]-3,4,5,6-tetrahydro-2H-thiopyran-2-acetamide 1,1-dioxide (3.68 g)

NMR ($CDCl_3$, δ): 1.01 (3H, t, J=7Hz), 1.36-1.78 (10H, m), 1.88-1.99 (2H, m), 2.03-2.25 (2H, m), 2.65-2.93 (2H, m), 2.98-3.18 (2H, m), 3.28-3.52 (1H, m), 3.58 (2H, t, J=7Hz), 3.62-3.74 (1H, m), 4.07 (2H, s), 4.52, 4.82 (1H, s), 7.23-7.38 (4H, m), 7.52-7.59 (1H, m), 7.80 (1H, s), 8.19, 8.32 (1H, s), 8.56 (1H, s)

MS (ESI-): 563 (M-H)

Example 212

(2S)-N-(2-Tetrahydropyranyloxy)-2-[5-(3-(n-butyloxyacetylamino)phenyl}-2-thienyl]-3,4,5,6-tetrahydro-2H-thiopyran-2-acetamide 1,1-dioxide (140 mg)

NMR ($CDCl_3$, δ): 1.95 (2H, br), 2.08-2.23 (2H, m), 2.64-2.88 (2H, m), 3.06 (2H, s), 3.16 (2H, br), 3.39-3.50 (2H, m), 3.62 (2H, t, J=7.5Hz), 4.06 (2H, s), 4.54 (1/2H, br), 4.80 (1/2H, br), 7.21-7.30 (2H, m), 7.34-7.36 (2H, m), 7.52-7.56 (1H, m), 7.80 (1H, s), 7.94 (1/2H, s), 8.12 (1/2H, s), 8.34 (1H, s)

MS (ESI-): 577.3 (M-H)

Example 213

(2S)-N-(2-Tetrahydropyranyloxy)-2-[5-{3-(3-methoxypropionylamino)phenyl}-2-thienyl]-3,4,5,6-tetrahydro-2H-thiopyran-2-acetamide 1,1-dioxide (4.34 g)

NMR ($CDCl_3$, δ): 1.46 (2H, br), 1.57-1.70 (2H, m), 1.95 (2H, br), 2.07-2.20 (2H, m), 2.66 (2H, t, J=6.0Hz), 2.70-2.89 (2H, m), 3.06 (2H, s), 3.10-3.16 (2H, m), 3.26-3.44 (1H, m), 3.47 (3H, s), 3.62-3.70 (2H, m), 3.75 (2H, t, J=6.0Hz), 4.52 (1/2H, br), 4.82 (1/2H, br), 7.20-7.25 (2H, m), 7.28-7.31 (2H, m), 7.46-7.51 (1H, m), 7.70-7.73 (1H, m), 8.18 (1/2H, s), 8.31 (1/2H, s), 8.31 (1H, s)

MS (ESI-): 549.4 (M-H)

Example 214

(2S)-N-(2-Tetrahydropyranyloxy)-2-[5-{3-(methoxyacetylamino)phenyl}-2-thienyl]-3,4,5,6-tetrahydro-2H-thiopyran-2-acetamide 1,1-dioxide (3.24 g).

NMR ($CDCl_3$, δ): 1.46 (4H, br), 1.55-1.58 (2H, m), 1.94 (2H, br), 2.07-2.20 (2H, m), 2.66-2.88 (2H, m), 3.05 (2H, s), 3.09-3.14 (2H, m), 3.27-3.48 (1H, m), 3.53 (3H, s), 3.60-3.72 (1H, m), 4.04 (2H, s), 4.52 (1/2H, br), 4.81 (1/2H, br), 7.23-7.30 (2H, m), 7.43-7.46 (2H, m), 7.55-7.59 (1H, in), 7.80 (1H, s), 8.04 (1/2H, s), 8.20 (1/2H, s), 8.30 (1H, s)

MS (ESI-): 535.3 (M-H)

Example 215

(2S)-N-(2-Tetrahydropyranyloxy)-2-[5-(3-(3-(9-fluorenylmethoxycarbonylamino)propionylamino)phenyl)-2-thienyl]-3,4,5,6-tetrahydro-2H-thiopyran-2-acetamide 1,1-dioxide (2.70 g)

NMR ($CDCl_3$, δ): 1.31-1.81 (8H, m), 1.84-2.28 (4H, m), 2.58-2.76 (2H, m), 2.92-3.22 (4H, m), 3.30-3.84 (4H, m), 4.02-4.22 (2H, m), 4.37-4.49 (1H, m), 4.62, 4.94 (1H, s), 6.89-7.90 (13H, m), 7.99-8.14 (2H, m), 8.66, 8.78 (1H, s), 10.00, 10.46 (1H, s)

MS (ESI-): 756 (M-H)

Example 216

(2S)-N-(2-Tetrahydropyranyloxy)-2-[5-(3-phenoxyacetylamino)phenyl)-2-thienyl]-3,4,5,6-tetrahydro-2H-thiopyran-2-acetamide 1,1-dioxide (4.90 g)

NMR ($CDCl_3$, δ): 1.37-1.76 (8H, m), 1.88-1.99 (2H, m), 2.05-2.26 (2H, m), 2.67-2.92 (2H, m), 2.98-3.18 (2H, m), 3.28-3.51 (1H, m), 3.62-3.75 (1H, m), 4.62 (2H, s), 4.52, 4.82 (1H, s), 6.98-7.11 (3H, m), 7.23-7.30 (2H, m), 7.32-7.40 (4H, m), 7.58-7.62 (1H, m), 7.79 (1H, s), 8.24, 8.35 (1H, s), 8.36 (1H, s)

MS (ESI-): 597 (M-H)

Example 217

(2S)-N-(2-Tetrahydropyranyloxy)-2-[5-(3-(2-(3-methoxyphenoxy)acetylamino)phenyl)-2-thienyl]-3,4,5,6-tetrahydro-2H-thiopyran-2-acetamide 1,1-dioxide (140 mg)

NMR (DMSO-$d_6$, δ): 1.34-1.77 (8H, m), 1.86-1.99 (2H, m), 2.05-2.24 (2H, m), 2.66-2.90 (2H, m), 3.03-3.17 (2H, m), 3.28-3.52 (1H, m), 3.62-3.74 (1H, m), 3.82 (3H, s), 4.52, 4.82 (1H, s), 4.61 (2H, s), 6.52-6.65 (3H, m), 7.22-7.30 (3H, m), 7.33-7.42 (2H, m), 7.55-7.62 (1H, m), 7.72-7.82 (1H, m), 8.26-8.42 (2H, m)

MS (ESI-): 627 (M-H)

Example 218

(2S)-N-(2-Tetrahydropyranyloxy)-2-[5-(3-(3-phenoxypropionylamino)phenyl)-2-thienyl]-3,4,5,6-tetrahydro-2H-thiopyran-2-acetamide 1,1-dioxide (135 mg)

NMR ($CDCl_3$, δ): 1.32-1.78 (8H, m), 1.86-1.98 (2H, m), 2.06-2.25 (2H, m), 2.71-2.92 (2H, m), 2.86 (2H, t, J=7Hz), 3.01-3.20 (2H, m), 3.28-3.52 (1H, m), 3.63-3.77 (1H, m), 4.35 (2H, t, J=7Hz), 4.54, 4.84 (1H, s), 6.78-7.03 (4H, m),

7.12-7.36 (5H, m), 7.47-7.65 (2H, m), 8.13 (1H, s), 8.73 (1H, s)
MS (ESI-): 644 (M-H)

Example 219

(2S)-N-(2-Tetrahydropyranyloxy)-2-[5-(3-(2-(4-fluorophenoxy)acetylamino)phenyl)-2-thienyl]-3,4,5,6-tetrahydro-2H-thiopyran-2-acetamide 1,1-dioxide (158 mg)

NMR ($CDCl_3$, δ): 1.35-2.01 (10H, m), 2.05-2.23 (2H, m), 2.66-2.94 (2H, m), 3.01-3.19 (2H, m), 3.29-3.53 (1H, m), 3.62-3.26 (1H, m), 4.53, 4.82 (1H, s), 4.58 (2H, s), 6.92-7.10 (5H, m), 7.24-7.39 (3H, m), 7.56-7.62 (1H, m), 7.73-7.77 (1H, m), 8.34 (1H, s), 8.39, 8.49 (1H, s)

MS (ESI-): 615 (M-H)

Example 220

(2S)-N-(2-Tetrahydropyranyloxy)-2-[5-(3-(2-(4-methoxyphenoxy)acetylamino)phenyl)-2-thienyl]-3,4,5,6-tetrahydro-2H-thiopyran-2-acetamide 1,1-dioxide (150 mg)

NMR ($CDCl_3$, δ): 1.36-1.78 (8H, m), 1.87-2.01 (2H, m), 2.06-2.26 (2H, m), 2.65-2.94 (2H, m), 3.02-3.23 (2H, m), 3.27-3.56 (1H, m), 3.62-3.36 (1H, m), 3.80 (3H, s), 4.52, 4.83 (1H, s), 4.57 (2H, s), 6.84-7.03 (4H, m), 7.24-7.43 (4H, m), 7.54-7.67 (1H, m), 7.80 (1H, s), 8.28-8.49 (2H, m)

MS (ESI-): 627 (M-H)

Example 221

(2S)-N-(2-Tetrahydropyranyloxy)-2-[5-(3-(3-(N-tert-butoxycarbonyl-N-methylamino)propionylamino)phenyl)-2-thienyl]-3,4,5,6-tetrahydro-2H-thiopyran-2-acetamide 1,1-dioxide (160 mg)

NMR ($CDCl_3$, δ): 1.38-1.75 (8H, m), 1.48 (9H, s), 1.88-2.01 (2H, m), 2.06-2.24 (2H, m), 2.62-2.88 (4H, m) , 2.91 (3H, s), 3.02-3.17 (2H, m), 3.27-3.50 (1H, m), 3.58-3.75 (3H, m), 4.53, 4.32 (1H, s), 7.18-7.31 (6H, m), 7.52-7.59 (1H, m), 7.70-7.83 (1H, m)

MS (ESI-): 648 (M-H)

Example 222

(2S)-N-(2-Tetrahydropyranyloxy)-2-[5-{3-((2S)-2-(tert-butoxycarbonylamino)-3-(3-pyridyl)propionylamino) phenyl}-2-thienyl]-3,4,5,6-tetrahydro-2H-thiopyran-2-acetamide 1,1-dioxide (1.54 g)

NMR (DMSO-$d_6$, δ): 1.22-1.27 (2H, m), 1.43 (9H, s), 1.63-1.68(4H, m), 1.98 (2H, br), 2.07-2.25 (2H, m), 2.74-2.98 (2H, m), 3.04-3.20 (6H, m), 3.41-3.48 (1H, m), 3.66-3.76 (2H, m), 4.45 (1/2H, br), 4.54-4.63 (1H, br), 4.86 (1/2H, br), 5.31-5.45 (1H, m), 6.82-7.00 (2H, m), 7.04-7.20 (3H, m), 71.22-7.27 (2H, m), 7.52-7.65 (2H, m), 8.43-8.59 (3H, m)

MS (ESI+): 713.1 (M+H)

Example 223

(2S)-N-(2-Tetrahydropyranyloxy-2-[5-[3-[2-(2-propen-1-yloxy)acetyl]aminophenyl]-2-thienyl]-3,4,5,6-tetrahydro-2H-thiopyran-2-acetamide 1,1-dioxide (14.6 g)

NMR ($CDCl_3$, δ): 1.37-1.76 (6H, m), 1.88-2.01 (2H, m), 2.04-2.26 (2H, m), 2.64-2.92 (2H, m), 3.00-3.17 (4H, m), 3.27-3.51 (1H, m), 3.61-3.72 (1H, m), 4.08 (2H, s), 4.16 (2H, d, J=6Hz), 4.52 (0.5H, s), 4.81 (0.5H, s), 5.33 (1H, d, J=9Hz), 5.37 (1H, d, J=16.5Hz), 5.90-6.04 (1H, m), 7.23-7.39 (5H, m), 7.53-7.60 (1H, m), 7.80 (1H, s), 8.16 (0.5H, s), 8.22 (0.5H, s), 8.35 (1H, s) .

MS (ESI-): 561 (M-H)

Example 224

(2S)-N-(2-Tetrahydropyranyloxy)-2-[5-{3-(2-(ethoxycarbonylamino)acetylamino)phenyl}-2-thienyl]-3-4,5,6-tetrahydro-2H-thiopyran-2-acetamide 1,1-dioxide (4 g)

NMR ($CDCl_3$, δ): 1.28 (3H, t, J=7.5Hz), 1.41 (2H, br), 1.92 (2H, br), 2.04-2.17 (2H, m), 2.55 (4H, br), 2.73-2.91 (2H, m), 3.02-3.13 (4H, m), 3.28-3.49 (1H, m), 3.64-3.74 (1H, m), 4.03-4.05 (2H, m), 4.20 (2H, q, J=7.5Hz), 4.52 (1/2H, br); 4.83 (1/2H, br), 7.11-7.21 (3H, m), 7.37-7.43 (1H, m), 7.48-7.56 (2H, m), 7.74-7.80 (1H, m), 8.46 (1H, br)

MS (ESI-): 628.2 (M-H+Cl)

Example 225

(2S)-N-(2-Tetrahydropyranyloxy)-2-[5-(3-(2-(N-tert-butoxycaronyl-N-methylamino)acetylamino)phenyl)-2-thienyl]-3,4,5,6-tetrahydro-2H-thiopyran-2-acetamide 1,1-dioxide (119 mg)

NMR ($CDCl_3$, δ): 1.35-1.74 (8H, m), 1.51 (9H, s), 1.88-2.00 (2H, m), 2.05-2.55 (2H, m), 2.18-2.92 (2H, m), 3.03 (3H, s), 3.05-3.16 (2H, m), 3.28-3.50 (1H, m), 3.62-3.76 (1H, m), 3.99 (2H, s), 4,53, 4.83 (1H, s), 7.22-7.34 (4H, m), 7.43 (1H, br s), 7.67-7.85 (1H, m), 8.42, 8.50 (1H, s)

MS (ESI-): 634 (M-H)

Example 226

(2S)-N-(2-Tetrahydropyranyloxy)-2-[5-(3-(3-(tert-butoxycaronylamino)propionylamino)phenyl)-2-thienyl]-3,4,5,6-tetrahydro-2H-thiopyran-2-acetamide 1,1-dioxide (150 mg)

NMR ($CDCl_3$, δ): 1.35-1.72 (8H, m), 1.44 (9H, s), 1.88-2.01 (2H, m), 2.05-2.23 (2H, m), 2.57-2.67 (2H, m), 2.75-2.89 (2H, m), 3.00-3.17 (4H, m), 3.28-3.56 (3H, m), 3.65-3.76 (1H, m), 4.54, 4.84 (1H, s), 5.28 (1H, br s), 7.13-7.28 (4H, m), 7.48-7.66 (2H, m), 8.14 (1H, br s), 8.75, 8.78 (1H, s)

MS (ESI-): 634 (M-H)

The following compounds were obtained in a similar manner to that of Example 125.

Example 227

(2S)-N-(2-Tetrahydropyranyloxy)-2-[5-(3-(propionylamino)phenyl)-2-thienyl]-3,4,5,6-tetrahydro-2H-thiopyran-2-acetamide 1,1-dioxide (160 mg)

NMR (DMSO-$d_6$, δ): 1.09 (3H, t, J=7Hz), 1.32-2.07 (10H, m), 2.36 (2H,'q, J=7Hz), 2.37-2.47 (1H, m), 2.88-3.22 (5H, m), 3.35-3.54 (1H, m), 3.74-3.92 (1H, m), 4.44, 4.75 (1H, s), 7.17-7.25 (1H, m), 7.30-7.42 (3H, m), 7.43-7.52 (1H, m), 8.00 (1H, s), 9.98 (1H, s), 11.36 (1H, s)

MS (ESI-): 519 (M-H)

Example 228

(2S)-N-(2-Tetrahydropyranyloxy)-2-[5-(3-(butyrylamino)phenyl)-2-thienyl]-3,4,5,6-tetrahydro-2H-thiopyran-2-acetamide 1,1-dioxide (166 mg)

NMR ($CDCl_3$, δ): 1.01 (3H, t, J=7Hz), 1.35-1.84 (10H, m), 2.05-2.25 (2H, m), 2.37 (2H, t, J=7Hz), 2.66-2.92 (2H, m), 2.97-3.17 (2H, m), 3.27-3.52 (1H, m), 3.60-3.77 (1H, m), 4.54, 4.74 (1H, s), 7.08-7.32 (5H, m), 7.48-7.66 (2H, m), 8.64 (1H, br)

MS (ESI-): 533 (M-H)

Example 229

(2S)-N-(2-Tetrahydropyranyloxy)-2-[5-(3-(2-methoxyethoxycarbonylamino)phenyl)-2-thienyl]-3,4,5,6-tetrahydro-2H-thiopyran-2-acetamide 1,1-dioxide (120 mg) NMR ($CDCl_3$, δ): 1.37-1.78 (8H, m), 1.89-2.00 (2H, m), 2.05-2.25 (2H, m), 2.67-2.94 (2H, m), 3.02-3.18 (3H, m), 3.26-3.52 (1H, m), 3.58-3.69 (2H, m), 3.66 (3H, s), 4.25-4.32 (2H, m), 4.52, 4.83 (1H, s), 6.90-7.00 (1H, m), 7.20-7.32 (5H, m), 7.63 (1H, s), 8.34, 8.46 (1H, s)

MS (ESI-): 565 (M-H)

Example 230

(2S)-N-(2-Tetrahydropyranyloxy)-2-[5-(3-(2-(9-fluorenylmethoxycarbonylamino)acetylamino)phenyl)-2-thienyl]-3,4,5,6-tetrahydro-2H-thiopyran-2-acetamide 1,1-dioxide (486 mg)

NMR (DMSO-$d_6$, δ): 1.34-1.62 (6H, m), 1.71-2.08 (4H, m), 2.36-2.47 (1H, m), 2.88-3.34 (5H, m), 3.39-3.52 (2H, m), 3.74-3.92 (3H, m)_, 4.19-4.37 (3H, m), 4.42, 4.77 (1H, s), 7.18-7.27 (1H, m), 7.32-7.52 (7H, m), 7.62-7.70 (1H, m), 7.75 (2H, d, J=7Hz), 7.92 (2H, d, J=7Hz), 8.02 (1H, s)

Example 231

(2S)-N-(2-Tetrahydropyranyloxy)-2-[5-(3-(acetoxyacetylamino)phenyl)-2-thienyl]-3,4,5,6-tetrahydro-2H-thiopyran-2-acetamide 1,1-dioxide (260 mg)

NMR ($CDCl_3$, δ): 1.35-1.77 (8H, m), 1.86-2.00 (2H, m), 2.03-2.24 (2H, m), 2.71-2.92 (2H, m), 3.00-3.20 (2H, m),

3.38-3.54 (1H, m), 3.65-3.78 (1H, m), 4.52, 4.72 (1H, s), 4.72 (2H, s), 7.14-7.35 (4H, m), 7.52, 7.59 (1H, s), 7.56-7.67 (1H, m), 8.06-8.15 (1H, m), 8.59-8.70 (1H, m)

MS (ESI-): 563 (M-H)

Example 232

(2S)-N-(2-Tetrahydropyranyloxy)-2-[5-(3-((2S)-2-acetoxypropionylamino)phenyl)-2-thienyl]-3,4,5,6-tetrahydro-2H-thiopyran-2-acetamide 1,1-dioxide (313 mg)

NMR ($CDCl_3$, δ): 1.38-1.78 (8H, m), 1.49, 1.57 (3H, d, J=8Hz), 1.88-2.00 (2H, m), 2.07-2.22 (2H, m), /2.72-2.92 (2H, m), 3.03-3.19 (2H, m), 3.29-3.56 (1H, m), 3.64-3.78 (1H, m), 4.53, 4.82 (1H, s), 5.06, 5.34 (1H, q, J=8Hz), 7.18-7.36 (4H, m), 7.53-7.68 (2H, m), 8.10 (1H, br s), 8.55, 8.59 (1H, s)

MS (ESI-): 577 (M-H)

Example 233

(2S)-N-(2-Tetrahydropyranyloxy)-2-[5-(3-(chloroacetylamino)phenyl)-2-thienyl]-3,4,5,6-tetrahydro-2H-thiopyran-2-acetamide 1,1-dioxide (201 mg)

NMR ($CDCl_3$, δ): 1.40-1.54 (4H, m), 1.60-1.73 (2H, m), 1.88-2.00 (2H, m), 2.06-2.25 (2H, m), 2.67-2.91 (2H, m), 3.02-3.18 (4H, m), 3.29-3.51 (1H, m), 3.62-3.75 (1H, m), 4.21 (2H, s), 4.53 (0.5H, s), 4.82 (0.5H, s), 7.23-7.29 (2H, m), 7.32-7.41 (2H, m), 7.51-7.59 (1H, m), 7.70-7.76 (1H, m), 8.22 (0.5H, s), 8.28-8.36 (1.5H, s)

MS (ESI-): 539 (M-H)

Example 234

(2S)-N-(2-Tetrahydropyranyloxy)-2-[5-(3-(2-(9-(fluorenylmethoxycarbonylamino)acetylamino)phenyl)-2-thienyl]-3,4,5,6-tetrahydro-2H-thiopyran-2-acetamide 1,1-dioxide (478 mg) was dissolved in a solution of 20% piperidine in N,N-dimethylformamide (8 ml) at room temperature. After being stirred at the same temperature for 1 hour, the reaction mixture was concentrated in vacuo. The residue was purified by $SiO_2$ column chromatography (eluent: 1-5% MeOH in $CHCl_3$) to give (2S)-N-(2-tetrahydropyranyloxy)-2-[5-(3-(2-aminoacetylamino)phenyl)-2-thienyl]-3,4,5,6-tetrahydro-2H-thiopyran-2-acetamide 1,1-dioxide (335 mg).

NMR (DMSO-$d_6$, δ): 1.37-1.65 (6H, m), 1.70-2.08 (4H, m), 2.34-2.50 (1H, m), 2.87-3.52 (8H, m), 3.72-3.90 (1H, m), 4.44, 4.75 (1H, s), 7.18-7.25 (1H, m), 7.32-7.44 (3H, m), 7.52-7.60 (1H, m), 8.02 (1H, s)

MS (ESI+) : 522 (M+H)

Example 235

(2S)-N-(2-Tetrahydropyranyloxy)-2-[5-(3-(3-aminopropionylamino)phenyl)-2-thienyl]-3,4,5,6-tetrahydro-2H-thiopyran-2-acetamide 1,1-dioxide (1.56 g) was obtained in a similar manner to that of Example 234.

NMR (DMSO-$d_6$, δ): 1.32-2.09 (10H, m), 2.32-2.48 (3H, m), 2.72-3.56 (8H, m), 3.72-3.90 (1H, m), 4.45, 4.75 (1H, s), 7.17-7.24 (1H, m), 7.28-7.51 (3H, m), 7.43-7.54 (1H, m), 8.01 (1H, s), 12.4 (1H, br s)

MS (ESI-): 534 (M-H)

Example 236

(2S)-N-(2-Tetrahydropyranyloxy)-2-[5-(3-(2-(methoxycarbonylamino)acetylamino)phenyl)-2-thienyl]-3,4,5,6-tetrahydro-2H-thiopyran-2-acetamide 1,1-dioxide (88 mg) was obtained in a similar manner to that of Example 125.

NMR ($CDCl_3$, δ): 1.36-1.72 (8H, m), 1.87-1.99 (2H, m), 2.07-2.27 (2H, m), 2.79-2.92 (2H, m), 3.00-3.23 (2H, m), 3.28-3.52 (1H, m), 3.68-3.76 (1H, m), 3.75 (3H, s), 3.96-4.12 (2H, m), 4.53, 4.84 (1H, s), 5.76-5.88 (1H, m), 7.05-7.22 (5H, m), 7.36-7.55 (2H, m), 8.40 (1H, s)

MS (ESI-): 578 (M-H)

Example 237

To a solution of (2S)-N-(2-tetrahydropyranyloxy)-2-[5-(3-formylphenyl)-2-thienyl]-3,4,5,6-tetrahydro-2H-thiopyran-2-acetamide 1,1-dioxide (100 mg) in tetrahydrofuran (1.5 ml) was added dropwise sodium borohydride (8.71 mg) in water (0.7 ml) at room temperature. After being stirred for 30 minutes, the reaction is stopped by adding 1% aqueous citric acid solution. The reaction mixture is extracted with ethyl acetate and the solution was washed with

brine, dried over $MgSO_4$ and concentrated in vacuo. The resulting residue was purified by silica gel column chromatography (eluent: 0.5-3% methanol in chloroform) to give (2S)-N-(2-tetrahydropyranyloxy)-2-[5-(3-hydroxymethylphenyl)-2-thienyl]-3,4,5,6-tetrahydro-2H-thiopyran-2-acetamide 1,1-dioxide (96 mg) as an amorphous solid.

NMR ($CDCl_3$, δ): 1.38-1.72 (6H, m), 1.81-2.00 (2H, m), 2.03-2.25 (2H, m), 2.67-3.17 (6H, m), 3.29-3.51 (1H, m), 3.61-3.72 (1H, m), 4.52, 4.81 (1H, s), 4.72 (2H, d, J=6.0Hz), 7.25-7.41 (4H, m), 7.52 (1H, d, J=8.5Hz), 7.60 (1H, s)

MS (ESI-): 478 (M-H)

Example 238

A solution of (2S)-N-(2-tetrahydropyranyloxy)-2-[5-(4-ethenylphehyl)-2-thienyl]-3,4,5,6-tetrahydro-2H-thiopyran-2-acetamide 1,1-dioxide (200 mg), microencapsulated osmium tetraoxide (5.34 mg) and N-methylmorphorine N-oxide (98.5 mg) in a mixture of $H_2O$-acetone-acetonitrile (1:1:1) (3.0 ml) was stirred at room temperature for 12 hours. After the reaction was completed, the catalyst was separated by filtration. After washing with methanol, combined filtrates were concentrated in vacuo. The resulting residue was purified by silica gel column chromatography (eluent: 0.5-3% methanol in chloroform) to give (2S)-N-(2-tetrahydropyranyloxy)-2-[5-(4-(cis-1,2-dihydroxyethyl)phenyl)-2-thienyl]-3,4,5, 6-tetrahydro-2H-thiopyran-2-acetamide 1,1-dioxide as an amorphous solid (200 mg).

NMR ($CDCl_3$, δ): 1.38-1.75 (6H, m), 1.90-2.00 (2H, m), 2.07-2.23 (2H, m), 2.60-2.89 (2H, m), 3.09-3.17 (4H, m), 3.25-3.50 (1H, m), 3.59-3.71 (1H, m), 3.73-3.82 (1H, m), 4.52, 4.80 (1H, s), 4.80-4.88 (1H, m), 7.34-7.40 (3H, m), 7.54-7.59 (3H, m)

MS (ESI-): 508 (M-H)

Example 239

(2S)-N-(2-Tetrahydropyranyloxy)-2-[5-(3-(phenoxycarbonyloxymethyl)phenyl)-2-thienyl]-3,4,5,6-tetrahydro-2H-thiopyran-2-acetamide 1,1-dioxide (620 mg) was obtained from (2S)-N-(2-tetrahydropyranyloxy)-2-[5-(3-hydroxymethylphenyl)-2-thienyl)-3,4,5,6-tetrahydro-2H-thiopyran-2-acetamide 1,1-dioxide (500 mg) in a similar manner to that of Example 125.

NMR ($CDCl_3$, δ): 1.38-1.75 (6H, m), 1.90-2.01 (2H, m), 2.06-2.23 (2H, m), 2.65-2.90 (2H, m), 3.04-3.17 (4H, m), 3.30-3.50 (1H, m), 3.62-3.70 (1H, m), 4.53, 4.81 (1H, s), 5.28 (2H, s), 7.19 (2H, d, J=8.5Hz), 7.25-7.31 (4H, m), 7.37-7.42 (4H, m), 7.59-7.61 (1H, m), 7.66 (1H, s)

MS (ESI-): 598 (M-H)

Example 240

To a solution of (2S)-N-(2-tetrahydropyranyloxy)-2-[5-(3-(phenoxycarbonyloxymethyl)phenyl)-2-thienyl]-3,4,5,6-tetrahydro-2H-thiopyran-2-acetamide 1,1-dioxide (200 mg) in N,N-dimethylformamide (3 ml) was added 40% aqueous methylamine (0.17 ml). After being stirred for 3 hours at the same temperature, the mixture was concentrated in vacuo. The residue was diluted with ethyl acetate, washed with 1% aqueous citric acid solution, sat. $NaHCO_3$ solution and brine, dried over $MgSO_4$ and concentrated in vacuo. The resulting residue was purified by silica gel column chromatography (eluent: $CHCl_3$) to give (2S)-N-(2-tetrahydropyranyloxy)-2-[5-(3-(methylaminocarbonyloxymethyl)phenyl)-2-thienyl]-3,4,5,6-tetrahydro-2H-thiopyran-2-acetamide 1,1-dioxide (150 mg) as an amorphous solid.

NMR ($CDCl_3$, δ): 1.37-1.75 (6H, m), 1.90-2.00 (2H, m), 2.05-2.23 (2H, m), 2.83 (3H, d, J=5.0Hz), 2.89-3.17 (6H, m), 3.29-3.50 (1H, m), 3.61-3.71 (1H, m), 4.53, 4.81 (1H, s), 5.11 (2H, s), 7.25-7.31 (2H, m), 7.36 (1H, dd, J=8.0, 8.0Hz), 7.51-7.59 (3H, m)

MS (ESI-): 535 (M-H)

Example 241

(2S)-N-(2-Tetrahydropyranyloxy)-2-[5-(4-(2-(methylaminocarbonyl)ethenyl)phenyl)-2-thienyl]-3,4,5,6-tetrahydro-2H-thiopyran-2-acetamide 1,1-dioxide (270 mg) was obtained from (2S)-N-(2-tetrahydropyranyloxy)-2-[5-(4-(2-carboxyethenyl)phenyl)-2-thienyl]-3,4,5,6-tetrahydro-2H-thiopyran-2-acetamide 1,1-dioxide (300 mg) in a similar manner to that of Example 31.

NMR ($CDCl_3$, δ): 1.38-1.73 (6H, m), 1.88-2.02 (2H, m), 2.09-2.25 (2H, m), 2.78-2.89 (2H, m), 2.96 (3H, d, J=5Hz), 3.05-3.19 (4H, m), 3.29-3.51 (1H, m), 3.65-3.75 (1H, m), 4.54, 4.84 (1H, s), 6.40 (1H, d, J=15Hz), 7.20-7.59 (7H, m), 8.78 (1H, d, J=5Hz)

Example 242

(2S)-N-(2-Tetrahydropyranyloxy)-2-[5-(4-(2-(ethylaminocarbonyl)ethenyl)phenyl)-2-thienyl]-3,4,5,6-tetrahydro-2H-thiopyran-2-acetamide 1,1-dioxide (280 mg) was obtained in a similar manner to that of Example 31.

NMR (DMSO-$d_6$, δ): 1.23 (3H, t, J=7.2Hz), 1.38-1.73 (6H, m), 1.90-2.01 (2H, m), 2.07-2.23 (2H, m), 2.78-2.88 (2H, m), 3.02-3.20 (4H, m), 3.38-3.48 (3H, m), 3.67-3.76 (1H, m), 4.54, 4.84 (1H, s), 6.41 (1H, d, J=15Hz), 7.21-7.59 (7H, m), 8.78 (1H, br)

Example 243

(2S)-N-(2-Tetrahydropyranyloxy)-2-[5-(4-(methylaminocarbonylmethoxy)phenyl)-2-thienyl]-3,4,5,6-tetrahydro-2H-thiopyran-2-acetamide 1,1-dioxide (720 mg) was obtained in a similar manner to that of Example 247.

NMR (DMSO-$d_6$, δ): 1.37-1.62 (6H, m), 1.67-1.81 (2H, m), 1.82-2.05 (2H, m), 2.33-2.45 (1H, m), 2.66 (3H, d, J=4.5Hz), 2.90-3.50 (6H, m), 3.73-3.91 (1H, m), 4.45, 4.75 (1H, s) , 4.50 (2H, s), 7.01 (2H, d, J=9.0Hz), 7.16 (1H, d, J=4.0Hz), 7.32 (1H, d, J=4.0Hz), 7.59 (2H, d, J=9.0Hz), 8.07 (1H, br), 11.23 (1H, s)

Example 244

A mixture of (2S)-N-(2-tetrahydropyranyloxy)-2-[5-[3-(chloroacetylamino)phenyl]-2-thienyl]-3,4,5,6-tetrahydro-2H-thiopyran-2-acetamide 1,1-dioxide (98 mg), n-tetrabutylammonium iodide (5 mg) and 2-aminoethanol (27.7 mg) in N,N-dimethylformamide (1.5 ml) was stirred for 14 hours at room temperature. The mixture was concentrated in vacuo and taken up between ethyl acetate and saturated aqueous sodium bicarbonate. The separated organic layer was washed with brine, dried over sodium sulfate and evaporated in vacuo to give (2S)-N-(2-tetrahydropyranyloxy)-2-[5-[3-[(2-hydroxyethylamino)acetylamino]phenyl]-2-thienyl]-3,4,5,6-tetrahydro-2H-thiopyran-2-acetamide 1,1-dioxide .(100 mg) as an amorphous solid.

NMR ($CDCl_3$, δ): 1.38-1.77 (6H, m), 1.89-2.00 (2H, m), 2.05-2.24 (2H, m), 2.68-2.91 (4H, m), 3.00-3.18 (4H, m), 3.29-3.52 (3H, m), 3.62-3.80 (3H, m), 4.53 (0.5H, s), 4.82 (0.5H, s), 7.22-7.32 (4H, m), 7.59-7.67 (1H, m), 7.69-7.75 (1H, m), 8.01 (1H, s), 9.48 (1H, s)

MS (ESI-): 564 (M-H)

Example 245

(2S)-N-(2-Tetrahydropyranyloxy)-2-[5-[3-[(4-morpholino)acetylamino]phenyl]-2-thienyl]-3,4,5,6-tetrahydro-2H-thiopyran-2-acetamide 1,1-dioxide (96 mg) was obtained in a similar manner to that of Example 244.

NMR ($CDCl_3$, δ): 1.40-1.56 (4H, m), 1.61-1.73 (2H, m), 1.89-2.01 (2H, m), 2.06-2.32 (2H, m), 2.61-2.87 (6H, m), 3.05-3.19 (4H, m), 3.31-3.51 (1H, m), 3.62-3.74 (1H, m), 3.77-3.85 (4H, m), 4.53 (0.5H, s), 4.81 (0.5H, s), 7.25-7.31 (2H, m), 7.33-7.38 (2H, m), 7.52-7.61 (1H, m), 7.74-7.80 (1H, m), 8.00-8.23 (1H, m), 9.10 (1H, s)

MS (ESI-): 590 (M-H)

The following compounds were obtained in a similar manner to that of Example 52.

Example 246

(2S)-N-Hydroxy-2-[5-(4-aminocarbonylphenyl)-2-thienyl]-3,4,5,6-tetrahydro-2H-thiopyran-2-acetamide 1,1-dioxide (40 mg)

NMR (DMSO-$d_6$, δ): 1.74-2.04 (4H, m), 2.37-2.46 (1H, m), 2.96-3.27 (4H, m), 3.4-3.54 (1H, m), 7.25 (1H, d, J=4.0Hz), 7.48 (1H, br), 7.59 (1H, d, J=4.0Hz), 7.74 (2H, d, J=7.5Hz), 7.92 (2H, d, J=7.5Hz), 8.02 (1H, br), 8.85 (1H, br)

MS (ESI-): 407.1 (M-H)

Example 247

(2S)-N-Hydroxy-2-[5-(4-benzylaminocarbonyl)phenyl)-2-thienyl]-3,4,5,6-tetrahydro-2H-thiopyran-2-acetamide 1,1-dioxide (20 mg)

NMR (DMSO-$d_6$, δ): 1.74-2.08 (4H, m), 2.37-2.45 (1H, m), 2.95-3.26 (4H, m), 3.43-3.52 (1H, m), 4.49 (2H, d, J=6.0Hz), 7.24 (2H, m), 7.31-7.34 (4H, m), 7.61 (1H, d, J=3.0Hz), 7.75 (2H, d, J=7.5Hz), 7.95 (2H, d, J=7.5Hz), 8.84 (1H, s), 9.10 (1H, t, J=6.0Hz)

MS (ESI+): 523.1(M+H+Na)

Example 248

(2S)-N-Hydroxy-2-[5-(3-(ethoxycarbonylamino)phenyl)-2-thienyl]-3,4,5,6-tetrahydro-2H-thiopyran-2-acetamide 1,1-dioxide (115 mg)

NMR (DMSO-$d_6$, δ): 1.28 (3H, t, J=7.0Hz), 1.73-2.05 (4H, m), 2.37-2.45 (1H, m), 2.96-3.25 (4H, m), 3.42-3.53 (1H, m), 4.15 (2H, q, J=7.0Hz), 7.20 (1H, d, J=4.0Hz), 7.28-7.34 (3H, m), 7.48 (1H, d, J=4.0Hz), 7.82 (1H, s), 8.84 (1H, br), 9.85 (1H, s), 10.60 (1H, br)

MS (ESI-): 451.2 (M-H)

Example 249

(2S)-N-Hydroxy-2-[5-(3-(benzylaminocarbonyl)phenyl)-2-thienyl]-3,4,5,6-tetrahydro-2H-thiopyran-2-acetamide 1,1-dioxide (50 mg)

NMR (DMSO-$d_6$, δ): 1.73-2.05 (4H, m), 2.36-2.47 (1H, m), 2.95-3.25 (4H, m), 3.41-3.52 (1H, m), 4.31 (2H, d, J=7.0Hz), 6.68 (1H, t, J=7.0Hz), 7.18-7.38 (10H, m), 7.86 (1H, s), 8.72 (1H, s), 8.84 (1H, s)

MS (ESI-): 512.3 (M-H)

Example 250

(2S)-N-Hydroxy-2-[5-(3-(n-propyloxycarbonylamino)-phenyl)-2-thienyl]-3,4,5,6-tetrahydro-2H-thiopyran-2-acetamide 1,1-dioxide (120 mg)

NMR (DMSO-$d_6$, δ): 0.95 (3H, t, J=7.0Hz), 1.66 (2H, qt, J=7.0, 7.0Hz), 1.72-2.05 (4H, m), 2.35-2.46 (1H, m), 2.94-3.29 (4H, m), 3.40-3.53 (1H, m), 4.05 (2H, t, J=7.0Hz), 7.20 (1H, d, J=4.0Hz), 7.29-7.34 (3H, m), 7.38 (1H, d, J=4.0Hz), 7.83 (1H, s), 8.83 (1H, s), 9.73 (1H, s), 10.59 (1H, s)

MS (ESI-): 465.3 (M-H)

Example 251

(2S)-N-Hydroxy-2-[5-(3-(isopropoxycarbonylamino)-phenyl)-2-thienyl]-3,4,5,6-tetrahydro-2H-thiopyran-2-acetamide 1,1-dioxide (90 mg)

NMR (DMSO-$d_6$, δ): 1.26 (6H, d, J=6.0Hz), 1.74-2.04 (4H, m), 2.35-2.55 (1H, m), 2.94-3.25 (4H, m), 3.40-3.51 (1H, m), 4.40 (1H, qq, J=6.0, 6.0Hz), 7.20 (1H, d, J=4.0Hz), 7.29-7.33 (3H, m), 7.36 (1H, d, J=4.0Hz), 7.85 (1H, s), 8.83 (1H, s), 9.67 (1H, s), 10.59 (1H, s)

MS (ESI-): 465.3 (M-H)

Example 252

(2S)-N-Hydroxy-2-[5-{3-(2-chloroethoxycarbonylamino)-phenyl}-2-thienyl]-3,4,5,6-tetrahydro-2H-thiopyran-2-acetamide 1,1-dioxide (100 mg)

NMR (DMSO-$d_6$, δ): 1.75-2.05 (4H, m), 2.36-2.47 (1H, m), 2.94-3.29 (4H, m), 3.42-3.52 (1H, m), 3.88 (2H, d, J=6.0Hz), 4.35 (2H, d, J=6.0Hz), 7.21 (1H, d, J=4.0Hz), 7.32-7.36 (2H, m), 7.40 (1H, d, J=4.0Hz), 7.84 (1H, s), 8.83 (1H, s), 9.94 (1H, s), 10.08 (1H, s)

MS (ESI-): 485.2 (M-H)

Example 253

(2S)-N-Hydroxy-2-[5-(3-valerylaminophenyl)-2-thienyl]-3,4,5,6-tetrahydro-2H-thiopyran-2-acetamide 1,1-dioxide (102 mg)

NMR (DMSO-$d_6$, δ): 0.91 (3H, t, J=8Hz), 1.24-1.41 (2H, m), 1.52-1.66 (2H, m), 1.69-2.08 (4H, m), 2.33 (2H, t, J=8Hz), 2.32-2.48 (1H, m), 2.92-3.28 (4H, m), 3.37-3.54 (1H, m), 7.20 (1H, d, J=3Hz), 7.34 (2H, d, J=3Hz), 7.40 (1H, d, J=3Hz), 7.42-7.52 (1H, m), 7.99 (1H, s), 10.00 (1H, s), 10.60 (1H, s)

MS (ESI-): 463 (M-H)

Example 254

(2S)-N-Hydroxy-2-[5-(3-(ethylthiocarbonylamino)-phenyl)-2-thienyl]-3,4,5,6-tetrahydro-2H-thiopyran-2-acetamide 1,1-dioxide (92 mg)

NMR (DMSO-$d_6$, δ): 1.26 (3H, t, J=8Hz), 1.72-2.08 (4H, m), 2.36-2.49 (1H, m), 2.89 (2H, q, J=8Hz), 2.94-3.30

(4H, m), 3.39-3.55 (1H, m), 7.21 (1H, d, J=3Hz), 7.32-7.44 (4H, m), 7.90 (1H, s), 8.85 (1H, s), 10.39 (1H, s), 10.60 (1H, s)

MS (ESI+): 469 (M+H)

Example 255

(2S)-N-Hydroxy-2-[5-(3-(methylthiocarbonylamino)-phenyl)-2-thienyl]-3,4,5,6-tetrahydro-2H-thiopyran-2-acetamide 1,1-dioxide (130 mg)

NMR (DMSO-$d_6$, δ): 1.68-2.08 (4H, m), 2.33 (3H, s), 2.35-2.46 (1H, m), 2.92-3.38 (4H, m), 3.39-3.53 (1H, m), 7.21 (1H, d, J=3Hz), 7.29-7.46 (4H, m), 7.90 (1H, s)

MS (ESI-): 453 (M-H)

Example 256

(2S)-N-Hydroxy-2-[5-(3-(benzyloxycarbonylamino)-phenyl)-2-thienyl]-3,4,5,6-tetrahydro-2H-thiopyran-2-acetamide 1,1-dioxide (60.2 mg)

NMR (DMSO-$d_6$, δ): 1.68-2.07 (4H, m), 2.32-2.48 (1H, m), 2.92-3.34 (4H, m), 3.52-3.65 (1H, m), 5.18 (2H, s), 7.21 (1H, d, J=3Hz), 7.26-7.50 (10H, m), 7.85 (1H, s), 9.91 (1H, s), 10.60 (1H, s)

MS (ESI-): 513 (M-H)

Example 257

(2S)-N-Hydroxy-2-[5-(3-(2-(t-butoxycarbonylamino)-acetylamino)phenyl)-2-thienyl]-3,4,5,6-tetrahydro-2H-thiopyran-2-acetamide 1,1-dioxide (40 mg)

NMR (DMSO-$d_6$, δ): 1.40 (9H, s), 1.66-2.08 (4H, m), 2.35-2.48 (1H, m), 2.92-3.32 (4H, m), 3.45-3.53 (1H, m), 3.73 (2H, d, J=7Hz), 7.07 (1H, t, J=7Hz), 7.22 (1H, d, J=3Hz), 7.32-7.54 (4H, m), 7.97 (1H, s), 10.05 (1H, s), 10.61 (4H, s)

MS (ESI-): 536 (M-H)

Example 258

(2S)-N-Hydroxy-2-[5-(3-(2-(4-chlorophenoxy)-acetylamino)phenyl)-2-thienyl]-3,4,5,6-tetrahydro-2H-thiopyran-2-acetamide 1,1-dioxide (90 mg)

NMR (DMSO-$d_6$, δ): 1.71-2.09 (4H, m), 2.35-2.49 (1H, m), 2.94-3.29 (4H, m), 3.42-3.55 (1H, m), 4.74 (2H, s), 7.00-7.12 (2H, m), 7.22 (1H, br s), 7.32-7.47 (5H, m), 7.51-7.62 (1H, m), 8.02 (1H, s), 8.84 (1H, s), 10.22 (1H, s), 10.60 (1H, s)

MS (ESI-): 547 (M-H)

Example 259

(2S)-N-Hydroxy-2-(5-(3-(phenoxycarbonylamino)-phenyl)-2-thienyl]-3,4,5,6-tetrahydro-2H-thiopyran-2-acetamide 1,1-dioxide (88 mg)

NMR (DMSO-$d_6$, δ): 1.68-2.09 (4H, m), 2.32-2.48 (1H, m), 2.92-3.30 (4H, m), 3.56-3.72 (1H, m), 7.17-7.30 (4H, m), 7.31-7.51 (6H, m), 7.89 (1H, s), 10.37 (1H, s), 10.61 (1H, s)

MS (ESI-): 499 (M-H)

Example 260

(2S)-N-Hydroxy-2-[5-(3-(2-(4-chlorophenyl)acetylamino)-phenyl)-2-thienyl]-3,4,5,6-tetrahydro-2H-thiopyran-2-acetamide 1,1-dioxide (95 mg)

NMR (DMSO-$d_6$, δ): 1.68-2.09 (4H, m), 2.35-2.48 (1H, m), 2.92-3.32 (4H, m), 3.54-3.66 (1H, m), 3.68 (2H, s), 7.20 (1H, d, J=3Hz), 7.28-7.52 (8H, m), 7.99 (1H, s), 10.33 (1H, s), 10.60 (1H, s)

MS (ESI-): 531 (M-H)

Example 261

(2S)-N-Hydroxy-2-[5-(3-(2-(4-morpholinocarbonylamino)-acetylamino)phenyl)-2-thienyl]-3,4,5,6-tetrahydro-2H-thiopyran-2-acetamide 1,1-dioxide (50 mg)

NMR (DMSO-$d_6$, δ): 1.71-2.08 (4H, m), 2.36-2.48 (1H, m), 2.94-3.62 (13H, m), 3.82 (2H, d, J=7Hz), 6.96 (1H, t, J=7Hz), 7.21 (1H, d, J=3Hz), 7.31-7.49 (4H, m), 8.02 (1H, s), 8.84 (1H, br s)

MS (ESI-): 549 (M-H)

Example 262

(2S)-N-Hydroxy-2-[5-(3-(2-(N-ethyl-N-methylamino-carbonyloxy)acetylamino)phenyl)-2-thienyl]-3,4,5,6-tetrahydro-2H-thiopyran-2-acetamide 1,1-dioxide (133 mg)

NMR (DMSO-$d_6$, δ): 1.05, 1.12 (3H, t, J=8Hz), 1.68-2.07 (4H, m), 2.35-2.48 (1H, m), 2.76-3.36 (9H, m), 3.39-3.54 (1H, m), 4.63 (2H, s), 7.21 (1H, d, J=3Hz), 7.32-7.47 (4H, m), 8.00 (1H, s), 8.85 (1H, s), 10.18 (1H, s), 10.61 (1H, s)

MS (ESI-): 522 (M-H)

Example 263

(2S)-N-Hydroxy-2-[5-(3-(isopropoxyacetylamino)-phenyl)-2-thienyl]-3,4,5,6-tetrahydro-2H-thiopyran-2-acetamide 1,1-dioxide (204 mg)

NMR (DMSO-$d_6$, δ): 1.78 (6H, d, J=8Hz), 1.72-2.08 (4H, m), 2.35-2.49 (1H, m), 2.94-3.31 (4H, m), 3.42-3.56 (1H, m), 3.63-3.77 (1H, m), 4.04 (2H, s), 7.22 (1H, d, J=3Hz), 7.32-7.43 (2H, m), 7.40 (1H, d, J=3Hz), 7.56-7.64 (1H, m), 8.02 (1H, s), 8.85 (1H, s), 9.68 (1H, s), 10.60 (1H, s)

MS (ESI+): 482 (M+H)

Example 264

(2S)-N-Hydroxy-2-[5-(3-(2-(2-oxo-1,3-oxazolidinyl-3-yl)acetylamino)phenyl)-2-thienyl]-3,4,5,6-tetrahydro-2H-thiopyran-2-acetamide 1,1-dioxide (480 mg)

NMR (DMSO-$d_6$, δ): 1.65-2.09 (4H, m), 2.32-2.51 (1H, m), 2.88-3.53 (5H, m), 3.66 (2H, t, J=8Hz), 4.04 (2H, s), 4.34 (2H, t, J=8Hz), 7.21 (1H, d, J=3Hz), 7.30-7.51 (4H, m), 8.03 (1H, s), 10.37 (1H, s), 10.62 (1H, s)

MS (ESI-): 506 (M-H)

Example 265

(2S)-N-Hydroxy-2-[5-(3-(4-methoxyphenylaminocarbonylamino)phenyl)-2-thienyl]-3,4,5,6-tetrahydro-2H-thiopyran-2-acetamide 1,1-dioxide (140 mg)

NMR (DMSO-$d_6$, δ): 1.73-2.04 (4H, m), 2.38-2.45 (1H, m), 2.95-3.26 (4H, m), 3.39-3.54 (1H, m), 3.72 (3H, s), 6.86 (2H, d, J=7.5Hz), 7.20 (1H, d, J=4.0Hz), 7.23-7.32 (3H, m), 7.37 (2H, d, J=7.5Hz), 7.50 (1H, d, J=4.0Hz), 7.87 (1H, s), 8.50 (1H, s), 8.72 (1H, s), 8.84 (1H, s), 10.59 (1H, s)

MS (ESI-): 528.3 (M-H)

Example 266

(2S)-N-Hydroxy-2-[5-(3-(3-pyridylaminocarbonylamino)-phenyl)-2-thienyl]-3,4,5,6-tetrahydro-2H-thiopyran-2-acetamide 1,1-dioxide (70 mg)

NMR (DMSO-$d_6$, δ): 1.75-2.05 (4H, m), 2.37-2.48 (1H, m), 2.96-3.25 (4H, m), 3.58-3.69 (1H, m), 7.21 (1H, d, J=4.0Hz), 7.30-7.36 (3H, m), 7.42 (1H, d, J=4.0Hz), 7.82 (1H, dd, J=7.5, 5.0Hz), 7.90 (1H, s), 8.29 (12H, d, J=7.5Hz), 8.45 (1H, d, J=5.0Hz), 9.03 (1H, s), 9.48 (1H, s), 9.85 (1H, s), 10.60 (1H, s)

MS (ESI-): 499.2 (M-H)

Example 267

(2S)-N-Hydroxy-2-[5-{3-(((2S)-2-aminopropionyl)amino)-phenyl}-2-thienyl]-3,4,5,6-tetrahydro-2H-thiopyran-2-acetamide 1,1-dioxide hydrochloride (120 mg) from (2S)-N-(2-tetrahydropyranyloxy)-2-[5-{3-(((2S)-2-tert-butoxycarbonylaminopropionyl)amino)phenyl}-2-thienyl]-3,4,5,6-tetrahydro-2H-thiopyran-2-acetamide 1,1-dioxide

NMR (DMSO-$d_6$, δ): 1.50 (3H, d, J=7.0Hz), 1.72-2.03 (4H, m), 2.35-2.46 (1H, m), 2.95-3.27 (4H, m), 3.43-3.54 (1H, m), 4.05 (1H, br t, J=7.0Hz), 7.21 (1H, d, J=4.0Hz), 7.37-7.43 (3H, m), 7.55 (1H, d, J=7.5Hz), 7.95 (1H, s), 8.28-8.32 (3H, m), 10.63 (1H, s), 10.81 (1H, s)

MS (ESI-): 450.7 (M-H)

Example 268

(2S)-N-Hydroxy-2-[5-{3-(((2R)-2-(tert-butoxycarbonylamino)propionyl)amino)phenyl}-2-thienyl]-3,4,5,6-tetrahydro-2H-thiopyran-2-acetamide 1,1-dioxide (30 mg)

NMR (DMSO-$d_6$, δ): 1.27 (3H, d, J=6.0Hz), 1.38 (9H, s), 1.74-2.04 (4H, m), 2.37-2.42 (1H, m), 2.95-3.25 (4H, m), 3.38-3.51 (1H, m), 4.08-4.15 (1H, m), 7.10 (1H, d, J=7.0Hz), 7.20 (1H, d, J=4.0Hz), 7.31-7.36 (2H, m), 7.40 (1H, d, J=4.0Hz), 7.50 (1H, br), 7.96 (1H, s), 8.83 (1H, s), 10.04 (1H, s), 10.60 (1H, s)

MS (ESI-): 550.3 (M-H)

Example 269

(2S)-N-Hydroxy-2-[5-{3-(((2R)-2-aminopropionyl)amino)-phenyl}-2-thienyl]-3,4,5,6-tetrahydro-2H-thiopyran-2-acetamide 1,1-dioxide (130 mg) from (2S)-N-(2-tetrahydropyranyloxy)-2-[5-{3-(((2R)-2-tert-butoxycarbonylaminopropionyl)amino)phenyl}-2-thienyl]-3,4,5,6-tetrahydro-2H-thiopyran-2-acetamide 1,1-dioxide

NMR (DMSO-$d_6$, δ): 1.50 (3H, d, J=7.0Hz), 1.72-2.03 (4H, m), 2.35-2.46 (1H, m), 2.95-3.27 (4H, m), 3.43-3.54 (1H, m), 4.05 (1H, br t, J=7.0Hz), 7.21 (1H, d, J=4.0Hz), 7.37-7.43 (3H, m), 7.55 (1H, d, J=7.5Hz), 7.95 (2H, s), 8.28-8.32 (3H, m), 10.63 (1H, s), 10.81 (1H, s)

MS (ESI-): 452.1 (M-H)

Example 270

(2S)-N-Hydroxy-2-[5-{3-(2-(4-methylphenoxy)-acetylamino)phenyl}-2-thienyl]-3,4,5,6-tetrahydro-2H-thiopyran-2-acetamide 1,1-dioxide (150 mg)

NMR (DMSO-$d_6$, δ): 1.74-2.04 (4H, m), 2.24 (3H, s), 2.37-2.45 (1H, m), 2.95-3.26 (4H, m), 3.44-3.53 (1H, m), 4.67 (2H, s), 6.90 (2H, d, J=7.5Hz), 7.11 (2H, d, J=7.5Hz), 7.21 (1H, d, J=4.0Hz), 7.34-7.37 (2H, m), 7.42 (1H, d, J=4.0Hz), 7.56 (1H, d, J=6.0Hz), 8.01 (1H, s), 8.82 (1H, s), 10.17 (1H, s), 10.59 (1H, s)

MS (ESI-): 528.1 (M-H)

Example 271

(2S)-N-Hydroxy-2-[5-{3-(2-N,N-dimethylamino)acetylamino)phenyl}-2-thienyl]-3,4,5,6-tetrahydro-2H-thiopyran-2-acetamide 1,1-dioxide (65 mg)

NMR (DMSO-$d_6$, δ): 1.74-2.05 (4H, m), 2.36-2.45 (1H, m), 2.89 (6H, s), 2.96-3.27 (4H, m), 3.44-3.54 (1H, m), 4.17 (2H, s), 7.22 (1H, d, J=4.0Hz), 7.38-7.42 (2H, m), 7.50 (1H, d, J=5.0Hz), 7.95 (1H, s), 8.84 (1H, br) , 9.88 (1H, br), 10.60 (1H, s), 10.82 (1H, s)

MS (ESI-): 464.3 (M-H)

Example 272

(2S)-N-Hydroxy-2-[5-(3-(allyloxycarbonylamino)-phenyl)-2-thienyl]-3,4,5,5-tetrahydro-2H-thiopyran-2-acetamide 1,1-dioxide (50 mg)

NMR (DMSO-$d_6$, δ): 1.73-2.04 (4H, m), 2.36-2.45 (1H, m), 2.95-3.25 (4H, m), 3.41-3.49 (1H, m), 4.63 (2H, d, J=5.0Hz), 5.25 (1H, d, J=8.0Hz), 5.38 1H, d, J=8.0Hz), 5.93-6.06 (1H, m), 7.20 (1H, d, J=4.0Hz), 7.30-7.35 (3H, m), 7.49 (1H, d, J=4.0Hz), 7.84 (1H, s), 8.83 (1H, s), 9.85 (1H, s)

MS (ESI-): 463.3 (M-H).

Example 273

(2S)-N-Hydroxy-2-[5-(3-ethoxycarbanylmethyliminocarbonylamino)phenyl)-2-thienyl]-3,4,5,6-tetrahydro-2H-thiopyran-2-acetamide 1,1-dioxide (40 mg)

NMR (DMSO-$d_6$, δ): 1.23 (3H, t, J=7.5Hz), 1.73-2.05 (4H, m), 2.36-2.47 (1H, m), 2.94-3.25 (4H, m), 3.42-3.54 (1H, m), 3.87 (2H, d, J=7.0Hz), 4.13 (2H, q, J=7.5Hz), 6.50 (1H, t, J=7.0Hz), 7.18 (1H, d, J=4.0Hz), 7.21-7.30 (3H, m), 7.37 (1H, d, J=4.0Hz), 7.83 (1H, s), 8.83 (1H, s), 8.96 (1H, s)

MS (ESI-): 508.3 (M-H)

Example 274

(2S)-N-Hydroxy-2-[5-(3-(benzyloxyacetylamino)phenyl)-2-thienyl]-3,4,5,6-tetrahydro-2H-thiopyran-2-acetamide 1,1-dioxide (55 mg)

NMR (DMSO-$d_6$, δ): 1.75-2.06 (4H, m), 2.36-2.46 (1H, m), 2.95-3.25 (4H, m), 3.42-3.53 (1H, m), 4.11 (2H, s), 4.64 (2H, s), 7.21 (1H, d, J=4.0Hz), 7.28-7.43 (8H, m), 7.54-7.57 (1H, m), 8.01 (1H, s), 8.82 (1H, s), 9.90 (1H, s), 10.60

(1H, s)
MS (ESI-): 527.3 (M-H)

Example 275

(2S)-N-Hydroxy-2-[5-(3-(cyclopentylcarbonylamino)-phenyl)-2-thienyl]-3,4,5,6-tetrahydro-2H-thiopyran-2-acetamide 1,1-dioxide (120 mg)
NMR (DMSO-$d_6$, δ): 1.52-2.06 (12H, m), 2.36-2.47 (1H, m), 2.78 (1H, tt, J=7.0, 7.0Hz), 2.95-3.25 (4H, m), 3.40-3.54 (1H, m), 7.20 (1H, d, J=4.0Hz), 7.32-7.34 (2H, m), 7.40 (1H, d, J=4.0Hz), 7.45-7.49 (1H, m), 8.03 (1H, s), 8.84 (1H, s), 9.98 (1H, s), 10.61 (1H, s)
MS (ESI-): 475.3 (M-H)

Example 276

(2S)-N-Hydroxy-2-[5-(3-((2-hydroxyethyl)-aminocarbonylamino)phenyl)-2-thienyl]-3,4,5,6-tetrahydro-2H-thiopyran-2-acetamide 1,1-dioxide (20 mg)
NMR (DMSO-$d_6$, δ): 1.72-2.04 (4H, m), 2.40-2.45 (1H, m), 2.93-3.25 (6H, m), 3.41-3.50 (3H, m), 4.75 (1H, t, J=5.0Hz), 6.22 (1H, t, J=5.0Hz), 7.16-7.25 (3H, m), 7.35 (1H, d, J=4.0Hz), 7.82 (1H, s), 8.71 (1H, s), 8.83 (1H, s)
MS (ESI-): 466.4 (M-H)

Example 277

(2S)-N-Hydroxy-2-[5-(3-((2-aminoethoxy)carbonylamino)-phenyl)-2-thienyl]-3,4,5,6-tetrahydro-2H-thiopyran-2-acetamide 1,1-dioxide hydrochloride (20 mg)
NMR (DMSO-$d_6$, δ): 1.74-2.07 (4H, m), 2.35-2.44 (1H, m), 2.95-3.27 (4H, m), 3.48-3.66 (5H, m), 7.12-7.51 (4H, m), 7.86-7.90 (2H, m), 8.00-8.08 (2H, m), 9.10 (1H, s), 9.85 (1H, s), 10.62 (1H, s)
MS (ESI+): 468.3 (M+H)

Example 278

(2S)-N-Hydroxy-2-[5-(3-(3-chloropropionylamino)phenyl)-2-thienyl]-3,4,5,6-tetrahydro-2H-thiopyran-2-acetamide 1,1-dioxide (95 mg)
NMR (DMSO-$d_6$, δ): 1.73-2.06 (4H, m), 2.37-2.46 (1H, m), 2.84 (2H, t, J=6.0Hz), 2.95-3.26 (4H, m), 3.43-3.56 (1H, m), 3.90 (2H, t, J=6.0Hz), 7.21 (1H, d, J=4.0Hz), 7.35-7.40 (2H, m), 7.42 (1H, d, J=4.0Hz), 7.47-7.52 (1H, m), 8.00 (1H, s), 8.85 (1H, s)
MS (ESI-): 469.1 (M-H)

Example 279

(2S)-N-Hydroxy-2-[5-(4-(methanesulfonylamino)phenyl)-2-thienyl]-3,4,5,6-tetrahydro-2H-thiopyran-2-acetamide 1,1-dioxide (40 mg)
NMR (DMSO-$d_6$, δ): 1.72-2.05 (4H, m), 2.34-2.44 (1H, m), 2.89 (3H, s), 2.93-3.23 (4H, m), 3.41-3.51 (1H, m), 7.12 (2H, d, J=7.5Hz), 7.15 (1H, d, J=4.0Hz), 7.31 (1H, d, J=4.0Hz), 7.50 (2H, d, J=7.5Hz)
MS (ESI-): 457.3 (M-H)

Example 280

(2S)-N-Hydroxy-2-[5-(4-(2-(phenylaminocarbonyl)-ethenyl)phenyl)-2-thienyl]-3,4,5,6-tetrahydro-2H-thiopyran-2-acetamide 1,1-dioxide (40 mg)
NMR (DMSO-$d_6$, δ): 1.73-2.07 (4H, m), 2.38-2.48 (1H, m), 2.97-3.54 (5H, m), 6.86 (1H, d, J=16Hz), 7.06 (1H, dd, J=8.0, 8.0Hz), 7.24 (1H, d, J=4.0Hz), 7.34 (2H, dd, J=8.0, 8.0Hz), 7.56 (1H, d, J=4.0Hz), 7.61 (1H, d, J=16Hz), 7.65-7.75 (6H, m), 8.85 (1H, s), 10.2 (1H, s), 10.6 (1H, s)

Example 281

(2S)-N-Hydroxy-2-[5-(4-(2-(4-methoxyphenylaminocarbonyl)ethenyl)phenyl)-2-thienyl]-3,4,5,6-tetrahydro-2H-thiopyran-2-acetamide 1,1-dioxide (100 mg)
NMR (DMSO-$d_6$, δ): 1.72-2.07 (4H, m), 2.37-2.46 (1H, m), 2.97-3.52 (5H, m), 3.74 (3H, s), 6.83 (1H, d, J=16Hz),

6.92 (2H, d, J=8.5Hz), 7.24 (1H, d, J=4.0Hz), 7.54-7.75 (8H, m), 8.85 (1H, s), 10.08 (1H, s), 10.6 (1H, s)

Example 282

(2S)-N-Hydroxy-2-[5-(4-(2-(4-fluorophenylaminocarbonyl)ethenyl)phenyl)-2-thienyl]-3,4,5,6-tetrahydro-2H-thiopyran-2-acetamide 1,1-dioxide (80 mg).

NMR (DMSO-$d_6$, δ): 1.73-2.07 (4H, m), 2.37-2.46 (1H, m), 2.98-3.53 (5H, m), 6.83 (1H, d, J=16Hz), 17.18 (2H, dd, J=8.5, 8.5Hz), 7.24 (1H, d, J=4.0Hz), 7.57-7.75 (8H, m), 8.85 (1H, s), 10.3 (1H, s), 10.6 (1H, s)

Example 283

(2S)-N-Hydroxy-2-[5-(4-(ethylcarbonyloxy)phenyl)-2-thienyl]-3,4,5,6-tetrahydro-2H-thiopyran-2-acetamide 1,1-dioxide (100 mg)

NMR (DMSO-$d_6$, δ): 1.13 (3H, dd, J=7.5, 7.5Hz), 1.71-2.07 (4H, m), 2.36-2.46 (1H, m), 2.62 (2H, ddd, J=7.5, 7.5, 7.5Hz), 2.96-3.52 (5H, m), 7.20-7.21 (3H, m), 7.46 (1H, d, J=4.0Hz), 7.68 (2H, d, J=8.5Hz), 8.85 (1H, s)

MS (ESI-): 436 (M-H)

Example 284

(2S)-N-Hydroxy-2-[5-(4-(methoxycarbonylaminomethyl)-phenyl)-2-thienyl]-3,4,5,6-tetrahydro-2H-thiopyran-2-acetamide 1,1-dioxide (45 mg)

NMR (DMSO-$d_6$, δ): 1.70-2.06 (4H, m), 2.35-2.45 (1H, m), 2.96-3.50 (5H, m), 3.56 (3H, s), 4.19 (2H, d, J=7.0Hz), 7.20 (1H, d, J=4.0Hz), 7.29 (2H, d, J=8.5Hz), 7.43 (1H, d, J=4.0Hz), 7.60 (2H, d, J=8.5Hz), 7.70 (1H, t, J=7.0Hz), 8.85 (1H, s), 10.60 (1H, s)

MS (ESI-): 451 (M-H)

Example 285

(2S)-N-Hydroxy-2-[5-(4-(ethylcarbonylmethoxy)phenyl)-2-thienyl]-3,4,5,6-tetrahydro-2H-thiopyran-2-acetamide 1,1-dioxide (120 mg)

NMR (DMSO-$d_6$, δ): 0.98 (3H, t, J=7.2Hz), 1.70-2.07 (4H, m), 2.33-2.45 (1H, m), 2.54 (2H, q, J=7.2Hz), 2.95-3.50 (5H, m), 4.86 (2H, s), 6.95 (2H, d, J=8.5Hz), 7.16 (1H, d, J=4.0Hz), 7.34 (1H, d, J=4.0Hz), 7.55 (2H, d, J=8.5Hz), 8.84 (1H, br)

MS (ESI-): 450 (M-H)

Example 286

(2S)-N-Hydroxy-2-[5-(4-(cyclopropylaminocarbonylmethoxy)phenyl)-2-thienyl]-3,4,5,6-tetrahydro-2H-thiopyran-2-acetamide 1,1-dioxide (70 mg)

NMR (DMSO-$d_6$, δ): 0.46-0.51 (2H, m), 0.60-0.66 (2H, m), 1.70-2.07 (4H, m), 2.34-2.45 (1H, m), 2.66-2.75 (1H, m), 2.95-3.50 (5H, m), 4.47 (2H, s), 6.99 (2H, d, J=8.5Hz), 7.16 (1H, d, J=4.0Hz), 7.35 (1H, d, J=4.0Hz), 7.57 (2H, d, J=8.5Hz), 8.15 (1H, br), 8.84 (1H, s), 10.59 (1H, s)

Example 287

(2S)-N-Hydroxy-2-[5-(4-(n-propylaminocarbonylmethoxy)phenyl)-2-thienyl]-3,4,5,6-tetrahydro-2H-thiopyran-2-acetamide 1,1-dioxide (70 mg)

NMR (DMSO-$d_6$, δ): 0.82 (3H, t, J=7.5Hz), 1.39-1.50 (2H, m), 1.73-2.07 (4H, m), 2.35-2.45 (1H, m), 2.95-3.52 (7H, m), 4.50 (2H, s), 7.01 (2H, d, J=8.5Hz), 7.17 (1H, d, J=4.0Hz), 7.35 (1H, d, J=4.0Hz), 7.58 (2H, d, J=8.5Hz), 8.11 (1H, br), 8.85 (1H, s), 10.59 (1H, s)

MS (ESI-): 479 (M-H)

Example 288

(2S)-N-Hydroxy-2-[5-(3-hydroxyphenyl)-2-thienyl]-3,4,5,6-tetrahydro-2H-thiopyran-2-acetamide 1,1-dioxide (120 mg)

NMR (DMSO-$d_6$, δ): 1.70-2.05 (4H, m), 2.36-2.45 (1H, m), 2.95-3.50 (5H, m), 6.71-6.74 (1H, m), 7.00 (1H, s), 7.06 (1H, d, J=8.0Hz), 7.17-7.25 (2H, m), 7.39 (1H, d, J=4.0Hz), 8.85 (1H, s), 9.62 (1H, s), 10.60 (1H, s)

MS (ESI-): 380 (M-H)

Example 289

(2S)-N-Hydroxy-2-[5-(3-butylaminocarbonylamino)phenyl)-2-thienyl]-3,4,5,6-tetrahydro-2H-thiopyran-2-acetamide 1,1-dioxide (170 mg)

NMR (DMSO-$d_6$, δ): 0.90 (3H, dd, J=7.2, 7.2Hz), 1.27-1.37 (2H, m), 1.37-1.47 (2H, m), 1.70-2.05 (4H, m), 2.37-2.45 (1H, m), 2.95-3.50 (7H, m), 6.16 (1H, dd, J=7.0, 7.0Hz), 7.17-7.28 (4H, m), 7.37 (1H, d, J=4.0Hz), 7.84 (1H, s), 8.55 (1H, s), 10.6 (1H, s)

MS (ESI-): 478 (M-H)

Example 290

(2S)-N-Hydroxy-2-[5-(3-(1-naphtyl)aminocarbonylamino)-phenyl)-2-thienyl]-3,4,5,6-tetrahydro-2H-thiopyran-2-acetamide 1,1-dioxide (250 mg)

NMR (DMSO-$d_6$, δ): 1.72-2.07 (4H, m), 2.38-2.47 (1H, m), 2.95-3.31 (5H, m), 7.22 (1H, d, J=4.0Hz), 7.29-7.37 (3H, m), 7.44 (1H, d, J=4.0Hz), 7.47-7.68 (4H, m), 7.94-7.96 (2H, m), 8.02 (1H, d, J=8.3Hz), 8.15 (1H, d, J=8.3Hz), 8.85 (1H, s), 9.27 (1H, s), 10.6 (1H, s)

MS (ESI-): 548 (M-H)

Example 291

(2S)-N-Hydroxy-2-[5-(3-(allylaminocarbonylamino)-phenyl)-2-thienyl]-3,4,5,6-tetrahydro-2H-thiopyran-2-acetamide 1,1-dioxide (200 mg)

NMR (DMSO-$d_6$, δ): 1.70-2.07 (4H, m), 2.36-2.45 (1H, m), 2.96-3.25 (5H, m), 3.72-3.76 (2H, m), 5.02-5.19 (2H, m), 5.80-5.94 (1H, m), 6.29-6.32 (1H, m), 7.18-7.26 (4H, m), 7.38 (1H, d, J=4.0Hz), 7.84 (1H, s), 8.68 (1H, s), 10.6 (1H, s)

MS (ESI-): 462 (M-H)

Example 292

(2S)-N-Hydroxy-2-[S-(3-(isobutylaminocarbonylamino)-phenyl)-2-thienyl]-3,4,5,6-tetrahydro-2H-thiopyran-2-acetamide 1,1-dioxide (170 mg)

NMR (DMSO-$d_6$, δ): 0.88 (6H, d, J=6.6Hz), 1.65-2.07 (5H, m), 2.40-2.47 (1H, m), 2.94 (2H, dd, J=6.5, 6.5Hz), 3.00-3.50 (5H, m), 6.23 (1H, dd, J=6.5, 6.5Hz), 7.17-7.20 (3H, m), 7.37 (1H, d, J=4.0Hz), 7.84 (1H, s), 8.54 (1H, s), 10.6 (1H, s)

MS (ESI-): 478 (M-H)

Example 293

(2S)-N-Hydroxy-2-[S-(3-(cyclohexylmethylaminocarbonylamino)phenyl)-2-thienyl]-3,4,5,6-tetrahydro-2H-thiopyran-2-acetamide 1,1-dioxide (110 mg)

NMR (DMSO-$d_6$, δ): 0.83-0.95 (2H, m), 1.11-1.25 (3H, m), 1.60-1.75 (6H, m), 1.83-2.07 (4H, m), 2.37-2.45 (1H, m), 2.93-3.07 (4H, m), 3.08-3.30 (3H, m), 6.22 (1H, br), 7.19-7.28 (4H, m), 7.37 (1H, d, J=4.0Hz), 8.55 (1H, s), 10.6 (1H, s)

MS (ESI-): 518 (M-H)

Example 294

(2S)-N-Hydroxy-2-[5-(3-(2-methoxyethylaminocarbonylamino)phenyl)-2-thienyl]-3,4,5,6-tetrehydro-2H-thiopyran-2-acetamide 1,1-dioxide (150 mg)

NMR (DMSO-$d_6$, δ): 1.71-2.06 (4H, m), 2.37-2.46 (1H, m), 2.95-3.01 (2H, m), 3.05-3.25 (3H, m), 3.28 (3H, s), 3.35-3.51 (4H, m), 6.21-6.25 (1H, m), 7.16-7.25 (4H, m), 7.37 (1H, d, J=4.0Hz), 7.83 (1H, s), 8.68 (1H, s), 8.79-8.86 (1H, br), 10.6 (1H, s)

MS (ESI-): 480 (M-H)

Example 295

(2S)-N-Hydroxy-2-[5-(3-(N-methyl-N-ethylaminocarbonyl)-amino)phenyl)-2-thienyl]-3,4,5,6-tetrahydro-2H-thiopyran-2-acetamide 1,1-dioxide (55 mg)

NMR (DMSO-$d_6$, δ): 1.08 (3H, t, J=7.0Hz), 1.70-2.08 (4H, m), 2.37-2.45 (1H, m), 2.94 (3H, s), 2.97-3.54 (7H, m), 7.20 (1H, d, J=4.0Hz), 7.25 (2H, d, J=8.0Hz), 7.37 (1H, d, J=4.0Hz), 7.45 (2H, d, J=8.0Hz), 7.84 (1H, s), 8.84 (1H, s), 10.59 (1H, s)

MS (ESI-): 464 (M-H)

Example 296

(2S)-N-Hydroxy-2-[5-(4-(2-(N,N-dimethylaminocarbonyl)-ethenyl)phenyl)-2-thienyl]-3,4,5,6-tetrahydro-2H-thiopyran-2-acetamide 1,1-dioxide (180 mg)

NMR (DMSO-$d_6$, δ): 1.70-2.08 (4H, m), 2.37-2.45 (1H, m), 2.93 (3H, s), 2.94-3.10 (2H, m), 3.17 (3H, s), 3.18-3.55 (3H, m), 7.20 (1H, d, J=4.0Hz), 7.25 (1H, d, J=16Hz), 7.45 (1H, d, J=16Hz), 7.55 (1H, d, J=4.0Hz), 7.66 (2H, d, J=8.5Hz), 7.75 (2H, d, J=8.5Hz), 8.84 (1H, br), 10.60 (1H, s)

MS (ESI-): 461 (M-H)

Example 297

(2S)-N-Hydroxy-2-[5-(4-(2-(isopropylaminocarbonyl)-ethenyl)phenyl)-2-thienyl]-3,4,5,6-tetrahydro-2H-thiopyran-2-acetamide 1,1-dioxide (110 mg)

NMR (DMSO-$d_6$, δ): 1.11 (6H, d, J=6.5Hz), 1.70-2.07 (4H, m), 2.36-2.44 (1H, m), 2.97-3.55 (5H, m), 3.40-4.00 (1H, m), 6.62 (1H, d, J=16Hz), 7.23 (1H, d, J=4.0Hz), 7.40 (1H, d, J=16Hz), 7.55 (1H, d, J=4.0Hz), 7.58 (2H, d, J=8.5Hz), 7.69 (2H, d, J=8.5Hz), 7.99 (1H, d, J=7.5Hz), 10.6 (1H, s)

MS (ESI-): 475 (M-H)

Example 298

(2S)-N-Hydroxy-2-[5-(4-(2-(propylaminocarbonyl)-ethenyl)phenyl)-2-thienyl]-3,4,5,6-tetrahydro-2H-thiopyran-2-acetamide 1,1-dioxide (120 mg)

NMR (DMSO-$d_6$, δ): 0.88 (3H, t, J=7.5Hz), 1.47 (2H, q, J=7.5Hz), 1.70-2.07 (4H, m), 2.35-2.45 (1H, m), 2.96-3.55 (7H, m), 6.65 (1H, d, J=16Hz), 7.23 (1H, d, J=4.0Hz), 7.40 (1H, d, J=16Hz), 7.55 (1H, d, J=4.0Hz), 7.60 (2H, d, J=8.5Hz), 7.69 (2H, d, J=8.5Hz), 8.10 (1H, t, J=7.0Hz), 10.6 (1H, s)

MS (ESI-): 475 (M-H)

Example 299

(2S)-N-Hydroxy-2-[5-(4-(methylaminocarbonyloxy)phenyl)-2-thienyl]-3,4,5,6-tetrahydro-2H-thiopyran-2-acetamide 1,1-dioxide (150 mg)

NMR (DMSO-$d_6$, δ): 1.71-2.05 (4H, m), 2.35-2.45 (1H, m), 2.67 (3H, d, J=4.5Hz), 2.95-3.51 (5H, m), 7.15 (2H, d, J=8.7Hz), 7.21 (1H, d, J=4.0Hz), 7.43 (1H, d, J=4.0Hz), 7.63 (2H, d, J=8.7Hz), 7.65-7.68 (1H, m), 8.85 (1H, s), 10,6 (1H, s)

MS (ESI-): 437 (M-H)

Example 300

(2S)-N-Hydroxy-2-[5-(4-(ethylaminocarbonyloxy)phenyl)-2-thienyl]-3,4,5,6-tetrahydro-2H-thiopyran-2-acetamide 1,1-dioxide (130 mg)

NMR (DMSO-$d_6$, δ): 1.08 (3H, dd, J=7.2, 7.2Hz), 1.71-2.05 (4H, m), 1.85-1.96 (1H, m), 2.95-3.30 (6H, m), 3.41-3.53 (1H, m), 7.15 (2H, d, J=8.7Hz), 7.20 (1H, d, J=4.0Hz), 7.43 (1H, d, J=4.0Hz), 7.63 (2H, d, J=8.7Hz), 7.80 (1H, dd, J=7.0, 7.0Hz), 8.85 (1H, s), 10.6 (1H, s)

MS (ESI-): 451 (M-H)

Example 301

(2S)-N-Hydroxy-2-[5-(5-acetyl-2-thienyl)-2-thienyl]-3,4,5,6-tetrahydro-2H-thiopyran-2-acetamide 1,1-dioxide (20 mg)

NMR (DMSO-$d_6$, δ): 1.68-2.07 (4H, m), 2.35-2.45 (1H, m), 2.53 (3H, s), 2.95-3.53 (5H, m), 7.21 (1H, d, J=4.0Hz), 7.44 (1H, d, J=4.0Hz), 7.51 (1H, d, J=4.0Hz), 7.91 (1H, d, J=4.0Hz), 8.85 (1H, s)

MS (ESI-): 412 (M-H)

The following compounds were obtained in a similar manner to that of Example 166.

Example 302

(2S)-N-Hydroxy-2-[5-(4-methoxyacetoxy)phenyl)-2-thienyl]-3,4,5,6-tetrahydro-2H-thiopyran-2-acetamide 1,1-dioxide (100 mg)

NMR (DMSO-$d_6$, δ): 1.71-2.05 (4H, m), 2.36-2.45 (1H, m), 2.97-3.28 (4H, m), 3.41 (3H, s), 3.42-3.51 (1H, m), 4.36 (2H, s), 7.21 (1H, d, J=4.0Hz), 7.22 (2H, d, J=8.5Hz), 7.48 (1H, d, J=4.0Hz), 7.70 (2H, d, J=8.5Hz), 8.84 (1H, s)

MS (ESI-): 452 (M-H)

Example 303

(2S)-N-Hydroxy-2-[5-(4-(ethoxycarbonyloxy)phenyl)-2-thienyl]-3,4,5,6-tetrahydro-2H-thiopyran-2-acetamide 1,1-dioxide (80 mg)

NMR (DMSO-$d_6$, δ): 1.31 (3H, dd, J=7.5, 7.5Hz), 1.70-1.93 (4H, m), 2.36-2.47 (1H, m), 2.97-3.51 (5H, m), 4.27 (2H, ddd, J=7.5, 7.5, 7.5Hz), 7.21 (1H, d, J=4.0Hz), 7.30 (2H, d, J=8.5Hz), 7.49 (1H, d, J=4.0Hz), 7.69 (2H, d, J=8.5Hz)

MS (ESI-): 452 (M-H)

Example 304

(2S)-N-Hydroxy-2-[5-(4-(cyclopropylcarbonyloxy)phenyl)-2-thienyl]-3,4,5,6-tetrahydro-2H-thiopyran-2-acetamide 1,1-dioxide (100 mg)

NMR (DMSO-$d_6$, δ): 1.03-1.06 (4H, m), 1.70-2.05 (5H, m), 2.36-2.47 (1H, m), 2.95-3.51 (5H, m), 7.17-7.21 (3H, m), 7.46 (1H, d, J=4.0Hz), 7.66 (2H, d, J=8.5Hz), 8.84 (1H, s), 10.58 (1H, s)

MS (ESI-): 448 (M-H)

Example 305

(2S)-N-Hydroxy-2-[5-(4-(ethoxycarbonylmethoxy)-phenyl)-2-thienyl]-3,4,5,6-tetrahydro-2H-thiopyran-2-acetamide 1,1-dioxide (150 mg)

NMR (DMSO-$d_6$, δ): 1.22 (3H, t, J=7.2Hz), 1.70-2.07 (4H, m), 2.35-2.45 (1H, m), 2.95-3.52 (5H, m), 4.17 (2H, q, J=7.2Hz), 4.82 (2H, s), 6.97 (2H, d, J=8.5Hz), 7.16 (1H, d, J=4.0Hz), 7.34 (1H, d, J=4.0Hz), 7.56 (2H, d, J=8.5Hz), 8.83 (1H, s), 10.6 (1H, s)

Example 306

To a solution of 0.5M 4-chlorophenylisocyanate (192 mg) in dichloromethane (2.5 ml) was added N-[2-[2-(5-(3-aminophenyl)-2-thienyl)-1,1-dioxo-3,4,5,6-tetrahydro-2H-thiopyran-2-yl]acetyl]hydroxylamine trityl crowns (26 µmol, 13.2 µmol/crown x 2). The reaction mixture was left overnight at ambient temperature. The crowns were washed with N,N-dimethylformamide, methanol and dichloromethane, successively. The crowns were treated with 5% trifluoroacetic acid in dichloromethane for 1 hour at ambient temperature and removed from the solution. After the solution was evaporated under a stream of nitrogen, the residue was purified by reverse phase HPLC (0.1% trifluoroacetic acid in acetonitrile, 10-40% gradient) to give (2S)-N-hydroxy-2-[5-(3-(4-chlorophenylaminocarbonylamino)phenyl)-2-thienyl]-3,4,5,6-tetrahydro-2H-thiopyran-2-acetamide 1,1-dioxide (1.6 mg) as a white powder.

MS (ESI+): 551.3 (M+H+$NH_3$)

The following compounds were obtained in substantially the same manner as that of Example 306.

Example 307

(2S)-N-Hydroxy-2-[5-(3-(n-phenylaminocarbonylamino)-phenyl)-2-thienyl]-3,4,5,6-tetrahydro-2H-thiopyran-2-acetamide 1,1-dioxide (1.2 mg)

MS (ESI-): 492.3 (M-H)

Example 308

(2S)-N-Hydroxy-2-[5-(3-(n-hexylaminocarbonylamino)-phenyl)-2-thienyl]-3,4,5,6-tetrahydro-2H-thiopyran-2-acetamide 1,1-dioxide (3.0 mg)

MS (ESI-): 506.4 (M-H)

Example 309

(2S)-N-Hydroxy-2-[5-(3-(3-chlorophenylaminocarbonylamino)phenyl)-2-thienyl]-3,4,5,6-tetrahydro-2H-thiopyran-2-acetamide 1,1-dioxide (1.3 mg)
MS (ESI+): 550.7 (M+H+$NH_3$)

Example 310

(2S)-N-Hydroxy-2-[5-(3-(((R)-2-amino-2-phenylacetyl)-amino)phenyl)-2-thienyl]-3,4,5,6-tetrahydro-2H-thiopyran-2-acetamide 1,1-dioxide hydrochloride (76 mg) was obtained in a similar manner to that of Example 52.
NMR (DMSO-$d_6$, δ): 1.64-2.11 (4H, m), 2.32-2.98 (1H, m), 2.87-3.62 (5H, m), 5.30 (1H, br s), 7.22 (1H, s), 7.34-7.82 (10H, m), 7.97 (1H, s), 8.91 (2H, br s), 10.66 (1H, br s), 11.27 (1H, s)
MS (ESI+): 514 (M+H)

Example 311

(2S)-N-Hydroxy-2-[5-(3-(3-(benzoylamino)-propionylamino)phenyl)-2-thienyl]-3,4,5,6-tetrahydro-2H-thiopyran-2-acetamide 1,1-dioxide (96 mg) was obtained in a similar manner to that of Example 194.
NMR (DMSO-$d_6$, δ): 1.64-2.10 (4H, m), 2.33-2.48 (1H, m), 2.58-2.76 (2H, m), 2.88-3.56 (9H, m), 7.21 (1H, br s), 7.30-7.64 (8H, m), 7.84 (2H, br s), 8.03 (1H, br s), 8.64 (1H, br s)
MS (ESI-): 554 (M-H)

Example 312

(2S)-N-Hydroxy-2-[5-(3-(3-(ethylaminocarbonylamino)-propionylamino)phenyl)-2-thienyl]-3,4,5,6-tetrahydro-2H-thiopyran-2-acetamide 1,1-dioxide (98 mg) was obtained in a similar manner to that of Example 194.
NMR (DMSO-$d_6$, δ): 0.96 (3H, t, J=7Hz), 1.69-2.10 (4H, m), 2.33-2.48 (1H, m), 2.90-3.64 (11H, m), 7.19 (1H, d, J=3Hz), 7.28-7.51 (5H, m), 8.04 (1H, s)
MS (ESI-): 521 (M-H)

Example 313

(2S)-N-Hydroxy-2-[5-(3-(3-(methanesulfonylamino)-propionylamino)phenyl)-2-thienyl]-3,4,5,6-tetrahydro-2H-thiopyran-2-acetamide 1,1-dioxide (54 mg) was obtained in a similar manner to that of Example 194.
NMR (DMSO-$d_6$, δ): 1.22-2.12 (4H, m), 2.35-2.49 (1H, m), 2.58 (2H, t, J=7Hz), 2.99 (3H, s), 2.94-3.62 (7H, m), 7.06-7.16 (1H, m), 7.22 (1H, d, J=3Hz), 7.33-7.39 (2H, m), 7.40 (1H, d, J=3Hz), 7.43-7.52 (1H, m), 8.01 (1H, s)
MS (ESI-): 528 (M-H)

Example 314

(2S)-N-Hydroxy-2-[5-(3-((4-piperidinyloxy)acetylamino)-phenyl)-2-thienyl]-3,4,5,6-tetrahydro-2H-thiopyran-2-acetamide 1,1-dioxide hydrochloride (55 mg) was obtained from (2S)-N-(2-tetrahydropyranyloxy)-2-[5-(3-((1-tert-butoxycarbonyl-4-piperidinyloxy)acetylamino)phenyl)-2-thienyl]-3,4,5,6-tetrahydro-2H-thiopyran-2-acetamide 1,1-dioxide in a similar manner to that of Example 52.
NMR (DMSO-$d_6$, δ): 1.68-2.11 (8H, m), 2.34-2.98 (1H, m), 2.89-3.56 (8H, m), 3.66-3.80 (1H, m), 4.14 (2H, s), 7.11-7.62 (5H, m), 8.00 (1H, s), 8.85 (1H, s), 9.83 (1H, s), 10.60 (1H, s)
MS (ESI+): 522 (M+H)

The following compounds were obtained in a similar manner to that of Example 85.

Example 315

(2S)-N-(2-Tetrahydropyranyloxy)-2-[5-(4-(methanesulfonylamino)phenyl)-2-thienyl]-3,4,5,6-tetrahydro-2H-thiopyran-2-acetamide 1,1-dioxide (70 mg)
NMR ($CDCl_3$, δ): 1.32 (2H, br), 1.40-1.49 (2H, m), 1.64-1.68 (2H, m), 1.94 (2H, br), 2.10-2.20 (2H, m), 2.76-2.80 (2H, m), 3.00 (2H, s), 3.06-3.10 (2H, m), 3.15 (2H, br), 3.33-3.51 (1H, m), 3.65-3.74 (1H, m), 4.55 (1/2H, br), 4.82 (1/2H, br), 7.01 (1H, br), 7.11-7.16 (3H, m), 7.45 (2H, d, J=7.5Hz), 8.25 (1/2H, s), 8.33 (1/2H, s)
MS (ESI-): 541.4 (M-H)

Example 316

(2S)-N-(2-Tetrahydropyranyloxy)-2-[5-(4-(methoxycarbonylaminomethyl)phenyl)-2-thienyl]-3,4,5,6-tetrahydro-2H-thiopyran-2-acetamide 1,1-dioxide (80 mg)

NMR (DMSO-$d_6$, δ): 1.37 (6H, m), 1.69-1.81 (2H, m), 1.82-2.07 (2H, m), 2.35-2.45 (1H, m), 2.90-3.50 (6H, m), 3.57 (3H, s), 3.72-3.88 (1H, m), 4.20 (2H, d, J=7.0Hz), 4.45, 4.75 (1H, s), 7.20-7.22 (1H, m), 7.28 (2H, d, J=8.5Hz), 7.41-7.43 (1H, m), 7.61 (2H, d, J=8.5Hz), 7.72 (1H, t, J=7.0Hz)

MS (ESI-): 535 (M-H)

Example 317

(2S)-N-(2-Tetrahydropyranyloxy)-2-[5-(4-(cyclopropylcarbonyloxy)phenyl)-2-thienyl]-3,4,5,6-tetrahydro-2H-thiopyran-2-acetamide 1,1-dioxide (150 mg)

NMR (DMSO-$d_6$, δ): 1.03-1.07 (4H, m), 1.39-1.64 (6H, m), 1.70-2.05 (5H, m), 2.35-2.45 (1H, m), 2.90-3.51 (6H, m), 3.75-3.90 (1H, m), 4.45, 4.75 (1H, s), 7.02-7.05 (1H, m), 7.19 (2H, d, J=8.5Hz), 7.44-7.47 (1H, m), 7.76 (2H, d, J=8.5Hz)

Example 318

(2S)-N-(2-Tetrahydropyranyloxy)-2-[5-(4-(ethoxycarbonylmethoxy)phenyl)-2-thienyl]-3,4,5,6-tetrahydro-2H-thiopyran-2-acetamide 1,1-dioxide (220 mg)

NMR (DMSO-$d_6$, δ): 1.22 (3H, t, J=7.0Hz), 1.35-1.62 (6H, m), 1.66-2.05 (4H, m), 2.35-2.45 (1H, m), 2.90-3.50 (6H, m), 3.73-3.90 (1H, m), 4.18 (2H, q, J=7.0Hz), 4.45, 4.75 (1H, s), 4.82 (2H, s), 6.98 (2H, d, J=8.5Hz), 7.16-7.21 (1H, m), 7.32-7.36 (1H, m), 7.57 (2H, d, J=8.5Hz)

Example 319

(2S)-N-(2-Tetrahydropyranyloxy)-2-[5-(4-(ethylcarbonylmethoxy)phenyl)-2-thienyl]-3,4,5,6-tetrahydro-2H-thiopyran-2-acetamide 1,1-dioxide (200 mg)

NMR (DMSO-$d_6$, δ): 0.98 (3H, t, J=7.2Hz), 1.35-1.65 (6H, m), 1.67-2.05 (4H, m), 2.32-2.45 (1H, m), 2.54 (2H, q, J=7.2Hz), 2.90-3.50 (6H, m), 3.74-3.89 (1H, m), 4.45, 4.75 (1H, s), 4.86 (2H, s), 6.95 (2H, d, J=8.5Hz), 7.16-7.20 (1H, m), 7.31-7.35 (1H, m), 7.55 (2H, d, J=8.5Hz)

Example 320

(2S)-N-(2-Tetrahydropyranyloxy)-2-[5-(4-(cyclopropylaminocarbonylmethoxy)phenyl)-2-thienyl]-3,4,5,6-tetrahydro-2H-thiopyran-2-acetamide 1,1-dioxide (140 mg)

NMR (DMSO-$d_6$, δ): 0.45-0.50 (2H, m), 0.60-0.65 (2H, m), 1.35-1.65 (6H, m), 1.70-2.07 (4H, m), 2.35-2.45 (1H, m), 2.65-2.74 (1H, m), 2.90-3.50 (6H, m), 3.75-3.90 (1H, m), 4.47 (2H, s), 4.45, 4.75 (1H, s), 6.98 (2H, d, J=8.5Hz), 7.15-7.20 (1H, m), 7.30-7.35 (1H, m), 7.58 (2H, d, J=8.5Hz), 8.15 (1H, br), 11.20 (1H, s)

Example 321

(2S)-N-(2-Tetrahydropyranyloxy)-2-[5-(3-hydroxyphenyl)-2-thienyl]-3,4,5,6-tetrahydro-2H-thiopyran-2-acetamide 1,1-dioxide (205 mg)

NMR (DMSO-$d_6$, δ): 1.35-1.64 (6H, m), 1.66-2.05 (4H, m), 2.35-2.45 (1H, m), 2.90-3.50 (6H, m), 3.75-3.90 (1H, m), 4.45, 4.75 (1H, s), 6.70-6.73 (1H, m), 7.00-7.07 (2H, m), 7.16-7.21 (2H, m), 7.35-7.40 (1H, m), 9.61 (1H, s)

Example 322

(2S)-N-(2-Tetrahydropyranyloxy)-2-[5-(5-acetyl-2-thienyl)-2-thienyl]-3,4,5,6-tetrahydro-2H-thiopyran-2-acetamide 1,1-dioxide (100 mg)

NMR ($CDCl_3$, δ): 1.43-1.78 (6H, m), 1.85-1.98 (2H, m), 2.05-2.25 (2H, m), 2.58 (3H, s), 2.63-2.86 (2H, m), 2.95-3.17 (4H, m), 2.95-3.61 (1H, m), 3.65-3.82 (1H, m), 4.51, 4.83 (1H, s), 7.18 (1H, d, J=4.0Hz), 7.57 (1H, d, J=4.0Hz), 7.64 (1H, d, J=4.0Hz), 7.70 (1H, d, J=4.0Hz)

Example 323

(2S)-N-(2-Tetrahydropyranyloxy)-2-[5-(4-aminocarbonylphenyl)-2-thienyl]-3,4,5,6-tetrahydro-2H-thiopyran-2-acetamide 1,1-dioxide (49 mg) was obtained from (2S)-N-(2-tetrahydropyranyloxy)-2-[5-(4-carboxyphenyl)-2-thienyl]-3,4,5,6-tetrahydro-2H-thiopyran-2-acetamide 1,1-dioxide (133 mg) in a similar manner to that of Example 32.

NMR ($CDCl_3$, δ): 1.45 (2H, br), 1.63-1.68 (2H, m), 1.94 (2H, br), 2.08-2.17 (2H, m), 2.30-2.36 (2H, m), 3.00-3.22 (4H, m), 3.26-3.47 (1H, m), 3.68-3.76 (1H, m), 4.55 (1/2H, br), 4.85 (1/2H, br), 7.43-7.57 (5H, m), 7.63-7.70 (3H, m), 7.75-7.80 (1H, m)

MS (ESI+): 493.6 (M+H)

Example 324

(2S)-N-(2-Tetrahydropyranyloxy)-2-[5-(4-(benzylaminocarbonyl)phenyl)-2-thienyl]-3,4,5,6-tetrahydro-2H-thiopyran-2-acetamide 1,1-dioxide (85 mg) was obtained in a similar manner to that of Example 323.

NMR ($CDCl_3$, δ): 1.51-1.60 (2H, m), 1.70-1.79 (4H, m), 1.89-1.96 (2H, m), 2.08-2.18 (2H, m), 2.76-2.83 (2H, br), 2.97-3.14 (4H, m), 3.40-3.64 (1H, m), 3.72-3.92 (1H, m), 4.60-4.65 (2H, m), 4.68 (1/2H, br), 4.84 (1/2H, br), 7.20-7.37 (7H, m), 7.56-7.83 (4H, m)

MS (ESI-): 581.2 (M-H)

The following compounds were obtained in a similar manner to that of Example 125.

Example 325

(2S)-N-(2-Tetrahydropyranyloxy)-2-[5-(3-(ethoxycarbonylamino)phenyl)-2-thienyl]-3,4,5,6-tetrahydro-2H-thiopyran-2-acetamide 1,1-dioxide (190 mg)

NMR ($CDCl_3$, δ): 1.34 (1H, t, J=7.0Hz), 1.46 (2H, br), 1.64-1.69 (4H, m), 1.94 (2H, br), 2.07-2.20 (2H, m), 2.67-2.88 (2H, m) , 3.06 (2H, s), 3.11-3.16 (2H, m), 3.30-3.48 (1H, m), 3.60-3.70 (1H, m), 4.23 (2H, q, J=7.0Hz), 4.53 (1/2H, br), 4.81 (1/2H, br), 6.69 (1H, s), 7.27-7.32 (5H, m), 7.64 (1H, s), 8.05 (1/2H, s), 8.20 (1/2H, s)

MS (ESI-): 535.2 (M-H)

Example 326

(2S)-N-(2-Tetrahydropyranyloxy)-2-[5-(3-(n-propoxycarbonylamino)phenyl)-2-thienyl]-3,4,5,6-tetrahydro-2H-thiopyran-2-acetamide 1,1-dioxide (170 mg)

NMR ($CDCl_3$, δ): 0.99 (3H, t, J=6.0Hz), 1.45 (2H, br), 1.63-1.75 (6H, m), 1.95 (2H, br), 2.06-2.21 (2H, m), 2.63-2.91 (2H, m), 3.05 (2H, s), 3.12 (2H, br), 3.28-3.50 (1H, m), 3.58-3.69 (1H, m), 4.07-4.15 (2H, m), 4.52 (1/2H, br), 4.80 (1/2H, br), 6.65 (1H, s), 7.20-7.30 (5H, m), 7.66 (1H, s), 7.96 (1/2H, s), 8.12 (1/2H, s)

MS (ESI-): 549.4 (M-H)

Example 327

(2S)-N-(2-Tetrahydropyranyloxy)-2-[5-(3-(isopropoxycarbonylamino)phenyl)-2-thienyl]-3,4,5,6-tetrahydro-2H-thiopyran-2-acetamide 1,1-dioxide (160 mg)

NMR ($CDCl_3$, δ): 1.31 (6H, d, J=7.5Hz), 1.45 (2H, br), 1.64-1.68 (4H, m), 1.94 (2H, br), 2.05-2.21 (4H, m), 2.64-2.86 (2H, m), 3.05 (2H, s), 3.10-3.15 (2H, m), 3.30-3.38 (1H, m), 3.60-3.70 (1H, m), 4.52 (1/2H, br), 4.80 (1/2H, br), 5.02 (1H, qq, J=7.5, 7.5Hz), 6.59 (1H, s), 7.24-7.28 (5H, m), 7.67 (1H, s), 7.94 (1/2H, s), 8.10 (1/2H, s)

MS (ESI-): 549.4 (M-H)

Example 328

(2S)-N-(2-Tetrahydropyranyloxy)-2-[5-{3-(2-chloroethoxycarbonylamino)phenyl}-2-thienyl]-3,4,5,6-tetrahydro-2H-thiopyran-2-acetamide 1,1-dioxide (155 mg)

NMR ($CDCl_3$, δ): 1.45 (2H, br), 1.62-1.68 (4H, m), 1.95 (2H, br), 2.07-2.22 (2H, m), 2.63-2.89 (2H, m), 3.05 (2H, s), 3.10-3.16 (2H, m), 3.28-3.48 (1H, m), 3.61-3.66 (1H, m), 3.75 (2H, t, J=5.0Hz), 4.43 (2H, t, J=5.0Hz), 4.52 (1/2H, br), 4.80 (1/2H, br), 6.80 (1H, s), 7.23-7.31 (5H, m), 7.65 (1H, s), 8.02 (1/2H, s), 8.17 (1/2H, s)

MS (ESI-): 569.3 (M-H)

Example 329

(2S)-N-(2-Tetrahydropyranyloxy)-2-[5-(3-(valerylaminophenyl)-2-thienyl]-3,4,5,6-tetrahydro-2H-thiopyran-2-acetamide 1,1-dioxide (148 mg)

NMR ($CDCl_3$, δ): 0.96 (3H, t, J=7Hz), 1.32-1.78 (12H, m), 1.85-1.99 (2H, m), 2.03-2.25 (2H, m), 2.40 (2H, t, J=7Hz), 2.69-2.93 (2H, m), 2.98-3.18 (2H, m), 3.28-3.51 (1H, m), 3.62-3.77 (1H, m), 4.55, 4.83 (1H, s), 7.10-7.32 (4H, m), 7.47-7.69 (3H, m), 8.66 (1H, s)

MS (ESI-): 547 (M-H)

Example 330

(2S)-N-(2-Tetrahydropyranyloxy)-2-[5-(3-(ethylthiocarbonylamino)phenyl)-2-thienyl]-3,4,5,6-tetrahydro-2H-thiopyran-2-acetamide 1,1-dioxide (130 mg)

NMR ($CDCl_3$, δ): 1.26 (3H, t, J=8Hz), 1.39-1.77 (8H, m), 1.34-2.00 (2H, m), 2.06-2.75 (2H, m), 2.71-2.96 (2H, m), 2.99 (2H, q, J=8Hz), 3.04-3.17 (2H, m), 3.29-3.52 (2H, m), 3.64-3.76 (1H, m), 4.55, 4.84 (1H, s), 7.15-7.32 (4H, m), 7.38-7.54 (3H, m), 8.49, 8.58 (1H, s)

MS (ESI-): 551 (M-H)

Example 331

(2S)-N-(2-Tetrahydropyranyloxy)-2-[5-(3-(methylthiocarbonylamino)phenyl)-2-thienyl]-3,4,5,6-tetrahydro-2H-thiopyran-2-acetamide 1,1-dioxide (192 mg)

NMR ($CDCl_3$, δ): 1.36-1.78 (8H, m), 1.86-2.00 (2H, m), 2.07-2.75 (2H, m), 2.43, 2.45 (3H, s), 2.72-2.97 (2H, m), 3.03-3.22 (2H, m), 3.30-3.54 (1H, m), 3.64-3.76 (1H, m), 4.56, 4.74 (1H, br s), 7.12-7.32 (4H, m), 7.40-7.53 (2H, m), 7.62-7.71 (1H, m), 8.65, 8.72 (1H, s)

MS (ESI-): 537 (M-H)

Example 332

(2S)-N-(2-Tetrahydropyranyloxy)-2-[5-(3-(benzyloxycarbonylamino)phenyl)-2-thienyl)-3,4,5,6-tetrahydro-2H-thiopyran-2-acetamide 1,1-dioxide (87 mg)

NMR ($CDCl_3$, δ): 1.35-1.72 (8H, m), 1.86-1.98 (2H, m), 2.02-2.22 (2H, m), 2.65-2.92 (2H, m), 2.98-3.16 (2H, m), 3.28-3.50 (1H, m), 3.62-3.73 (1H, m), 4.52, 4.71 (1H, s), 5.21 (2H, s), 6.88 (1H, br s), 7.19-7.46 (10H, m), 7.62 (1H, br s), 8.26, 8.38 (1H, s)

MS (ESI-): 597 (M-H)

Example 333

(2S)-N-(2-Tetrahydropyranyloxy)-2-[5-(3-(2-(4-chlorophenyl)acetylamino)phenyl)-2-thienyl]-3,4,5,6-tetrahydro-2H-thiopyran-2-acetamide 1,1-dioxide (158 mg)

NMR ($CDCl_3$, δ): 1.35-1.79 (8H, m), 1.87-1.99 (2H, m), 2.04-2.24 (2H, m), 2.17-2.93 (1H, m), 3.02-3.17 (2H, m), 3.27-3.52 (1H, m), 3.63-3.74 (1H, m), 4.53, 4.82 (1H, s), 4.59 (2H, s), 6.83 (1H, d, J=8Hz), 6.96 (2H, d, J=8Hz), 7.22-7.39 (5H, m), 7.56-7.62 (1H, m), 7.22-7.28 (1H, m), 8.30 (1H, s), 8.34, 8.45 (1H, s)

MS (ESI+): 633, 634 (M+H)

Example 334

(2S)-N-(2-Tetrahydropyranyloxy)-2-[5-(3-(phenoxycarbonylamino)phenyl)-2-thienyl]-3,4,5,6-tetrahydro-2H-thiopyran-2-acetamide 1,1-dioxide (124 mg)

NMR ($CDCl_3$, δ): 1.34 (8H, m), 1.85-1.99 (2H, m), 2.03-2.24 (2H, m), 2.68-2.94 (2H, m), 3.00-3.20 (2H, m), 3.29-3.53 (1H, m), 3.62-3.77 (1H, m), 4.53, 4.82 (1H, s), 7.11-7.47 (11H, m), 7.63, 7.67 (1H, s), 8.49, 8.57 (1H, s)

MS (ESI-): 583 (M-H)

Example 335

(2S)-N-(2-Tetrahydropyranyloxy)-2-[5-(3-(2-(4-morpholinocarbonylamino)acetylamino)phenyl)-2-thienyl]-3,4,5,6-tetrahydro-2H-thiopyran-2-acetamide 1,1-dioxide (85 mg)

NMR (DMSO-$d_6$, δ): 1.32-2.09 (10H, m), 2.34-2.51 (1H, m), 2.87-3.62 (14H, m), 3.73-3.92 (1H, m), 3.80 (2H, d,

J=7Hz), 4.43, 4.76 (1H, s), 6.96 (1H, t, J=7Hz), 7.17-7.26 (1H, m), 7.32-7.53 (4H, m), 8.02 (1H, s)
MS (ESI-): 633 (M-H)

Example 336

(2S)-N-(2-Tetrahydropyranyloxy)-2-[5-(3-(2-(N-ethyl-N-methylaminocarbonyloxy)acetylamino)phenyl)-2-thienyl]-3,4,5,6-tetrahydro-2H-thiopyran-2-acetamide 1,1-dioxide (220 mg) was obtained in a similar manner to that of Example 211.

NMR ($CDCl_3$, δ): 1.18, 1.23 (3H, t, J=8Hz), 1.37-1.74 (8H, m), 1.89-2.01 (2H, m), 2.06-2.23 (2H, m), 2.72-2.88 (2H, m), 2.99, 3.03 (3H, s), 3.05-3.18 (2H, m), 3.15-3.28 (1H, m), 4.53, 4.83 (1H, s), 4.72, 4.74 (2H, s), 7.15-7.32 (4H, m), 7.52 (1H, br s), 7.59, 7.64 (1H, br s), 8.23 (1H, s), 8.62-8.75 (1H, m)
MS (ESI-): 606 (M-H)

The following compounds were obtained in a similar manner to that of Example 124.

Example 337

(2S)-N-(2-Tetrahydropyranyloxy)-2-[5-(3-(benzylaminocarbonylamino)phenyl)-2-thienyl]-3,4,5,6-tetrahydro-2H-thiopyran-2-acetamide 1,1-dioxide (80 mg)

NMR ($CDCl_3$, δ): 1.42 (2H, br), 1.60-1.66 (4H, m), 1.83-1.90 (2H, m), 2.00-2.18 (2H, m), 2.74 (2H, br), 2.92-3.00 (2H, m), 3.04-3.10 (2H, m), 3.30-. 3.50 (1H, m), 3.68-3.78 (1H, m), 4.33-4.42 (2H, m), 4.57 (1/2H, br), 4.72 (1/2H, br), 6.93-7.12 (6H, m), 7.27-7.25 (2H, m), 7.28-7.32 (4H, m)
MS (ESI-): 596.4 (M-H)

Example 338

(2S)-N-(2-Tetrahydropyranyloxy)-2-[5-(3-(4-methoxyphenylaminocarbonylamino)phenyl)-2-thienyl]-3,4,5,6-tetrahydro-2H-thiopyran-2-acetamide 1,1-dioxide (224 mg)

NMR ($CDCl_3$, δ): 1.43 (2H, br), 1.62-1.73 (4H, m), 1.90 (2H, br), 2.03-2.20 (2H, m), 2.77 (2H, br), 2.96-3.03 (2H, m), 3.08-3.14 (2H, m), 3.33-3.54 (1H, m), 3.68-3.75 (1H, m), 3.77 (3H, s), 4.61 (1/2H, br), 4.86 (1/2H, br), 6.80-6.93 (4H, m), 7.10-7.29 (6H, m), 7.38-7.53 (2H, m), 9.04 (1/2H, s), 9.42 (1/2H, s)
MS (ESI-): 611.5 (M-H)

Example 339

(2S)-N-(2-Tetrahydropyranyloxy)-2-[5-(3-(ethoxycarbonylmethylaminocarbonylamino)phenyl)-2-thienyl]-3,4,5,6-tetrahydro-2H-thiopyran-2-acetamide 1,1-dioxide (490 mg)

NMR ($CDCl_3$, δ): 1.22-1.33 (5H, m), 1.42-1.47 (2H, m), 1.62-1.70 (2H, m), 1.94 (2H, br), 2.07-2.20 (2H, m), 2.74-2.86 (2H, m), 3.08-3.20 (2H, m), 3.49-3.52 (2H, m), 3.68-3.78 (2H, m), 3.97-4.27 (4H, m), 4.58 (1/2H, br), 4.85 (1/2H, br), 5.68-5.80 (1H, m), 6.96-7.24 (5H, m), 7.28-7.34 (1H, m), 7.40-7.46 (1H, m), 9.12 (1/2H, s), 9.31 (1/2H, s)
MS (ESI-): 592.3 (M-H)

Example 340

(2S)-N-(2-Tetrahydropyranyloxy)-2-[5-(3-(butylaminocarbonylamino)phenyl)-2-thienyl]-3,4,5,6-tetrahydro-2H-thiopyran-2-acetamide 1,1-dioxide (330 mg)

NMR ($CDCl_3$, δ): 0.90 (3H, dd, J=7.2, 7.2Hz), 1.26-1.74 (10H, m), 1.85-1.99 (2H, m), 2.02-2.21 (2H, m), 2.74-2.89 (2H, m), 2.97-3.26 (6H, m), 3.31-3.54 (1H, m), 3.71-3.80 (1H, m), 4.60, 4.83 (1H, s), 5.42-5.53 (1H, m), 6.95 (1H, d, J=4Hz), 7.01-7.30 (5H, m), 7.38-7.43 (1H, m), 9.3, 9.51 (1H, s)

Example 341

(2S)-N-(2-Tetrahydropyranyloxy)-2-[5-(3-(1-naphtylaminocarbonylamino)phenyl)-2-thienyl]-3,4,5,6-tetrahydro-2H-thiopyran-2-acetamide 1,1-dioxide (350 mg)

NMR ($CDCl_3$, δ): 1.30-1.65 (6H, m), 1.75-1.92 (2H, m), 1.93-2.19 (2H, m), 2.70-2.88 (2H, m), 2.92-3.20 (4H, m), 3.30-3.46 (1H, m), 3.70 (1H, br), 4.56, 4.83 (1H, s), 6.93-7.80 (12H, m), 7.95 (1H, dd, J=8.0, 8.0Hz)

Example 342

(2S)-N-(2-Tetrahydropyranyloxy)-2-[5-(3-(allylaminocarbonylamino)phenyl)-2-thienyl]-3,4,5,6-tetrahydro-2H-thiopyran-2-acetamide 1,1-dioxide (300 mg)

NMR ($CDCl_3$, δ): 1.35-1.70 (6H, m), 1.85-2.25 (4H, m), 2.75-2.88 (2H, m), 2.95-3.17 (4H, m), 3.30-3.52 (1H, m), 3.70-3.90 (3H, m), 4.59, 4.83 (1H, s), 5.10-5.23 (2H, m), 5.40-5.49 (1H, m), 5.80-5.94 (1H, m), 6.95-7.28 (6H, m)

MS (ESI-): 546 (M-H)

Example 343

To a solution of (2S)-N-(2-tetrahydropyranyloxy)-2-[5-(3-aminophenyl)-2-thienyl]-3,4,5,6-tetrahydro-2H-thiopyran-2-acetamide 1,1-dioxide (150 mg) and thienylamine (163 mg) in N,N-dimethylformamide (3 ml) was added 3-(phenoxycarbonylamino)pyridine (83 mg) and the reaction mixture was stirred at ambient temperature for 14 hours. The mixture was poured into water and extracted with ethyl acetate. The organic layer was washed with water, saturated aqueous sodium hydrogen carbonate and brine, dried over anhydrous magnesium sulfate and concentrated in vacuo. The residue was purified by flash column chromatography on silica gel 60 (eluent: 3% methanol-chloroform) to give (2S)-N-(2-tetrahydropyranyloxy)-2-[5-(3-(3-pyridylaminocarbonylamino)phenyl)-2-thienyl]-3,4,5,6-tetrahydro-2H-thiopyran-2-acetamide 1,1-dioxide (150 mg) as a white amorphous.

NMR ($CDCl_3$, δ): 1.38 (2H, br), 1.45-1.62 (4H, m), 1.94 (4H, br), 2.05-2.25 (2H, m), 2.92 (2H, br), 3.02-3.11 (4H, m), 3.32-3.50 (1H, m), 3.78 (1H, br), 4.20-4.23 (1H, m), 4.62 (1/2H, br), 4.94 (1/2H, br), 6.89-7.27 (4H, m), 7.30-7.46 (1H, m), 7.51-7.72 (2H, m), 8.03-8.21 (1H, m), 8.40 (1/2H, s), 8.45 (1/2H, s)

MS (ESI+): 585.4 (M+H)

The following compounds were obtained in a similar manner to that of Example 125.

Example 344

(2S)-N-(2-Tetrahydropyranyloxy)-2-[5-(3-(allyloxycarbonylamino)phenyl)-2-thienyl]-3,4,5,6-tetrahydro-2H-thiopyran-2-acetamide 1,1-dioxide (160 mg)

NMR ($CDCl_3$, δ): 1.45 (2H, br), 1.58-1.68 (4H, m), 1.94 (2H, br), 2.07-2.22 (2H, m), 2.66-2.90 (2H, m), 3.04-3.52 (2H, m), 3.09-3.15 (2H, m), 3.30-3.49 (1H, m), 3.63-3.72 (1H, m), 4.52 (1/2H, br), 4.67 (2H, d, J=7.0Hz), 4.80 (1/2H, br), 5.27 (1H, d, J=8.0Hz), 5.38 (1H, d, J=8.0Hz), 5.89-6.04 (1H, m), 6.83 (1H, s), 7.24-7.34 (5H, m), 7.63 (1H, s), 8.20 (1/2H, s), 8.35 (1/2H, s)

MS (ESI-): 547.3 (M-H)

Example 345

(2S)-N-(2-Tetrahydropyranyloxy)-2-[5-(3-(2-benzyloxyacetylamino)phenyl)-2-thienyl]-3,4,5,6-tetrahydro-2H-thiopyran-2-acetamide 1,1-dioxide (410 mg)

NMR ($CDCl_3$, δ): 1.45 (2H, br), 1.61-1.68 (4H, m), 1.95 (2H, br), 2.06-2.22 (2H, m), 2.64-2.87 (2H, m), 3.03 (2H, br s), 3.08-3.13 (2H, m), 3.28-3.47 (1H, m), 3.61-3.67 (1H, m), 4.08 (2H, d, J=7.5Hz), 4.52 (1/2H, br), 4.63 (1H, s), 4.68 (1H, s), 4.67 (2H, d, J=15Hz), 4.82 (1/2H, br), 7.30-7.40 (9H, m), 7.53 (1H, d, J=5.0Hz), 7.77 (1H, s), 8.01 (1H, s), 8.16 (1H, s), 8.34 (1H, s)

MS (ESI-): 611.5 (M-H)

The following compounds were obtained in a similar manner to that of Example 206.

Example 346

(2S)-N-(2-Tetrahydropyranyloxy)-2-[5-(3-((2S)-2-(tert-butoxycarbonylamino)propionyl)amino)phenyl)-2-thienyl]-3,4,5,6-tetrahydro-2H-thiopyran-2-acetamide 1,1-dioxide (310 mg)

NMR ($CDCl_3$, δ): 1.43-1.48 (14H, m), 1.65-1.68 (4H, m), 1.94 (42H, br), 2.04-2.20 (2H, m), 2.67-2.87 (2H, m), 3.04-3.07 (2H, m), 3.10-3.15 (2H, m), 3.28-3.62 (1H, m), 3.63-3.75 (1H, m), 4.32 (1H, br), 4.52 (1/2H, br), 4.82 (1/2H, br), 7.20-7.30 (4H, m), 7.40-7.50 (1H, m), 7.67 (1/2H, s), 7.77 (1/2H, s), 8.36 (1/2H, s), 8.54 (1/2H, s)

MS (ESI-): 634.3 (M-H)

Example 347

(2S)-N-(2-Tetrahydropyranyloxy)-2-[5-{3-(((2R)-2-(tert-butoxycarbonylamino)propionyl)amino)phenyl}-2-thienyl]-3,4,5,6-tetrahydro-2H-thiopyran-2-acetamide 1,1-dioxide (475 mg)

NMR ($CDCl_3$, δ): 1.43-1.48 (14H, m), 1.65-1.68 (4H, m), 1.94 (42H, br), 2.04-2.20 (2H, m), 2.67-2.87 (2H, m), 3.04-3.07 (2H, m), 3.10-3.15 (2H, m), 3.28-3.62 (1H, m), 3.63-3.75 (1H, m), 4.32 (1H, br), 4.52 (1/2H, br), 4.82 (1/2H, br), 7.20-7.30 (4H, m), 7.40-7.50 (1H, m), 7.67 (1/2H, s), 7.77 (1/2H, s), 8.36 (1/2H, s), 8.54 (1/2H, s)

MS (ESI-): 634.3 (M-H)

Example 348

(2S)-N-(2-Tetrahydropyranyloxy)-2-[5-{3-(2-(4-methylphenoxy)acetylamino)phenyl}-2-thienyl]-3,4,5,6-tetrahydro-2H-thiopyran-2-acetamide 1,1-dioxide (230 mg)

NMR ($CDCl_3$, δ): 1.44 (2H, br), 1.56-1.68 (4H, m), 1.95 (2H, br), 2.07-2.21 (2H, m), 2.33 (3H, s), 2.63-2.90 (2H, m), 3.05 (2H, s), 3.11-3.13 (2H, br), 3.30-3.48 (1H, m), 3.60-3.70 (1H, m), 4.52 (1/2H, br), 4.60 (2H, s) , 4.80 (1/2H, br), 6.90 (2H, d, J=8.0Hz), 7.15 (2H, d, J=8.0Hz), 7.22-7.29 (2H, m), 7.33-7.36 (2H, m), 7.55-7.59 (1H, m), 7.80 (1H, s), 8.00 (1/2H, s), 8.14 (1/2H, s), 8.32 (1H, s)

MS (ESI-): 611.3 (M-H)

Example 349

(2S)-N-(2-Tetrahydropyranyloxy)-2-[5-{3-(2-(N,N-dimethylamino)acetylamino)phenyl}-2-thienyl]-3,4,5,6-tetrahydro-2H-thiopyran-2-acetamide 1,1-dioxide (90 mg)

NMR ($CDCl_3$, δ): 1.46 (2H, br), 1.62-1.68 (4H, m), 1.95 (4H, br), 2.04-2.23 (2H, m), 2.41 (6H, s), 2.65-2.88 (1H, m), 3.05 (2H, s), 3.10 (2H, m), 3.12-3.15 (2H, m), 3.26-3.49 (1H, m), 3.62-3.72 (1H, m), 4.52 (1/2H, br), 4.80 (1/2H, br), 7.23-7.29 (2H, m), 7.34 (2H, d, J=5.0Hz), 7.60-7.66 (1H, m), 7.76 (1H, br), 8.00 (1/2H, br), 8.18 (1/2H, br), 9.19 (1H, s)

MS (ESI+): 550.3 (M+H)

Example 350

(2S)-N-(2-Tetrahydropyranyloxy)-2-[5-(3-(2-(4-chlorophenyl)acetylamino)phenyl)-2-thienyl]-3,4,5,6-tetrahydro-2H-thiopyran-2-acetamide 1,1-dioxide (158 mg)

NMR ($CDCl_3$, δ): 1.34-1.98 (10H, m), 2.03-2.25 (2H, m), 2.69-2.88 (2H, m), 3.02-3.18 (2H, m), 3.27-3.51 (1H, m), 3.62-3.26 (1H, m), 3.68 (2H, s), 4.53, 4.83 (1H, br s), 7.00-7.66 (10H, m), 7.73, 7.76 (1H, s), 8.65-8.75 (1H, m)

MS (ESI-): 616 (M-H)

Example 351

(2S)-N-(2-Tetrahydropyranyloxy)-2-[5-(3-(((R)-2-tert-butoxycarbonylamino-2-phenylacetyl)amino)phenyl)-2-thienyl]-3,4,5,6-tetrahydro-2H-thiopyran-2-acetamide 1,1-dioxide (117 mg)

NMR (DMSO-$d_6$, δ): 1.20-1.62 (6H, m), 1.40 (9H, s), 1.68-2.06 (4H, m), 2.34-2.48 (1H, m), 2.86-3.32 (5H, m), 3.73-3.86 (1H, m), 4.40, 4.75 (1H, br s), 5.86 (1H, d, J=8Hz), 7.16-7.23 (1H, m), 7.28-7.61 (10H, m), 7.96 (1H, s), 10.37 (1H, s), 11.23 (1H, s)

MS (ESI-): 696 (M-H)

Example 352

(2S)-N-(2-Tetrahydropyranyloxy)-2-[5-(3-(2-isopropoxyacetylamino)phenyl)-2-thienyl]-3,4,5,6-tetrahydro-2H-thiopyran-2-acetamide 1,1-dioxide (276 mg)

NMR ($CDCl_3$, δ): 1.29 (6H, d, J=7Hz), 1.36-1.77 (8H, m), 1.88-2.00 (2H, m), 2.65-2.92 (2H, m), 3.02-3.18 (2H, m), 3.28-3.51 (1H, m), 3.62-3.73 (1H, m), 3.77 (1H, q, J=7Hz), 4.07 (1H, s), 4.52, 4.82 (1H, br s), 7.23-7.48 (4H, m), 7.52-7.60 (1H, m), 7.78 (1H, s), 8.18, 8.32 (1H, br s), 8.40 (1H, s)

MS (ESI+): 565 (M+H)

Example 353

(2S)-N-(2-Tetrahydropyranyloxy)-2-[5-(3-((1-tert-butoxycarbonyl-4-piperidinyloxy)acetylamino)phenyl)-2-thienyl]-3,4,5,6-tetrahydro-2H-thiopyran-2-acetamide 1,1-dioxide (360 mg)

NMR ($CDCl_3$, δ): 1.40-1.77 (10H, m), 1.47 (9H, s), 1.89-2.01 (4H, m), 2.06-2.24 (2H, m), 2.68-2.92 (2H, m), 3.02-3.18 (4H, m), 3.28-3.52 (1H, m), 3.58-3.74 (2H, m), 3.79-3.92 (2H, m), 4.12 (2H, m), 4.53, 4.82 (1H, s), 7.22-7.38 (4H, m), 7.52-7.58 (1H, m), 7.76 (1H, s), 8.27, 8.84 (1H, s), 8.34, 8.38 (1H, s)

MS (ESI+): 706 (M+H)

Example 354

(2S)-N-(2-Tetrahydropyranyloxy)-2-[5-(3-(2-(2-oxo-1,3-oxazolidin-3-yl)acetylamino)phenyl)-2-thienyl]-3,4,5,6-tetrahydro-2H-thiopyran-2-acetamide 1,1-dioxide (600 mg)

NMR (DMSO-$d_6$, δ): 1.36-1.64 (6H, m), 1.69-2.06 (4H, m), 2.34-2.48 (1H, m), 2.88-3.32 (5H, m), 3.42-3.53 (1H, m), 3.66 (2H, t, J=8Hz), 3.73-3.90 (1H, m), 4.34 (2H, t, J=8Hz), 4.44, 4.75 (1H, s), 7.17-7.25 (1H, m), 7.33-7.48 (4H, m), 8.03 (1H, s)

MS (ESI-): 590 (M-H)

Example 355

To a suspension of (2S)-N-(2-tetrahydropyranyloxy)-2-[5-(3-aminophenyl)-2-thienyl]-3,4,5,6-tetrahydro-2H-thiopyran-2-acetamide 1,1-dioxide (300 mg) and triethylamine (392 mg) in chloroform (4 ml) was added triphosgene (192 mg) at 0°C and the reaction mixture was stirred at ambient temperature for 30 minutes. To the mixture was added (2-((tert-butyl)(diphenyl)sillyloxy)ethyl)amine (232 mg) at 0°C and the reaction mixture was stirred at ambient temperature for 2 hours. The mixture was poured into water and was extracted with chloroform. The organic layer was washed with water, 10% aqueous citric acid solution, saturated aqueous sodium hydrogen carbonate and brine, dried over anhydrous magnesium sulfate and concentrated in vacuo. The residue was purified by flash column chromatography on silica gel 60 (eluent: 2% methanol-chloroform) to give (2S)-N-(2-tetrahydropyranyloxy)-2-[5-(3-(2-((tert-butyl) (diphenyl)sillyloxy)ethylaminocarbonylamino)phenyl)-2-thienyl]-3,4,5,6-tetrahydro-2H-thiopyran-2-acetamide 1,1-dioxide (350 mg) as a white amorphous.

NMR ($CDCl_3$, δ): 1.04 (9H, s), 1.44 (2H, br), 1.62-1.68 (4H, m), 1.85-1.94 (2H, br), 2.05-2.19 (2H, m), 2.69-2.90 (2H, m), 2.98-3.10 (4H, m), 3.31-3.44 (3H, m), 3.62-3.80 (3H, m), 4.56 (1/2H, br), 4.80 (1/2H, br), 6..64 (1H, s), 7.09-7.30 (5H, m), 7.32-7.42 (7H, m), 7.56-7.64 (4H, m), 8.62 (1H, s)

MS (ESI-): 788.5 (M-H)

The following compounds were obtained in a similar manner to that of Example 125.

Example 356

(2S)-N-(2-Tetrahydropyranyloxy)-2-[5-(3-(cyclopentylcarbonylamino)phenyl)-2-thienyl]-3,4,5,6-tetrahydro-2H-thiopyran-2-acetamide 1,1-dioxide (205 mg)

NMR ($CDCl_3$, δ): 1.46 (2H, br), 1.60-1.70 (6H, m), 1.77-1.84 (2H, m), 1.91-1.97 (6H, m), 2.07-2.21 (2H, m), 2.66-2.89 (2H, m), 2.71 (1H, tt, J=7.5, 7.5Hz), 3.05 (1H, s), 3.10-3.15 (2H, m), 3.30-3.49 (1H, m), 3.63-3.70 (1H, m), 4.54 (1/2H, br), 4.82 (1/2H, br), 7.20-7.30 (4H, m), 7.41 (2H, br), 7.52 (1H, br), 7.71 (1H, br), 8.25 (1/2H, s), 8.37 (1/2H, s)

MS (ESI-): 559.4 (M-H)

Example 357

(2S)-N-(2-Tetrahydropyranyloxy)-2-[5-(3-(3-chloropropionylamino)phenyl)-2-thienyl]-3,4,5,6-tetrahydro-2H-thiopyran-2-acetamide 1,1-dioxide (145 mg)

NMR ($CDCl_3$, δ): 1.45 (2H, br), 1.54-1.72 (4H, m), 1.95 (4H, br), 2.09-2.24 (2H, m), 2.72-2.82 (2H, m), 2.85 (2H, t, J=6.4Hz), 3.00-3.08 (2H, m), 3.11-3.16 (2H, m), 3.29-3.52 (1H, m), 3.63-3.73 (1H, m), 3.91 (2H, t, J=6.5Hz), 4.54 (1/2H, br), 4.83 (1/2H, br), 7.15-7.30 (4H, m), 7.54-7.72 (3H, m), 8.50 (1H, s)

MS (ESI-): 553.3 (M-H)

The following compounds were obtained in a similar manner to that of Example 355.

Example 358

(2S)-N-(2-Tetrahydropyranyloxy)-2-[5-(3-(isobutylaminocarbonylamino)phenyl)-2-thienyl]-3,4,5,6-tetrahydro-2H-thiopyran-2-acetamide 1,1-dioxide (230 mg)

NMR ($CDCl_3$, δ): 0.90 (6H, d, J=6.6Hz), 1.40-1.70 (7H, m), 1.88-1.97 (2H, m), 2.05-2.22 (2H, m), 2.99-3.18 (6H, m), 3.31-3.51 (1H, m), 6.96-7.29 (6H, m), 7.47-7.53 (1H, m), 9.25, 9.47 (1H, s)

MS (ESI-): 562 (M-H)

Example 359

(2S)-N-(2-Tetrahydropyranyloxy)-2-[5-(3-(cyclohexylmethylaminocarbonylamino)phenyl)-2-thienyl]-3,4,5,6-tetrahydro-2H-thiopyran-2-acetamide 1,1-dioxide (200 mg)

NMR ($CDCl_3$, δ): 0.83-1.00 (2H, m), 1.10-1.25 (4H, m), 1.35-1.54 (5H, m), 1.55-1.79 (6H, m), 1.85-1.98 (2H, m), 2.01-2.22 (2H, m), 2.75-2.89 (2H, m), 3.00-3.22 (6H, m), 3.30-3.52 (1H, m), 3.66-3.82 (1H, m), 1.59, 4.84 (1H, s), 5.45-5.55 (1H, m), 6.95-7.30 (6H, m), 7.40 (1H, br)

Example 360

(2S)-N-(2-Tetrahydropyranyloxy)-2-[5-(3-(2-methoxyethylaminocarbonylamino)phenyl)-2-thienyl]-3,4,5,6-tetrahydro-2H-thiopyran-2-acetamide 1,1-dioxide (220 mg)

NMR ($CDCl_3$, δ): 1.38-1.68 (6H, m), 1.85-1.98 (2H, m),

2.03-2.18 (2H, m), 2.75-2.90 (2H, m), 3.01-3.17 (4H, m), 3.30-3.35 (1H, m), 3.38 (3H, s), 3.42-3.55 (4H, m), 3.66-3.77 (1H, m), 4.55, 4.84 (1H, s), 5.51-5.60 (1H, m), 7.05-7.40 (6H, m), 9.10, 9.20 (1H, s)

MS (ESI-):564 (M-H

Example 361

(2S)-N-(2-Tetrahydropyranyloxy)-2-[5-(3-((N-methyl-N-ethylaminocarbonyl)amino)phenyl)-2-thienyl]-3,4,5,6-tetrahydro-2H-thiopyran-2-acetamide 1,1-dioxide (200 mg)

NMR ($CDCl_3$, δ): 1.33 (3H, t, J=7.0Hz), 1.40-1.75 (6H, m), 1.85-1.98 (2H, m), 2.05-2.20 (2H, m), 2.60-2.90 (4H, m), 3.00-3.20 (5H, m), 3.29-3.50 (1H, m), 3.62-3.73 (1H, m), 4.23 (2H, q, J=7.0Hz), 4.53, 4.80 (1H, s), 7.15-7.35 (6H, m)

MS (ESI-): 548 (M-H)

Example 362

(2S)-N-(2-Tetrahydropyranyloxy)-2-[5-(3-((2-phthalimidoethoxy)carbonylamino)phenyl)-2-thienyl]-3,4,5,6-tetrahydro-2H-thiopyran-2-acetamide 1,1-dioxide (155 mg) from (2S)-N-(2-tetrahydropyranyloxy)-2-[5-(3-aminophenyl)-2-thienyl]-3,4,5,6-tetrahydro-2H-thiopyran-2-acetamide 1,1-dioxide (300 mg) and 2-phthaloylethanol

NMR ($CDCl_3$, δ): 1.42-1.70 (6H, m), 1.81-1.96 (2H, m), 2.02-2.23 (2H, m), 2.87-3.27 (4H, m), 3.49 (2H, br), 3.52-3.75 (2H, m), 4.04 (2H, br), 4.16-4.48 (1H, m), 5.05-5.23 (1H, m), 6.76-7.36 (8H, m), 7.66-7.74 (2H, m), 7.79-7.87 (2H, m)

MS (ESI-): 680.5 (M-H)

The following compounds were obtained in a similar manner to that of Example 31.

Example 363

(2S)-N-(2-Tetrahydropyranyloxy)-2-[5-(4-(2-phenylaminocarbonylethenyl)phenyl)-2-thienyl]-3,4,5,6-tetrahydro-2H-thiopyran-2-acetamide 1,1-dioxide (100 mg)

NMR (DMSO-$d_6$, δ): 1.38-1.62 (6H, m), 1.72-2.06 (4H, m), 2.38-2.47 (1H, m), 2.90-3.52 (6H, m), 3.75-3.90 (1H, m), 4.45, 4.75 (1H, s), 6.86 (1H, d, J=16Hz), 7.06 (1H, dd, J=8.0, 8.0Hz), 7.24-7.27 (1H, m), 7.34 (2H, d, J=8.0, 8.0Hz), 7.55-7.58 (2H,. m), 7.65-7.76 (6H, m)

Example 364

(2S)-N-(2-Tetrahydropyranyloxy)-2-[5-(4-(2-(4-methoxyphenylaminocarbonyl)ethenyl)phenyl)-2-thienyl]-3,4,5,6-tetrahydro-2H-thiopyran-2-acetamide 1,1-dioxide (150 mg)

NMR (DMSO-$d_6$, δ): 1.37-1.64 (6H, m); 1.71-2.08 (4H, m), 2.36-2.47 (1H, m), 2.90-3.51 (6H, m), 3.74 (3H, s), 3.75-3.90 (1H, m), 4.45, 4.75 (1H, s), 6.82 (1H, d, J=16Hz), 6.92 (2H, d, J=8.5Hz), 7.24-7.27 (1H, m), 7.54-7.75 (8H, m)

Example 365

(2S)-N-(2-Tetrahydropyranyloxy)-2-[5-(4-(2-(4-fluorophenylaminocarbonyl)ethenyl)phenyl)-2-thienyl]-3,4,5,6-tetrahydro-2H-thiopyran-2-acetamide 1,1-dioxide (150 mg)

NMR (DMSO-$d_6$, δ): 1.37-1.62 (6H, m), 1.70-2.08 (4H, m), 2.36-2.46 (1H, m), 2.91-3.54 (6H, m), 3.75-3.90 (1H, m), 4.45, 4.75 (1H, s), 6.83 (1H, d, J=16Hz), 7.18 (2H, dd, J=8.5, 8.5Hz), 7.24-7.27 (1H, m), 7.55-7.75 (8H, m), 10.3 (1H, s)

Example 366

(2S)-N-(2-Tetrahydropyranyloxy)-2-[5-(4-(2-(N,N-dimethylaminocarbonyl)ethenyl)phenyl)-2-thienyl]-3,4,5,6-tetrahydro-2H-thiopyran-2-acetamide 1,1-dioxide (250 mg)

NMR ($CDCl_3$, δ): 1.35-1.75 (6H, m), 1.88-2.01 (2H, m), 2.05-2.25 (2H, m), 2.65-3.18 (9H, m), 3.20 (3H, s), 3.26-3.49 (1H, m), 3.64-3.70 (1H, m), 4.54, 4.83 (1H, s), 6.91 (1H, d, J=16Hz), 7.29 (1H, d, J=4.0Hz), 7.47-7.69 (6H, m)

Example 367

(2S)-N-(2-Tetrahydropyranyloxy)-2-[5-(4-(2-(isopropylaminocarbonyl)ethenyl)phenyl)-2-thienyl]-3,4,5,6-tetrahydro-2H-thiopyran-2-acetamide 1,1-dioxide (220 mg)

NMR (DMSO-$d_6$, δ): 1.11 (6H, d, J=5Hz), 1.37-1.64 (6H, m), 1.70-2.07 (4H, m), 2.35-2.46 (1H, m), 2.90-3.37 (5H, m), 3.40-3.54 (1H, m), 3.74-3.90 (1H, m), 3.90-4.01 (1H, m), 4.45, 4.75 (1H, s), 6.62 (1H, d, J=16Hz), 7.21-7.25 (1H, m), 7.40 (1H, d, J=16Hz), 7.51-7.55 (1H, m), 7.57 (2H, d, J=8.5Hz), 7.69 (2H, d, J=8.5Hz), 7.99 (1H, d, J=7.5Hz)

Example 368

(2S)-N-(2-Tetrahydropyranyloxy)-2-[5-(4-(2-(propylaminocarbonyl)ethenyl)phenyl)-2-thienyl]-3,4,5,6-tetrahydro-2H-thiopyran-2-acetamide 1,1-dioxide (210 mg)

NMR (DMSO-$d_6$, δ): 0.88 (3H, t, J=7.5Hz), 1.36-1.62 (8H, m), 1.70-2.06 (4H, m), 2.35-2.45 (1H, m), 2.40-3.30 (7H, m), 3.41-3.52 (1H, m), 3.74-3.90 (1H, m), 4.45, 4.75 (1H, s), 6.65 (1H, d, J=16Hz), 7.22-7.25 (1H, m), 7.40 (1H, d, J=16Hz), 7.51-7.55 (1H, m), 7.60 (2H, d, J=8.5Hz), 7.70 (2H, d, J=8.5Hz), 8.10 (1H, t, J=7.0Hz), 11.2 (1H, s)

Example 369

(2S)-N-(2-Tetrahydropyranyloxy)-2-[5-(4-(n-propylaminocarbonylmethoxy)phenyl)-2-thienyl]-3,4,5,6-tetrahydro-2H-thiopyran-2-acetamide 1,1-dioxide (140 mg)

NMR (DMSO-$d_3$, δ): 0.82 (3H, t, J=7.5Hz), 1.36-1.63 (8H, m), 1.70-2.05 (4H, m), 2.33-2.44 (1H, m), 2.93-3.50. (8H, m), 3.74-3.91 (1H, m), 4.45, 4.75 (1H, s), 4.50 (2H, s), 7.01 (2H, d, J=8.5Hz), 7.16-7.20 (1H, m), 7.33-7.37 (1H, m), 7.58 (2H, d, J=8.5Hz), 8.10 (1H, br)

Example 370

To a suspension of (2S)-N-(2-tetrahydropyranyloxy)-2-[5-(3-((2-phthalimidoethoxy)carbonylamino)phenyl)-2-thienyl]-3,4,5,6-tetrahydro-2H-thiopyran-2-acetamide 1,1-dioxide (155 mg) in methanol (4 ml) was added hydrazine monohydrate (13.7 mg) and the reaction mixture was stirred at ambient temperature for 3 hours, the resulting mixture was filtrated and washed with methanol. The filtrate was concentrated in vacuo to give (2S)-N-(2-tetrahydropyranyloxy)-2-[5-(3-(2-aminoethylaminocarbonylamino)phenyl)-2-thienyl]-3,4,5,6-tetrahydro-2H-thiopyran-2-acetamide 1,1-dioxide (125 mg) as a white amorphous.

NMR ($CDCl_3$, δ): 1.24-1.35 (2H, m), 1.42-1.52 (2H, m), 1.62-1.73 (2H, m), 1.96 (2H, br), 2.08-2.25 (2H, m), 2.86 (2H, br), 3.01-3.05 (2H, m), 3.15 (2H, br), 3.44-3.50 (4H, m), 3.74-3.84 (1H, m), 4.22-4..25 (1H, m), 4.45 (1/2H, br), 4.83 (1/2H, br), 7.22-7.30 (4H, m), 7.37-7.44 (1H, m), 7.68 (1H, br s), 7.83-7.86 (1H, m), 8.20-8.24 (1H, m)

MS (ESI+): 552.3 (M+H)

The following compounds were obtained in a similar manner to that of Example 125.

Example 371

(2S)-N-(2-Tetrahydropyranyloxy)-2-[5-(4-(ethylcarbonyloxy)phenyl)-2-thienyl]-3,4,5,6-tetrahydro-2H-thiopyran-2-acetamide 1,1-dioxide (150 mg)

NMR (DMSO-$d_6$, δ): 1.15 (3H, dd, J=7.5, 7.5Hz), 1.38-1.61 (6H, m), 1.70-2.07 (4H, m), 2.37-2.46 (1H, m), 2.58-2.66 (2H, m), 2.90-3.51 (6H, m), 3.75-3.88 (1H, m), 4.43, 4.75 (1H, s), 7.18-7.25 (3H, m), 7.43-7.48 (1H, m), 7.68 (2H, d, J=8.5Hz)

MS (ESI-): 520 (M-H)

Example 372

(2S)-N-(2-Tetrahydropyranyloxy)-2-[5-(4-(methoxyacetoxy)phenyl)-2-thienyl]-3,4,5,6-tetrahydro-2H-thiopyran-2-acetamide 1,1-dioxide (150 mg)

NMR (DMSO-$d_6$, δ): 1.36-1.62 (6H, m), 1.69-2.05 (4H, m), 2.34-2.43 (1H, m), 2.87-3.29 (5H, m), 3.38 (3H, s), 3.40-3.51 (1H, m), 3.75-3.90 (1H, m), 4.37 (2H, s), 4.44, 4.75 (1H, s), 7.20-7.26 (3H, m), 7.42-7.48 (1H, m), 7.70 (2H, d, J=8.5Hz)

MS (ESI-): 536 (M-H)

Example 373

(2S)-N-(2-Tetrahydropyranyloxy)-2-[5-(4-(ethoxycarbonyloxyphenyl)phenyl)-2-thienyl]-3,4,5,6-tetrahydro-2H-thiopyran-2-acetamide 1,1-dioxide (150 mg)

NMR (DMSO-$d_6$, δ): 1.30 (3H, dd, J=7.5, 7.5Hz), 1.38-1.65 (6H, m), 1.70-1.95 (4H, m), 2.36-2.45 (1H, m), 2.90-3.51 (6H, m), 3.72-3.90 (1H, m), 4.27 (2H, ddd, J=7.5, 7.5, 7.5Hz), 7.43, 4.75 (1H, s), 7.20-7.26 (1H, m), 7.29 (2H, d, J=8.5Hz), 7.45-7.49 (1H, m), 7.69 (2H, d, J=8.5Hz)

MS (ESI-): 536 (M-H)

The following compounds were obtained in a similar manner to that of Example 124.

Example 374

(2S)-N-(2-Tetrahydropyranyloxy)-2-[5-(4-(methylaminocarbonyloxy)phenyl)-2-thienyl]-3,4,5,6-tetrahydro-2H-thiopyran-2-acetamide 1,1-dioxide (250 mg)

NMR (DMSO-$d_6$, δ): 1.37-1.62 (4H, m), 1.70-2.05 (4H, m), 2.35-2.46 (1H, m), 2.67 (3H, d, J=4.5Hz), 2.90-3.30 (5H, m), 3.40-3.55 (1H, m), 3.75-3.88 (1H, m), 4.44, 4.75 (1H, s), 7.15 (2H, d, J=8.7Hz), 7.18-7.24 (1H, m), 740-7.45 (1H, m), 7.63 (2H, d, J=8.7Hz)

MS (ESI-): 521 (M-H)

Example 375

(2S)-N-(2-Tetrahydropyranyloxy)-2-[5-(4-(ethylaminocarbonyloxy)phenyl)-2-thienyl]-3,4,5,6-tetrahydro-2H-thiopyran-2-acetamide 1,1-dioxide (230 mg)

NMR ($CDCl_3$, δ): 1.23 (3H, dd, J=7.2, 7.2Hz), 1.36-1.75 (6H, m), 1.86-2.00 (2H, m), 2.03-2.25 (2H, m), 2.65-2.87 (2H, m), 3.01-3.18 (4H, m), 3.26-3.37 (2H, m), 3.60-3.69 (1H, m), 4.51, 4.80 (1H, s), 5.01-5.09 (1H, m), 7.14 (2H, d, J=8.7Hz), 7.16-7.25 (2H, m), 7.54 (2H, d, J=8.7Hz), 8.09, 8.24 (1H, s)

MS (ESI-): 535 (M-H)

Example 376

(2S)-N-(2-Tetrahydropyranyloxy)-2-[5-(4-hydroxyphenyl)-2-thienyl]-3,4,5,6-tetrahydro-2H-thiopyran-2-acetamide 1,1-dioxide (1 g) was obtained in a similar manner to that of Example 196.

NMR ($CDCl_3$, δ): 1.38-1.66 (6H, m), 1.70-2.04 (4H, m), 1.84-1.94 (1H, m), 2.89-3.48 (6H, m), 3.75-3.90 (1H, m), 4.45, 4.75 (1H, s), 8.80 (2H, d, J=8.7Hz), 7.13-7.17 (1H, m), 7.21-7.25 (1H, m), 7.45 (2H, d, J=8.7Hz), 9.68 (1H, s)

The following compounds were obtained in a similar manner to that of Example 196.

Example 377

(2S)-N-(2-Tetrahydropyranyloxy)-2-[5-(6-methyl-3-pyridyl)-2-thienyl]-3,4,5,6-tetrahydro-2H-thiopyran-2-acetamide 1,1-dioxide (99 mg)

NMR ($CDCl_3$, δ): 1.40-1.75 (6H, m), 1.90-2.00 (2H, m), 2.07-2.25 (2H, m), 2.59 (3H; s), 2.70-2.94 (2H, m), 3.05-3.17 (4H, m), 3.30-3.52 (1H, m), 3.63-3.74 (1H, m), 4.52 (0.5H, s), 4.81 (0.5H, s), 7.17 (2H, d, J=8Hz), 7.26-7.33 (2H, m), 7.75 (1H, dd, J=1.5, 8Hz), 8.12 (0.5H, s), 8.24 (0.5, s), 8.74 (1H, d, J=1.5Hz)

MS (ESI-): 463 (M-H)

Example 378

(2S)-N-(2-Tetrahydropyranyloxy)-2-[5-(6-methyl-3-pyridyl)-2-thienyl]-3,4,5,6-tetrahydro-2H-thiopyran-2-acetamide 1,1-dioxide (110 mg)

NMR ($CDCl_3$, δ): 1.40-1.74 (6H, m), 1.88-2.00 (2H, m), 2.07-2.20 (2H, m), 2.67-2.87 (2H, m), 3.04-3.08 (2H, m), 3.09-3.18 (2H, m), 3.30-3.52 (1H, m), 3.60-3.73 (1H, m), 3.96 (3H, s), 4.53 (0.5H, s), 4.81 (0.5H, s), 6.77 (1H, d, J=8Hz), 6.83 (1H, d, J=8Hz), 7.16-7.20 (1H, m), 7.64-7.79 (1H, m), 7.99 (0.5H, s), 8.13 (0.5H, s), 8.39-8.42 (1H, m)

MS (ESI-): 479 (M-H)

The following compounds were obtained in a similar manner to that of Example 125.

Example 379

(2S)-N-(2-Tetrahydropyranyloxy)-2-[5-{3-(2-acetylamino) phenyl}-2-thienyl]-3,4,5,6-tetrahydro-2H-thiopyran-2-acetamide 1,1-dioxide (105 mg)

NMR ($CDCl_3$, δ): 1.33-1.77 (8H, m), 1.87-2.00 (2H, m), 2.11 (3H, m), 2.06-2.77 (2H, m), 2.85-3.36 (5H, m), 3.66-3.83 (2H, m), 4.36-4.48 (1H, m), 4.53 (1/2H, br), 4.88 (1/2H, br), 7.12-7.37 (5H, m), 7.59-7.67 (1H, m), 8.82 (1/2H, br s), 8.84 (1/2H, br s), 9.83 (1H, s), 10.04 (1H, s)

MS (ESI-): 563.0 (M-H)

Example 380

(2S)-N-(2-Tetrahydropyranyloxy)-2-[5-(3-(3-(methoxycarbonyl)propionylamino)phenyl)-2-thienyl]-3,4,5,6-tetrahydro-2H-thiopyran-2-acetamide 1,1-dioxide (130 mg)

NMR ($CDCl_3$, δ) : 1.44 (4H, br), 1.65-1.67 (2H, m), 1.95 (2H, br), 2.08-2.21 (2H, m), 2.67-2.77 (6H, m), 3.06-3.14 (4H, m), 3.29-3.48 (1H, m), 3.62-3.70 (1H, m), 3.72 (3H, s), 4.53 (1/2H, br), 4.81 (1/2H, br), 7.20-7.27 (3H, m), 7.51 (1H, br), 7.60 (1/2H, s), 7.66 (1/2H, s), 7.80 (1/2H, br), 7.84 (1/2H, br), 8.40 (1/2H, s), 8.44 (1/2H, s)

MS (ESI-): 577.2 (M-H)

Example 381

(2S)-N-(2-Tetrahydropyranyloxy)-2-[5-{3-(2-(N-methoxycarbonyl-N-methylamino)acetylamino)phenyl}-2-thienyl]-3,4,5,6-tetrahydro-2H-thiopyran-2-acetamide 1,1-dioxide (160 mg)

NMR ($CDCl_3$, δ): 1.46 (2H, br), 1.68 (4H, br), 1.96 (2H, br), 2.07-2.24 (2H, m), 2.70-2.94 (2H, m), 3.02-3.09 (2H, m), 3.10 (3H, s), 3.16 (2H, br), 3.31-3.53 (1H, m), 3.65-3.75 (1H, m), 3.80 (3H, s), 4.02-4.09 (2H, m), 4.54 (1/2H, br), 4.83 (1/2H, br), 7.24-7.31 (5H, m), 7.53 (1H, br), 7.63 (1/2H, s), 7.67 (1/2H, s), 8.44 (1/2H, s), 8.50 (1/2H, s)

MS (ESI-): 592.1 (M-H)

Example 382

(2S)-N-(2-Tetrahydropyranyloxy)-2-[5-[4-(3-pyridyl-2-propenyloxy)phenyl)-2-thienyl]-3,4,5,6-tetrahydro-2H-thiopyran-2-acetamide 1,1-dioxide (270 mg)

NMR ($DMSOd_6$, δ): 1.36-1.65 (6H, m), 1.70-2.06 (4H, m), 2.36-2.47 (1H, m), 2.91-3.28 (5H, m), 3.41-3.52 (1H, m), 3.75-3.91 (1H, m), 4.45, 4.75 (1H, s), 7.07 (1H, d, J=16Hz), 7.21-7.26 (1H, m), 7.30 (2H, d, J=8.0Hz), 7.45-7.51 (2H, m), 7.72 (2H, d, J=8.0Hz), 7.94 (1H, d, J=16Hz), 8.28-8.32 (1H, m), 8.60-8.62 (1H, m), 8.99 (1H, s)

MS (ESI-): 595 (M-H)

The following compounds were obtained in a similar manner to that of Example 206.

Example 383

(2S)-N-(2-Tetrahydropyranyloxy)-2-[5-(3-(2-(2-methoxyethoxy)acetylamino)phenyl}-2-thienyl]-3,4,5,6-tetrahydro-2H-thiopyran-2-acetamide 1,1-dioxide (175 mg)

NMR ($CDCl_3$, δ): 1.35-1.74 (8H, m), 1.89-1.99 (2H, m), 2.06-2.25 (2H, m), 2.68-2.89 (2H, m), 3.02-3.17 (2H, m), 3.25-3.46 (1H, m), 3.52 (3H, s), 3.58-3.67 (2H, m), 3.74-3.81 (2H, m), 4.13 (2H, s), 4.52 (1/2H, br), 4.79 (1/2H, br), 7.22-7.38 (4H, m), 7.55-7.62 (1H, m), 7.83 (1H, br s), 8.04 (1/2H, s), 8.19 (1/2H, s), 8.97 (1H, s)

MS (ESI-): 579.9 (M-H)

Example 384

(2S)-N-(2-Tetrahydropyranyloxy)-2-[5-(3-(2-(N-9-fluorenylmethoxycarbonyl-N-methylamino)acetylamino) phenyl}-2-thienyl]-3,4,5,6-tetrahydro-2H-thiopyran-2-acetamide 1,1-dioxide (1.77 g)

NMR ($CDCl_3$, δ): 1.42 (2H, br), 1.64 (4H, br), 1.95 (2H, br), 2.08-2.23 (2H, m), 2.70-2.90 (2H, m), 3.00-3.07 (5H, m), 3.10-3.16 (2H, m), 3.27-3.48 (1H, m), 3.60-3.70 (1H, m), 4.00-4.09 (1H, m), 4.28 (1H, br), 4.52 (2H, s), 4.56 (1/2H, br), 4.80 (1/2H, br), 7.21-7.48 (9H, m), 7.58-7.65 (3H, m), 7.75 (2H, br), 8.20 (1/2H, s), 8.32 (1/2H, s)

MS (ESI-): 791.9 (M-H+Cl)

Example 385

(2S)-N-(2-Tetrahydropyranyloxy)-2-[5-{3-(((2S)-2,6-bis(tert-butoxycarbonylamino)hexanoyl)amino)phenyl}-2-thienyl]-3,4,5,6-tetrahydro-2H-thiopyran-2-acetamide 1,1-dioxide (290 mg)

NMR ($CDCl_3$, δ): 1.45 (18H, s), 1.48-1.70 (14H, m), 1.95 (2H, br), 2.09-2.20 (2H, m), 2.68-2.92 (2H, m), 3.00-3.15 (4H, m), 3.25-3.49 (1H, m), 3.63-3.74 (1H, m), 4.16-4.28 (1H, m), 4.52 (1/2H, br), 4.65 (1/2H, br), 7.22-7.24 (2H, m), 7.27-7.30 (2H, m), 7.51 (2H, br), 7.62-7.82 (1H, m)

MS (ESI-): 791.3 (M-H)

Example 386

(2S)-N-(2-Tetrahydropyranyloxy)-2-5-{3-(2-tert-butoxycarbonylamino-3-(3-pyridyl)propionylamino)phenyl}-2-thienyl]-3,4,5,6-tetrahydro-2H-thiopyran-2-acetamide 1,1-dioxide (1.54 g)

NMR (DMSO-$d_6$, δ): 1.22-1.27 (2H, m), 1.43 (9H, s), 1.63-1.68 (4H, m), 1.98 (2H, br), 2.07-2.25 (2H, m), 2.74-2.98 (2H, m), 3.04-3.20 (6H, m), 3.41-3.48 (1H, m), 3.66-3.76 (1H, m), 4.45 (1/2H, br), 4.54-4.63 (1H, br), 4.86 (1/2H, br), 5.31-5.45 (1H, m), 6.82-7.00 (2H, m), 7.04-7.20 (3H, m), 7.22-7.27 (2H, m), 7.52-7.65 (2H, m), 8.43-8.59 (3H, m)

MS (ESI+): 713.1 (M+H)

Example 387

(2S)-N-(2-Tetrahydropyranyloxy)-2-[5-(3-(3-carboxypropionylamino)phenyl}-2-thienyl]-3,4,5,6-tetrahydro-2H-thiopyran-2-acetamide 1,1-dioxide (50/mg) was obtained in a similar manner to that of Example 3.

NMR (DMSO-$d_6$, δ): 1.36-2.06 (10H, m), 2.34-2.63 (5H, m), 2.88-3.52 (6H, m), 3.65-3.90 (1H, m), 4.43 (1/2H, br), 4.75 (1/2H, br), 7.17-7.32 (1H, m), 7.28-7.48 (4H, m), 8.00 (1H, s), 10.08 (1H, s), 10.59 (1H, s), 11.22 (1H, s), 12.13 (1H, br s)

MS (ESI-) : 563.4 (M-H)

Example 388

(2S)-N-(2-Tetrahydropyranyloxy)-2-[5-(3-(3-(methylaminocarbonyl)propionylamino)phenyl}-2-thienyl]-3,4,5,6-tetrahydro-2H-thiopyran-2-acetamide 1,1-dioxide (50 mg) was obtained in a similar manner to that of Example 31.

NMR ($CDCl_3$, δ): 1.48 (2H, br), 1.62 (4H, br), 1.96 (2H, br), 2.06-2.26 (2H, m), 2.62-2.66 (2H, m), 2.75 (3H, br s), 2.88 (2H, br), 3.05-3.22 (5H, m), 3.36-3.49 (1H, m), 3,73-3.99 (1H, m), 4.15-4.25 (1H, m), 4.70 (1/2H, s), 4.95 (1/2H, s), 6.97-7.12 (4H, m), 7.32-7.43 (2H, m), 9.12 (1/2H, br), 9.24 (1/2H, br)

MS (ESI-): 576.3 (M-H)

Example 389

(2S)-N-(2-Tetrahydropyranyloxy)-2-[5-(3-(tert-butoxycarbonylaminomethyl)phenyl)-2-thienyl]-3,4,5,6-tetrahydro-2H-thiopyran-2-acetamide 1,1-dioxide (200 mg) was obtained in a similar manner to that of Example 85.

NMR ($CDCl_3$, δ): 1.47 (9H, s), 1.52-1.68 (6H, m), 1.81-1.95 (2H, m), 2.06-2.23 (2H, m), 2.63-2.89 (2H, m), 3.05 (2H, br s), 3.08-3.13 (2H, m), 3.27-3.50 (1H, m), 3.61-3.70 (1H, m), 4.24-4.39 (2H, m), 4.52 (1/2H, br), 4.80 (1/2H, br), 6.71-6.82 (1H, m), 7.14-7.70 (5H, m), 8.07 (1/2H, s), 8.24 (1/2H, s)

MS (ESI-): 577.3 (M-H)

Example 390

(2S)-N-(2-Tetrahydropyranyloxy)-2-[5-{3-(2-(methylamino)acetylamino)phenyl}-2-thienyl]-3,4,5,6-tetrahydro-2H-thiopyran-2-acetamide 1,1-dioxide (915 mg) was obtained in a similar manner to that of Example 234.

NMR (DMSO-$d_6$, δ): 1.42-1.59 (6H, m), 1.73-2.05 (4H, m), 2.32 (3H, s), 2.37-2.46 (1H, m), 2.89-3.20 (4H, m), 3.25 (2H, s), 3.42-3.48 (2H, m), 3.75-3.78 (1H, m), 4.44 (1/2H, br), 4.75 (1/2H, br), 7.20-7.24 (1H, m), 7.34-7.36 (2H, m), 7.38-7.42 (1H, m), 7.55-7.57 (1H, m), 8.02 (1H, s)

MS (ESI+): 536.3 (M+H)

The following compounds were obtained in a similar manner to that of Example 52.

Example 391

(2S)-N-Hydroxy-2-[5-(6-methyl-3-pyridyl)-2-thienyl]-3,4,5,6-tetrahydro-2H-thiopyran-2-acetamide 1,1-dioxide hydrochloride (89 mg)

NMR (DMSO-$d_6$, δ): 1.71-2.10 (4H, m), 2.35-2.54 (1H, m), 2.68 (3H, s), 2.96-3.08 (2H, m), 3.14-3.60 (3H, m), 7.32 (1H, d, J=3.5Hz), 7.74-7.81 (2H, m), 8.49 (1H, dd, J=1.5, 8Hz), 9.01 (1H, d, J=1.5Hz), 10.66 (1H, s)

MS (ESI-): 379 (M-H)

Example 392

(2S)-N-Hydroxy-2-[5-(6-methoxy-3-pyridyl)-2-thienyl]-3,4,5,6-tetrahydro-2H-thiopyran-2-acetamide, 1,1-dioxide (50 mg)

NMR (DMSO-$d_6$, δ): 1.71-2.09 (4H, m), 2.33-2.52 (1H, m), 2.95-3.08 (2H, m), 3.10-3.29 (2H, m), 3.42-3.58 (1H, m), 3.89 (3H, s), 6.89 (1H, d, J=8Hz), 7.21 (1H, d, J=3.5Hz), 7.43 (1H, d, J=3.5Hz), 7.97 (1H, dd, J=1.5, 8Hz), 8.47 (1H, d, J=1.5Hz)

MS (ESI-): 395 (M-H)

Example 393

(2S)-N-Hydroxy-2-[5-[4-(5-methyl-1,2,4-oxadiazol-3-yl)phenyl]-2-thienyl]-3,4,5,6-tetrahydro-2H-thiopyran-2-acetamide 1,1-dioxide (67 mg)

NMR (DMSO-$d_6$, δ): 1.71-2.09 (4H, m), 2.20-2.52 (1H, m), 2.68 (3H, s), 2.94-3.08 (2H, m), 3.11-3.33 (2H, m), 3.36-3.56 (1H, m), 7.26 (1H, d, J=3.5), 7.63 (1H, d, J=3.5Hz), 7.84 (2H, d, J=8Hz), 8.03 (2H, d, J=8Hz), 8.84 (1H, s)

MS (ESI-): 446 (M-H)

Example 394

(2S)-N-Hydroxy-2-[5-{3-(2-(acetylamino)acetylamino)-phenyl}-2-thienyl]-3,4,5,6-tetrahydro-2H-thiopyran-2-acetamide 1,1-dioxide (75 mg)

NMR (DMSO-$d_6$, δ): 1.69-2.08 (4H, m), 1.89 (3H, s), 2.34-2.48 (1H, m), 2.94-3.30 (4H, m), 3.38-3.55 (1H, m), 3.88 (2H, d, J=8.0Hz), 7.21 (1H, d, J=4.0Hz), 7.32-7.40 (2H, m), 7.42 (1H, d, J=4.0Hz), 7.44-7.51 (1H, m), 7.98 (1H, s), 8.22 (1H, t, J=8.0Hz), 8.83 (1H, s), 10.08 (1H, s), 10.60 (1H, s)

MS (ESI-): 478.3 (M-H)

Example 395

(2S)-N-Hydroxy-2-[5-(3-(3-methoxycarbonylpropionylamino)phenyl}-2-thienyl]-3,4,5,6-tetrahydro-2H-thiopyran-2-acetamide 1,1-dioxide (60 mg)

NMR (DMSO-$d_6$, δ): 1.72-2.09 (4H, m), 2.33-2.48 (1H, m), 2.63 (3H, s), 2.77-3.40 (6H, m), 3.42-3.55 (1H, m), 7.21 (1H, d, J=4.0Hz), 7.28-7.48 (4H, m), 7.98 (1H, s), 8.84 (1H, s), 10.12 (1H, s), 10.60 (1H, s)

MS (ESI-): 493.4 (M-H)

Example 396

(2S)-N-Hydroxy-2-[5-{3-(3-(methylaminocarbonyl)-propionylamino)phenyl}-2-thienyl]-3,4,5,6-tetrahydro-2H-thiopyran-2-acetamide 1,1-dioxide (30 mg)

NMR (DMSO-$d_6$, δ): 1.73-2.05 (4H, m), 2.40 (2H, t, J=6.0Hz), 2.45-2.49 (1H, m), 2.53-2.58 (3H, m), 2.95-3.26 (4H, m), 3.40-3.54 (3H, m), 7.20 (1H, d, J=4.0Hz), 7.32 (2H, d, J=4.5Hz), 7.39 (1H, d, J=4.0Hz), 7.43-7.47 (2H, m), 7.82 (1H, br), 8.00 (1H, s), 10.08 (1H, s), 10.60 (1H, s)

MS (ESI-): 492.1 (M-H)

Example 397

(2S)-N-Hydroxy-2-[5-{3-(2-(2-methoxyethoxy)-acetylamino)phenyl}-2-thienyl]-3,4,5,6-tetrahydro-2H-thiopyran-2-acetamide 1,1-dioxide (120 mg)

NMR (DMSO-$d_6$, δ): 1.70-2.07 (4H, m), 2.36-2.46 (1H, m), 2.94-3.28 (4H, m), 3.31 (3H, s), 3.48-3.51 (1H, m), 3.52-3.58 (2H, m), 3.66-3.72 (2H, m), 4.10 (2H, s), 7.22 (1H, d, J=4.0Hz), 7.33-7.40 (1H, m), 7.40 (1H, d, J=4.0Hz), 7.52-7.62 (1H, m), 8.00 (1H, s), 8.84 (1H, s)., 9.76 (1H, s), 10.60 (1H, s)

MS (ESI-): 495.3 (M-H)

Example 398

(25)-N-Hydroxy-2-[5-(3-aminomethylphenyl)-2-thienyl]-3,4,5,6-tetrahydro-2H-thiopyran-2-acetamide 1,1-dioxide (132 mg) from (2S)-N-(2-tetrahydropyranyloxy)-2-[5-(3-(tert-butoxycarbonylaminomethyl)phenyl)-2-thienyl]-3,4,5,6-tetrahydro-2H-thiopyran-2-acetamide 1,1-dioxide

NMR ($DMSO_6$, δ): 1.73-2.08 (4H, m), 2.35-2.45 (1H, m). 2.9.5-3.26 (4H, m), 3.43-3.55 (1H, m), 4.08 (2H, br), 7.24 (1H, d, J=4.0Hz), 7.37-7.39 (1H, m), 7.45-7.50 (2H, m), 7.68 (1H, d, J=7.5Hz), 7.75 (1H, s), 8.15 (2H, br), 8.83 (1H, s), 10.60 (1H, s)

MS (ESI-): 435.2 ($M-H+CH_3CN$)

Example 399

(2S)-N-Hydroxy-2-[5-{3-(2-(N-methoxycarbonyl-N-methylamino)acetylamino)phenyl)-2-thienyl]-3,4,5,6-tetrahydro-2H-thiopyran-2-acetamide 1,1-dioxide (110 mg)

NMR (DMSO-$d_6$, δ): 1.75-2.06 (4H, m), 2.37-2.46 (1H, m), 2.90 (3/2H, s), 2.94 (3/2H, s), 2.95-3.26 (4H, m), 3.40-3.50 (1H, m), 3.56 (3/2H, s), 3.63 (3/2H, s), 4.05 (2H, s), 7.20 (1H, d, J=4.0Hz), 7.32-7.39 (3H, m), 7.42 (1H, d, J=4.0Hz), 8.02 (1H, s), 8.84 (1H, s)

MS (ESI-): 508.4 (M-H)

Example 400

(2S)-N-Hydroxy-2-[5-{3-(((2S)-2,6-diaminohexanoyl)-amino)phenyl}-2-thienyl]-3,4,5,6-tetrahydro-2H-thiopyran-2-acetamide 1,1-dioxide (200 mg) from (2S)-N-(2-tetrahydropyranyloxy)-2-[5-{3-(((2S)-2,6-bis(tert-butoxycarbonylamino)hexanoyl)amino)phenyl}-2-thienyl]-3,4,5,6-tetrahydro-2H-thiopyran-2-acetamide 1,1-dioxide

NMR (DMSO-$d_6$, δ): 1.33-1.44 (2H, m), 1.50-1.60 (2H, m), 1.73-1.93 (4H, m), 1.95-2.07 (1H, m), 2.35-2.45 (1H, m), 2.73-2.80 (2H, m), 2.95-3.28 (4H, m), 3.50-3.55 (1H, m), 3.94 (1H, br), 7.22 (1H, d, J=4.0Hz), 7.42-7.45 (3H, m), 7.53 (1H, d, J=7.0Hz), 7.94 (1H, s), 8.38 (1H, s)

MS (ESI+): 509.3 (M+H)

Example 407

(2S)-N-Hydroxy-2-[5-{3-(2-amino-3-(3-pyridyl)-propionylamino)phenyl}-2-thienyl]-3,4,5,6-tetrahydro-2H-thiopyran-2-acetamide 1,1-dioxide (460 mg) from (2S)-N-(2-tetrahydropyranyloxy)-2-[5-(3-(2-tert-butoxycarbonylamino-3-(3-pyridyl)propionylamino)phenyl)-2-thienyl]-3,4,5,6-tetrahydro-2H-thiopyran-2-acetamide 1,1-dioxide

NMR (DMSO-$d_6$, δ): 1.74-2.07 (4H, m), 2.36-2.46 (1H, m), 2.96-3.32 (6H, m), 3.45-3.55 (1H, m), 4.24 (1H, br), 7.22 (1H, d, J=4.0Hz), 7.39-7.48 (4H, m), 7.52-7.57 (1H, m), 7.83-7.88 (2H, m), 8.39 (2H, br), 8.56-8.60 (2H, m), 10.57 (1H, s), 10.60 (1H, s)

MS (ESI+): 529.1 (M+H)

Example 402

(2S)-N-Hydroxy-2-[5-(4-(3-pyridyl-2-propenyloxy)-phenyl)-2-thienyl]-3,4,5,6-tetrahydro-2H-thiopyran-2-acetamide 1,1-dioxide (180 mg)

NMR (DMSO-$d_6$, δ): 1.70-2.06 (4H, m), 2.36-2.47 (1H, m), 2.96-3.56 (5H, m), 7.16 (1H, d, J=16Hz), 7.23 (1H, d, J=4.0Hz), 7.30 (2H, d, J=8.5Hz), 7.49 (1H, d, J=16Hz), 7.70-7.76 (3H, m), 7.97 (1H, d, J=8.5Hz), 8.53-8.57 (1H, m), 8.75 (1H, d, J=5Hz), 9.12 (1H, s), 10.6 (1H, s)

MS (ESI-): 511 (M-H)

Example 403

To a solution of (2S)-2-tert-butoxycarbonylamino-3-hydroxypropionic acid (66 mg), 1-hydroxybenzotriazole (44 mg) and 1-ethyl-3-(3-dimethylaminopropyl)carbodiimide hydrochloride (62 mg) in N,N-dimethylformamide (1.3 ml) was added a solution of (2S)-N-(2-tetrahydropyranyloxy)-2-[5-(3-aminophenyl)-2-thienyl]-3,4,5,6-tetrahydro-2H-thiopyran-2-acetamide 1,1-dioxide (100 mg) in N,N-dimethylformamide (0.5 ml) at ambient temperature. After being stirred at the same temperature overnight, the reaction mixture was added ethyl acetate and the solution was washed successively with water, a 5% aqueous citric acid solution, a saturated aqueous sodium hydrogen carbonate and brine,

dried over anhydrous magnesium sulfate and evaporated under a stream of nitrogen. After the residue was dissolved in methanol (1 ml), the solution was added 4N hydrogen chloride in ethyl acetate. The mixture was stirred at ambient temperature for 1 hour. After the solution was evaporated under a stream of nitrogen, the residue was purified by reverse phase HPLC (0.1% trifluoroacetic acid in acetonitrile, 1-60% gradient) to give (2S)-N-hydroxy-2-[5-(3-(((2S)-2-amino-3-hydroxypropionyl)amino)phenyl}-2-thienyl]-3,4,5,6-tetrahydro-2H-thiopyran-2-acetamide 1,1-dioxide (30 mg) as a white powder.

NMR (DMSO-$d_6$, δ): 1.76-2.10 (4H, m), 2.38-2.46 (1H, m), 3.00 (2H, d, J=10Hz), 3.13-3.52 (4H, m), 3.82-3.93 (1H, m), 3.98-4.06 (1H, m), 7.23 (1H, d, J=4.0Hz), 7.40-7.45 (3H, m), 7.50-7.54 (1H, m), 7.98 (1H, s), 8.32 (2H, br), 10.73 (1H, s)

MS (ESI+): 468.3 (M+H)

The following compound was obtained in a similar manner to that of Example 403.

Example 404

(2S)-N-Hydroxy-2-[5-(3-(2-dimethylaminocarbonyl)amino)-acetylamino)phenyl)-2-thienyl]-3,4,5,6-tetrahydro-2H-thiopyran-2-acetamide 1,1-dioxide (30 mg) from (2S)-N-(2-tetrahydropyranyloxy)-2-[5-(3-((2-aminoacetyl)amino)phenyl)-2-thienyl]-3,4,5,6-tetrahydro-2H-thiopyran-2-acetamide 1,1-dioxide (150 mg) and dimethylaminocarbonyl chloride.

NMR (DMSO-$d_6$, δ): 1.74-2.04 (4H, m), 2.35-2.44 (1H, m), 2.85 (6H, s), 2.95-3.25 (4H, m), 3.46-3.53 (1H, m), 3.78 (2H, d, J=4.0Hz), 6.65 (1H, t, J=4.0Hz), 7.20 (1H, d, J=4.0Hz), 7.34-7.40/(2H, m), 7.40-7.48 (2H, m), 8.01 (1H, s), 8.08 (1H, br), 9.98 (1H, s), 10.60 (1H, s)

MS (ESI+): 509.2 (M+H)

Example 405

(2S)-N-Hydroxy-2-[5-{3-(2-(N-(dimethylaminocarbonyl)-N-methylamino)acetylamino)phenyl}-2-thienyl]-3,4,5,6-tetrahydro-2H-thiopyran-2-acetamide 1,1-dioxide (30 mg) was obtained in a similar manner to that of Example 404.

NMR (DMSO-$d_6$, δ): 1.73-2.05 (4H, m), 2.37-2.48 (1H, m), 2.74 (6H, s), 2.87 (3H, s), 2.95-3.25 (4H, m), 3.44-3.52 (1H, m), 3.91 (2H, s), 7.20 (1H, d, J=4.0Hz), 7.34-7.45 (3H, m), 7.42 (1H, d, J=4.0Hz), 8.02 (1H, s), 10.06 (1H, s), 10.60 (1H, s)

MS (ESI-): 521.2 (M-H)

Example 406

(2S)-N-(2-Tetrahydropyranyloxy)-2-[5-[4-(5-methyl-1,2,4-oxadiazol-3-yl)phenyl]-2-thienyl]-3,4,5,6-tetrahydro-2H-thiopyran-2-acetamide 1,1-dioxide (113 mg) was obtained in a similar manner to that of Example 196.

NMR ($CDCl_3$, δ): 1.38-1.74 (6H, m), 1.90-2.01 (2H, m), 2.06-2.25 (2H, m), 2.67 (3H, s), 2.70-2.89 (2H, m), 3.03-3.08 (2H, m), 3.10-3.18 (2H, m), 3.28-3.50 (1H, m), 3.59-3.72 (1H, m), 4.53 (0.5H, s), 4.81 (0.5H, s), 7.28-7.32 (1H, m), 7.35-7.39 (1H, m), 7.71 (2H, d, J=8Hz), 7.99 (0.5H, s), 8.08 (2H, d, J=8Hz), 8.13 (0.5H, s)

MS (ESI-): 530 (M-H)

Example 407

tert-Butyl 2-(5-bromo-2-thienyl)-2,3,4,5-tetrahydrothiophene-2-acetate (2.77 g) was obtained in substantially the same manner as that of Example 89.

NMR ($CDCl_3$, δ): 1.34 (9H, s), 1.90-2.05 (1H, m), 2.07-2.20 (2H, m), 2.26-2.36 (1H, m), 2.83 (1H, d, J=15Hz), 2.98-3.09 (2H, m), 3.12 (1H, d, J=15Hz), 6.70 (1H, d, J=4Hz), 6.87 (2H, d, J=4Hz)

Example 408

tert-Butyl 2-[5-(4-fluorophenyl)-2-thienyl]-2,3,4,5-tetrahydrothiophene-2-acetate (479 mg) was obtained in substantially the same manner as that of Example 100.

NMR ($CDCl_3$, δ): 1.34 (9H, s), 2.02-2.29 (3H, m), 2.36-2.46 (1H, m), 3.00 (1H, d, J=16Hz), 3.01-3.14 (2H, m), 3.17 (1H, d, J=16Hz), 6.92 (1H, d, J=4Hz), 7.00-7.08 (3H, m), 7.52 (2H, dd, J=4, 9Hz)

Example 409

2-[5-(4-Fluorophenyl)-2-thienyl]-2,3,4,5-tetrahydrothiophene-2-acetic acid (298 mg) was obtained in a similar manner to that of Example 90.

NMR ($CDCl_3$, δ): 2.02-2.31 (3H, m), 2.39-2.50 (1H, m), 2.98-3.17 (3H, m), 3.30 (1H, d, J=16Hz), 6.93 (1H, d, J=4Hz), 6.99-7.08 (3H, m), 7.50 (2H, dd, J=5, 9Hz)

MS (ESI-): 321 (M-H)

Example 410

2-[5-(4-Fluorophenyl)-2-thienyl]-2,3,4,5-tetrahydrothiophene-2-acetic acid 1,1-dioxide (272 mg) was obtained in a similar manner to that of Preparation 1-4).

NMR ($CDCl_3$, δ): 2.18-2.43 (2H, m), 2.70-2.82 (1H, m), 2.89-3.01 (1H, m), 3.09 (1H, d, J=16Hz), 3.14-3.25 (2H, m), 3.36 (1H, d, J=16Hz), 7.07 (2H, t, J=9Hz), 7.13-7.18 (2H, m), 7.54 (2H, dd, J=5, 9Hz)

The following compounds were obtained in a similar manner to that of Example 125.

Example 411

(2S)-N-(2-Tetrahydropyranyloxy)-2-[5-{3-(2-(benzyloxycarbonylamino)acetylamino)phenyl}-2-thienyl]-3,4,5,6-tetrahydro-2H-thiopyran-2-acetamide 1,1-dioxide (230 mg)

NMR ($CDCl_3$, δ): 1.43 (4H, br), 1.63-1.65 (2H, m), 1.94 (2H, br), 2.09-2.23 (2H, m), 2.82 (2H, br), 3.06-3.17 (4H, m), 3.27-3.50 (1H, m), 3.64-3.76 (1H, m), 4.03-4.05 (2H, m), 4.52 (1/2H, br), 4.83 (1/2H, br), 5.18 (2H, 's), 7.10-7.22 (4H, m), 7.30-7.38 (6H, m), 7.45-7.55 (2H, m), 8.23 (1H, br), 8.77 (1/2H, br), 8.85 (1/2H, br)

MS (ESI-): 654.2 (M-H)

Example 412

(2S)-N-(2-Tetrahydrogyranyloxy)-2-[5-{3-(2-(4-methoxybenzenesulfonylamino)acetylamino)phenyl)-2-thienyl]-3,4,5,6-tetrahydro-2H-thiopyran-2-acetamide 1,1-dioxide (220 mg)

NMR ($CDCl_3$, δ): 1.43 (4H, br), 1.62-1.72 (2H, m), 1.95 (2H, br), 2.06-2.25 (2H, m), 2.75-2.90 (2H, m), 3.00-3.18 (4H, m), 3.28-3.50 (1H, m), 3.64-3.70 (1H, m), 3.75 (2H, d, J=6.0Hz), 3.82 (3H, s), 4.54 (1/2H, br), 4.85 (1/2H, br), 5.72-5.86 (1H, m), 6.98 (2H, d, J=8.0Hz), 7.14-7.23 (4H, m), 7.85 (2H, d, J=8.0Hz), 8.40 (1H, br), 8.78 (1/2H, s), 8.94 (1/2H, s)

MS (ESI-): 690.1 (M-H)

Example 413

(2S)-N-(2-Tetrahydropyranyloxy)-2-[5-[3-[2-(1,5,5-trimethylhydantoin-3-yl)acetylamino]phenyl]-2-thienyl]-3,4,5,6-tetrahydro-2H-thiopyran-2-acetamide 1,1-dioxide (212 mg) was obtained in a similar manner to that of Example 206.

NMR ($CDCl_3$, δ): 1.38-1.75 (12H, m), 1.87-2.05 (2H, m), 2.07-2.28 (2H, m), 2.77-2.96 (5H, m), 3.00-3.25 (4H, m), 3.27-3.54 (1H, m), 3.68-3.81 (1H, m), 4.40 (2H, s), 4.53 (0.5H, s), 4.85 (0.5H, s), 6.93-7.06 (1H, m), 7.09-7.34 (4H, m), 7.54-7.66 (1H, m), 8.25-8.35 (1H, m), 9.05 (0.5H, s), 9.20 (0.5H, s)

MS (ESI-): 645 (M-H)

Example 414

(2S)-N-(2-Tetrahydropyranyloxy)-2-[5-[4-(phenoxycarbonyloxymethyl)phenyl]-2-thienyl]-3,4,5,6-tetrahydro-2H-thiopyran-2-acetamide 1,1-dioxide (120 mg) was obtained in a similar manner to that of Example 239.

NMR ($CDCl_3$, δ): 1.35-1.76 (6H, m), 1.87-2.00 (2H, m), 2.04-2.25 (2H, m), 2.65-2.91 (2H, m), 3.00-3.18 (4H, m), 3.25-3.51 (1H, m), 3.59-3.72 (1H, m), 4.53 (0.5H, s), 4.81 (0.5H, s), 5.27 (2H, s), 7.17 (2H, d, J=8Hz), 7.23-7.32 (1H, m), 7.35-7.49 (6H, m), 7.62 (2H, d, J=8Hz), 8.01 (0.5H, s), 8.15 (0.5H, s)

MS (ESI-): 598 (M-H)

Example 415

(2S)-N-(2-Tetrahydropyranyloxy)-2-[5-[4-(methylaminocarbonyloxymethyl)phenyl]-2-thienyl]-3,4,5,6-tetrahydro-2H-thiopyran-2-acetamide 1,1-dioxide (70 mg) was obtained in a similar manner to that of Example 240.

NMR ($CDCl_3$, δ): 1.36-1.75 (6H, m), 1.86-2.01 (2H, m), 2.05-2.24 (2H, m), 2.64-2.85 (5H, m) , 3.01-3.18 (4H, m), 3.27-3.50 (1H, m), 3.56-3.70 (1H, m), 4.53 (0.5H, s), 4.61-4.74 (1H, m), 4.80 (0.5H, s), 5.11 (2H, s), 7.21-7.29 (2H, m), 7.36 (2H, d, J=8Hz), 7.56 (2H, d, J=8Hz), 7.96 (0.5H, s), 8.11 (0.5H, s)

MS (ESI-): 535 (M-H)

Example 416

(2S)-N-(2-Tetrahydropyranyloxy)-2-[5-[5-(methoxycarbonylamino)-3-pyridyl]-2-thienyl]-3,4,5,6-tetrahydro-2H-thiopyran-2-acetamide 1,1-dioxide (170 mg) was obtained in a similar manner to that of Example 196.

NMR ($CDCl_3$, δ): 1.40-1.74 (6H, m), 1.89-2.01 (2H, m), 2.05-2.25 (2H, m), 2.70-2.95 (2H, m), 3.01-3.17 (4H, m), 3.30-3.56 (1H, m), 3.62-3.76 (1H, m), 3.83 (3H, s), 4.53 (0.5H, s), 4.84 (0.5H, s), 6.97-7.06 (1H, m), 7.25-7.33 (1H, m), 7.36-7.65 (1H, m), 8.10-8.19 (1H, m), 8.40-8.56 (3H, m)

MS (ESI-): 522 (M-H)

Example 417

(2S)-N-(2-Tetrahydropyranyloxy)-2-[5-(3-(methylaminocarbonylmethylaminocarbonylamino)phenyl)-2-thienyl]-3,4,5,6-tetrahydro-2H-thiopyran-2-acetamide 1,1-dioxide (45 mg) was obtained in a similar manner to that of Example 31.

NMR ($CDCl_3$, δ): 1.40-1.47 (4H, m), 1.64-1.69 (2H, m), 2.15 (2H, br), 2.28 (2H, br), 2.80 (3H, br s), 2.81-2.88 (2H, m), 2.98-3.15 (4H, m), 3.25-3.52 (1H, m), 3.72-3.81 (1H, m), 3.85-3.94 (2H, m), 4.50 (1/2H, br), 4.85 (1/2H, br), 5.27-5.43 (1H, m), 7.12-7.19 (6H, m), 7.48-7.55 (2H, m), 7.67-7.72 (1H, m)

MS (ESI-): 577.2 (M-H)

Example 418

(2S)-N-(2-Tetrahydropyranyloxy)-2-[5-(3-(carboxymethylaminocarbonylamino)phenyl)-2-thienyl]-3,4,5,6-tetrahydro-2H-thiopyran-2-acetamide 1,1-dioxide (130 mg) was obtained in a similar manner to that of Example 3.

NMR ($CDCl_3$, δ): 1.43-1.50 (2H, m), 1.64-1.68 (4H, m), 1.92 (2H, br), 2.08-2.21 (2H, m), 2.40-2.48 (2H, m), 2.95-3.14 (4H, m), 3.30-3.53 (1H, m), 3.75-3.81 (1H, m), 4.00 (2H, br s), 4.45 (1/2H, br), 4.82 (1/2H, br), 7.15-7.22 (5H, m), 7.53 (1/2H, s), 7.57 (1/2H, s)

$MS^-$ (ESI-): 565.0 (M-H)

The following compounds were obtained in a similar manner to that of Example 244.

Example 419

(2S)-N-(2-Tetrahydropyranyloxy)-2-(5-(3-(2-(allylamino)acetylamino)phenyl}-2-thienyl]-3,4,5,6-tetrahydro-2H-thiopyran-2-acetamide 1,1-dioxide (106 mg)

NMR ($CDCl_3$, δ): 1.45 (2H, br), 1.64-1.68 (4H, m), 1.95 (2H, br), 2.06-2.24 (2H, m), 2.64-2.86 (2H, m), 3.07 (2H, br s), 3.13-3.16 (2H, m), 3.30-3.49 (1H, m), 3.46 (3H, d, J=7.0Hz), 3.45 (2H, s), 3.62-3.71 (1H, m), 4.52 (1/2H, s), 4.80 (1/2H, s), 5.20-5.30 (2H, m), 5.85-5.98 (1H, m), 7.23-7.32 (4H, m), 7.56 (1H, br), 7.80 (1H, s), 9.40 (1H, s)

MS (ESI+): 562.2 (M+H)

Example 420

(2S)-N-(2-Tetrahydropyranyloxy)-2-[5-{3-(2-(2-ethoxyethylamino)acetylamino)phenyl}-2-thienyl]-3,4,5,6-tetrahydro-2H-thiopyran-2-acetamide 1,1-dioxide (65 mg)

NMR ($CDCl_3$, δ): 1.23 (3H, t, J=7.5Hz), 1.46 (2H, br), 1.65-1.80 (4H, m), 1.95 (2H, br), 2.05-2.25 (2H, m), 2.65-2.85 (1H, m), 2.88 (2H, t, J=6.0Hz), 3.30-3.49 (1H, m), 3.44 (2H, s), 3.51-3.58 (4H, m), 3.62-3.70 (1H, m), 4.52 (1/2H, br), 4.80 (1/2H, br), 7.27-7.35 (5H, m), 7.57-7.61 (1H, m), 7.83 (1H, s), 8.03 (1/2H, br), 8.20 (1/2H, br s), 9.52 (1H, s)

MS (ESI+): 594.2 (M+H)

Example 421

(2S)-N-(2-Tetrahydropyranyloxy)-2-[5-{3-(2-(benzylamino)acetylamino)phenyl}-2-thienyl]-3,4,5,6-tetrahydro-2H-thiopyran-2-acetamide 1,1-dioxide (95 mg)

NMR ($CDCl_3$, δ): 1.43 (2H, br), 1.52-1.68 (4H, m), 1.96 (2H, br), 2.10-2.22 (2H, m), 2.66-2.89 (2H, m), 3.06 (2H, br s), 3.10-3.16 (2H, m), 3.29-3.50 (1H, m), 3.45 (2H, br s), 3.60-3.70 (1H, m), 3.88 (2H, s), 4.52 (1/2H, br), 4.80 (1/2H,

br), 7.27-7.38 (10H, m), 7.52 (1H, br), 7.79 (1H, br), 8.05 (1/2H, br), 8.20 (1/2H, br), 9.33 (1H, s)
MS (ESI+): 612.2 (M+H)

Example 422

(2S)-N-(2-Tetrahydropyranyloxy)-2-[5-{3-(2-(pentylamino)acetylamino)phenyl}-2-thienyl]-3,4,5,6-tetrahydro-2H-thiopyran-2-acetamide 1,1-dioxide (83 mg)
NMR ($CDCl_3$, δ): 0.87-0.94 (5H, m), 1.29-1.64 (6H, m), 1.42-1.64 (4H, m), 1.95 (2H, br), 2.08-2.22 (2H, m), 2.62-2.88 (2H, m), 2.69 (2H, t, J=7.0Hz), 3.06 (2H, s), 3.10-3.14 (2H, m), 3.24-3.50 (1H, m), 3.39 (2H, s), 3.61-3.69 (1H, m), 4.54 (1/2H, br), 4.80 (1/2H, br), 7.25-7.33 (5H, m), 7.56 (1H, br), 7.80 (1H, s), 8.00 (1/2H, br), 8.16 (1/2H, s), 9.44 (1H, s)
MS (ESI+): 592.3 (M+H)

Example 423

(2S)-N-(2-Tetrahydropyranyloxy)-2-[5-{3-((2R)-2-tert-butoxycarbonylamino-3-benzyloxypropionyl)aminophenyl}-2-thienyl]-3,4,5,6-tetrahydro-2H-thiopyran-2-acetamide 1,1-dioxide (400 mg)
NMR ($CDCl_3$, δ): 1.40-1.48 (2H, br), 1.49 (9H, s), 1.60-1.68 (4H, m), 1.94 (2H, br), 2.06-2.24 (2H, m), 2.65-2.90 (2H, m), 3.06 (1H, br s), 3.11-3.16 (2H, br), 3.27-3.47 (1H, m), 3.63-3.70 (2H, m), 3.97-4.04 (1H, m), 4.44 (1H, br), 4.52 (1/2H, br), 4.55 (1H, d, J=11.0Hz), 4.65 (1H, d, J=11.0Hz), 4.80 (1/2H, br), 5.50 (1H, br), 7.22-7.41 (11H, m), 7.72 (1H, br), 8.07 (1/2H, s), 8.20 (1/2H, s), 8.47 (1H, br)
MS (ESI-): 776.2 (M-H+Cl)

The following compounds were obtained in a similar manner to that of Example 52.

Example 424

(2S)-N-Hydroxy-2-[5-(3-cyclobutanecarbonylaminophenyl)-2-thienyl]-3,4,5,6-tetrahydro-2H-thiopyran-2-acetamide 1,1-dioxide (250 mg)
NMR (DMSO-$d_6$, δ): 1.75-2.30 (10H, m), 2.37-2.46 (1H, m), 2.95-3.52 (5H, m), 7.20 (1H, d, J=4.0Hz), 7.33-7.35 (2H, m), 7.40 (1H, d, J=4.0Hz), 7.46-7.50 (1H, m), 8.02 (1H, s), 9.84 (1H, s), 10.60 (1H, s)
MS (ESI-): 461 (M-H)

Example 425

(2S)-Hydroxy-2-[5-[3-[2-(1,5,5-trimethylhydantoin-3-yl)acetylamino]phenyl]-2-thienyl]-3,4,5,6-tetrahydro-2H-thiopyran-2-acetamide 1,1-dioxide (117 mg)
NMR (DMSO-$d_6$, δ): 1.36 (6H, s), 1.70-2.09 (4H, m), 2.35-2.54 (1H, m), 2.84 (3H, s), 2.94-3.08 (2H, m), 3.10-3.29 (2H, m), 3.36-3.55 (1H, m), 4.23 (2H, s), 7.21 (1H, d, J=3.5Hz), 7.35-7.43 (4H, m), 7.95 (1H, s), 8.83 (1H, s), 10.41 (1H, s), 10.60 (1H, s)
MS (ESI-): 561 (M-H)

Example 426

(2S)-N-Hydroxy-2-[5-[4-(methylaminocarbonyloxymethyl)-phenyl]-2-thienyl]-3,4,5,6-tetrahydro-2H-thiopyran-2-acetamide 1,1-dioxide (46 mg)
NMR (DMSO-$d_6$, δ): 1.67-2.09 (4H, m), 2.34-2.53 (1H, m), 2.59 (3H, d, J=4.8Hz), 2.94-3.08 (2H, m), 3.10-3..30 (2H, m), 3.38-3.55 (1H, m), 5.02 (2H, s), 7.21 (1H, d, J=3.5Hz), 7.38 (2H, d, J=8Hz), 7.48 (1H, d, J=3.5Hz), 7.64 (2H, d, J=8Hz), 8.85 (1H, br s)
MS (ESI-): 451 (M-H)

Example 427

(2S)-N-Hydroxy-2-[5-(5-(methoxycarbonylamino)-3-pyridyl]-2-thienyl]-3,4,5,6-tetrahydro-2H-thiopyran-2-acetamide 1,1-dioxide (94 mg)
NMR (DMSO-$d_6$, δ): 1.71-2.09 (4H, m), 2.35-2.56 (1H, m), 2.95-3.08 (2H, m), 3.11-3.32 (2H, m), 3.41-3.57 (1H, m), 3.74 (3H, s), 7.29 (1H, d, J=3.5Hz), 7.65 (1H, d, J=3.5Hz), 8.32 (1H, s), 8.63 (1H, d, J=1.5Hz), 8.71 (1H, d, J=1.5Hz)
MS (ESI+): 440 (M+H)

Example 428

(2S)-N-Hydroxy-2-[5-[3-(2,2-dimethylpropionylamino)-phenyl]-2-thienyl]-3,4,5,6-tetrahydro-2H-thiopyran-2-acetamide 1,1-dioxide (100 mg)

NMR (DMSO-$d_6$, δ): 1.24 (9H, s), 1.71-2.08 (4H, m), 2.35-2.53 (1H, m), 2.94-3.07 (2H, m), 3.10-3.28 (2H, m), 3.39-3.55 (1H, m), 7.20 (1H, d, J=3.5Hz), 7.30-7.39 (2H, m), 7.41 (1H, d, J=3.5Hz), 7.59-7.65 (1H, m), 8.01 (1H, s), 8.84 (1H, br s), 9.30 (1H, s)

MS (ESI-): 463 (M-H)

Example 429

(2S)-N-Hydroxy-2-[5-[3-((E)-2-butenoylamino)phenyl]-2-thienyl]-3,4,5,6-tetrahydro-2H-thiopyran-2-acetamide 1,1-dioxide (104 mg)

NMR (DMSO-$d_6$, δ): 1.71-2.08 (7H, m), 2.36-2.55 (1H, m), 2.94-3.08 (2H, m), 3.10-3.29 (2H, m), 3.39-3.85 (1H, m), 6.14 (1H, dd, J=1.5, 14Hz), 6.75-6.88 (1H, m), 7.21 (1H, d, J=3.5Hz), 7.32-7.39 (2H, m), 7.41 (1H, d, J=3.5Hz), 7.50-7.56 (1H, m), 8.05 (1H, s), 10.08 (1H, s), 10.60 (1H, s)

MS (ESI-): 447 (M-H)

Example 430

(2S)-N-Hydroxy-2-[5-{3-(2-(benzyloxycarbonylamino)-acetylamino)phenyl}-2-thienyl]-3,4,5,6-tetrahydro-2H-thiopyran-2-acetamide 1,1-dioxide (150 mg)

NMR (DMSO-$d_6$, δ): 1.75-2.06 (4H, m), 2.38-2.47 (1H, m), 2.96-3.27 (4H, m), 3.40-3.55 (1H, m), 3.83 (2H, d, J=6.5Hz), 5.06 (2H, s), 7.22 (1H, d, J=4.0Hz), 7.29-7.40 (8H, m), 7.45-7.50 (1H, m), 7.58 (1H, t, J=6.5Hz), 8.00 (1H, s), 8.84 (1H, br), 10.10 (1H, s), 10.60 (1H, br)

MS (ESI-): 570.1 (M-H)

Example 431

(2S)-N-Hydroxy-2-[5-(3-(2-(4-methoxybenzenesulfonylamino)acetylamino)phenyl}-2-thienyl]-3,4,5,6-tetrahydro-2H-thiopyran-2-acetamide 1,1-dioxide (170 mg)

NMR (DMSO-$d_6$, δ): 1.73-2.05 (4H, m), 2.35-2.46 (1H, m), 2.95-3.26 (4H, m), 3.49-3.53 (1H, m), 3.62 (2H, s), 3.77 (3H, s), 7.08 (2H, d, J=8.0Hz), 7.20 (1H, d, J=4.0Hz), 7.30-7.38 (4H, m), 7.75 (12H, d, J=8.0Hz), 7.84 (1H, s), 8.84 (1H, br), 10.02 (1H, s)

MS (ESI-): 606.2 (M-H)

Example 432

(2S)-N-Hydroxy-2-[5-(3-(methylaminocarbonylmethylaminocarbonylamino)phenyl)-2-thienyl]-3,4,5,6-tetrahydro-2H-thiopyran-2-acetamide 1,1-dioxide (25 mg)

NMR (DMSO-$d_6$, δ): 1.72-2.08 (4H, m), 2.36-2.45 (1H, m), 2.61 (3H, d, J=4.5Hz), 2.95-3.25 (4H, m), 3.42-3.50 (1H, m), 3.70 (2H, d, J=6.5Hz), 6.45 (1H, t, J=6.5Hz), 7.17-7.27 (4H, m), 7.37 (1H, d, J=4.0Hz), 7.84 (4H, s), 7.88 (1H, br), 8.84 (1H, br), 9.01 (1H, s), 10.63 (1H, s)

MS (ESI-): 493.3 (M-H)

Example 433

(2S)-N-Hydroxy-2-[5-{3-(((2R)-2-amino-3-benzyloxypropionyl)amino)phenyl}-2-thienyl]-3,4,5,6-tetrahydro-2H-thiopyran-2-acetamide 1,1-dioxide hydrochloride (125 mg) from (2S)-N-(2-tetrahydropyranyloxy)-2-[5-(3-(((2R)-2-tert-butoxycarbonylamino-3-benzyloxypropionyl)amino)phenyl}-2-thienyl]-3,4,5,6-tetrahydro-2H-thiopyran-2-acetamide 1,1-dioxide

NMR (DMSO-$d_6$, δ): 1.73-2.06 (4H, m), 2.36-2.46 (1H, m), 2.95-3.26 (4H, m), 3.40-3.55 (1H, m), 3.48 (2H, d, J=4.5Hz), 4.26 (1H, br t, J=4.5Hz), 4.58 (2H, d, J=5.0Hz), 7.23 (1H, d, J=4.0Hz), 7.27-7.35 (6H, m), 7.40-7.44 (2H, m), 7.53 (1H, d, J=6.0Hz), 7.94 (1H, s), 8.41 (2H, br), 8.83 (1H, s), 10.62 (1H, s), 10.82 (1H, s)

MS (ESI-): 558.3 (M-H)

Example 434

(2S)-N-Hydroxy-2-[5-{3-(2-(allylamino)acetylamino)-phenyl}-2-thienyl]-3,4,5,6-tetrahydro-2H-thiopyran-2-acetamide 1,1-dioxide (70 mg)

NMR (DMSO-$d_6$, δ): 1.75-2.06 (4H, m), 2.36-2.45 (1H, m), 2.96-3.27 (4H, m), 3.40-3.50 (1H, m), 3.69 (2H, d, J=6.0Hz), 3.93 (2H, s), 5.42-5.54 (2H, m), 5.84-5.98 (1H, m), 7.22 (1H, d, J=4.0Hz), 7.38-7.45 (3H, m), 7.48-7.52 (1H, m), 7.95 (1H, s), 8.85 (1H, br), 10.63 (1H, s), 10.77 (1H, s)

MS (ESI-): 476.1 (M-H)

Example 435

(2S)-N-Hydroxy-2-[5-{3-(2-(2-ethoxyethylamino)-acetylamino)phenyl}-2-thienyl]-3,4,5,6-tetrahydro-2H-thiopyran-2-acetamide 1,1-dioxide (45 mg)

NMR (DMSO-$d_6$, δ): 1.14 (3H, t, J=7.0Hz), 1.74-2.06 (4H, m), 2.35-2.45 (1H, m), 2.93-3.26 (4H, m), 3.41-3.47 (1H, m), 3.57 (2H, td, J=7.0, 7.0Hz), 3.55 (2H, t, J=4.5Hz), 3.65 (2H, s), 7.22 (1H, d, J=4.0Hz), 7.38-7.44 (3H, m), 7.50-7.54 (1H, m), 7.98 (1H, s), 8.84 (1H, s), 10.33 (1H, br), 10.62 (1H, s)

MS (ESI-): 508.2 (M-H)

Example 436

(2S)-N-Hydroxy-2-[5-(3-(2-pentylamino)acetylamino)-phenyl}-2-thienyl]-3,4,5,6-tetrahydro-2H-thiopyran-2-acetamide 1,1-dioxide (55 mg)

NMR (DMSO-$d_6$, δ): 0.90 (3H, t, J=4.5Hz), 1.24-1.34 (4H, m), 1.63 (2H, br), 1.73-2.05 (4H, m), 2.37-2.46 (1H, m), 2.92-3.28 (6H, m), 3.43-3.53 (1H, m), 3.92 (2H, s), 7.22 (1H, d, J=4.0Hz), 7.38-7.44 (3H, m), 7.50-7.54 (1H, m), 7.96 (1H, s), 8.84 (2H, br s), 10.64 (1H, s), 10.75 (1H, s)

MS (ESI+): 508.1 (M+H)

Example 437

(2S)-N-Hydroxy-2-[5-(3-isobutyrylaminophenyl)-2-thienyl]-3,4,5,6-tetrahydro-2H-thiopyran-2-acetamide 1,1-dioxide (250 mg)

NMR (DMSO-$d_6$, δ): 1.11 (6H, d, J=7.0Hz), 1.70-2.08 (4H, m), 2.35-2.45 (1H, m), 2.55-2.64 (1H, m), 2.95-3.54 (5H, m), 7.20 (1H, d, J=4.0Hz), 7.34-7.35 (2H, m), 7.40 (1H, d, J=4.0Hz), 7.46-7.51 (1H, m), 8.01 (1H, s), 8.84 (1H, s), 9.94 (1H, s), 10.60 (1H, s)

MS (ESI-): 449 (M-H)

Example 438

(2S)-N-Hydroxy-2-[5-{3-(2-benzylamino)acetylamino)-phenyl}-2-thienyl]-3,4,5,6-tetrahydro-2H-thiopyran-2-acetamide 1,1-dioxide (55 mg)

NMR (DMSO-$d_6$, δ): 1.74-2.06 (4H, m), 2.35-2.45 (1H, m), 2.95-3.28 (4H, m), 3.40-3.50 (3H, m), 3.89 (1H, s), 4.24 (1H, s), 7.22 (1H, d, J=4.0Hz), 7.36-7.55 (9H, m), 7.92 (1H, s), 8.84 (1H, s), 10.62 (1H, s), 10.68 (1H, s)

MS (ESI-): 528.1 (M-H)

The following compounds were obtained in a similar manner to that of Example 403.

Example 439

(2S)-N-Hydroxy-2-[5-{3-(((2S)-2-amino-3-benzyloxypropionyl)amino)phenyl}-2-thienyl]-3,4,5,6-tetrahydro-2H-thiopyran-2-acetamide 1,1-dioxide (40 mg) from L-Boc-Ser(bzl)-OH

NMR (DMSO-$d_6$, δ): 1.73-2.06 (4H, m), 2.36-2.46 (1H, m), 2.95-3.26 (4H, m), 3.40-3.55 (1H, m), 3.48 (2H, d, J=4.5Hz), 4.26 (1H, br t, J=4.5Hz), 4.58 (2H, d, J=5.0Hz), 7.23 (1H, d, J=4.0Hz), 7.27-7.35 (6H, m), 7.40-7.44 (2H, m), 7.53 (1H, d, J=6.0Hz), 7.94 (1H, s), 8.41 (2H, br), 8.83 (1H, s), 10.62 (1H, s), 10.82 (1H, s)

MS (ESI+): 558.3 (M+H)

Example 440

(2S)-N-Hydroxy-2-[5-{3-((2S)-2-pyrrolidinylcarbonylamino)phenyl}-2-thienyl]-3,4,5,6-tetrahydro-2H-thiopyran-2-acetamide 1,1-dioxide (40 mg) from L-Boc-Pro-OH

NMR (DMSO-$d_6$, δ): 1.84-2.05 (7H, m), 2.35-2.45 (2H, m), 2.95-3.30 (4H, m), 3.44-3.53 (3H, m), 4.35 (1H, br), 7.22 (1H, d, J=4.0Hz), 7.39-7.50 (4H, m), 7.96 (1H, s), 8.72 (1H, br), 8.83 (1H, s), 9.27 (1H, br)

MS (ESI+): 468.3 (M+H)

Example 441

(2S)-N-Hydroxy-2-[5-{3-(((2S)-2-amino-3-cyclohexylpropionyl)amino)phenyl}-2-thienyl]-3,4,5,6-tetrahydro-2H-thiopyran-2-acetamide 1,1-dioxide (50 mg) from L-Boc-CHA-OH

NMR (DMSO-$d_6$, δ): 0.87-0.98 (2H, m), 1.10-1.25 (4H, m), 1.40 (1H, br), 1.60-1.77 (6H, m), 1.85-2.06 (4H, m), 2.35-2.47 (1H, m), 2.95-3.27 (4H, m), 3.44-3.53 (1H, m), 3.96 (1H, br), 7.22 (1H, d, J=4.0Hz), 7.40-7.48 (3H, m), 7.60 (1H, d, J=7.0Hz), 7.90 (1H, s), 8.23 (2H, br), 8.84 (1H, s), 10.55 (1H, s), 10.60 (1H, s)

MS (ESI+): 534.8 (M+H)

Example 442

(2S)-N-Hydroxy-2-[5-{3-((2-amino-2-methylpropionyl)-amino)phenyl}-2-thienyl]-3,4,5,6-tetrahydro-2H-thiopyran-2-acetamide 1,1-dioxide (20 mg) from L-Boc-AiB-OH

NMR (DMSO-$d_6$, δ): 1.62 (6H, s), 1.74-2.06 (4H, m), 2.35-2.44 (1H, m), 2.95-3.27 (4H, m), 3.37-3.53 (1H, m), 7.22 (1H, d, J=4.0Hz), 7.38-7.48 (3H, m), 7.60 (1H, d, J=7.5Hz), 7.92 (1H, s), 8.24 (2H, br), 8.83 (1H, s)

MS.(ESI+): 466.3 (M+H)

Example 443

(2S)-N-Hydroxy-2-[5-{3-(((2R)-2-methoxypropionyl)-amino)phenyl)-2-thienyl]-3,4,5,6-tetrahydro-2H-thiopyran-2-acetamide 1,1-dioxide (30 mg)

NMR (DMSO-$d_6$, δ): 1.32 (3H, d, J=6.0Hz), 1.73-2.06 (4H, m), 2.36-2.43 (1H, m), 2.95-3.26 (4H, m), 3.34 (3H, s), 3.45-3.54 (1H, m), 3.87 (1H, q, J=6.0Hz), 7.20 (1H, d, J=4.0Hz), 7.32-7.35 (2H, m), 7.40 (1H, d, J=4.0Hz), 7.62 (1H, d, J=6.5Hz), 8.60 (1H, s), 9.93 (1H, s), 10.60 (1H, s)

MS (ESI-): 465.2 (M-H)

Example 444

(2S)-N-Hydroxy-2-[5-{3-(((2S)-2-amino-4-carboxybutyryl)amino)phenyl}-2-thienyl]-3,4,5,6-tetrahydro-2H-thiopyran-2-acetamide 1,1-dioxide (33 mg) from L-Boc-Glu(tBu)-OH

NMR (DMSO-$d_6$, δ): 1.74-2.25 (6H, m), 2.37-2.46 (1H, m), 2.95-3.26 (4H, m), 3.45-3.62 (1H, m), 3.95-4.04 (3H, m), 7.23 (1H, d, J=4.0Hz), 7.40-7.45 (3H, m), 7.52 (1H, d, J=7.0Hz), 7.90 (1H, s), 8.28 (2H, br), 8.84 (1H, br)

MS (ESI+): 510.7 (M+H)

Example 445

(2S)-N-Hydroxy-2-[5-{3-(2-(benzoylamino)acetylamino)-phenyl}-2-thienyl]-3,4,5,6-tetrahydro-2H-thiopyran-2-acetamide 1,1-dioxide (42 mg)

NMR (DMSO-$d_6$, δ): 1.72-2.04 (4H, m), 2.36-2.47 (1H, m), 2.95-3.25 (4H, m), 3.43-3.53 (1H, m), 4.09 (2H, d, J=6.0Hz), 7.20 (1H, d, J=4.0Hz), 7.36-7.39 (2H, m), 7.42 (1H, d, J=4.0Hz), 7.47-7.57 (4H, m), 7.92 (2H, d, J=7.5Hz), 8.30 (1H, s), 8.88 (1H, t, J=6.0Hz), 10.20. (1H, s), 10.60 (1H, s)

MS (ESI-): 541.3 (M-H)

Example 446

(2S)-N-Hydroxy-2-[5-{3-(2-(ethylamino)acetylamino)-phenyl}-2-thienyl]-3,4,5,6-tetrahydro-2H-thiopyran-2-acetamide 1,1-dioxide (60 mg) from 2-(N-ethyl-N-tert-butoxycarbonylamino)acetic acid

NMR (DMSO-$d_6$, δ): 1.22 (3H, t, J=7.0Hz), 1.74-2.06 (4H, m), 2.35-2.45 (1H, m), 2.95-3.27 (6H, m), 3.51-3.57 (1H, m), 3.96 (2H, t, J=5.0Hz), 7.22 (1H, d, J=4.0Hz), 7.42-7.46 (3H, m), 7.48-7.51 (1H, m), 7.93 (1H, s), 8.87 (2H, br), 10.60 (1H, s), 10.54 (1H, s)

MS (ESI+): 466.2 (M+H)

Example 447

(2S)-N-Hydroxy-2-[5-{3-(((2S)-2-(aminobutyryl)amino)-phenyl}-2-thienyl]-3,4,5,6-tetrahydro-2H-thiopyran-2-acetamide 1,1-dioxide (105 mg) from L-Boc-Abu-OH

NMR (DMSO-$d_6$, δ): 0.98 (3H, t, J=6.5Hz), 1.74-2.06 (4H, m), 1.89 (2H, qt, J=6.5, 6.5Hz), 2.36-2.45 (1H, m), 2.96-3.27 (4H, m), 3.44-3.54 (1H, m), 3.90 (1H, br), 7.22 (1H, d, J=4.0Hz), 7.39-7.46 (3H, m), 7.52-7.56 (1H, m), 7.91 (1H, s), 8.32 (2H, br), 8.85 (1H, s), 10.56 (1H, s), 10.60 (1H, s)

MS (ESI+): 467.2 (M+H)

Example 448

(2S)-N-Hydroxy-2-[5-{3-(2-(piperizinocarbonyloxy)-acetylamino)phenyl}-2-thienyl]-3,4,5,6-tetrahydro-2H-thiopyran-2-acetamide 1,1-dioxide (47 mg)

NMR (DMSO-$d_6$, δ): 1.48-1.58 (6H, m), 1.75-2.05 t4H, m), 2.37-2.46 (1H, m), 2.95-3.26 (4H, m), 3.46-3.55 (5H, m), 4.62 (2H, s), 7.20 (1H, d, J=4.0Hz), 7.35-7.38 (3H, m), 7.62 (1H, d, J=4.0Hz), 8.00 (1H, s), 10.17 (1H, s), 10.60 (1H, s)

MS (ESI-): 548.2 (M-H)

Example 449

(2S)-N-Hydroxy-2-[5-(3-(2-(benzylaminocarbonyloxy)-acetylamino)phenyl}-2-thienyl]-3,4,5,6-tetrahydro-2H-thiopyran-2-acetamide 1,1-dioxide (55 mg)

NMR (DMSO-$d_6$, δ): 1.73-2.05 (4H, m), 2.37-2.46 (1H, m), 2.94-3.26 (4H, m), 3.42-3.53 (1H, m), 4.22 (2H, d, J=5.0Hz), 4.61 (2H, s), 7.20-7.48 (10H, m), 7.93-7.97 (2H, m), 8.84 (1H, br), 10.15 (1H, s), 10.60 (1H, s)

MS (ESI-): 570.1 (M-H)

Example 450

(2S)-N-Hydroxy-2-[5-{3-(2-(3-methylphenoxy)-acetylamino)phenyl}-2-thienyl]-3,4,5,6-tetrahydro-2H-thiopyran-2-acetamide 1,1-dioxide (30 mg)

NMR (DMSO-$d_6$, δ): 1.73-2.06 (4H, m), 2.30 (3H, s), 2.37-2.47 (1H, m), 2.95-3.26 (4H, m), 3.40-3.50 (1H, m), 4.70 (2H, s), 6.70-6.75 (3H, m), 7.20-7.22 (2H, m), 7.38-7.40 (2H, m), 7.43 (1H, d, J=4.0Hz), 7.56 (1H, d, J=7.0Hz), 8.03 (1H, s), 8.84 (1H, s), 10.17 (1H, s), 10.60 (1H, s)

MS (ESI+): 565.2 (M+H+Cl)

Example 451

(2S)-N-Hydroxy-2-[5-{3-(2-(3-pyridyloxy)-acetylamino)phenyl}-2-thienyl]-3,4,5,6-tetrahydro-2H-thiopyran-2-acetamide 1,1-dioxide (50 mg)

NMR (DMSO-$d_6$, δ):1.72-2.06 (4H, m), 2.37-2.48 (1H, m), 2.96-3.27 (4H, m), 3.40-3.50 (1H, m), 4.90 (2H, s), 7.20 (1H, d, J=4.0Hz), 7.35-7.42 (3H, m), 7.52-7.56 (2H, m), 7.64-7.66 (1H, m), 8.00 (1H, s), 8.30 (1H, d, J=4.5Hz), 8.50 (1H, d, J=2.0Hz), 10.30 (1H, s), 10.60 (1H, s)

MS (ESI-): 514.1 (M-H)

Example 452

(2S)-N-Hydroxy-2-[5-{3-(2-(4-pyridyloxy)acetylamino)-phenyl}-2-thienyl]-3,4,5,6-tetrahydro-2H-thiopyran-2-acetamide 1,1-dioxide (35 mg)

NMR (DMSO-$d_6$, δ): 1.72-2.05 (4H, m), 2.35-2.44 (1H, m), 2.95-3.25 (4H, m), 3.42-3.53 (1H, m), 5.26 (2H, s), 7.07 (2H, d, J=7.0Hz), 7.20 (1H, d, J=4.0Hz), 7.40-7.42 (4H, m), 8.03 (1H, s), 8.41 (2H, d, J=7.0Hz)

MS (ESI+): 516.1 (M+H)

Example 453

(2S)-N-Hydroxy-2-[5-{3-(((2S)-2-amino-3-(4-pyridyl)-propionyl)amino)phenyl}-2-thienyl]-3,4,5,6-tetrahydro-2H-thiopyran-2-acetamide 1,1-dioxide (160 mg) L-Boc4-PyAla-OH

NMR (DMSO-$d_6$, δ): 1.72-2.06 (4H, m), 2.35-2.46 (1H, m), 2.96-3.39 (6H, m), 3.44-3.55 (1H, m), 4.30 (1H, br), 7.22 (1H, d, J=4.0Hz), 7.42-7.48 (4H, m), 7.55 (1H, d, J=6.0Hz), 7.85 (1H, s), 8.39 (2H, br), 8.70 (2H, d, J=5.5Hz),

10.60 (1H, s), 10.62 (1H, s)

MS (ESI+): 529.2 (M+H)

Example 454

(2S)-N-Hydroxy-2-[5-{3-(((2S)-2-amino-3-phenylpropionyl)amino)phenyl}-2-thienyl]-3,4,5,6-tetrahydro-2H-thiopyran-2-acetamide 1,1-dioxide (40 mg) from L-Boc-Phe-OH

NMR (DMSO-$d_6$, δ): 1.74-2.06 (4H, m), 2.35-2.44 (1H, m), 2.95-3.27 (6H, m), 3.43-3.53 (1H, m), 4.15 (1H, br), 7.22 (1H, d, J=4.0Hz), 7.25-7.35 (5H, m), 7.39-7.46 (4H, m), 7.80 (1H, s), 8.32 (2H, br), 8.85 (1H, br), 10.47 (1H, s), 10.73 (1H, s)

MS (ESI+): 528.3 (M+H)

The following compounds were obtained in a similar manner to that of Example 125.

Example 455

(2S)-N-(2-Tetrahydropyranyloxy)-2-[5-(3-(2-(methanesulfonylamino)acetylamino)phenyl)-2-thienyl]-3,4,5,6-tetrahydro-2H-thiopyran-2-acetamide 1,1-dioxide (75 mg)

NMR (DMSO-$d_6$, δ): 1.46-1.72 (8H, m), 1.87-1.99 (2H, m), 2.06-2.24 (2H, m), 2.81-2.92 (2H, m), 3.02 (3H, s), 3.05-3.21 (2H, m), 3.28-3.52 (1H, m), 3.68-3.80 (1H, m), 3.98-4.06 (2H, m), 4.53, 4.87 (1H, br s), 5.94-6.08 (1H, m), 7.06-7.21 (4H, m), 7.42-7.52 (2H, m), 8.58, 8.59 (1H, br s)

MS (ESI-): 598 (M-H)

Example 456

(2S)-N-(2-Tetrahydropyranyloxy)-2-(5-(3-(phenylacetylamino)phenyl)-2-thienyl]-3,4,5,6-tetrahydro-2H-thiopyran-2-acetamide 1,1-dioxide (132 mg)

NMR ($CDCl_3$, δ): 1.30-1.73 (8H, m), 1.84-1.97 (2H; m), 2.04-2.23 (2H, m), 2.70-2.90 (2H, m), 2.97-3.16 (2H, m), 3.26-3.51 (1H, m), 3.63-3.73 (1H, m), 3.73 (2H, s), 4.52, 4.72 (1H, br s), 7.08-7.62 (12H, m), 8.68 (1H, br s)

MS (ESI-): 580 (M-H)

Example 457

(2S)-N-(2-Tetrahydropyranyloxy)-2-[5-(3-(cyclopropanecarbonylamino)phenyl)-2-thienyl]-3,4,5,6-tetrahydro-2H-thiopyran-2-acetamide 1,1-dioxide (550 mg)

NMR (DMSO-$d_6$, δ): 0.72-0.89 (4H, m), 1.32-1.64 (6H, m), 1.67-2.06 (5H, m), 2.34-2.49 (1H, m), 2.87-3.30 (5H, m), 3.41-3.53 (1H, m), 3.72-3.91 (1H, m), 4.44, 4.75 (1H, s), 7.16-7.26 (1H, m), 7.39-7.52 (4H, m), 8.02 (1H, s)

MS (ESI-): 531 (M-H)

Example 458

(2S)-N-(2-Tetrahydropyranyloxy)-2-[5-(3-(2-(propoxycarbonylamino)acetylamino)phenyl)-2-thienyl]-3,4,5,6-tetrahydro-2H-thiopyran-2-acetamide 1,1-dioxide (93 mg)

NMR ($CDCl_3$, δ): 0.93-0.99 (3H, m), 1.44 (2H, br), 1.65-1.75 (6H, m), 1.95 (2H, br), 2.04-2.26 (2H, m), 2.72-2.92 (2H, m), 3.01-3.15 (4H, m), 3.28-3.48 (1H, m), 3.70 (1H, br), 3.95-4.12 (4H, m), 4.53 (1/2H, s), 4.84 (1/2H, s), 5.54-5.64 (1H, m), 7.12-7.23 (4H, m), 7.50 (1H, br s), 7.58 (1H, s), 8.28 (1H, br), 8.70-8.78 (1H, m)

MS (ESI-): 606.1 (M-H)

Example 459

(2S)-N-(2-Tetrahydropyranyloxy)-2-[5-(3-(2-(cyclopentyloxycarbonylamino)acetylamino)phenyl}-2-thienyl]-3,4,5,6-tetrahydro-2H-thiopyran-2-acetamide 1,1-dioxide (53 mg)

NMR ($CDCl_3$, δ): 1.44 (4H, br), 1.60-1.74 (4H, m), 1.86-1.96 (6H, m), 2.05-2.24 (2H, m), 2.70-2.89 (2H, m), 2.96-3.20 (4H, m), 3.29-3.52 (1H, m), 3.64-3.75 (1H, m), 3.94-4.02 (2H, m), 4.54 (1/2H, s), 4.83 (1/2H, s), 5.16 (1H, br), 5.45-5.54 (1H, m), 7.16-7.25 (3H, m), 7.51-7.60 (2H, m), 8.30 (1H, br)

MS (ESI-): 632.1 (M-H)

The following compounds were obtained in a similar manner to that of Example 206.

Example 460

(2S)-N-(2-Tetrahydropyranyloxy)-2-[5-(3-(2-(2-oxopyrrolidinyl)acetylamino)phenyl)-2-thienyl]-3,4,5,6-tetrahydro-2H-thiopyran-2-acetamide 1,1-dioxide (520 mg)

NMR (DMSO-$d_6$, δ): 1.32-1.63 (6H, m), 1.68-2.09 (6H, m), 2.28 (2H, t, J=7Hz), 2.35-2.48 (1H, m), 2.86-3.53 (6H, m), 3.46 (2H, t, J=7Hz), 3.22-3.40 (1H, m), 4.05 (2H, s), 4.44, 4.75 (1H, s), 7.16-7.25 (1H, m), 7.32-7.46 (4H, m), 8.02 (1H, s), 10.20 (1H, s), 11.25 (1H, s)

MS (ESI-): 588 (M-H)

Example 461

(2S)-N-(2-Tetrahydropyranyloxy)-2-[5-(3-(((3S)-N-tert-butoxycarbonyl-1,2,3,4-tetrahydroisoquinoline-3-carbonyl)amino)phenyl)-2-thienyl]-3,4,5,6-tetrahydro-2H-thiopyran-2-acetamide 1,1-dioxide (135 mg)

NMR (DMSO-$d_6$, δ): 1.18-2.06 (10H, m), 1.31 (9H, s), 2.34-2.47 (1H, m), 2.88-3.31 (7H, m), 3.38-3.54 (1H, m), 3.72-3,88 (1H, m), 4.36-4.85 (4H, m), 7.14-7.50 (9H, m), 7.88-8.00 (1H, m), 10.16 (1H, s), 11.24 (1H, s)

MS (ESI-): 722 (M-H)

Example 462

(2S)-N-(2-Tetrahydropyranyloxy)-2-[5-(3-(oxolane-2-carbonylamino)phenyl)-2-thienyl]-3,4,5,6-tetrahydro-2H-thiopyran-2-acetamide 1,1-dioxide (670 mg)

NMR (DMSO-$d_6$, δ): 1.38-1.62 (6H, m), 1.70-2.08 (7H, m), 2.14-2.78 (1H, m), 2.36-2.50 (1H, m), 2.90-3.32 (5H, m), 3.40-3.53 (1H, m), 3.74-3.90 (1H, dd, J=7Hz), 4.02 (1H, dd, J=7Hz), 4.42 (1H, dd, J=7Hz), 4.45, 4.75 (1H, s), 7.19-7.25 (1H, m), 7.31-7.44 (3H, m), 7.65 (1H, d, J=8Hz), 8.07 (1H, s), 9.78 (1H, s), 11.25 (1H, s)

MS (ESI-): 561 (M-H)

Example 463

(2S)-N-(2-Tetrahydropyranyloxy)-2-[5-(3-(oxolane-3-carbonylamino)phenyl)-2-thienyl]-3,4,5,6-tetrahydro-2H-thiopyran-2-acetamide 1,1-dioxide (700 mg)

NMR (DMSO-$d_6$, δ): 1.33-1.63 (6H, m), 1.69-2.15 (4H, m), 2.09 (2H, dd, J=8Hz), 2.35-2.48 (1H, m), 2.86-3.32 (4H, m), 3.39-3.56 (1H, m), 3.65-3.86 (4H, m), 3.88-4.00 (1H, m), 4.45, 4.75 (1H, s), 7.18-7.25 (1H, m), 7.30-7.52 (4H, m), 8.02 (1H, s), 10.15 (1H, s), 11.24, 11.25 (1H, s)

MS (ESI-): 561 (M-H)

Example 464

(2S)-N-(2-Tetrahydropyranyloxy)-2-[5-(3-(2-(ethylaminocarbonylamino)acetylamino)phenyl}-2-thienyl]-3,4,5,6-tetrahydro-2H-thiopyran-2-acetamide 1,1-dioxide (4.7 g)

NMR ($CDCl_3$, δ): 0.85-0.93 (3H, m), 1.45 (4H, br), 1.55-1.68 (2H, m), 1.92 (2H, br), 2.01-2.25 (2H, m), 2.92-3.10 (7H, m), 3.26-3.75 (1H, m), 3.44-3.54 (2H, m), 3.68-4.02 (2H, m), 4.30-4.40 (1H, m), 4.65 (1/2H, br), 4.92 (1/2H, br), 6.02 (1H, br), 7.07-7.26 (7H, m), 7.54 (1/2H, s), 7.60 (1/2H, s), 9.27 (1/2H, s), 9.32 (1/2H, s)

MS (ESI-): 591.2 (M-H)

Example 465

(2S)-N-(2-Tetrahydropyranyloxy)-2-[5-(3-isovalerylaminophenyl)-2-thienyl]-3,4,5,6-tetrahydro-2H-thiopyran-2-acetamide 1,1-dioxide (200 mg)

NMR ($CDCl_3$, δ): 1.04 (6H, d, J=6.0Hz), 1.16 (2H, d, J=7.5Hz), 1.45 (2H, br), 1.65-1.75 (2H, m), 1.92-1.95 (2H, m), 2.07-2.30 (2H, m), 2.28 (2H, br), 2.30-2.40 (1H, m), 2.71-2.90 (2H, m), 3.04-3.15 (4H, m), 3.30-3.50 (1H, m), 3.64-3.75 (1H, m), 4.52 (1/2H, br s), 4.82 (1/2H, br s), 7.20-7.30 (3H, m), 7.39-7.46 (1H, m), 7.50-7.57 (2H, m), 7.62-7.66 (1H, m), 8.37-8.47 (1H, m)

MS (ESI-): 547.1 (M-H)

Example 466

(2S)-N-(2-Tetrahydropyranyloxy)-2-[5-{3-(2-(methoxyacetylamino)acetylamino)phenyl}-2-thienyl]-3,4,5,6-tetrahydro-2H-thiopyran-2-acetamide 1,1-dioxide (195 mg)

NMR ($CDCl_3$, δ): 1.45 (2H, br), 1.67 (4H, br), 1.97 (2H, br), 2.07-2.24 (2H, m), 2.80-2.87 (2H, m), 3.01-3.06 (1H, m), 3.11-3.20 (3H, m), 3.30-3.44 (1H, m), 3.46 (3H, s), 3.68-3.78 (1H, m), 4.04 (2H, s), 4.09-4.13 (1H, m), 4.25-4.32 (1H, m), 4.53 (1/2H, s), 4.84 (1/2H, s), 7.08-7.24 (3H, m), 7.42 (2H, br), 7.48-7.54 (2H, m), 8.56 (1H, br), 9.08 (1/2H, s), 9.18 (1/2H, br s)

MS (ESI+): 591.1 (M+H)

Example 467

(2S)-N-(2-Tetrahydropyranyloxy)-2-[5-(3-(2-(3-pyridylcarbonylamino)acetylamino)phenyl}-2-thienyl]-3,4,5,6-tetrahydro-2H-thiopyran-2-acetamide 1,1-dioxide (90 mg)

NMR ($CDCl_3$, δ): 1.36 (2H, br), 1.65 (4H, br), 1.94 (2H, br), 2.06-2.23 (2H, m), 2.80-3.20 (7H, m), 3.55-3.72 (1H, m), 4.36 (1/2H, br s), 4.37-4.65 (2H, m), 4.86 (1/2H, s), 7.13-7.30 (5H, m), 7.43-7.49 (1H, m), 7.54 (1H, br s), 7.75-7.81 (1H, m), 8.23 (1H, d, J=7.5Hz), 8.76 (1H, br), 9.27 (1/2H, s), 9.31-9.34 (1H, m), 9.40 (1/2H, s)

MS (ESI+): 625.1 (M+H)

Example 468

(2S)-N-(2-Tetrahydropyranyloxy)-2-[5-{3-(methoxymethylaminocarbonylamino)phenyl}-2-thienyl]-3,4,5,6-tetrahydro-2H-thiopyran-2-acetamide 1,1-dioxide (100 mg) was obtained in a similar manner to that of Example 343.

NMR ($CDCl_3$, δ): 1.25 (2H, br), 1.43 (2H, br), 1.64 (2H, br), 1.93 (2H, br), 2.05-2.22 (2H, m), 2.70-2.88 (2H, m), 3.00-3.13 (4H, m), 3.37 (3H, s), 3.45-3.52 (4H, m), 4.55 (1/2H, br), 4.81 (1/2H, br), 5.40-5.49 (1H, m), 7.08-7.20 (6H, m), 7.27-7.40 (1H, m), 8.91 (1/2H, br), 8.98 (1/2H, br)

MS (ESI-): 567.1 (M-H+$NH_3$)

Example 469

(2S)-N-(2-Tetrahydropyranyloxy)-2-[5-{3-(2-(3-pyridylmethylamino)acetylamino)phenyl}-2-thienyl]-3,4,5,6-tetrahydro-2H-thiopyran-2-acetamide 1,1-dioxide (100 mg) was obtained in a similar manner to that of Example 249.

NMR ($CDCl_3$, δ): 1.42 (2H, br), 1.63-1.68 (4H, m), 1.95 (2H, br), 2.07-2.23 (2H, m), 2.68-2.95 (2H, m), 3.08 (2H, br.s), 3.13 (2H, br), 3.27-3.44 (1H, m), 3.46 (2H, s), 3.62-3.70 (1H, m), 3.90 (2H, s), 4.52 (1/2H, br), 4.82 (1/2H, br), 7.24-7.34 (6H, m), 7.53-7.57 (1H, m), 7.65-7.70 (2H, m), 8.57- (1H, d, J=5.0Hz), 8.62 (1/2H, br), 8.65 (1H, s), 8.90 (1/2H, s), 9.17 (1H, d, J=6.0Hz)

MS (ESI+): 613.2 (M+H)

Example 470

(2S)-N-(2-Tetrahydropyranyloxy)-2-[5-{3-(2-(tert-butylamino)acetylamino)phenyl}-2-thienyl]-3,4,5,6-tetrahydro-2H-thiopyran-2-acetamide 1,1-dioxide (95 mg) was obtained in a similar manner to that of Example 244.

NMR ($CDCl_3$, δ): 1.17 (9H, s), 1.46 (2H, br), 1.65-1.70 (4H, m), 1.96 (2H, br), 2.09-2.23 (2H, m), 2.64-2.86 (2H, m), 3.06 (2H, br s), 3.10-3.15 (2H, m), 3.36 (2H, br s), 3.43-3.70 (2H, m), 4.53 (1/2H, br), 4.80 (1/2H, br), 7.22-7.34 (5H, m), 7.56-7.60 (1H, m), 7.78 (1H, s), 9.53 (1H, s)

MS (ESI-): 593.6 (M-H+$NH_3$)

The following compounds were obtained in a similar manner to that of Example 52.

Example 471

(2S)-N-Hydroxy-2-[5-(3-(2-(N-methanesulfonylamino)-acetylamino)phenyl)-2-thienyl]-3,4,5,6-tetrahydro-2H-thiopyran-2-acetamide 1,1-dioxide (32 mg)

NMR (DMSO-$d_6$, δ): 1.72-2.08 (4H, m), 2.33-2.48 (1H, m), 2.92-3.56 (7H, m), 3.00 (3H, s), 3.87 (1H, d, J=7Hz), 7.21 (1H, d, J=3Hz), 7.34-7.52 (4H, m), 7.96 (1H, s), 8.84 (1H, s)

MS (ESI-): 514 (M-H)

Example 472

(2S)-N-Hydroxy-2-[5-(3-phenylacetylamino)phenyl)-2-thienyl]-3,4,5,6-tetrahydro-2H-thiopyran-2-acetamide 1,1-dioxide (92 mg)

NMR (DMSO-$d_6$, δ): 1.67-2.08 (4H, m), 2.32-2.51 (1H, m), 2.89-3.54 (5H, m), 3.66 (2H, s), 7.17-7.53 (11H, m), 8.00 (1H, s), 10.30 (1H, s), 10.60 (1H, s)

MS (ESI-): 497 (M-H)

Example 473

(2S)-N-Hydroxy-2-[5-(3-(2-(2-oxopyrrolidinyl)-acetylamino)phenyl)-2-thienyl]-3,4,5,6-tetrahydro-2H-thiopyran-2-acetamide 1,1-dioxide (420 mg)

NMR (DMSO-$d_6$, δ): 1.68-2.12 (6H, m), 2.19-2.48 (3H, m), 2.90-3.61 (7H, m), 4.06 (2H, s), 7.20 (1H, d, J=3Hz), 7.31-7.52 (5H, m), 8.02 (1H, s), 10.23 (1H, s), 10.62 (1H, s)

MS (ESI-): 504 (M-H)

Example 474

(2S)-N-Hydroxy-2-[5-(3-(((3S)-1,2,3,4-tetrahydroisolquinoline-3-carbonyl)amino)phenyl)-2-thienyl]-3,4,5,6-tetrahydro-2H-thiopyran-2-acetamide 1,1-dioxide hydrochloride (88 mg) from (2S)-N-(2-tetrahydropyranyloxy-2-[5-(3-(((3S)-N-tert-butoxycarbonyl-1,2,3,4-tetrahydroisoquinoline-3-carbonyl)amino)phenyl)-2-thienyl]-3,4,5,6-tetrahydro-2H-thiopyran-2-acetamide 1,1-dioxide

NMR (DMSO-$d_6$, δ): 1.66-2.14 (4H, m), 2.32-2.48 (1H, m), 2.86-3.92 (7H, m), 4.26-4.51 (3H, m), 7.12-7.52 (8H, m), 7.56-7.68 (1H, m), 8.03 (1H, s), 9.55 (1H, br s), 9.96 (1H, br s), 10.67 (1H, s), 11.22 (1H, s)

MS (ESI+): 540 (M+H)

Example 475

(2S)-N-Hydroxy-2-[5-(3-(cyclopropanecarbonylamino)-phenyl)-2-thienyl]-3,4,5,6-tetrahydro-2H-thiopyran-2-acetamide 1,1-dioxide (410 mg)

NMR (DMSO-$d_6$, δ): 0.70-0.90 (4H, m), 1.66-2.10 (5H, m), 2.34-2.49 (1H, m), 3.42-3.55 (1H, m), 7.21 (1H, d, J=3Hz), 7.28-7.39 (2H, m), 7.40 (1H, d, J=3Hz), 7.42-7.52 (1H, m), 8.01 (1H, s), 10.33 (1H, s), 10.61 (1H, s)

MS (ESI-): 447 (M-H)

Example 476

(2S)-N-Hydroxy-2-[5-(3-(oxolane-2-carbonylamino)-phenyl)-2-thienyl]-3,4,5,6-tetrahydro-2H-thiopyran-2-acetamide 1,1-dioxide (340 mg)

NMR (DMSO-$d_6$, δ): 1.71-2.07 (7H, m), 2.14-2.27 (1H, m), 2.93-3.27 (4H, m), 3.40-3.55 (1H, m), 3.84 (1H, dd, J=7Hz), 4.00 (1H, dd, J=7Hz), 4.41 (1H, d, J=7Hz), 7.21 (1H, d, J=3Hz), 7.32-7.44 (3H, m), 7.65 (1H, d, J=8Hz), 8.07 (1H, s), 8.85 (1H, s), 9.78 (1H, s), 10.60 (1H, s)

MS (ESI-): 477 (M-H)

Example 477

(2S)-N-Hydroxy-2-[5-(3-(oxolane-3-carbonylamino)-phenyl)-2-thienyl]-3,4,5,6-tetrahydro-2H-thiopyran-2-acetamide 1,1-dioxide (541 mg)

NMR (DMSO-$d_6$, δ): 1.68-2.20 (4H, m), 2.09 (2H, dd, J=8Hz), 2.24-2.49 (1H, m), 2.92-3.31 (4H, m), 3.43-3.54 (1H, m), 3.65-3.76 (4H, m), 3.95 (1H, t, J=8Hz), 7.21 (1H, d, J=3Hz), 7.30-7.54 (4H, m), 8.02 (1H, s), 10.17 (1H, s), 10.61 (1H, s)

MS (ESI-): 477 (M-H)

Example 478

(2S)-N-Hydroxy-2-[5-(3-(2-(ethylaminocarbonylamino)-acetylamino)phenyl}-2-thienyl]-3,4,5,6-tetrahydro-2H-thiopyran-2-acetamide 1,1-dioxide (3.0 mg)

NMR (DMSO-$d_6$, δ): 1.00 (3H, t, J=7.5Hz), 1.73-2.05 (4H, m), 2.38-2.46 (1H, m), 2.95-3.25 (6H, m), 3.43-3.53 (1H, m), 3.83 (2H, d, J=6.0Hz), 6.10-6.18 (2H, m), 7.20 (1H, d, J=4.0Hz), 7.35-7.37 (2H, m), 7.40 (1H, d, J=4.0Hz), 7.46-7.50 (1H, m), 7.97 (1H, s), 8.84 (1H, s)

MS (ESI-): 507.2 (M-H)

Example 479

(2S)-N-Hydroxy-2-[5-(3-isovalerylaminophenyl)-2-thienyl]-3,4,5,6-tetrahydro-2H-thiopyran-2-acetamide 1,1-dioxide (120 mg)

NMR (DMSO-$d_6$, δ): 0.95 (6H, d, J=7.5Hz), 1.74-2.15 (5H, m), 2.20 (2H, d, J=7.0Hz), 2.38-2.45 (1H, m), 2.95-3.26 (4H, m), 3.40-3.53 (1H, m), 7.20 (1H, d, J=4.0Hz), 7.32-7.37 (2H, m), 7.46-7.50 (1H, m), 8.00 (1H, s), 9.97 (1H, s), 10.60 (1H, s)

MS (ESI-): 463.0 (M-H)

Example 480

(2S)-N-Hydroxy-2-[5-(3-(methoxymethylaminocarbonylamino)phenyl)-2-thienyl]-3,4,5,6-tetrahydro-2H-thiopyran-2-acetamide 1,1-dioxide (70 mg)

NMR (DMSO-$d_6$, δ): 1.73-2.04 (4H, m), 2.35-2.45 (1H, m), 2.95-3.28 (6H, m), 3.26 (3H, s), 3.37-3.42 (1H, m), 6.24 (1H, t, J=7.0Hz), 7.16-7.27 (4H, m), 7.37 (1H, d, J=4.0Hz), 7.83 (1H, s), 8.67 (1H, s)

MS (ESI-): 466.4 (M-H)

Example 481

(2S)-N-Hydroxy-2-[5-{3-(2-(3-pyridylmethylamino)-acetylamino)phenyl}-2-thienyl]-3,4,5,6-tetrahydro-2H-thiopyran-2-acetamide 1,1-dioxide (60 mg)

NMR (DMSO-$d_6$, δ): 1.72-2.06 (4H, m), 2.35-2.45 (1H, m), 2.97-3.28 (4H, m), 3.42-3.50 (1H, m), 3.98 (2H, br), 4.35 (2H, br), 7.22 (1H, d, J=4.0Hz), 7.38-7.43 (2H, m), 7.48-7.52 (1H, m), 7.68-7.73 (1H, m), 7.93 (1H, s), 8.23 (1H, d, J=7.0Hz), 8.74 (1H, d, J=6.0Hz), 8.85 (1H, s), 9.68 (1H, br)

MS (ESI-): 527.1 (M-H)

Example 482

(2S)-N-Hydroxy-2-[5-(3-(2-(propoxycarbonylamino)-acetylamino)phenyl}-2-thienyl]-3,4,5,6-tetrahydro-2H-thiopyran-2-acetamide 1,1-dioxide (70 mg)

NMR (DMSO-$d_6$, δ): 0.91 (3H, t, J=7.0Hz), 1.58 (2H, qt, J=7.0, 7.0Hz), 1.73-2.05 (4H, m), 2.37-2.48 (1H, m), 2.94-3.27 (4H, m), 3.44-3.53 (1H, m), 3.79 (2H, d, J=6.0Hz), 3.93 (2H, t, J=7.0Hz), 7.21 (1H, d, J=4.0Hz), 7.35-7.42 (4H, m), 7.45-7.48 (1H, m), 7.97 (1H, s), 8.84 (1H, s), 10.08 (1H, s), 10.61 (1H, s)

MS (ESI-): 522.1 (M-H)

Example 483

(2S)-N-Hydroxy-2-[5-{3-(2-(cyclopentyloxycarbonylamino)acetylamino)phenyl)-2-thienyl]-3,4,5,6-tetrahydro-2H-thiopyran-2-acetamide 1,1-dioxide (30 mg)

NMR (DMSO-$d_6$, δ): 1.50-1.70 (6H, m), 1.75-2.05 (6H, m), 2.37-2.48 (1H, m), 2.95-3.25 (4H, m), 3.43-3.53 (1H, m), 3.77 (2H, d, J=5.0Hz), 4.97 (1H, br), 7.20 (1H, d, J=4.0Hz), 7.30 (1H, t, J=7.0Hz), 7.35-7.38 (2H, m), 7.41 (1H, d, J=4.0Hz), 7.45-7.48 (1H, m), 7.98 (1H, s), 8.83 (1H, br), 10.07 (1H, s), 10.60 (1H, br)

MS (ESI-): 548.1 (M-H)

Example 484

(25)-N-Hydroxy-2-[5-(3-(2-(methoxyacetylamino)phenyl)-2-thienyl]-3,4,5,6-tetrahydro-2H-thiopyran-2-acetamide 1,1-dioxide (70 mg)

NMR (DMSO-$d_6$, δ) : 1.74-2.05 (4H, m), 2.36-2.48 (1H, m), 2.95-3.26 (4H, m), 3.37 (3H, s), 3.43-3.54 (1H, m), 3.39 (2H, s), 3.45 (2H, d, J=6.0Hz), 7.20 (1H, d, J=4.0Hz), 7.36-7.40 (2H, m), 7.42 (1H, d, J=4.0Hz), 7.43-7.48 (1H, m), 7.94 (1H, s), 8.05 (1H, t, J=6.0Hz), 8.84 (1H, s), 10.13 (1H, s), 10.60 (1H, s)

MS (ESI-): 508.1 (M-H)

Example 485

(2S)-N-Hydroxy-2-[5-{3-(2-(tert-butylamino)-acetylamino)phenyl}-2-thienyl]-3,4,5,6-tetrahydro-2H-thiopyran-2-acetamide 1,1-dioxide (50 mg)

NMR (DMSO-$d_6$, δ): 1.32 (9H, s), 1.72-2.06 (4H, m), 2.35-2.46 (1H, m), 2.96-3.28 (4H, m), 3.38-3.52 (1H, m),

3.96 (2H, t, J=7.0Hz), 7.22 (1H, d, J=4.0Hz), 7.41-7.44 (3H, m), 7.51-7.54 (1H, m), 7.97 (1H, s), 8.84 (1H, br), 8.95-8.98 (2H, br)

MS (ESI+): 494.1 (M+H)

Example 486

(2S)-N-Hydroxy-2-[5-{3-(2-(3-pyridylcarbonylamino)-acetylamino)phenyl}-2-thienyl]-3,4,5,6-tetrahydro-2H-thiopyran-2-acetamide 1,1-dioxide (60 mg)

NMR (DMSO-$d_6$, δ): 1.72-2.04 (4H, m), 2.37-2.45 (1H, m), 2.95-3.25 (4H, m), 3.40-3.52 (1H, m), 4.00-4.06 (2H, br), 4.13 (2H, d, J=7.0Hz), 7.20 (1H, d, J=4.0Hz), 7.32-7.39 (2H, m), 7.42 (1H, d, J=4.0Hz), 7.46-7.50 (1H, m), 7.59-7.64 (1H, m), 8.02 (1H, s), 8.30-8.33 (1H, m), 8.77 (1H, d, J=6.0Hz), 9.10 (1H, s), 9.15 (1H, t, J=7.5Hz)

MS (ESI-): 527.3 (M-H)

Example 487

(2S)-N-Hydroxy-2-[5-{3-(3,3-dimethylbutyrylamino)-phenyl)-2-thienyl]-3,9,5,6-tetrahydro-2H-thiopyran-2-acetamide 1,1-dioxide (160 mg) was obtained from 3,3-dimethylbutyric acid in a similar manner to that of Example 403.

NMR (DMSO-$d_6$, δ): 1.03 (9H, s), 1.66-2.10 (4H, m), 2.21 (2H, s), 2.34-2.48 (1H, m), 2.92-3.29 (4H, m), 3.42-3.56 (1H, m), 7.20 (1H, d, J=3Hz), 7.28-7.38 (2H, m), 7.40 (1H, d, J=3Hz), 7.45-7.54 (1H, m), 7.99 (1H, s), 9.93 (1H, s), 10.61 (1H, s)

MS (ESI-): 477 (M-H)

Example 488

(2S)-N-Hydroxy-2-[5-(4-ethoxyphenyl)-2-thienyl]-3,4,5,6-tetrahydro-2H-thiopyran-2-acetamide 1,1-dioxide (8.5 mg) was obtained in a similar manner to that of Example 163.

NMR (DMSO-$d_6$, δ): 1.35 (3H, t, J=7.0Hz), 1.73-2.04 (4H, m), 2.34-2.44 (1H, m), 2.95-3.25 (4H, m), 3.62-3.68 (1H, m), 4.06 (2H, q, J=7.0Hz), 6.97 (2H, d, J=7.5Hz), 7.16 (1H, d, J=4.0Hz), 7.33 (1H, d, J=4.0Hz), 7.56 (1H, d, J=7.5Hz)

MS (ESI+): 410.2 (M+H)

The following compounds were obtained in a similar manner to that of Example 125.

Example 489

(2S)-N-(2-Tetrahydropyranyloxy)-2-[5-(3-(cyclobutylcarbonylamino)phenyl)-2-thienyl]-3,4,5,6-tetrahydro-2H-thiopyran-2-acetamide 1,1-dioxide (340 mg)

NMR (DMSO-$d_6$, δ): 1.37-1.65 (6H, m), 1.70-2.30 (10H, m), 2.37-2.46 (1H, m), 2.90-3.55 (7H, m), 3.75-3.89 (1H, m), 4.44, 4.75 (1H, s), 7.19-7.22 (1H, m), 7.34-7.40 (3H, m), 7.45-7.51 (1H, m), 8.03 (1H, s), 9.83 (1H, s)

Example 490

(2S)-N-(2-Tetrahydropyranyloxy)-2-[5-(3-(isobutyrylaminophenyl)-2-thienyl]-3,4,5,6-tetrahydro-2H-thiopyran-2-acetamide 1,1-dioxide (330 mg)

NMR (DMSO-$d_6$, δ): 1.11 (6H, d, J=7.0Hz), 1.36-1.64 (6H, m), 1.70-2.05 (4H, m), 2.35-2.45 (1H, m), 2.55-2.64 (1H, m), 2.90-3.54 (6H, m), 3.75-3.90 (1H, m), 4.45, 4.75 (1H, s), 7.20-7.23 (1H, m), 7.33-7.41 (3H, m), 7.45-7.51 (1H, m), 8.03 (1H, s)

Example 491

(2S)-N-(2-Tetrahydropyranyloxy)-2-[5-(3-(2,2-dimethylpropionylamino)phenyl]-2-thienyl]-3,4,5,6-tetrahydro-2H-thiopyran-2-acetamide 1,1-dioxide (267 mg)

NMR (DMSO-$d_6$, δ): 1.24 (9H, s), 1.36-1.64 (6H, m), 1.69-2.09 (4H, m), 2.36-2.53 (1H, m), 2.88-3.29 (4H, m), 3.30-3.51 (2H, m), 3.74-3.91 (1H, m), 4.44 (0.5H, s), 4.76 (0.5H, s), 7.19-7.24 (1H, m), 7.30-7.42 (3H, m), 7.59-7.65 (1H, m), 8.01 (1H, s), 9.30 (1H, s)

MS (ESI-): 547 (M-H)

Example 492

(2S)-N-(2-Tetrahydropyranyloxy)-2-[5-(3-((E)-2-butenoylamino)phenyl]-2-thienyl]-3,4,5,6-tetrahydro-2H-thiopyran-2-acetamide 1,1-dioxide (160 mg)

NMR ($CDCl_3$, δ): 1.37-1.76 (6H, m),. 1.86-2.02 (5H, m), 2.04-2.26 (2H, m), 2.68-2.94 (2H, m), 3.00-3.19 (4H, m), 3.30-3.51 (1H, m), 3.62-3.76 (1H, m), 4.55 (0.5H, s), 4.83 (0.5H, s), 6.00 (1H, dd, J=1.5, 15Hz), 6.94-7.09 (1H, m), 7.13-7.33 (4H, m), 7.49-7.67 (3H, m), 8.45-8.55 (1H, m)

MS (ESI-): 531 (M-H)

The following compounds were obtained in a similar manner to that of Example 196.

Example 493

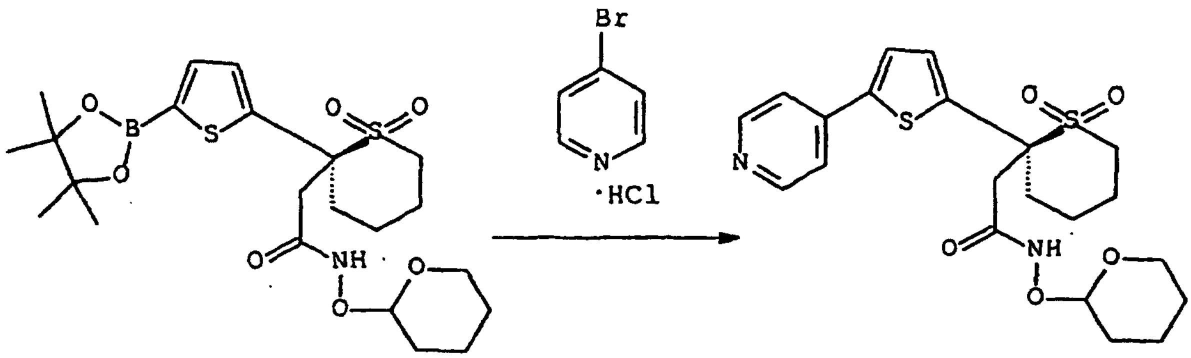


(2S)-N-(2-Tetrahydropyranyloxy)-2-[5-(4-pyridyl)-2-thienyl]-3,4,5,6-tetrahydro-2H-thiopyran-2-acetamide 1,1-dioxide (60 mg)

NMR ($CDCl_3$, δ): 1.40-1.76 (6H, m), 1.86-2.00 (2H, m), 2.06-2.25 (2H, m), 2.71-2.93 (2H, m), 3.00-3.19 (4H, m), 3.25-3.55 (1H, m), 3.60-3.799 (1H, m), 4.50 (0.5H, s), 4.84 (0.5H, s), 7.16-7.35 (1H, m), 7.44-7.49 (3H, m), 8.21 (0.5H, s), 8.28 (0.5H, s), 8.59 (2H, d, J=8Hz)

MS (ESI+): 451 (M+H)

Example 494

(2S)-N-(2-Tetrahydropyranyloxy)-2-[5-(4-(methylaminocarbonylmethyl)phenyl]-2-thienyl]-3,4,5,6-tetrahydro-2H-thiopyran-2-acetamide 1,1-dioxide (67 mg)

MS (ESI-): 519 (M-H)

Example 495

(2S)-N-(2-Tetrahydropyranyloxy)-2-[5-(2-(methylaminocarbonyl)-5-benzofuranyl]-2-thienyl]-3,4,5,6-tetrahydro-2H-thiopyran-2-acetamide 1,1-dioxide (283 mg)

NMR ($CDCl_3$, δ): 1.38-1.76 (6H, m), 1.88-2.02 (2H, m), 2.04-2.25 (2H, m), 2.65-2.93 (2H, m), 3.01-3.18 (7H, m), 3.26-3.51 (1H, m), 3.60-3.74 (1H, m), 4.55 (0.5H, s), 4.83 (0.5H, s), 6.61-6.71 (1H, m), 7.21-7.31 (2H, m), 7.43-7.50 (2H, m), 7.60-7.66 (1H, m), 7.83-7.88 (1H, m), 8.20 (0.5H, s), 8.27 (0.5H, s)

Example 496

(2S)-N-(2-Tetrahydropyranyloxy)-2-[5-(4-methylthiophenyl)-2-thienyl]-3,4,5,6-tetrahydro-2H-thiopyran-2-acetamide 1,1-dioxide (129 mg) was obtained in a similar manner to that of Example 89.

NMR ($CDCl_3$, δ): 1.38-1.76 (6H, m), 1.86-2.00 (2H, m), 2.05-2.24 (2H, m), 2.51 (3H, s), 2.61-2.90 (2H, m), 3.03-3.17 (4H, m), 3.26-3.50 (1H, m), 3.56-3.70 (1H, m), 4.52 (0.5H, s), 4.79 (0.5H, s), 7.20-7.36 (3H, m), 7.45-7.54 (2H, m), 7.62-7.68 (1H, m), 7.98 (0.5H, s), 8.09-8.15 (0.5H, m)

Example 497

(2S)-N-(2-Tetrahydropyranyloxy)-2-[5-(4-methanesulfonyl)phenyl]-2-thienyl]-3,4,5,6-tetrahydro-2H-thiopyran-2-acetamide 1,1-dioxide (57 mg) was obtained from (2S)-N-(2-tetrahydropyranyloxy)-2-[5-(4-methylthiophenyl)-2-thienyl]-3,4,5,6-tetrahydro-2H-thiopyran-2-acetamide 1,1-dioxide in a similar manner to that of Preparation 1-4).

NMR ($CDCl_3$, δ): 1.40-1.59 (4H, m), 1.62-1.74 (2H, m), 1.88-2.02 (2H, m), 2.05-2.25 (2H, m), 2.76-2.88 (2H, m), 3.04-3.20 (7H, m), 3.40-3.54 (1H, m), 3.60-3.77 (1H, m), 4.54 (0.5H, s), 4.82 (0.5H, s), 7.26-7.35 (1H, m), 7.37-7.43 (1H, m), 7.73-7.80 (2H, m), 7.90-7.97 (2H, m), 8.14 (0.5H, s), 8.20 (0.5H, s)

The following compounds were obtained in a similar manner to that of Example 52.

Example 498

(2S)-N-Hydroxy-2-[5-/(4-methanesulfonyl)phenyl]-2-thienyl]-3,4,5,6-tetrahydro-2H-thiopyran-2-acetamide 1,1-dioxide (27 mg)

NMR (DMSO-$d_6$, δ): 1.72-2.10 (4H, m), 2.36-2.55 (1H, m), 2.96-3.08 (2H, m), 3.12-3.36 (5H, m), 3.39-3.56 (1H, m), 7.28 (1H, d, J=3.9Hz), 7.70 (1H, d, J=3.9Hz), 7.91 (2H, d, J=8Hz), 7.96 (2H, d, J=8Hz), 8.86 (1H, s)

MS (ESI-): 442 (M-H)

Example 499

(2S)-N-Hydroxy-2-[5-(4-methylaminocarbonylmethyl)-phenyl]-2-thienyl]-3,4,5,6-tetrahydro-2H-thiopyran-2-acetamide 1,1-dioxide (25 mg)

NMR (DMSO-$d_6$, δ): 1.70-2.06 (4H, m), 2.34-2.52 (1H, m), 2.59 (3H, d, J=4.5Hz), 2.95-3.34 (4H, m), 3.36-3.54 (3H, m), 7.20 (1H, d, J=3.9Hz), 7.29 (2H, d, J=8Hz), 7.43 (1H, d, J=3.9Hz), 7.56 (1H, d, J=8Hz), 7.94-8.02 (1H, m), 8.85 (1H, s)

MS (ESI-): 435 (M-H)

Example 500

(2S)-N-Hydroxy-2-[5-(4-pyridyl)-2-thienyl]-3,4,5,6-tetrahydro-2H-thiopyran-2-acetamide 1,1-dioxide (3.1 mg)

MS (ESI+): 367 (M-H)

Example 501

(2S)-N-Hydroxy-2-[5-(2-(methylaminocarbonyl)-5-benzofuranyl)-2-thienyl]-3,4,5,6-tetrahydro-2H-thiopyran-2-acetamide 1,1-dioxide hydrochloride (119 mg)

NMR (DMSO-$d_6$, δ): 1.72-2.10 (4H, m), 2.36-2.56 (1H, m), 2.81 (3H, d, J=4.8Hz), 2.95-3.31 (4H, m), 3.40-3.55 (1H, m), 7.22 (1H, d, J=3.9Hz), 7.49 (1H, d, J=3.9Hz), 7.53 (1H, s), 7.69 (1H, d, J=8Hz), 7.75 (1H, d, J=8Hz), 8.03 (1H, s), 8.73 (1H, q, J=4.8Hz0, 8.86 (1H, br s), 10.60 (1H, br s)

MS (ESI-): 461 (M-H)

Example 502

(2S)-N-(2-Tetrahydropyranyloxy)-2-[5-[4-(methanesulfinyl)phenyl]-2-thienyl]-3,4,5,6-tetrahydro-2H-thiopyran-2-acetamide 1,1-dioxide (45 mg) was obtained from (2S)-N-(2-tetrahydropyranyloxy)-2-[5-(4-methylthiophenyl)-2-thienyl]-3,4,5,6-tetrahydro-2H-thiopyran-2-acetamide 1,1-dioxide in a similar manner to that of Preparation 1-4).

NMR ($CDCl_3$, δ): 1.38-1.76 (6H, m), 1.84-2.01 (2H, m),2.05-2.25 (2H, m), 2.77 (3H, s), 2.77-2.88 (2H, m), 3.00-3.18 (4H, m), 3.30-3.54 (1H, m), 3.64-3.79 (1H, m), 4.53 (0.5H, s), 4.83 (0.5H, s), 7.25-7.34 (2H, m), 7.65 (2H, d, J=8Hz), 7.70-7.78 (2H, m), 8.66 (0.5H, s), 8.71 (0.5H, s)

MS (ESI-): 510 (M-H)

Example 503

(2S)-N-Hydroxy-2-[5-[4-(methanesulfinyl)phenyl]-2-thienyl]-3,4,5,6-tetrahydro-2H-thiopyran-2-acetamide 1,1-dioxide (11 mg) was obtained in a similar manner to that of Example 52.

NMR (DMSO-$d_6$, δ): 1.71-2.10 (4H, m), 2.35-2.55 (1H, m), 2.78 (3H, s), 2.95-3.08 (2H, m), 3.11-3.38 (2H, m), 3.41-3.55 (1H, m), 7.25 (1H, d, J=3.9Hz), 7.61 (1H, d, J=3.9Hz), 7.72 (2H, d, J=8Hz), 7.85 (2H, d, J=8Hz), 8.85 (1H, s)

MS (ESI-): 426 (M-H)

## Claims

**1.** A compound of the formula:

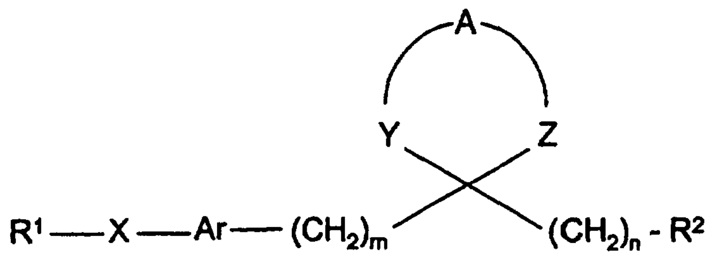

in which $R^1$ is ($C_1$-$C_6$) alkyl, optionally substituted heterocyclic group or optionally substituted ($C_6$-$C_{10}$) aryl,
$R^2$ is carboxy, protected carboxy, hydroxyaminocarbonyl, tetrahydropyranyloxyaminocarbonyl, or phenyl ($C_1$-$C_6$) alkylaminocarbonyl,
Ar is optionally substituted ($C_6$-$C_{10}$)aryl or optionally substituted heterocyclic group,
A is ($C_1$-$C_6$) alkylene,
X is oxa or a single bond,
Y is thia, sulfinyl or sulfonyl,
Z is methylene,
m and n are each an integer of 0 to 6, and
$1 \leq m+n \leq 6$,
and its salt,
wherein the heterocyclic group of $R^1$ and Ar are selected from the group consisting of the following (1) to (14),

(1 ) unsaturated 3- to 8-membered, heteromonocyclic group containing 1 to 4 nitrogen atoms,
(2) saturated 3- to 8-membered, heteromonocyclic group containing 1 to 4 nitrogen atoms,
(3) unsaturated 3- to 8-membered, heteromonocyclic group containing 1 or 2 sulfur atoms,
(4) unsaturated condensed 7- to 13-membered, heterocyclic group containing 1 to 5 nitrogen atoms,
(5) unsaturated 3- to 8-membered, heteromonocyclic group containing 1 or 2 oxygen atoms,
(6) saturated 3- to 8-membered, heteromonocyclic group containing 1 or 2 oxygen atoms,
(7) unsaturated 3- to 8-membered, heteromonocyclic group containing 1 or 2 oxygen atoms and 1 to 3 nitrogen atoms,
(8) unsaturated condensed 7- to 13-membered, heterocyclic group containing 1 or 2 oxygen atoms,
(9) unsaturated condensed 7- to 13-membered, heterocyclic group containing 1 or 2 sulfur atoms,
(10) saturated 3- to 8-membered, heteromonocyclic group containing 1 or 2 oxygen atoms and 1 to 3 nitrogen atoms,
(11) unsaturated condensed 7- to 13-membered, heterocyclic group containing 1 or 2 oxygen atoms and 1 to 3 nitrogen atoms,
(12) unsaturated 3- to 8-membered, heteromonocyclic group containing 1 or 2 sulfur atoms and 1 to 3 nitrogen atoms,
(13) saturated 3- to 8-membered, heteromonocyclic group containing 1 or 2 sulfur atoms and 1 to 3 nitrogen atoms, and
(14) unsaturated condensed 7- to 13-membered, heterocyclic group containing 1 or 2 sulfur atoms and 1 to 3 nitrogen atoms, and

each of the above-mentioned heterocyclic group and ($C_6$-$C_{10}$) aryl group are optionally substituted by the group consisting of the following (A1) to (A35);

(A1) halogen,
(A2) ($C_1$-$C_6$) alkyl,
(A3) ($C_1$-$C_6$) alkoxy,
(A4) halo ($C_1$-$C_6$) alkyl,

(A5) halo ($C_1$-$C_6$) alkoxy,
(A6) ($C_2$-$C_6$) alkenyl,
(A7) acyl,
(A8) ($C_1$-$C_6$) alkylthio, ($C_1$-$C_6$) alkylsulfinyl, ($C_1$-$C_6$) alkylsulfonyl,
(A9) ($C_6$-$C_{10}$) aryl,
(A10) halo ($C_6$-$C_{10}$) aryl,
(A11) hydroxy,
(A12) hydroxy ($C_1$-$C_6$) alkyl, protected hydroxy ($C_1$-$C_6$) alkyl,
(A13) amino,
(A14) carboxy,
(A15) protected carboxy,
(A16) nitro ($C_2$-$C_6$) alkenyl,
(A17) ($C_1$-$C_6$) alkylenedioxy,
(A18) acylamino,
(A19) nitro,
(A20) ($C_6$-$C_{10}$) aryl ($C_1$-$C_6$) alkoxy,
(A21) carbamoyl ($C_2$-$C_6$) alkenyl optionally N-substituted by the group consisting of ($C_1$-$C_6$) alkyl, ($C_6$-$C_{10}$) aryl, ($C_1$-$C_6$) alkoxy ($C_6$-$C_{10}$) aryl, and halo ($C_6$-$C_{10}$) aryl,
(A22) ($C_1$-$C_6$) alkylaminocarbonyloxy,
(A23) ($C_1$-$C_6$) alkanoyloxy,
(A24) ($C_1$-$C_6$) alkoxy ($C_1$-$C_6$) alkanoyloxy,
(A25) ($C_1$-$C_6$) alkoxycarbonyloxy,
(A26) ($C_2$-$C_6$) alkenoyloxy optionally substituted by heterocyclic group of the above (1) to (14),
(A27) ($C_3$-$C_6$) cycloalkanecarbonyloxy,
(A28) ($C_1$-$C_6$) alkoxy substituted by the group consisting of carboxy, protected carboxy, ($C_1$-$C_6$) alkanoyl, ($C_3$-$C_7$) cycloalkanecarbamoyl, and ($C_1$-$C_6$) alkylcarbamoyl,
(A29) ($C_1$-$C_6$) alkylcarbamoyloxy ($C_1$-$C_6$) alkyl,
(A30) ($C_1$-$C_6$) alkoxycarbonylamino ($C_1$-$C_6$) alkyl,
(A31) amino ($C_1$-$C_6$) alkyl,
(A32) ($C_1$-$C_6$) alkylcarbamoyl ($C_1$-$C_6$) alkyl,
(A33) heterocyclic-carbonylamino, the heterocyclic group being selected from the above (1) to (14) and optionally being substituted N-protective group,
(A34) the above heterocyclic groups (1) to (14) being optionally substituted by ($C_1$-$C_6$) alkyl, and
(A35) oxo.

**2.** The compound of claim 1, In which
$R^1$ is ($C_1$-$C_6$) alkyl; optionally substituted heterocyclic group consisting of the following (1) to (10); or optionally substituted ($C_6$-$C_{10}$) aryl;
$R^2$ is carboxy,or hydroxyaminocarbonyl,
Ar is phenyl or heterocyclic group of the following (3),
and
m and n are each an integer of 0 or 1, and m+n=1 or 2,
wherein the heterocyclic group is:

(1) unsaturated 5- or 6-membered heteromonocyclic group containing 1 to 4 nitrogen atoms,
(2) saturated 5- or 6-membered, heteromonocyclic group containing 1 to 4 nitrogen atoms,
(3) unsaturated 5- or 6-membered heteromonocyclic group containing 1 to 2 sulfur atoms,
(4) unsaturated bicyclic 9- or 10-membered, heterocyclic group containing 1 to 5 nitrogen atoms,
(5) unsaturated 5- or 6-membered heteromonocyclic group containing 1 to 2 oxygen atoms,
(6) saturated 5- or 6-membered, heteromonocyclic group containing 1 or 2 oxygen atoms,
(7) unsaturated 5- or 6-membered, heteromonocyclic group containing 1 or 2 oxygen atoms and 1 to 3 nitrogen atoms,
(8) unsaturated bicyclic 9- or 10-membered, heterocyclic group containing 1 or 2 oxygen atoms,
(9) unsaturated bicyclic 9- or 10-membered, heterocyclic group containing 1 or 2 sulfur atoms, or
(10) saturated 5- or 6-membered, heteromonocyciic group containing 1 or 2 oxygen atoms and 1 to 3 nitrogen atoms,

wherein the heterocyclic group being optionaliy substituted by the group consisting of the following (B1) to (B8);

(B1) ($C_1$-$C_6$) alkanoyl,
(B2) ($C_1$-$C_6$) alkyl,
(B3) ($C_1$-$C_6$) alkoxy,
(B4) ($C_1$-$C_6$) alkoxycarbonylamino,
(B5) carbamoyl or ($C_1$-$C_6$) alkylcarbamoyl,
(B6) ($C_1$-$C_6$) alkoxycarbonyl,
(B7) halo, and
(B8) oxo;

and the above-mentioned aryl is optionally substituted by the group consisting of the (A1) to (A35) as defined in claim 1.

**3.** The compound of claim 2, in which a group of the formula:

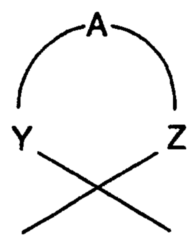

is one of the following formulae:

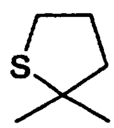 , 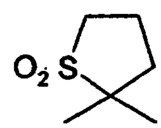 ,

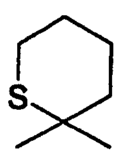 , 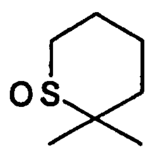 , 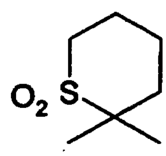 or 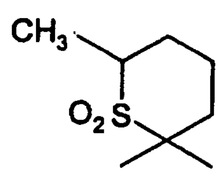 ,

$R^1$ is ($C_1$-$C_6$) alkyl; optionally substituted heterocyclic group consisting of the following (1) to (10); optionally substituted phenyl; or optionally substituted naphtyl;
$R^2$ is the same as defined in claim 2,
Ar is phenyl or thienyl, and
m and n are each an integer of 0 or 1, and m+n=1 or 2,
wherein the above-mentioned heterocyclic group is

(1) pyrrolyl, pyrrolinyl, imidazolyl, pyrazolyl, pyridyl, pyridyl N-oxide, pyrimidyl, pyrazinyl, pyridazinyl, triazolyl, tetrazolyl, dihydrotriazinyl,
(2) azetidinyl, pyrrolidinyl, imidazolidinyl, piperidinyl, piperidino, pyrazolidinyl, piperazinyl,
(3) thienyl,
(4) indolyl, isoindolyl, indolizinyl, benzimidazolyl, quinolyl, isoquinolyl, tetrahydroisoquinolyl, indazolyl, benzotriazolyl, tetrazolopyridyl, tetrazolopyridozinyl, dihydrotriazolopyridazinyl,
(5) furyl,
(6) oxolanyl,

(7) oxazolyl, isoxazolyl, oxadiazolyl,
(8) benzofuranyl, benzodihydrofuranyl, benzodioxolenyl,
(9) benzothienyl, dihydrobenzothienyl, or
(10) morpholinyl, morpholino,

wherein the heterocyclic group being optionally substituted by the group consisting of the (B1) to (B8) as defined in claim 2,
and the above-mentioned phenyl or naphthyl is optionally substituted by the group consisting of following (A1) to (A34),

(A1) halogen,
(A2) ($C_1$-$C_6$) alkyl,
(A3) ($C_1$-$C_6$) alkoxy,
(A4) halo ($C_1$-$C_6$) alkyl,
(A5) halo ($C_1$-$C_6$) alkoxy,
(A6) ($C_2$-$C_6$) alkenyl,
(A7) acyl,
(A8) ($C_1$-$C_6$) alkylthio, ($C_1$-$C_6$) alkylsulfinyl, ($C_1$-$C_6$) alkylsulfonyl,
(A9) ($C_6$-$C_{10}$) aryl,
(A10) halo($C_6$-$C_{10}$) aryl,
(A11) hydroxy,
(A12) hydroxy ($C_1$-$C_6$) alkyl or protected hydroxy ($C_1$-$C_6$) alkyl,
(A13) amino,
(A14) carboxy,
(A15) protected carboxy,
(A16) nitro ($C_2$-$C_6$) alkenyl,
(A17) ($C_1$-$C_6$) alkylenedioxy,
(A18) acylamino,
(A19) nitro,
(A20) ($C_6$-$C_{10}$) aryl ($C_1$-$C_6$) alkoxy,
(A21) carbamoyl ($C_2$-$C_6$) alkenyl optionally N-substituted by the group, consisting of ($C_,$-$C_6$) alkyl, ($C_6$-$C_{10}$) aryl, ($C_1$-$C_6$) alkoxy ($C_6$-$C_{10}$) aryl, and halo($C_6$-$C_{10}$) aryl,
(A22) ($C_1$-$C_6$) alkylaminocarbonyloxy,
(A23) ($C_1$-$C_6$) alkanoyloxy,
(A24) ($C_1$-$C_6$) alkoxy ($C_1$-$C_6$) alkanoyloxy,
(A25) ($C_1$-$C_6$) alkoxycarbonyloxy,
(A26) ($C_2$-$C_6$)alkenoyloxy optionally substituted by the above heterocyclic group (1),
(A27) ($C_3$-$C_6$)cycloalkanecarbonyloxy,
(A28) ($C_1$-$C_6$) alkoxy substituted by the group consisting of carboxy, protected carboxy, ($C_1$-$C_6$) alkanoyl, ($C_3$-$C_7$) cycloalkanecarbamoyl, and ($C_1$-$C_6$) alkylcarbamoyl,
(A29) ($C_1$-$C_6$) alkylcarbamoyloxy ($C_1$-$C_6$) alkyl,
(A30) ($C_1$-$C_6$) alkoxycarbonylamino ($C_1$-$C_6$) alkyl,
(A31) amino ($C_1$-$C_6$) alkyl,
(A32) ($C_1$-$C_6$) alkylcarbamoyl ($C_1$-$C_6$) alkyl,
(A33) heterocyclic-carbonylamino, the heterocyclic group being selected from the above (2), (4) and (5) and optionally substituted by N-protective group, and
(A34) the heterocyclic group of the above (7) being optionally substituted by ($C_1$-$C_6$) alkyl,

**4.** The compound of claim 3, having the following formula:

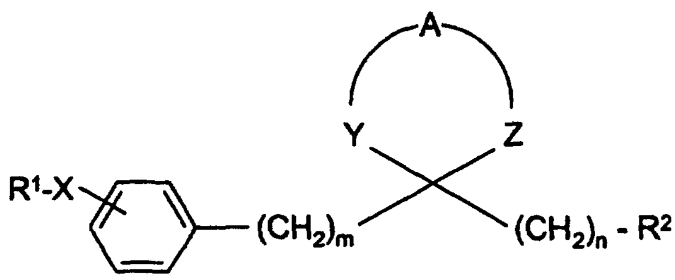

wherein a group of the formula:

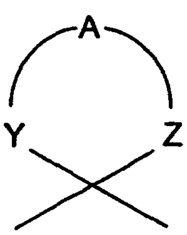

is the same as defined in claim 3,
$R^1$ is ($C_1$-$C_6$) alkyl, phenyl, halophenyl, or (halo)(phenyl)phenyl,
$R^2$ is the same as defined in claim 3, and
m and n are each an integer of 0 or 1, and m+n=1.

**5.** The compound of claim 3, having the following formula:

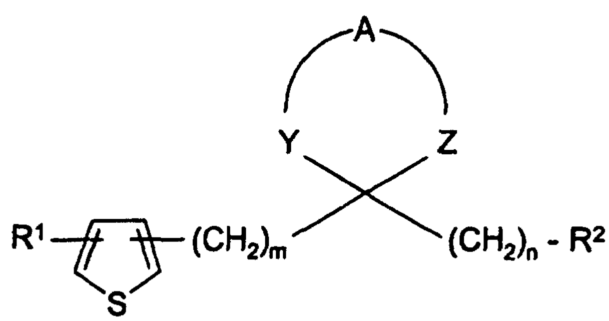

wherein a group of the formula;

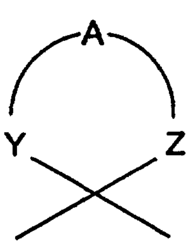

Is the some as defined In claim 3,

$R^2$ is the same as defined in claim 3,
m and n are each an Integer of 0 or 1, and m+n=1,
$R^1$ is heterocyclic group consisting of pyridyl, thienyl, furyl, benzofuranyl or benzothienyl, wherein the heterocyclic group is optionally substituted by the group consisting of ($C_1$-$C_6$) alkanoyl, ($C_1$-$C_6$) alkyl, ($C_1$-$C_6$) alkoxy, ($C_1$-$C_6$) alkoxycarbonylamino and ($C_1$-$C_6$) alkylcarbamoyl; naphtyl or phenyl, each of which is optional substituted by the group consisting of the following (C1) to (C31);

(C1) halogen,
(C2) ($C_1$-$C_6$) alkyl,
(C3) ($C_1$-$C_6$) alkoxy,
(C4) halo ($C_1$-$C_6$) alkyl,
(C5) halo ($C_1$-$C_6$) alkoxy,
(C6) ($C_2$-$C_6$) alkenyl,
(C7) ($C_1$-$C_6$) alkylcarbamoyl, carbamoyl, phenyl ($C_1$-$C_6$) alkylcarbamoyl, ($C_1$-$C_6$) alkanoyl,
(C8) ($C_1$-$C_6$) alkylthio, ($C_1$-$C_6$) alkylsulfinyl, ($C_1$-$C_6$) alkylsulfonyl,
(C9) phenyl, naphthyl,
(C10) halophenyl,
(C11) hydroxy,
(C12) mono- or dihydroxy ($C_1$-$C_6$) alkyl, phenoxycarbonyloxy ($C_1$-$C_6$) alkyl,
(C13) amino,
(C14) carboxy,
(C15) ($C_1$-$C_6$) alkylenedioxy,
(C16) ($C_1$-$C_6$) alkanoylamino, phenyl ($C_1$-$C_6$) alkanoylamino, halophenyl ($C_1$-$C_6$) alkanoylamino, ($C_1$-$C_6$) alkoxy ($C_1$-$C_6$) alkanoylamino, phenoxy ($C_1$-$C_6$) alkanoylamino, ($C_1$-$C_6$) alkoxyphenoxy ($C_1$-$C_6$) alkanoylamino, ($C_1$-$C_6$) alkylphenoxy ($C_1$-$C_6$) alkanoylamino, halophenoxy ($C_1$-$C_6$) alkanoylamino, corboxy ($C_1$-$C_6$)alkanoylamino, ($C_1$-$C_6$) alkoxycarbonyl ($C_1$-$C_6$) alkanoylamino, ($C_1$-$C_6$) alkylcarbamoyl ($C_1$-$C_6$) alkanoylamino, halo ($C_1$-$C_6$) alkanoylamino, ($C_2$-$C_6$)alkenyl ($C_1$-$C_6$) alkanoylamino, ($C_1$-$C_6$) alkoxy ($C_1$-$C_6$) alkanoylamino, phenyl ($C_1$-$C_6$) alkoxy ($C_1$-$C_6$) alkanoylamino, piperidinyloxy ($C_1$-$C_6$) alkanoylamino, N- ($C_1$-$C_6$) alkoxycarbonylpiperidinyloxy ($C_1$-$C_6$) alkanoylamino, pyridyloxy ($C_1$-$C_6$) alkanoylamino, hydroxy ($C_1$-$C_6$) alkanoylamino, ($C_1$-$C_6$) alkanoyloxy ($C_1$-$C_6$) alkanoylamino, ($C_1$-$C_6$) alkylcarbamoyloxy ($C_1$-$C_6$) alkanoylamino, N, N-di (($C_1$-$C_6$) alkyl)carbamoyloxy, ($C_1$-$C_6$) alkanoylamino, piperidino-carbonyloxy ($C_1$-$C_6$) alkanoylamino, phenyl ($C_1$-$C_6$) alkylcarbamoyloxy ($C_1$-$C_6$) alkanoylamino, amino ($C_1$-$C_6$) alkanoylamino, ($C_1$-$C_6$) alkoxycarbonylamino ($C_1$-$C_6$) alkanoylamino, fluorenylmethoxycarbonylamino ($C_1$-$C_6$) alkanoylamino, ($C_1$-$C_6$) alkylamino ($C_1$-$C_6$) alkanoylamino, [N,N di(($C_1$-$C_6$) alkyl)amino]($C_1$-$C_6$)alkanoylamino, [N-($C_1$-$C_6$) alkyl-N-(($C_1$-$C_6$) alkoxycarbonyl) amino] ($C_1$-$C_6$) alkanoylamino, [N-($C_1$-$C_6$) alkyl-N-(fluorenylmethoxycarbonyl)amino] ($C_1$-$C_6$) alkanoylamino, [N-($C_1$-$C_6$)-alkyl-N-(mono- or di($C_1$-$C_6$) alkylcarbamoyl)amino] ($C_1$-$C_6$) alkanoylamino, [N-(mono- or di(($C_1$-$C_6$) alkyl)carbamoyl) amino] ($C_1$-$C_6$) alkanoylamino, benzoylamino ($C_1$-$C_6$) alkanoylamino, ($C_1$-$C_6$) alkanoylamino ($C_1$-$C_6$) alkanoylamino, ($C_1$-$C_6$) alkanesulfonylamino ($C_1$-$C_6$) alkanoylamino, ($C_1$-$C_6$) alkoxy ($C_1$-$C_6$) alkanoylamino ($C_1$-$C_6$) alkanoylamino, cyclo ($C_3$-$C_6$) alkyloxycarbonylamino ($C_1$-$C_6$) alkanoylamino, pyridylcarbonylamino ($C_1$-$C_6$) alkanoylamino, morpholinocarbonylamino ($C_1$-$C_6$)-alkanoylamino, phenyl ($C_1$-$C_6$) alkoxycarbonylamino($C_1$-$C_6$) alkanoylamino, ($C_1$-$C_6$) alkoxyphenylsulfonylamino, ($C_1$-$C_6$) alkanoylamino, hydroxy ($C_1$-$C_6$) alkylamino ($C_1$-$C_6$) alkanoylamino, morpholino ($C_1$-$C_6$) alkanoylamino, oxooxazolidinyl ($C_1$-$C_6$) alkanoylamino, oxopyrrolidinyl ($C_1$-$C_6$) alkanoylamino, trimethylhydantoinyl ($C_1$-$C_6$) alkanoylamino, ($C_2$-$C_6$) alkenylamino ($C_1$-$C_6$) alkanoylamino, ($C_1$-$C_6$) alkoxy ($C_1$-$C_6$) alkylamino ($C_1$-$C_6$) alkanoylamino, phenyl ($C_1$-$C_6$) alkylamino ($C_1$-$C_6$) alkanoylamino, pyridyl ($C_1$-$C_6$) alkylamino ($C_1$-$C_6$) alkanoylamino, ($C_1$-$C_6$) alkoxycarbonylamino, phenyl ($C_1$-$C_6$) alkoxycarbonylamino, ($C_1$-$C_6$) alkoxy ($C_1$-$C_6$) alkoxycarbonylamino, halo ($C_1$-$C_6$) alkoxycarbonylamino, amino ($C_1$-$C_6$) alkoxycarbonylamino, phthalimido ($C_1$-$C_6$) alkoxycarbonylamino, carbamoylamino, (mono- or di(($C_1$-$C_6$) alkyl) carbamoylamino, naphthylcarbamoylamino, halophenylcarbamoylamino, ($C_1$-$C_6$) alkoxyphenylcarbamoylamino, ($C_2$-$C_6$) alkenylcarbamoylamino; cyclo ($C_3$-$C_6$) alkyl ($C_1$-$C_6$) alkylcarbamoylamino, phenyl ($C_1$-$C_6$) alkylcarbamoylamino, halo ($C_1$-$C_6$) alkylcarbamoylamino, ($C_1$-$C_6$) alkoxy ($C_1$-$C_6$) alkylcarbamoylamino, hydroxy ($C_1$-$C_6$) alkylcarbamoylamino, (($C_1$-$C_6$) alkyl)(diphenyl) silyloxy ($C_1$-$C_6$) alkyl carbamoylamino, carboxy ($C_1$-$C_6$) alkylcarbamoylamino, ($C_1$-$C_6$) alkoxycarbonyl ($C_1$-$C_6$) alkylcarbamoylamino, ($C_1$-$C_6$) alkylcarbamoyl ($C_1$-$C_6$) alkyl carbamoylamino, or pyridylcarbamoylamino, ($C_1$-$C_6$) alkylsulfonylamino, ($C_2$-$C_6$) alkenoylamino, ($C_3$-$C_6$) cycloalkanecarbonylamino, ($C_2$-$C_6$) alkenyloxycarbonylamino, phenoxycarbonylamino, ($C_1$-$C_6$) alkylthiocarbonylamino,
(C17) phenyl ($C_1$-$C_6$) alkoxy,
(C18) ($C_2$-$C_6$) alkenyl, mono- or di(($C_1$-$C_6$) alkyl) carbamoyl($C_2$-$C_6$) alkenyl, 2-(methylcarbamoyl) ethenyl,

2-(ethylcarbamoyl)ethenyl, 2-(propylcarbamoyl)ethenyl, 2-(isopropylcarbamoyl) ethenyl, 2-(dimethylcarbamoyl) ethenyl, phenylcarbamoyl ($C_2$-$C_6$) alkenyl, ($C_1$-$C_6$) alkoxycarbamoyl ($C_2$-$C_6$) alkenyl, halophenylcarbamoyl ($C_2$-$C_6$) alkenyl,
(C19) ($C_1$-$C_6$) alkylaminocarbonyloxy,
(C20) ($C_1$-$C_6$) alkanoyloxy,
(C21) ($C_1$-$C_6$) alkoxy ($C_1$-$C_6$) alkanoyloxy,
(C22) ($C_1$-$C_6$) alkoxycarbonyloxy,
(C23) pyridyl ($C_2$-$C_6$) alkenoyloxy,
(C24) ($C_3$-$C_6$) cycloalkanecarbonyloxy,
(C25) carboxy ($C_1$-$C_6$) alkoxy, ($C_1$-$C_6$) alkoxycarbonyl ($C_1$-$C_6$) alkoxy, ($C_1$-$C_6$) alkanoyl ($C_1$-$C_6$) alkoxy, ($C_3$-$C_7$) cycloalkanecarbamoyl ($C_1$-$C_6$) alkoxy, ($C_1$-$C_6$) alkylcarbamoyl ($C_1$-$C_6$) alkoxy,
(C26) ($C_1$-$C_6$) alkylcarbamoyloxy ($C_1$-$C_6$) alkyl,
(C27) ($C_1$-$C_6$) alkoxycarbonylamino ($C_1$-$C_6$) alkyl,
(C28) amino ($C_1$-$C_6$) alkyl,
(C29) ($C_1$-$C_6$) alkylcarbamoyl ($C_1$-$C_6$) alkyl,
(C30) furylcarbonylamino, tetrahydroisoquinolylcarbonylamino, N-($C_1$-$C_6$) alkoxycarbonyl tetrahydroisoquinolylcarbonylamino, pyrrolidinylcarbonylamino,
(C31) oxazolyl, ($C_1$-$C_6$) alkyloxadiazolyl.

**6.** The compound of claim 5, in which a group of the formula:

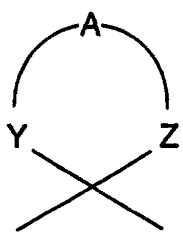

is one of the following formulae:

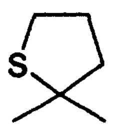 , 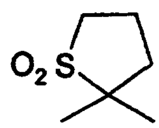 ,

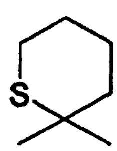 , 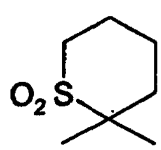 ,

$R^2$ is the same as defined in claim 5,
m is 0 and n is 1, a group of the formula:

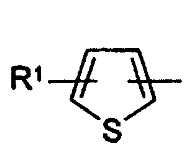

Is the group of the following formulae (a) to (e);

(a)

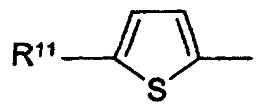

wherein

$R^{11}$ is halo, naphtyl, phenyl, mono- or dihalophenyl, mono- or di ($C_1$-$C_6$) alkylphenyl, ($C_1$-$C_6$) alkoxyphenyl, trihalo ($C_1$-$C_6$) alkylphenyl, trihalo ($C_1$-$C_6$) alkoxyphenyl, ($C_2$-$C_6$) alkenylphenyl, ($C_1$-$C_6$) alkylcarbamoylphenyl, carbamoylphenyl, phenyl ($C_1$-$C_6$) alkylcarbamoylphenyl, ($C_1$-$C_6$) alkanoylphenyl, ($C_1$-$C_6$) alkylthiophenyl, ($C_1$-$C_6$) alkylsulfinylphenyl, ($C_1$-$C_6$) alkylsulfonylphenyl, phenylphenyl, (halo)(phenyl)phenyl, halophenylphenyl, hydroxyphenyl, mono- or dihydroxy ($C_1$-$C_6$) alkylphenyl, phenoxycarbonyloxy ($C_1$-$C_6$) alkylphenyl, aminophenyl, carboxyphenyl, ($C_1$-$C_6$) alkylendioxyphenyl, ($C_1$-$C_6$) alkanesulfonylaminophenyl, ($C_2$-$C_6$) alkenoylaminophenyl, ($C_3$-$C_6$) cycloalkanecarbonylaminophenyl, phenyl ($C_1$-$C_6$) alkoxyphenyl, mono- or di (($C_1$-$C_6$) alkyl) carbamoyl ($C_2$-$C_6$) alkenylphenyl, phenylcarbamoyl ($C_2$-$C_6$) alkenylphenyl, ($C_1$-$C_6$) alkoxycarbamoyl ($C_2$-$C_6$) alkenylphenyl, halophenylcarbamoyl ($C_2$-$C_6$) alkenylphenyl, ($C_1$-$C_6$) alkylcarbamoyloxyphenyl, ($C_1$-$C_6$) alkanoyloxyphenyl, ($C_1$-$C_6$) alkoxy ($C_1$-$C_6$) alkanoyloxyphenyl, ($C_1$-$C_6$) alkoxycarbonyloxyphenyl, pyridyl ($C_2$-$C_6$) alkenoyloxyphenyl, cyclo ($C_3$-$C_6$) alkylcarbonyloxyphenyl, carboxy ($C_1$-$C_6$) alkoxyphenyl, ($C_1$-$C_6$) alkoxycarbonyl ($C_1$-$C_6$) alkoxyphenyl, ($C_1$-$C_6$) alkanoyl ($C_1$-$C_6$) alkoxyphenyl, ($C_3$-$C_6$) cycloalkanecarbamoyl ($C_1$-$C_6$) alkoxyphenyl, ($C_1$-$C_6$) alkylcarbamoyl ($C_1$-$C_6$) alkoxyphenyl, ($C_1$-$C_6$) alkylcarbamoyloxy ($C_1$-$C_6$) alkylphenyl, ($C_1$-$C_6$) alkoxycarbonylamino ($C_1$-$C_6$) alkylphenyl, amino ($C_1$-$C_6$) alkylphenyl, ($C_1$-$C_6$) alkylcarbamoyl ($C_1$-$C_6$) alkylphenyl, furylcarbonylaminophenyl, 1,2,3,4-tetrahydroisoquinolylcarbonylaminophenyl, N-t-butoxycarbonyl, 1,2,3,4-tetrahydroisoquinolylcarbonylaminophenyl, pyrrolidinylcarbonylaminophenyl, oxazolylphenyl, ($C_1$-$C_6$) alkyloxadiazolylphenyl,

(b)

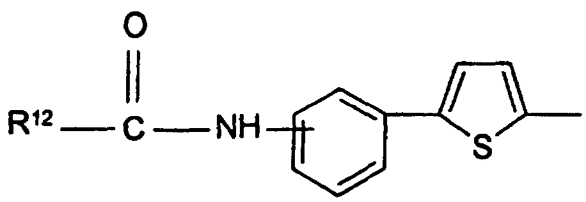

wherein $R^{12}$ is ($C_1$-$C_6$) alkyl optionally substituted by the group consisting of phenyl, halophenyl, ($C_1$-$C_6$) alkoxyphenyl, ($C_1$-$C_6$) alkoxy, phenoxy, ($C_1$-$C_6$) alkoxyphenoxy, halophenoxy, ($C_1$-$C_6$) alkylphenoxy, carboxy, ($C_1$-$C_6$) alkoxycarbonyl, ($C_1$-$C_6$) alkylcarbamoyl, halo, ($C_2$-$C_6$) alkenyloxy, ($C_1$-$C_6$) alkoxy ($C_1$-$C_6$) alkoxy, phenyl ($C_1$-$C_6$) alkoxy, piperidinyloxy, N-($C_1$-$C_6$) alkoxycarbonyl-piperidinyloxy, pyridyloxy, hydroxy, ($C_1$-$C_6$) alkanoyloxy, mono- or di ($C_1$-$C_6$) alkylcarbamoyloxy, piperidinylcarbonyloxy, phenyl ($C_1$-$C_6$) alkylcarbamoyloxy, amino, ($C_1$-$C_6$) alkoxycarbonylamino, fluorenylmethoxycarbonylamino, mono- or di ($C_1$-$C_6$) alkylamino, N-($C_1$-$C_6$) alkyl-N-(($C_1$-$C_6$) alkoxycarbonyl)amino, N-($C_1$-$C_6$) alkyl-N-(fluorenylmethoxycarbonyl)amino, N-($C_1$-$C_6$) alkyl-N (mono- or di ($C_1$-$C_6$) alkylcarbamoyl)amino, N-(mono- or di(($C_1$-$C_6$) alkyl)carbamoyl)amino, benzoylamino, ($C_1$-$C_6$) alkanoylamino, ($C_1$-$C_6$) alkanesulfonylamino, ($C_1$-$C_6$) alkoxy ($C_1$-$C_6$) alkanoylamino, cyclo ($C_3$-$C_6$) alkyloxycarbonylamino, pyridylcarbonylamino, morpholinocarbonylamino, phenyl ($C_1$-$C_6$) alkoxycarbonylamino, ($C_1$-$C_6$) alkoxyphenylsulfonylamino, hydroxy ($C_1$-$C_6$) alkylamino, morpholino, oxooxazolidinyl, oxopyrrolidinyl, trimethylhydantoinyl, pyridyl, ($C_2$-$C_6$) alkenylamino, ($C_1$-$C_6$) alkoxy ($C_1$-$C_6$) alkylamino, phenyl ($C_1$-$C_6$) alkylamino, pyridyl ($C_1$-$C_6$) alkylamino, and cyclo ($C_3$-$C_6$) alkyl,

(c)

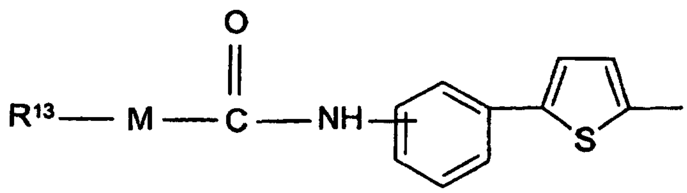

wherein
M is oxygen or sulfur,
$R^{13}$ is ($C_1$-$C_6$) alkyl, phenyl ($C_1$-$C_6$) alkyl, ($C_1$-$C_6$) alkoxy ($C_1$-$C_6$) alkyl, halo ($C_1$-$C_6$) alkyl, amino ($C_1$-$C_6$) alkyl, or phthalimido ($C_1$-$C_6$) alkoxycarbonylamino, ($C_2$-$C_6$) alkenyl, phenyl,

(d)

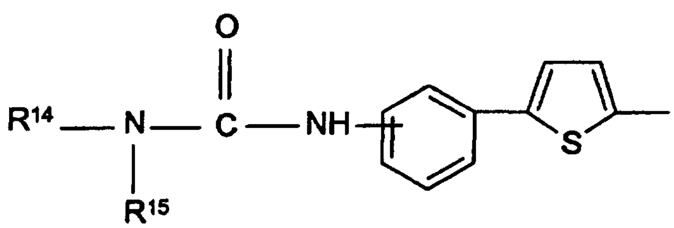

wherein
$R^{15}$ Is hydrogen or ($C_1$-$C_6$) alkyl,
$R^{14}$ is hydrogen, ($C_1$-$C_6$) alkyl, naphthyl, halophenyl, ($C_1$-$C_6$) alkoxyphenyl, ($C_2$-$C_6$) alkenyl, ($C_3$-$C_6$) cycloalkyl ($C_1$-$C_6$) alkyl, phenyl ($C_1$-$C_6$) alkyl, halo ($C_1$-$C_6$) alkyl, ($C_1$-$C_6$) alkoxy ($C_1$-$C_6$) alkyl, hydroxy ($C_1$-$C_6$) alkyl, (($C_1$-$C_6$) alkyl)(diphenyl)silyloxy ($C_1$-$C_6$) alkyl, carboxy ($C_1$-$C_6$) alkyl, ($C_1$-$C_6$) alkoxycarbonyl ($C_1$-$C_6$) alkyl, ($C_1$-$C_6$) alkylcarbamoyl ($C_1$-$C_6$) alkyl, or pyridyl,

(e)

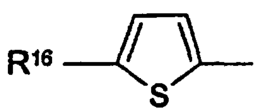

wherein
$R^{16}$ is benzothienyl, benzofuranyl, thienyl, furyl, ($C_1$-$C_6$) alkylpyridyl, pyridyl, ($C_1$-$C_6$) alkoxypyridyl, ($C_1$-$C_6$) alkoxycarbonylaminopyridyl, ($C_1$-$C_6$) alkanoylthienyl, ($C_1$-$C_6$) alkylcarbamoylbenzofuranyl.

**7.** The compound of claim 6, wherein
a group of the formula:

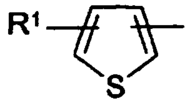

Is the same group as (a), (c), (d) and (e) of claim 6,
and the following formula (b):

(b)

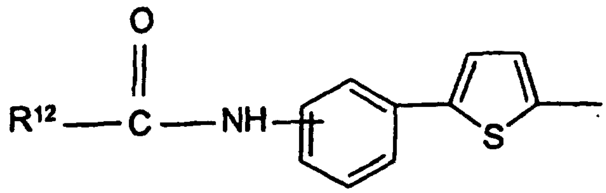

wherein

$R^{12}$ is ($C_1$-$C_6$) alkyl, phenyl ($C_1$-$C_6$) alkyl, halophenyl ($C_1$-$C_6$) alkyl, ($C_1$-$C_6$) alkoxyphenyl ($C_1$-$C_6$) alkyl, ($C_1$-$C_6$) alkoxy ($C_1$-$C_6$) alkyl, phenoxy ($C_1$-$C_6$) alkyl, ($C_1$-$C_6$) alkoxyphenoxy ($C_1$-$C_6$) alkyl, halophenoxy ($C_1$-$C_6$) alkyl, ($C_1$-$C_6$) alkylphenoxy ($C_1$-$C_6$) alkyl, carboxy ($C_1$-$C_6$) alkyl, ($C_1$-$C_6$) alkoxycorbonyl ($C_1$-$C_6$) alkyl, ($C_1$-$C_6$) alkylcorbamoyl ($C_1$-$C_6$) alkyl, halo ($C_1$-$C_6$) alkyl, ($C_2$-$C_6$) alkenyloxy ($C_1$-$C_6$) alkyl, ($C_1$-$C_6$) alkoxy ($C_1$-$C_6$) alkoxy ($C_1$-$C_6$) alkyl, phenyl ($C_1$-$C_6$) alkoxy ($C_1$-$C_6$) alkyl, piperidinyloxy ($C_1$-$C_6$) alkyl, N-t-butoxycarbonylpiperidinyloxy ($C_1$-$C_6$) alkyl, pyridyloxy ($C_1$-$C_6$) alkyl, hydroxy ($C_1$-$C_6$) alkyl, ($C_1$-$C_6$) alkanoyloxy ($C_1$-$C_6$) alkyl, mono- or di ($C_1$-$C_6$) alkylcarbamoyloxy ($C_1$-$C_6$) alkyl, piperidinylcarbonyloxy ($C_1$-$C_6$) alkyl, phenyl($C_1$-$C_6$) alkylcarbamoyloxy ($C_1$-$C_6$) alkyl, amino ($C_1$-$C_6$) alkyl, ($C_1$-$C_6$) alkoxycarbonylamino ($C_1$-$C_6$) alkyl, fluorenylmethoxycarbonylamino ($C_1$-$C_6$) alkyl, mono- or di ($C_1$-$C_6$) alkylamino ($C_1$-$C_6$) alkyl, N-($C_1$-$C_6$) alkyl-N-(($C_1$-$C_6$) alkoxycarbonyl)amino($C_1$-$C_6$)alkyl, N-($C_1$-$C_6$) alkyl-N-(fluorenylmethoxycarbonyl) amino ($C_1$-$C_6$) alkyl, N-($C_1$-$C_6$) alkyl-N-(mono- or di ($C_1$-$C_6$) alkylcarbamoyl)amino ($C_1$-$C_6$) alkyl, N-(mono- or di (($C_1$-$C_6$) alkyl)carbamoyl) amino ($C_1$-$C_6$) alkyl, benzoylamino ($C_1$-$C_6$) alkyl, ($C_1$-$C_6$) alkanoylamino ($C_1$-$C_6$) alkyl, ($C_1$-$C_6$) alkanesulfonylamino ($C_1$-$C_6$) alkyl, ($C_1$-$C_6$) alkoxy ($C_1$-$C_6$) alkanoylamino ($C_1$-$C_6$) alkyl, cyclo ($C_3$-$C_6$) alkyloxycarbonylamino ($C_1$-$C_6$) alkyl, pyridylcarbonylamino ($C_1$-$C_6$) alkyl, morpholinocarbonylamino ($C_1$-$C_6$) alkyl, phenyl ($C_1$-$C_6$) alkoxyoxycarbonylamino ($C_1$-$C_6$) alkyl, ($C_1$-$C_6$) alkoxyphenylsulfonylamino ($C_1$-$C_6$) alkyl, hydroxy ($C_1$-$C_6$) alkylamino ($C_1$-$C_6$) alkyl, morpholino ($C_1$-$C_6$) alkyl, oxooxazolidinyl ($C_1$-$C_6$) alkyl, oxopyrrolidinyl ($C_1$-$C_6$) alkyl, trimethylhydantoinyl ($C_1$-$C_6$) alkyl, pyridyl ($C_1$-$C_6$) alkyl, ($C_2$-$C_6$) alkenylamino ($C_1$-$C_6$) alkyl, ($C_1$-$C_6$) alkoxy ($C_1$-$C_6$) alkylamino ($C_1$-$C_6$) alkyl, phenyl ($C_1$-$C_6$) alkylamino ($C_1$-$C_6$) alkyl, pyridyl ($C_1$-$C_6$) alkylamino ($C_1$-$C_6$) alkyl, cyclo ($C_3$-$C_6$) alkyl, (amino)(phenyl) ($C_1$-$C_6$) alkylamino, (($C_1$-$C_6$) alkoxycarbonylamino)(phenyl) ($C_1$-$C_6$) alkyl, (amino) (($C_1$-$C_6$) alkoxy) ($C_1$-$C_6$) alkyl, (($C_1$-$C_6$) alkoxycarbonylamino) (($C_1$-$C_6$) alkoxy) ($C_1$-$C_6$) alkyl, (amino)(carboxy) ($C_1$-$C_6$) alkyl, (($C_1$-$C_6$) alkoxycarbonylamino)(carboxy) ($C_1$-$C_6$) alkyl, (amino)(($C_1$-$C_6$) alkoxycarbonyl) ($C_1$-$C_6$) alkyl, (($C_1$-$C_6$) alkoxycarbonylamino)(($C_1$-$C_6$) alkoxycarbonyl) ($C_1$-$C_6$) alkyl, (amlno)(Phenyl ($C_1$-$C_6$) alkoxy) ($C_1$-$C_6$) alkyl, (($C_1$-$C_6$) alkoxycarbonylamino) (Phenyl ($C_1$-$C_6$) alkoxy) ($C_1$-$C_6$) alkyl, (amino)(pyridyl) ($C_1$-$C_6$) alkyl, (($C_1$-$C_6$) alkoxycarbonylamino)(pyridyl) ($C_1$-$C_6$) alkyl, (amino)(hydroxy) ($C_1$-$C_6$) alkyl, (($C_1$-$C_6$) alkoxycarbonylamino)(hydroxy) ($C_1$-$C_6$) alkyl, (amino) (amino) ($C_1$-$C_6$) alkyl, (($C_1$-$C_6$) alkoxycarbonylamino)(amino) ($C_1$-$C_6$) alkyl, (amino) (($C_1$-$C_6$) alkoxycarbonylamino) ($C_1$-$C_6$) alkyl, (($C_1$-$C_6$) alkoxycarbonylamino) (($C_1$-$C_6$) alkoxycarbonylamino) ($C_1$-$C_6$) alkyl, (amino) (($C_3$-$C_6$) cycloalkane) ($C_1$-$C_6$) alkyl, (($C_1$-$C_6$) alkoxycarbonylamino)(($C_3$-$C_6$)cycloalkane)($C_1$-$C_6$)alkyl.

**8.** The compound of claim 6, In which a group of the formula:

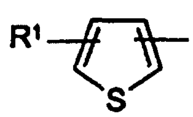

Is the group of the following formula (a) to (e):

(a)

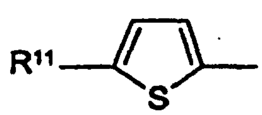

wherein

$R^{11}$ is bromo, 2-naphthyl, phenyl, 3(or 4)-chlorophenyl, 2(or 3 or 4)-fluorophenyl, 3,4-dichlorophenyl, 3,5-difluorophenyl, 3(or 4)-methylphenyl, 4-ethylphenyl, 4-Isopropylphenyl, 4-(t-butyl)phenyl, 3,4-dimethylphenyl, 4-methoxyphenyl, 4-ethoxyphenyl, 4-trifluoromethylphenyl, 4-trifluoromethoxyphenyl, 4-ethenylphenyl, 4-methylcarbamoylphenyl, 4-ethylcarbamoylphenyl, 4 carbamoylphenyl, 4-benzylcarbamoylphenyl, 4-acetylphenyl, 4-methytthiophenyl, 4-ethylthiophenyl, 4-methylsulfinylphenyl, 4-methylsulfonylphenyl, phenylphenyl, 4-phenyl-3 fluorophenyl, 4-(4-fluorophenyl)phenyl, 3 (or 4) hydroxyphenyl, 3 (or 4)-hydroxymethylphenyl, 4-(1,2-dihydroxyethyl)phenyl, 4-(phenoxycarbonyloxymethyl)phenyl, 3 (or 4) aminophenyl, 4-carboxyphenyl, 3,4-methylendioxyphenyl, 4-(methanesulfonylamino)phenyl, 3-(2-butenoylamino)phenyl, 3-(cyclopropanecarbonylamino)phenyl, 3-(cyclobutanecarbonylamino)phenyl, 3-(cyclopentanecorbonylamlno)phenyl, 4-benzyloxyphenyl, 4-(2-(methylcarbamoyl)ethenyl)phenyl, 4-(2-(ethylcarbamoyl)ethenyl)phenyl, 4-(2-(propylcarbamoyl)ethenyl)phenyl, 4-(2-(isopropylcarbamoyl)ethenyl)phenyl, 4-(2-(dimethylcarbamoyl)ethenyl)phenyl, 4-(2-(phenylcarbamoyl)ethenyl)phenyl, 4-(2-(methoxyphenylcarbamoyl)ethenyl)phenyl, 4-(2-(4-fluorophenylcarbamoyl)ethenyl)phenyl, 4-(methylaminocarbonyloxy)phenyl, 4-(ethylaminocarbonyloxy)phenyl, 4-propanoyloxyphenyl, 4-(methoxyacetyloxy)phenyl, 4-(ethoxycarbonyloxy)phenyl, 4-(3-(3-pyridyl)acryloyloxy) phenyl, 4-(cyclopropylcarbonyloxy)phenyl, 4-(carboxymethoxy)phenyl, 4-(ethoxycarbonylmethoxy)phenyl, 4-(t-butoxycarbonylmethoxy)phenyl, 4-(propanoylmethoxy)phenyl, 4-(cyclopropylcarbamoylmethoxy)phenyl, 3 (or 4)-(methylcarbamoylmethoxy)phenyl, 4-(ethylcarbamoylmethoxy)phenyl, 4-(propylcarbamoylmethoxy) phenyl, 3(or 4)-(methylcarbamoyloxymethyl)phenyl, 4-(methoxycarbonylaminomethyl)phenyl, 4-(t-butoxycarbonylaminomethyl)phenyl, 4-aminomethylphenyl, 4-(methylcarbamoylmethyl)phenyl, 3-(2(or 3)-furylcarbonylamino)phenyl, 3-(1,2,3,4-tetrahydroisoquinolylcarbonylamino)phenyl, 3-(N-(t-butoxycarbonyl)-1,2,3,4-tetrahydroisoquinolylcarbonylamino)phenyl, 3-(pyrrolidinylcarbonylamino)phenyl, 4-(1,3-oxazolyl)phenyl, 4-(5-methyl-1,2,4-oxadiazol-3-yl)phenyl,

(b)

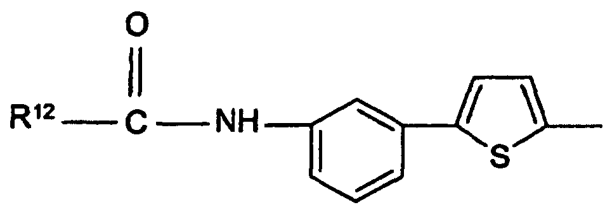

wherein
$R^{12}$ is methyl, ethyl, propyl, Isopropyl, butyl, isobutyl, t-butyl, neopentyl, phenylmethyl, 4-chlorophenylmethyl, 4-methoxyphenylmethyl, methoxymethyl, ethoxymethyl, propoxymethyl, butoxymethyl, isopropyloxymethyl, 1-methoxyethyl, 2-methoxyethyl, phenoxymethyl, 2-phenoxyethyl, 3(or 4)-methoxyphenoxymethyl, 4-fluoro(or chloro)phenoxymethyl, 3 (or 4)-methylphenoxymethyl, 2-carboxyethyl, 2-methoxycarbonylethyl, 2-t-butoxycarbonylethyl, 2-methylcarbamoylethyl, 2-chloroethyl, chloromethyl, allyloxymethyl, (2-ethoxyethoxy)methyl, benzyloxymethyl, 4-piperidinyloxymethyl, (N-t-butoxycarbonyl-4-piperidinyl)oxymethyl, 3(or 4)-pyridyloxymethyl, hydroxymethyl, 2-hydroxyethyl, acetoxymethyl, 1-acetoxyethyl, methylcarbamoyloxymethyl, 1-(N-methyl-N-ethylcarbamoyloxy)methyl, (plperidinocarbonyloxy)methyl, (benzylcarbamoyloxy)methyl, (t-butoxycarbonylamino)methyl, aminomethyl, 1-aminoethyl, 1-(t-butoxycarbonylamino)ethyl, 2-aminoethyl, methoxycarbonylaminomethyl, 2-(methoxycarbonylamino)ethyl, ethoxycarbonylaminomethyl, propoxycarbonylaminomethyl, 1-(fluorenylmethoxycarbonylamino)methyl, 2-(t-butoxycarbonylamino)ethyl, 2-(fluorenylmethoxycarbonylamino)ethyl, 1-aminoisopropyl, 1-aminopropyl, 1-(t-butoxycarbonylamino)propyl, 1-(t-butoxycarbonylamino)isopropyl, 1,5-diaminopentyl, 1,5-bis (t-butoxycarbonylamino)-pentyl, methylaminomethyl, ethylaminomethyl, 1-(N-methyl-N-ethylamino)methyl, dimethylaminomethyl, pentylaminomethyl, t-butylaminomethyl, 2-methylaminoethyl, 1-(N-methyl-N-methoxycarbonylamino)methyl, 1-(N-methyl-N-t-butoxycarbonylamino) methyl, 1-(N-ethyl-N-t-butoxycarbonylamino]methyl, 2-(N-methyl-N-(fluorenylmethoxycarbonyl)amino)-ethyl, 2-(N-methyl-N-(t-butoxycarbonyl)amino)ethyl, 1-(N-methyl-N-(dimethylcarbamoyl)amino)methyl, 1-(dimethylcarbamoylamino)methyl, 1-(N-(ethylcarbamoyl)amino]methyl, 2-(N-(ethylcarbamoyl)amino)ethyl, benzoylaminomethyl, 2-benzoylaminoethyl, acetylaminomethyl, isobutyrylaminomethyl, pivaloylaminomethyl, 1-(methanesulfonylamino]methyl, 2-(methanesulfonylamino)ethyl, methoxyacetylaminomethyl, cyclopentyloxycarbonylaminomethyl, pyridylcarbonylaminomethyl, morpholinocarbonylaminomethyl, benzyloxycarbonylaminomethyl, 1-(4-methoxyphenylsulfonylamino)methyl, 1-(2-hydroxyethylamino)methyl, morpholinomethyl, 1-(2-oxo-1,3-oxazolidin-1-yl)methyl, 1-(2-oxopyrrolidin-1-yl)methyl, 1-(3,4,4-trimethylhydantoin-1-yl)methyl, allylaminomethyl, 1-(2-ethoxyethylamino)methyl, benzylaminomethyl, 1-(3-pyridylmethylamino)methyl, 2-phenyl-1-aminoethyl, 1-amino-1-phenylmethyl, 1-t-butoxycarbonylamino-1-phenylmethyl, 1-amino-2-phenylethyl, 1-t-butoxycarbonylamino-2-phenylethyl, 1-amino-2-methoxyethyl, 1-t-butoxycarbonylamino-2-methoxyethyl, 1-amino-3 carboxypropyl, 1-t-butoxycarbonylamino-3 carboxypropyl, 1-amino-3-(t-butoxycarbonyl) propyl, 1-t-butoxycarbonylamino-3-t-butoxycarbonylpropyl, 1-amino-2-benzyloxyethyl, 1-t-butoxycarbonylamino-2-benzyloxyaminoethyl, 1-amino-2-(3-pyridyl)ethyl, 1-t-butoxycarbonylamino-2-(3-pyridyl)ethyl, 1-amino-2-(4-pyridyl)ethyl, 1-t-butoxycarbonylamino-2-(4-pyridyl)ethyl, 1-amino-2-hydroxyethyl, 1-t-butoxycarbonylamino-2-hydroxyethyl, 1,5-diaminopentyl, 1-t-butoxycarbonylamino-5-aminopentyl, 1,5-bis(t-butoxycarbonylamino)pentyl, 1-amino-5-(t-butoxycarbonylamino)pentyl, 1-amino-2-cyclohexylethyl, 1-t-butoxycarbonylamino-2-cyclohexylethyl,

(c)

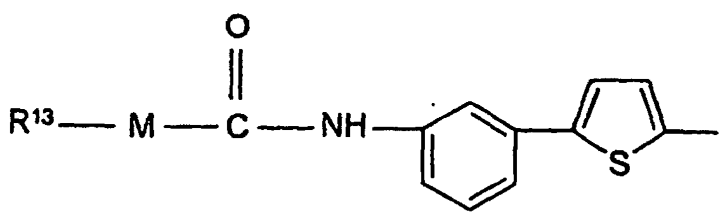

wherein
M= O and $R^{13}$ is methyl, ethyl, propyl, isopropyl, benzyl, 2-methoxyethyl, 2-chloroethyl, 2-aminoethyl, 2-phthalimidoethyl, allyl, phenyl, or M=S and $R^{13}$ is methyl, ethyl,

(d)

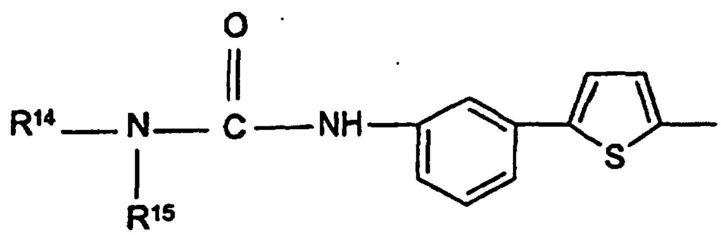

wherein
$R^{15}$ is hydrogen and
$R^{14}$ is hydrogen, methyl, ethyl, propyl, isopropyl, butyl, isobutyl, pentyl, hexyl, 1-naphthyl, 3 (or 4)-chlorophenyl, 3-methoxyphenyl, allyl, cyclohexylmethyl, benzyl, 2-chloroethyl, methoxymethyl, 2-methoxyethyl, 2-hydroxyethyl, 2-((t-butyl)(diphenyl)silyloxy)ethyl, carboxymethyl, ethoxycarbonylmethyl, methylcarbamoylmethyl, or 3-pyridyl, or
$R^{14}$ is ethyl and
$R^{15}$ is methyl,

(e)

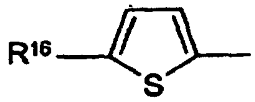

wherein $R^{16}$ is 2-benzothienyl, 2-benzofuranyl, 2 (or 3)-thienyl, 2-furyl, 3-pyridyl, 1-methyl-4-pyridyl, 6-methyl-3-pyridyl, 6-methoxy-3-pyridyl, 5-methoxycarbonylamino-3-pyridyl, 5-acetyl-2-thienyl, 2-methylcarbamoyl-5-benzofuranyl.

**9.** A process for the preparation of o compound of the formula:

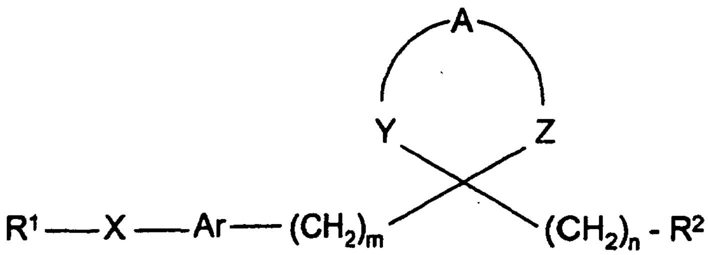

(I)

in which $R^1$, $R^2$, Ar, A, X, Y, Z, m and n are each as defined in Claim 1, which comprises

(1) subjecting a compound of the formula:

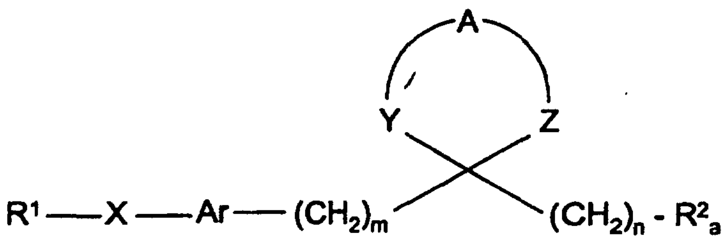

(I-a)

or a salt thereof to removal reaction of the carboxy-protective group, to give a compound of the formula:

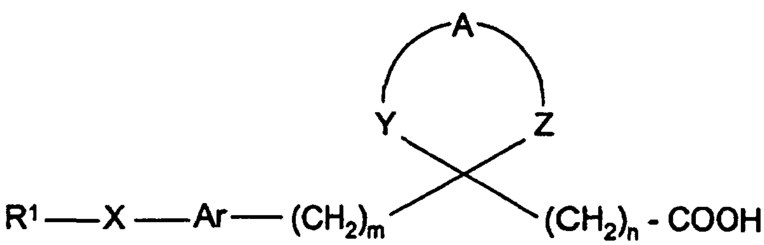

(I-b)

or a salt thereof; or

(2) oxidating the vinyl group of a compound of the formula:

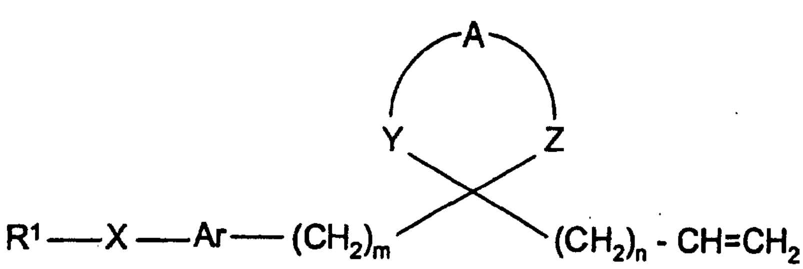

(II)

or a salt thereof, to give a compound of the above formula (I-b) or a salt thereof; or

(3) reducing a compound of the formula:

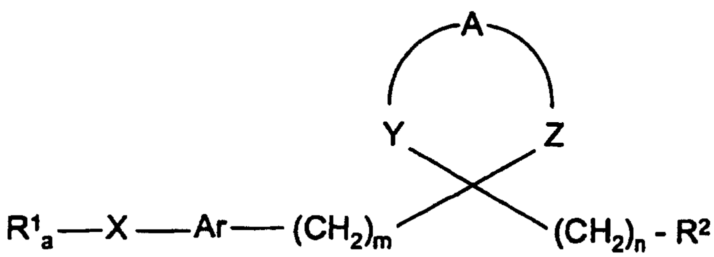

(I-c)

or a salt thereof, to give a compound of the formula:

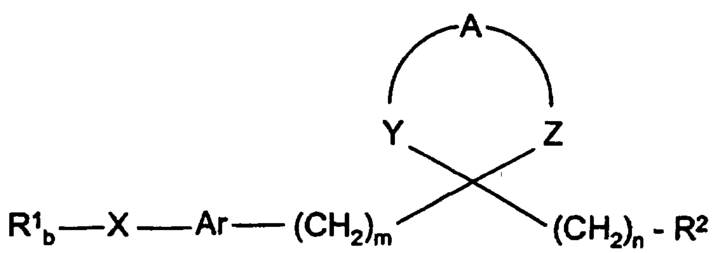

(I-d)

or a salt thereof; or

(4) reacting a compound of the above formula (I-b) or its reactive derivative at the carboxy-group, or a salt thereof, with a compound of the formula:

$$NH_2 - OR^3 \quad (IV)$$

or its reactive derivative at the amino-group, or a salt thereof, to give a compound of the formula:

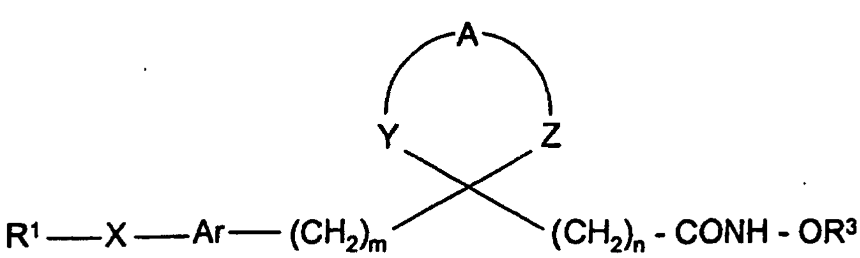

(I-e)

or a salt thereof; or

(5) cyclizing a compound of the formula:

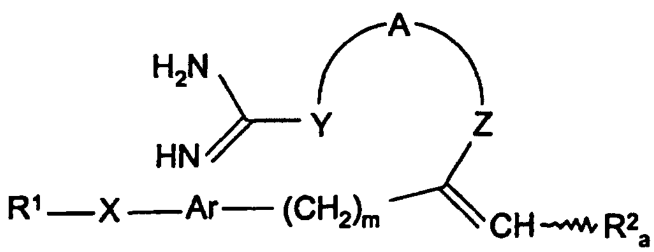

(III)

or a salt thereof, to give a compound of the formula:

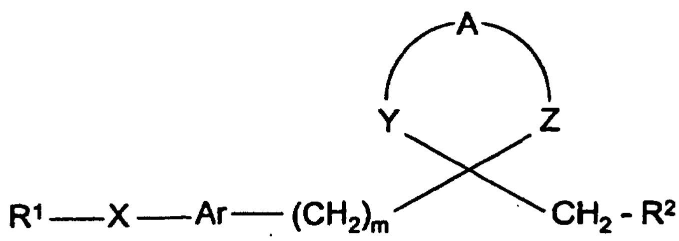

(I-f)

or a salt thereof; or

(6) reacting a compound of the above formula (I-b) or its reactive derivative at the carboxy-group, or a salt thereof, with an optically active amine or its reactive derivative at the amino-group, or a salt thereof, to give a compound of the formula:

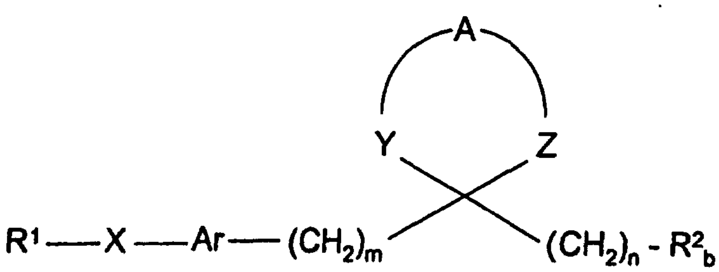

(I-g)

or a salt thereof; or

(7) subjecting a compound of the formula:

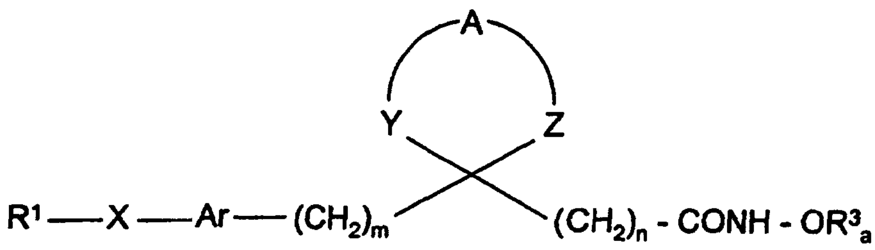

(I-h)

or a salt thereof to removal reaction of the hydroxy-protective group, to give a compound of the formula:

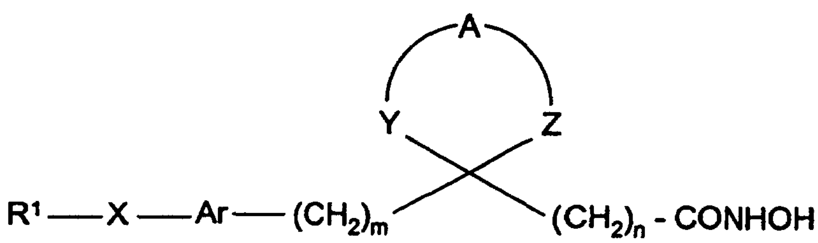

(I-i)

or a salt thereof; or

(8) oxidating a compound of the formula:

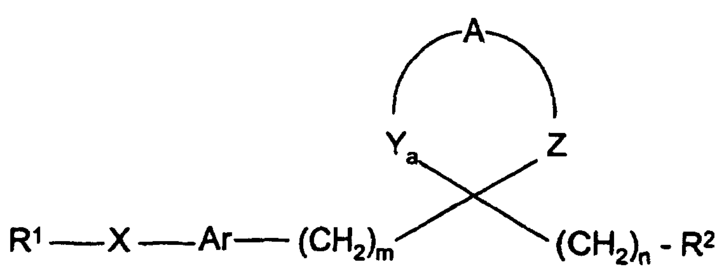

(I-j)

or a salt thereof, to give a compound of the formula:

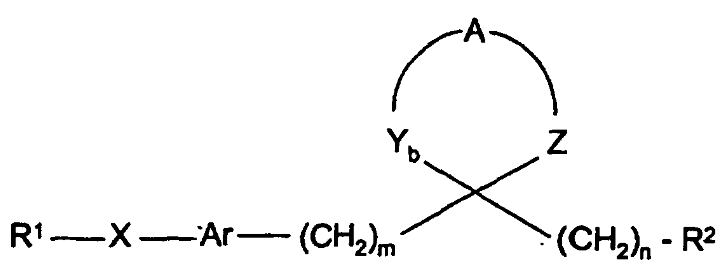

(I-k)

or a salt thereof; or

(9) reacting a compound of the above formula (I-c) or a salt thereof, with a compound of the formula:

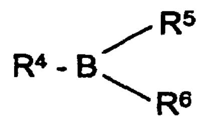

(V)

to give a compound of the formula:

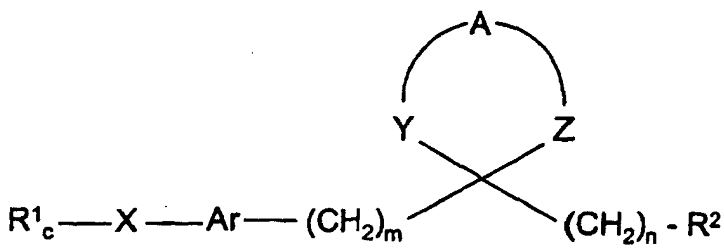

(I-l)

or a salt thereof; or

(10) reacting a compound of the formula:

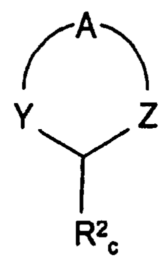

(VI)

or a salt thereof, with a compound of the formula:

$$R^1\text{-X-Ar-}(CH_2)_{m}\text{I - L} \qquad \text{(VII)}$$

or a salt thereof, to give a compound of the formula:

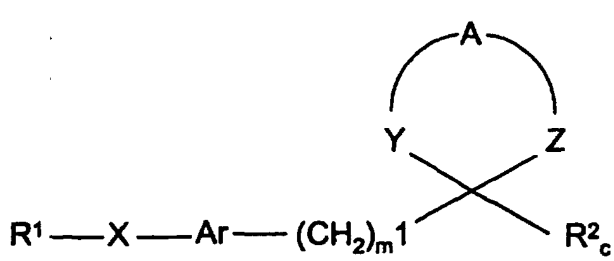

(I-m)

or a salt thereof; or

(11) cyclizing a compound of the formula:

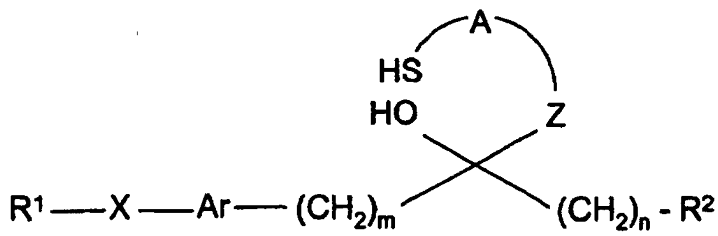

(VIII)

or a salt thereof, to give a compound of the formula:

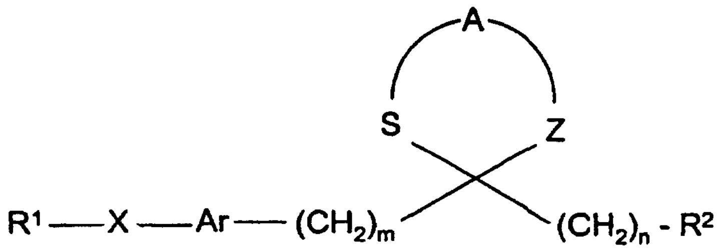

(I-n)

or a salt thereof; or

(12) amidating a compound of the formula:

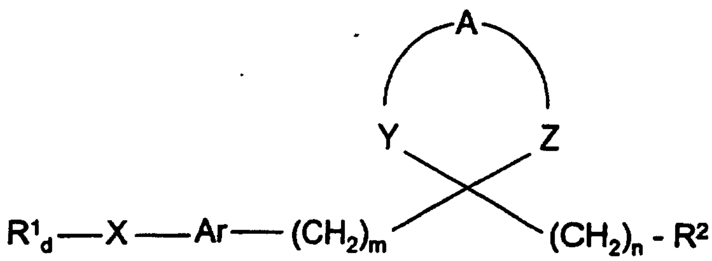

(I-p)

or its reactive derivative at the carboxy group,
or a salt thereof, to give a compound of the formula

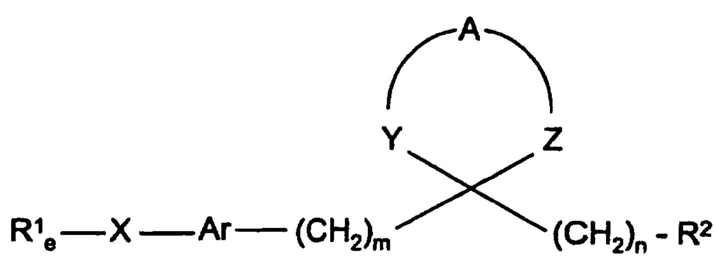

(I-q)

or a salt thereof; or

(13) acylating a compound of the formula:

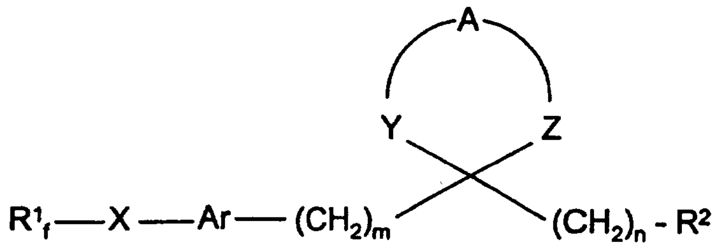

(I-r)

or its reactive derivative at the amino group,
or a salt thereof, to give a compound of the formula:

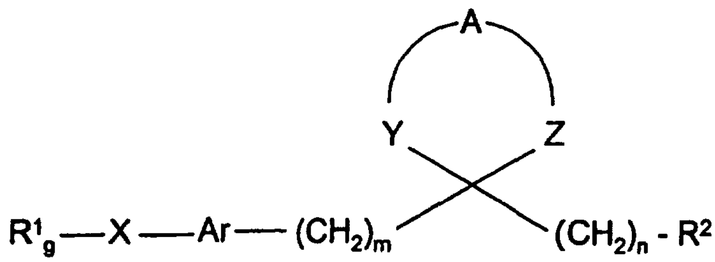

(I-s)

or a salt thereof, or

(14) subjecting a compound of the formula:

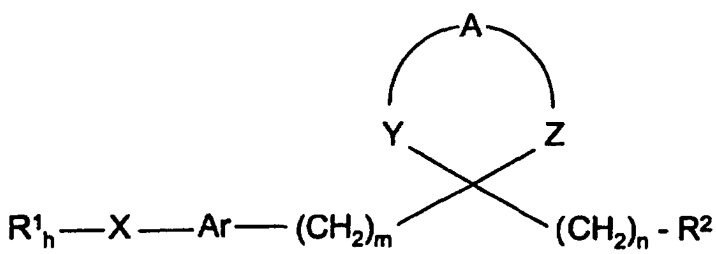

(I-t)

or a salt thereof to a removal reaction of the amino-protective group,
to give a compound of the formula:

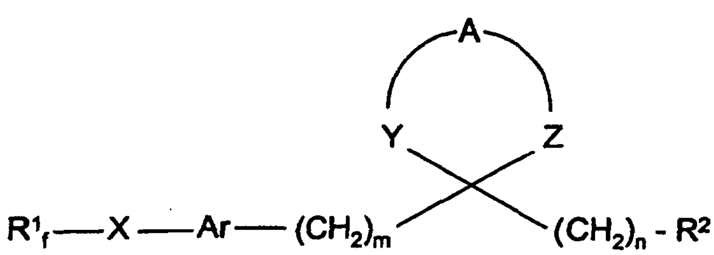

(I-r)

or a salt thereof; or

(15) subjecting a compound of the formula:

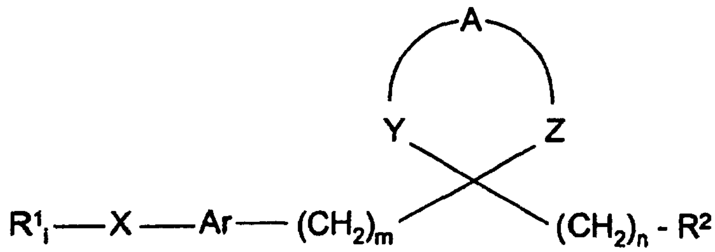

(I-u)

or a salt thereof to a removal reaction of the hydroxyprotective group, to give a compound of the formula:

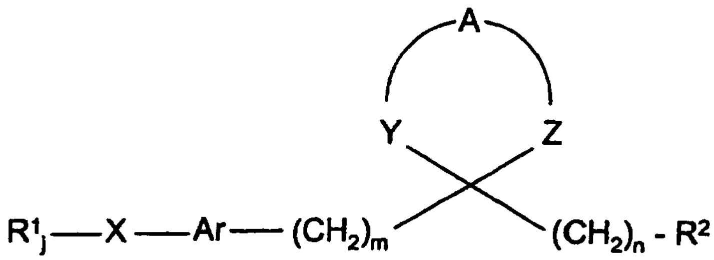

(I-v)

or a salt thereof; or

(16) oxidating a compound of the formula:

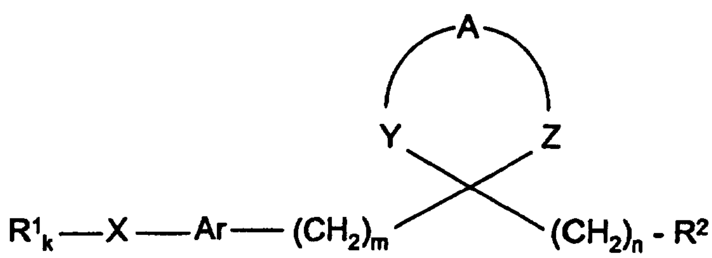

(I-w)

or a salt thereof, to give a compound of the formula:

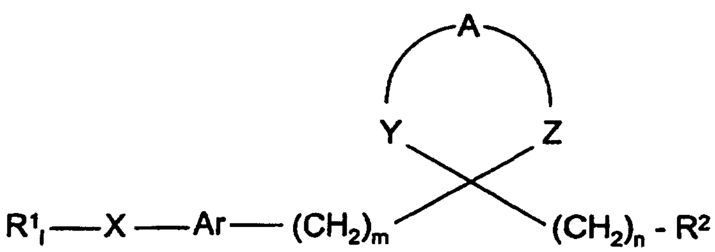

(I-x)

or a salt thereof; or

(17) reducing a compound of the formula:

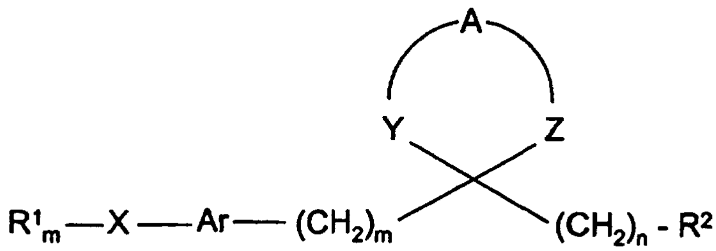

(I-y)

or a salt thereof, to give a compound of the formula:

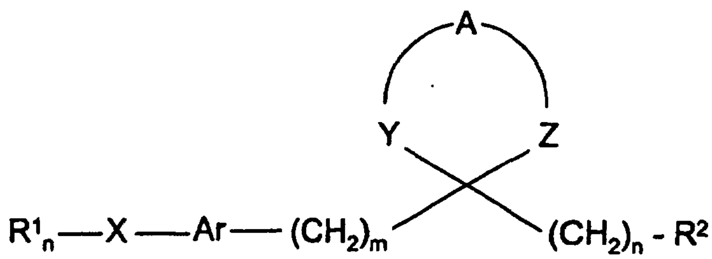

(I-z)

or a salt thereof; or

(18) oxidating a compound of the formula:

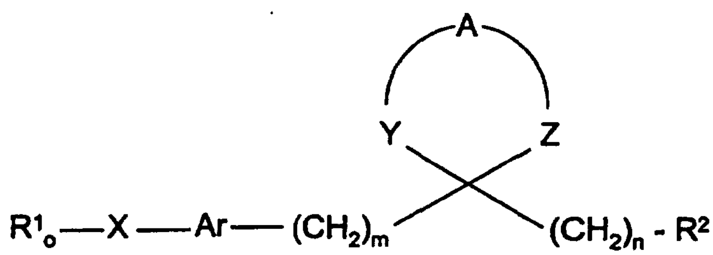

(I-aa)

or a salt thereof, to give a compound of the formula:

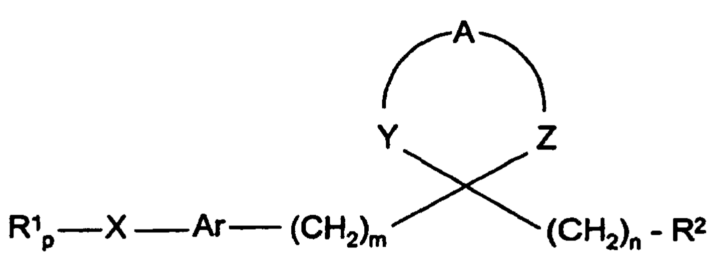

(I-ab)

or a salt thereof; or

(19) acylating a compound of the formula:

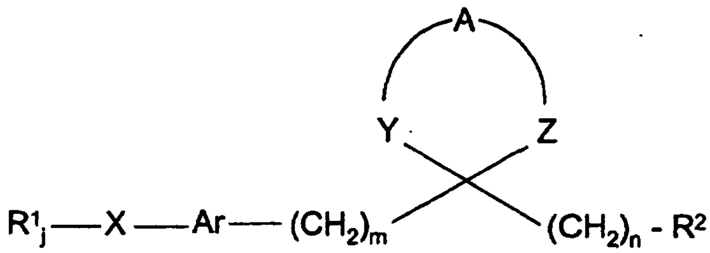

(I-v)

or a salt thereof, to give a compound of the formula:

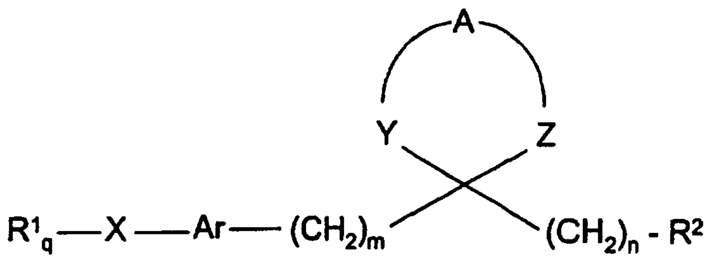

(I-ac)

or a salt thereof; or

(20) reacting a compound of the formula:

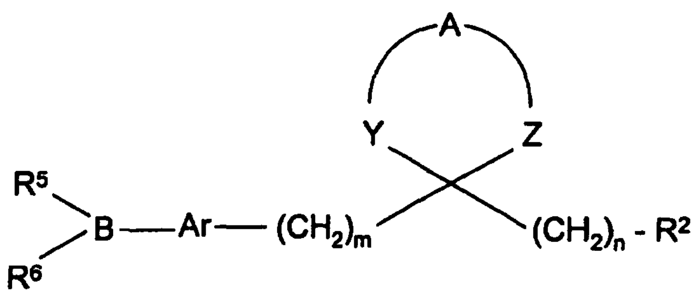

(XI)

or a salt thereof, with a compound of the formula;

$$R^1 - L \quad (XII)$$

or a satt thereof, to give a compound of the formula:

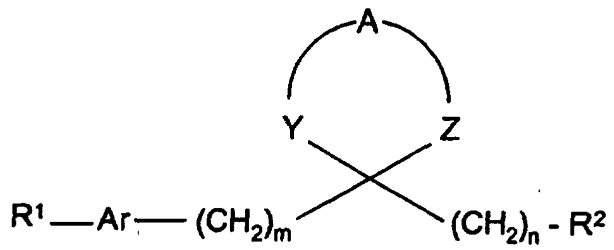

(I-ad)

or a salt thereof; or

(21) subjecting a compound of the formula:

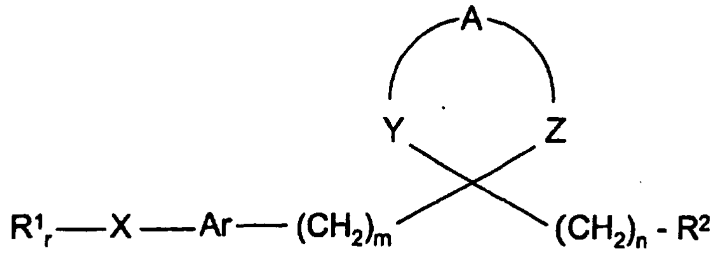

(I-ae)

or a salt thereof, to a removal reaction of the carboxy-protective group, to give a compound of the formula:

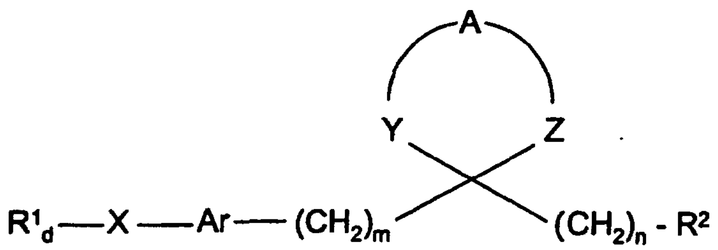

(I-p)

or a salt thereof; or

(22) reacting a compound of the formula:

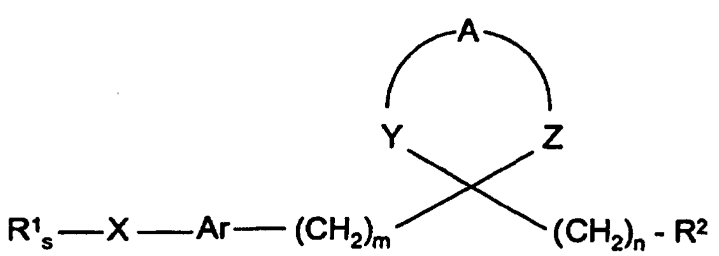

(I-af)

or a salt thereof, with a substituted amine, to give a compound of the formula:

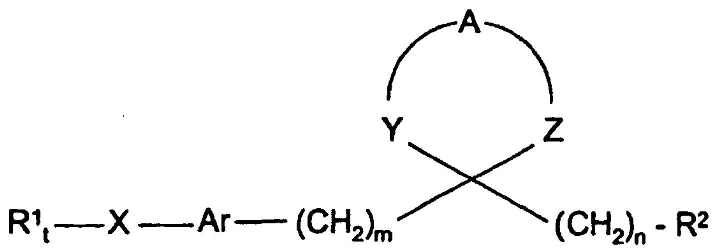

(I-ag)

or a salt thereof,

in which $R^1$, $R^2$, Ar, A, X, Y, Z, m and n are each os defined above,
$R^1_a$ is haloaryl or halo,
$R^1_b$ is aryl,
$R^1_c$ is aryl at least substituted by optionally substituted aryl,
$R^1_d$ is aryl at least having carboxy moiety,
$R^1_e$ is aryl at least having amido moiety,
$R^1_f$ is aryl at least having amino moiety,
$R^1_g$ is aryl at least having acylamino moiety,
$R^1_h$ is aryl at least having protected amino moiety,
$R^1_i$ is aryl at least having protected hydroxy moiety,
$R^1_j$ is aryl at least having hydroxy moiety,
$R^1_k$ is aryl at least having thia moiety,
$R^1_l$ is aryl at least having sulfinyl or sulfonyl moiety,
$R^1_m$ is aryl at least having formyl moiety,
$R^1_n$ is aryl at least having hydroxymethyl moiety,
$R^1_o$ is aryl at least having vinyl moiety,
$R^1_p$ is aryl at least having 1,2-dihydroxyethyl moiety,
$R^1_q$ is aryl at least having acyloxy moiety,
$R^1_r$ is aryl at least having protected carboxy moiety,
$R^1_s$ is aryl at least having halo ($C_1$-$C_6$) alkanoyl moiety,
$R^1_t$ is aryl at least having substituted amino ($C_1$-$C_6$) alkanoyl moiety,
$R^2_a$ is protected carboxy,
$R^2_b$ is optically active amide,
$R^2_c$ is protected carboxy,
$R^3$ is hydrogen or hydroxy-protective group,
$R^3_a$ is hydroxy-protective group,
$R^4$ is optionally substituted aryl,
$R^5$ and $R^6$ are each hydrogen or combined together to form ($C_1$-$C_6$) alkylene, $Y_a$ is thia or sulfinyl,
$Y_b$ is sulfinyl or sulfonyl,
L is a leaving group, and
ml is an Integer of 1 to 6.

**10.** A pharmaceutical composition which comprises the compound of any of Claims 1 to 8 or a pharmaceutically acceptable salt thereof and a pharmaceutically acceptable carrier or excipient.

**11.** A process for preparing a pharmaceutical composition which comprises admixing the compound of any of Claims 1 to 8 or a pharmaceutically acceptable salt thereof with a pharmaceutically acceptable carrier or excipient.

**12.** Use of the compound of any of Claims 1 to 8 or a pharmaceutically acceptable salt thereof for the manufacture of a medicament for the inhibition of matrix metalloproteinases (MMP) or tumor necrosis factor a (TNF $\alpha$).

**13.** Use of the compound of any of Claims 1 to 8 or a pharmaceutically acceptable salt thereof for the manufacture of a medicament for treating and/or preventing MMP- or TNF $\alpha$-mediated diseases.

## Patentansprüche

1. Eine Verbindung der Formel:

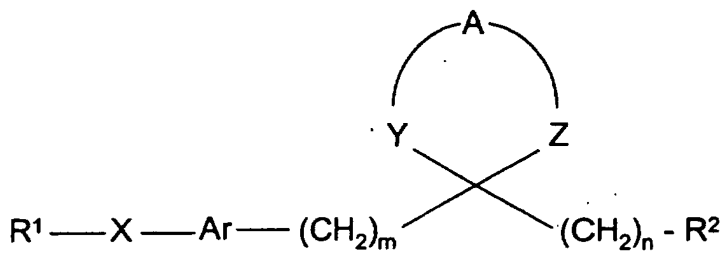

worin $R^1$ $(C_1\text{-}C_6)$ Alkyl, eine optional substituierte heterocyclische Gruppe oder optional substituiertes $(C_6\text{-}C_{10})$ Aryl ist,
$R^2$ Carboxy, geschütztes Carboxy, Hydroxyaminocarbonyl, Tetrahydropyranyloxyaminocarbonyl, oder Phenyl $(C_1\text{-}C_6)$ alkylaminocarbonyl ist,
Ar optional substituiertes $(C_6\text{-}C_{10})$ Aryl oder eine optional substituierte heterocyclische Gruppe ist,
A $(C_1\text{-}C_6)$ Alkylen ist,
X Oxa oder eine einfache Bindung ist,
Y Thia, Sulfinyl oder Sulfonyl ist,
Z Methylen ist,
m und n beide eine ganze Zahl von 0 bis 6 sind, und
$1 \leq m+n \leq 6$ ist,
und deren Salz,
wobei die heterocyclische Gruppe für $R^1$ und Ar ausgewählt wird aus der Gruppe bestehend aus den nachfolgenden (1) bis (14),

(1) ungesättigte 3- bis 8-gliedrige, heteromonocyclische Gruppe welche 1 bis 4 Stickstoffatome enthält,
(2) gesättigte 3- bis 8-gliedrige, heteromonocyclische Gruppe welche 1 bis 4 Stickstoffatome enthält,
(3) ungesättigte 3- bis 8-gliedrige, heteromonocyclische Gruppe welche 1 oder 2 Schwefelatome enthält,
(4) ungesättigte kondensierte 7- bis 13-gliedrige heterocyclische Gruppe weiche 1 bis 5 Stickstoffatome enthält,
(5) ungesättigte 3- bis 8-gliedrige, heteromonocyclische Gruppe welche 1 oder 2 Sauerstoffatome enthält,
(6) gesättigte 3- bis 8-gliedrige, heteromonocyclische Gruppe welche 1 oder 2 Sauerstoffatome enthält,
(7) ungesättigte 3- bis 8-gliedrige, heteromonocyclische Gruppe welche 1 oder 2 Sauerstoffatome und 1 bis 3 Stickstoffatome enthält,
(8) ungesättigte kondensierte 7- bis 13-gliedrige, heterocyclische Gruppe welche 1 oder 2 Sauerstoffatome enthält,
(9) ungesättigte kondensierte 7- bis 13-gliedrige, heterocyclische Gruppe welche 1 oder 2 Schwefelatome enthält,
(10) gesättigte 3- bis 8-gliedrige, heteromonocyclische Gruppe welche 1 oder 2 Sauerstoffatome und 1 bis 3 Stickstoffatome enthält,
(11) ungesättigte kondensierte 7- bis 13-gliedrige, heterocyclische Gruppe welche 1 oder 2 Sauerstoffatome und 1 bis 3 Stickstoffatome enthält,
(12) ungesättigte 3- bis 8-gliedrige, heteromonocyclische Gruppe welche 1 oder 2 Schwefelatome und 1 bis 3 Stickstoffatome enthält,
(13) gesättigte 3- bis 8-gliedrige, heteromonocyclische Gruppe welche 1 oder 2 Schwefelatome und 1 bis 3 Stickstoffatome enthält, und
(14) ungesättigte kondensierte 7- bis 13-gliedrige, heterocyclische Gruppe welche 1 oder 2 Schwefelatome und 1 bis 3 Stickstoffatome enthält, und

jede der obigen heterocyclischen Gruppen und $(C_6\text{-}C_{10})$ Arylgruppe optional mit der Gruppe bestehend aus den folgenden (A1) bis (A35) substituiert ist:

(A1) Halogen,
(A2) ($C_1$-$C_6$) Alkyl,
(A3) ($C_1$-$C_6$) Alkoxy,
(A4) Halo ($C_1$-$C_6$) alkyl,
(A5) Halo ($C_1$-$C_6$) alkoxy,
(A6) ($C_2$-$C_6$) Alkenyl,
(A7) Acyl,
(A8) ($C_1$-$C_6$) Alkylthio, ($C_1$-$C_6$) Alkylsulfinyl, ($C_1$-$C_6$) Alkylsulfonyl,
(A9) ($C_6$-$C_{10}$) Aryl,
(A10) Halo ($C_6$-$C_{10}$) aryl,
(A11) Hydroxy,
(A12) Hydroxy ($C_1$-$C_6$) alkyl, geschütztes Hydroxy ($C_1$-$C_6$) alkyl,
(A13) Amino,
(A14) Carboxy,
(A15) geschütztes Carboxy,
(A16) Nitro ($C_2$-$C_6$) alkenyl,
(A17) ($C_1$-$C_6$) Alkylendioxy,
(A18) Acylamino,
(A19) Nitro,
(A20) ($C_6$-$C_{10}$) Aryl ($C_1$-$C_6$) alkoxy,
(A21) Carbamoyl ($C_2$-$C_6$) alkenyl optional N-substituiert mit der Gruppe bestehend aus ($C_1$-$C_6$) Alkyl, ($C_6$-$C_{10}$) Aryl, ($C_1$-$C_6$) Alkoxy ($C_6$-$C_{10}$) aryl, und Halo ($C_6$-$C_{10}$) aryl,
(A22) ($C_1$-$C_6$) Alkylaminocarbonyloxy,
(A23) ($C_1$-$C_6$) Alkanoyloxy,
(A24) ($C_1$-$C_6$) Alkoxy ($C_1$-$C_6$) alkanoyloxy,
(A25) ($C_1$-$C_6$) Alkoxycarbonyloxy,
(A26) ($C_2$-$C_6$) Alkenoyloxy optional mit der heterocyclischen Gruppe gemäß den obigen (1) bis (14) substituiert,
(A27) ($C_3$-$C_6$) Cycloalkancarbonyloxy,
(A28) ($C_1$-$C_6$) Alkoxy substituiert mit der Gruppe bestehend aus Carboxy, geschütztem Carboxy. ($C_1$-$C_6$) Alkanoyl, ($C_3$-$C_7$) Cycloalkancarbamoyl, und ($C_1$-$C_6$) Alkylcarbamoyl,
(A29) ($C_1$-$C_6$) Alkylcarbamoyloxy ($C_1$-$C_6$) alkyl,
(A30) ($C_1$-$C_6$) Alkoxycarbonylamino ($C_1$-$C_6$) alkyl,
(A31) Amino ($C_1$-$C_6$) alkyl,
(A32) ($C_1$-$C_6$) Alkylcarbamoyl ($C_1$-$C_6$) alkyl,
(A33) Heterocyclus-carbonylamino, wobei die heterocyclische Gruppe aus den obigen (1) bis (14) ausgewählt ist und optional eine substituierte N-Schutzgruppe ist,
(A34) die obigen heterocyclischen Gruppen (1) bis (14) optional mit ($C_1$-$C_6$) Alkyl substituiert, und
(A35) Oxo.

**2.** Die Verbindung nach Anspruch 1, wobei
$R^1$ ($C_1$-$C_6$) Alkyl, optional substituierte heterocyclische Gruppe bestehend aus den folgenden (1) bis (10), oder optional substituiertes ($C_6$-$C_{10}$) Aryl ist;
$R^2$ Carboxy oder Hydroxyaminocarbonyl ist,
Ar Phenyl oder heterocyclische Gruppe gemäß der folgenden (3) ist, und
m und n jeweils eine ganze Zahl von 0 oder 1 sind, und m+n=1 oder 2 ist,
wobei die heterocyclische Gruppe:

(1) ungesättigte 5- oder 6-gliedrige heteromonocyclische Gruppe welche 1 bis 4 Stickstoffatome enthält,
(2) gesättigte 5- oder 6-gliedrige heteromonocyclische Gruppe welche 1 bis 4 Stickstoffatome enthält,
(3) ungesättigte 5- oder 6-gliedrige heteromonocyclische Gruppe welche 1 bis 2 Schwefelatome enthält,
(4) ungesättigte bicyclische 9- oder 10-gliedrige, heterocyclische Gruppe welche 1 bis 5 Stickstoffatome enthält,
(5) ungesättigte 5- oder 6-gliedrige heteromonocyclische Gruppe welche 1 bis 2 Sauerstoffatome enthält,
(6) gesättigte 5- oder 6-gliedrige, heteromonocyclische Gruppe welche 1 oder 2 Sauerstoffatome enthält,
(7) ungesättigte 5- oder 6-gliedrige, heteromonocyclische Gruppe welche 1 oder 2 Sauerstoffatome und 1 bis 3 Stickstoffatome enthält,
(8) ungesättigte bicyclische 9- oder 10-gliedrige, heterocyclische Gruppe welche 1 oder 2 Sauerstoffatome enthält,

(9) ungesättigte bicyclische 9- oder 10-gliedrige, heterocyclische Gruppe welche 1 oder 2 Schwefelatome enthält, oder

(10) gesättigte 5- oder 6-gliedrige, heteromonocyclische Gruppe weiche 1 oder 2 Sauerstoffatome und 1 bis 3 Stickstoffatome enthält, ist,

wobei die heterocyclische Gruppe optional mit der Gruppe bestehend aus den folgenden (B1) bis (B8):

(B1) ($C_1$-$C_6$) Alkanoyl,
(B2) ($C_1$-$C_6$) Alkyl,
(B3) ($C_1$-$C_6$) Alkoxy,
(B4) ($C_1$-$C_6$) Alkoxycarbonylamino,
(B5) Carbamoyl oder ($C_1$-$C_6$) Alkylcarbamoyl,
(B6) ($C_1$-$C_6$) Alkoxycarbonyl,
(B7) Halo, und
(B8) Oxo,

substituiert ist;
und das obige Aryl optional mit der Gruppe bestehend aus (A1) bis (A35) gemäß der Definition in Anspruch 1 substituiert Ist.

**3.** Die Verbindung nach Anspruch 2, wobei eine Gruppe der Formel:

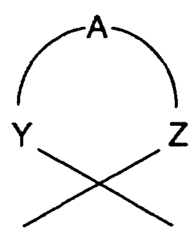

eine der folgenden Formeln darstellt:

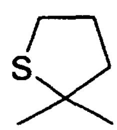 , 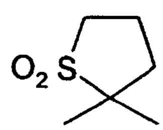 ,

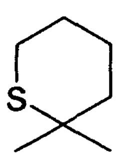 , 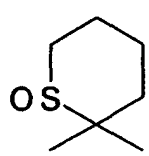 , 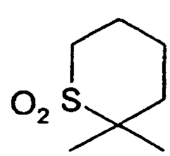 or 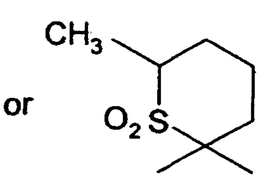 ,

wobei
$R^1$ ($C_1$-$C_6$) Alkyl; optional substituierte heterocyclische Gruppe bestehend aus den folgenden (1) bis (10); optional substituiertes Phenyl; oder optional substituiertes Naphtyl ist;
$R^2$ wie in Anspruch 2 definiert ist,
Ar Phenyl oder Thienyl ist, und
m und n jeweils eine ganze Zahl von 0 oder 1 sind, und m+n=1 oder 2 ist,

wobei die obige heterocyclische Gruppe

(1) Pyrrolyl, Pyrrolinyl, Imidazolyl, Pyrazolyl, Pyridyl, Pyridyl N-oxid, Pyrimidyl, Pyrazinyl, Pyridazinyl, Triazolyl, Tetrazolyl, Dihydrotriazinyl,
(2) Azetidinyl, Pyrrolidinyl, Imidazolidinyl, Piperidinyl, Piperidino, Pyrazolidinyl, Piperazinyl,
(3) Thienyl,
(4) Indolyl, Isoindolyl, Indolizinyl, Benzimidazolyl, Chinolyl, Isochinolyl, Tetrahydroisochinolyl, Indazolyl, Benzotriazolyl, Tetrazolopyridyl, Tetrazolopyridazinyl, Dihydrotriazolopyridazinyl,
(5) Furyl,
(6) Oxolanyl,
(7) Oxazolyl, Isoxazolyl, Oxadiazolyl,
(8) Benzofuranyl, Benzodihydrofuranyl, Benzodioxolenyl,
(9) Benzothienyl, Dihydrobenzothienyl, oder
(10) Morpholinyl, Morpholino, ist,

wobei die heterocyclische Gruppe optional durch die Gruppe bestehend aus (B1) bis (B8), wie in Anspruch 2 definiert, substituiert ist, und das genannte Phenyl oder Naphthyl optional durch die Gruppe bestehend aus den folgenden (A1) bis (A34) substituiert ist,

(A1) Halogen,
(A2) ($C_1$-$C_6$) Alkyl,
(A3) ($C_1$-$C_6$) Alkoxy,
(A4) Halo ($C_1$-$C_6$) alkyl,
(A5) Halo ($C_1$-$C_6$) alkoxy,
(A6) ($C_2$-$C_6$) Alkenyl,
(A7) Acyl,
(A8) ($C_1$-$C_6$) Alkylthio, ($C_1$-$C_6$) Alkylsulfinyl, ($C_1$-$C_6$) Alkylsulfonyl,
(A9) ($C_6$-$C_{10}$) Aryl,
(A10) Halo($C_6$-$C_{10}$) aryl,
(A11) Hydroxy,
(A12) Hydroxy ($C_1$-$C_6$) alkyl oder gechütztes Hydroxy ($C_1$-$C_6$) alkyl,
(A13) Amino,
(A14) Carboxy,
(A15) geschütztes Carboxy,
(A16) Nitro ($C_2$-$C_6$) alkenyl,
(A17) ($C_1$-$C_6$) Alkylendioxy,
(A18) Acylamino,
(A19) Nitro,
(A20) ($C_6$-$C_{10}$) Aryl ($C_1$-$C_6$) alkoxy,
(A21) Carbamoyl ($C_2$-$C_6$) alkenyl optional N-substituiert mit der Gruppe bestehend aus ($C_1$-$C_6$) Alkyl, ($C_6$-$C_{10}$) Aryl, ($C_1$-$C_6$) Alkoxy ($C_6$-$C_{10}$) aryl, und
Halo($C_6$-$C_{10}$) aryl,
(A22) ($C_1$-$C_6$) Alkylaminocarbonyloxy,
(A23) ($C_1$-$C_6$) Alkanoyloxy,
(A24) ($C_1$-$C_6$) Alkoxy ($C_1$-$C_6$) alkanoyloxy,
(A25) ($C_1$-$C_6$) Alkoxycarbonyloxy,
(A26) ($C_2$-$C_6$) Alkenoyloxy optional substituiert mit der obigen heterocyclischen Gruppe (1),
(A27) ($C_3$-$C_6$) Cycloalkancarbonyloxy,
(A28) ($C_1$-$C_6$) Alkoxy substituiert mit der Gruppe bestehend aus Carboxy, geschützem Carboxy, ($C_1$-$C_6$) Alkanoyl, ($C_3$-$C_7$) Cycloalkoncarbamoyl, und ($C_1$-$C_6$) Alkylcarbamoyl,
(A29) ($C_1$-$C_6$) Alkylcarbamoyloxy ($C_1$-$C_6$) alkyl,
(A30) ($C_1$-$C_6$) Alkoxycarbonylamino ($C_1$-$C_6$) alkyl,
(A31) Amino ($C_1$-$C_6$) alkyl,
(A32) ($C_1$-$C_6$) Alkylcarbamoyl ($C_1$-$C_6$) alkyl,
(A33) Heterocyclus-carbonylamino, wobei die heterocyclische Gruppe ausgewählt ist aus der Gruppe bestehend aus den obigen (2), (4) und (5) und optional mit einer N-Schutzgruppe substituiert ist, und
(A34) die heterocyclische Gruppe gemäß dem obigen (7) optional mit ($C_1$-$C_6$) Alkyl substituiert ist.

**4.** Die Verbindung noch Anspruch 3, mit der folgenden Formel:

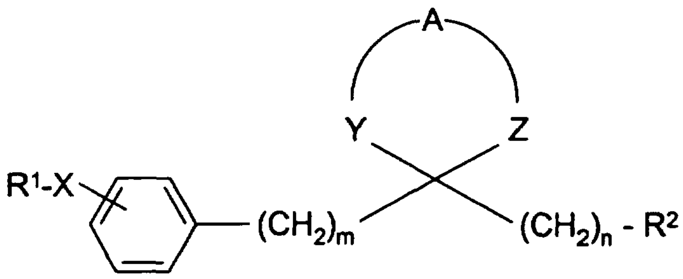

wobei eine Gruppe der Formel:

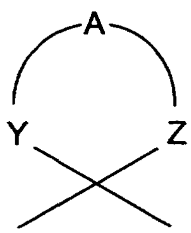

die gleiche ist wie in Anspruch 3 definiert,
$R^1$ ($C_1$-$C_6$) Alkyl, Phenyl, Halophenyl, oder (Halo)(phenyl)phenyl ist,
$R^2$ gleich ist wie in Anspruch 3 definiert, und
m und n jeweils eine ganze Zahl von 0 oder 1 sind, und m+n=1 ist,

**5.** Die Verbindung nach Anspruch 3, mit der folgenden Formel :

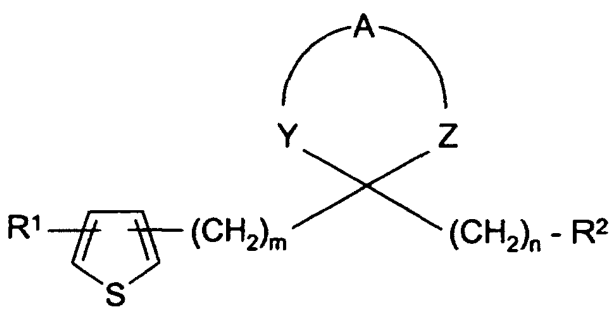

wobei eine Gruppe der Formel:

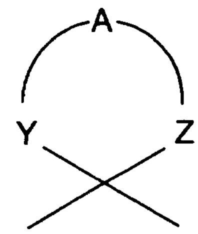

die gleiche ist wie in Anspruch 3 definiert,
$R^2$ gleich ist wie in Anspruch 3 definiert,
m und n jeweils eine ganze Zahl von 0 oder 1 sind , und m+n=1 ist,
$R^1$ eine heterocyclische Gruppe bestehend aus Pyridyl, Thienyl, Furyl, Benzofuranyl oder Benzothienyl ist, wobei die heterocyclische Gruppe optional substituiert ist mit der Gruppe bestehend aus ($C_1$-$C_6$) Alkanoyl, ($C_1$-$C_6$) Alkyl, ($C_1$-$C_6$) Alkoxy, ($C_1$-$C_6$) Alkoxycarbonylamino und ($C_1$-$C_6$) Alkylcarbamoyl; Naphtyl oder Phenyl, jedes optional mit der Gruppe bestehend aus den folgenden (C1) bis (C31) substituiert:

(C1) Halogen,
(C2) ($C_1$-$C_6$) Alkyl,
(C3) ($C_1$-$C_6$) Alkoxy,
(C4) Halo ($C_1$-$C_6$) alkyl,
(C5) Halo ($C_1$-$C_6$) alkoxy,
(C6) ($C_2$-$C_6$) Alkenyl,
(C7) ($C_1$-$C_6$) Alkylcarbamoyl, Carbamoyl, Phenyl ($C_1$-$C_6$) alkylcarbamoyl, ($C_1$-$C_6$) alkanoyl,
(C8) ($C_1$-$C_6$) Alkylthio, ($C_1$-$C_6$) Alkylsulfinyl, ($C_1$-$C_6$) Alkylsulfonyl,
(C9) Phenyl, Naphthyl,
(C10) Halophenyl,
(C 11) Hydroxy,
(C12) Mono- oder Dihydroxy ($C_1$-$C_6$) alkyl, Phenoxycarbonyloxy ($C_1$-$C_6$) alkyl,
(C 13) Amino,
(C14) Carboxy,
(C15) ($C_1$-$C_6$) Alkylendioxy,
(C16) ($C_1$-$C_6$) Alkanoylamino, Phenyl ($C_1$-$C_6$) alkanoylomino, Halophenyl ($C_1$-$C_6$) alkanoylamino, ($C_1$-$C_6$) Alkoxy ($C_1$-$C_6$) alkanoylamino, Phenoxy ($C_1$-$C_6$) alkanoylamino, ($C_1$-$C_6$) Alkoxyphenoxy ($C_1$-$C_6$) alkanoylomino, ($C_1$-$C_6$) Alkylphenoxy ($C_1$-$C_6$) alkanoylamino, Halophenoxy ($C_1$-$C_6$) alkanoylamino, Carboxy ($C_1$-$C_6$) alkanoylamino, ($C_1$-$C_6$) Alkoxycarbonyl ($C_1$-$C_6$) alkanoylamino, ($C_1$-$C_6$) alkylcarbamoyl ($C_1$-$C_6$) alkanoylamino, Halo ($C_1$-$C_6$) alkanoylamino, ($C_2$-$C_6$) Alkenyl ($C_1$-$C_6$) alkanoylamino, ($C_1$-$C_6$) Alkoxy ($C_1$-$C_6$) alkanoylamino, Phenyl ($C_1$-$C_6$) alkoxy ($C_1$-$C_6$) alkanoylamino, Piperidinyloxy ($C_1$-$C_6$) alkanoylamino, N- ($C_1$-$C_6$) Alkoxycarbonylpiperidinyloxy ($C_1$-$C_6$) alkanoylamino, Pyridyloxy ($C_1$-$C_6$) alkanoylamino, Hydroxy ($C_1$-$C_6$) alkanoylamino, ($C_1$-$C_6$) Alkanoyloxy ($C_1$-$C_6$) alkanoylamino, ($C_1$-$C_6$) Alkylcarbamoyloxy ($C_1$-$C_6$) alkanoylamino, N, N-Di (($C_1$-$C_6$) alkyl) carbamoyloxy, ($C_1$-$C_6$) Alkanoylamino, Piperidino-carbonyloxy ($C_1$-$C_6$) alkanoylamino, Phenyl ($C_1$-$C_6$) alkylcarbamoyloxy ($C_1$-$C_6$) alkanoylamino, Amino ($C_1$-$C_6$) alkanoylamino, ($C_1$-$C_6$) Alkoxycarbonylamino ($C_1$-$C_6$) alkanoylamino, Fluorenylmethoxycarbonylamino ($C_1$-$C_6$) alkanoylamino, ($C_1$-$C_6$) Alkylamino ($C_1$-$C_6$) alkanoylamino, [N,N Di(($C_1$-$C_6$) alkyl)amino]( $C_1$-$C_6$)alkanoylamino, [N-($C_1$-$C_6$) Alkyl-N-(($C_1$-$C_6$) alkoxycarbonyl) amino] ($C_1$-$C_6$) alkanoylamino, [N-($C_1$-$C_6$) Alkyl-N-(fluorenylmethoxycarbonyl)amino] ($C_1$-$C_6$) alkanoylamino, [N-($C_1$-$C_6$)- Alkyl-N-(mono- or di($C_1$-$C_6$) alkylcarbamoyl)amino] ($C_1$-$C_6$) alkanoylamino, [N-(Mono- oder Di(($C_1$-$C_6$) alkyl)carbamoyl) amino] ($C_1$-$C_6$) alkanoylamino, Benzoylamino ($C_1$-$C_6$) alkanoylamino, ($C_1$-$C_6$) Alkanoylamino ($C_1$-$C_6$) alkanoylamino, ($C_1$-$C_6$) Alkansulfonylamino ($C_1$-$C_6$) alkanoylamino, ($C_1$-$C_6$) Alkoxy ($C_1$-$C_6$) alkanoylamino ($C_1$-$C_6$) alkanoylamino, Cyclo ($C_3$-$C_6$) alkyloxycarbonylamino ($C_1$-$C_6$) alkanoylamino, Pyridylcarbonylamino ($C_1$-$C_6$) alkanoylamino, Morpholinocarbonylamino ($C_1$-$C_6$)- alkanoylamino, Phenyl ($C_1$-$C_6$) alkoxycarbonylamino ($C_1$-$C_6$) Alkanoylamino, ($C_1$-$C_6$) Alkoxyphenylsulfonylamino, ($C_1$-$C_6$) Alkanoylamino, Hydroxy ($C_1$-$C_6$) alkylamino ($C_1$-$C_6$) alkanoylamino, Morpholino ($C_1$-$C_6$) alkanoylamino, Oxooxazolidinyl ($C_1$-$C_6$) alkanoylamino, Oxopyrrolidinyl ($C_1$-$C_6$) alkanoylamino, Trimethylhydantoinyl ($C_1$-$C_6$) alkanoylamino, ($C_2$-$C_6$) Alkenylamino ($C_1$-$C_6$) alkanoylamino, ($C_1$-$C_6$) Alkoxy ($C_1$-$C_6$) alkylamino ($C_1$-$C_6$) alkanoylamino, Phenyl ($C_1$-$C_6$) alkylamino ($C_1$-$C_6$) alkanoylamino, Pyridyl ($C_1$-$C_6$) alkylamino ($C_1$-$C_6$) alkanoylamino, ($C_1$-$C_6$) Alkoxycarbonylamino, Phenyl ($C_1$-$C_6$) alkoxycarbonylamino, ($C_1$-$C_6$) Alkoxy ($C_1$-$C_6$) alkoxycarbonylamino, Halo ($C_1$-$C_6$) alkoxycarbonylamino, Amino ($C_1$-$C_6$) alkoxycarbonylamino, Phthalimido ($C_1$-$C_6$) alkoxy-

carbonylamino, Carbamoylamino, (Mono- oder Di(($C_1$-$C_6$) alkyl) carbamoylamino, Naphthylcarbamoylamino, Halophenylcarbamoylamino, ($C_1$-$C_6$) Alkoxyphenylcarbamoylamino, ($C_2$-$C_6$) Alkenylcarbamoylamino, Cyclo ($C_3$-$C_6$) alkyl ($C_1$-$C_6$) alkylcarbamoylamino, Phenyl ($C_1$-$C_6$) alkylcarbamoylamino, Halo ($C_1$-$C_6$) alkylcarbamoylamino, ($C_1$-$C_6$) Alkoxy ($C_1$-$C_6$) alkylcarbamoylamino, Hydroxy ($C_1$-$C_6$) alkylcarbamoylamino, (($C_1$-$C_6$) Alkyl)(diphenyl) silyloxy ($C_1$-$C_6$) alkyl carbamoylamino, Carboxy ($C_1$-$C_6$) alkylcarbamoylamino, ($C_1$-$C_6$) Alkoxycarbonyl ($C_1$-$C_6$) alkylcarbamoylamino, ($C_1$-$C_6$) Alkylcarbamoyl ($C_1$-$C_6$) alkyl carbamoylamino, oder Pyridylcarbamoylamino, ($C_1$-$C_6$) Alkylsulfonylamino, ($C_2$-$C_6$) Alkenoylamino, ($C_3$-$C_6$) Cycloalkancarbonylamino, ($C_2$-$C_6$) Alkenyloxycarbonylamino, Phenoxycarbonylamino, ($C_1$-$C_6$) Alkylthiocarbonylamino,
(C17) Phenyl ($C_1$-$C_6$) alkoxy,
(C18) ($C_2$-$C_6$) Alkenyl, Mono- oder Di($C_1$-$C_6$) alkyl) carbamoyl($C_2$-$C_6$) alkenyl, 2-(Methylcarbamoyl) ethenyl, 2-(Ethylcarbamoyl)ethenyl, 2-(Propylcarbamoyl)ethenyl, 2-(Isopropylcarbamoyl) ethenyl, 2-(Dimethylcarbamoyl) ethenyl, Phenylcarbamoyl ($C_2$-$C_6$) alkenyl, ($C_1$-$C_6$) Alkoxycarbamoyl ($C_2$-$C_6$) alkenyl, Halophenylcarbamoyl ($C_2$-$C_6$) alkenyl,
(C19) ($C_1$-$C_6$) Alkylaminocarbonyloxy,
(C20) ($C_1$-$C_6$) Alkanoyloxy,
(C21) ($C_1$-$C_6$) Alkoxy ($C_1$-$C_6$) alkanoyloxy,
(C22) ($C_1$-$C_6$) Alkoxycarbonyloxy,
(C23) Pyridyl ($C_2$-$C_6$) alkenoyloxy,
(C24) ($C_3$-$C_6$) Cycloalkancarbonyloxy,
(C25) Carboxy ($C_1$-$C_6$) alkoxy, ($C_1$-$C_6$) Alkoxycarbonyl ($C_1$-$C_6$) alkoxy, ($C_1$-$C_6$) Alkanoyl ($C_1$-$C_6$) alkoxy, ($C_3$-$C_7$) Cycloalkoncorbamoyl ($C_1$-$C_6$) alkoxy, ($C_1$-$C_6$) Alkylcarbamoyl ($C_1$-$C_6$) alkoxy,
(C26) ($C_1$-$C_6$) Alkylcarbamoyloxy ($C_1$-$C_6$) alkyl,
(C27) ($C_1$-$C_6$) Alkoxycarbonylamino ($C_1$-$C_6$) alkyl,
(C28) Amino ($C_1$-$C_6$) alkyl,
(C29) ($C_1$-$C_6$) Alkylcarbamoyl ($C_1$-$C_6$) alkyl,
(C30) Furylcarbonylamino, Tetrahydroisochinolylcarbonylamino, N-($C_1$-$C_6$) Alkoxycarbonyltetrahydroisochinolylcarbonylamino, Pyrrolidinylcarbonylamino,
(C31) Ooxazolyl, ($C_1$-$C_6$) Alkyloxadiazolyl.

**6.** Die Verbindung nach Anspruch 5, wobei die Gruppe der Formel:

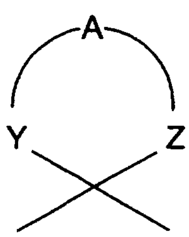

eine der folgenden Formeln darstellt:

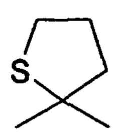 , 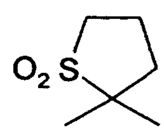 ,

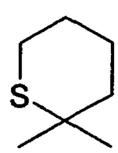 , 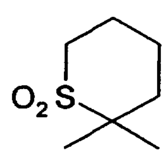 ,

$R^2$ gleich ist wie in Anspruch 5 definiert,
m = 0 ist und n = 1 ist, die Gruppe der Formel:

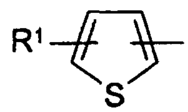

eine Gruppe der folgenden Formeln (a) bis (e) darstellt;

(a)

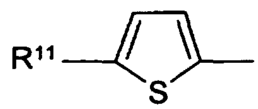

wobei
$R^{11}$ Halogen, Naphtyl, Phenyl, Mono- oder Dihalophenyl, Mono- oder Di ($C_1$-$C_6$) alkylphenyl, ($C_1$-$C_6$) Alkoxyphenyl, Trihalo ($C_1$-$C_6$) alkylphenyl, Trihalo ($C_1$-$C_6$) alkoxyphenyl, ($C_2$-$C_6$) Alkenylphenyl, ($C_1$-$C_6$) Alkylcarbamoylphenyl, Carbamoylphenyl, Phenyl ($C_1$-$C_6$) alkylcarbamoylphenyl, ($C_1$-$C_6$) Alkonoylphenyl, ($C_1$-$C_6$) Alkylthiophenyl, ($C_1$-$C_6$) Alkylsulfinylphenyl, ($C_1$-$C_6$) Alkylsulfonylphenyl, Phenylphenyl, (Halo)(phenyl)phenyl, Halophenylphenyl, Hydroxyphenyl, Mono- oder Dihydroxy ($C_1$-$C_6$) alkylphenyl, Phenoxycorbonyloxy ($C_1$-$C_6$) alkylphenyl, Aminophenyl, Corboxyphenyl, ($C_1$-$C_6$) Alkylendioxyphenyl, ($C_1$-$C_6$) Alkansulfonylaminophenyl, ($C_2$-$C_6$) Alkenoylaminophenyl, ($C_3$-$C_6$) Cycloalkancarbonylaminophenyl, Phenyl ($C_1$-$C_6$) alkoxyphenyl, Mono- oder Di (($C_1$-$C_6$) alkyl) carbamoyl ($C_2$-$C_6$) alkenylphenyl, Phenylcarbamoyl ($C_2$-$C_6$) alkenylphenyl, ($C_1$-$C_6$) Alkoxycarbamoyl ($C_2$-$C_6$) alkenylphenyl, Halophenylcarbomoyl ($C_2$-$C_6$) alkenylphenyl, ($C_1$-$C_6$) Alkylcarbamoyloxyphenyl, ($C_1$-$C_6$) Alkanoyloxyphenyl, ($C_1$-$C_6$) Alkoxy ($C_1$-$C_6$) alkanoyloxyphenyl, ($C_1$-$C_6$) Alkoxycarbonyloxyphenyl, Pyridyl ($C_2$-$C_6$) alkenoyloxyphenyl, Cyclo ($C_3$-$C_6$) alkylcarbonyloxyphenyl, Carboxy ($C_1$-$C_6$) alkoxyphenyl, ($C_1$-$C_6$) Alkoxycarbonyl ($C_1$-$C_6$) alkoxyphenyl, ($C_1$-$C_6$) Alkanoyl ($C_1$-$C_6$) alkoxyphenyl, ($C_3$-$C_6$) Cycloalkancarbamoyl ($C_1$-$C_6$) alkoxyphenyl, ($C_1$-$C_6$) Alkylcarbamoyl ($C_1$-$C_6$) alkoxyphenyl, ($C_1$-$C_6$) Alkylcarbamoyloxy ($C_1$-$C_6$) alkylphenyl, ($C_1$-$C_6$) Alkoxycarbonylamino ($C_1$-$C_6$) alkylphenyl, Amino ($C_1$-$C_6$) alkylphenyl, ($C_1$-$C_6$) Alkylcarbamoyl ($C_1$-$C_6$) alkylphenyl, Furylcarbonylaminophenyl, 1,2,3,4-Tetrahydroisochinolylcarbonylaminophenyl, N-t-Butoxycarbonyl, 1,2,3,4-Tetrahydroisochinolylcarbonylaminophenyl, Pyrrolidinylcarbonylaminophenyl, Oxazolylphenyl, ($C_1$-$C_6$) Alkyloxadiazolylphenyl,
ist;

(b)

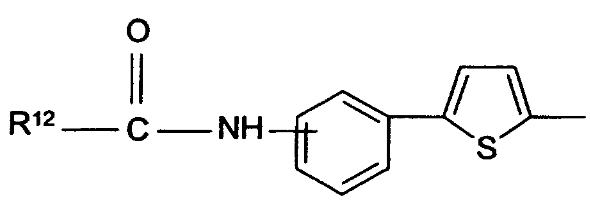

wobei $R^{12}$ ($C_1$-$C_6$) Alkyl optional substituiert mit der Gruppe bestehend aus Phenyl, Halophenyl, ($C_1$-$C_6$) Alkoxyphenyl, ($C_1$-$C_6$) Alkoxy, Phenoxy, ($C_1$-$C_6$) Alkoxyphenoxy, Halophenoxy, ($C_1$-$C_6$) Alkylphenoxy, Carboxy, ($C_1$-$C_6$) Alkoxycarbonyl, ($C_1$-$C_6$) Alkylcarbamoyl, Halo, ($C_2$-$C_6$) Alkenyloxy, ($C_1$-$C_6$) Alkoxy ($C_1$-$C_6$) alkoxy, Phenyl ($C_1$-$C_6$) alkoxy, Piperidinyloxy, N-($C_1$-$C_6$) Alkoxycarbonyl-piperidinyloxy, Pyridyloxy, Hydroxy, ($C_1$-$C_6$) Alkanoyloxy, Mono- oder Di ($C_1$-$C_6$) alkylcarbamoyloxy, Piperidinylcarbonyloxy, Phenyl ($C_1$-$C_6$) alkylcarbamoyloxy, Amino, ($C_1$-$C_6$) Alkoxycarbonylamino, Fluorenylmethoxycarbonylamino, Mono- oder Di ($C_1$-$C_6$) alkylamino, N-($C_1$-$C_6$) Alkyl-N-(($C_1$-$C_6$) alkoxycarbonyl)amino, N-($C_1$-$C_6$) Alkyl-N-(fluorenylmethoxycarbonyl) amino, N-($C_1$-$C_6$) Alkyl-N (mono- or di ($C_1$-$C_6$) alkylcarbamoyl)amino, N-(Monooder Di(($C_1$-$C_6$) alkyl)carba-

moyl)amino, Benzoylamino, $(C_1-C_6)$ Alkanoylamino, $(C_1-C_6)$ Alkansulfonylamino, $(C_1-C_6)$ Alkoxy $(C_1-C_6)$ alkanoylamino, Cyclo $(C_3-C_6)$ alkyloxycarbonylamino, Pyridylcarbonylamino, Morpholinocarbonylamino, Phenyl $(C_1-C_6)$ alkoxycarbonylamino, $(C_1-C_6)$ Alkoxyphenylsulfonylamino, Hydroxy $(C_1-C_6)$ alkylamino, Morpholino, Oxooxazolidinyl, Oxopyrrolidinyl, Trimethylhydontoinyl, Pyridyl, $(C_2-C_6)$ Alkenylamino, $(C_1-C_6)$ Alkoxy $(C_1-C_6)$ alkylamino, Phenyl $(C_1-C_6)$ alkylamino, Pyridyl $(C_1-C_6)$ alkylamino, und Cyclo $(C_3-C_6)$ alkyl, ist;

(c)

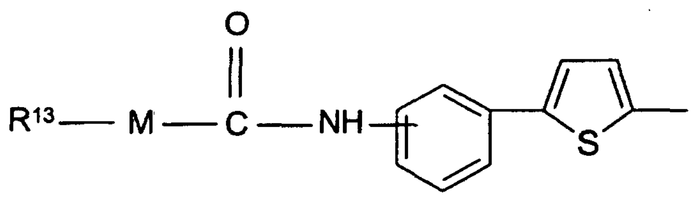

wobei
M Sauerstoff oder Schwefel ist,
$R^{13}$ $(C_1-C_6)$ Alkyl, Phenyl $(C_1-C_6)$ alkyl, $(C_1-C_6)$ Alkoxy $(C_1-C_6)$ alkyl, Halo $(C_1-C_6)$ alkyl, Amino $(C_1-C_6)$ alkyl, oder Phthalimido $(C_1-C_6)$ alkoxycarbonylamino, $(C_2-C_6)$ Alkenyl, Phenyl, ist;

(d)

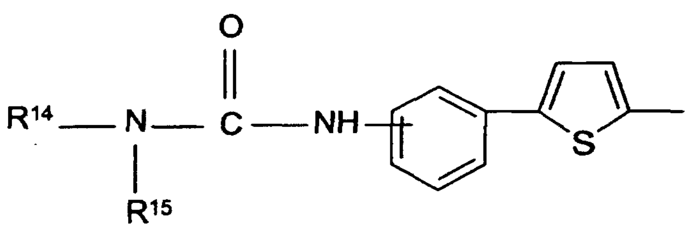

wobei
$R^{15}$ Wasserstoff oder $(C_1-C_6)$ Alkyl ist,
$R^{14}$ Wasserstoff, $(C_1-C_6)$ Alkyl, Naphthyl, Halophenyl, $(C_1-C_6)$ Alkoxyphenyl, $(C_2-C_6)$ Alkenyl, $(C_3-C_6)$ Cycloalkyl $(C_1-C_6)$ alkyl, Phenyl $(C_1-C_6)$ alkyl, Halo $(C_1-C_6)$ alkyl, $(C_1-C_6)$ Alkoxy $(C_1-C_6)$ alkyl, Hydroxy $(C_1-C_6)$ alkyl, $((C_1-C_6)$ Alkyl)(diphenyl)silyloxy $(C_1-C_6)$ alkyl, Corboxy $(C_1-C_6)$ alkyl, $(C_1-C_6)$ Alkoxycarbonyl $(C_1-C_6)$ alkyl, $(C_1-C_6)$ Alkylcarbamoyl $(C_1-C_6)$ alkyl, oder Pyridyl, ist;

(e)

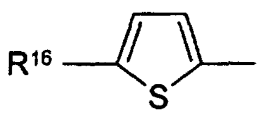

wobei
$R^{16}$ Benzothienyl, Benzofuranyl, Thienyl, Furyl, $(C_1-C_6)$ Alkylpyridyl, Pyridyl, $(C_1-C_6)$ Alkoxypyridyl, $(C_1-C_6)$ Alkoxycarbonylaminopyridyl, $(C_1-C_6)$ Alkanoylthienyl, $(C_1-C_6)$ Alkylcarbamoylbenzofuranyl, ist.

**7.** Die Verbindung nach Anspruch 6, wobei die Gruppe der Formel:

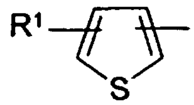

die gleiche Gruppe ist wie (a), (c), (d) und (e) des Anspruchs 6, und die folgende Formel (b):

(b)

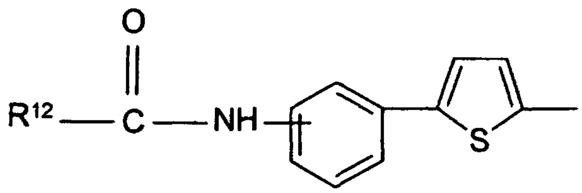

wobei

$R^{12}$ ($C_1$-$C_6$) Alkyl, Phenyl ($C_1$-$C_6$) alkyl, Halophenyl ($C_1$-$C_6$) alkyl, ($C_1$-$C_6$) Alkoxyphenyl ($C_1$-$C_6$) alkyl, ($C_1$-$C_6$) Alkoxy ($C_1$-$C_6$) alkyl, Phenoxy ($C_1$-$C_6$) alkyl,

($C_1$-$C_6$) Alkoxyphenoxy ($C_1$-$C_6$) alkyl, Halophenoxy ($C_1$-$C_6$) alkyl, ($C_1$-$C_6$) Alkylphenoxy ($C_1$-$C_6$) alkyl, Carboxy ($C_1$-$C_6$) alkyl, ($C_1$-$C_6$) Alkoxycarbonyl ($C_1$-$C_6$) alkyl, ($C_1$-$C_6$) Alkylcarbamoyl ($C_1$-$C_6$) alkyl, Halo ($C_1$-$C_6$) alkyl, ($C_2$-$C_6$) Alkenyloxy ($C_1$-$C_6$) alkyl, ($C_1$-$C_6$) Alkoxy ($C_1$-$C_6$) alkoxy ($C_1$-$C_6$) alkyl, Phenyl ($C_1$-$C_6$) alkoxy ($C_1$-$C_6$) alkyl, Piperidinyloxy ($C_1$-$C_6$) alkyl, N-t-Butoxycarbonylpiperidinyloxy ($C_1$-$C_6$) alkyl, Pyridyloxy ($C_1$-$C_6$) alkyl, Hydroxy ($C_1$-$C_6$) alkyl, ($C_1$-$C_6$) Alkanoyloxy ($C_1$-$C_6$) alkyl, Mono- oder Di ($C_1$-$C_6$) alkylcarbamoyloxy ($C_1$-$C_6$) alkyl, Piperidinylcarbonyloxy ($C_1$-$C_6$) alkyl, Phenyl ($C_1$-$C_6$) alkylcarbamoyloxy ($C_1$-$C_6$) alkyl, Amino ($C_1$-$C_6$) alkyl, ($C_1$-$C_6$) Alkoxycarbonylamino ($C_1$-$C_6$) alkyl, Fluorenylmethoxycarbonylamino ($C_1$-$C_6$) alkyl, Mono- oder Di ($C_1$-$C_6$) alkylamino ($C_1$-$C_6$) alkyl, N-($C_1$-$C_6$) Alkyl-N-(($C_1$-$C_6$) alkoxycarbonyl)amino ($C_1$-$C_6$) alkyl, N-($C_1$-$C_6$) Alkyl-N-(fluorenylmethoxycarbonyl) amino ($C_1$-$C_6$) alkyl, N-($C_1$-$C_6$) Alkyl-N-(mono- or di ($C_1$-$C_6$) alkylcarbamoyl) amino ($C_1$-$C_6$) alkyl, N-(Mono- oder Di (($C_1$-$C_6$) alkyl)carbamoyl)amino ($C_1$-$C_6$) alkyl, Benzoylamino ($C_1$-$C_6$) alkyl, ($C_1$-$C_6$) Alkanoylamino ($C_1$-$C_6$) alkyl, ($C_1$-$C_6$) Alkonsulfonylamino ($C_1$-$C_6$) alkyl, ($C_1$-$C_6$) Alkoxy ($C_1$-$C_6$) alkanoylamino ($C_1$-$C_6$) alkyl, Cyclo ($C_3$-$C_6$) alkyloxycarbonylamino ($C_1$-$C_6$) alkyl, Pyridylcarbonylamino ($C_1$-$C_6$) alkyl, Morpholinocorbonylamino ($C_1$-$C_6$) alkyl, Phenyl ($C_1$-$C_6$) alkoxyoxycarbonylamino ($C_1$-$C_6$) alkyl, ($C_1$-$C_6$) Alkoxyphenylsulfonylamino ($C_1$-$C_6$) alkyl, Hydroxy ($C_1$-$C_6$) alkylamino ($C_1$-$C_6$) alkyl, Morpholino ($C_1$-$C_6$) alkyl, Oxooxazolidinyl ($C_1$-$C_6$) alkyl, Oxopyrrolidinyl ($C_1$-$C_6$) alkyl, Trimethylhydantoinyl ($C_1$-$C_6$) alkyl, Pyridyl ($C_1$-$C_6$) alkyl, ($C_2$-$C_6$) Alkenylamino ($C_1$-$C_6$) alkyl, ($C_1$-$C_6$) Alkoxy ($C_1$-$C_6$) alkylamino ($C_1$-$C_6$) alkyl, Phenyl ($C_1$-$C_6$) alkylamino ($C_1$-$C_6$) alkyl, Pyridyl ($C_1$-$C_6$) alkylamino ($C_1$-$C_6$) alkyl, Cyclo ($C_3$-$C_6$) alkyl, (Amino) (phenyl) ($C_1$-$C_6$) alkylamino, (($C_1$-$C_6$) Alkoxycorbonylamino)(phenyl) ($C_1$-$C_6$) alkyl, (Amino) (($C_1$-$C_6$) alkoxy) ($C_1$-$C_6$) alkyl, (($C_1$-$C_6$) Alkoxycarbonylamino) (($C_1$-$C_6$) alkoxy) ($C_1$-$C_6$) alkyl, (Amino)(corboxy) ($C_1$-$C_6$) alkyl, (($C_1$-$C_6$) Alkoxycarbonylamino)(carboxy) ($C_1$-$C_6$) alkyl, (Amino)( ($C_1$-$C_6$) alkoxycarbonyl) ($C_1$-$C_6$) alkyl, (($C_1$-$C_6$) Alkoxycarbonylamino)(($C_1$-$C_6$) alkoxycarbonyl) ($C_1$-$C_6$) alkyl, (Amino)(phenyl ($C_1$-$C_6$) alkoxy) ($C_1$-$C_6$) alkyl, (($C_1$-$C_6$) Alkoxycarbonylamino) (phenyl ($C_1$-$C_6$) alkoxy) ($C_1$-$C_6$) alkyl, (Amino)(pyridyl) ($C_1$-$C_6$) alkyl, (($C_1$-$C_6$) Alkoxycarbonylamino)(pyridyl) ($C_1$-$C_6$) alkyl, (Amino)(hydroxy) ($C_1$-$C_6$) alkyl, (($C_1$-$C_6$) Alkoxycarbonylamino)(hydroxy) ($C_1$-$C_6$) alkyl, (Amino) (amino) ($C_1$-$C_6$) alkyl, (($C_1$-$C_6$) Alkoxycorbonylamino)(amino) ($C_1$-$C_6$) alkyl, (Amino) (($C_1$-$C_6$) alkoxycarbonylamino) ($C_1$-$C_6$) alkyl, (($C_1$-$C_6$) Alkoxycarbonylamino) (($C_1$-$C_6$) alkoxycarbonylamino) ($C_1$-$C_6$) alkyl, (Amino) (($C_3$-$C_6$) cycloalkan) ($C_1$-$C_6$) alkyl, (($C_1$-$C_6$) Alkoxycarbonylamino)(($C_3$-$C_6$) cycloalkan) ($C_1$-$C_6$) alkyl,

ist.

**8.** Die Verbindung nach Anspruch 6, wobei die Gruppe der Formel:

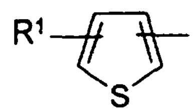

eine Gruppe der folgenden Formeln (a) bis (e) ist:

(a)

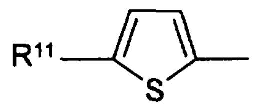

wobei

$R^{11}$ Brom, 2-Naphthyl, Phenyl, 3- (oder 4-) Chlorphenyl, 2- (oder 3- oder 4-) Fluorphenyl, 3,4-Dichlorphenyl, 3,5-Difluorphenyl, 3-(oder 4-) Methylphenyl, 4-Ethylphenyl, 4-Isopropylphenyl, 4-(t-Butyl)phenyl, 3,4-Dimethylphenyl, 4-Methoxyphenyl, 4-Ethoxyphenyl, 4-Trifluormethylphenyl, 4-Trifluormethoxyphenyl, 4-Ethenylphenyl, 4-Methylcarbamoylphenyl, 4-Ethylcarbamoylphenyl, 4-Carbamoylphenyl, 4-Benzylcarbamoylphenyl, 4-Acetylphenyl, 4-Methylthiophenyl, 4-Ethylthiophenyl, 4-Methylsulfinylphenyl, 4-Methylsulfonylphenyl, Phenylphenyl, 4-Phenyl-3-Fluorphenyl, 4-(4-Fluorphenyl)phenyl, 3- (oder 4-) Hydroxyphenyl, 3- (oder 4-) Hydroxymethylphenyl, 4-(1,2-Dihydroxyethyl)phenyl, 4-(Phenoxycarbonyloxymethyl)phenyl, 3- (oder 4-) Aminophenyl, 4-Carboxyphenyl, 3,4-Methylendioxyphenyl, 4-(Methansulfonylamino)phenyl, 3-(2-Butenoylamino)phenyl, 3-(Cyclopropancarbonylamino)phenyl, 3-(Cyclobutancarbonylamino)phenyl, 3-(Cyclopentancarbonylamino)phenyl, 4-Benzyloxyphenyl, 4-(2-(Methylcarbamoyl)ethenyl)phenyl, 4-(2-(Ethylcarbamoyl)ethenyl)phenyl, 4-(2-(Propylcarbamoyl)ethenyl)phenyl, 4-(2-(Isopropylcarbamoyl)ethenyl)phenyl, 4-(2-(Dimethylcarbamoyl) ethenyl)phenyl, 4-(2-(Phenylcarbamoyl)ethenyl)phenyl, 4-(2-(Methoxyphenylcarbamoyl)ethenyl)phenyl, 4-(2-(4-Fluorphenylcarbamoyl)ethenyl)phenyl, 4-(Methylaminocarbonyloxy)phenyl, 4-(Ethylaminocarbonyloxy)phenyl, 4-Propanoyloxyphenyl, 4-(Methoxyacetyloxy)phenyl, 4-(Ethoxycarbonyloxy)phenyl, 4-(3-(3-Pyridyl)acryloyloxy)phenyl, 4-(Cyclopropylcarbonyloxy)phenyl, 4-(Carboxymethoxy)phenyl, 4-(Ethoxycarbonylmethoxy)phenyl, 4-(t-Butoxycarbonylmethoxy)phenyl, 4-(Propanoylmethoxy)phenyl, 4-(Cyclopropylcarbamoylmethoxy)phenyl, 3- (oder 4-) (Methylcarbamoylmethoxy)phenyl, 4-(Ethylcarbamoylmethoxy)phenyl, 4-(Propylcarbamoylmethoxy)phenyl, 3- (oder 4-) (Methylcarbamoyloxymethyl)phenyl, 4-(Methoxycarbonylaminomethyl)phenyl, 4-(t-Butoxycarbonylaminomethyl)phenyl, 4-Aminomethylphenyl, 4-(Methylcarbamoylmethyl)phenyl, 3-(2-(oder 3-) Furylcarbonylamino)phenyl, 3-(1,2,3,4-Tetrahydroisochinolylcarbonylamino)phenyl, 3-(N-(t-Butoxycarbonyl)-1,2,3,4-tetrahydroisochinolylcarbonylamino)phenyl, 3-(Pyrrolidinylcarbonylamino)phenyl, 4-(1,3-Oxazolyl)phenyl, 4-(5-Methyl-1,2,4-oxadiazol-3-yl)phenyl,
ist;

(b)

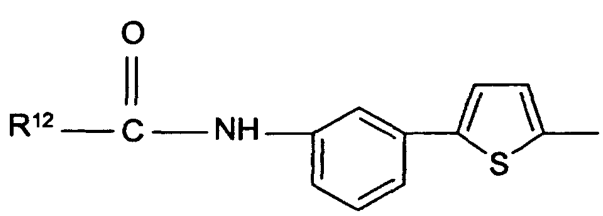

wobei

$R^{12}$ Methyl, Ethyl, Propyl, Isopropyl, Butyl, Isobutyl, t-Butyl, Neopentyl, Phenylmethyl, 4-Chlorphenylmethyl, 4-Methoxyphenylmethyl, Methoxymethyl, Ethoxymethyl, Propoxymethyl, Butoxymethyl, Isopropyloxymethyl, 1-Methoxyethyl, 2-Methoxyethyl, Phenoxymethyl, 2-Phenoxyethyl, 3-(oder 4-) Methoxyphenoxymethyl, 4-Flu-

or(oder Chlor)phenoxymethyl, 3- (oder 4-) Methylphenoxymethyl, 2-Carboxyethyl, 2-Methoxycarbonylethyl, 2-t-Butoxycarbonylethyl, 2-Methylcarbamoylethyl, 2-Chlorethyl, Chlormethyl, Allyloxymethyl, (2-Ethoxyethoxy)methyl, Benzyloxymethyl, 4-Piperidinyloxymethyl, (N-t-Butoxycarbonyl-4-piperidinyl)oxymethyl, 3-(oder 4-) Pyridyloxymethyl, Hydroxymethyl, 2-Hydroxyethyl, Acetoxymethyl, 1-Acetoxyethyl, Methylcarbamoyloxymethyl, 1-(N-Methyl-N-ethylcarbamoyloxy)methyl, (Piperidinocarbonyloxy)methyl, (Benzylcarbamoyloxy)methyl, (t-Butoxycarbonylamino)methyl, Aminomethyl, 1-Aminoethyl, 1-(t-Butoxycarbonylamino)ethyl, 2-Aminoethyl, Methoxycarbonylaminomethyl, 2-(Methoxycarbonylamino)ethyl, Ethoxycarbonylaminomethyl, Propoxycarbonylaminomethyl, 1-(Fluorenylmethoxycarbonylamino)methyl, 2-(t-Butoxycarbonylamino)ethyl, 2-(Fluorenylmethoxycarbonylamino)ethyl, 1-Aminoisopropyl, 1-Aminopropyl, 1-(t-Butoxycarbonylamino)propyl, 1-(t-Butoxycarbonylamino)isopropyl, 1,5-Diaminopentyl, 1,5-bis(t-Butoxycarbonylamino)-pentyl, Methylaminomethyl, Ethylaminomethyl, 1 -(N-Methyl-N-ethylamino)methyl, Dimethylaminomethyl, Pentylaminomethyl, t-Butylaminomethyl, 2-Methylaminoethyl, 1-(N-Methyl-N-methoxycarbonylamino)methyl, 1 -(N-Methyl-N-t-butoxycarbonylamino)methyl, 1-(N-Ethyl-N-t-butoxycarbonylamino)methyl, 2-(N-Methyl-N-(fluorenylmethoxycarbonyl)amino)-ethyl, 2-(N-Methyl-N-(t-butoxycarbonyl)amino)ethyl, 1-(N- Methyl-N-(dimethylcarbamoyl)amino) methyl, 1-(Dimethylcarbamoylamino)methyl, 1-(N-(Ethylcarbamoyl)amino)methyl, 2-(N-(Ethylcarbamoyl)amino)ethyl, Benzoylaminomethyl, 2-Benzoylaminoethyl, Acetylaminomethyl, Isobutyrylaminomethyl, Pivaloylaminomethyl, 1-(Methansulfonylamino)methyl, 2-(Methansulfonylamino)ethyl, Methoxyacetylaminomethyl, Cyclopentyloxycarbonylaminomethyl, Pyridylcarbonylaminomethyl, Morpholinocarbonylaminomethyl, Benzyloxycarbonylaminomethyl, 1-(4-Methoxyphenylsulfonylamino)methyl, 1-(2-Hydroxyethylamino)methyl, Morpholinomethyl, 1-(2-Oxo-1,3-oxazolidin-1-yl)methyl, 1-(2-Oxopyrrolidin-1-yl)methyl, 1-(3,4,4-Trimethythydantoin-1-yl)methyl, Allylaminomethyl, 1-(2-Ethoxyethylamino)methyl, Benzylaminomethyl, 1-(3-Pyridylmethylamino)methyl, 2-Phenyl-1-aminoethyl, 1-Amino-1-phenylmethyl, 1-t-Butoxycarbonylamino-1-phenylmethyl, 1-Amino-2-phenylethyl, 1-t-Butoxycarbonylamino-2-phenylethyl, 1-Amino-2-methoxyethyl, 1-t-Butoxycarbonylamino-2-methoxyethyl, 1-Amino-3-carboxypropyl, 1-t-Butoxycarbonylamino-3-carboxypropyl, 1-Amino-3-(t-butoxycarbonyl)propyl, 1-t-Butoxycarbonylamino-3-t-butoxycarbonylpropyl, 1-Amino-2-benzyloxyethyl, 1-t-Butoxycarbonylamino-2-benzyloxyaminoethyl, 1-Amino-2-(3-pyridyl)ethyl, 1-t-Butoxycarbonylamino-2-(3-pyridyl)ethyl, 1-Amino-2-(4-pyridyl)ethyl, 1-t-Butoxycarbonylamino-2-(4-pyridyl)ethyl, 1-Amino-2-hydroxyethyl, 1-t-Butoxycarbonylamino-2-hydroxyethyl, 1,5-Diaminopentyl, 1 -t-Butoxycarbonylamino-5-aminopentyl, 1,5-bis(t-Butoxycarbonylamino)pentyl, 1-Amino-5-(t-butoxycarbonylamino)pentyl, 1-Amino-2-cyclohexylethyl, 1-t-Butoxycarbonylamino-2-cyclohexylethyl,
ist;

(c)

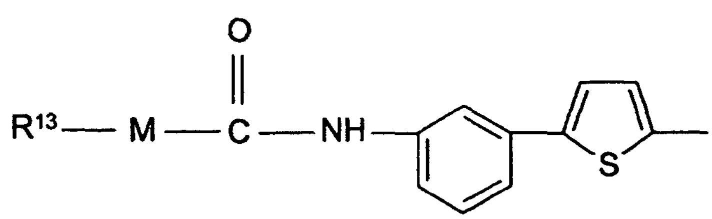

wobei
M= O und $R^{13}$ Methyl, Ethyl, Propyl, Isopropyl, Benzyl, 2-Methoxyethyl, 2-Chlorethyl, 2-Aminoethyl, 2-Phthalimidoethyl, Allyl, Phenyl ist, oder
M=S und $R^{13}$ Methyl, Ethyl ist,

(d)

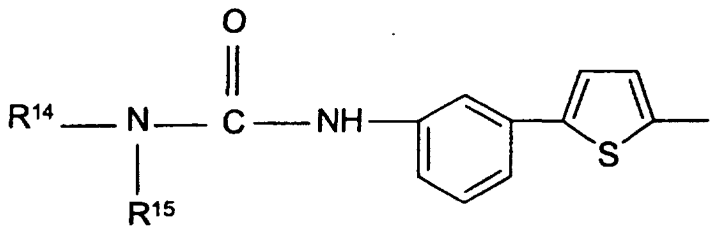

wobei
$R^{15}$ Wasserstoff ist und
$R^{14}$ Wasserstoff, Methyl, Ethyl, Propyl, Isopropyl, Butyl, Isobutyl, Pentyl, Hexyl, 1 -Naphthyl, 3- (oder 4-) Chlorphenyl, 3-Methoxyphenyl, Allyl, Cyclohexylmethyl, Benzyl, 2-Chlorethyl, Methoxymethyl, 2-Methoxyethyl, 2-Hydroxyethyl, 2-((t-Butyl)(diphenyl)silyloxy)ethyl, Carboxymethyl, Ethoxycarbonylmethyl, Methylcarbamoylmethyl, oder 3-Pyridyl ist, oder
$R^{14}$ Ethyl und $R^{15}$ Methyl ist,

(e)

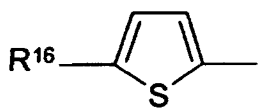

wobei $R^{16}$ 2-Benzothienyl, 2-Benzofuranyl, 2- (oder 3-) Thienyl, 2-Furyl, 3-Pyridyl, 1-Methyl-4-pyridyl, 6-Methyl-3-pyridyl, 6-Methoxy-3-pyridyl, 5-Methoxycarbonylamino-3-pyridyl, 5-Acetyl-2-thienyl, 2-Methylcarbamoyl-5-benzofuranyl ist.

**9.** Verfahren zur Herstellung einer Verbindung der Formel:

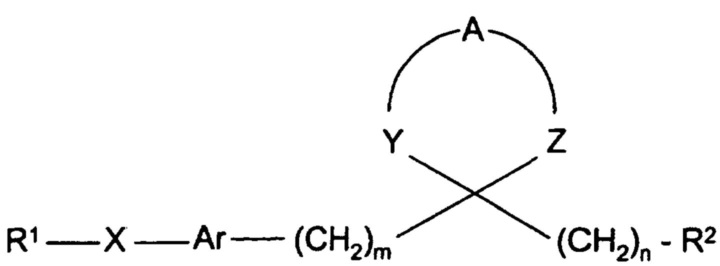

(I)

worin $R^1$, $R^2$, Ar, A, X, Y, Z, m und n jeweils wie in Anspruch 1 definiert sind,
wobei das Verfahren umfasst

(1) Unterwerfen einer Verbindung der Formel:

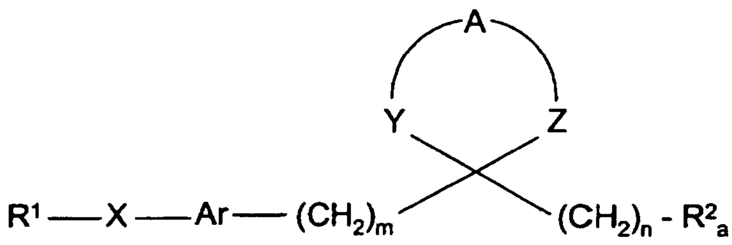

(I-a)

oder eines Salzes davon unter eine Eliminierungsreaktion der CarboxySchutzgruppe, um eine Verbindung mit der folgenden Formel zu erzielen:

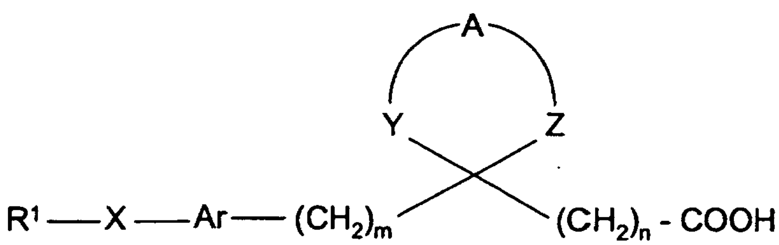

(I-b)

oder ein Salz davon; oder

(2) Oxidation der Vinylgruppe einer Verbindung der Formel:

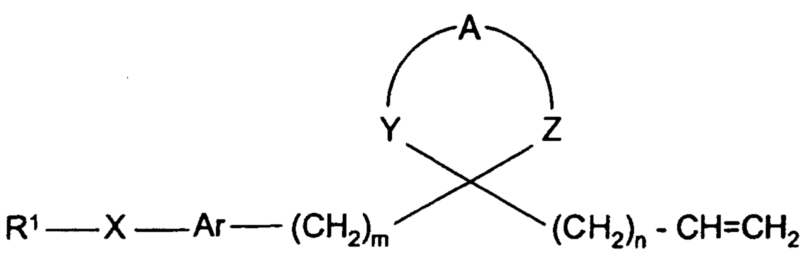

(II)

oder eines Salzes davon, um eine Verbindung mit der obigen Formel (I-b) oder ein Salz davon zu erzielen; oder

(3) Reduktion einer Verbindung der Formel:

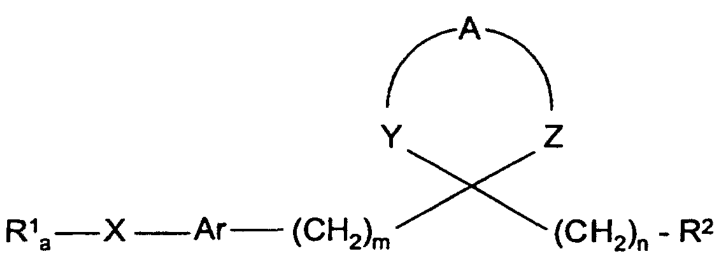

(I-c)

oder eines Salzes davon, um eine Verbindung mit der Formel:

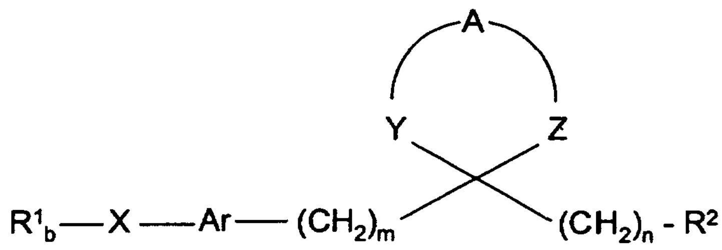

(I-d)

zu erzielen, oder ein Salz davon; oder

(4) Reaktion einer Verbindung der obigen Formel (I-b) oder des reaktiven Derivates an der Carboxygruppe davon, oder eines Salzes davon, mit einer Verbindung der Formel:

$$NH_2 - OR^3 \qquad (IV)$$

oder des reaktiven Derivates an der Aminogruppe davon, oder eines Salzes davon, um eine Verbindung mit der Formel zu erzielen:

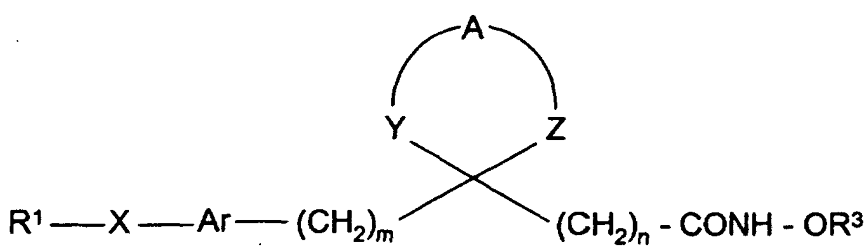

(I-e)

oder ein Satz davon; oder

(5) Cyclisieren einer Verbindung der Formel:

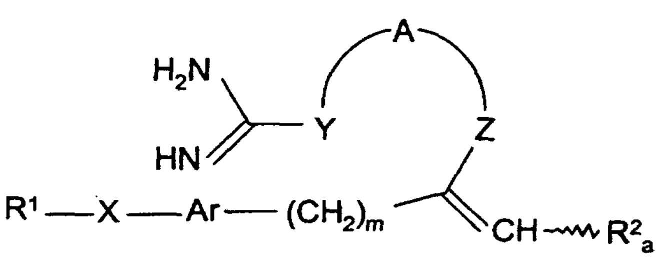

(III)

oder eines Salzes davon, um eine Verbindung mit der Formel:

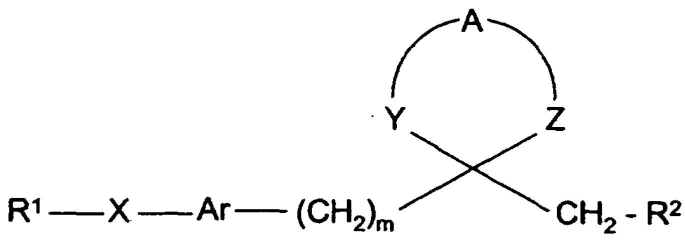

(I-f)

zu erzielen,
oder ein Salz davon; oder

(6) Reaktion der obigen Verbindung (I-b) oder des reaktiven Derivates an der Carboxygruppe davon, oder eines Salzes davon, mit einem optisch aktiven Amin oder dem reaktiven Derivat an der Aminogruppe davon, oder einem Salz davon, um eine Verbindung mit der Formel:

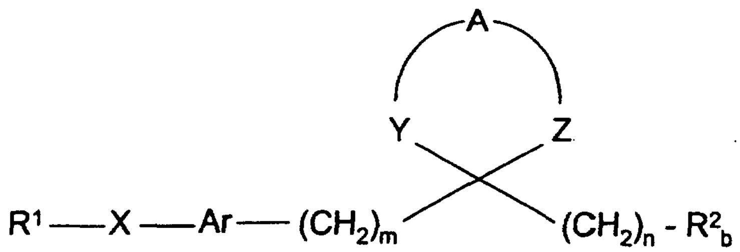

(I-g)

zu erzielen,
oder ein Salz davon; oder

(7) Unterwerfen einer Verbindung der Formel:

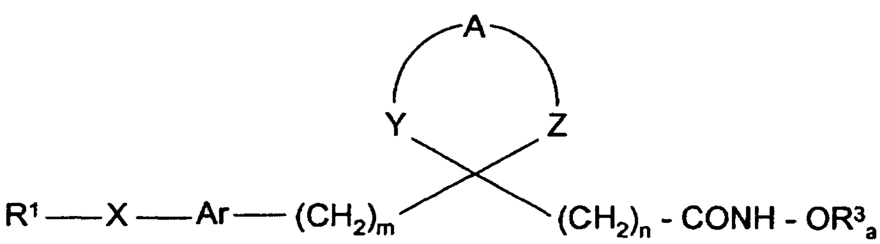

(I-h)

oder eines Salzes davon unter eine Eliminierungsreaktion der Hydroxy-Schutzgruppe, um eine Verbindung der Formel:

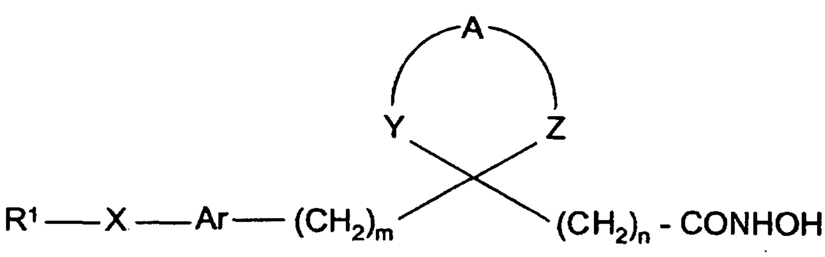

(I-i)

zu erzielen,

oder ein Salz davon; oder

(8) Oxidation einer Verbindung der Formel:

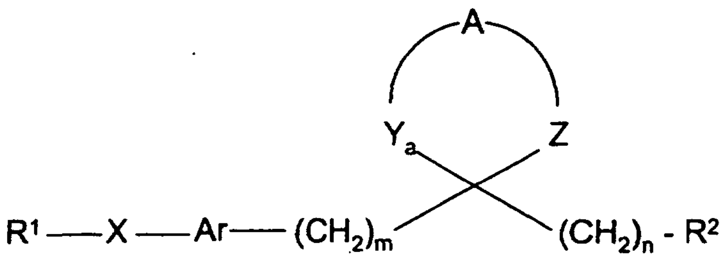

(I-j)

oder eines Salzes davon, um eine Verbindung der Formel:

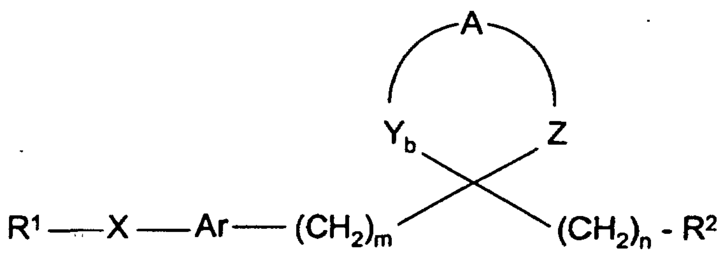

(I-k)

zu erzielen,
oder ein Salz davon; oder

(9) Reaktion der obigen Verbindung (I-c) oder eines Salzes davon, mit einer Verbindung der Formel:

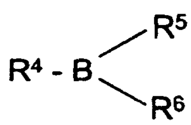

(V)

um eine Verbindung der Formel:

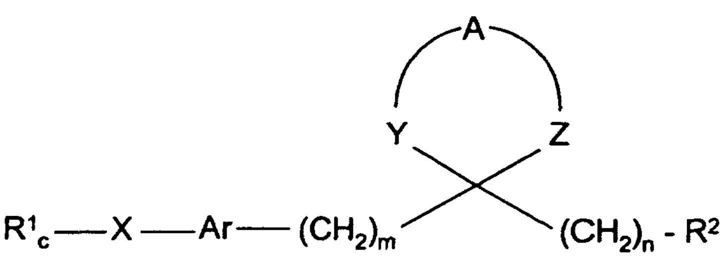

(I-l)

zu erzielen,
oder ein Salz davon; oder

(10) Reaktion einer Verbindung der Formel:

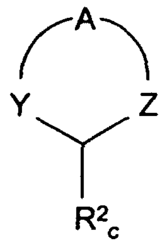

(VI)

oder eines Salzes davon, mit einer Verbindung der Formel:

$$R^1\text{-X-Ar-}(CH_2)_m\text{l - L} \qquad \text{(VII)}$$

oder eines Salzes davon, um eine Verbindung der Formel:

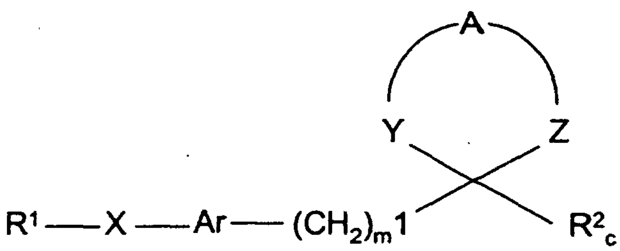

(I-m)

zu erzielen,
oder ein Salz davon; oder

(11) Cyclisieren einer Verbindung der Formel:

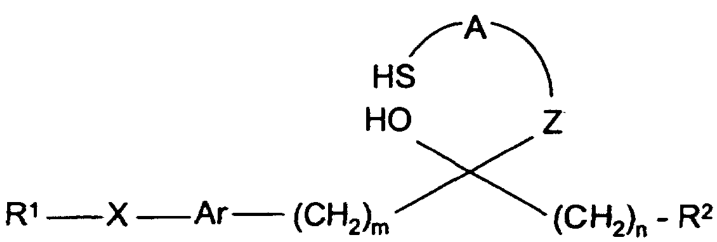

(VIII)

oder eines Salzes davon, um eine Verbindung der Formel:

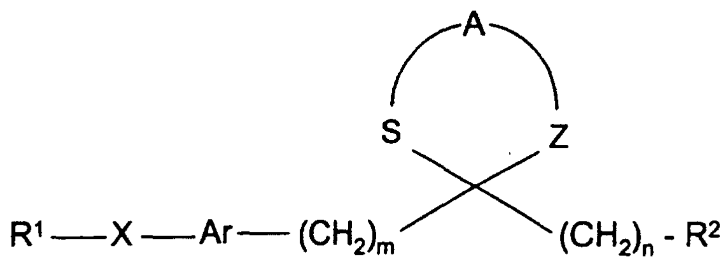

(I-n)

zu erzielen,
oder ein Salz davon; oder

(12) Amidieren einer Verbindung der Formel:

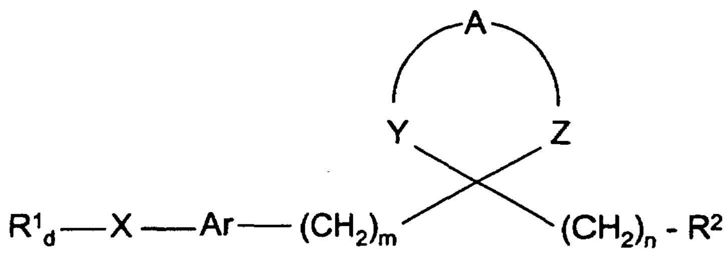

(I-p)

oder des reaktiven Derivates davon an der Carboxygruppe, oder eines Salzes davon, um eine Verbindung der Formel:

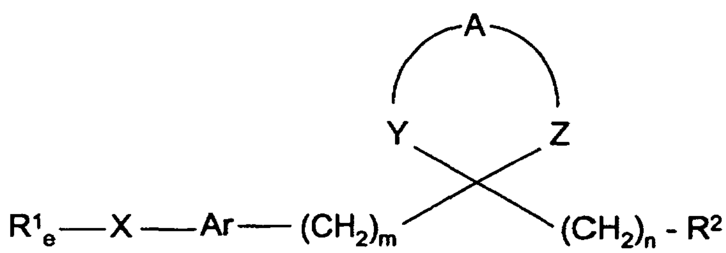

(I-q)

zu erzielen,
oder ein Salz davon; oder

(13) Acylieren einer Verbindung der Formel:

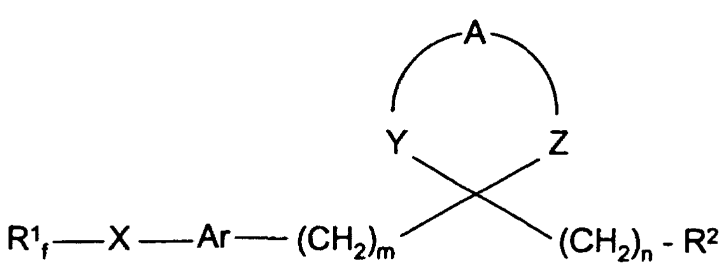

(I-r)

oder des reaktiven Derivatives an der Aminogruppe davon, oder eines Salzes davon, um eine Verbindung der Formel:

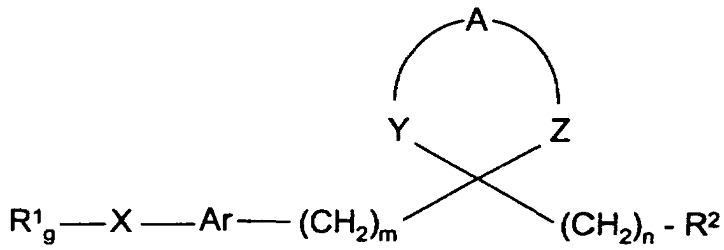

(I-s)

zu erzielen,
oder ein Salz davon, oder

(14) Unterwerfen einer Verbindung der Formel:

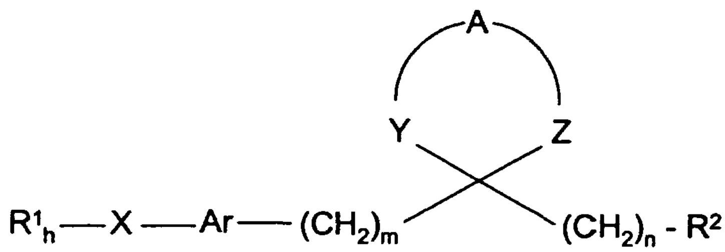

(I-t)

oder eines Salzes davon unter eine Eliminierungsreaktion der AminoSchutzgruppe, um eine Verbindung der Formel:

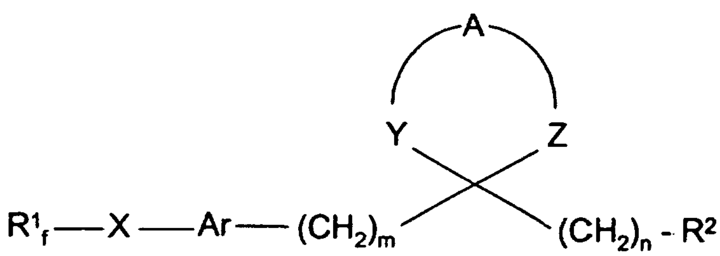

(I-r)

zu erzielen,
oder ein Salz davon; oder

(15) Unterwerfen einer Verbindung der Formel:

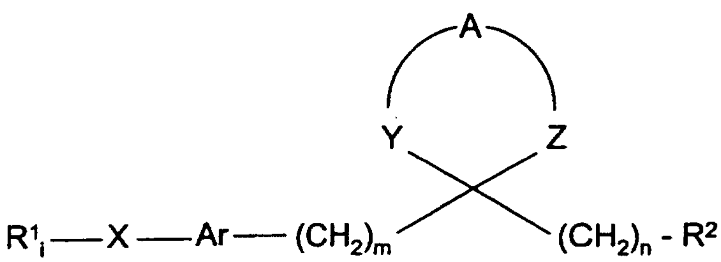

(I-u)

oder eines Salzes davon unter eine Eliminierungsreaktion der Hydroxy-Schutzgruppe, um eine Verbindung der Formel:

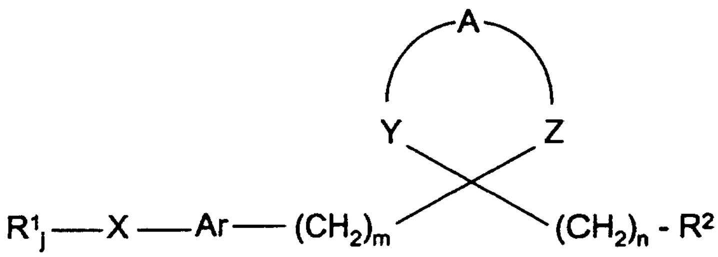

(I-v)

zu erzielen,
oder ein Salz davon; oder

(16) Oxidieren einer verbindung der Formel:

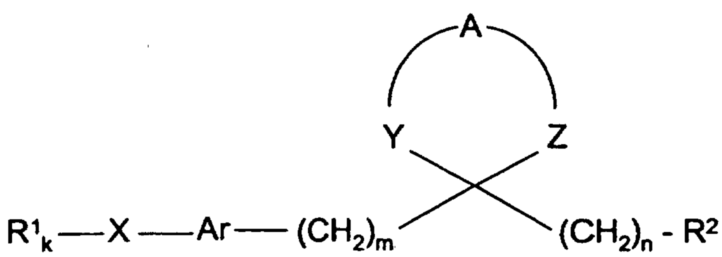

(I-w)

oder eines Salzes davon, um eine Verbindung der Formel:

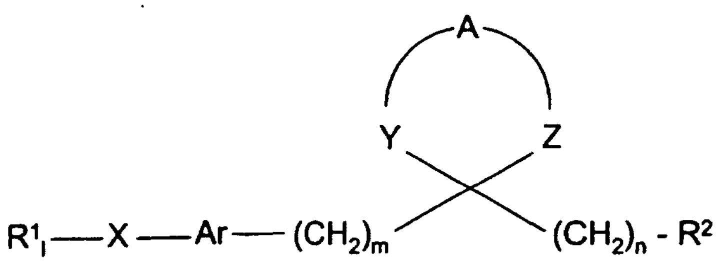

(I-x)

zu erzielen,
oder ein Salz davon; oder

(1 7) Reduktion einer Verbindung der Formel:

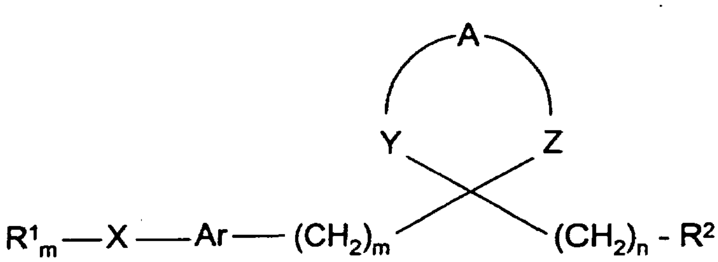

(I-y)

oder eines Salzes davon, um eine Verbindung der Formel:

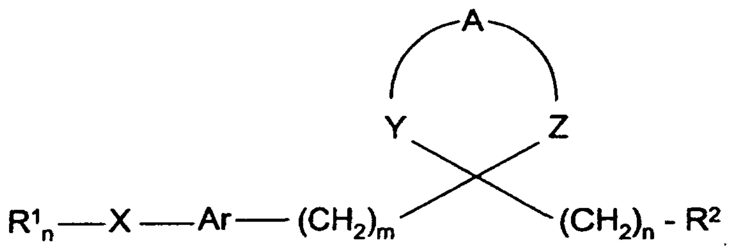

(I-z)

zu erzielen,
oder ein Salz davon; oder

(18) Oxidieren einer Verbindung der Formel:

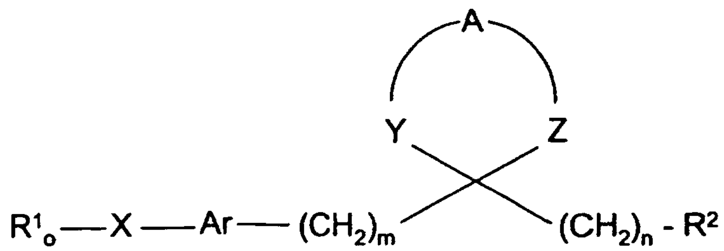

(I-aa)

oder eines Salzes davon, um eine Verbindung der Formel:

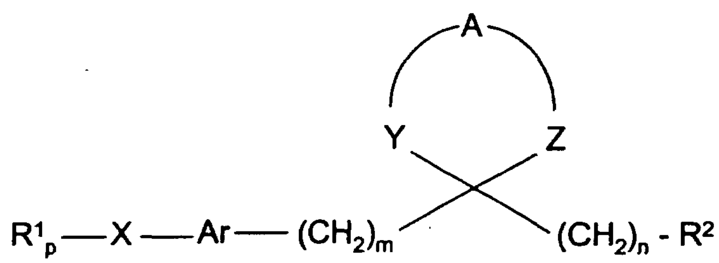

(I-ab)

zu erzielen,
oder ein Salz davon; oder

(19) Acylieren einer Verbindung der Formel:

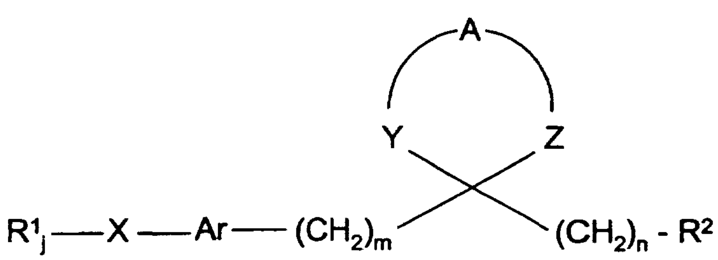

(I-v)

oder eines Salzes davon, um eine Verbindung der Formel:

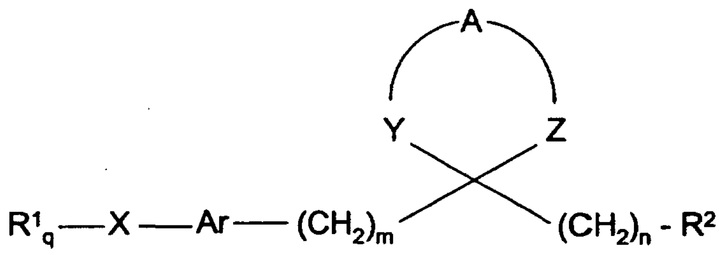

(I-ac)

zu erzielen,
oder ein Salz davon; oder

(20) Reaktion einer Verbindung der Formel:

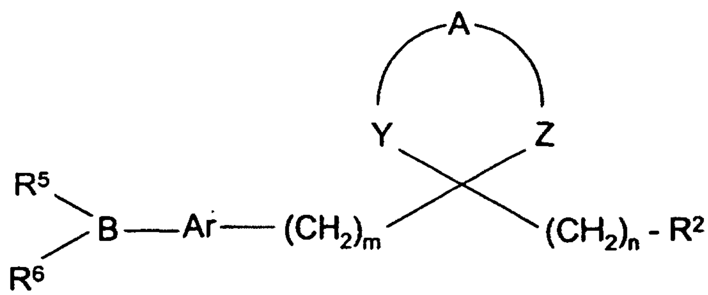

(XI)

oder eines Salzes davon, mit einer Verbindung der Formel:

$$R^1 - L \quad (XII)$$

oder einem Salz davon, um eine Verbindung der Formel:

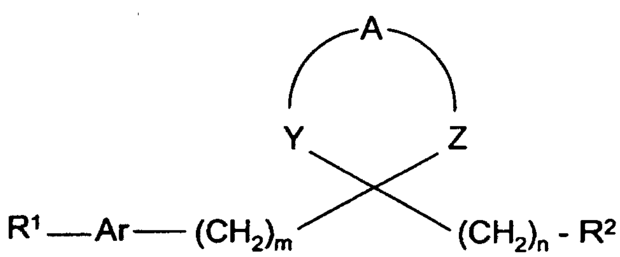

(I-ad)

zu erzielen,
oder ein Salz davon; oder

(21) Unterwerfen einer Verbindung der Formel:

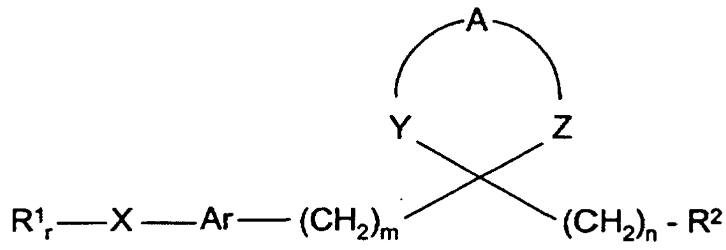

(I-ae)

oder eines Salzes davon, unter eine Eliminierungsreaktion der CarboxySchutzgruppe, um eine Verbindung der Formel:

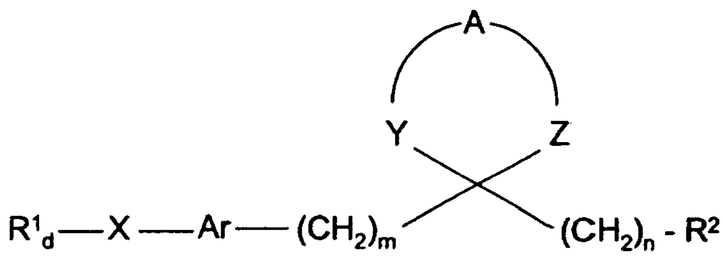

(I-p)

zu erzielen,
oder ein Salz davon; oder

(22) Reaktion einer Verbindung der Formel:

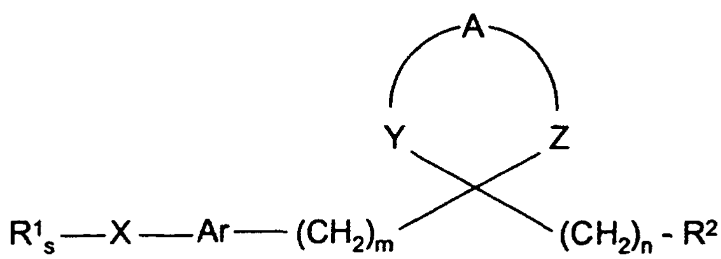

(I-af)

oder eines Salzes davon, mit einem substituierten Amin, um eine Verbindung der Formel:

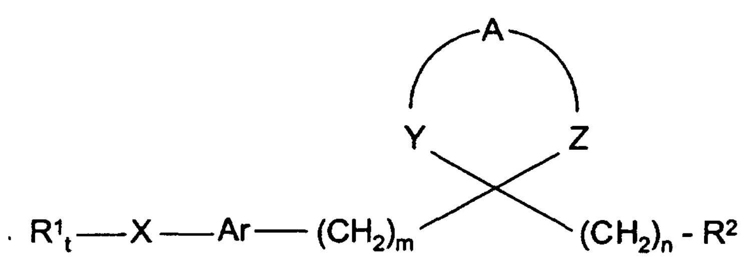

(I-ag)

zu erzielen,
oder ein salz davon,

worin $R^1$, $R^2$, Ar, A, X, Y, Z, m und n jeweils den obigen Definitionen entsprechen,

$R^1_a$ Haloaryl oder Halo ist,
$R^1_b$ Aryl ist,
$R^1_c$ Aryl ist welches mindestens mit einem optional substituierten Aryl substituiert ist,
$R^1_d$ Aryl mit mindestens einer Carboxygruppe ist,
$R^1_e$ Aryl mit mindestens einer Amidogruppe ist,
$R^1_f$ Aryl mit mindestens einer Aminogruppe ist,
$R^1_g$ Aryl mit mindestens einer Acylaminogruppe ist,
$R^1_h$ Aryl mit mindestens einer geschützten Aminogruppe ist,
$R^1_i$ Aryl mit mindestens einer geschützten Hydroxygruppe ist,
$R^1_j$ Aryl mit mindestens einer Hydroxygruppe ist,
$R^1_k$ Aryl mit mindestens einer Thiagruppe ist,
$R^1_l$ Aryl mit mindestens einer Sulfinyl- oder Sulfonylgfruppe ist,
$R^1_m$ Aryl mit mindestens einer Formylgruppe ist,
$R^1_n$ Aryl mit mindestens einer Hydroxymethylgruppe ist,
$R^1_o$ Aryl mit mindestens einer Vinylgruppe ist,
$R^1_p$ Aryl mit mindestens einer 1,2-Dihydroxyethylgruppe ist,
$R^1_q$ Aryl mit mindestens einer Acyloxygruppe ist,
$R^1_r$ Aryl mit mindestens einer Carboxygruppe ist,
$R^1_s$ Aryl mit mindestens einer Halo ($C_1$-$C_6$) alkanoylgruppe ist,
$R^1_t$ Aryl mit mindestens einer substituierten Amino ($C_1$-$C_6$) alkanoylgruppe ist,
$R^2_a$ geschütztes Carboxy ist,
$R^2_b$ optisch actives Amid ist,
$R^2_c$ geschütztes Carboxy ist,
$R^3$ Wasserstoff oder eine Hydroxy-Schutzgruppe ist,
$R^3_a$ Hydroxy-Schutzgruppe ist,
$R^4$ optional substituiertes Aryl ist,
$R^5$ und $R^6$ jeweils Wasserstoff sind oder zusammen ($C_1$-$C_6$) Alkylen bilden,
$Y_a$ Thia oder Sulfinyl ist,
$Y_b$ Sulfinyl oder Sulfonyl ist,
L eine austretende Gruppe ist, und
ml eine ganze Zahl von 1 bis 6 ist.

**10.** Pharmazeutische Zusammensetzung, welche eine Verbindung nach irgendeinem der Ansprüche 1 bis 8 enthält oder ein pharmazeutisch akzeptables Salz davon und einen pharmazeutisch akzeptablen Träger oder Hilfsstoff.

**11.** Verfahren zur Herstellung einer pharmazeutischen Zusammensetzung welches das Vermischen einer Verbindung nach irgendeinem der Ansprüche 1 bis 8 oder eines pharmazeutisch akzeptablen Salzes davon mit einem pharmazeutisch akzeptablen Träger oder Hilfsstoff umfasst.

**12.** Verwendung der Verbindung nach irgendeinem der Ansprüche 1 bis 8 oder eines pharmazeutisch akzeptablen Salzes davon für die Herstellung eines Medikaments für die Inhibition von Matrix Metalloproteinasen (MMP) oder Tumor necrosis factor $\alpha$ (TNF $\alpha$) .

**13.** Verwendung der Verbindung nach irgendeinem der Ansprüche 1 bis 8 oder eines pharmazeutisch akzeptablen Salzes davon für die Herstellung eines Medikaments für die Behandlung oder Vorbeugung von MMP- oder TNF $\alpha$ -vermittelten Krankheiten.

## Revendications

**1.** Composé de la formule :

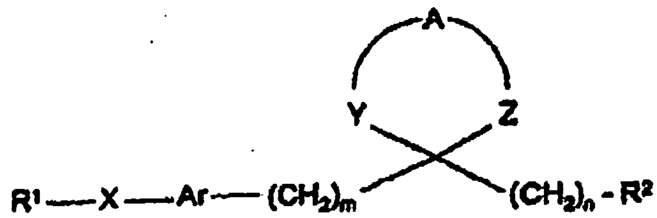

dans laquelle $R^1$ est un alkyle en $C_1$-$C_6$, un groupe hétérocyclique optionnellement substitué ou un aryle en $C_6$-$C_{10}$ optionnellement substitué,
$R^2$ est un carboxy, un carboxy protégé, un hydroxyaminocarbonyle, un tétrahydropyranyloxyaminocarbonyle, ou un phénylalkyl(en $C_1$-$C_6$)aminocarbonyle,
Ar est un aryle en $C_6$-$C_{10}$ optionnellement substitué ou un groupe hétérocyclique optionnellement substitué,
A est un alkylène en $C_1$-$C_6$,
X est un oxa ou une liaison simple,
Y est un thia, un sulfinyle ou un sulfonyle,
Z est un méthylène,
m et n sont chacun un nombre entier de 0 à 6, et
$1 \leq m+n \leq 6$,
et son sel,
où le groupe hétérocyclique de $R^1$ et Ar sont choisis dans le groupe constitué par les (1) à (14) suivants,

(1) un groupe hétéromonocyclique insaturé à de 3 à 8 membres, contenant 1 à 4 atomes d'azote,
(2) un groupe hétéromonocyclique saturé de 3 à 8 membres, contenant 1 à 4 atomes d'azote,
(3) un groupe hétéromcaocyclique insaturé à de 3 à 8 membres, contenant 1 ou 2 atomes de soufre,
(4) un groupe hétérocyclique insaturé condensé de 7 à 13 membres, contenant 1 à 5 atomes d'azote,
(5) un groupe hétéromonocyclique insaturé de 3 à 8 membres, contenant 1 ou 2 atomes d'oxygène,
(6) un groupe hétéromonocyclique saturé de 3 à 8 membres, contenant 1 ou 2 atomes d'oxygène,
(7) un groupe hétéromonocyclique insaturé de 3 à 8 membres, contenant 1 ou 2 atomes d'oxygène et 1 à 3 atomes d'azote,
(8) un groupe hétérocyclique insaturé condensé de 7 à 13 membres, contenant 1 ou 2 atomes d'oxygène,
(9) un groupe hétérocyclique insaturé condensé de 7 à 13 membres, contenant 1 ou 2 atomes de soufre,
(10) un groupe hétéromonocyclique saturé de 3 à 8 membres, contenant 1 ou 2 atomes d'oxygène et 1 à 3 atomes d'azote,
(11) un groupe hétérocyclique insaturé condensé de 7 à 13 membres, contenant 1 ou 2 atomes d'oxygène et 1 à 3 atomes d'azote,
(12) un groupe hétéromonocyclique insaturé de 3 à 8 membres, contenant 1 ou 2 atomes de soufre et 1 à 3 atomes d'azote,
(13) un groupe hétéromonocyclique saturé de 3 à 8 membres, contenant 1 ou 2 atomes de soufre et 1 à 3 atomes d'azote, et
(14) un groupe hétérocyclique insaturé condensé de 7 à 13 membres, contenant 1 ou 2 atomes de soufre et 1 à 3 atomes d'azote, et

chacun des groupes hétérocycliques mentionnés plus haut et groupes aryle en $C_6$-$C_{10}$ sont optionnellement substitués par le groupe constitué des (A1) à (A35) suivants :

(A1) un halogène
(A2) un alkyle en $C_1$-$C_6$
(A3) un alcoxy en $C_1$-$C_6$
(A4) un haloalkyle en $C_1$-$C_6$
(A5) un haloalcoxy en $C_1$-$C_6$
(A6) un alcényle en $C_2$-$C_6$
(A7) un acyle,
(A8) un alkyl (en $C_1$-$C_6$)thio, un alkyl(en $C_1$-$C_6$)sulfinyle, un alkyl(en $C_1$-$C_6$)sulfonyle,
(A9) un aryle en $C_6$-$C_{10}$,
(A10) un haloaryle en $C_6$-$C_{10}$,

(A11) un hydroxy,
(A12) un hydroxyalkyle en $C_1$-$C_6$, un hydroxy(protégé)alkyle en $C_1$-$C_6$,
(A13) un amino,
(A14) un carboxy,
(A15) un carboxy protégé,
(A16) un nitroalcényle en $C_2$-$C_6$,
(A17) un alkylène(en $C_1$-$C_6$)dioxy,
(A18) un acylamino,
(A19) un nitro,
(A20) un aryl(en $C_6$-$C_{10}$)alcoxy en $C_1$-$C_6$,
(A21) un carbamoylalcényle en $C_2$-$C_6$ optionnellement N-substitué par le groupe constitué des alkyle en $C_1$-$C_6$, aryle en $C_6$-$C_{10}$, alcoxy(en $C_1$-$C_6$)aryle en $C_6$-$C_{10}$, et haloaryle en $C_6$-$C_{10}$,
(A22) un alkyl(en $C_1$-$C_6$)aminocarbonyloxy,
(A23) un alcanoyloxy en $C_1$-$C_6$,
(A24) un alcoxy(en $C_1$-$C_6$)alcanoyl(en $C_1$-$C_6$)oxy,
(A25) un alcoxy(en $C_1$-$C_6$)carbonyloxy,
(A26) un alcénoyloxy en $C_2$-$C_6$ optionnellement substitué par un groupe hétérocyclique des points (1) à (14) ci-dessus,
(A27) un cycloalcane(en $C_3$-$C_6$)carbonyloxy,
(A28) un alcoxy en $C_1$-$C_6$ substitué par le groupe constitué des carboxy, carboxy protégé, alcanoyle en $C_1$-$C_6$, cycloalcane(en $C_3$-$C_7$)carbamoyle, et alkyl (en $C_1$-$C_6$)-carbamoyle,
(A29) un alkyl (en $C_1$-$C_6$) carbamoyloxyalkyle en $C_1$-$C_6$,
(A30) un alcoxy(en $C_1$-$C_6$) carbonylaminoalkyle en $C_1$-$C_6$,
(A31) un aminoalkyle en $C_1$-$C_6$,
(A32) un alkyl (en $C_1$-$C_6$) carbamoylalkyle en $C_1$-$C_6$,
(A33) un hétérocycle-carbonylamino, le groupe hétérocyclique étant choisi parmi (1) à (14) ci-dessus et étant optionnellement un groupe protecteur N-substitué,
(A34) les groupes hétérocycliques (1) à (14) ci-dessus étant optionnellement substitués par des alkyle en $C_1$-$C_6$, et
(A35) un oxo.

**2.** Composé de la revendication 1, dans lequel
$R^1$ est un alkyle en $C_1$-$C_6$, un groupe hétérocyclique optionnellement substitué constitué des (1) à (10) suivants; ou un aryle en $C_6$-$C_{10}$ optionnellement substitué,
$R^2$ est un carboxy ou un hydroxyaminocarbonyle,
Ar est un phényle ou un groupe hétérocyclique du point (3) suivant,
et
m et n sont chacun un nombre entier de 0 ou 1, et m+n = 1 ou 2,
où le groupe hétérocyclique est :

(1) un groupe hétéromonocyclique insaturé à 5 ou 6 membres, contenant 1 à 4 atomes d'azote,
(2) un groupe hétéromonocyclique saturé à 5 ou 6 membres, contenant 1 à 4 atomes d'azote,
(3) un groupe hétéromonocyclique insaturé à 5 ou 6 membres, contenant 1 à 2 atomes de soufre,
(4) un groupe hétérocyclique insaturé bicyclique à 9 ou 10 membres, contenant 1 à 5 atomes d'azote,
(5) un groupe hétéromonocyclique insaturé à 5 ou 6 membres, contenant 1 à 2 atomes d'oxygène,
(6) un groupe hétéromonocyclique saturé à 5 ou 6 membres, contenant 1 ou 2 atomes d'oxygène,
(7) un groupe hétéromonocyclique insaturé à 5 ou 6 membres, contenant 1 ou 2 atomes d'oxygène et 1 à 3 atomes d'azote,
(8) un groupe hétérocyclique insaturé bicyclique à 9 ou 10 membres, contenant 1 ou 2 atomes d'oxygène,
(9) un groupe hétérocyclique insaturé bicyclique à 9 ou 10 membres, contenant 1 ou 2 atomes de soufre, ou
(10) un groupe hétéromonocyclique saturé à 5 ou 6 membres, contenant 1 ou 2 atomes d'oxygène et 1 à 3 atomes d'azote,

où le groupe hétérocyclique étant optionnellement substitué par le groupe constitué des (B1) à (B8) suivants:

(B1) un alcanoyle en $C_2$-$C_6$,
(B2) un alkyle en $C_1$-$C_6$,
(B3) un alcoxy en $C_1$-$C_6$,

(B4) un alcoxy (en $C_1$-$C_6$) carbonylamino,
(B5) un carbamoyle ou un alkyl(en $C_1$-$C_6$)carbamoyle,
(B6) un alcoxy (en $C_1$-$C_6$)carbonyle,
(B7) un halo, et
(B8) un oxo.

et l'aryle mentionné ci-dessus est optionnellement substitué par le groupe constitué des (A1) à (A35) comme définis dans la revendication 1.

**3.** Composé de la revendication 2, dans lequel un groupe de formule :

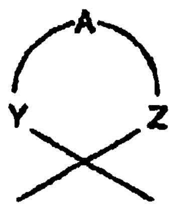

est l'une des formules suivantes :

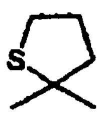 , 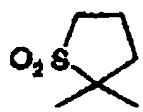

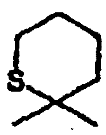 , 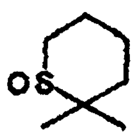 , 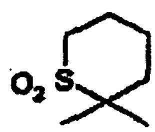 ou 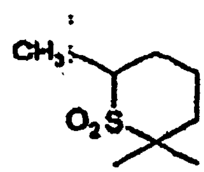

$R^1$ est un alkyle en $C_1$-$C_6$ , un groupe hétérocyclique optionnellement substitué constitué des (1) à (10) suivants ; un phényle optionnellement substitué : ou un naphtyle optionnellement substitué,
$R^2$ est le même que défini dans la revendication 2,
Ar est un phényle ou un thiényle, et
m et n sont chacun un nombre entier de 0 ou 1, et m+n = 1 ou 2,
où le groupe hétérocyclique mentionné ci-dessus est :

(1) un pyrrolyle, un pyrrolinyle, un imidazolyle, un pyrazolyle, un pyridyle, un N-oxyde de pyridyle, un pyrimidyle, un pyrazinyle, un pyridazinyle, un triazolyle, un tétrazolyle, un dihydrotriazinyle,
(2) un azétidinyle, un pyrrolidinyle, un imidazolidinyle, un pipéridinyle, un pipéridino, un pyrazolidinyle, un pipérazinyle,
(3) un thiényle,
(4) un indolyle, un isoindolyle, un indolizinyle, un benzimidazolyle, un quinolyle, un isoquinolyle, un tétrahydroisoquinolyle, un indazolyle, un benzotriazolyle, un tétrazolopyridyle, un tétrazolopyridazinyle, un dihydrotriazolopyridazinyle,
(5) un furyle,
(6) un oxolanyle,
(7) un oxazolyle, un isoxazolyle, un oxadiazolyle,
(8) un benzofuranyle, un benzodihydrofuranyle, un benzodioxolényle,
(9) un benzothiényle, un dihydrobenzothiényle, ou
(10) un morpholinyle, un morpholino,

où le groupe hétérocyclique étant optionnellement substitué par le groupe constitué des (B1) à (B8) comme définis dans la revendication 2,
et le phényle ou naphtyle mentionné ci-dessus est optionnellement substitué par le groupe constitué des (A1) à (A34) suivants :

(A1) un halogène
(A2) un alkyle en $C_1$-$C_6$
(A3) un alcoxy en $C_1$-$C_6$
(A4) un haloalkyle en $C_1$-$C_6$
(A5) un haloalcoxy en $C_1$-$C_6$
(A6) un alcényle en $C_2$-$C_6$
(A7) un acyle,
(A8) un alkyl(en $C_1$-$C_6$)thio, un alkyl(en $C_1$-$C_6$)sulfinyle, un alkyl(en $C_1$-$C_6$)sulfonyle,
(A9) un aryle en $C_6$-$C_{10}$,
(A10) un haloaryle en $C_6$-$C_{10}$,
(A11) un hydroxy,
(A12) un hydroxyalkyle en $C_1$-$C_6$, un hydroxy(protégé)alkyle en $C_1$-$C_6$,
(A13) un amino,
(A14) un carboxy,
(A15) un carboxy protégé,
(A16) un nitroalcényle en $C_2$-$C_6$,
(A17) un alkylène(en $C_1$-$C_6$)dioxy,
(A18) un acylamino,
(A19) un nitro,
(A20) un aryl(en $C_6$-$C_{10}$)alcoxy en $C_1$-$C_6$,
(A21) un carbamoylalcényle en $C_2$-$C_6$ optionnellement N-substitué par le groupe constitué des alkyle en $C_1$-$C_6$, aryle en $C_6$-$C_{10}$, alcoxy(en $C_1$-$C_6$)aryle en $C_6$-$C_{10}$, et haloaryle en $C_6$-$C_{10}$,
(A22) un alkyl(en $C_1$-$C_6$)aminocarbonyloxy,
(A23) un alcanoyloxy en $C_1$-$C_6$,
(A24) un alcoxy(en $C_1$-$C_6$) alcanoyloxy en $C_1$-$C_6$,
(A25) un alcoxy(en $C_1$-$C_6$) carbonyloxy,
(A26) un alcénoyloxy en $C_1$-$C_6$ optionnellement substitué par le groupe hétérocyclique (1) ci-dessus,
(A27) un cycloalcane (en $C_3$-$C_6$) carbonyloxy,
(A28) un alcoxy en $C_1$-$C_6$ substitué par le groupe constitué des carboxy, carboxy protégé, alcanoyle en $C_1$-$C_6$, cycloalcane(en $C_3$-$C_7$)carbamoyle, et alkyl (en $C_1$-$C_6$)-carbamoyle,
(A29) un alkyl(en $C_1$-$C_6$)carbamoyloxyalkyle en $C_1$-$C_6$,
(A30) un alcoxy(en $C_1$-$C_6$)carbonylaminoalkyle en $C_1$-$C_6$,
(A31) un aminoalkyle en $C_1$-$C_6$,
(A32) un alkyl (en $C_1$-$C_6$)carbamoylalkyle en $C_1$-$C_6$,
(A33) un hétérocyle-carbonylamino, le groupe hétérocyclique étant choisi parmi (2), (4) et (5) ci-dessus et optionnellement substitué par un groupe N-protecteur, et
(A34) le groupe hétérocyclique de (7) ci-dessus étant optionnellement substitué par un alkyle en $C_1$-$C_6$.

**4.** Composé de la revendication 3, ayant la formule suivante

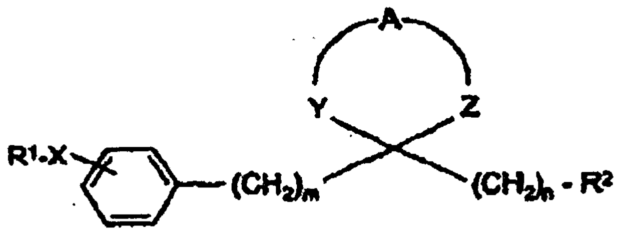

dans laquelle un groupe de la formule :

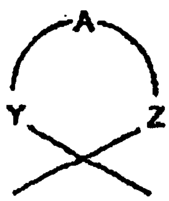

est le même que défini dans la revendication 3,
$R^1$ est un alkyle en $C_1$-$C_6$, un phényle, un halophényle, ou un (halophényl)phényle,
$R^2$ est le même que défini dans la revendication 3, et
m et n sont chacun un nombre entier de 0 ou 1, et m+n = 1.

**5.** Composé de la revendication 3, ayant la formule suivante

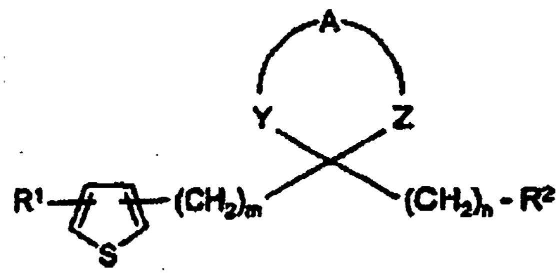

dans laquelle un groupe de la formule :

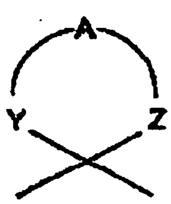

est le même que défini dans la revendication 3,
$R^2$ est le même que défini dans la revendication 3, et
m et n sont chacun un nombre entier de 0 ou 1, et m+n = 1, $R^1$ est un groupe hétérocyclique constitué de pyridyle, thiényle, furyle, benzofuranyle ou benzothiényle, où le groupe hétérocyclique est optionnellement substitué par le groupe constitué des alcanoyle en $C_1$-$C_6$, alkyle en $C_1$-$C_6$, alcoxy en $C_1$-$C_6$, alcoxy(en $C_1$-$C_6$)carbonylamino et alkyl(en $C_1$-$C_6$)carbamoyle ; un naphtyle ou un phényle, chacun de ceux-ci étant optionnellement substitué par le groupe constitué des (C1) à (C31) suivants ;

(C1) un halogène,
(C2) un alkyle en $C_1$-$C_6$,
(C3) un alcoxy en $C_1$-$C_6$,
(C4) un haloalkyle en $C_1$-$C_6$,
(C5) un haloalcoxy en $C_1$-$C_6$,
(C6) un alcényle en $C_2$-$C_6$,
(C7) un alkyl (en $C_1$-$C_6$)carbamoyle, un carbamoyle, un phénylalkyl(en $C_1$-$C_6$)carbamoyle, un alcanoyle en $C_1$-$C_6$,
(C8) un alkyl(en $C_1$-$C_6$)thio, un alkyl(en $C_1$-$C_6$)sulfinyle, un alkyl (en $C_1$-$C_6$)sulfonyle,
(C9) un phényle, un naphthyle,

(C10) un halophényle,
(C11) un hydroxy,
(C12) un mono- ou dihydroxyalkyle en $C_1$-$C_6$, un phénoxycarbonyloxyalkyle en $C_1$-$C_6$,
(C13) un amino,
(C14) un carboxy,
(C15) un alkylène(en $C_1$-$C_6$)dioxy,
(C16) un alcanoyl (en $C_1$-$C_6$) amino, un phénylalcanoyl(en $C_1$-$C_6$)amino, un halophénylalcanoyl(en $C_1$-$C_6$)-amino, un alcoxy(en $C_1$-$C_6$)alcanoyl(en $C_1$-$C_6$)amino, un phénoxyalcanoyl (en $C_1$-$C_6$)amino, un alcoxy(en $C_1$-$C_6$)-phénoxyalcanoyl(en $C_1$-$C_6$)amino, un alkyl(en $C_1$-$C_6$)-phénoxyalcanoyl(en $C_1$-$C_6$)amino, un halophénoxyalcanoyl(en $C_1$-$C_6$)amino, un carboxyalcanoyl(en $C_1$-$C_6$)amino, un alcoxy(en $C_1$-$C_6$)carbonylalcanoyl(en $C_1$-$C_6$)amino, un alkyl (en $C_1$-$C_6$)carbamoylalcanoyl (en $C_1$-$C_6$)amino, un haloalcanoyl (en $C_1$-$C_6$)amino, un alcényl(en $C_2$-$C_6$)-alcanoyl (en $C_1$-$C_6$)amino, un alcoxy(en $C_1$-$C_6$)-alcanoyl(en $C_1$-$C_6$)amino, un phénylalcoxy (en $C_1$-$C_6$)-alcanoyl(en $C_1$-$C_6$) amino, un pipéridinyloxyalcanoyl(en $C_1$-$C_6$)-amino, un N-alcoxy(en $C_1$-$C_6$)carbonylpipéridinyloxyalcanoyl(en $C_1$-$C_6$)amino, un pyridyloxyalcanoyl(en $C_1$-$C_6$)-amino, un hydroxyalcanoyl(en $C_1$-$C_6$) amino, un alcanoyl (en $C_1$-$C_6$)oxyalcanoyl (en $C_1$-$C_6$)amino, un alkyl(en $C_1$-$C_6$)carbamoyloxyalcanoyl (en $C_1$-$C_6$)amino, un N,N-di(alkyl(en $C_1$-$C_6$))carbamoyloxy, un alcanoyl(en $C_1$-$C_6$)-amino, un pipéridinocarbonyloxyalcanoyl(en $C_1$-$C_6$)amino, un phénylalkyl(en $C_1$-$C_6$)carbamoyloxyalcanoyl(en $C_1$-$C_6$)amino, un aminoalcanoyl((en $C_1$-$C_6$)amino, un alcoxy(en $C_1$-$C_6$)carbonylaminoalcanoyl(en $C_1$-$C_6$)amino, un fluorénylméthoxycarbonylaminoalcanoyl(en $C_1$-$C_6$)amino, un alkyl(en $C_1$-$C_6$)aminoalcanoyl (en $C_1$-$C_6$)amino, un [N,N-di (alkyl(en $C_1$-$C_6$))amino]-alcanoyl(en $C_1$-$C_6$)amino, un [N- alkyl(en $C_1$-$C_6$)-N-alcoxy(en $C_1$-$C_6$)carbonyl)amino] alcanoyl(en $C_1$-$C_6$)amino, un [N-alkyl (en $C_1$-$C_6$)-N-fluorénylméthoxycarbonyl)amino]-alcanoyl (en $C_1$-$C_6$)amino, un [N-alkyl(en $C_1$-$C_6$)-N- (mono- ou di (alkyl(en $C_1$-$C_6$)carbamoyl) amino]alcanoyl(en $C_1$-$C_6$)amino, un [N-(mono- or di ((alkyl (en $C_1$-$C_6$)carbamoyl)amino]-alcanoyl (en $C_1$-$C_6$)amino, un benzoylaminoalcanoyl(en $C_1$-$C_6$)-amino, un alcanoyl (en $C_1$-$C_6$)aminoalcanoyl(en $C_1$-$C_6$)amino, un alcane (en $C_1$-$C_6$)sulfonylaminoalcanoyl(en $C_1$-$C_6$)amino, un alcoxy(en $C_1$-$C_6$)alcanoyl(en $C_1$-$C_6$)aminoalcanoyl(en $C_1$-$C_6$)-amino, un cycloalkyl (en $C_3$-$C_6$)oxycarbonylaminoalcanoyl(en $C_1$-$C_6$)amino, un pyridylcarbonylaminoalcanoyl(en $C_1$-$C_6$)amino, un morpholinocarbonylaminoalcanoyl(en $C_1$-$C_6$)amino, un phénylalcoxy(en $C_1$-$C_6$)carbonylaminoalcanoyl(en $C_1$-$C_6$)amino, un alcoxy(en $C_1$-$C_6$)phénylsulfonylaminoalcanoyl (en $C_1$-$C_6$)amino, un hydroxyalkyl(en $C_1$-$C_6$) aminoalcanoyl(en $C_1$-$C_6$)amino, un morpholinoalcanoyl(en $C_1$-$C_6$)amino, un oxooxazolidinylalcanoyl(en $C_1$-$C_6$)amino, un oxopyrrolidinylalcanoyl(en $C_1$-$C_6$)amino, un triméthylhydantoinylalcanoyl(en $C_1$-$C_6$)amino, un alcényl(en $C_2$-$C_6$)aminoalcanoyl(en $C_1$-$C_6$)amino, un alcoxy(en $C_1$-$C_6$)alkyl(en $C_1$-$C_6$)-aminoalcanoyl(en $C_1$-$C_6$)amino, un phénylalkyl(en $C_1$-$C_6$)aminoalcanoyl(en $C_1$-$C_6$)amino, un pyridylalkyl(en $C_1$-$C_6$)aminoalcanoyl(en $C_1$-$C_6$)amino, un alcoxy(en $C_1$-$C_6$)carbonylamino, un phénylalcoxy(en $C_1$-$C_6$)carbonylamino, un alcoxy(en $C_1$-$C_6$)-alcoxy(en $C_1$-$C_6$)carbonylamino, un haloalcoxy(en $C_1$-$C_6$)-carbonylamino, un aminoalaoxy(en $C_1$-$C_6$)carbonylamino, un phtalimidoalcoxy(en $C_1$-$C_6$)carbonylamino, un carbamoylamino, un (mono- ou di (alkyl(en $C_1$-$C_6$))carbamoylamino, un naphtylcarbamoylamino, un halophénylcarbamoylamino, un alcoxy(en $C_1$-$C_6$)phénylcarbamoylamino, un alcényl(en $C_2$-$C_6$)-carbamoylamino, un cycloalkyl(en $C_3$-$C_5$)alkyl(en $C_1$-$C_6$)-carbamoylamino, un phénylalkyl(en $C_1$-$C_6$)carbamoylamino, un haloalkyl (en $C_1$-$C_6$)carbamoylamino, un alcoxy(en $C_1$-$C_6$)-alkyl (en $C_1$-$C_6$)carbamoylamino, un hydroxyalkyl(en $C_1$-$C_6$)-carbamoylamino, un ((alkyl (en $C_1$-$C_6$))(diphényl)silyloxyalkyl(en $C_1$-$C_6$)carbamoylamino, un carboxyalkyl(en $C_1$-$C_6$)-carbamoylamino, un alcoxy(en $C_1$-$C_6$)carbonylalkyl(en $C_1$-$C_6$)-carbamoylamino, un alkyl(en $C_1$-$C_6$)carbamoylalkyl(en $C_1$-$C_6$)-carbamoylamino, ou un pyridylcarbamoylamino, un alkyl(en $C_1$-$C_6$)sulfonylamino, un alcénoyl(en $C_2$-$C_6$)amino, un cycloalcane(en $C_3$-$C_6$)carbonylamino, un alcényl (en $C_2$-$C_6$)oxycarbonylamino, un phénoxycarbonylamino, un alkyl(en $C_1$-$C_6$)-thiocarbonylamino,
(C17) un phénylalcoxy en $C_1$-$C_6$,
(C18) un alcényle en $C_2$-$C_6$, un mono- ou di (alkyl (en $C_1$-$C_6$))-carbamoylalcényle en $C_2$-$C_6$, un 2-(méthylcarbamoyl)éthényle, un 2-(éthylcarbamoyl)éthényle, un 2-(propylcarbamoyl)-éthényle, un 2-(isopropylcarbamoyl)éthényle, un 2-(diméthylcarbamoyl)éthényle, un phénylcarbamoylalcényl en $C_2$-$C_6$, un alcoxy(en $C_1$-$C_6$) carbamoylalcényle en $C_2$-$C_6$, un halophénylcarbamoylalcényle en $C_2$-$C_6$,
(C19) un alkyl(en $C_1$-$C_6$)aminocarbonyloxy,
(C20) un alcanoyl(en $C_1$-$C_6$)oxy,
(C21) un alcoxy(en $C_1$-$C_6$)alcanoyl(en $C_1$-$C_6$)oxy,
(C22) un alcoxy(en $C_1$-$C_6$)carbonyloxy,
(C23) un pyridylalcénoyl(en $C_2$-$C_6$)oxy,
(C24) un cycloalcane(en $C_3$-$C_6$)carbonyloxy,
(C25) un carboxyalcoxy en $C_1$-$C_6$, un alcoxy(en $C_1$-$C_6$)carbonylalcoxy en $C_1$-$C_6$, un alcanoyl(en $C_1$-$C_6$)alcoxy en $C_1$-$C_6$, un cycloalcane(en $C_3$-$C_7$)carbamoylalcoxy en $C_1$-$C_6$, un alkyl(en $C_1$-$C_6$)carbamoylalcoxy en $C_1$-$C_6$,
(C26) un alkyl(en $C_1$-$C_6$)carbamoyloxyalkyle en $C_1$-$C_6$,

(C27) un alcoxy(en $C_1$-$C_6$)carbonylaminoalkyle en $C_1$-$C_6$,
(C28) un aminoalkyle en $C_1$-$C_6$,
(C29) un alkyl (en $C_1$-$C_6$)carbamoylalkyle en $C_1$-$C_6$,
(C30) un furylcarbonylamino, un tétrahydroisoquinolylcarbonylamino, un N-alcoxy(en $C_1$-$C_6$)-carbonyltétrahydroisoquiuolylcarbonylamino, un pyrrolidinylcarbonylamino,
(C31) un oxazolyle, un alkyl(en $C_1$-$C_6$)oxadiazolyle.

**6.** Composé de la revendication 5, dans lequel un groupe de la formule :

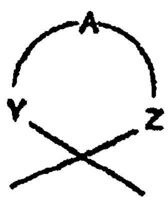

est l'une des formules suivantes :

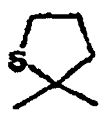 , 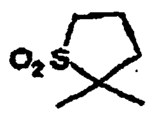

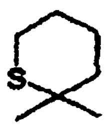 , 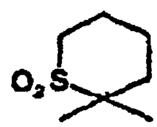

$R^2$ est le même que défini dans la revendication 5,
m est 0 et n est 1,
un groupe de formule :

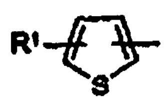

est le groupe des formules (a) à (e) suivantes :

(a)

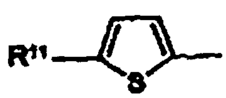

dans laquelle
$R^{11}$ est un halo, un naphtyle, un phényle, un mono- ou di halophényle, un mono ou dialkyl (en $C_1$-$C_6$)phényle, un alcoxy(en $C_1$-$C_6$)phényle, un trihaloalkyl(en $C_1$-$C_6$)phényle, un trihaloalcoxy(en $C_1$-$C_6$)phényle, un alcényl (en $C_2$-$C_6$)phényle, un alkyl(en $C_1$-$C_6$)carbamoylphényle, un carbamoylphényle, un phénylalkyl(en $C_1$-$C_6$)carbamoylphényle, un alcanoyl(en $C_1$-$C_6$)phényle, un alkyl(en $C_1$-$C_6$)thiophényle, un alkyl(en $C_1$-$C_6$)sulfinylphé-

nyle, un alkyl(en $C_1$-$C_6$)-sulfonylphényle, un phénylphényle, un (halo) (phényl)phényle, un halophénylphényle, un hydroxyphényle, un mono ou dihydroxy- alkyl(en $C_1$-$C_6$)phényle, un phénoxycarbonyloxyalkyl(en $C_1$-$C_6$) phényle, un aminophényle, un caboxyphényle, un alkylène(en $C_1$-$C_6$)dioxyphényle, un alcane(en $C_1$-$C_6$)sulfonylaminophényle, un alcénoyl(en $C_2$-$C_6$)aminophényle, un cycloalcane (en $C_3$-$C_6$)carbonylaminophényle, un phénylalcoxy(en $C_1$-$C_6$)phényle, un mono ou di(alkyle(en $C_1$-$C_6$))carbamoylalcényl(en $C_2$-$C_6$)phényle, un phénylcarbamoylalcényl(en $C_2$-$C_6$)phényle, un alcoxy(en $C_1$-$C_6$)carbamoylalcényle(en $C_2$-$C_6$)phényle, un halophénylcarbamoylalcényle(en $C_2$-$C_6$)phényle, un alkyle (en $C_1$-$C_6$)carbamoyloxyphényle, un alcanoyl(en $C_1$-$C_6$)oxyphényle, un alcoxy(en $C_1$-$C_6$)alcanoyl(en $C_1$-$C_6$)oxyphényle, un alcoxy(en $C_1$-$C_6$)carbonyloxyphényle, un pyridylalcénoyl(en $C_2$-$C_6$)oxyphényle, un cycloalkyl(en $C_3$-$C_6$)-carbonyloxyphényle, un carboxyalcoxy (en $C_1$-$C_6$)phényle, un alcoxy(en $C_1$-$C_6$)carbonylalcoxy(en $C_1$-$C_6$)phényle, un alcanoyl(en $C_1$-$C_6$)alcoxy(en $C_1$-$C_6$)phényle, un cycloalcane(en $C_3$-$C_6$)carbamoylalcoxy(en $C_1$-$C_6$)phényle, un alkyl(en $C_1$-$C_6$)carbamoylalcoxy(en $C_1$-$C_6$)phényle, un alkyl (en $C_1$-$C_6$)carbamoyloxyalkyl(en $C_1$-$C_6$)phényle, un alcoxy(en $C_1$-$C_6$)carboxylaminoalkyl(en $C_1$-$C_6$)phényle, un aminoalkyl(en $C_1$-$C_6$)phényle, un alkyl(en $C_1$-$C_6$)carbamoylalkyl(en $C_1$-$C_6$)phényle, un furylcarbonylaminophényle, un 1,2,3,4-tétrahydroisoquinolylcarbonylaminophényle, un N-t-butoxycarbonyle, un 1,2,3,4-tétrahydroisoquinolylcarbonylaminophényle, un pyrrolidinylcarbonylaminophényle, un oxazolylphényle, un alkyl(en $C_1$-$C_6$)oxadiazolylphényle.

(b)

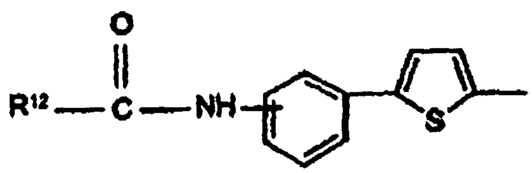

dans laquelle $R^{12}$ est un alkyle en $C_1$-$C_6$ optionnellement substitué par le groupe constitué des phényle, halophényle, alcoxy(en $C_1$-$C_6$)phényle, alcoxy en $C_1$-$C_6$, phénoxy, alcoxy(en $C_1$-$C_6$)phénoxy, halophénoxy, alkyl(en $C_1$-$C_6$)phénoxy, carboxy, alcoxy(en $C_1$-$C_6$)carbonyle, alkyl(en $C_1$-$C_6$)carbamoyle, halo, un alcényloxy en $C_2$-$C_6$, alcoxy(en $C_1$-$C_6$)alcoxy en $C_1$-$C_6$, phénylalcoxy en $C_1$-$C_6$, pipéridinyloxy, N-alcoxy(en $C_1$-$C_6$)-carbonylpipéridinyloxy, pyridyloxy, hydroxy, alcanoyloxy en $C_1$-$C_6$, mono- ou dialkyl(en $C_1$-$C_6$)carbamoyloxy, pipéridinylcarbonyloxy, phénylalkyl(en $C_1$-$C_6$)carbamoyloxy, amino, alcoxy(en $C_1$-$C_6$)carbonylamino, fluorénylméthoxycarbonylamino, mono- ou dialkyl(en $C_1$-$C_6$)-amino, N-alkyl(en $C_1$-$C_6$)-N-(alcoxy(en $C_1$-$C_6$)carbonyl) amino, N-alkyl (en $C_1$-$C_6$)-N-(fluorénylméthoxycarbonyl)amino, N-alkyl(en $C_1$-$C_6$)-N-(mono- ou dialkyl(en $C_1$-$C_6$)carbamoyl)-amino, N-(mono- ou dialkyl (en $C_1$-$C_6$)carbamoyl)amino, benzoylamino, alcanoyl(en $C_1$-$C_6$) amino, alcane(en $C_2$-$C_6$)-sulfonylamino, alcoxy(en $C_1$-$C_6$)alcanoyl(en $C_1$-$C_6$)amino, cycloalkyl(en $C_3$-$C_6$)oxycarbonylamino, pyridylcarbonylamino, morpholinocarbonylamino, phénylalcoxy(en $C_1$-$C_6$) carbonylamino, alcoxy (en $C_1$-$C_6$)phénylsulfonylamino, hydroxyalkyl(en $C_1$-$C_6$)-amino, morpholino, oxooxazolidinyle, oxopyrrolidinyle, triméthylhydantoinyle, pyridyle, alcényl(en $C_2$-$C_6$)amino, alcoxy(en $C_1$-$C_6$)alkyl(en $C_1$-$C_6$)amino, phénylalkyl(en $C_1$-$C_6$)-amino, pyridylalkyl(en $C_1$-$C_6$)amino, et cycloalkyle en $C_3$-$C_6$,

(c)

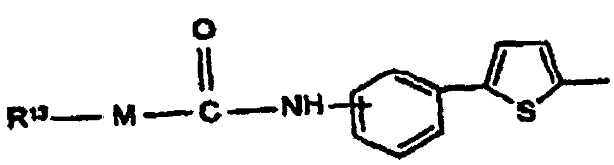

dans laquelle

M est un oxygène ou un soufre,

$R^{13}$ est un alkyle en $C_1$-$C_6$, un phénylalkyle en $C_1$-$C_6$, un alcoxy(en $C_1$-$C_6$)alkyle en $C_1$-$C_6$, un haloalkyle en $C_1$-$C_6$, un aminoalkyle en $C_1$-$C_6$, ou un phthalimidoalcoxy(en $C_1$-$C_6$)-carbonylamino, un alcényle en $C_2$-$C_6$, un phényle,

(d)

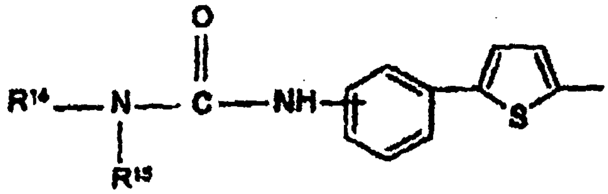

dans laquelle
$R^{15}$ est un hydrogène ou un alkyle en $C_1$-$C_6$,
$R^{14}$ est un hydrogène, un alkyle en $C_1$-$C_6$, un naphtyle, un halophényle, un alcoxy(en $C_1$-$C_6$)phényle, un alcényle en $C_2$-$C_6$, un cycloalkyl (en $C_3$-$C_6$) alkyle en $C_1$-$C_6$, un phénylalkyle en $C_1$-$C_6$, un haloalkyle en $C_1$-$C_6$, un alcoxy(en $C_1$-$C_6$)alkyle en C1-C6, un hydroxyalkyle en $C_1$-$C_6$, un alkyle(en $C_1$-$C_6$) (diphényl)silyloxyalkyle en $C_1$-$C_6$, un carboxyalkyle en $C_1$-$C_6$, un alcoxy(en $C_1$-$C_6$)carbonylalkyle en $C_1$-$C_6$, un alkyl(en $C_1$-$C_6$)carbamoylalkyle en $C_1$-$C_6$, ou un pyridyle,

(e)

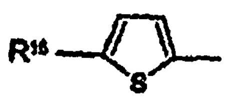

dans laquelle
$R^{16}$ est un benzothiényle, un benzofuranyle, un thiényle, un furyle, un alkyl(en $C_1$-$C_6$)pyridyle, un pyridyle, un alcoxy(en $C_1$-$C_6$)pyridyle, un alcoxy(en $C_1$-$C_6$)carbonylaminopyridyle, un alcanoyl (en $C_1$-$C_6$)thiényle, un alkyl (en $C_1$-$C_6$)-carbamoylbenzofuranyle.

**7.** Composé de la revendication 6, dans lequel un groupe de la formule :

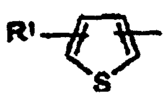

est le même que (a), (c), (d) et (e) de la revendication 6, et la formule suivante (b) :

(b)

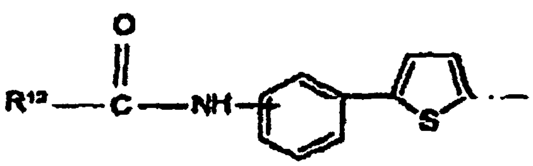

dans laquelle
$R^{12}$ est un alkyle en $C_1$-$C_6$, un phénylalkyl en $C_1$-$C_6$, un halophénylalkyle en $C_1$-$C_6$, un alcoxy(en $C_1$-$C_6$) phénylalkyle en $C_1$-$C_6$, un alcoxy(en $C_1$-$C_6$)alkyle en $C_1$-$C_6$, un phénoxyalkyle en $C_1$-$C_6$, un alcoxy (en $C_1$-$C_6$)

phénoxyalkyle en $C_1$-$C_6$, un halophénoxyalkyle en $C_1$-$C_6$, un alkyl(en $C_1$-$C_6$)phénoxyalkyle en $C_1$-$C_6$, un carboxyalkyle en $C_1$-$C_6$, un alcoxy(en $C_1$-$C_6$)-carbonylalkyle en $C_1$-$C_6$, un alkyl(en $C_1$-$C_6$)carbamoylalkyle en $C_1$-$C_6$, un haloalkyle en $C_1$-$C_6$, un alcéayl(en $C_2$-$C_6$)oxy-alkyle en $C_1$-$C_6$, un alcoxy(en $C_1$-$C_6$)alcoxy(en $C_1$-$C_6$) alkyle en $C_1$-$C_6$, un phénylalcoxy(en $C_1$-$C_6$)alkyle en $C_1$-$C_6$, un pipéridinyloxyalkyle en $C_1$-$C_6$, un N-t-butoxycarbonylpipéridinyloxyalkyle en $C_1$-$C_6$, un pyridyloxyalkyle en $C_1$-$C_6$, un hydroxyalkyle en $C_1$-$C_6$, un alcanoyloxy(en $C_1$-$C_6$)alkyle en $C_1$-$C_6$, un mono- ou dialkyl(en $C_1$-$C_6$)carbamoyloxyalkyle en $C_1$-$C_6$, un pipéridinylcarbonyloxyalkyle en $C_1$-$C_6$, un phénylalkyl(en $C_1$-$C_6$)carbamoyloxyalkyle en $C_1$-$C_6$, un aminoalkyle en $C_1$-$C_6$, un alcoxy(en $C_1$-$C_6$)carbonylaminoalkyle en $C_1$-$C_6$, un fluorénylméthoxycarbonylaminoalkyle en $C_1$-$C_6$, un mono- ou dialkyl(en $C_1$-$C_6$)aminoalkyle en $C_1$-$C_6$, un N-alkyl(en $C_1$-$C_6$)-N- (alcoxy(en $C_1$-$C_6$)carbonyl)aminoalkyle en $C_1$-$C_6$, un N-alkyl(en $C_1$-$C_6$)-N- (fluorénylméthoxycarbonyl)-aminoalkyle en $C_1$-$C_6$, un N-alkyl(en $C_1$-$C_6$)-N- (mono- ou dialkyl(en $C_1$-$C_6$)carbamoyl)aminoalkyle en $C_1$-$C_6$, un N-(monoou di (alkyl (en $C_1$-$C_6$)) carbamoyl)aminoalkyle en $C_1$-$C_6$, un benzoylaminoalkyle en $C_1$-$C_6$, un alcanoyl (en $C_1$-$C_6$)aminoalkyle en $C_1$-$C_6$, un alcane(en $C_1$-$C_6$)sulfonylaminoalkyle en $C_1$-$C_6$, un alcoxy(en $C_1$-$C_6$)alcanoyl(en $C_1$-$C_6$)aminoalkyle en $C_1$-$C_6$, un cycloalkyl (en $C_1$-$C_6$)oxycarbonylaminoalkyle en $C_1$-$C_6$, un pyridylcarbonylaminoalkyle en $C_1$-$C_6$, un morpholinocarbonylaminoalkyle en $C_1$-$C_6$, un phénylalcoxy(en $C_1$-$C_6$)oxycarbonylaminoalkyle en $C_1$-$C_6$, un alcoxy(en $C_1$-$C_6$)phénylsulfonylaminoalkyle en $C_1$-$C_6$, un hydroxyalkyl(en $C_1$-$C_6$)aminoalkyle en $C_1$-$C_6$, un morpholinoalkyle en $C_1$-$C_6$, un oxooxazolidinylalkyle en $C_1$-$C_6$, un oxopyrrolidinylalkyle en $C_1$-$C_6$, un triméthylhydantoinylalkyle en $C_1$-$C_6$, un pyridylalkyle en $C_1$-$C_6$, un alcényl(en $C_2$-$C_6$)aminoalkyle en $C_1$-$C_6$, un alcoxy(en $C_1$-$C_6$)alkyl(en $C_1$-$C_6$)aminoalkyle en $C_1$-$C_6$, un phénylalkyl(en $C_1$-$C_6$)aminoalkyle en $C_1$-$C_6$, un pyridylalkyl(en $C_1$-$C_6$)aminoalkyle en $C_1$-$C_6$, un cycloalkyle en $C_3$-$C_6$, un (amino)(phényle)alkyl(en $C_1$-$C_6$)amino, un (alcoxy(en $C_1$-$C_6$) carbonylamino) (phényle)alkyle en $C_1$-$C_6$, un (amino) (alcoxy(en $C_1$-$C_6$)) alkyle en $C_1$-$C_6$, un (alcoxy(en en $C_1$-$C_6$)carboylamino)-(alcoxy(en $C_1$-$C_6$)alkyle en $C_1$-$C_6$, un (amino) (carboxy)alkyle en $C_1$-$C_6$, un (alcoxy(en $C_1$-$C_6$)carbonylamino) (carboxy)alkyle en $C_1$-$C_6$, un (amino) (alcoxy(en $C_1$-$C_6$)carbonyl) alkyle en $C_1$-$C_6$, un (alcoxy(en $C_1$-$C_6$)carbonylamino) (alcoxy(en $C_1$-$C_6$)carbonyl)-alkyle en $C_1$-$C_6$, un (amino) (phénylalcoxy(en $C_1$-$C_6$)alkyle en $C_1$-$C_6$, un (alcoxy(en $C_1$-$C_6$)carbonylamino) (phénylalcoxy(en $C_1$-$C_6$) alkyle en $C_1$-$C_6$, un (amino) (pyridyl)alkyle en $C_1$-$C_6$, un (alcoxy(en $C_1$-$C_6$) carbonylamino) (pyridyl) alkyle en $C_1$-$C_6$, un (amino) (hydroxy)alkyle en $C_1$-$C_6$, un (alcoxy(en $C_1$-$C_6$) carbonylamino) (hydroxy)alkyle en $C_1$-$C_6$, un (amino) (amino)alkyle en $C_1$-$C_6$, un (alcoxy(en $C_1$-$C_6$) carbonylamino) (amino)alkyle en $C_1$-$C_6$, un (amino) (alcoxy (en $C_1$-$C_6$)-carbonylaminoalkyle en $C_1$-$C_6$, un (alcoxy(en $C_1$-$C_6$)carbonylamino) (alcoxy(en $C_1$-$C_6$) carbonylamino) alkyle en $C_1$-$C_6$, un (amino) (cycloalcane (en $C_1$-$C_6$) alkyle e en $C_1$-$C_6$, un (alcoxy(en $C_1$-$C_6$)carbonylamino) (cycloalcane en $C_3$-$C_6$)alkyle en $C_1$-$C_6$.

**8.** Composé de la revendication 6, dans lequel un groupe de la formule :

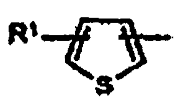

est le groupe de la formule (a) à (e) suivante :

(a)

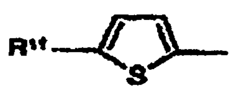

dans laquelle

$R^{11}$ est un brome, un 2-naphtyle, un phényle, un 3 (ou 4)-chlorophényle, un 2 (ou 3 ou 4)-fluorophényle, un 3,4-dichlorcphényle, un 3,5-difluorophényle, un 3 (ou 4)-méthylphényle, un 4-éthylphényle, un 4-isopropylphényle, un 4-(t-butyl)phényle, un 3,4-diméthylphényle, un 4-méthoxyphényle, un 4-éthoxyphényle, un 4-trifluorométhylphényle, un 4-trifluorométhoxyphényle, un 4-éthénylphényle, un 4-méthylcarbamoylphényle, un 4-éthylcarbamoylphényle, un 4-carbamoylphényle, un 4-bénzylcarbamoylphényle, un 4-acétylphényle, un 4-méthylthiophényle, un 4-éthylthiophényle, un 4-méthylsulfinylphényle, un 4-méthylsulfonylphényle, un phénylphényle, un 4-phényl-3-fluorophényle, un 4-(4-fluorophényl)phényle, un 3 (ou 4)-hydroxyphényle, un 3 (ou

4)-hydroxyméthylphényle, un 4-(1,2-dihydroxyéthyl)-phényle, un 4-(phénoxycarbonyloxyméthyl)phényle, un 3 (ou 4)aminophényle, un 4-carboxyphényle, un 3,4-méthylènedioxyphényle, un 4- (méthanesulfonylamino) phényle, un 3-(2-buténoylamino)phényle, un 3-(cyclopropanecarbonylamino)phényle, un 3-(cyclobutanecarbonylamino)phényle, un 3- (cyclopentanecarbonylamino)phényle, un 4-benzyloxyphényle, un 4-(2-(méthylcarbamoyl)éthényl)phényle, un 4-(2-(éthylcarbamoyl)éthényl)phényle, un 4-(2-(propylcarbamoyl)éthényl)phényle, un 4-(2-(isopropylcarbamoyl)éthényl)phényle, un 4-(2-(diméthylcarbamoyl)éthényl) phényle, un 4-(2-(phénylcarbamoyl)éthényl)phényle, un 4-(2-(méthoxyphénylcarbamoyl)éthényl)phényle, un 4-(2-(4-fluorophénylcarbemoyl)éthényl)phényle, un 4-(méthylaminocarbonyloxy)phényle, un 4-(éthylaminocarbonyloxy)phényle, un 4-propanoyloxyphényle, un 4-(méthoxyacetyloxy)phényle, un 4-(éthoxycarbonyloxy)phényle, un 4-(3-(3-pyridyl)acryloylcocy)phényle, un 4-(cyclopropylcarbonyloxy)phényle, un 4-(carboxyméthoxy)phényle, un 4-(éthoxycarbonylméthoxy)phényle, un 4-(t-butoxycarbonylméthoxy)phényle, un 4-(propanoylméthoxy)phényle, un 4-(cyclopropylcarbamoylméthoxy)phényle, un 3 (ou 4)-(méthylcarbamoylméthoxy)phényle, un 4-(éthylcarbamoylméthoxy)phenyle, un 4-(propylcarbamoylméthoxy)phényle, un 3 (ou 4)-(méthylcarbamoyloxyméthyl)phényle, un 4-(méthoxycarbonylaminométhyl)phényle, un 4-(t-butoxycarbonylaminométhyl)phényle, un 4-aminométhylphényle, un 4-(méthylcarbamoylméthyl)phényle, un 3-(2-(ou 3)-furylcarbonylamino) phényle, un 3-(1,2,3,4-tétrahydroisoquinolylcarbonylamino)phényle, un 3-(N-(t-butoxycarbonyl)-1,2,3,4-tétrahydroisoquinolylcarbonylamino)phényle, un 3-(pyrrolidinylcarbonylamino)phényle, un 4-(1,3-oxazolyl)phényle. un 4-(5-méthyl-1,2,4-oxadiazol-3-yl)phényle,

(b)

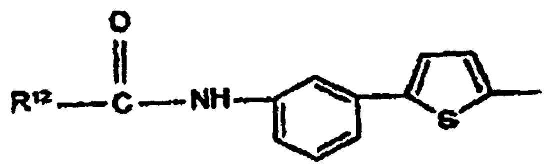

dans laquelle

$R^{12}$ est un méthyle, un éthyle, un propyle, un isopropyle, un butyle, un isobutyle, un t-butyle, un néopentyle, un phénylméthyle, un 4-chlorophénylméthyle, un 4-méthoxyphénylméthyle, un méthoxyméthyle, un éthoxyméthyle, un propoxyméthyle, un butoxyméthyle, un isopropyloxyméthyle, un 1-méthoxyéthyle, un 2-méthoxyéthyle, un phénoxyméthyle, un 2-phénoxyéthyle, un 3 (ou 4)-méthoxyphénoxyméthyle, un 4-fluoro (ou chloro) phénoxyméthyle, un 3 (ou 4)-méthylphénoxyméthyle, un 2-carboxyéthyle, un 2-méthoxycarbonyléthyle, un 2-t-butoxycarbonyléthyle, un 2-méthylcarbamoyléthyle, un 2-chloroéthyle, un chlorométhyle, un allyloxyméthyle, un (2-éthoxyéthoxy)méthyle, un benzyloxyméthyle, un 4-pipéridinyloxyméthyle, un (N-t-butoxycarbonyl-4-pipéridinyl)oxyméthyle, un 3 (ou 4)-pyridyloxyméthyle, un hydroxyméthyle, un 2-hydroxyéthyle, un acétoxyméthyle, un 1-acétoxyéthyle, un méthylcarbamoyloxyméthyle, un 1-(N-méthyl-N-éthylcarbamoyloxy)méthyle, un (pipéridinocarbonyloxy)méthyle, un (benzylcarbamoyloxy)méthyle, un (t-butoxycarbonylamino)-méthyle, un aminométhyle, un 1-aminoéthyle, un 1-(t-butoxycarbonylamino)éthyle, un 2-aminoéthyle, un méthoxycarbonylaminométhyle, un 2- (méthoxycarbonylamino)-éthyle, un éthoxycarbonylaminométhyle, un propoxycarbouylaminométhyle, un 1-(fluorénylméthoxycarbonylamino)méthyle, un 2-(t-butoxycarbouylamino)éthyle, un 2-(fluorénylméthoxycarbonylamino)éthyle, un 1-aminoisopropyle, un 1-aminopropyle, un 1-(t-butoxycarbonylamino)propyle, un 1-(t-butoxycarbonylamino)isopropyle, un 1,5-diaminopentyle, un 1,5-bis-(t-butoxycarbonylamino)pentyle, un méthylaminométhyle, un éthylaminométhyle, un 1-(N-méthyl-N-éthylamino)méthyle, un diméthylaminométhyle, un pentylaminométhyle, un t-butylaminométhyle, un 2-méthylaminoéthyle, un 1-(N-méthyl-N-méthoxycarbonylamino)méthyle, un 1-(N-méthyl-N-t-butoxycarbonylamino)-méthyle, un 1-(N-éthyl-N-t-butoxycarbonylamino) méthyle, un 2-(N-méthyl-N-(fluorénylméthoxycarbonyl)amino) éthyle, un 2-(N-méthyl-N-(t-butoxycarbonyl)amino)éthyle, un 1-(N-méthyl-N-(diméthylcarbamoyl)amino)méthyle, un 1-(diméthylcarbamoyl-amino)méthyle, un 1-(N-(éthylcarbamoyl)amino)méthyle, un 2-(N-(éthylcarbamoyl)amino)éthyle, un benzoylaminométhyle, un 2-benzoylaminoéthyle, un acetylaminométhyle, un isobutyrylaminométhyle, un pivaloylaminométhyle, un 1-(méthanesulfonylamino)méthyle, un 2-(méthanesulfonylamino)éthyle, un méthoxyacétylaminométhyle, un cyclopentyloxycarbonylaminométhyle, un pyridylcarbonylaminométhyle, un morpholinocarbonylaminométhyle, un benzyloxycarbonylaminométhyle, un 1-(4-méthoxyphénylsulfonylamino)méthyle, un 1-(2-hydroxyéthylamino)méthyle, un morpholinométhyle, un 1-(2-oxo-1,3-oxazolidin-1-yl)méthyle, un 1-(2-oxopyrrolidin-1-yl)méthyle, un 1-(3,4,4-triméthylhydantoin-1-yl)méthyle, un allylaminométhyle, un 1-(2-éthoxyéthylamino)méthyle, un benzylaminométhyle, un 1- (3-pyridylméthylamino)méthyle, un 2-phényl-1-aminoéthyle, un 1-amino-1-phénylméthyle, un 1-t-butoxycarbonylamino-1-phénylméthyle, un 1-amino-2-phényléthyle, un 1-t-butoxycarbonylamino-2-phényléthyle, un 1-amino-2-méthoxyéthyle, un 1-t-butoxycarbonylamino-2-méthoxyéthyle, un 1-amino-3-carboxypropyle, un 1-t-butoxycarbonylamino-3-carboxypropyle, un 1-amino-3-(i-butoxycarbonyl)propyle, un 1-t-butoxycarbonylamino-3-t-butoxycarbonylpropyle, un 1-amino-2-benzyloxyéthyle, un 1-t-butoxycarbonylamino-2-benzyloxyaminoéthyle, un 1-amino-2-(3-pyridyl)éthyle, un 1-t-butoxycarbonylamino-2-(3-pyridyl)éthyle, un 1-amino-2-(4-pyridyl)éthyle, un 1-t-butoxycarbonylamino-2-(pyridyl)éthyle, un 1-amino-2-hydroxyéthyle, un t-butoxycarbonylamino-2-hydroxyéthyle, un 1,5-diaminopentyle, un 1-t-butoxycarbonylamino-5-aminopentyle, un 1,5-bis(t-butoxycarbonylamino)pentyle, un 1-amino-5-(t- butoxycarbonylamino)pentyle, un 1-amino-2-cyclohexyléthyle, un 1-t-butoxycarbonylamino-2-cyclohexyléthyle,

(c)

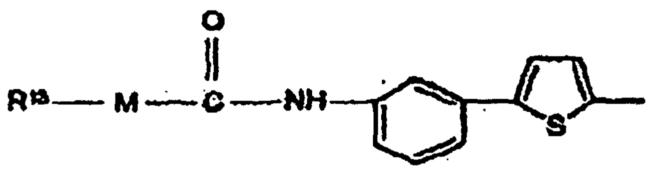

dans laquelle
M = O et $R^{13}$ est un méthyle, un éthyle, un propyle, un isopropyle, un benzyle, un 2-méthoxyéthyle, un 2-chloroéthyle, un 2-aminoéthyle, un 2-phtalimidoézhyle, un allyle, un phényle, ou
M = S et $R^{13}$ est un méthyle, un éthyle,

(d)

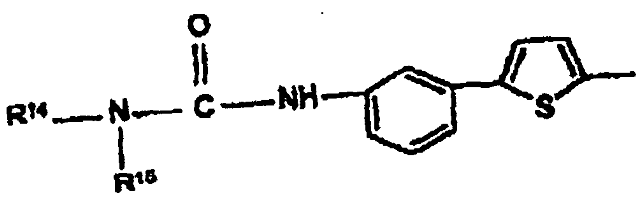

dans laquelle
$R^{15}$ est un hydrogène et
$R^{14}$ est un hydrogène, un méthyle, un éthyle, un propyle, un isopropyle, un butyle, un isobutyle, un pentyle, un hexyle, un 1-naphtyle, un 3 (ou 4)-chlorophényle, un 3-méthoxy- phényle, un allyle, un cyclohexylméthyle, un benzyle, un 2-chloroéthyle, un méthoxyméthyle, un 2-méthoxyéthyle, un 2-hydroxyéthyle, un 2-((t-butyl)-(diphényl)silyloxy)éthyle, un carboxyméthyle, un éthoxycarbonylméthyle, un méthylcarbamoylméthyle, ou un 3-pyridyle, ou
$R^{14}$ est un éthyle et
$R^{15}$ est un méthyle,

(e)

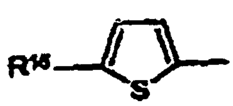

dans laquelle
$R^{16}$ est un 2-benzothiényle, un 2-benzofuranyle, un 2 (ou 3)-thiényle, un 2-furyle, un 3-pyridyle, un 1-méthyl-4-pyridyle, un 6-méthyl-3-pyridyle, un 6-méthoxy-3-pyridyle, un 5-méthoxycarbonylamino-3-pyridyle, un 5-acétyl-2-thiényle, un 2-méthylcarbamoyl-5-benzofuranyle.

**9.** Procédé pour la préparation d'un composé de la formule :

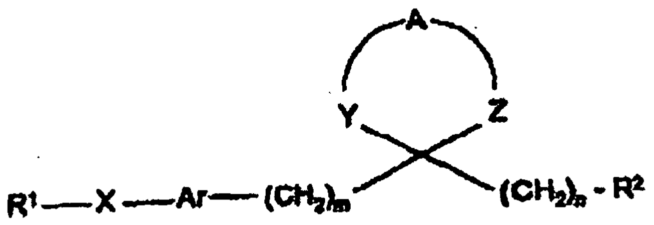

(I)

dans laquelle $R^1$, $R^2$, Ar, A, X, Y, Z, m et n sont chacun tels que définis dans la revendication 1, qui comprend

(1) de soumettre un composé de formule

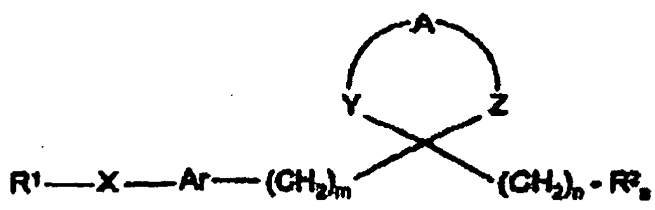

(I-a)

ou un sel de celui-ci à une réaction d'enlèvement du groupe protecteur du carboxy, pour donner un composé de formule :

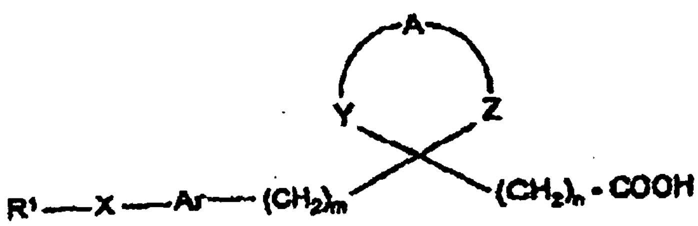

(I-b)

ou un sel de celui-ci ; ou

(2) d'oxyder le groupe vinyle d'un composé de formule :

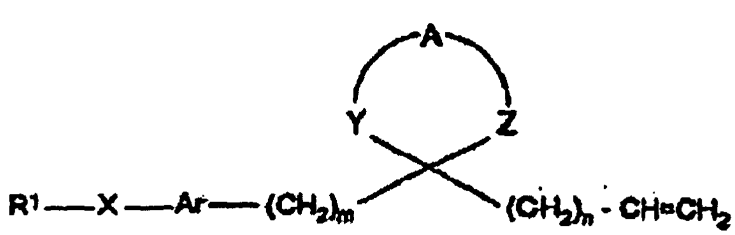

(II)

ou un sel de celui-ci, pour donner un composé de la formule (I-b) ci-dessus ou un sel de celui-ci ; ou

(3) de réduire un composé de formule :

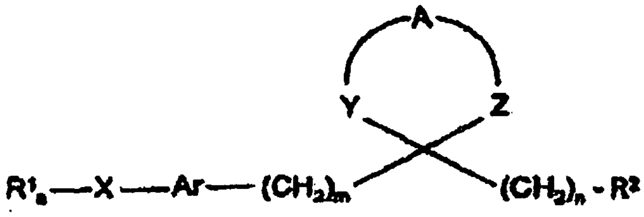

(I-c)

ou un sel de celui-ci, pour donner un composé de formule :

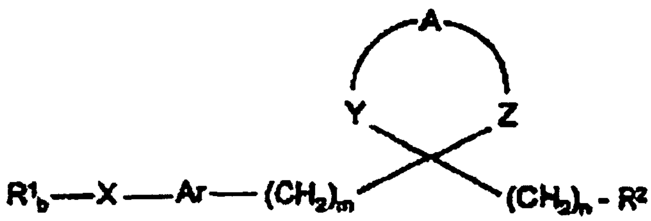

(I-d)

ou un sel de celui-ci ; ou

(4) de faire réagir un composé de la formule (I-b) ci-dessus ou son dérivé réactif au groupe carboxy, ou un sel de celui-ci, avec un composé de formule :

$$NH_2\text{-}OR^3 \quad \text{(IV)}$$

ou son dérivé réactif au groupe amino, ou un sel de celui-ci, pour donner un composé de formule :

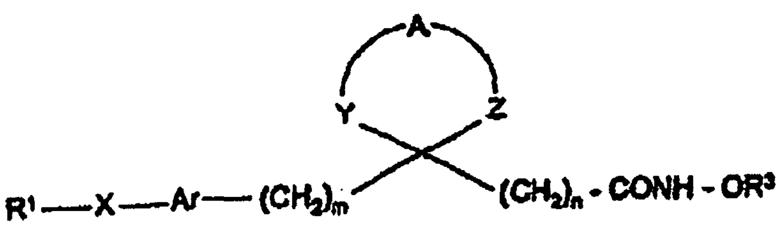

(I-e)

ou un sel de celui-ci ; ou

(5) de cycliser un compose de formule :

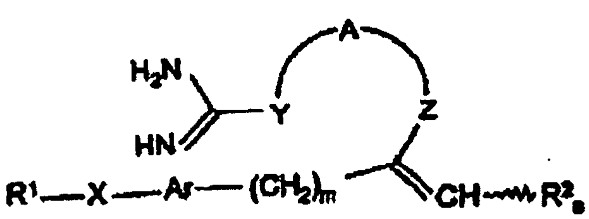

(III)

ou un sel de celui-ci, pour donner un composé de formule :

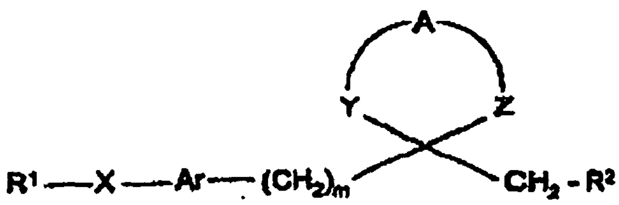

(I-f)

ou un sel de celui-ci ; ou

(6) de faire réagir un composé de la formule (I-b) ci-dessus ou son dérivé réactif au groupe carboxy, ou un sel de celui-ci, avec une amine optiquement active ou son dérivé réactif au groupe amino, ou un sel de celui-ci, pour donner un composé de la formule :

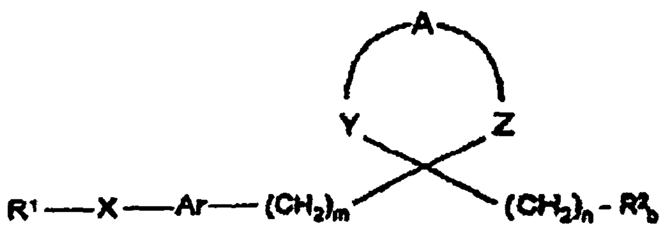

(I-g)

ou un sel de celui-ci ; ou

(7) de soumettre un composé de la formule :

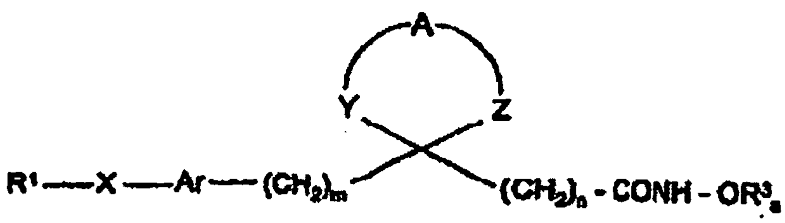

(I-h)

ou un sel de celui-ci à une réaction d'enlèvement du groupe protecteur de l'hydroxy, pour donner un composé de formule :

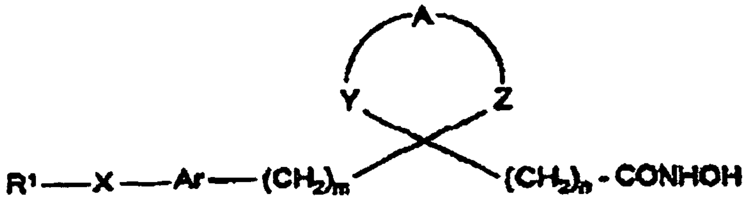

(I-i).

ou un sel de celui-ci ; ou

(8) d'oxyder un composé de la formule :

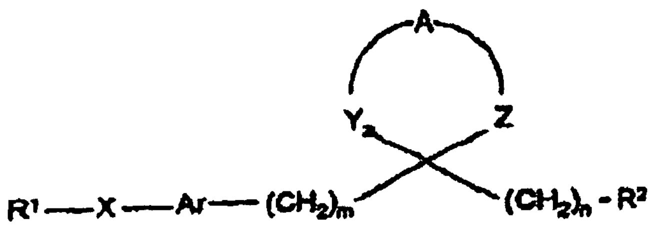

(I-j)

ou un sel de celui-ci, pour donner un compose de formule :

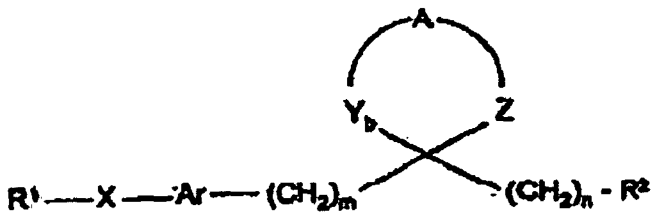

(I-k)

ou un sel de celui-ci , ou

(9) de faire réagir un composé de la formule (I-c) ci-dessus ou un sel de celui-ci, avec un composé de formule :

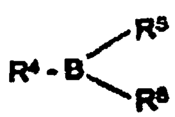

(V)

pour donner un composé de formule :

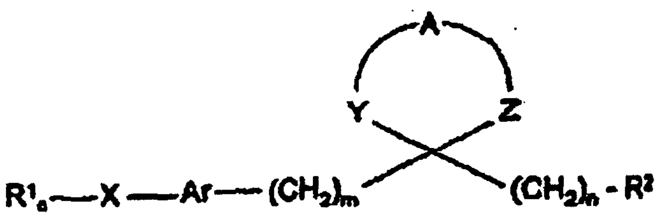

(I-l)

ou un sel de celui-ci ; ou

(10) de faire réagir un composé de formule :

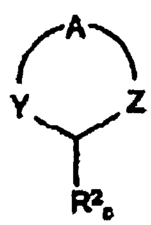

(VI)

ou un sel de celui-ci, avec un composé de la formule :

$$R^1\text{-X-Ar-}(CH_2)_m1\text{ - L} \qquad \text{(VII)}$$

ou un sel de celui-ci, pour donner un composé de formule :

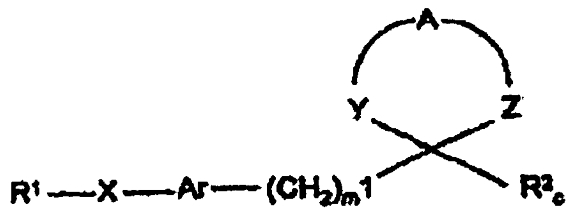

(I-m)

ou un sel de celui-ci ; ou

(11) de cycliser un composé de formule :

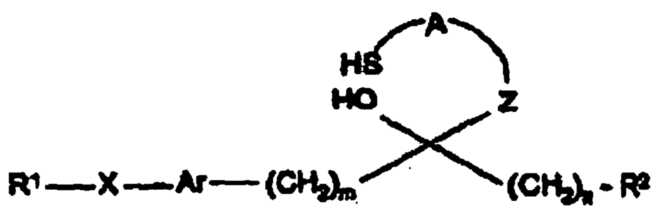

(VIII)

ou un sel de celui-ci, pour donner un composé de formule :

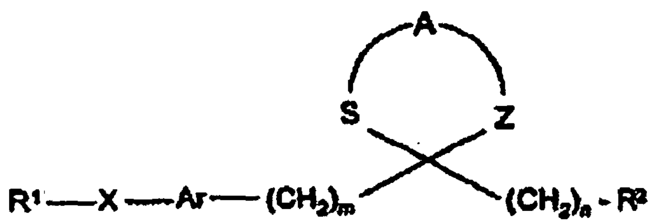

(I-n)

ou un sel de celui-ci ; ou

(12) d'amider un composer de formule :

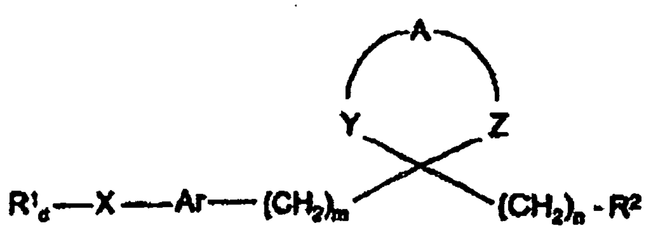

(I-p)

ou son dérivé réactif au groupe carboxy,
ou un sel de celui-ci, pour donner un composé de formule :

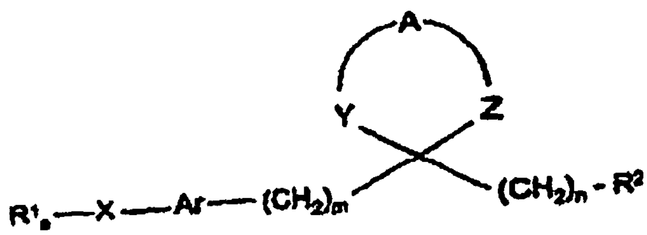

(I-q)

ou un sel de celui-ci ; ou

(13) d'acyler un composé de la formule :

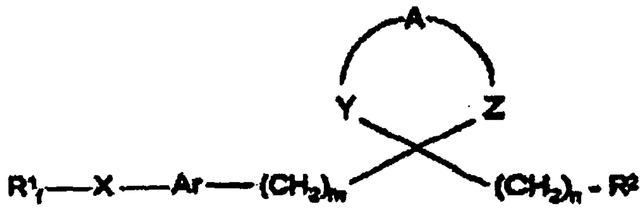

(I-r)

ou son dérivé réactif au groupe amino,
ou un sel de celui-ci, pour donner un composé de formule :

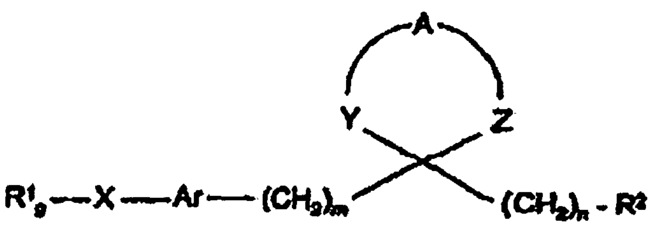

(I-s)

ou un sel de celui-ci ; ou

(14) de soumettre un composé de formule :

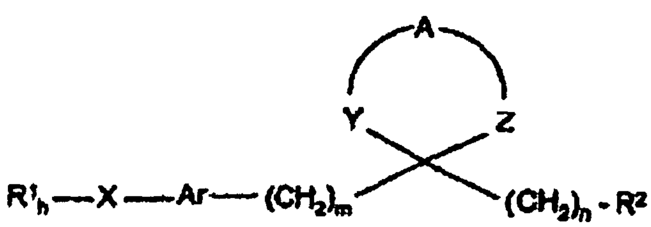

(I-t)

ou un sel de celui-ci, à une réaction d'enlèvement du groupe protecteur de l'amino, pour donner un composé de formule :

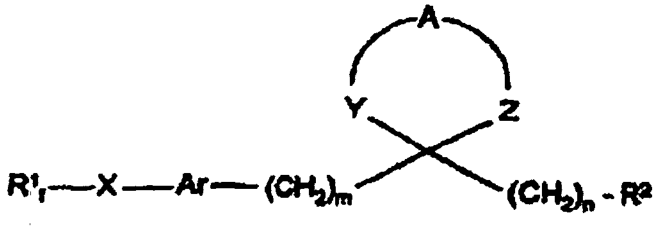

(I-r)

ou un sel de celui-ci ; ou

(15) de soumettre un composé de la formule :

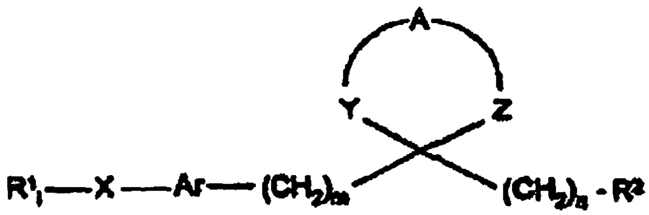

(I-u)

ou un sel de celui-ci, à une réaction d'enlèvement du groupe protecteur de l'hydroxy, pour donner un composé de formule :

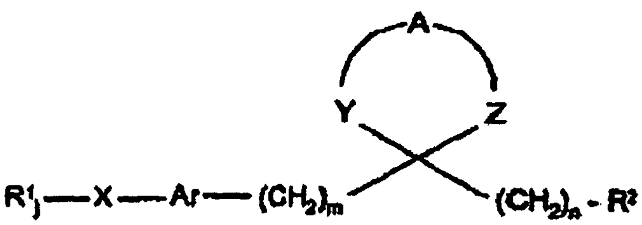

(I-v)

ou un sel de celui-ci ; ou

(16) d'oxyder un composé de formule :

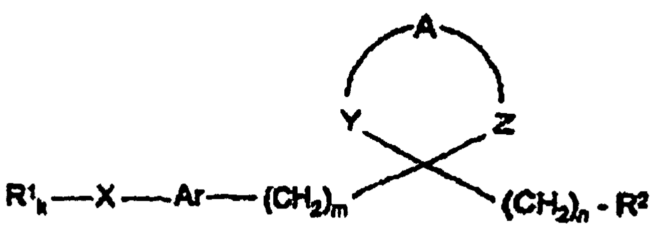

(I-w)

ou un sel de celui-ci, pour donner un composé de formule :

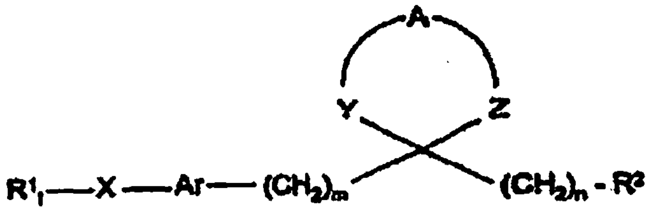

(I-x)

ou un sel de celui-ci ; ou

(17) de réduire un composé de formule :

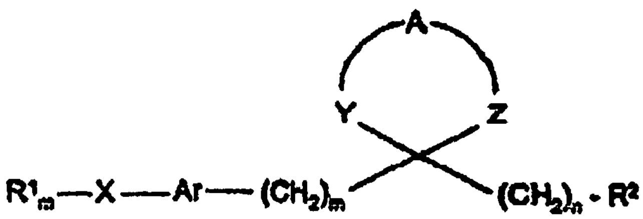

(I-y)

ou un sel de celui-ci, pour donner un composé de formule :

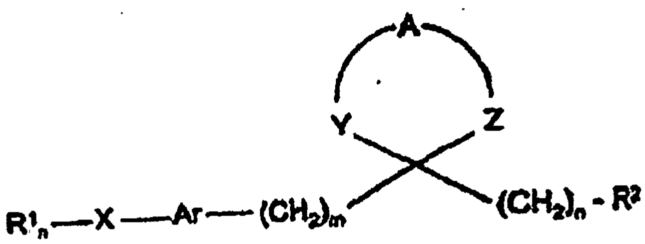

(I-z)

ou un sel de celui-ci ; ou

(18) d'oxyder un composé de formule :

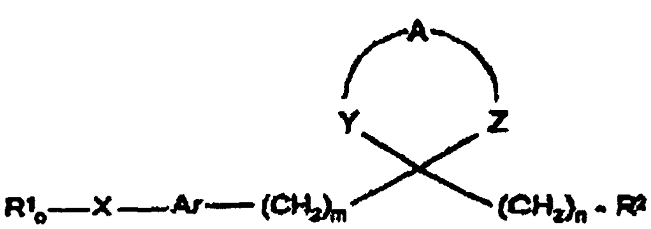

(I-aa)

ou un sel de celui-ci, pour donner un composé de formule :

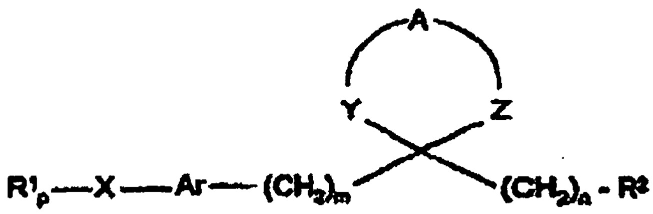

(I-ab)

ou un sel de celui-ci ; ou

(19) d'acyler un composé de la formule :

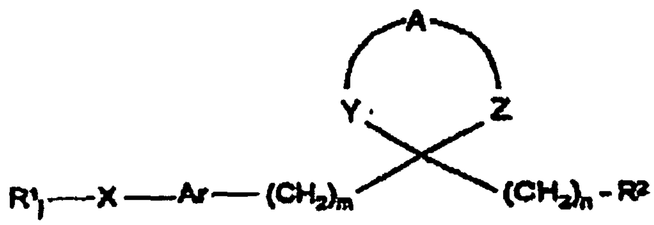

(I-v)

ou un sel de celui-ci, pour donner un composé de formule :

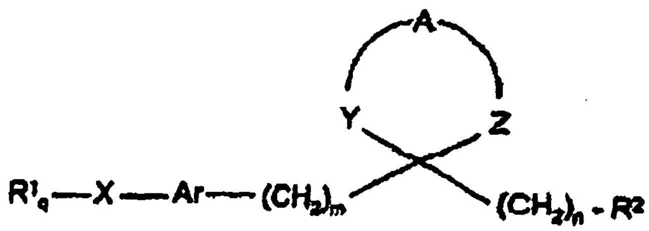

(I-ac)

ou un sel de celui-ci ; ou

(20) de soumettre un composé de la formule :

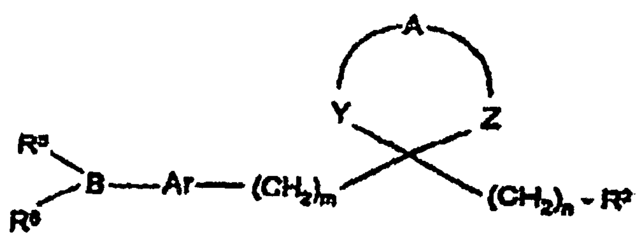

(XI)

ou un sel de celui-ci, avec un composé de la formule :

$$R^1 - L \quad (XII)$$

ou un sel de celui-ci, pour donner un composé de formule :

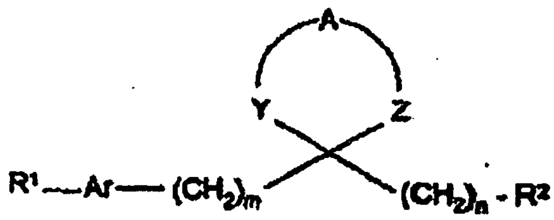

(I-ad)

ou un sel de celui-ci ; ou

(21) de soumettre un composé de la formule :

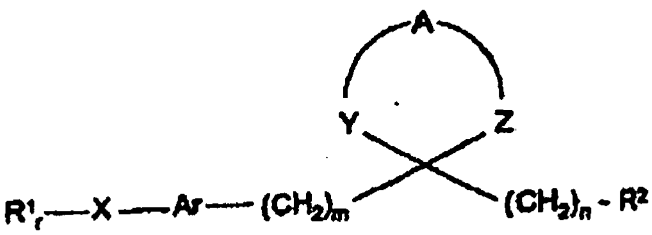

(I-ae)

ou un sel de celui-ci, à une réaction d'enlèvement du groupe protecteur du carboxy, pour donner un composé de formule :

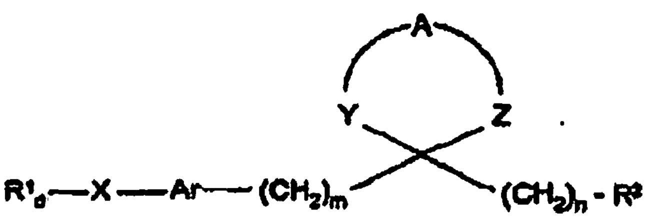

(I-p)

ou un sel de celui-ci ; ou

(22) de faire réagir un composé de la formule :

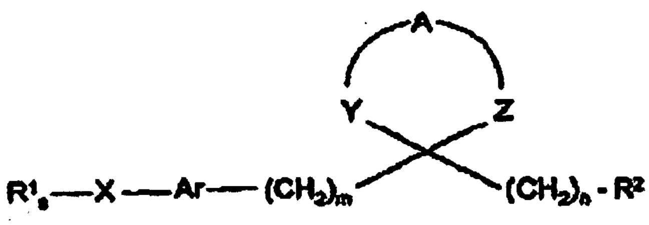

(I-af)

ou un sel de celui-ci, avec une amine substituée, pour donner un composé de formule :

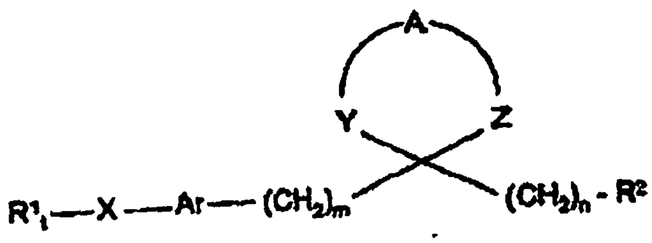

(I-ag)

ou un sel de celui-ci,
où $R^1$, $R^2$, Ar, A, X, Y, Z, m et n sont chacun tels que définis ci-dessus,
$R^1_a$ est un haloaryle ou un halo,
$R^1_b$ est un aryle,
$R^1_c$ est un aryle au moins substitué par un aryle optionnellement substitué,
$R^1_d$ est un aryle au moins ayant un fragment carboxy,
$R^1_e$ est un aryle au moins ayant un fragment amido,
$R^1_f$ est un aryle au moins ayant un fragment amino,
$R^1_g$ est un aryle au moins ayant un fragment acylamino,
$R^1_h$ est un aryle au moins ayant un fragment amino protégée,
$R^1_i$ est un aryle au moins ayant un fragment hydroxy protégée,
$R^1_j$ est un aryle au moins ayant un fragment hydroxy,
$R^1_k$ est un aryle au moins ayant un fragment thia,
$R^1_l$ est un aryle au moins ayant un fragment sulfinyle ou sulfonyle,
$R^1_m$ est un aryle au moins ayant un fragment formyle,
$R^1_n$ est un aryle au moins ayant un fragment hydroxyméthyle,
$R^1_o$ est un aryle au moins ayant un fragment vinyle,
$R^1_p$ est un aryle au moins ayant un fragment 1,2-dihydroxyéthyle,
$R^1_q$ est un aryle au moins ayant un fragment acyloxy,
$R^1_r$ est un aryle au moins ayant un fragment carboxy protégée,
$R^1_s$ est un aryle au moins ayant un fragment haloalcanoyle en $C_1$-$C_6$,
$R^1_t$ est un aryle au moins ayant un fragment aminoalcanoyle en $C_1$-$C_6$ substitué,
$R^2_a$ est un carboxy protégé,
$R^2_b$ est un amide optiquement actif,
$R^2_c$ est un carboxy protégé,
$R^3$ est un hydrogène ou un groupe protecteur de l'hydroxy,
$R^3_a$ est un groupe protecteur de l'hydroxy,
$R^4$ est un aryle optionnellement substitué,
$R^5$ et $R^6$ sont chacun un hydrogène ou combinés ensemble pour former un alkylène en $C_1$-$C_6$,
$Y_a$ est un thia ou un sulfinyle,
$Y_b$ est un sulfinyle ou un sulfonyle,
L est un groupe partant, et
ml est un nombre entier de 1 à 6.

**10.** composition pharmaceutique qui comprend le composé de l'une quelconque des revendications 1 à 8 ou un sel pharmaceutiquement acceptable de celui-ci et un support ou excipient pharmaceutiquement acceptable.

**11.** Procédé pour préparer une composition pharmaceutique qui comprend de mélanger le composé de l'une quelconque des revendications 1 à 8 ou un sel pharmaceutiquement acceptable de celui-ci avec un support ou excipient pharmaceutiquement acceptable.

**12.** Utilisation du composé de l'une quelconque des revendications 1 à 8 ou un sel pharmaceutiquement acceptable de celui-ci pour la fabrication d'un médicament pour l'inhibition des métalloprotéinases de la matrice extracellulaire ("*matrix metalloproteinases*") (MMP) ou du facteur de nécrose de tumeur $\alpha$ ("*tumor necrosis factor* $\alpha$") (TNF $\alpha$).

**13.** Utilisation du composé de l'une quelconque des revendications 1 à 8 ou d'un sel pharmaceutiquement acceptable de celui-ci pour la fabrication d'un médicament pour traiter et/ou prévenir des maladies liées aux MMP ou TNP $\alpha$.